(19) Europäisches Patentamt / European Patent Office / Office européen des brevets

(11) **EP 4 670 791 A2**

(12) **EUROPEAN PATENT APPLICATION**


(43) Date of publication:
**31.12.2025 Bulletin 2026/01**

(21) Application number: **25195382.4**

(22) Date of filing: **21.10.2021**

(51) International Patent Classification (IPC):
***A61P 35/00*** *(2006.01)*

(52) Cooperative Patent Classification (CPC):
**C07D 207/416; A61P 1/16; A61P 13/12; A61P 19/10; A61P 29/00; A61P 35/00; C07D 213/30; C07D 213/64; C07D 231/12; C07D 231/14; C07D 235/16; C07D 277/587; C07D 401/06; C07D 413/04; C07D 417/04;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2020 PCT/CN2020/122794**
**17.08.2021 PCT/CN2021/113042**
**09.10.2021 PCT/CN2021/122851**

(62) Document number(s) of the earlier application(s) in accordance with Art. 76 EPC:
**21802197.0 / 4 232 431**



(71) Applicant: **Lhotse Bio, Inc.**
**South San Francisco, CA 94080 (US)**

(72) Inventors:
- **ZHANG, Haizhen**
  **Shanghai, 200003 (CN)**
- **HANSON, Michael**
  **San Francisco, 94080 (US)**
- **JENNINGS, Andrew**
  **San Francisco, 94080 (US)**
- **LEI, Hui**
  **Shanghai, 200003 (CN)**
- **LIN, Xichen**
  **Shanghai, 200003 (CN)**
- **ZHANG, Qiong**
  **Shanghai, 200003 (CN)**
- **COTE, Alexandre**
  **Laval, H7X 1X7 (CA)**
- **RUVINSKY, Anatoly M**
  **San Francisco, 94080 (US)**

(74) Representative: **Carpmaels & Ransford LLP**
**One Southampton Row**
**London WC1B 5HA (GB)**


Remarks:
•This application was filed on 12.08.2025 as a divisional application to the application mentioned under INID code 62.
•Claims filed after the date of filing of the application / after the date of receipt of the divisional application (Rule 68(4) EPC).

(54) **COMPOUNDS AND COMPOSITIONS FOR TREATING CONDITIONS ASSOCIATED WITH LPA RECEPTOR ACTIVITY**


(57) This disclosure relates to LPA antagonists of Formula (**I**):

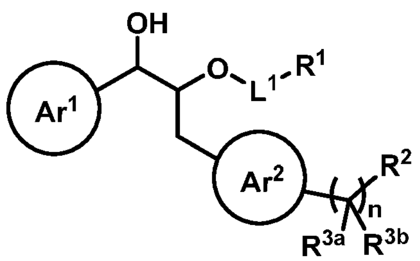

Formula (**I**),

including pharmaceutically acceptable salts and solvates thereof, and pharmaceutical compositions including the same.


EP 4 670 791 A2

(52) Cooperative Patent Classification (CPC): (Cont.)
**C07D 471/04; C07D 487/04**

## Description

### TECHNICAL FIELD

The present application describes LPA antagonists, as well as pharmaceutical compositions comprising the compounds disclosed herein. Also provided are methods for treating LPA-associated diseases, disorders, and conditions.

### BACKGROUND

Various lipid mediators, including eicosanoid and platelet activating factor (PAF) are produced by the activity of phospholipase from cell membranes. Lysophospholipids are one class of these membrane-derived bioactive lipid mediators, and include lysophosphatidic acid (LPA). LPA is not a single molecular entity but a collection of endogenous structural variants with fatty acids of varied lengths and degrees of saturation (Fujiwara et al., J Biol. Chem., 2005, 280, 35038-35050). The structural backbone of the LPAs is derived from glycerol-based phospholipids such as phosphatidylcholine (PC) or phosphatidic acid (PA) and has the general structure:

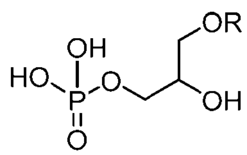

wherein R is acyl, alkyl, or alkenyl. LPAs include, for example, lysophosphatidic acid (1-acylshydroxy-sn-glycero-3-phosphate; LPA), sphingosine 1-phosphate (SIP), lysophosphatidylcholine (LPC), and sphingosylphosphorylcholine (SPC).

LPAs affect cellular functions that include cellular proliferation, differentiation, survival, migration, adhesion, invasion, and morphogenesis. These functions influence many biological processes that include neurogenesis, angiogenesis, wound healing, immunity, and carcinogenesis. LPA has a role as a biological effector molecule, and has a diverse range of physiological actions such as, but not limited to, effects on blood pressure, platelet activation, and smooth muscle contraction, and a variety of cellular effects, which include cell growth, cell rounding, neurite retraction, and actin stress fiber formation and cell migration. The effects of LPA are predominantly receptor mediated. Activation of the LPA receptors ($LPA_1$, $LPA_2$, $LPA_3$, $LPA_4$, $LPA_5$, $LPA_6$) with LPA mediates a range of downstream signaling cascades.

Thus, antagonizing LPA receptors (such as the $LPA_1$ receptor) may be useful for the treatment of a variety of disorders, including fibrosis such as pulmonary fibrosis, hepatic fibrosis, renal fibrosis, arterial fibrosis and systemic sclerosis, and thus the diseases that result from fibrosis (e.g., pulmonary fibrosis, for example, Idiopathic Pulmonary Fibrosis (IPF), hepatic fibrosis, including Non-alcoholic Steatohepatitis (NASH), renal fibrosis, such as diabetic nephropathy, systemic sclerosis-scleroderma, etc.).

### SUMMARY

The present application describes LPA antagonists, as well as pharmaceutical compositions comprising the compounds disclosed herein. Also provided are methods for treating LPA-associated diseases, disorders, and conditions.

Accordingly, provided herein are compounds of Formula (I):

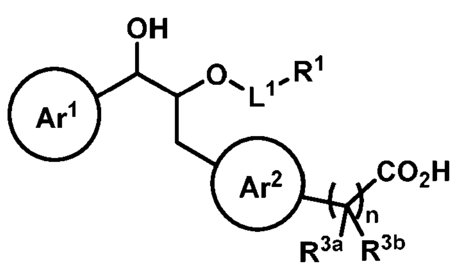

Formula (**I**)

or a pharmaceutically acceptable salt thereof, wherein:

**$L^1$** is selected from the group consisting of:

- a bond; and
- $C_{1-6}$ alkylene optionally substituted with from 1-6 $\mathbf{R^a}$;

$\mathbf{R^1}$ is selected from the group consisting of: $\mathbf{R^b}$; $C_{2-6}$ alkenyl optionally substituted with from 1-6 $\mathbf{R^a}$; and $C_{2-6}$ alkynyl optionally substituted with from 1-6 $\mathbf{R^a}$;

$\mathbf{Ar^1}$ is selected from the group consisting of:

- $C_{6-10}$ aryl optionally substituted with from 1-4 substituents each independently selected from the group consisting of: $\mathbf{R^{c1}}$ and $\mathbf{-(L^b)_b-R^b}$; and
- heteroaryl including from 5-10 ring atoms, wherein from 1-4 ring atoms are ring heteroatoms each independently selected from the group consisting of: N, N(H), N($\mathbf{R^d}$), O, and S, wherein the heteroaryl is optionally substituted with from 1-4 substituents each independently selected from the group consisting of: $\mathbf{R^{c1}}$ and - $\mathbf{(L^b)_b-R^b}$;

$\mathbf{Ar^2}$ is selected from the group consisting of:

- $C_{6-10}$ arylene optionally substituted with from 1-4 $\mathbf{R^{c2}}$; and
- heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$;

n is 0 or 1;

$\mathbf{R^{3a}}$ and $\mathbf{R^{3b}}$ are independently H, -halo, $C_{1-6}$ alkyl, or $C_{1-4}$ haloalkyl; or

$\mathbf{R^{3a}}$ and $\mathbf{R^{3b}}$ taken together with the carbon atom to which each is attached forms a $C_{3-6}$ cycloalkyl;

each occurrence of $\mathbf{R^a}$ is independently selected from the group consisting of: -OH; -halo; -N$\mathbf{R^eR^f}$; $C_{1-4}$ alkoxy; $C_{1-4}$ haloalkoxy; -C(=O)O($C_{1-4}$ alkyl); -C(=O)($C_{1-4}$ alkyl); -C(=O)OH; -CON**R'R''**; -S(O)$_{1-2}$N**R'R''**; -S(O)$_{1-2}$($C_{1-4}$ alkyl); and cyano;

each occurrence of $\mathbf{R^b}$ is independently selected from the group consisting of:

- $C_{3-10}$ cycloalkyl or $C_{3-10}$ cycloalkenyl, each of which is optionally substituted with from 1-4 $\mathbf{R^g}$;
- heterocyclyl or heterocycloalkenyl including from 3-10 ring atoms, wherein from 1-3 ring atoms are heteroatoms, each independently selected from the group consisting of N, N(H), N($\mathbf{R^d}$), O, and S(O)$_{0-2}$, and wherein the heterocyclyl or heterocycloalkenyl is optionally substituted with from 1-4 $\mathbf{R^g}$;
- heteroaryl including from 5-10 ring atoms, wherein from 1-4 ring atoms are heteroatoms, each independently selected from the group consisting of N, N(H), N($\mathbf{R^d}$), O, and S(O)$_{0-2}$, and wherein the heteroaryl is optionally substituted with from 1-4 $\mathbf{R^g}$; and
- $C_{6-10}$ aryl optionally substituted with from 1-4 $\mathbf{R^g}$;

b is 0, 1, 2, or 3;

each occurrence of $\mathbf{L^b}$ is selected from the group consisting of: $C_{1-3}$ alkylene; - N(H)-; N($\mathbf{R^d}$)-; -O-; -S-; C(=O); and S(O)$_{1-2}$;

each occurrence of $\mathbf{R^{c1}}$ and $\mathbf{R^{c2}}$ is independently selected from the group consisting of: halo; cyano; $C_{1-10}$ alkyl which is optionally substituted with from 1-6 independently selected $\mathbf{R^a}$; $C_{2-6}$ alkenyl; $C_{2-6}$ alkynyl; $C_{1-4}$ alkoxy; $C_{1-4}$ haloalkoxy; - S(O)$_{0-2}$($C_{1-4}$ alkyl); -N$\mathbf{R^eR^f}$; -OH; -S(O)$_{1-2}$N**R'R''**; -$NO_2$; -C(=O)($C_{1-10}$ alkyl); - C(=O)O($C_{1-4}$ alkyl); -C(=O)OH; and -C(=O)N**R'R''**;

each occurrence of $\mathbf{R^d}$ is independently selected from the group consisting of: $C_{1-6}$ alkyl optionally substituted with from 1-3 independently selected $\mathbf{R^a}$; -C(O)($C_{1-4}$ alkyl); -C(O)O($C_{1-4}$ alkyl); -CON**R'R''**; -S(O)$_{1-2}$N**R'R''**; -S(O)$_{1-2}$($C_{1-4}$ alkyl); -OH; and $C_{1-4}$ alkoxy;

each occurrence of $\mathbf{R^e}$ and $\mathbf{R^f}$ is independently selected from the group consisting of: H; $C_{1-6}$ alkyl; -C(O)($C_{1-4}$ alkyl); -C(O)O($C_{1-4}$ alkyl); -CON**R'R''**; - S(O)$_{1-2}$N**R'R''**; -S(O)$_{1-2}$($C_{1-4}$ alkyl); -OH; and $C_{1-4}$ alkoxy; and

each occurrence of **R^g** is independently selected from the group consisting of: halo; cyano; $C_{1-6}$ alkyl; $C_{1-6}$ haloalkyl; $C_{1-4}$ alkoxy; $C_{1-4}$ haloalkoxy; -OH; and N**R'R"**; and

each occurrence of **R'** and **R"** is independently selected from the group consisting of: H; -OH; and $C_{1-4}$ alkyl.

Also provided herein are pharmaceutical compositions comprising a compound of Formula **(I),** or a pharmaceutically acceptable salt or solvate thereof, and a pharmaceutically acceptable excipient.
Also provided herein are methods for treating or preventing an LPA-associated disease in a subject in need thereof, the method comprising administering to subject a therapeutically effective amount of a compound of Formula **(I)** (e.g., a compound of Formulas **I-A, I-1**, **I-2**, or **I-3**), or a pharmaceutically acceptable salt or solvate thereof, or a pharmaceutical composition thereof. In some embodiments, the LPA-associated disease is an $LPA_1$-associated disease.
In some embodiments, the LPA-associated disease is selected from the group consisting of fibrosis, transplant rejection, cancer, osteoporosis, or inflammatory disorders. In certain of these embodiments, the fibrosis is pulmonary, liver, renal, cardiac, dermal, ocular, or pancreatic fibrosis. In certain embodiments, the cancer is of the bladder, blood, bone, brain, breast, central nervous system, cervix, colon, endometrium, esophagus, gall bladder, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid.
In some embodiments, the LPA-associated disease is selected from the group consisting of idiopathic pulmonary fibrosis (IPF), non-alcoholic steatohepatitis (NASH), non-alcoholic fatty liver disease (NAFLD), chronic kidney disease, diabetic kidney disease, and systemic sclerosis.
Also provided herein are methods for treating or preventing fibrosis in a subject in need thereof, the method comprising administering to subject a therapeutically effective amount of a compound of Formula **(I)** (e.g., a compound of Formulas **I-A, I-1**, **I-2**, or **I-3**), or a pharmaceutically acceptable salt or solvate thereof, or a pharmaceutical composition thereof.
In some embodiments, the fibrosis is idiopathic pulmonary fibrosis (IPF), nonalcoholic steatohepatitis (NASH), chronic kidney disease, diabetic kidney disease, and systemic sclerosis. For example, the fibrosis can be IPF.
All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.
Other features and advantages of the invention will be apparent from the following detailed description and figures, and from the claims.

## DETAILED DESCRIPTION

Provided herein are LPA antagonists for use in the management of fibrosis and other conditions where inactivation or a reduction of LPA activity is useful.

### *Definitions*

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.
As used herein, the term "halo" or "halogen" means -F (sometimes referred to herein as "fluoro" or "fluoros"), -Cl (sometimes referred to herein as "chloro" or "chloros"), -Br (sometimes referred to herein as "bromo" or "bromos"), and -I (sometimes referred to herein as "iodo" or "iodos").
As used herein, the term "alkyl" refers to saturated linear or branched-chain monovalent hydrocarbon radicals, containing the indicated number of carbon atoms. For example, "$C_{1-6}$ alkyl" refers to saturated linear or branched-chain monovalent hydrocarbon radicals of one to six carbon atoms. Non-limiting examples of alkyl include methyl, ethyl, 1-propyl, isopropyl, 1-butyl, isobutyl, sec-butyl, tert-butyl, 2-methyl-2-propyl, pentyl, neopentyl, and hexyl.
As used herein, the term "alkylene" refers to a divalent alkyl containing the indicated number of carbon atoms. For example, "$C_{1-3}$ alkylene" refers to a divalent alkyl having one to three carbon atoms (e.g.,$-CH_2-$, $-CH(CH_3)-$, $-CH_2CH_2-$, or $-CH_2CH_2CH_2-$).
As used herein, the term "alkenyl" refers to a linear or branched mono-unsaturated hydrocarbon chain, containing the indicated number of carbon atoms. For example, "$C_{2-6}$ alkenyl" refers a linear or branched mono unsaturated hydrocarbon chain of two to six carbon atoms. Non-limiting examples of alkenyl include ethenyl, propenyl, butenyl, or pentenyl.

As used herein, the term "alkynyl" refers to a linear or branched di-unsaturated hydrocarbon chain, containing the indicated number of carbon atoms. For example, "$C_{2-6}$ alkynyl" refers to a linear or branched di-unsaturated hydrocarbon chain having two to six carbon atoms. Non-limiting examples of alkynyl include ethynyl, propynyl, butynyl, or pentynyl.

As used herein, the term "haloalkyl" refers to an alkyl radical as defined herein, wherein one or more hydrogen atoms is replaced with one or more halogen atoms. Non-limiting examples include fluoromethyl, difluoromethyl, trifluoromethyl, 2-fluoroethyl, 2,2-difluoroethyl, 2,2,2-trifluoroethyl, chloromethyl, dichloromethyl, chloroethyl, trichloroethyl, bromomethyl, and iodomethyl.

As used herein, the term "alkoxy" refers to an -O-alkyl radical, wherein the radical is on the oxygen atom. For example, "$C_{1-6}$ alkoxy" refers to an -O-($C_{1-6}$ alkyl) radical, wherein the radical is on the oxygen atom. Examples of alkoxy include methoxy, ethoxy, propoxy, isopropoxy, butoxy and tert-butoxy. Accordingly, as used herein, the term "haloalkoxy" refers to an -O-haloalkyl radical, wherein the radical is on the oxygen atom.

As used herein, the term "alkynyl" refers to an acyclic hydrocarbon chain that may be a straight chain or branched chain having one or more carbon-carbon triple bonds. The alkynyl moiety contains the indicated number of carbon atoms. For example, $C_{2-6}$ indicates that the group may have from 2 to 6 (inclusive) carbon atoms in it. Alkynyl groups can either be unsubstituted or substituted with one or more substituents.

As used herein, the term "aryl" refers to a 6-20 carbon mono-, bi-, tri- or polycyclic group wherein at least one ring in the system is aromatic (e.g., 6-carbon monocyclic, 10-carbon bicyclic, or 14-carbon tricyclic aromatic ring system); and wherein 0, 1, 2, 3, or 4 atoms of each ring may be substituted by a substituent. Examples of aryl groups include phenyl, naphthyl, tetrahydronaphthyl, and the like.

As used herein, the term "cycloalkyl" as used herein refers to cyclic saturated hydrocarbon groups having, e.g., 3 to 20 ring carbons, preferably 3 to 16 ring carbons, and more preferably 3 to 12 ring carbons or 3-10 ring carbons or 3-6 ring carbons, wherein the cycloalkyl group may be optionally substituted. Examples of cycloalkyl groups include, without limitation, cyclopropyl, cyclobutyl, cyclopentyl, cyclohexyl, cycloheptyl, and cyclooctyl. Cycloalkyl may include multiple fused and/or bridged rings. Non-limiting examples of fused/bridged cycloalkyl includes: bicyclo[1.1.0]butane, bicyclo[2.1.0]pentane, bicyclo[1.1.1]pentane, bicyclo[3.1.0]hexane, bicyclo[2.1.1]hexane, bicyclo[3.2.0]heptane, bicyclo[4.1.0]heptane, bicyclo[2.2.1]heptane, bicyclo[3.1.1]heptane, bicyclo[4.2.0]octane, bicyclo[3.2.1]octane, bicyclo[2.2.2]octane, and the like. Cycloalkyl also includes spirocyclic rings (e.g., spirocyclic bicycle wherein two rings are connected through just one atom). Non-limiting examples of spirocyclic cycloalkyls include spiro[2.2]pentane, spiro[2.5]octane, spiro[3.5]nonane, spiro[3.5]nonane, spiro[3.5]nonane, spiro[4.4]nonane, spiro[2.6]nonane, spiro[4.5]decane, spiro[3.6]decane, spiro[5.5]undecane, and the like. The term "saturated" as used in this context means only single bonds present between constituent carbon atoms.

As used herein, the term "cycloalkenyl" as used herein means partially unsaturated cyclic hydrocarbon groups having 3 to 20 ring carbons, preferably 3 to 16 ring carbons, and more preferably 3 to 12 ring carbons or 3-10 ring carbons or 3-6 ring carbons, wherein the cycloalkenyl group may be optionally substituted. Examples of cycloalkenyl groups include, without limitation, cyclopentenyl, cyclohexenyl, cycloheptenyl, and cyclooctenyl. As partially unsaturated cyclic hydrocarbon groups, cycloalkenyl groups may have any degree of unsaturation provided that one or more double bonds is present in the ring, none of the rings in the ring system are aromatic, and the cycloalkenyl group is not fully saturated overall. Cycloalkenyl may include multiple fused and/or bridged and/or spirocyclic rings.

As used herein, the term "heteroaryl", as used herein, means a mono-, bi-, tri- or polycyclic group having 5 to 20 ring atoms, alternatively 5, 6, 9, 10, or 14 ring atoms; and having 6, 10, or 14 pi electrons shared in a cyclic array; wherein at least one ring in the system is aromatic, and at least one ring in the system contains one or more heteroatoms independently selected from the group consisting of N, O, and S (but does not have to be a ring which contains a heteroatom, e.g. tetrahydroisoquinolinyl, e.g., tetrahydroquinolinyl). Heteroaryl groups can either be unsubstituted or substituted with one or more substituents. Examples of heteroaryl include thienyl, pyridinyl, furyl, oxazolyl, oxadiazolyl, pyrrolyl, imidazolyl, triazolyl, thiodiazolyl, pyrazolyl, isoxazolyl, thiadiazolyl, pyranyl, pyrazinyl, pyrimidinyl, pyridazinyl, triazinyl, thiazolyl benzothienyl, benzoxadiazolyl, benzofuranyl, benzimidazolyl, benzotriazolyl, cinnolinyl, indazolyl, indolyl, isoquinolinyl, isothiazolyl, naphthyridinyl, purinyl, thienopyridinyl, pyrido[2,3-*d*]pyrimidinyl, pyrrolo[2,3-*b*]pyridinyl, quinazolinyl, quinolinyl, thieno[2,3-*c*]pyridinyl, pyrazolo[3,4-*b*]pyridinyl, pyrazolo[3,4-*c*]pyridinyl, pyrazolo[4,3-*c*]pyridine, pyrazolo[4,3-*b*]pyridinyl, tetrazolyl, chromane, 2,3-dihydrobenzo[*b*][1,4]dioxine, benzo[*d*][1,3]dioxole, 2,3-dihydrobenzofuran, tetrahydroquinoline, 2,3-dihydrobenzo[*b*][1,4]oxathiine, isoindoline, and others. In some embodiments, the heteroaryl is selected from thienyl, pyridinyl, furyl, pyrazolyl, imidazolyl, isoindolinyl, pyranyl, pyrazinyl, and pyrimidinyl.

As used herein, the term "heterocyclyl" refers to a mon-, bi-, tri-, or polycyclic saturated ring system with 3-16 ring atoms (e.g., 5-8 membered monocyclic, 8-12 membered bicyclic, or 11-14 membered tricyclic ring system) having 1-3 heteroatoms if monocyclic, 1-6 heteroatoms if bicyclic, or 1-9 heteroatoms if tricyclic or polycyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2 or 3 atoms of each ring may be substituted by a substituent. Examples of heterocyclyl groups include piperazinyl, pyrrolidinyl, dioxanyl, morpholinyl, tetrahydrofuranyl, and the like. Heterocyclyl may include multiple fused and bridged rings. Non-limiting examples of fused/bridged heteorocyclyl includes: 2-azabicyclo[1.1.0]butane, 2-azabicyclo[2.1.0]pentane, 2-azabicyclo[1.1.1]pentane, 3-azabicyclo[3.1.0]hexane, 5-azabicyclo[2.1.1]hexane, 3-azabicyclo[3.2.0]heptane, octahydrocyclopenta[c]pyrrole, 3-azabicyclo[4.1.0]heptane, 7-azabicyclo[2.2.1]heptane, 6-azabicyclo[3.1.1]heptane, 7-azabicyclo[4.2.0]octane, 2-azabicyclo[2.2.2]octane, 3-azabicyclo[3.2.1]octane, 2-oxabicyclo[1.1.0]butane, 2-oxabicyclo[2.1.0]pentane, 2-oxabicyclo[1.1.1]pentane, 3-oxabicyclo[3.1.0]hexane, 5-oxabicyclo[2.1.1]hexane, 3-oxabicyclo[3.2.0]heptane, 3-oxabicyclo[4.1.0]heptane, 7-oxabicyclo[2.2.1]heptane, 6-oxabicyclo[3.1.1]heptane, 7-oxabicyclo[4.2.0]octane, 2-oxabicyclo[2.2.2]octane, 3-oxabicyclo[3.2.1]octane, and the like. Heterocyclyl also includes spirocyclic rings (e.g., spirocyclic bicycle wherein two rings are connected through just one atom). Non-limiting examples of spirocyclic heterocyclyls include 2-azaspiro[2.2]pentane, 4-azaspiro[2.5]octane, 1-azaspiro[3.5]nonane, 2-azaspiro[3.5]nonane, 7-azaspiro[3.5]nonane, 2-azaspiro[4.4]nonane, 6-azaspiro[2.6]nonane, 1,7-diazaspiro[4.5]decane, 7-azaspiro[4.5]decane 2,5-diazaspiro[3.6]decane, 3-azaspiro[5.5]undecane, 2-oxaspiro[2.2]pentane, 4-oxaspiro[2.5]octane, 1-oxaspiro[3.5]nonane, 2-oxaspiro[3.5]nonane, 7-oxaspiro[3.5]nonane, 2-oxaspiro[4.4]nonane, 6-oxaspiro[2.6]nonane, 1,7-dioxaspiro[4.5]decane, 2,5-dioxaspiro[3.6]decane, 1-oxaspiro[5.5]undecane, 3-oxaspiro[5.5]undecane, 3-oxa-9-azaspiro[5.5]undecane and the like. The term "saturated" as used in this context means only single bonds present between constituent ring atoms and other available valences occupied by hydrogen and/or other substituents as defined herein.

As used herein, the term "heterocycloalkenyl" as used herein means partially unsaturated cyclic ring system with 3-16 ring atoms (e.g., 5-8 membered monocyclic, 8-12 membered bicyclic, or 11-14 membered tricyclic ring system) having 1-3 heteroatoms if monocyclic, 1-6 heteroatoms if bicyclic, or 1-9 heteroatoms if tricyclic or polycyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2 or 3 atoms of each ring may be substituted by a substituent. Examples of heterocycloalkenyl groups include, without limitation, tetrahydropyridyl, dihydropyrazinyl, dihydropyridyl, dihydropyrrolyl, dihydrofuranyl, dihydrothiophenyl. As partially unsaturated cyclic groups, heterocycloalkenyl groups may have any degree of unsaturation provided that one or more double bonds is present in the ring, none of the rings in the ring system are aromatic, and the heterocycloalkenyl group is not fully saturated overall. Heterocycloalkenyl may include multiple fused and/or bridged and/or spirocyclic rings.

As used herein, "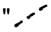" indicates an optional single or double bond, as allowed by valence. As used herein, "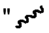" indicates the point of attachment to the parent molecule.

As used herein, the term "compound," is meant to include all stereoisomers, geometric isomers, tautomers, and isotopes of the structures depicted. Compounds herein identified by name or structure as one particular tautomeric form are intended to include other tautomeric forms unless otherwise specified.

The term "tautomer" as used herein refers to compounds whose structures differ markedly in arrangement of atoms, but which exist in easy and rapid equilibrium, and it is to be understood that compounds provided herein may be depicted as different tautomers, and when compounds have tautomeric forms, all tautomeric forms are intended to be within the scope of the disclosure, and the naming of the compounds does not exclude any tautomer.

As used herein, the term "carboxylic acid bioisostere" means a group which has chemical and physical similarities producing broadly similar biological properties to a carboxylic acid (see Lipinski, Annual Reports in Medicinal Chemistry, 1986,21,p283 "Bioisosterism In Drug Design"; Yun, Hwahak Sekye, 1993, 33, pages 576-579 "Application Of Bioisosterism To New Drug Design"; Zhao, Huaxue Tongbao, 1995, pages 34-38 25 "Bioisosteric Replacement And Development Of Lead Compounds In Drug Design"; Graham, Theochem, 1995, 343, pages 105-109 "Theoretical Studies Applied To Drug Design:ab initio Electronic Distributions In Bioisosteres"). Examples of suitable carboxylic acid bioisostere include: sulfo, phosphono, alkylsulfonylcarbamoyl, tetrazolyl, arylsulfonylcarbamoyl, heteroarylsulfonylcarbamoyl, N-methoxycarbamoyl, 3-hydroxy-3-cyclobutene-1,2-dione, 3,5-dioxo-1,2,4-oxadiazolidinyl, heterocyclic phenols such as 3-hydroxyisoxazolyl and 3-hydoxy-1-methylpyrazolyl, amido such as $-CONH_2$, $-CONHSO_2Me$, or

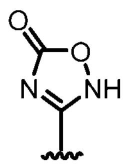

.

The term "LPA-associated disease" as used herein is meant to include, without limitation, those diseases, disorders, or conditions in which activation of at least one LPA receptor by LPA contributes to the symptomology or progression of the disease, disorder or condition. These diseases, disorders, or conditions may arise from one or more of a genetic, iatrogenic, immunological, infectious, metabolic, oncological, toxic, surgical, and/or traumatic etiology. Accordingly, inhibiting of one or more lysophosphatidic acid (LPA) receptors (e.g., $LPA_1$, $LPA_2$, $LPA_3$, $LPA_4$, $LPA_5$, or $LPA_6$ receptor) signaling can alter the pathology and/or symptoms and/or progression of the disease, disorder, or condition. In some embodiments, the LPA-associated disease is an LPA1-associated disease, wherein modulating LPA1 receptor

signaling can alter the pathology and/or symptoms and/or progression of the disease, disorder, or condition.

The terms "fibrosis" or "fibrosing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

The term "pharmaceutically acceptable" as used herein indicates that the compound, or salt or composition thereof is compatible chemically and/or toxicologically with the other ingredients comprising a formulation and/or the subject being treated therewith.

The term "therapeutic compound" as used herein is meant to include, without limitation, all compounds of Formula **(I),** or pharmaceutically acceptable salts or solvates thereof (e.g., a compound of any one of Formulas **I-A, I-1**, **I-2**, or **I-3**, or a pharmaceutically acceptable salt or solvate thereof), and all compositions (e.g., pharmaceutical compositions) wherein a compound of Formula **(I),** or a pharmaceutically acceptable salt or solvate thereof (e.g., a compound of any one of Formulas **I-A, I-1, I-2**, or **I-3**, or a pharmaceutically acceptable salt or solvate thereof) is a component of the composition.

The term "administration" or "administering" refers to a method of giving a dosage of a compound or pharmaceutical composition to a vertebrate or invertebrate, including a mammal, a bird, a fish, or an amphibian. The method of administration can vary depending on various factors, e.g., the components of the pharmaceutical composition, the site of the disease, and the severity of the disease.

The terms "effective amount" or "effective dosage" or "pharmaceutically effective amount" or "therapeutically effective amount," as used herein, refer to a sufficient amount of a chemical entity (e.g., a compound of Formula **(I),** or a pharmaceutically acceptable salt or solvate thereof (e.g., a compound of any one of Formulas **I-A, I-1**, **I-2**, or **I-3**, or a pharmaceutically acceptable salt or solvate thereof)) being administered which will relieve to some extent one or more of the symptoms of the disease or condition being treated, and can include curing the disease. "Curing" means that the symptoms of active disease are eliminated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study. In some embodiments, a "therapeutically effective amount" of a compound as provided herein refers to an amount of the compound that is effective as a monotherapy or combination therapy.

The term "excipient" or "pharmaceutically acceptable excipient" means a pharmaceutically-acceptable material, composition, or vehicle, such as a liquid or solid filler, diluent, carrier, solvent, or encapsulating material. In some embodiments, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, e.g., Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutical composition" refers to a mixture of a compound of Formula (I), or a pharmaceutically acceptable salt or solvate thereof (e.g., a compound of any one of Formulas **I-A, I-1**, **I-2**, or **I-3** or a pharmaceutically acceptable salt or solvate thereof) as provided herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to, rectal, oral, intravenous, aerosol, parenteral, ophthalmic, pulmonary, and topical administration.

The terms "treat," "treating," and "treatment," in the context of treating a disease, disorder, or condition, are meant to include alleviating or abrogating a disorder, disease, or condition, or one or more of the symptoms associated with the disorder, disease, or condition; or to slowing the progression, spread or worsening of a disease, disorder or condition or of one or more symptoms thereof.

The term "preventing", as used herein, is the prevention of the onset, recurrence or spread, in whole or in part, of the disease or condition as described herein, or a symptom thereof.

The terms "subject", "patient", or "individual", as used herein, are used interchangeably and refers to any animal, including mammals such as mice, rats, other rodents, rabbits, dogs, cats, swine, cattle, sheep, horses, primates, and humans. In some embodiments, the term refers to a subject, particularly a mammalian subject, for whom diagnosis, prognosis, or therapy is desired or needed. In some embodiments, the subject is a human. In some embodiments, the subject has experienced and/or exhibited at least one symptom of the disease, disorder, or condition to be treated and/or prevented.

The terms "treatment regimen" and "dosing regimen" are used interchangeably to refer to the dose and timing of administration of each therapeutic agent in a combination.

The term "pharmaceutical combination", as used herein, refers to a pharmaceutical treatment resulting from the mixing or combining of more than one active ingredient and includes both fixed and non-fixed combinations of the active ingredients.

The term "combination therapy" as used herein refers to a dosing regimen of two different therapeutically active agents (i.e., the components or combination partners of the combination), wherein the therapeutically active agents are administered together or separately in a manner prescribed by a medical care taker or according to a regulatory agency as defined herein.

The term "modulate", "modulating" or "modulation", as used herein, refers to a regulation or an adjustment (e.g., increase or decrease) and can include, for example agonism, partial agonism or antagonism.

The ability of test compounds to act as inhibitors of an LGA receptor can be demonstrated by assays known in the art. The activity of the compounds and compositions provided herein as LGA receptor inhibitors can be assayed in vitro, in vivo, or in a cell line.

For example, Chinese hamster ovary cells overexpressing human LPA1 can be plated overnight (15,000 cells/well) in microplates in DMEM/F12 medium. Following overnight culture, cells are loaded with calcium indicator dye for 30 minutes at 37 °C. The cells are then equilibrated to room temperature for 30 minutes before the assay. Test compounds solubilized in DMSO are transferred to a multiwell non-binding surface plate and diluted with assay buffer (e.g., IX HBSS with calcium/magnesium, 20 mM HEPES, and 0.1% fatty acid free BSA) to a final concentration of 0.5% DMSO. Diluted compounds are added to the cells at final concentrations ranging from 0.08 nM to 5 mM and are then incubated for 20 min at room temperature at which time LPA is added at final concentrations of 10 nM to stimulate the cells. The compound $IC_{50}$ value is defined as the concentration of test compound which inhibited 50% of the calcium flux induced by LPA alone. $IC_{50}$ values can be determined by fitting data to a 4-parameter logistic equation.

In another example, a compound of Formula (I), or a pharmaceutically acceptable salt or solvate thereof (e.g., a compound of any one of Formulas I-A, **I-1**, **I-2**, or **I-3**, or a pharmaceutically acceptable salt or solvate thereof) as provided herein is dosed orally p.o. 2 hours to CD-1 female mice prior to an LPA challenge. The mice are then dosed via tail vein (IV) with 0.15 mL of LPA in 0.1%BSA/PBS (2 pg/pL). Exactly 2 minutes following the LPA challenge, the mice are euthanized by decapitation and the trunk blood is collected. These samples are collectively centrifuged and individual 75 pL samples are frozen at -20°C until performance of a histamine assay. The plasma histamine analysis can be run by standard EIA (Enzyme Immunoassay) methods. Plasma samples are thawed and diluted 1 :30 in 0.1% BSA in PBS. An EIA protocol for histamine analysis as previously described can be used in this assay.

LPA has a role as a biological effector molecule, and has a diverse range of physiological actions that include effects on blood pressure, platelet activation, and smooth muscle contraction, and a variety of cellular effects, which include cell growth, cell rounding, neurite retraction, and actin stress fiber formation and cell migration. These effectsare predominantly receptor mediated.

Activation of the LPA receptors ($LPA_1$, $LPA_2$, $LPA_3$, $LPA_4$, $LPA_5$, $LPA_6$) with LPA mediates a range of downstream signaling cascades. Non-limiting examples include, mitogen-activated protein kinase (MAPK) activation, adenylyl cyclase (AC) inhibition/activation, phospholipase C (PLC) activation/Ca2+ mobilization, arachidonic acid release, Akt/PKB activation, and the activation of small GTPases, Rho, ROCK, Rae, and Ras. Additional pathways that are affected by LPA receptor activation include, for example, cyclic adenosine monophosphate (cAMP), cell division cycle 42/GTP-binding protein (Cdc42), proto-oncogene serine/threonine-protein kinase Raf (c-RAF), proto- oncogene tyrosine-protein kinase Src (c-src), extracellular signal-regulated kinase (ERK), focal adhesion kinase (FAK), guanine nucleotide exchange factor (GEF), glycogen synthase kinase 3b (GSK3b), c-jun amino-terminal kinase (JNK), MEK, myosin light chain II (MLC II), nuclear factor kB (NF-kB), N-methyl-D-aspartate (NMDA) receptor activation, phosphatidylinositol 3-kinase (PBK), protein kinase A (PKA), protein kinase C (PKC), ms-related C3 botulinum toxin substrate 1 (RAC1). Nearly all mammalian cells, tissues and organs co-express several LPA-receptor subtypes, which indicates that LPA receptors signal in a cooperative manner. LPA1, LPA2, and LPA3 share high amino acid sequence similarity.

$LPA_1$ (previously called VZG-1/EDG-2/mrecl.3) couples with three types of G proteins, $G_{i/o}$, $G_q$, and $G_{12/13}$. Through activation of these G proteins, LPA induces a range of cellular responses through $LPA_1$ including, for example, cell proliferation, serum-response element (SRE) activation, mitogen-activated protein kinase (MAPK) activation, adenylyl cyclase (AC) inhibition, phospholipase C (PLC) activation, $Ca^{2+}$ mobilization, Akt activation, and Rho activation.

Expression of $LPA_1$ is observed in the testis, brain, heart, lung, small intestine, stomach, spleen, thymus, and skeletal muscle of in mice. Similarly, $LPA_1$ is expressed in human tissues such as the brain, heart, lung, placenta, colon, small intestine, prostate, testis, ovary, pancreas, spleen, kidney, skeletal muscle, and thymus.

$LPA_2$ (EDG-4) also couples with three types of G proteins, $G_{i/o}$, $G_q$, and $G_{12/13}$, to mediate LPA-induced cellular signaling. Expression of $LPA_2$ is observed in the testis, kidney, lung, thymus, spleen, and stomach of adult mice and in the human testis, pancreas, prostate, thymus, spleen, and peripheral blood leukocytes. Expression of $LPA_2$ is upregulated in various cancer cell lines, and several human $LPA_2$ transcriptional variants with mutations in the 3'-untranslated region

have been observed.

$LPA_3$ can mediate pleiotropic LPA-induced signaling that includes PLC activation, $Ca^{2+}$ mobilization, AC inhibition/activation, and MAPK activation. Overexpression of $LPA_3$ in neuroblastoma cells leads to neurite elongation. Expression of $LPA_3$ is observed in adult mouse testis, kidney, lung, small intestine, heart, thymus, and brain. In humans, it is found in the heart, pancreas, prostate, testis, lung, ovary, and brain (frontal cortex, hippocampus, and amygdala).

$LPA_4$ ($p2y_9$/GPR23) is of divergent sequence compared to $LPA_1$, $LPA_2$, and $LPA_3$ with closer similarity to the platelet-activating factor (PAF) receptor. $LPA_4$ mediates LPA induced $Ca^{2+}$ mobilization and cAMP accumulation, and functional coupling to the G protein Gs for AC activation, as well as coupling to other G proteins. The $LPA_4$ gene is expressed in the ovary, pancreas, thymus, kidney and skeletal muscle.

$LPA_5$ (GPR92) is a member of the purinocluster of GPCRs and is structurally most closely related to $LPA_4$. $LPA_5$ is expressed in human heart, placenta, spleen, brain, lung and gut. LPAs also shows very high expression in the CD8+ lymphocyte compartment of the gastrointestinal tract.

$LPA_6$ (p2y5) is a member of the purinocluster of GPCRs and is structurally most closely related to $LPA_4$. $LPA_6$ is an LPA receptor coupled to the GI2/13-Rho signaling pathways and is expressed in the inner root sheaths of human hair follicles.

*Compounds*

In one aspect, the disclosure features compounds of Formula **(I)**:

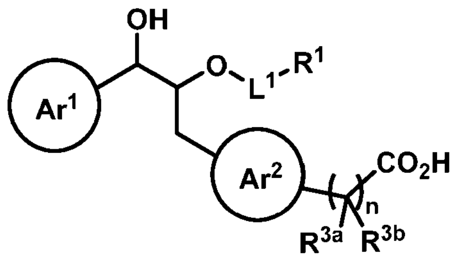

Formula (**I**)

or a pharmaceutically acceptable salt thereof, wherein:

**$L^1$** is selected from the group consisting of:

- a bond; and
- $C_{1-6}$ alkylene optionally substituted with from 1-6 **$R^a$**;

**$R^1$** is selected from the group consisting of: **$R^b$**; $C_{2-6}$ alkenyl optionally substituted with from 1-6 **$R^a$**; and $C_{2-6}$ alkynyl optionally substituted with from 1-6 **$R^a$**;

**$Ar^1$** is selected from the group consisting of:

- $C_{6-10}$ aryl optionally substituted with from 1-4 substituents each independently selected from the group consisting of: **$R^{c1}$** and **$-(L^b)_b-R^b$**; and
- heteroaryl including from 5-10 ring atoms, wherein from 1-4 ring atoms are ring heteroatoms each independently selected from the group consisting of: N, N(H), N(**$R^d$**), O, and S, wherein the heteroaryl is optionally substituted with from 1-4 substituents each independently selected from the group consisting of: **$R^{c1}$** and - **$(L^b)_b-R^b$**;

**$Ar^2$** is selected from the group consisting of:

- $C_{6-10}$ arylene optionally substituted with from 1-4 **$R^{c2}$**; and
- heteroarylene including from 5-10 ring atoms, wherein from 1-4 ring atoms are ring heteroatoms each independently selected from the group consisting of: N, N(H), N(**$R^d$**), O, and S, wherein the heteroarylene is optionally substituted with from 1-4 **$R^{c2}$**;

**n is 0 or 1;**

$R^{3a}$ and $R^{3b}$ are independently H, -halo, $C_{1-6}$ alkyl, or $C_{1-4}$ haloalkyl; or

$R^{3a}$ and $R^{3b}$ taken together with the carbon atom to which each is attached forms a $C_{3-6}$ cycloalkyl;

each occurrence of $R^a$ is independently selected from the group consisting of: -OH; -halo; -N$R^eR^f$; $C_{1-4}$ alkoxy; $C_{1-4}$ haloalkoxy; -C(=O)O($C_{1-4}$ alkyl); -C(=O)($C_{1-4}$ alkyl); -C(=O)OH; -CON**R'R"**; -S(O)$_{1-2}$N**R'R"**; -S(O)$_{1-2}$($C_{1-4}$ alkyl); and cyano;

each occurrence of $R^b$ is independently selected from the group consisting of:

- $C_{3-10}$ cycloalkyl or $C_{3-10}$ cycloalkenyl, each of which is optionally substituted with from 1-4 $R^g$;
- heterocyclyl or heterocycloalkenyl including from 3-10 ring atoms, wherein from 1-3 ring atoms are heteroatoms, each independently selected from the group consisting of N, N(H), N($R^d$), O, and S(O)$_{0-2}$, and wherein the heterocyclyl or heterocycloalkenyl is optionally substituted with from 1-4 $R^g$;
- heteroaryl including from 5-10 ring atoms, wherein from 1-4 ring atoms are heteroatoms, each independently selected from the group consisting of N, N(H), N($R^d$), O, and S(O)$_{0-2}$, and wherein the heteroaryl is optionally substituted with from 1-4 $R^g$; and
- $C_{6-10}$ aryl optionally substituted with from 1-4 $R^g$;

b is 0, 1, 2, or 3;

each occurrence of $L^b$ is selected from the group consisting of: $C_{1-3}$ alkylene; - N(H)-; N($R^d$)-; -O-; -S-; C(=O); and S(O)$_{1-2}$;

each occurrence of $R^{c1}$ and $R^{c2}$ is independently selected from the group consisting of: halo; cyano; $C_{1-10}$ alkyl which is optionally substituted with from 1-6 independently selected $R^a$; $C_{2-6}$ alkenyl; $C_{2-6}$ alkynyl; $C_{1-4}$ alkoxy; $C_{1-4}$ haloalkoxy; - S(O)$_{0-2}$($C_{1-4}$ alkyl); -N$R^eR^f$; -OH; -S(O)$_{1-2}$N**R'R"**; -$NO_2$; -C(=O)($C_{1-10}$ alkyl); - C(=O)O($C_{1-4}$ alkyl); -C(=O)OH; and -C(=O)N**R'R"**;

each occurrence of $R^d$ is independently selected from the group consisting of: $C_{1-6}$ alkyl optionally substituted with from 1-3 independently selected $R^a$; -C(O)($C_{1-4}$ alkyl); -C(O)O($C_{1-4}$ alkyl); -CON**R'R"**; -S(O)$_{1-2}$N**R'R"**; -S(O)$_{1-2}$($C_{1-4}$ alkyl); -OH; and $C_{1-4}$ alkoxy;

each occurrence of $R^e$ and $R^f$ is independently selected from the group consisting of: H; $C_{1-6}$ alkyl; -C(O)($C_{1-4}$ alkyl); -C(O)O($C_{1-4}$ alkyl); -CON**R'R"**; - S(O)$_{1-2}$N**R'R"**; -S(O)$_{1-2}$($C_{1-4}$ alkyl); -OH; and $C_{1-4}$ alkoxy; and

each occurrence of $R^g$ is independently selected from the group consisting of: halo; cyano; $C_{1-6}$ alkyl; $C_{1-6}$ haloalkyl; $C_{1-4}$ alkoxy; $C_{1-4}$ haloalkoxy; -OH; and N**R'R"**; and

each occurrence of **R'** and **R"** is independently selected from the group consisting of: H; -OH; and $C_{1-4}$ alkyl.

In one aspect, the disclosure features compounds of Formula **(Ia):**

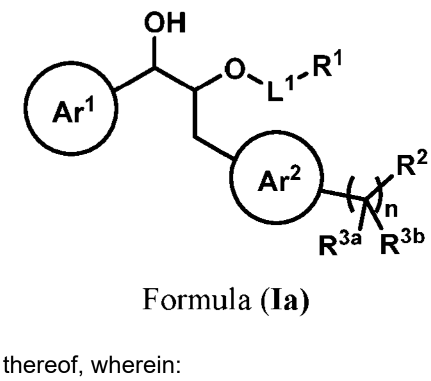

Formula **(Ia)**

or a pharmaceutically acceptable salt thereof, wherein:

$L^1$ is selected from the group consisting of:

- a bond; and
- $C_{1-6}$ alkylene optionally substituted with from 1-6 $\mathbf{R^a}$;

$\mathbf{R^1}$ is selected from the group consisting of: $\mathbf{R^b}$; $C_{1-6}$ alkyl optionally substituted with from 1-6 $\mathbf{R^a}$; $C_{2-6}$ alkenyl optionally substituted with from 1-6 $\mathbf{R^a}$; and $C_{2-6}$ alkynyl optionally substituted with from 1-6 $\mathbf{R^a}$;

$\mathbf{Ar^1}$ is selected from the group consisting of:

- $C_{6-10}$ aryl optionally substituted with from 1-4 substituents each independently selected from the group consisting of: $\mathbf{R^{c1}}$ and -$(\mathbf{L^b})_\mathbf{b}$-$\mathbf{R^b}$, and
- heteroaryl including from 5-10 ring atoms, wherein from 1-4 ring atoms are ring heteroatoms each independently selected from the group consisting of: N, N(H), N($\mathbf{R^d}$), O, and S, wherein the heteroaryl is optionally substituted with from 1-4 substituents each independently selected from the group consisting of: $\mathbf{R^{c1}}$ and - $(\mathbf{L^b})_\mathbf{b}$-$\mathbf{R^b}$;

$\mathbf{Ar^2}$ is selected from the group consisting of:

- $C_{6-10}$ arylene optionally substituted with from 1-4 substituents each independently selected from the group consisting of: $\mathbf{R^{c2}}$ and -$(\mathbf{L^b})_\mathbf{b}$-$\mathbf{R^b}$; and
- heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents each independently selected from the group consisting of: $\mathbf{R^{c2}}$ and -$(\mathbf{L^b})_\mathbf{3}$-$\mathbf{R^b}$;

$\mathbf{R^2}$ is -C(=O)OH or carboxylic acid bioisostere;

n is 0 or 1;

$\mathbf{R^{3a}}$ and $\mathbf{R^{3b}}$ are independently H, -halo, $C_{1-6}$ alkyl, or $C_{1-4}$ haloalkyl; or

$\mathbf{R^{3a}}$ and $\mathbf{R^{3b}}$ taken together with the carbon atom to which each is attached forms a $C_{3-6}$ cycloalkyl;

each occurrence of $\mathbf{R^a}$ is independently selected from the group consisting of: -OH; -halo; -N$\mathbf{R^eR^f}$; $C_{1-4}$ alkoxy; $C_{1-4}$ haloalkoxy; -C(=O)O($C_{1-4}$ alkyl); -C(=O)($C_{1-4}$ alkyl); -C(=O)OH; -CON**R'R"**; -S(O)$_{1-2}$N**R'R"**; -S(O)$_{1-2}$($C_{1-4}$ alkyl); and cyano;

each occurrence of $\mathbf{R^b}$ is independently selected from the group consisting of:

- $C_{3-10}$ cycloalkyl or $C_{3-10}$ cycloalkenyl, each of which is optionally substituted with from 1-4 $\mathbf{R^g}$;
- heterocyclyl or heterocycloalkenyl including from 3-10 ring atoms, wherein from 1-3 ring atoms are heteroatoms, each independently selected from the group consisting of N, N(H), N($\mathbf{R^d}$), O, and S(O)$_{0-2}$, and wherein the heterocyclyl or heterocycloalkenyl is optionally substituted with from 1-4 $\mathbf{R^g}$;
- heteroaryl including from 5-10 ring atoms, wherein from 1-4 ring atoms are heteroatoms, each independently selected from the group consisting of N, N(H), N($\mathbf{R^d}$), O, and S(O)$_{0-2}$, and wherein the heteroaryl is optionally substituted with from 1-4 $\mathbf{R^g}$; and
- $C_{6-10}$ aryl optionally substituted with from 1-4 $\mathbf{R^g}$**;**

b is 0, 1, 2, or 3;

each occurrence of $\mathbf{L^b}$ is selected from the group consisting of: $C_{1-3}$ alkylene; - N(H)-; N($\mathbf{R^d}$)-; -O-; -S-; C(=O); and S(O)$_{1-2}$;

each occurrence of $\mathbf{R^{c1}}$ and $\mathbf{R^{c2}}$ is independently selected from the group consisting of: halo; cyano; $C_{1-10}$ alkyl which is optionally substituted with from 1-6 independently selected $\mathbf{R^a}$; $C_{2-6}$ alkenyl; $C_{2-6}$ alkynyl; $C_{1-4}$ alkoxy; $C_{1-4}$ haloalkoxy; - S(O)$_{0-2}$($C_{1-4}$ alkyl); -N$\mathbf{R^eR^f}$; -OH; -S(O)$_{1-2}$N**R'R"**; -$NO_2$; -C(=O)($C_{1-10}$ alkyl); - C(=O)O($C_{1-4}$ alkyl); -C(=O)OH; and -C(=O)N**R'R"**;

each occurrence of $\mathbf{R^d}$ is independently selected from the group consisting of: $C_{1-6}$ alkyl optionally substituted with from 1-3 independently selected $\mathbf{R^a}$; -C(O)($C_{1-4}$ alkyl); -C(O)O($C_{1-4}$ alkyl); -CON**R'R"**; -S(O)$_{1-2}$N**R'R"**; -S(O)$_{1-2}$($C_{1-4}$

alkyl); -OH; and $C_{1-4}$ alkoxy;

each occurrence of $\mathbf{R^e}$ and $\mathbf{R^f}$ is independently selected from the group consisting of: H; $C_{1-6}$ alkyl; -C(O)($C_{1-4}$ alkyl); -C(O)O($C_{1-4}$ alkyl); -CON**R'R"; -** S(O)$_{1-2}$N**R'R"**; -S(O)$_{1-2}$($C_{1-4}$ alkyl); -OH; and $C_{1-4}$ alkoxy; and

each occurrence of $\mathbf{R^g}$ is independently selected from the group consisting of: halo; cyano; $C_{1-6}$ alkyl; $C_{1-6}$ haloalkyl; $C_{1-4}$ alkoxy; $C_{1-4}$ haloalkoxy; -OH; and N**R'R"**; and

each occurrence of **R'** and **R"** is independently selected from the group consisting of: H; -OH; and $C_{1-4}$ alkyl.

In some embodiments, the compound of Formula **(I)** is a compound of Formula **(I-A):**

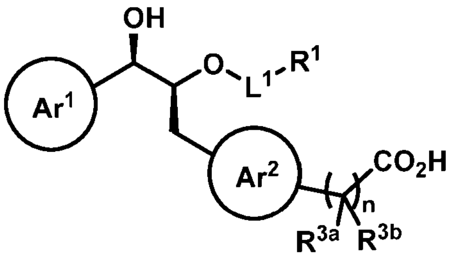

Formula **(I-A)**

or a pharmaceutically acceptable salt thereof.

In some embodiments, the compound of formula **(I)** is a compound of Formula **(I-Aa):**

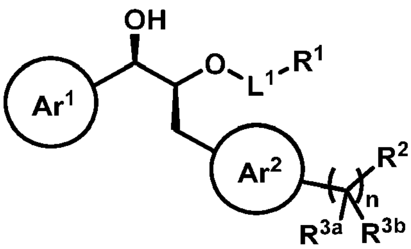

Formula **(I-Aa)**

or a pharmaceutically acceptable salt thereof.

***Variable Ar¹***

In some embodiments of Formula **(I), (Ia), (I-A) or (I-Aa),** $\mathbf{Ar^1}$ is $C_{6-10}$ aryl optionally substituted with from 1-4 substituents, each of which is independently selected from the group consisting of: $\mathbf{R^{c1}}$ and -($\mathbf{L^b}$)$_b$-$\mathbf{R^b}$.

In certain of these embodiments, $\mathbf{Ar^1}$ is phenyl optionally substituted with from 1-4 substituents, each of which is independently selected from the group consisting of: $\mathbf{R^{c1}}$ and -($\mathbf{L^b}$)$_b$-$\mathbf{R^b}$. In certain embodiments, $\mathbf{Ar^1}$ is phenyl substituted with from 1-4 substituents, each of which is independently selected from the group consisting of: $\mathbf{R^{c1}}$ and -($\mathbf{L^b}$)$_b$-$\mathbf{R^b}$. In certain embodiments, $\mathbf{Ar^1}$ is phenyl substituted with from 1-4 independently selected $\mathbf{R^{c1}}$.

In certain of the foregoing embodiments, each occurrence of $\mathbf{R^{c1}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; and cyano.

In certain of the foregoing embodiments, each occurrence of $\mathbf{R^{c1}}$ is independently selected from the group consisting of: halo; $C_{1-6}$ alkyl; $C_{1-6}$ alkyl substituted with from 1-6 independently selected halo; -C(=O)($C_{1-10}$ alkyl); $C_{1-4}$ alkoxy; $C_{1-4}$ haloalkoxy; and cyano.

In certain of the foregoing embodiments, each occurrence of -($\mathbf{L^b}$)$_b$-$\mathbf{R^b}$ is an independently selected $C_{3-6}$ cycloalkyl optionally substituted with from 1-2 $\mathbf{R^g}$.

In certain embodiments, $\mathbf{Ar^1}$ is

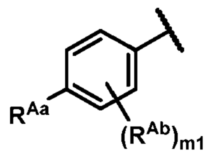,

wherein **m1** is 0, 1, 2, or 3; and $\mathbf{R^{Aa}}$ and $\mathbf{R^{Ab}}$ are each independently selected from the group consisting of: $\mathbf{R^{c1}}$ and $-(\mathbf{L^b})_b-\mathbf{R^b}$.

In certain of these embodiments, each occurrence of $\mathbf{R^{Aa}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

In certain of these embodiments, each occurrence of $\mathbf{R^{Aa}}$ is selected from the group consisting of: halo; $C_{1-6}$ alkyl; $C_{1-6}$ alkyl substituted with from 1-6 independently selected halo; $-C(=O)(C_{1-10}$ alkyl); $C_{1-4}$ alkoxy; $C_{1-4}$ haloalkoxy; cyano; and $C_{3-6}$ cycloalkyl optionally substituted with from 1-2 $\mathbf{R^g}$.

In certain embodiments, $\mathbf{R^{Aa}}$ is $C_{1-6}$ alkyl, e.g., $C_{1-3}$ alkyl. As a non-limiting example of the foregoing embodiments, $\mathbf{R^{Aa}}$ can be methyl.

In certain embodiments, $\mathbf{R^{Aa}}$ is $C_{1-6}$ alkyl substituted with from 1-6 independently selected halo. In certain of these embodiments, $\mathbf{R^{Aa}}$ is $C_{1-3}$ alkyl substituted with 1-6 F. As a non-limiting example of the foregoing embodiments, $\mathbf{R^{Aa}}$ can be $CF_3$ or $CHF_2$.

In certain embodiments, $\mathbf{R^{Aa}}$ is halo, e.g., -Cl.

In certain embodiments, $\mathbf{R^{Aa}}$ is $C_{3-6}$ cycloalkyl. As a non-limiting example of the foregoing embodiments, $\mathbf{R^{Aa}}$ can be cyclopropyl.

In certain embodiments, **m1** is 2. In certain embodiments, **m1** is 1 or 3, e.g., 1. In certain embodiments, **m1** is 0.

In certain embodiments, when **m1** is 1 or 2, one or both occurrences of $\mathbf{R^{Ab}}$ are attached to the ring atom or atoms that are *ortho* to the ring atom attached to $\mathbf{R^{Aa}}$.

In certain embodiments, each occurrence of $\mathbf{R^{Ab}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

In certain embodiments, each $\mathbf{R^{Ab}}$ is independently $C_{1-4}$ alkoxy or $C_{1-4}$ haloalkoxy. For example, each $\mathbf{R^{Ab}}$ can be $C_{1-4}$ alkoxy, e.g., methoxy.

As a non-limiting example, $\mathbf{Ar^1}$ can be

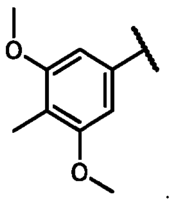.

As further non-limiting examples, $\mathbf{Ar^1}$ can be selected from the group consisting of:

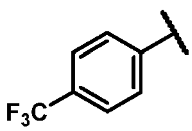; 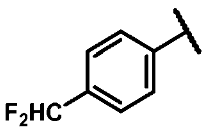; 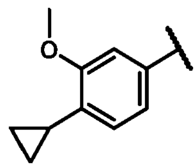; and 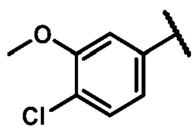.

As further non-limiting examples, $\mathbf{Ar^1}$ can be selected from the group consisting of: :

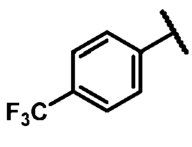; 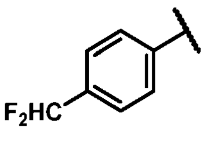; 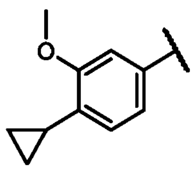; 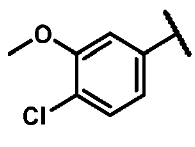; 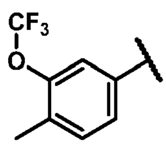;

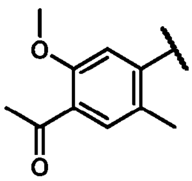 ; 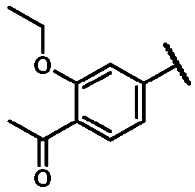 ; 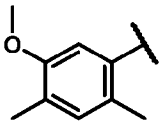 ; 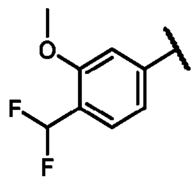 ; 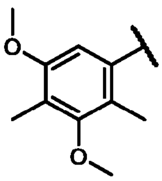 ;

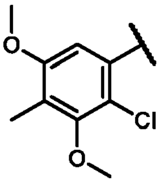 ; 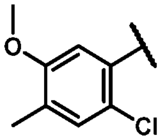 ; 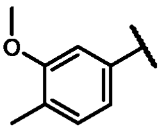 ; 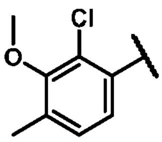 ; 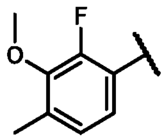 ; 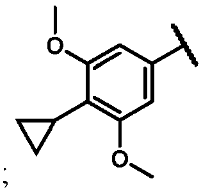 ;

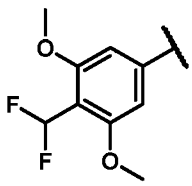 ; 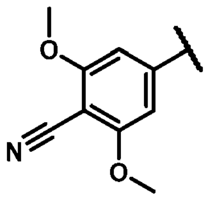 ; 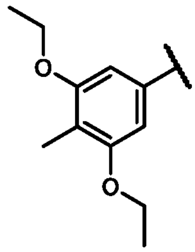 ; 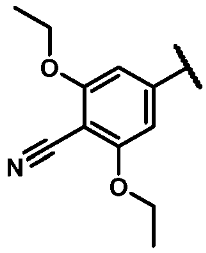 ; 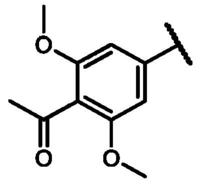 ;

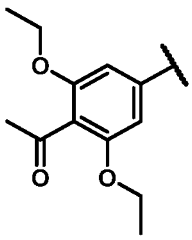 ; 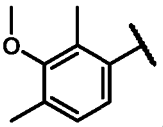 ; and 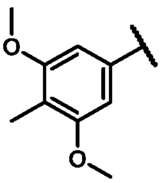 .

In some embodiments, **Ar$^1$** is heteroaryl including from 5-10 ring atoms, wherein from 1-4 ring atoms are ring heteroatoms each independently selected from the group consisting of: N, N(H), N(**R$^d$**), O, and S, wherein the heteroaryl is optionally substituted with from 1-4 substituents, each of which is independently selected from the group consisting of: **R$^{c1}$** and -(**L$^b$**)$_b$-**R$^b$**.

In certain embodiments, **Ar$^1$** is heteroaryl including from 5-6 ring atoms, wherein from 1-4 ring atoms are ring heteroatoms each independently selected from the group consisting of: N, N(H), N(**R$^d$**), O, and S, wherein the heteroaryl is optionally substituted with from 1-4 substituents, each of which is independently selected from the group consisting of: **R$^{c1}$** and -(**L$^b$**)$_b$-**R$^b$**.

In certain embodiments, **Ar$^1$** is heteroaryl including 6 ring atoms, wherein from 1-2 ring atoms are ring nitrogen atoms, wherein the heteroaryl is optionally substituted with from 1-4 substituents, each of which is independently selected from the group consisting of: **R$^{c1}$** and -(**L$^b$**)$_b$-**R$^b$**.

In certain embodiments, **Ar$^1$** is pyridyl optionally substituted with from 1-3 substituents selected from the group consisting of: **R$^{c1}$** and -(**L$^b$**)$_b$-**R$^b$**. As non-limiting examples of the foregoing embodiments, **Ar$^1$** can be 3-pyridyl optionally substituted with from 1-3 substituents selected from the group consisting of: **R$^{c1}$** and -(**L$^b$**)$_b$-**R$^b$**.

In certain embodiments (when **Ar$^1$** is heteroaryl as defined *supra*), each occurrence of **R$^{c1}$** is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; and cyano. In certain embodiments, each occurrence of -(**L$^b$**)$_b$-**R$^b$** is an independently selected $C_{3-6}$ cycloalkyl optionally substituted with from 1-2 **R$^g$**.

In certain embodiments, **Ar$^1$** is 3-pyridyl substituted with 1-3 independently selected $C_{1-6}$ alkyl.

In certain embodiments, **Ar$^1$** is 3-pyridyl substituted with 1-3 independently selected $C_{1-6}$ alkyl, or **Ar$^1$** is 4-pyridyl substituted with 1-3 independently selected $C_{1-6}$ alkoxy.

As a non-limiting example, **Ar$^1$** can be

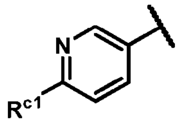

,

e.g.,

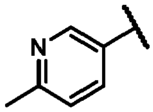

.

As further non-limiting examples, **$Ar^1$** is

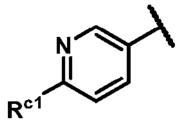

,

such as

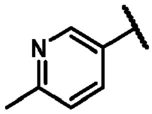

,

or **$Ar^1$** is

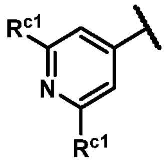

such as

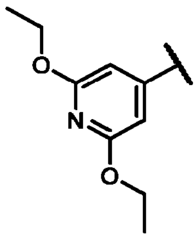

.

***Variable $L^1$***

In some embodiments of Formula **(I), (Ia), (I-A) or (I-Aa), $L^1$** is a bond.

In some embodiments of Formula **(I), (Ia), (I-A) or (I-Aa), $L^1$** is $C_{1-6}$ alkylene optionally substituted with from 1-6 **$R^a$**.

In certain or these embodiments, **$L^1$** is $C_{1-3}$ alkylene optionally substituted with from 1-6 **$R^a$**. In certain embodiments, **$L^1$** is unsubstituted $C_{1-3}$ alkylene. As a non-limiting example of the foregoing embodiments, **$L^1$** can be $CH_2CH_2$. As another non-limiting example, **$L^1$** can be $CH_2$.

***Variable $R^1$***

In some embodiments of Formula **(I), (Ia), (I-A) or (I-Aa), $R^1$** is **$R^b$**.

In certain of these embodiments, $\mathbf{R^1}$ is selected from the group consisting of:

- $C_{3-10}$ cycloalkyl or $C_{3-10}$ cycloalkenyl, each of which is optionally substituted with from 1-4 $\mathbf{R^g}$; and
- $C_{6-10}$ aryl optionally substituted with from 1-4 $\mathbf{R^g}$.

In certain of these embodiments, $\mathbf{R^1}$ is $C_{6-10}$ aryl optionally substituted with from 1-4 $\mathbf{R^g}$. In certain embodiments, $\mathbf{R^1}$ is phenyl optionally substituted with from 1-4 $\mathbf{R^g}$, such as phenyl which is optionally substituted with from 1-2 $\mathbf{R^g}$. As a non-limiting example of the foregoing embodiments, $\mathbf{R^1}$ can be unsubstituted phenyl.

In certain embodiments, $\mathbf{R^1}$ is $C_{8-10}$ bicyclic aryl optionally substituted with from 1-4 $\mathbf{R^g}$. In certain of these embodiments, $\mathbf{R^1}$ is $C_{9-10}$ bicyclic aryl optionally substituted with from 1-2 $\mathbf{R^g}$. In certain embodiments, $\mathbf{R^1}$ is indanyl optionally substituted with from 1-2 $\mathbf{R^g}$. As a non-limiting example of the foregoing embodiments, $\mathbf{R^1}$ can be

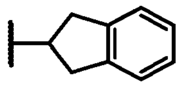,

which is optionally substituted with from 1-2 $\mathbf{R^g}$. For example, $\mathbf{R^1}$ can be

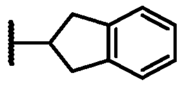.

In certain embodiments of Formula **(I), (Ia), (I-A) or (I-Aa)**, $\mathbf{R^1}$ is $C_{3-10}$ cycloalkyl or $C_{3-10}$ cycloalkenyl, each of which is optionally substituted with from 1-4 $\mathbf{R^g}$. In certain of these embodiments, $\mathbf{R^1}$ is $C_{3-10}$ cycloalkyl, which is optionally substituted with from 1-4 $\mathbf{R^g}$. In certain embodiments, $\mathbf{R^1}$ is $C_{3-6}$ cycloalkyl which is optionally substituted with from 1-2 $\mathbf{R^g}$. In certain embodiments, $\mathbf{R^1}$ is cyclobutyl or cyclopentyl, each of which is optionally substituted with from 1-2 $\mathbf{R^g}$, such as cyclopentyl optionally substituted with from 1-2 $\mathbf{R^g}$. As non-limiting examples of the foregoing embodiments, $\mathbf{R^1}$ can be unsubstituted cyclobutyl or cyclopentyl, such as unsubstituted cyclopentyl.

In some embodiments of Formula **(I), (Ia), (I-A) or (I-Aa),** $\mathbf{R^1}$ is $C_{2-6}$ alkynyl optionally substituted with from 1-6 $\mathbf{R^a}$. In certain of these embodiments, $\mathbf{R^1}$ is $C_{2-4}$ alkynyl optionally substituted with from 1-3 $\mathbf{R^a}$. As a non-limiting example of the foregoing embodiments, $\mathbf{R^1}$ can be

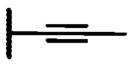.

In some embodiments of Formula **(I), (Ia), (I-A) or (I-Aa),** $\mathbf{R^1}$ is $C_{1-6}$ alkyl optionally substituted with from 1-6 $\mathbf{R^a}$.

In certain of these embodiments, $\mathbf{R^1}$ is $C_{1-6}$ alkyl.

In certain of these embodiments, $\mathbf{R^1}$ is $C_{2-4}$ alkyl.

In certain of these embodiments, $\mathbf{R^1}$ is $C_3$ alkyl, such as *n*-propyl and *i*-propyl.

As a non-limiting example, $\mathbf{R^1}$ can be *i*-propyl.

In certain of these embodiments, $\mathbf{R^1}$ is $C_{3-5}$ alkyl.

In certain of these embodiments, $\mathbf{R^1}$ is $C_4$ alkyl, such as *n*-butyl, *i*-butyl, *sec*-butyl, and tert-butyl.

As another non-limiting example, $\mathbf{R^1}$ can be *i*-butyl.

In certain of these embodiments, $\mathbf{L^1}$ is a bond.

### *Variable Ar²*

In some embodiments of Formula **(I), (Ia), (I-A) or (I-Aa),** $\mathbf{Ar^2}$ is heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$.

In some embodiments of Formula **(I), (Ia), (I-A) or (I-Aa),** $\mathbf{Ar^2}$ is heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 - $\mathbf{(L^b)_b}$-$\mathbf{R_b}$.

In certain of these embodiments, $\mathbf{Ar^2}$ is heteroarylene including from 5-6 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$.

In certain of these embodiments, $\mathbf{Ar^2}$ is heteroarylene including from 5-6 ring atoms, wherein from 1-4 ring atoms are ring heteroatoms each independently selected from the group consisting of: N, N(H), N($\mathbf{R^d}$), O, and S, wherein the

heteroarylene is optionally substituted with from 1-4 substituents, each of which is independently selected from the group consisting of: $\mathbf{R^{c2}}$ and -$\mathbf{(L^b)_b}$-$\mathbf{R^b}$.

In certain embodiments, $\mathbf{Ar^2}$ is heteroarylene including from 5 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$

In certain embodiments, $\mathbf{Ar^2}$ is heteroarylene including from 5 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents, each of which is independently selected from the group consisting of: $\mathbf{R^{c2}}$ and -$\mathbf{(L^b)_b}$-$\mathbf{R^b}$.

In certain embodiments, $\mathbf{Ar^2}$ can be selected from the group consisting of pyrrolylene, pyrazolylene, thiazolylene and 1,3,4-oxadiazolylene, each of which is optionally substituted with from 1-4 substituents, each of which is independently selected from the group consisting of: $\mathbf{R^{c2}}$ and -$\mathbf{(L^b)_b}$-$\mathbf{R^b}$ .

In certain embodiments, $\mathbf{Ar^2}$ is selected from the group consisting of pyrrolylene, pyrazolylene, and thiazolylene, each of which is optionally substituted with $\mathbf{R^{c2}}$.

In certain embodiments, $\mathbf{Ar^2}$ is

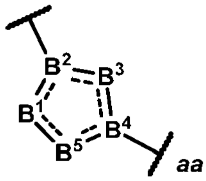
,

wherein:

each 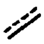 is independently a single bond or a double bond, provided that the ring including $\mathbf{B^1}$, $\mathbf{B^2}$, $\mathbf{B^3}$, $\mathbf{B^4}$, and $\mathbf{B^5}$ is heteroaryl;

***aa*** is the point of attachment to -(C$\mathbf{R^{3a}R^{3b}}$)$_n$ C(O)OH;

$\mathbf{B^2}$ and $\mathbf{B^4}$ are C or N; and

$\mathbf{B^1}$, $\mathbf{B^3}$, and $\mathbf{B^5}$ are independently O, S, N, N(H), N($\mathbf{R^d}$), CH, or $\mathbf{CR^{c2}}$, provided that:

from 1-4 of $\mathbf{B^1}$, $\mathbf{B^2}$, $\mathbf{B^3}$, $\mathbf{B^4}$, and $\mathbf{B^5}$ are independently selected heteroatoms.

In certain embodiments of Formula **(I), (Ia), (I-A) or (I-Aa)** (when $\mathbf{Ar^2}$ is

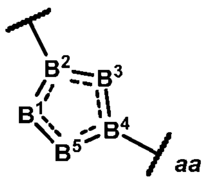

), $\mathbf{B^2}$ is N. In certain embodiments, $\mathbf{B^4}$ is C. In certain embodiments, $\mathbf{B^1}$, $\mathbf{B^3}$, and $\mathbf{B^5}$ are independently CH or C$\mathbf{R^{c2}}$. As non-limiting examples of the foregoing embodiments, $\mathbf{B^5}$ can be C$\mathbf{R^{c2}}$; and $\mathbf{B^1}$ and $\mathbf{B^3}$ can be CH.

In certain embodiments (when $\mathbf{Ar^2}$ is

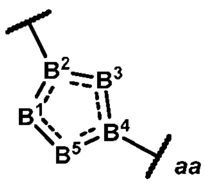

), $\mathbf{B^2}$ is N; $\mathbf{B^4}$ is C; and $\mathbf{B^1}$, $\mathbf{B^3}$, and $\mathbf{B^5}$ are independently CH or C$\mathbf{R^{c2}}$.

In certain embodiments, $\mathbf{Ar^2}$ is

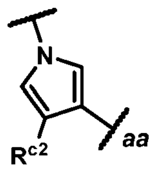

,

wherein ***aa*** is the point of attachment to -(C$\mathbf{R^{3a}R^{3b}}$)$_n$ C(O)OH. In certain of these embodiments, $\mathbf{R^{c2}}$ is C(O)OC$_{1-4}$ alkyl, such as C(O)OMe.

In certain embodiments of Formula **(I), (Ia), (I-A) or (I-Aa)** (when $\mathbf{Ar^2}$ is

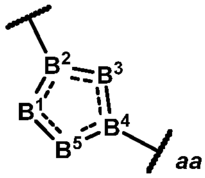

), $\mathbf{B^2}$ is C. In certain embodiments, $\mathbf{B^4}$ is C. In certain embodiments, $\mathbf{B^5}$ is CH or C$\mathbf{R^{c2}}$; one of $\mathbf{B^1}$ and $\mathbf{B^3}$ is N; and the other of $\mathbf{B^1}$ and $\mathbf{B^3}$ is NH, N($\mathbf{R^d}$), O, or S, such as S.

In certain of these embodiments, $\mathbf{Ar^2}$ is

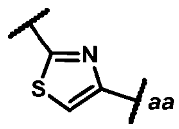

, wherein ***aa*** is the point of attachment to -(C$\mathbf{R^{3a}R^{3b}}$)$_n$-C(O)OH.

In certain embodiments of Formula **(I), (Ia), (I-A) or (I-Aa)** (when $\mathbf{Ar^2}$ is

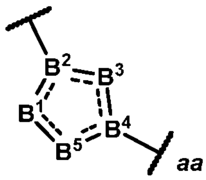

), $\mathbf{B^4}$ is N. In certain embodiments, $\mathbf{B^2}$ is C. In certain of embodiments, $\mathbf{B^3}$ is N; and $\mathbf{B^1}$ and $\mathbf{B^5}$ are independently CH or C$\mathbf{R^{c2}}$.

In certain embodiments, $\mathbf{Ar^2}$ is

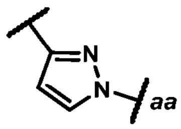

, wherein ***aa*** is the point of attachment to -(C$\mathbf{R^{3a}R^{3b}}$)$_n$ C(O)OH.

In certain embodiments, $\mathbf{Ar^2}$ is

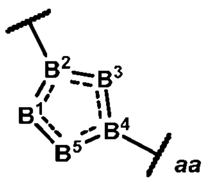

, wherein:

each 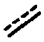 is independently a single bond or a double bond, provided that the ring including $\mathbf{B^1}$, $\mathbf{B^2}$, $\mathbf{B^3}$, $B^4$, and $\mathbf{B^5}$ is heteroaryl;

***aa*** is the point of attachment to -(C$\mathbf{R^{3a}R^{3b}}$)$_n$-$\mathbf{R^2}$;

$\mathbf{B^2}$ and $\mathbf{B^4}$ are C or N; and

$\mathbf{B^1}$, $\mathbf{B^3}$, and $\mathbf{B^5}$ are independently O, S, N, N(H), N($\mathbf{R^d}$), CH, C$\mathbf{R^{c2}}$, or C-$\mathbf{(L^b)_b}$-$\mathbf{R^b}$, provided that:

from 1-4 of $\mathbf{B^1}$, $\mathbf{B^2}$, $\mathbf{B^3}$, $\mathbf{B^4}$, and $\mathbf{B^5}$ are independently selected heteroatoms.

In certain embodiments of Formula **(I), (Ia), (I-A) or (I-Aa)** (when $\mathbf{Ar^2}$ is

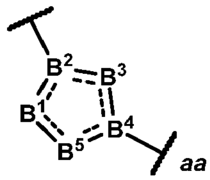

), $\mathbf{B^2}$ is N. In certain embodiments, $\mathbf{B^4}$ is C. In certain embodiments, $\mathbf{B^1}$, $\mathbf{B^3}$, and $\mathbf{B^5}$ are independently CH, C$\mathbf{R^{c2}}$, or C-$\mathbf{(L^b)_b}$-$\mathbf{R^b}$. As non-limiting examples of the foregoing embodiments, $\mathbf{B^5}$ is $\mathbf{CR^{c2}}$; and $\mathbf{B^1}$ and $\mathbf{B^3}$ are CH.

In certain embodiments of Formula **(I), (Ia), (I-A) or (I-Aa)** (when $\mathbf{Ar^2}$ is

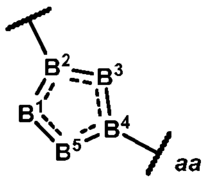

), $\mathbf{B^2}$ is N; $\mathbf{B^4}$ is C; and $\mathbf{B^1}$**,** $\mathbf{B^3}$, and $\mathbf{B^5}$ are independently CH, C$\mathbf{R^{c2}}$, or C-$\mathbf{(L^b)_b}$-$\mathbf{R^b}$**.**

In certain embodiments, $\mathbf{Ar^2}$ is

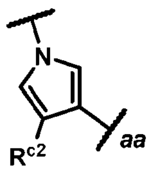

,

wherein ***aa*** is the point of attachment to -(C$\mathbf{R^{3a}R^{3b}}$)$_n$-$\mathbf{R^2}$. In certain the embodiments, $\mathbf{R^{c2}}$ is C(O)OC$_{1-4}$ alkyl, such as C(O)OMe.

In certain embodiments of Formula **(I), (Ia), (I-A) or (I-Aa)** (when $\mathbf{Ar^2}$ is

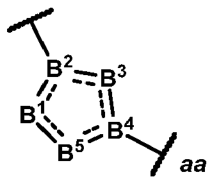

), $\mathbf{B^2}$ is C. In certain embodiments, $\mathbf{B^4}$ is C. In certain embodiments, $\mathbf{B^5}$ is CH, C$\mathbf{R^{c2}}$, or C-$\mathbf{(L^b)_b}$-$\mathbf{R^b}$; and one of $\mathbf{B^1}$ and $\mathbf{B^3}$ is N; and the other of $\mathbf{B^1}$ and $\mathbf{B^3}$ is NH, N($\mathbf{R^d}$), O, or S, such as S.

In certain embodimengts, $\mathbf{Ar^2}$ is selected from the list consisting of

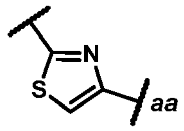 , 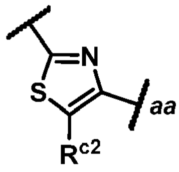 , and 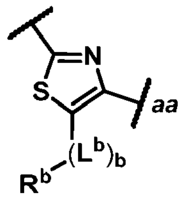 ,

wherein ***aa*** is the point of attachment to -(C$\mathbf{R^{3a}R^{3b}}$)$_n$-$\mathbf{R^2}$. In certain examples, $\mathbf{R^{C2}}$ is C(=O)$C_{1-4}$ alkyl, such as C(=O)Et, or C(=O)Me. In certain examples, $\mathbf{R^{C2}}$ is $C_{1-6}$ alkyl, such as methyl, ethyl, propyl, such as *n*-propyl. In certain examples, -($\mathbf{L^b}$)$_\mathbf{b}$-$\mathbf{R^b}$ is $C_{3-10}$ cycloalkyl, such as cycloproyl.

In cerntain embodiments, $\mathbf{Ar^2}$ is

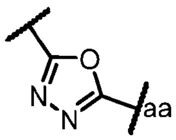 ,

wherein ***aa*** is the point of attachment to -(C$\mathbf{R^{3a}R^{3b}}$)$_n$-$\mathbf{R^2}$.

In certain embodiments of Formula **(I), (Ia), (I-A) or (I-Aa)** (when $\mathbf{Ar^2}$ is

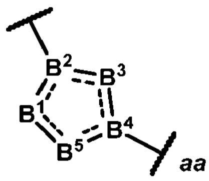

), $\mathbf{B^2}$ is C; and $\mathbf{B^4}$ is N. In certain embodiments, $\mathbf{B^3}$ is N; and $\mathbf{B^1}$ and $\mathbf{B^5}$ are independently CH, C$\mathbf{R^{c2}}$, or C-**(**$\mathbf{L^b}$**)**$_\mathbf{b}$-$\mathbf{R^b}$.

As certain embodiments, $\mathbf{Ar^2}$ can be

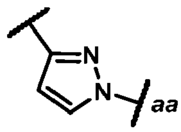 ,

wherein ***aa*** is the point of attachment to -(C$\mathbf{R^{3a}R^{3b}}$)$_n$-$\mathbf{R^2}$.

In certain embodiments of Formula **(I), (Ia), (I-A) or (I-Aa),** $\mathbf{Ar^2}$ is heteroarylene including 6 ring atoms, wherein from 1-2 ring atoms are ring nitrogen atoms, wherein the heteroarylene is optionally substituted with from 1-4 $\mathbf{R^{c2}}$.

In certain embodiments of Formula **(I), (Ia), (I-A) or (I-Aa),** $\mathbf{Ar^2}$ is heteroarylene including 6 ring atoms, wherein from 1-2 ring atoms are ring nitrogen atoms, wherein the heteroarylene is optionally substituted with from 1-4 substituents, each of which is independently selected from the group consisting of: $\mathbf{R^{c2}}$ and -($\mathbf{L^b}$)$_\mathbf{b}$-$\mathbf{R^b}$.

In certain of these embodiments, $\mathbf{Ar^2}$ is pyridylene which is optionally substituted with from 1-2 $\mathbf{R^{c2}}$.

In certain of these embodiments, $\mathbf{Ar^2}$ is pyridylene which is optionally substituted with from 1-4 substituents, each of which is independently selected from the group consisting of: $\mathbf{R^{c2}}$ and -($\mathbf{L^b}$)$_\mathbf{b}$-$\mathbf{R^b}$.

In certain of the foregoing embodiments, $\mathbf{Ar^2}$ is

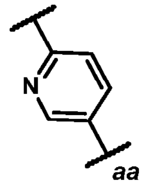 or 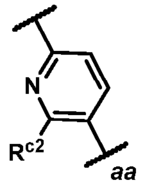 ,

wherein ***aa*** is the point of attachment to -(C$\mathbf{R^{3a}R^{3b}}$)$_n$ C(O)OH. In certain of these embodiments, $\mathbf{R^{c2}}$ is $C_{1-4}$ alkoxy or $C_{1-4}$ haloalkoxy, such as -OMe.

In certain of the foregoing embodiments, $\mathbf{Ar^2}$ is

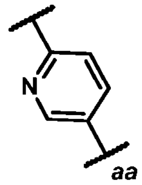 or 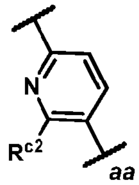 ,

wherein ***aa*** is the point of attachment to -(C$\mathbf{R^{3a}R^{3b}}$)$_n$-$\mathbf{R^2}$. In certain of these embodiments, $\mathbf{R^{c2}}$ is $C_{1-4}$ alkoxy or $C_{1-4}$ haloalkoxy, such as -OMe.

In certain embodiments of Formula **(I)** or **(I-A), Ar$^2$** is bicyclic heteroarylene including from 9-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$.

In certain embodiments of Formula **(I)** or **(I-A), Ar$^2$** is bicyclic heteroarylene including from 9-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents, each of which is independently selected from the group consisting of: $\mathbf{R^{c2}}$ and -($\mathbf{L^b}$)$_b$-$\mathbf{R^b}$.

In certain embodiments of Formula **(I)** or **(I-A), Ar$^2$** is selected from the group consisting of benzimidazolylene, indazolylene, benzothiazolylene, and imidazo[1,2-a]pyridylene (e.g. benzothiazolylene), each of which is optionally substituted with from 1-4 substituents, each of which is independently selected from the group consisting of: $\mathbf{R^{c2}}$ and -($\mathbf{L^b}$)$_b$-$\mathbf{R^b}$.

In certain of these embodiments, **Ar$^2$** is benzimidazolylene or indazolylene, each of which is optionally substituted with from 1-4 $\mathbf{R^{c2}}$.

In certain embodiments of Formula **(I), (Ia), (I-A) or (I-Aa), Ar$^2$** is:

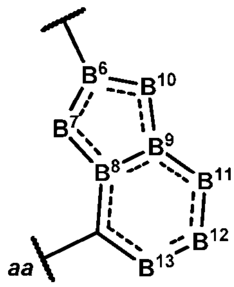

wherein:

each 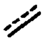 is independently a single bond or a double bond, provided that the 5-membered ring including $\mathbf{B^6}$, $\mathbf{B^7}$, $\mathbf{B^8}$, $\mathbf{B^9}$, and $\mathbf{B^{10}}$ is heteroaryl, and the 6-membered ring including $\mathbf{B^8}$, $\mathbf{B^9}$, $\mathbf{B^{11}}$, $\mathbf{B^{12}}$, and $\mathbf{B^{13}}$ is aryl or heteroaryl;

***aa*** is the point of attachment to -(C$\mathbf{R^{3a}R^{3b}}$)$_n$ C(O)OH;

$\mathbf{B^6}$, $\mathbf{B^8}$, and $\mathbf{B^9}$ are independently C or N;

$\mathbf{B^7}$ and $\mathbf{B^{10}}$ are independently selected from the group consisting of: O, S, N, N(H), N($\mathbf{R^d}$), CH, and C$\mathbf{R^{c2}}$;

$\mathbf{B^{11}}$, $\mathbf{B^{12}}$, and $\mathbf{B^{13}}$ are independently N, CH, or C$\mathbf{R^{c2}}$, provided that:

from 1-4 of $\mathbf{B^6}$, $\mathbf{B^7}$, $\mathbf{B^8}$, $\mathbf{B^9}$, $\mathbf{B^{10}}$, $\mathbf{B^{11}}$, $\mathbf{B^{12}}$, and $\mathbf{B^{13}}$ is an independently selected heteroatom; and no more than 3 of $\mathbf{B^7}$, $\mathbf{B^{10}}$, $\mathbf{B^{11}}$, $\mathbf{B^{12}}$, and $\mathbf{B^{13}}$ are C$\mathbf{R^{c2}}$.

In certain of these embodiments, $\mathbf{B^8}$ and $\mathbf{B^9}$ are C.

In certain embodiments, $\mathbf{B^{11}}$, $\mathbf{B^{12}}$, and $\mathbf{B^{13}}$ are independently CH or C$\mathbf{R^{c2}}$. As a non-limiting example of the foregoing embodiments, $\mathbf{B^{11}}$, $\mathbf{B^{12}}$, and $\mathbf{B^{13}}$ can each be CH.

In certain embodiments, $\mathbf{B^6}$ is N.

In certain embodiments, $\mathbf{B^7}$ is N.

In certain embodiments, $\mathbf{B^{10}}$ is CH or C$\mathbf{R^{c2}}$. As a non-limiting example of the foregoing embodiments, $\mathbf{B^{10}}$ can be CH.

As a non-limiting example of the foregoing embodiments, **Ar$^2$** can be

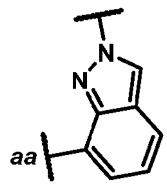

which is optionally substituted with from 1-2 $R^{c2}$, wherein ***aa*** is the point of attachment to $-(CR^{3a}R^{3b})_n$-C(O)OH.

In certain embodiments of Formula **(I), (Ia), (I-A) or (I-Aa), Ar²** is:

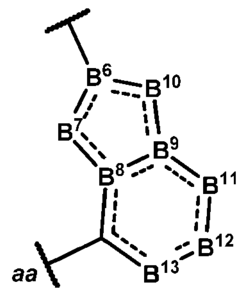

wherein:

each 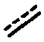 is independently a single bond or a double bond, provided that the 5-membered ring including $B^6$, $B^7$, $B^8$, $B^9$, and $B^{10}$ is heteroaryl, and the 6-membered ring including $B^8$, $B^9$, $B^{11}$, $B^{12}$, and $B^{13}$ is aryl or heteroaryl;
***aa*** is the point of attachment to $-(CR^{3a}R^{3b})_n-R^2$;
$B^6$, $B^8$, and $B^9$ are independently C or N;
$B^7$ and $B^{10}$ are independently selected from the group consisting of: O, S, N, N(H), N($R^d$), CH, $CR^{c2}$ and C-$(L^b)_b-R^b$;
$B^{11}$, $B^{12}$, and $B^{13}$ are independently N, CH, $CR^{c2}$, or C-$(L^b)_b-R^b$, provided that:
from 1-4 of $B^6$, $B^7$, $B^8$, $B^9$, $B^{10}$, $B^{11}$, $B^{12}$, and $B^{13}$ is an independently selected heteroatom; and no more than 3 of $B^7$, $B^{10}$, $B^{11}$, $B^{12}$, and $B^{13}$ are $CR^{c2}$, or C-$(L^b)_b-R^b$.

In certain embodiments, $B^8$ and $B^9$ are C.

In certain embodiments, $B^{11}$, $B^{12}$, and $B^{13}$ are independently CH, $CR^{c2}$, or C-$(L^b)_b-R^b$. As a non-limitinge example, $B^{11}$, $B^{12}$, and $B^{13}$ are CH.

In certain embodiments, $B^6$ is N. In certain embodiments, $B^7$ is N.

In certain enbodiments, $B^{10}$ is CH or $CR^{c2}$, or C-$(L^b)_b-R^b$. As a non-limiting example, $B^{10}$ can be CH.

As a non-limiting example, **Ar²** can be

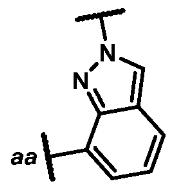

which is optionally substituted with from 1-2 substituents, each of which is independently selected from the group consisting of: $R^{c2}$ and $-(L^b)_b-R^b$ , wherein ***aa*** is the point of attachment to - $(CR^{3a}R^{3b})_n-R^2$.

In certain embodiments, $B^7$ is N. In certain embodiments, $B^{10}$ is S. As a non-limiting example, **Ar²** can be

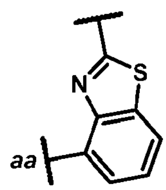

which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to $\mathbf{-(CR^{3a}R^{3b})_n-R^2}$.

In certain embodiment, $\mathbf{B^8}$ is C and $\mathbf{B^9}$ is N. In certain embodiments, $\mathbf{B^7}$ is N. In certain embodiments, $\mathbf{B^{10}}$, $\mathbf{B^{11}}$, $\mathbf{B^{12}}$, and $\mathbf{B^{13}}$ are CH, $\mathbf{CR^{c2}}$, or $\mathbf{C-(L^b)_b-R^b}$.

As a non-limiting example, $\mathbf{Ar^2}$ can be

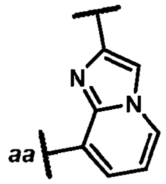,

which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to - $\mathbf{(CR^{3a}R^{3b})_n-R^2}$.

As further non-limiting examples, $\mathbf{Ar^2}$ can be selected from the group consisting of:

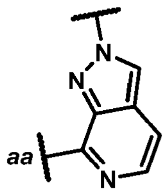, 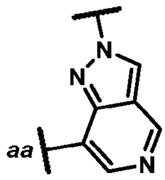 and 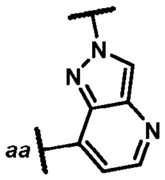,

each of which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to - $\mathbf{(CR^{3a}R^{3b})_n-R^2}$.

As further non-limiting examples, $\mathbf{Ar^2}$ can be selected from the group consisting of:

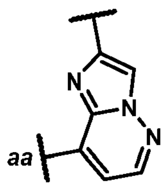, 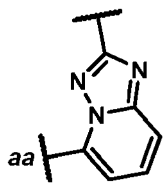, and 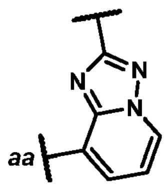,

each of which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to $\mathbf{-(CR^{3a}R^{3b})_n-R^2}$.

As further non-limiting examples, $\mathbf{Ar^2}$ can be selected from the group consisting of

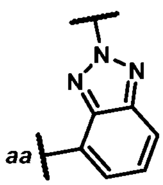, 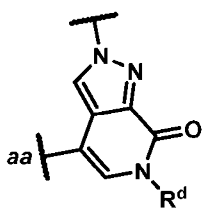, 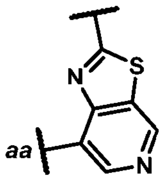, 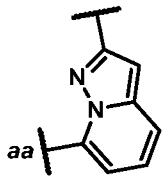, 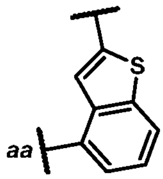,

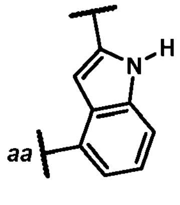 and 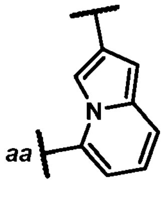,

each of which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to $\mathbf{-(CR^{3a}R^{3b})_n-R^2}$.

In certain embodiments of Formula **(I), (Ia), (I-A) or (I-Aa), Ar$^2$** is:

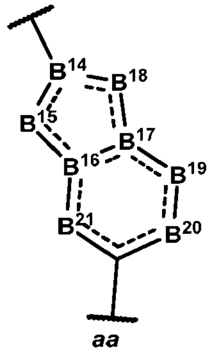

wherein:

each 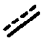 is independently a single bond or a double bond, provided that the 5-membered ring including **B$^{14}$**, **B$^{15}$**, **B$^{16}$**, **B$^{17}$**, and **B$^{18}$** is heteroaryl, and the 6-membered ring including **B$^{16}$**, **B$^{17}$, B$^{19}$**, **B$^{20}$**, and **B$^{21}$** is aryl or heteroaryl;
***aa*** is the point of attachment to **-(**C**R$^{3a}$R$^{3b}$)**$_n$-C(O)OH;
**B$^{16}$, B$^{17}$**, and **B$^{14}$** are independently C or N;
**B$^{15}$** and **B$^{18}$** are independently selected from the group consisting of: O, S, N, N(H), N(**R$^d$**), CH, and C**R$^{c2}$**;
**B$^{19}$**, **B$^{20}$**, and **B$^{21}$** are independently N, CH, or C**R$^{c2}$**, provided that:
from 1-4 of **B$^{14}$**, **B$^{15}$**, **B$^{16}$**, **B$^{17}$**, **B$^{18}$**, **B$^{19}$**, **B$^{20}$**, and **B$^{21}$** is an independently selected heteroatom; and no more than 3 of **B$^{15}$**, **B$^{18}$**, **B$^{19}$**, **B$^{20}$**, and **B$^{21}$** are C**R$^{c2}$**.

In certain embodiments of Formula **(I), (Ia), (I-A)** or **(I-Aa)**, **Ar$^2$** is:

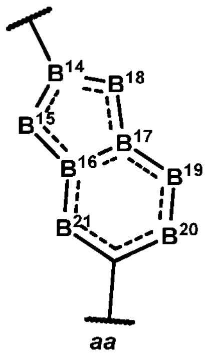

,

wherein:

each 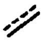 is independently a single bond or a double bond, provided that the 5-membered ring including **B$^{14}$**, **B$^{15}$**, **B$^{16}$**, **B$^{17}$**, and **B$^{18}$** is heteroaryl, and the 6-membered ring including **B$^{16}$**, **B$^{17}$**, **B$^{19}$**, **B$^{20}$**, and **B$^{21}$** is aryl or heteroaryl;
***aa*** is the point of attachment to -(C**R$^{3a}$R$^{3b}$**)$_n$-**R$^2$**;
**B$^{16}$**, **B$^{17}$**, and **B$^{14}$** are independently C or N;
**B$^{15}$** and **B$^{18}$** are independently selected from the group consisting of: O, S, N, N(H), N(**R$^d$**), CH, and C**R$^{c2}$**;
**B$^{19}$**, **B$^{20}$**, and **B$^{21}$** are independently N, CH, C**R$^{c2}$**, or C-**(L$^b$)$_b$-R$^b$,** provided that:
from 1-4 of **B$^{14}$**, **B$^{15}$**, **B$^{16}$**, **B$^{17}$**, **B$^{18}$**, **B$^{19}$**, **B$^{20}$**, and **B$^{21}$** is an independently selected heteroatom; and no more than 3 of **B$^{15}$, B$^{18}$**, **B$^{19}$**, **B$^{20}$**, and **B$^{21}$** are C**R$^{c2}$** or C-**(L$^b$)$_b$-R$^b$.**

In certain of these embodiments, **B$^{16}$** and **B$^{17}$** are C.
In certain embodiments, **B$^{19}$**, **B$^{20}$**, and **B$^{21}$** are independently N, CH, or C**R$^{c2}$**.
In Certain embodiments, **B$^{19}$**, **B$^{20}$**, and **B$^{21}$** are independently N, CH, C**R$^{c2}$**, or or C-**(L$^b$)$_b$-R$^b$**.
In certain embodiments, **B$^{14}$** is C.
In certain embodiments, one of **B$^{15}$** and **B$^{18}$** is N; and the other of **B$^{15}$** and **B$^{18}$** is O, S, NH, or N(**R$^d$**), such as NH or N(**R$^d$**), such as NH or N($C_{1-3}$ alkyl).

As non-limiting examples of the foregoing embodiments, $\mathbf{Ar^2}$ can be

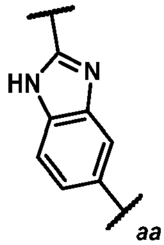 or 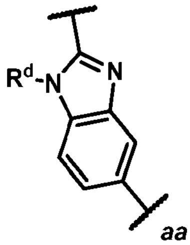,

each of which is optionally substituted with from 1-2 $\mathbf{R^{c2}}$, wherein ***aa*** is the point of attachment to -(C$\mathbf{R^{3a}R^{3b}}$)$_n$-C(O)OH.

As further non-limiting examples, $\mathbf{Ar^2}$ can be

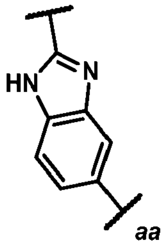 or 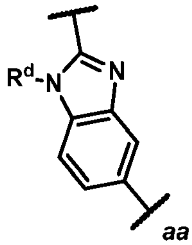,

each of which is optionally substituted with from 1-2 $\mathbf{R^{c2}}$, wherein ***aa*** is the point of attachment to -(C$\mathbf{R^{3a}R^{3b}}$)$_n$-$\mathbf{R^2}$.

In certain embodiments of Formula **(I), (Ia), (I-A)** or **(I-Aa)**, $\mathbf{Ar^2}$ can be

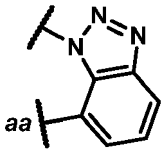,

which is optionally substituted with from 1-2 substituents, each of which is independently selected from the group consisting of: $\mathbf{R^{c2}}$ and **-($\mathbf{L^b}$)$_b$-$\mathbf{R^b}$,** wherein ***aa*** is the point of attachment to -(C$\mathbf{R^{3a}R^{3b}}$)$_n$-$\mathbf{R^2}$.

***Variables n, $R^{3a}$, and $R^{3b}$***

In some embodiments, **n** is 0.

In some embodiments, **n** is 1.

In some embodiments (when n is 1), $\mathbf{R^{3a}}$ and $\mathbf{R^{3b}}$ are H.

In some embodiments, (when n is 1), one of $\mathbf{R^{3a}}$ and $\mathbf{R^{3b}}$ is H, and the other one of $\mathbf{R^{3a}}$ and $\mathbf{R^{3b}}$ is $C_{1-6}$ alkyl, such as ethyl or methyl.

In some embodiments, (when n is 1), $\mathbf{R^{3a}}$ and $\mathbf{R^{3b}}$ together with the carbon atom to which each is attached forms a $C_{3-6}$ cycloalkyl, such as a cyclopropyl.

***Variable $R^2$***

In some embodiments, $\mathbf{R^2}$ is -C(=O)OH.

In some embodiments, $\mathbf{R^2}$ is carboxylic acid bioisostere.

In certain embodiments, $\mathbf{R^2}$ is -C(=O)$NH_2$, -C(=O)$NHSO_2$Me or

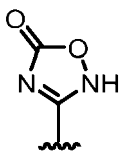.

***Non-Limiting Combinations***

Formula (**I-1**), (**I-1a**)

In certain embodiments, the compound of Formula **(I)** is a compound of Formula (**I-1**):

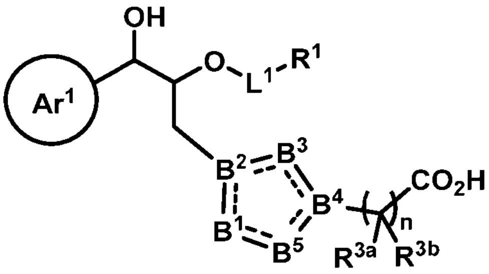

Formula (**I-1**)

or a pharmaceutically acceptable salt thereof, wherein:

each ⫽ is independently a single bond or a double bond, provided that the ring including **B^1^**, **B^2^**, **B^3^**, **B^4^**, and **B^5^** is heteroaryl;
**B**$^2$ and **B**$^4$ are C or N;
**B**$^1$, **B**$^3$, and **B**$^5$ are independently O, S, N, N(H), N(**R**$^d$), CH, or C**R**$^{c2}$, provided that: from 1-4 of **B**$^1$, **B**$^2$, **B**$^3$, **B**$^4$, and **B**$^5$ are independently selected heteroatoms; and
**Ar**$^1$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^g$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^g$.

In certain embodiments, the compound of Formula (I) is a compound of Formula (**I-1**):

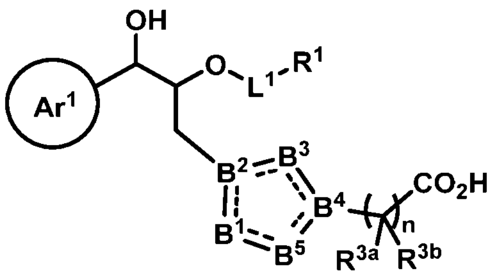

Formula (**I-1**)

or a pharmaceutically acceptable salt thereof, wherein:

each ⫽ is independently a single bond or a double bond, provided that the ring including **B**$^1$, **B**$^2$, **B**$^3$, **B**$^4$, and **B**$^5$ is heteroaryl;
**B**$^2$ and **B**$^4$ are C or N;
**B**$^1$, **B**$^3$, and **B**$^5$ are independently O, S, N, N(H), N(**R**$^d$), CH, or C**R**$^{c2}$, provided that: from 1-4 of **B**$^1$, **B**$^2$, **B**$^3$, **B**$^4$, and **B**$^5$ are independently selected heteroatoms; and
**Ar**$^1$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

$\mathbf{R^1}$ is selected from the group consisting of: $\mathbf{R^b}$; $C_{2-6}$ alkenyl optionally substituted with from 1-6 $\mathbf{R^a}$; and $C_{2-6}$ alkynyl optionally substituted with from 1-6 $\mathbf{R^a}$.

In certain embodiments, the compound of Formula **(I)** is a compound of Formula (**I-1**):

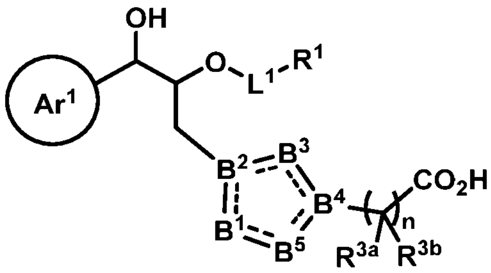

Formula (**I-1**)

or a pharmaceutically acceptable salt thereof, wherein:

each 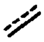 is independently a single bond or a double bond, provided that the ring including $\mathbf{B^1}$, $\mathbf{B^2}$, $\mathbf{B^3}$, $\mathbf{B^4}$, and $\mathbf{B^5}$ is heteroaryl;

$\mathbf{B^2}$ and $\mathbf{B^4}$ are C or N;

$\mathbf{B^1}$, $\mathbf{B^3}$, and $\mathbf{B^5}$ are independently O, S, N, N(H), N($\mathbf{R^d}$), CH, or C$\mathbf{R^{c2}}$, provided that: from 1-4 of $\mathbf{B^1}$, $\mathbf{B^2}$, $\mathbf{B^3}$, $\mathbf{B^4}$, and $\mathbf{B^5}$ are independently selected heteroatoms; and

$\mathbf{Ar^1}$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and $\mathbf{R^1}$ is $C_{1-6}$ alkyl optionally substituted with from 1-6 $\mathbf{R^a}$.

In certain embodiments, the compound of Formula **(I)** is a compound of Formula (I-**1a)**:

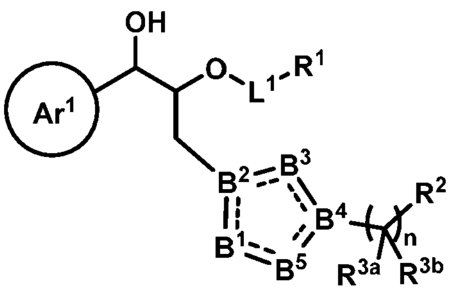

Formula (**I-1a)**

or a pharmaceutically acceptable salt thereof, wherein:

each 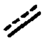 is independently a single bond or a double bond, provided that the ring including **B$^1$**, **B$^2$**, **B$^3$**, **B$^4$**, and **B$^5$** is heteroaryl;

**B$^2$** and **B$^4$** are C or N;

**B$^1$**, **B$^3$**, and **B$^5$** are independently O, S, N, N(H), N(**R$^d$**), CH, C**R$^{c2}$**, or C-**(L$^b$)$_b$-R$^b$**, provided that: from 1-4 of **B$^1$**, **B$^2$**, **B$^3$**, **B$^4$**, and **B$^5$** are independently selected heteroatoms; and

**Ar$^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**.

In certain embodiments, the compound of Formula (I) is a compound of Formula (I-1a):

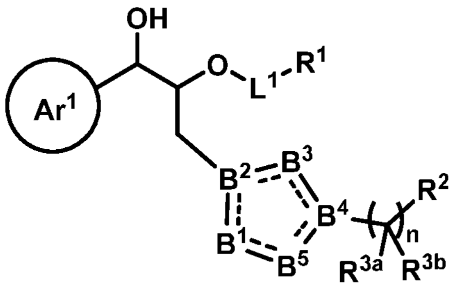

Formula (**I-1a**)

or a pharmaceutically acceptable salt thereof, wherein:

each 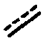 is independently a single bond or a double bond, provided that the ring including **B$^1$**, **B$^2$**, **B$^3$**, **B$^4$**, and **B$^5$** is heteroaryl;

**B$^2$** and **B$^4$** are C or N;

**B$^1$**, **B$^3$**, and **B$^5$** are independently O, S, N, N(H), N(**R$^d$**), CH, C**R$^{c2}$**, or C-**(L$^b$)$_b$-R$^b$**, provided that: from 1-4 of **B$^1$**, **B$^2$**, **B$^3$**, **B$^4$**, and **B$^5$** are independently selected heteroatoms; and

**Ar$^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and

**R$^1$** is selected from the group consisting of: **R$^b$**; $C_{2-6}$ alkenyl optionally substituted with from 1-6 **R$^a$**; and $C_{2-6}$ alkynyl optionally substituted with from 1-6 **R$^a$**.

In certain embodiments, the compound of Formula **(I)** is a compound of Formula **(I-1a):**

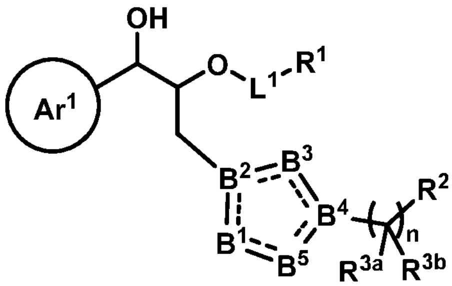

Formula **(I-1a)**

or a pharmaceutically acceptable salt thereof, wherein:

each 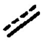 is independently a single bond or a double bond, provided that the ring including $\mathbf{B^1}$, $\mathbf{B^2}$, $\mathbf{B^3}$, $\mathbf{B^4}$, and $\mathbf{B^5}$ is heteroaryl;

$\mathbf{B^2}$ and $\mathbf{B^4}$ are C or N;

$\mathbf{B^1}$, $\mathbf{B^3}$, and $\mathbf{B^5}$ are independently O, S, N, N(H), N($\mathbf{R^d}$), CH, C$\mathbf{R^{c2}}$, or C-$\mathbf{(L^b)_b}$-$\mathbf{R^b}$, provided that: from 1-4 of $\mathbf{B^1}$, $\mathbf{B^2}$, $\mathbf{B^3}$, $\mathbf{B^4}$, and $\mathbf{B^5}$ are independently selected heteroatoms; and

$\mathbf{Ar^1}$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

$\mathbf{R^1}$ is $C_{1-6}$ alkyl optionally substituted with from 1-6 $\mathbf{R^a}$.

In certain embodiments of Formula **(I-1)** or **(I-1a),** $\mathbf{B^2}$ is N; and $\mathbf{B^4}$ is C.

In certain embodiments of Formula **(I-1)** or **(I-1a),** $\mathbf{B^1}$, $\mathbf{B^3}$, and $\mathbf{B^5}$ are independently CH or C$\mathbf{R^{c2}}$.

In certain embodiments of Formula **(I-1)** or **(I-1a),** $\mathbf{B^1}$, $\mathbf{B^3}$, and $\mathbf{B^5}$ are independently CH, C$\mathbf{R^{c2}}$ or C-$\mathbf{(L^b)_b}$-$\mathbf{R^b}$.

As a non-limiting example of the foregoing embodiments, the ring including $\mathbf{B^1}$-$\mathbf{B^5}$ can be

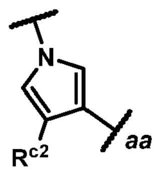

,

wherein ***aa*** is the point of attachment to -(C$\mathbf{R^{3a}R^3}$)$_n$-C(O)OH, optionally wherein $\mathbf{R^{c2}}$ is C(O)O$C_{1-4}$ alkyl.

As a non-limiting example of the foregoing embodiments, the ring including $\mathbf{B^1}$-$\mathbf{B^5}$ can be

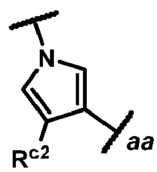

,

wherein ***aa*** is the point of attachment to -(C$\mathbf{R^{3a}R^{3b}}$)$_n$-$\mathbf{R^2}$, optionally wherein $\mathbf{R^{c2}}$ is C(O)O$C_{1-4}$ alkyl.

In certain embodiments of Formula **(I-1)** or **(I-1a),** $\mathbf{B^2}$ is C; and $\mathbf{B^4}$ is N.

In certain embodiments of Formula **(I-1)** or **(I-1a),** $\mathbf{B^3}$ is N; and $\mathbf{B^1}$ and $\mathbf{B^5}$ are independently CH or C$\mathbf{R^{c2}}$.

In certain embodiments of Formula **(I-1)** or **(I-1a)**or Formula **(I-1a),** $\mathbf{B^3}$ is N; and $\mathbf{B^1}$ and $\mathbf{B^5}$ are independently CH, C$\mathbf{R^{c2}}$ or C-$\mathbf{(L^b)_b}$-$\mathbf{R^b}$.

As a non-limiting example of the foregoing embodiments, the ring including $\mathbf{B^1}$-$\mathbf{B^5}$ can be

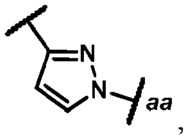

wherein ***aa*** is the point of attachment to -(C$\mathbf{R^{3a}R^{3b}}$)$_n$-C(O)OH.

As a non-limiting example of the foregoing embodiments, the ring including $\mathbf{B^1}$-$\mathbf{B^5}$ is

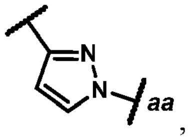

wherein ***aa*** is the point of attachment to -(C$\mathbf{R^{3a}R^{3b}}$)$_n$-$\mathbf{R^2}$.

In certain embodiments of Formula **(I-1)** or **(I-1a),** $\mathbf{B^2}$ is C; and $\mathbf{B^4}$ is C.

In certain embodiments of Formula **(I-1)** or **(I-1a),** $\mathbf{B^5}$ is CH or C$\mathbf{R^{c2}}$; one of $\mathbf{B^1}$ and $\mathbf{B^3}$ is N; and the other of $\mathbf{B^1}$ and $\mathbf{B^3}$ is NH, N($\mathbf{R^d}$), O, or S, such as S.

As non-limiting examples of the foregoing embodiments, the ring including $\mathbf{B^1}$-$\mathbf{B^5}$ is

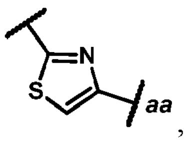

wherein ***aa*** is the point of attachment to -(C$\mathbf{R^{3a}R^{3b}}$)$_n$-C(O)OH. As another non-limiting examples of the foregoing embodiments, the ring including $\mathbf{B^1}$-$\mathbf{B^5}$ is

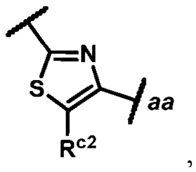

wherein ***aa*** is the point of attachment to - (C$\mathbf{R^{3a}R^{3b}}$)$_n$-$\mathbf{R^2}$, optionally wherein $\mathbf{R^{c2}}$ is C(O)OC$_{1-4}$ alkyl, C$_{1-4}$ alkyl, or C$_{1-4}$ alkoxy or C$_{1-4}$ haloalkoxy.

As another non-limiting examples of the foregoing embodiments, the ring including $\mathbf{B^1}$-$\mathbf{B^5}$ is ,

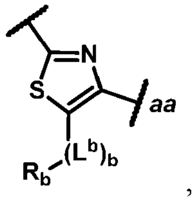

wherein ***aa*** is the point of attachment to - (C$\mathbf{R^{3a}R^{3b}}$)$_n$-$\mathbf{R^2}$, optionally wherein **-(L$^b$)$_b$-R$^b$** is C$_{3-10}$ cycloalkyl, such as cyclopropyl.

As another non-limiting examples of the foregoing embodiments, the ring including $\mathbf{B^1}$-$\mathbf{B^5}$ is

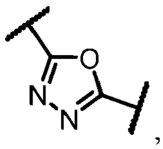

wherein ***aa*** is the point of attachment to -(C$\mathbf{R^{3a}R^{3b}}$)$_n$-$\mathbf{R^2}$.

Formula (**I-2**), **(I-2a)**

In certain embodiments, the compound of Formula **(I)** is a compound of Formula **(I-2)**:

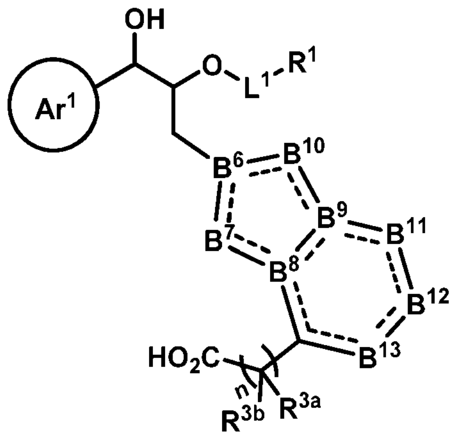

Formula (**I-2**)

or a pharmaceutically acceptable salt thereof, wherein:

each 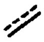 is independently a single bond or a double bond, provided that the 5-membered ring including $\mathbf{B^6}$, $\mathbf{B^7}$, $\mathbf{B^8}$, $\mathbf{B^9}$, and $\mathbf{B^{10}}$ is heteroaryl, and the 6-membered ring including $\mathbf{B^8}$, $\mathbf{B^9}$, $\mathbf{B^{11}}$, $\mathbf{B^{12}}$, and $\mathbf{B^{13}}$ is aryl or heteroaryl;

$\mathbf{B^6}$, $\mathbf{B^8}$, and $\mathbf{B^9}$ are independently C or N;

$\mathbf{B^7}$ and $\mathbf{B^{10}}$ are independently selected from the group consisting of: O, S, N, N(H), N($\mathbf{R^d}$), CH, or C$\mathbf{R^{c2}}$;

$\mathbf{B^{11}}$, $\mathbf{B^{12}}$, and $\mathbf{B^{13}}$ are independently N, CH, or C$\mathbf{R^{c2}}$, provided that:

from 1-4 of $\mathbf{B^6}$, $\mathbf{B^7}$, $\mathbf{B^8}$, $\mathbf{B^9}$, $\mathbf{B^{10}}$, $\mathbf{B^{11}}$, $\mathbf{B^{12}}$, and $\mathbf{B^{13}}$ is an independently selected heteroatom; and no more than 3 of $\mathbf{B^7}$, $\mathbf{B^{10}}$, $\mathbf{B^{11}}$, $\mathbf{B^{12}}$, and $\mathbf{B^{13}}$ are $\mathbf{CR^{c2}}$; and

$\mathbf{Ar^1}$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

In certain embodiments, the compound of Formula **(I)** is a compound of Formula (**I-2**):

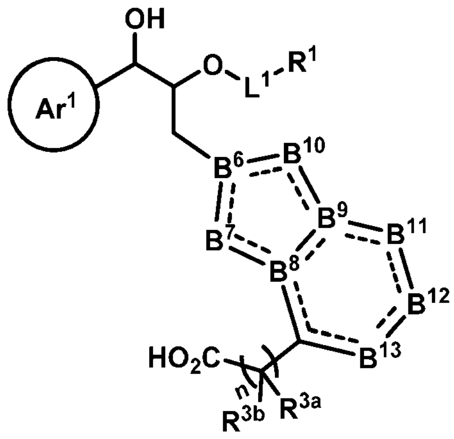

Formula (**I-2**)

or a pharmaceutically acceptable salt thereof, wherein:

each [bond symbol] is independently a single bond or a double bond, provided that the 5-membered ring including **B⁶**, **B⁷**, **B⁸**, **B⁹**, and **B¹⁰** is heteroaryl, and the 6-membered ring including **B⁸**, **B⁹**, **B¹¹**, **B¹²**, and **B¹³** is aryl or heteroaryl;
**B⁶**, **B⁸**, and **B⁹** are independently C or N;
**B⁷** and **B¹⁰** are independently selected from the group consisting of: O, S, N, N(H), N(**R^d**), CH, or C**R^c2**;
**B¹¹**, **B¹²**, and **B¹³** are independently N, CH, or C**R^c2**, provided that:

from 1-4 of **B⁶**, **B⁷**, **B⁸**, **B⁹**, **B¹⁰**, **B¹¹**, **B¹²**, and **B¹³** is an independently selected heteroatom; and no more than 3 of **B⁷**, **B¹⁰**, **B¹¹**, **B¹²**, and **B¹³** are C**R^c2**; and

**Ar¹** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and
$\mathbf{R^1}$ is selected from the group consisting of: $\mathbf{R^b}$; $C_{2-6}$ alkenyl optionally substituted with from 1-6 $\mathbf{R^a}$; and $C_{2-6}$ alkynyl optionally substituted with from 1-6 $\mathbf{R^a}$.

In certain embodiments, the compound of Formula **(I)** is a compound of Formula **(I-2)**:

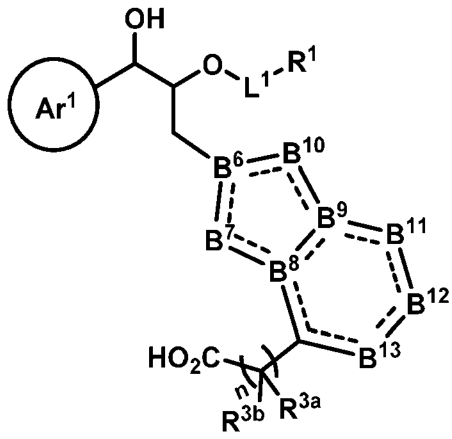

Formula **(I-2)**

or a pharmaceutically acceptable salt thereof, wherein:

each 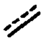 is independently a single bond or a double bond, provided that the 5-membered ring including **B$^6$**, **B$^7$**, **B$^8$**, **B$^9$**, and **B$^{10}$** is heteroaryl, and the 6-membered ring including **B$^8$**, **B$^9$**, **B$^{11}$**, **B$^{12}$**, and **B$^{13}$** is aryl or heteroaryl;
**B$^6$**, **B$^8$**, and **B$^9$** are independently C or N;
**B$^7$** and **B$^{10}$** are independently selected from the group consisting of: O, S, N, N(H), N(**R$^d$**), CH, or C**R$^{c2}$**;
**B$^{11}$**, **B$^{12}$**, and **B$^{13}$** are independently N, CH, or C**R$^{c2}$**, provided that:

from 1-4 of **B$^6$**, **B$^7$**, **B$^8$**, **B$^9$**, **B$^{10}$**, **B$^{11}$**, **B$^{12}$**, and **B$^{13}$** is an independently selected heteroatom; and no more than 3 of **B$^7$**, **B$^{10}$**, **B$^{11}$**, **B$^{12}$**, and **B$^{13}$** are C**R$^{c2}$**; and

**Ar$^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and
**R$^1$** is $C_{1-6}$ alkyl optionally substituted with from 1-6 **R$^a$**.

In certain embodiments of Formula **(I-2)**, **B$^8$** and **B$^9$** are C.
In certain embodiments of Formula **(I-2)**, **B$^{11}$**, **B$^{12}$**, and **B$^{13}$** are independently CH or C**R$^{c2}$**.
In certain embodiments of Formula **(I-2)**, **B$^6$** is N; **B$^7$** is N; and **B$^{10}$** is CH or C**R$^{c2}$**.
As a non-limiting example of the foregoing embodiments, the ring including **B$^6$-B$^{13}$** can be

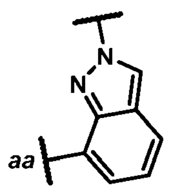

,

which is optionally substituted with from 1-2 **R$^{c2}$**, wherein ***aa*** is the point of attachment to -(C**R$^{3a}$R$^{3b}$**)$_n$ C(O)OH.
In certain embodiments, the compound of Formula **(I)** is a compound of Formula **(I-2a):**

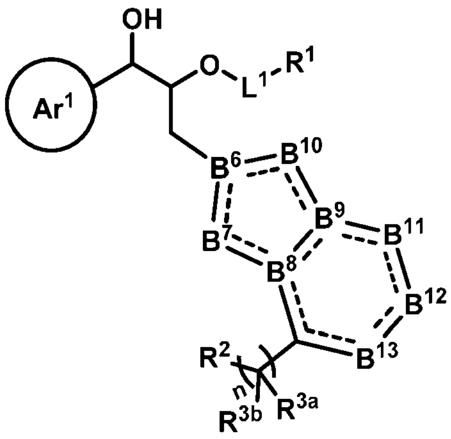

Formula (**I-2a**)

or a pharmaceutically acceptable salt thereof, wherein:

each 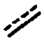 is independently a single bond or a double bond, provided that the 5-membered ring including **B⁶**, **B⁷**, **B⁸**, **B⁹**, and **B¹⁰** is heteroaryl, and the 6-membered ring including **B⁸**, **B⁹**, **B¹¹**, **B¹²**, and **B¹³** is aryl or heteroaryl;

**$B^6$**, **$B^8$**, and **$B^9$** are independently C or N;

**$B^7$** and **$B^{10}$** are independently selected from the group consisting of: O, S, N, N(H), N(**$R^d$**), CH, C**$R^{c2}$**, or C-**$(L^b)_b$-$R^b$**;

**$B^{11}$**, **$B^{12}$**, and **$B^{13}$** are independently N, CH, C**$R^{c2}$**, or C-**$(L^b)_b$-$R^b$**, provided that:

from 1-4 of **$B^6$**, **$B^7$**, **$B^8$**, **$B^9$**, **$B^{10}$**, **$B^{11}$**, **$B^{12}$**, and **$B^{13}$** is an independently selected heteroatom; and no more than 3 of **$B^7$**, **$B^{10}$**, **$B^{11}$**, **$B^{12}$**, and **$B^{13}$** are C**$R^{c2}$** or C-**$(L^b)_b$-$R^b$**;

**$Ar^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **$R^g$**; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **$R^g$**.

In certain embodiments, the compound of Formula **(I)** is a compound of Formula **(I-2a):**

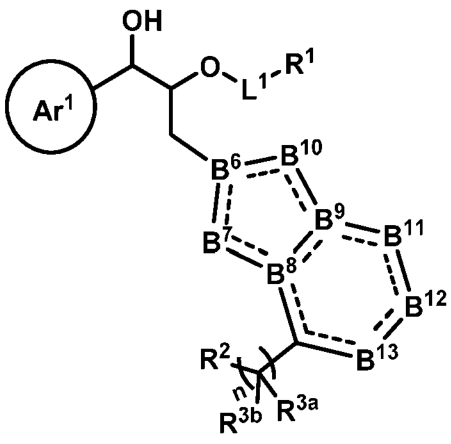

Formula (**I-2a**)

or a pharmaceutically acceptable salt thereof, wherein:

each 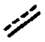 is independently a single bond or a double bond, provided that the 5-membered ring including **B⁶**, **B⁷**, **B⁸**, **B⁹**, and **B¹⁰** is heteroaryl, and the 6-membered ring including **B⁸**, **B⁹**, **B¹¹**, **B¹²**, and **B¹³** is aryl or heteroaryl;
**B⁶**, **B⁸**, and **B⁹** are independently C or N;
**B⁷** and **B¹⁰** are independently selected from the group consisting of: O, S, N, N(H), N($R^d$), CH, C$R^{c2}$, or C-$(L^b)_b$-$R^b$;
**B¹¹**, **B¹²**, and **B¹³** are independently N, CH, C$R^{c2}$, or C-$(L^b)_b$-$R^b$, provided that:

from 1-4 of **B⁶**, **B⁷**, **B⁸**, **B⁹**, **B¹⁰**, **B¹¹**, **B¹²**, and **B¹³** is an independently selected heteroatom; and no more than 3 of **B⁷**, **B¹⁰**, **B¹¹**, **B¹²**, and **B¹³** are C$R^{c2}$ or C-$(L^b)_b$-$R^b$;

**Ar¹** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and
$R^1$ is selected from the group consisting of: $R^b$; $C_{2-6}$ alkenyl optionally substituted with from 1-6 $R^a$; and $C_{2-6}$ alkynyl optionally substituted with from 1-6 $R^a$.

In certain embodiments, the compound of Formula **(I)** is a compound of Formula **(I-2a)**:

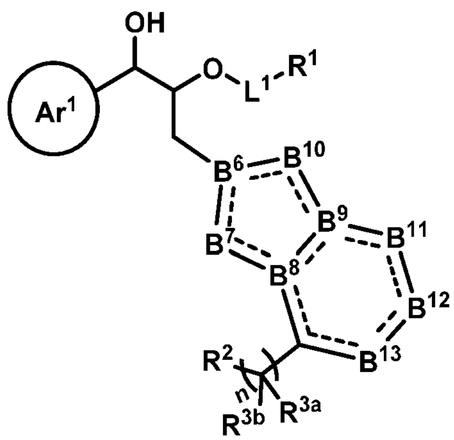

Formula (**I-2a**)

or a pharmaceutically acceptable salt thereof, wherein:

each 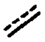 is independently a single bond or a double bond, provided that the 5-membered ring including **B⁶**, **B⁷**, **B⁸**, **B⁹**, and **B¹⁰** is heteroaryl, and the 6-membered ring including **B⁸**, **B⁹**, **B¹¹**, **B¹²**, and **B¹³** is aryl or heteroaryl;
**B⁶**, **B⁸**, and **B⁹** are independently C or N;
**B⁷** and **B¹⁰** are independently selected from the group consisting of: O, S, N, N(H), N($R^d$), CH, C$R^{c2}$, or C-$(L^b)_b$-$R^b$;
**B¹¹**, **B¹²**, and **B¹³** are independently N, CH, C$R^{c2}$, or C-$(L^b)_b$-$R^b$, provided that:

from 1-4 of **B⁶**, **B⁷**, **B⁸**, **B⁹**, **B¹⁰**, **B¹¹**, B¹², and **B¹³** is an independently selected heteroatom; and no more than 3 of **B⁷**, **B¹⁰**, **B¹¹**, **B¹²**, and **B¹³** are C$R^{c2}$ or C-$(L^b)_b$-$R^b$;

**Ar¹** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and
heteroaryl including 6 ring atoms, wherein from 1-2 ring atoms are ring nitrogen atoms, wherein the heteroaryl

is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **$R^g$**; and

**$R^1$** is $C_{1-6}$ alkyl optionally substituted with from 1-6 **$R^a$**.

In certain embodiments of Formula **(I-2)** or **(I-2a), $B^8$** and **$B^9$** are C.

In certain embodiments of Formula (I-2) or (I-2a), **$B^{11}$**, **$B^{12}$**, and **$B^{13}$** are independently CH or C**$R^{c2}$**.

In certain embodiments of Formula **(I-2)** or **(I-2a)**, **$B^6$** is N; **$B^7$** is N; and **$B^{10}$** is CH or C**$R^{c2}$**.

As a non-limiting example of the foregoing embodiments of Formula **(I-2)** or **(I-2a),** the ring including **$B^6$-$B^{13}$** can be

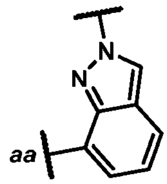

,

which is optionally substituted with from 1-2 **$R^{c2}$**, wherein ***aa*** is the point of attachment to -(C**$R^{3a}R^{3b}$**)$_n$-**$R^2$**.

In certain embodiments of Formula **(I-2)** or **(I-2a)**, **$B^7$** is N. In certain embodiments, **$B^{10}$** is S.

As a non-limitinge example of embodiments of Formula **(I-2)** or **(I-2a)**, the ring including **$B^6$-$B^{13}$** can be

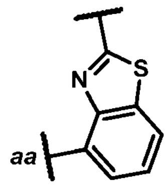

,

which is optionally substituted with from 1-2 substituents, each of which is independently selected from the group consisting of: **$R^{c2}$** and **-($L^b$)$_b$-$R^b$,** wherein ***aa*** is the point of attachment to -(C**$R^{3a}R^{3b}$**)$_n$-**$R^2$**.

In certain embodiments of Formula **(I-2)** or **(I-2a)**, **$B^8$** is C; and **$B^9$** is N. In certain embodiments, **B7** is N.

In certain embodiments of Formula **(I-2)** or **(I-2a)**, **$B^{10}$**, **$B^{11}$**, **$B^{12}$**, and **$B^{13}$** are CH, C**$R^{c2}$**, or C-**($L^b$)$_b$-$R^b$**.

As a non-limiting example of embodiments Formula **(I-2)** or **(I-2a),** the ring including **$B^6$-$B^{13}$** can be

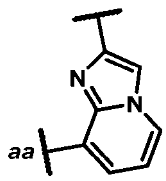

,

which is optionally substituted with from 1-2 substituents, each of which is independently selected from the group consisting of: **$R^{c2}$** and **-($L^b$)$_b$-$R^b$,** wherein ***aa*** is the point of attachment to -(C**$R^{3a}R^{3b}$**)$_n$-$R^2$**.**

As further non-limiting examples of embodiments of Formula **(I-2)** or **(I-2a),** the ring including **$B^6$-$B^{13}$** is selected from the group consisting of

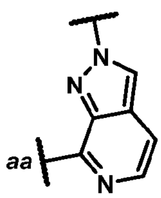

,

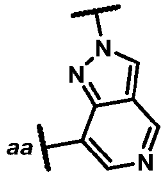 and 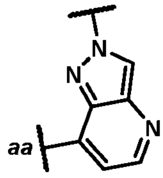,

each of which is optionally substituted with from 1-2 substituents, each of which is independently selected from the group consisting of: $\mathbf{R^{c2}}$ and -$(\mathbf{L^b})_\mathbf{b}$-$\mathbf{R^b}$, wherein ***aa*** is the point of attachment to -(C$\mathbf{R^{3a}R^{3b}}$)$_n$-$\mathbf{R^2}$.

As further non-limiting examples of embodiments of Formula **(I-2)** or **(I-2a)**, the ring including $\mathbf{B^6}$-$\mathbf{B^{13}}$ is selected from the group consisting of

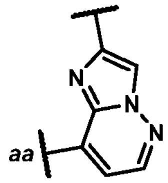,

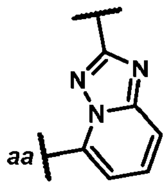, and 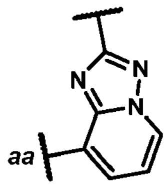,

each of which is optionally substituted with from 1-2 substituents, each of which is independently selected from the group consisting of: $\mathbf{R^{c2}}$ and **-$(\mathbf{L^b})_\mathbf{b}$-$\mathbf{R^b}$,** wherein ***aa*** is the point of attachment to -(C$\mathbf{R^{3a}R^{3b}}$)$_n$-$\mathbf{R^2}$.

As further non-limiting examples of embodiments of Formula **(I-2)** or **(I-2a)**, the ring including $\mathbf{B^6}$-$\mathbf{B^{13}}$ is selected from the group consisting of

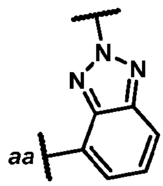,

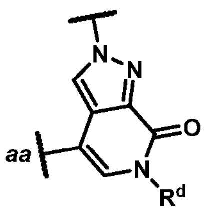, 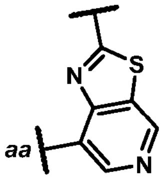, 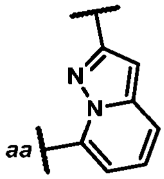, 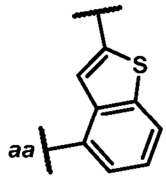, 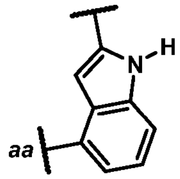

and

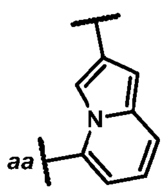,

each of which is optionally substituted with from 1-2 substituents, each of which is independently selected from the group

consisting of: $R^{c2}$ and **$-(L^b)_b-R^b$,** wherein ***aa*** is the point of attachment to $-(CR^{3a}R^{3b})_n-R^2$.

Formula (**I-3**), (**I-3a**)

In certain embodiments, the compound of Formula **(I)** is a compound of Formula **(I-3)**:

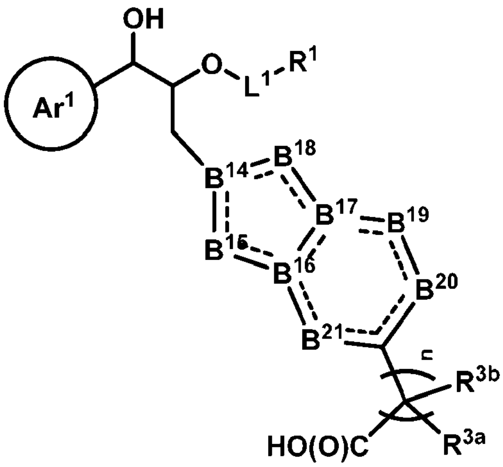

Formula (**I-3)**

or a pharmaceutically acceptable salt thereof, wherein:

each 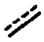 is independently a single bond or a double bond, provided that the 5-membered ring including $B^{14}$, $B^{15}$, $B^{16}$, $B^{17}$, and $B^{18}$ is heteroaryl, and the 6-membered ring including $B^{16}$, $B^{17}$, $B^{19}$, $B^{20}$, and $B^{21}$ is aryl or heteroaryl;
$B^{16}$, $B^{17}$, and $B^{14}$ are independently C or N;
$B^{15}$ and $B^{18}$ are independently selected from the group consisting of: O, S, N, N(H), N($R^d$), CH, and C$R^{c2}$;
$B^{19}$, $B^{20}$, and $B^{21}$ are independently N, CH, or C$R^{c2}$, provided that:

from 1-4 of $B^{14}$, $B^{15}$, $B^{16}$, $B^{17}$, $B^{18}$, $B^{19}$, $B^{20}$, and $B^{21}$ is an independently selected heteroatom; and no more than 3 of $B^{15}$, $B^{18}$, $B^{19}$, $B^{20}$, and $B^{21}$ are C$R^{c2}$; and
**$Ar^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$.

In certain embodiments, the compound of Formula **(I)** is a compound of Formula **(I-3)**:

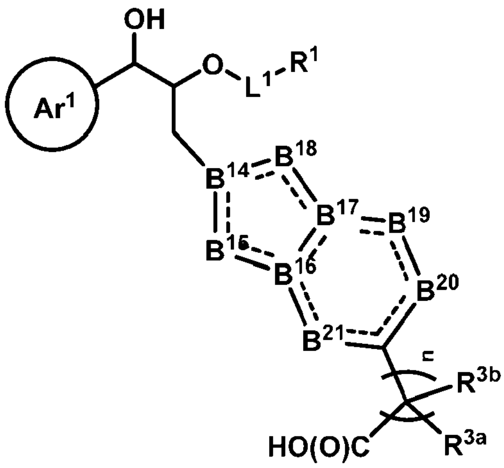

Formula (**I-3**)

or a pharmaceutically acceptable salt thereof, wherein:

each 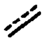 is independently a single bond or a double bond, provided that the 5-membered ring including $\mathbf{B^{14}}$, $\mathbf{B^{15}}$, $\mathbf{B^{16}}$, $\mathbf{B^{17}}$, and $\mathbf{B^{18}}$ is heteroaryl, and the 6-membered ring including $\mathbf{B^{16}}$, $\mathbf{B^{17}}$, $\mathbf{B^{19}}$, $\mathbf{B^{20}}$, and $\mathbf{B^{21}}$ is aryl or heteroaryl;
$\mathbf{B^{16}}$, $\mathbf{B^{17}}$, and $\mathbf{B^{14}}$ are independently C or N;
$\mathbf{B^{15}}$ and $\mathbf{B^{18}}$ are independently selected from the group consisting of: O, S, N, N(H), N($\mathbf{R^{d}}$), CH, and C$\mathbf{R^{c2}}$;
$\mathbf{B^{19}}$, $\mathbf{B^{20}}$, and $\mathbf{B^{21}}$ are independently N, CH, or C$\mathbf{R^{c2}}$, provided that:

from 1-4 of $\mathbf{B^{14}}$, $\mathbf{B^{15}}$, $\mathbf{B^{16}}$, $\mathbf{B^{17}}$, $\mathbf{B^{18}}$, $\mathbf{B^{19}}$, $\mathbf{B^{20}}$, and $\mathbf{B^{21}}$ is an independently selected heteroatom; and no more than 3 of $\mathbf{B^{15}}$, $\mathbf{B^{18}}$, $\mathbf{B^{19}}$, $\mathbf{B^{20}}$, and $\mathbf{B^{21}}$ are C$\mathbf{R^{c2}}$; and
$\mathbf{Ar^{1}}$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^{g}}$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^{g}}$; and
$\mathbf{R^{1}}$ is selected from the group consisting of: $\mathbf{R^{b}}$; $C_{2-6}$ alkenyl optionally substituted with from 1-6 $\mathbf{R^{a}}$; and $C_{2-6}$ alkynyl optionally substituted with from 1-6 $\mathbf{R^{a}}$.

In certain embodiments, the compound of Formula **(I)** is a compound of Formula **(I-3)**:

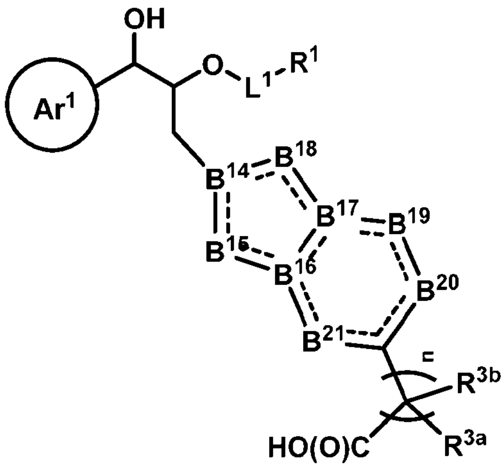

Formula (**I-3**)

or a pharmaceutically acceptable salt thereof, wherein:

each 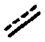 is independently a single bond or a double bond, provided that the 5-membered ring including **B^14^**, **B^15^**, **B^16^**, **B^17^**, and **B^18^** is heteroaryl, and the 6-membered ring including **B^16^**, **B^17^**, **B^19^**, **B^20^**, and **B^21^** is aryl or heteroaryl;

**B^16^**, **B^17^**, and **B^14^** are independently C or N;

**B^15^** and **B^18^** are independently selected from the group consisting of: O, S, N, N(H), N(**R^d^**), CH, and C**R^c2^**;

**B^19^**, **B^20^**, and **B^21^** are independently N, CH, or **CR^c2^**, provided that:

from 1-4 of **B^14^**, **B^15^**, **B^16^**, **B^17^**, **B^18^**, **B^19^**, **B^20^**, and **B^21^** is an independently selected heteroatom; and no more than 3 of **B^15^**, **B^18^**, **B^19^**, **B^20^**, and **B^21^** are **CR^c2^**; and

**Ar^1^** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R^g^**; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R^g^**; and

**R^1^** is $C_{1-6}$ alkyl optionally substituted with from 1-6 **R^a^**.

In certain embodiments, the compound of Formula (I) is a compound of Formula (I-3a):

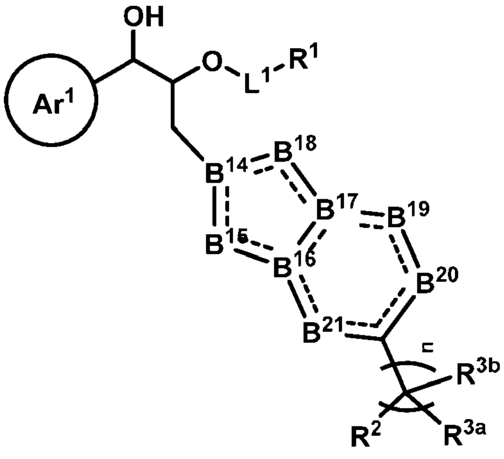

Formula (**I-3a**)

or a pharmaceutically acceptable salt thereof, wherein:

each 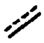 is independently a single bond or a double bond, provided that the 5-membered ring including **B$^{14}$**, **B$^{15}$**, **B$^{16}$**, **B$^{17}$**, and **B$^{18}$** is heteroaryl, and the 6-membered ring including **B$^{16}$**, **B$^{17}$**, **B$^{19}$**, **B$^{20}$**, and **B$^{21}$** is aryl or heteroaryl;
**B$^{16}$**, **B$^{17}$**, and **B$^{14}$** are independently C or N;
**B$^{15}$** and **B$^{18}$** are independently selected from the group consisting of: O, S, N, N(H), N(**R$^{d}$**), CH, **CR$^{c2}$**, and C-(**L$^{b}$**)$_b$-**R$^{b}$**.
**B$^{19}$**, **B$^{20}$**, and **B$^{21}$** are independently N, CH, **CR$^{c2}$**, or C-**(L$^{b}$)$_b$-R$^{b}$**, provided that:

from 1-4 of **B$^{14}$**, **B$^{15}$**, **B$^{16}$**, **B$^{17}$**, **B$^{18}$**, **B$^{19}$**, **B$^{20}$**, and **B$^{21}$** is an independently selected heteroatom; and no more than 3 of **B$^{15}$**, **B$^{18}$**, **B$^{19}$**, **B$^{20}$**, and **B$^{21}$** are CR$^{c2}$ or C-**(L$^{b}$)$_b$-R$^{b}$**; and

**Ar$^{1}$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^{g}$**; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^{g}$**.

In certain embodiments, the compound of Formula (I) is a compound of Formula **(I-3a):**

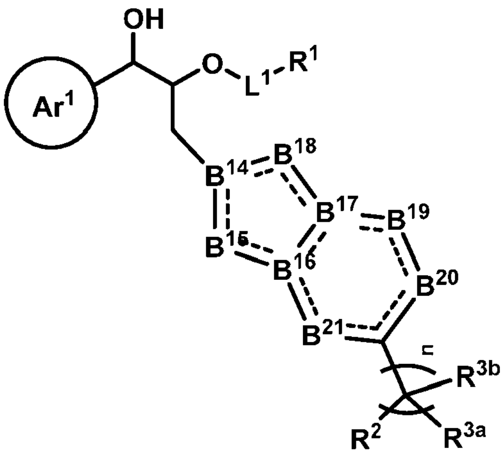

Formula (**I-3a**)

or a pharmaceutically acceptable salt thereof, wherein:

each 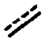 is independently a single bond or a double bond, provided that the 5-membered ring including **B^14**, **B^15**, **B^16**, **B^17**, and **B^18** is heteroaryl, and the 6-membered ring including **B^16**, **B^17**, **B^19**, **B^20**, and **B^21** is aryl or heteroaryl;
**B^16**, **B^17**, and **B^14** are independently C or N;
**B^15** and **B^18** are independently selected from the group consisting of: O, S, N, N(H), N(**R^d**), CH, **CR^c2**, and C-(**L^b**)$_b$-**R^b**.
**B^19**, **B^20**, and **B^21** are independently N, CH, **CR^c2**, or C-(**L^b**)$_b$-**R^b**, provided that:

from 1-4 of **B^14**, **B^15**, **B^16**, **B^17**, **B^18**, **B^19**, **B^20**, and **B^21** is an independently selected heteroatom; and no more than 3 of **B^15**, **B^18**, **B^19**, **B^20**, and **B^21** are $CR^{c2}$ or C-(**L^b**)$_b$-**R^b**; and

**Ar^1** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R^g**; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R^g**; and
**R^1** is selected from the group consisting of: **R^b**; $C_{2-6}$ alkenyl optionally substituted with from 1-6 **R^a**; and $C_{2-6}$ alkynyl optionally substituted with from 1-6 **R^a**.

In certain embodiments, the compound of Formula (**I**) is a compound of Formula **(I-3a)**:

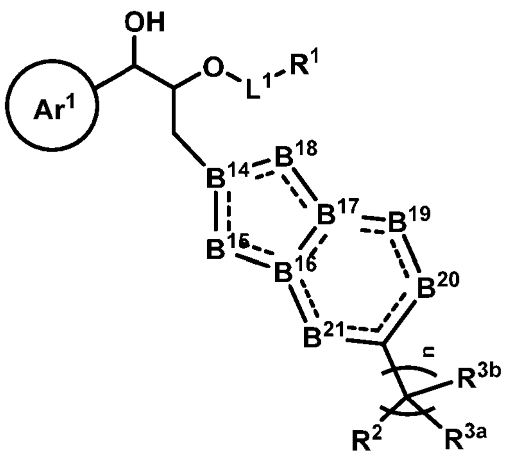

Formula (**I-3a**)

or a pharmaceutically acceptable salt thereof, wherein:

each 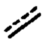 is independently a single bond or a double bond, provided that the 5-membered ring including **B^14**, **B^15**, **B^16**, **B^17**, and **B^18** is heteroaryl, and the 6-membered ring including **B^16**, **B^17**, **B^19**, **B^20**, and **B^21** is aryl or heteroaryl;
**B^16**, **B^17**, and **B^14** are independently C or N;
**B^15** and **B^18** are independently selected from the group consisting of: O, S, N, N(H), N(**R^d**), CH, **CR^c2**, and C-(**L^b**)$_b$-**R^b**.
**B^19**, **B^20**, and **B^21** are independently N, CH, **CR^c2**, or C-(**L^b**)$_b$-**R^b**, provided that:

from 1-4 of **B^14**, **B^15**, **B^16**, **B^17**, **B^18**, **B^19**, **B^20**, and **B^21** is an independently selected heteroatom; and no more than 3 of **B^15**, **B^18**, **B^19**, **B^20**, and **B^21** are $CR^{c2}$ or C-(**L^b**)$_b$-**R^b**; and

**Ar^1** is selected from the group consisting of:

phenyl substituted with from 1-4 substituents each independently selected from the group consisting of: halo;

$C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**; and

**R$^1$** is $C_{1-6}$ alkyl optionally substituted with from 1-6 **R$^a$**.

In certain embodiments of Formula **(I-3)** or **(I-3a), B$^{16}$** and **B$^{17}$** are **C.**

In certain embodiments of Formula **(I-3)** or **(I-3a), B$^{19}$**, **B$^{20}$**, and **B$^{21}$** are independently CH or **CR$^{c2}$**.

In certain embodiments of Formula **(I-3)** or **(I-3a), B$^{19}$**, **B$^{20}$**, and **B$^{21}$** are independently CH, **CR$^{c2}$**, or C-**(L$^b$)$_b$-R$^b$.**

In certain embodiments of Formula **(I-3)** or **(I-3a), B$^{14}$** is C; one of **B$^{15}$** and **B$^{18}$** is N; and the other of **B$^{15}$** and **B$^{18}$** is O, S, NH, or N(**R$^d$**), such as NH or N(**R$^d$**), such as NH or N($C_{1-3}$ alkyl).

As non-limiting examples of the foregoing embodiments, the ring including **B$^{14}$-B$^{21}$** can be

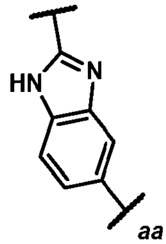 or 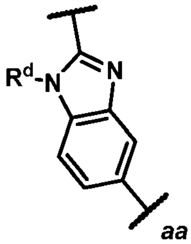,

each of which is optionally substituted with from 1-2 **R$^{c2}$**, wherein ***aa*** is the point of attachment to -(**CR$^{3a}$R$^{3b}$**)$_n$ C(O)OH.

As non-limiting examples of the foregoing embodiments, the ring including **B$^{14}$ - B$^{21}$** can be

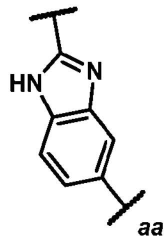 or 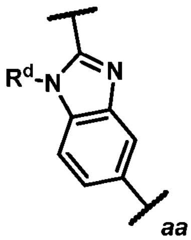,

each of which is optionally substituted with from 1-2 **R$^{c2}$**, wherein ***aa*** is the point of attachment to -(**CR $^{3a}$R $^{3b}$**)$_n$-**R$^2$**.

In certain embodiments of Formula **(I-1), (I-1a), (I-2), (I-2a)(I-3)**, or **(I-3a)**, **n** is 1, and optionally wherein **R$^{3a}$** and **R$^{3b}$** are H.

In certain embodiments of Formula **(I-1), (I-1a), (I-2), (I-2a), (I-3)**, or **(I-3a), n** is 1, and one of **R$^{3a}$** and **R$^{3b}$** is H, and the other one of **R$^{3a}$** and **R$^{3b}$** is $C_{1-6}$ alkyl, such as ethyl or methyl.

In certain embodiments of Formula **(I-1), (I-1a), (I-2), (I-2a), (I-3)**, or **(I-3a), n** is 1, and **R$^{3a}$** and **R$^{3b}$** together with the carbon atom to which each is attached forms a $C_{3-6}$ cycloalkyl, such as a cyclopropyl.

In certain embodiments of Formula **(I-1), (I-1a), (I-2), (I-2a), (I-3)**, or **(I-3a)**, **n** is 0.

In certain embodiments of Formula **(I-1), (I-1a), (I-2), (I-2a), (I-3)**, or **(I-3a)**, **Ar$^1$** is

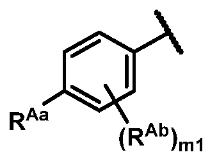;

**m1** is 0, 1, 2, or 3; and each occurrence of **R$^{Aa}$** and **R$^{Ab}$** are independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$**.

In certain embodiments of Formula **(I-1), (I-1a), (I-2), (I-2a), (I-3)**, or **(I-3a)**, **Ar$^1$** is

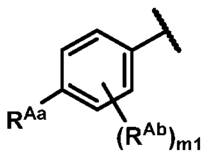 ;

**m1** is 0, 1, 2, or 3; and each occurrence of $\mathbf{R^{Aa}}$ and $\mathbf{R^{Ab}}$ are independently selected from the group consisting of: halo; -C(=O)($C_{1-10}$ alkyl); $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

In certain of these embodiments, **m1** is 0.

In certain embodiments, **m1** is 1 or 2, optionally wherein each $\mathbf{R^{Ab}}$ is *ortho* to $\mathbf{R^{Aa}}$.

In certain embodiments, each $\mathbf{R^{Ab}}$ when present is $C_{1-4}$ alkoxy or $C_{1-4}$ haloalkoxy, such as $C_{1-4}$ alkoxy, such as methoxy.

In certain embodiments, $\mathbf{R^{Aa}}$ is $C_{1-3}$ alkyl; $C_{1-3}$ alkyl substituted with 1-6 F; halo; or $C_{3-6}$ cycloalkyl.

As a non-limiting example of the foregoing embodiments, $\mathbf{Ar^1}$ can be

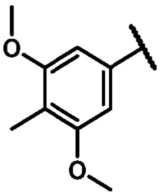 .

As further non-limiting examples, $\mathbf{Ar^1}$ can be selected from the group consisting of:

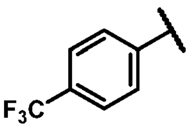 ; 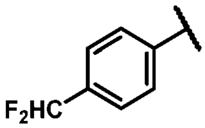 ; 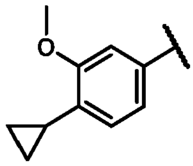 ; and 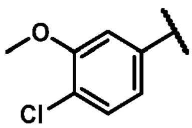 .

As further non-limiting examples, $\mathbf{Ar^1}$ can be selected from the group consisting of:

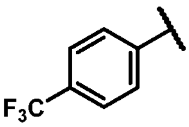 ; 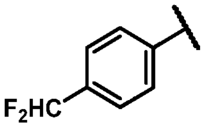 ; 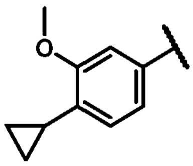 ; 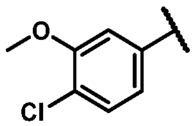 ; 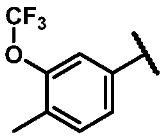 ;

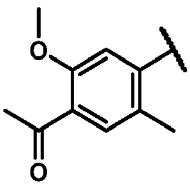 ; 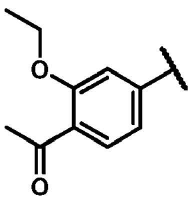 ; 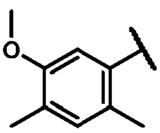 ; 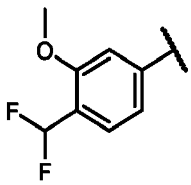 ; 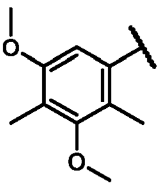 ;

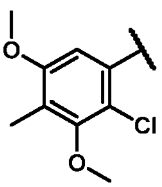 ; 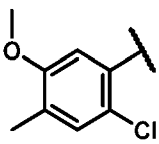 ; 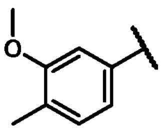 ; 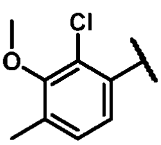 ; 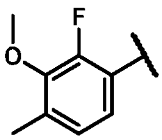 ; 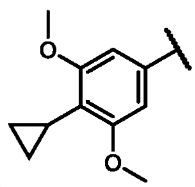 ;

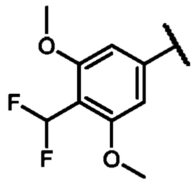 ; 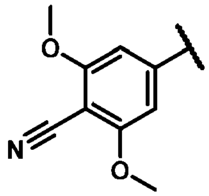 ; 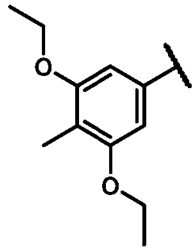 ; 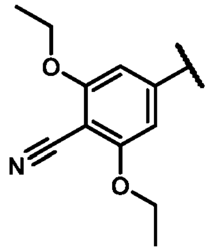 ; 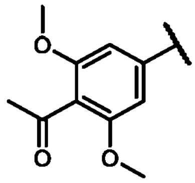 ;

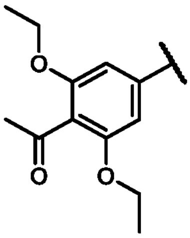 ; 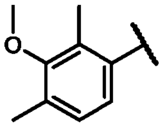 ; and 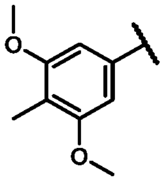 .

In certain embodiments of Formula **(I-1), (I-1a), (I-2), (I-2a), (I-3)**, or (I-3a), **$L^1$** is a bond.

In certain embodiments of Formula **(I-1), (I-1a), (I-2), (I-2a), (I-3)**, or **(I-3a)**, **$L^1$** is $C_{1-3}$ alkylene optionally substituted with from 1-6 **$R^a$**. For example, **$L^1$** can be $CH_2$. As another non-limiting example, **$L^1$** can be $CH_2CH_2$.

In certain embodiments of Formula **(I-1), (I-1a), (I-2), (I-2a), (I-3)**, or **(I-3a)**, **$R^1$** is phenyl optionally substituted with from 1-2 **$R^g$**.

In certain embodiments of Formula **(I-1), (I-1a), (I-2), (I-2a), (I-3)**, or **(I-3a)**, **$R^1$** is $C_{9-10}$ bicyclic aryl optionally substituted with from 1-2 **$R^g$**. As a non-limiting example of the foregoing embodiments, **$R^1$** can be

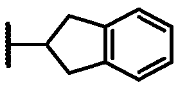 ,

which is optionally substituted with from 1-2 **$R^g$**.

In certain embodiments of Formula **(I-1), (I-1a), (I-2), (I-2a), (I-3)**, or **(I-3a)**, **$R^1$** is $C_{3-6}$ cycloalkyl which is optionally substituted with from 1-2 **$R^g$**. As non-limiting examples of the foregoing embodiments, **$R^1$** can be cyclobutyl or cyclopentyl, each of which is optionally substituted with from 1-2 **$R^g$**.

In certain embodiments of Formula **(I-1), (I-1a), (I-2), (I-2a), (I-3)**, or **(I-3a)**, **$R^1$** is $C_{1-6}$ alkyl optionally substituted with from 1-6 **$R^a$**. In certain embodiments Formula **(I-1), (I-1a), (I-2), (I-2a), (I-3)**, or **(I-3a), $R^1$** is $C_{1-6}$ alkyl.

In certain embodiments Formula **(I-1), (I-1a), (I-2), (I-2a), (I-3)**, or **(I-3a), $R^1$** is $C_{2-4}$ alkyl. As non-limiting examples of the foregoing embodiments, **$R^1$** is $C_3$ alkyl, such as *n*-propyl and *i*-propyl.

In certain embodiments Formula **(I-1), (I-1a), (I-2), (I-2a), (I-3)**, or **(I-3a), $R^1$** is $C_{3-5}$ alkyl. In certain embodiments of the foregoing embodiments, **$R^1$** is $C_4$ alkyl, such as *n*-butyl, *i*-butyl, *sec*-butyl, and *tert*-butyl. As a non-limiting example of the foregoing embodiments, **$R^1$** can be *i*-butyl.

In certain embodiments Formula **(I-1), (I-1a), (I-2), (I-2a), (I-3)**, or **(I-3a), $L^1$** is a bond.

In certain embodiments of Formula **(I-1), (I-1a), (I-2), (I-2a), (I-3)**, or **(I-3a),** the

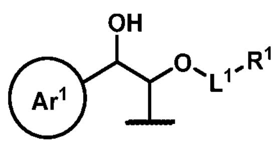 is 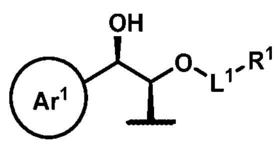 .

***Non-Limiting Exemplary Compounds***

In certain embodiments, the compound is selected from the group consisting of the compounds delineated in **Table C1,** or a pharmaceutically acceptable salt thereof.

Table C1

| Compound | Structure |
|---|---|
| 101 | 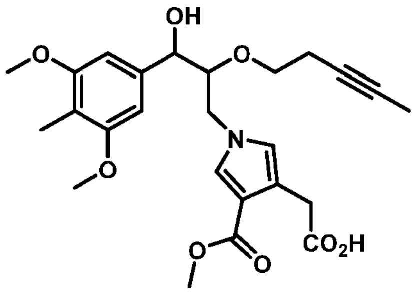 |
| 102 | 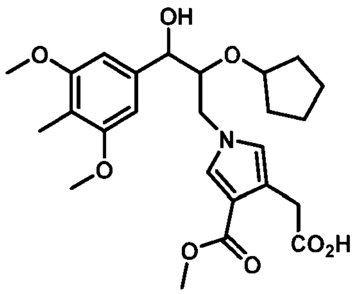 |
| 103 | 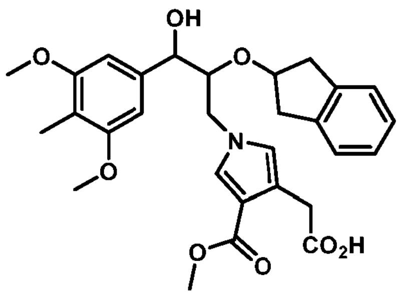 |
| 104 | 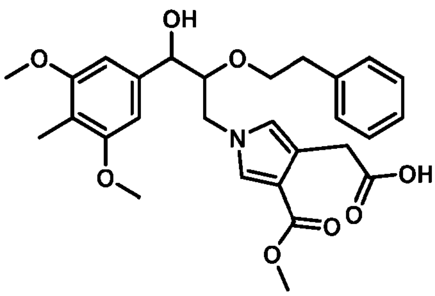 |
| 105 | 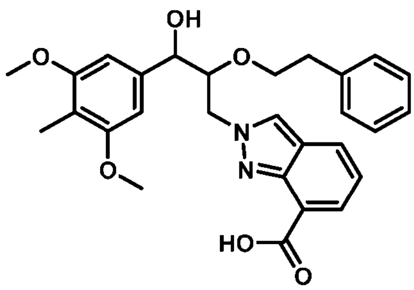 |

(continued)

| Compound | Structure |
|---|---|
| **106** | 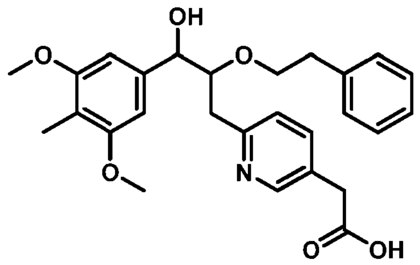 |
| **107** | 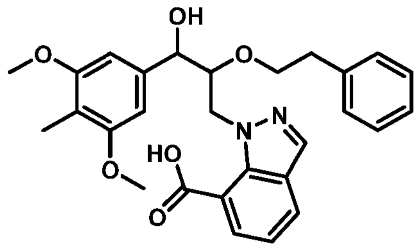 |
| **108** | 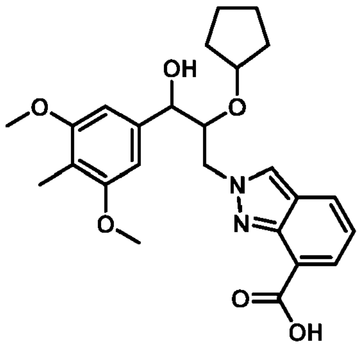 |
| **109** | 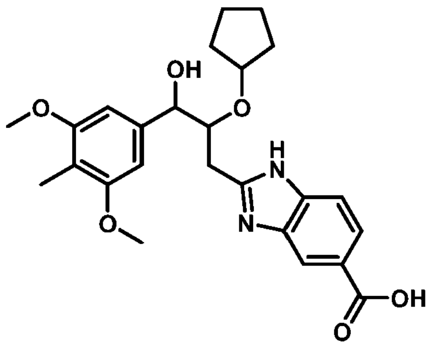 |
| **110** | 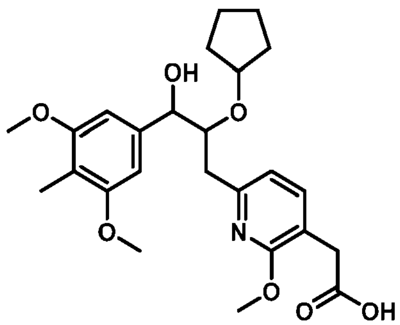 |

(continued)

| Compound | Structure |
|---|---|
| 111 | 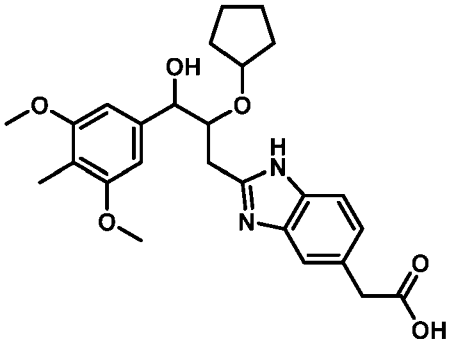 |
| 112 | 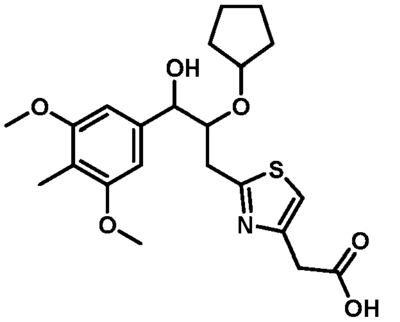 |
| 113 | 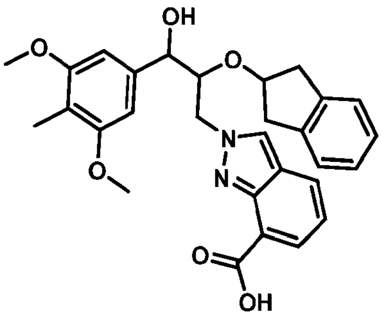 |
| 114 | 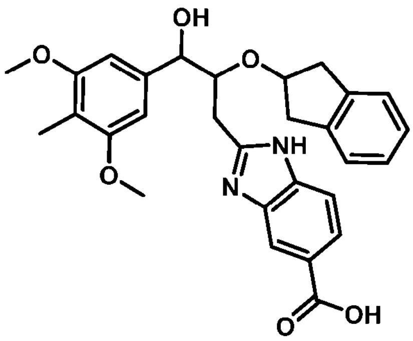 |

(continued)

| Compound | Structure |
|---|---|
| **115** | 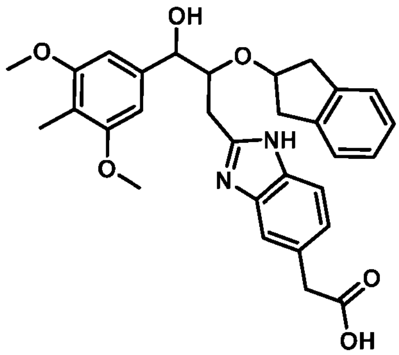 |
| **116** | 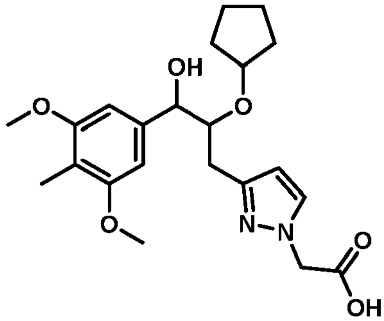 |
| **117** | 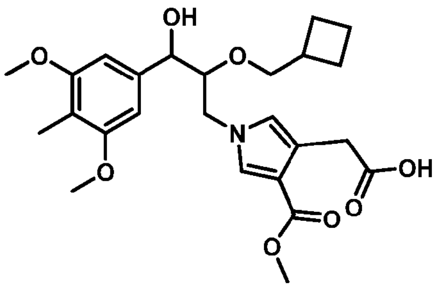 |
| **118** | 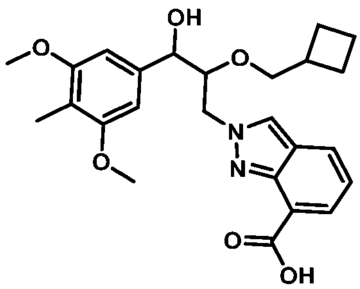 |
| **119** | 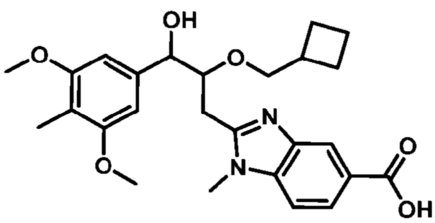 |

(continued)

| Compound | Structure |
|---|---|
| **120** | 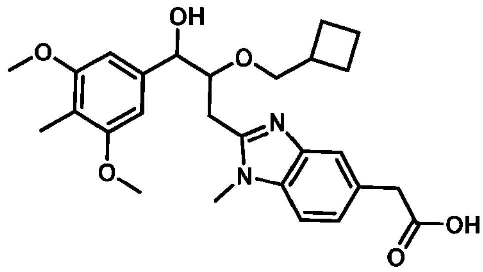 |
| **121** | 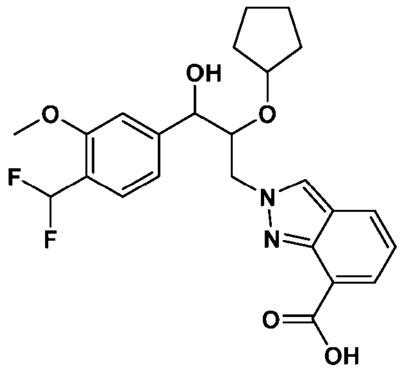 |
| **122** | 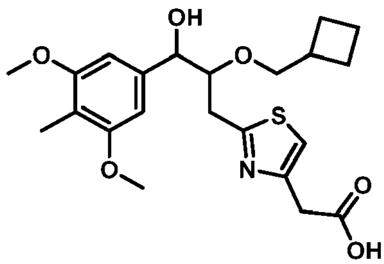 |
| **123** | 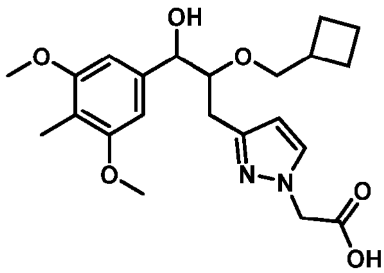 |
| **124** | 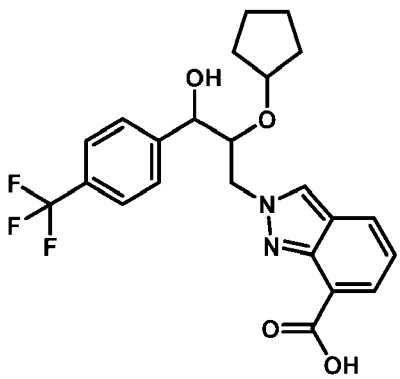 |

(continued)

| Compound | Structure |
| --- | --- |
| **125** | 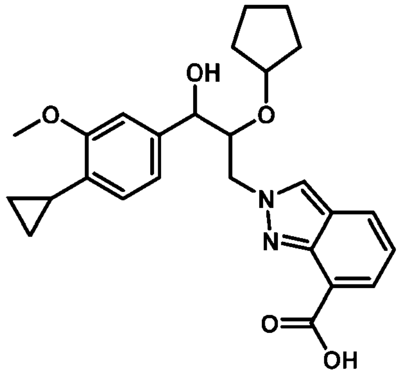 |
| **126** | 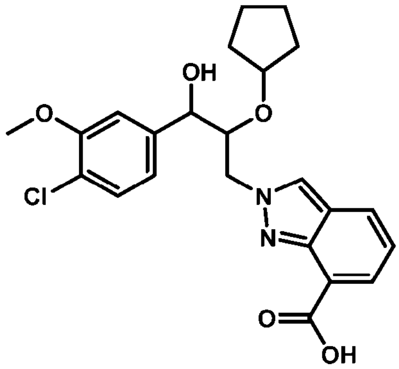 |
| **127** | 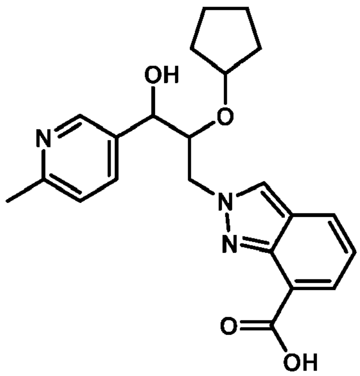 |
| **128** | 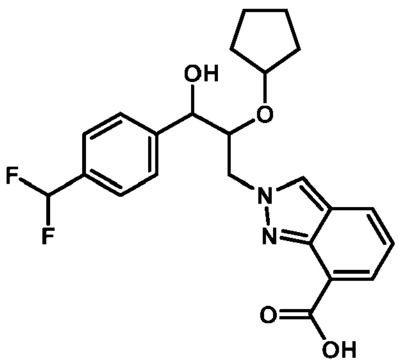 |

(continued)

| Compound | Structure |
|---|---|
| 129 | 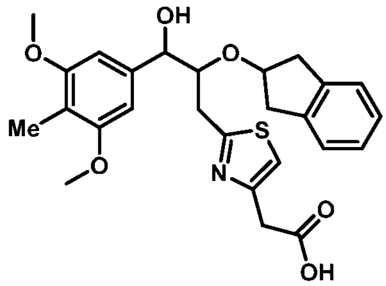 |
| 130 | 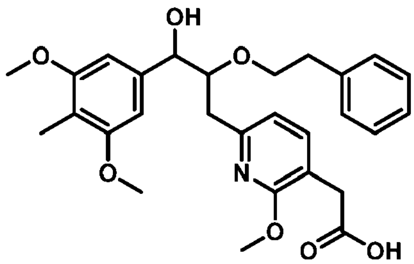 |
| 131 | 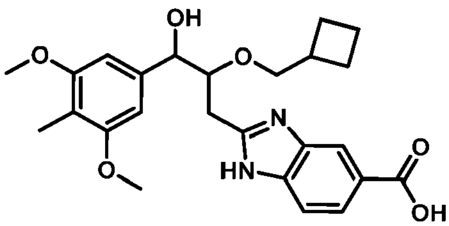 |

In certain embodiments, the compound is selected from the group consisting of the compounds delineated in **Table C2,** or a pharmaceutically acceptable salt thereof.

**Table C2**

| Compound | Structure |
|---|---|
| 101a | 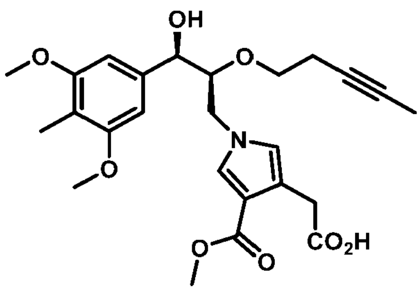 |
| 102a | 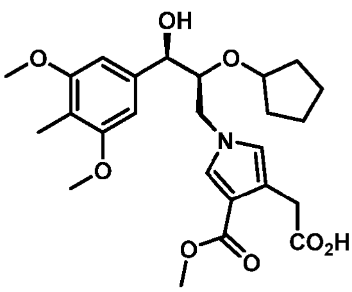 |

(continued)

| Compound | Structure |
|---|---|
| 103a | 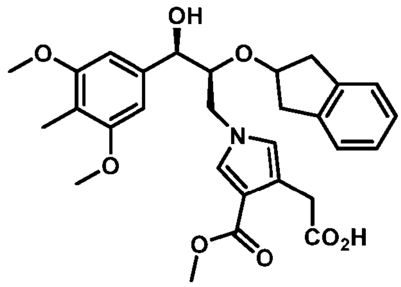 |
| 104a | 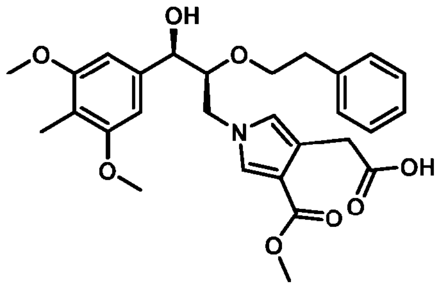 |
| 105a | 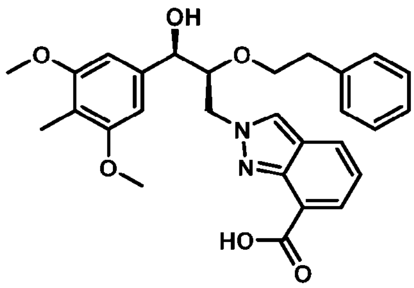 |
| 105b | 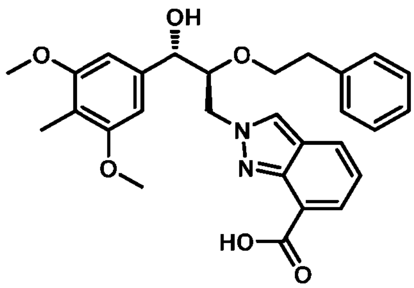 |
| 106a | 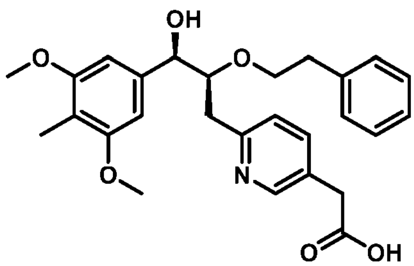 |

(continued)

| Compound | Structure |
|---|---|
| 107 | 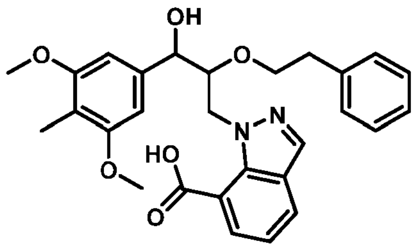 |
| 108a | 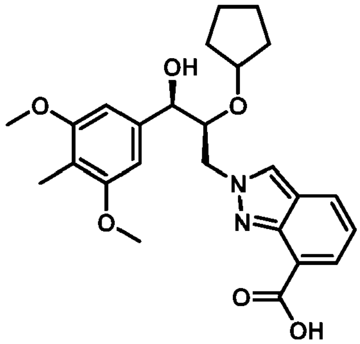 |
| 109a | 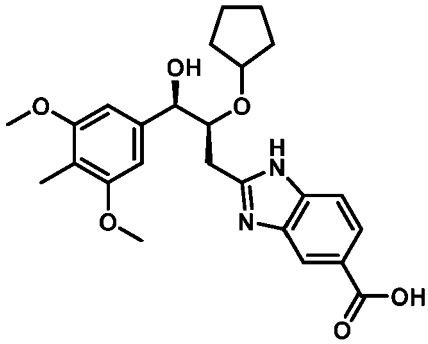 |
| 110a | 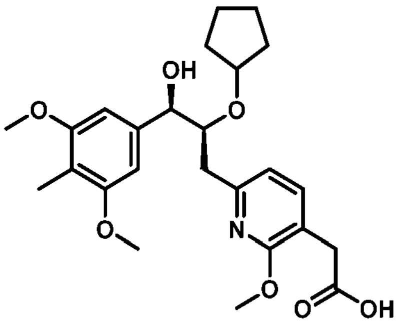 |
| 111a | 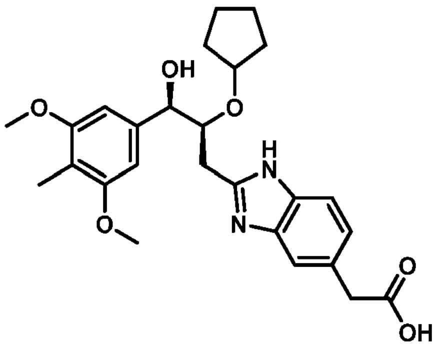 |

(continued)

| Compound | Structure |
|---|---|
| 112a | 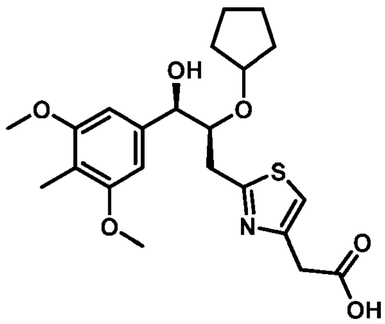 |
| 113a | 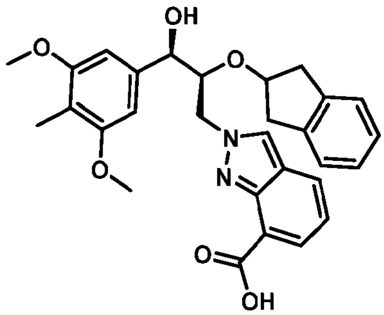 |
| 114a | 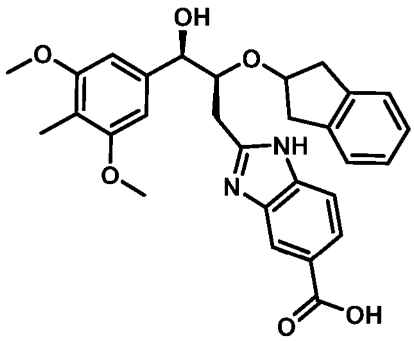 |
| 115a | 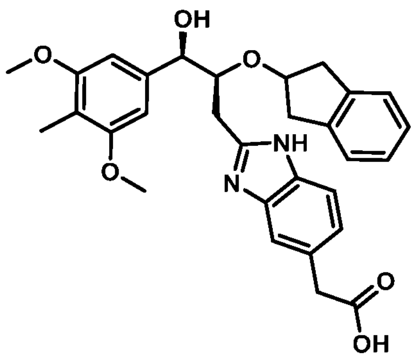 |

(continued)

| Compound | Structure |
|---|---|
| **116a** | 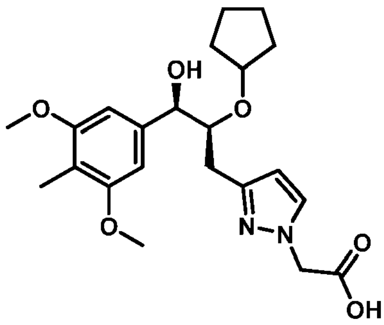 |
| **117a** | 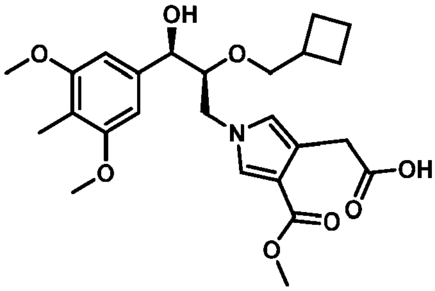 |
| **118a** | 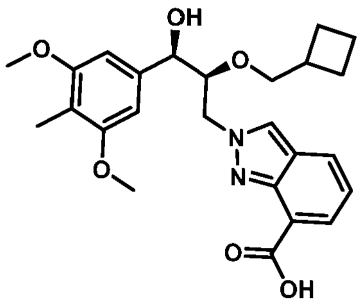 |
| **119a** | 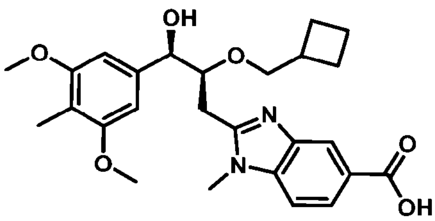 |
| **120a** | 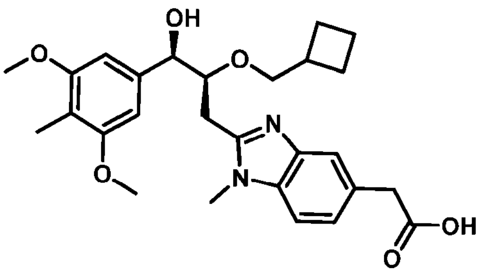 |

(continued)

| Compound | Structure |
|---|---|
| **121a** | 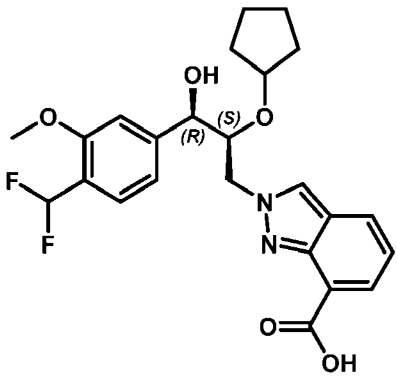 |
| **122a** | 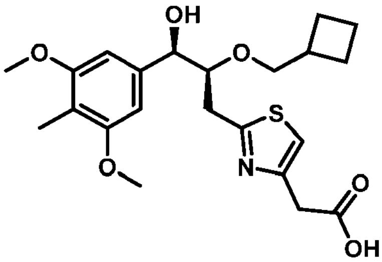 |
| **123a** | 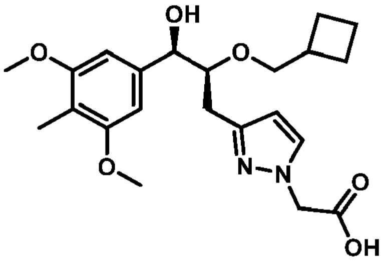 |
| **124a** | 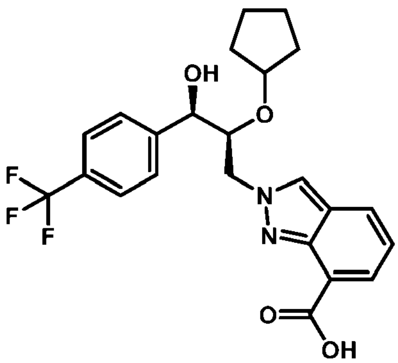 |
| **125a** | 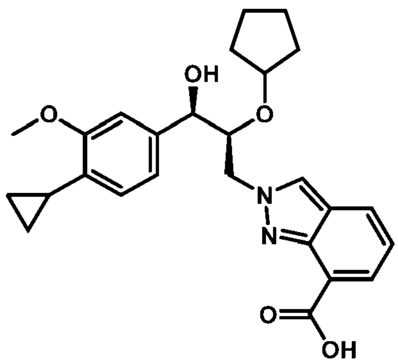 |

(continued)

| Compound | Structure |
|---|---|
| 126a | 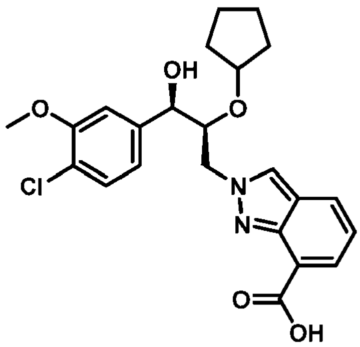 |
| 127a | 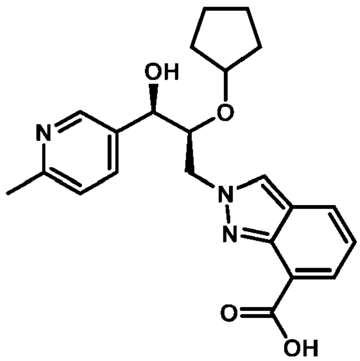 |
| 128a | 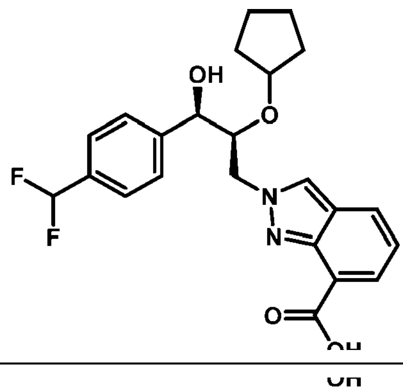 |
| 129a | 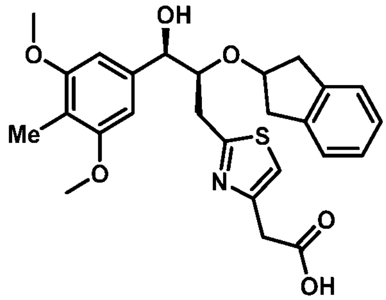 |
| 130a | 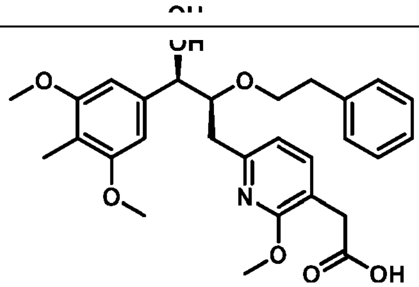 |

(continued)

| Compound | Structure |
|---|---|
| 131a | 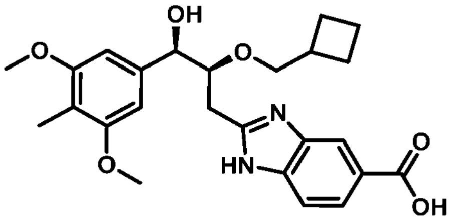 |

| Compound | Structure | LC-MS: m/z |
|---|---|---|
| 132 | 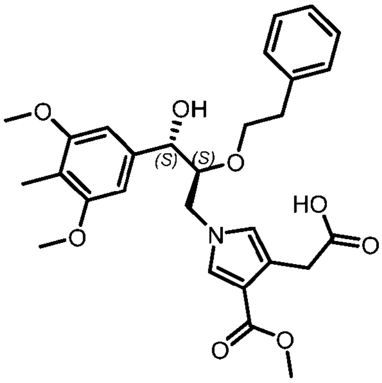 | 534.1 (M+Na)+. |
| 133 | 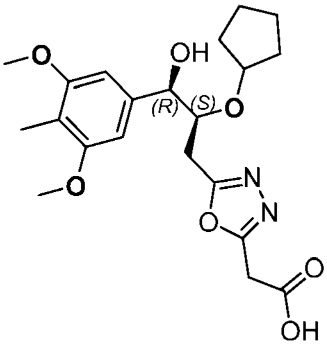 | 424.1 (M+H)+ |
| 134 | 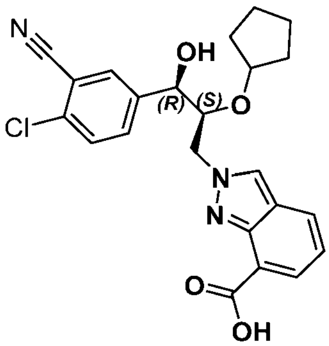 | 440.0 (M+H)+ |

(continued)

| Compound | Structure | LC-MS: m/z |
|---|---|---|
| **135** | 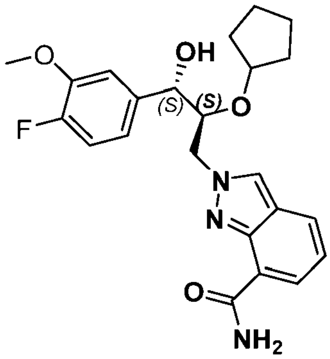 | **428.2 (M+H)+** |
| **136** | 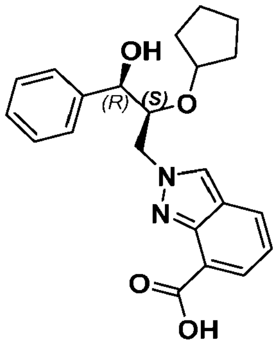 | **381.1 (M+H)+** |
| **137** | 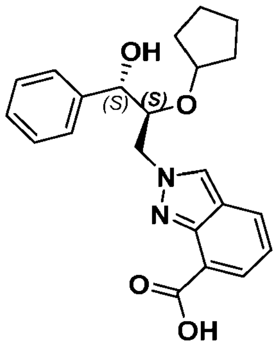 | **381.2 (M+H)+** |
| **138** | 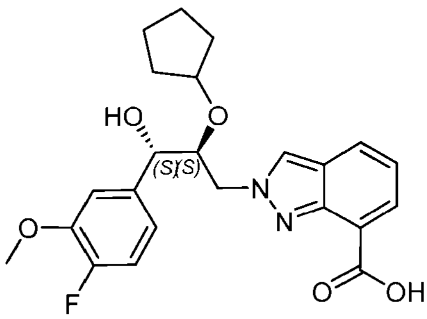 | **429.2 (M+H)+** |

(continued)

| Compound | Structure | LC-MS: m/z |
|---|---|---|
| **139** | 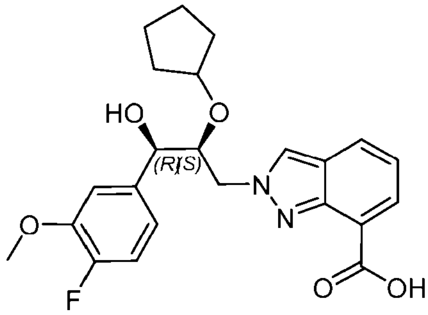 | **429.2 (M+H)+** |
| **140** | 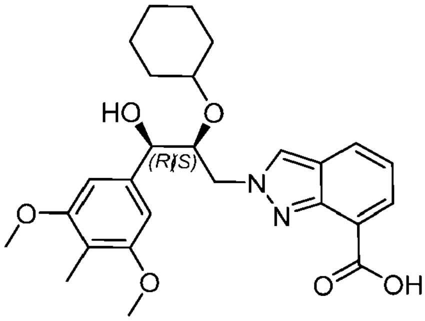 | **469.5 (M+H)+** |
| **141** | 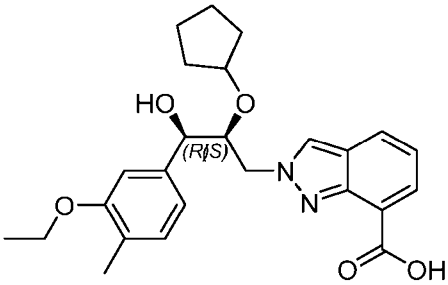 | **439.3 (M+H)+** |
| **142** | 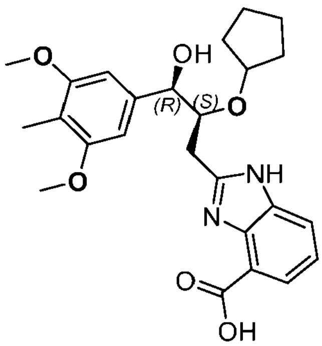 | **455.2 (M+H)+** |
| **143** | 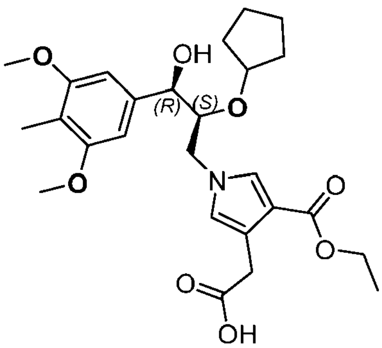 | **490.2 (M+H)+** |

(continued)

| Compound | Structure | LC-MS: m/z |
|---|---|---|
| **144** | 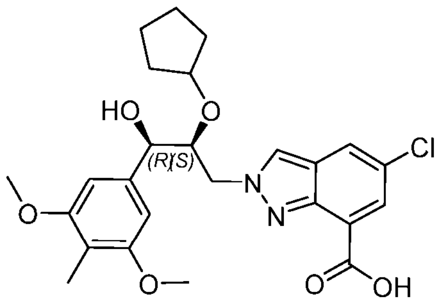 | **488.9 (M+H)+** |
| **145** | 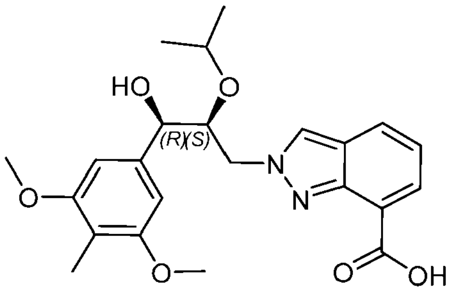 | **428.9 (M+H)+** |
| **146** | 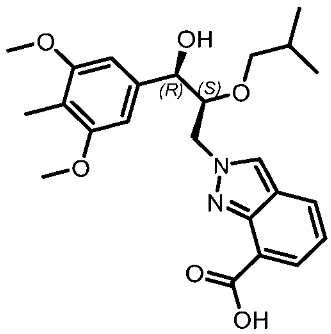 | **443.2 (M+H)+** |
| **147** | 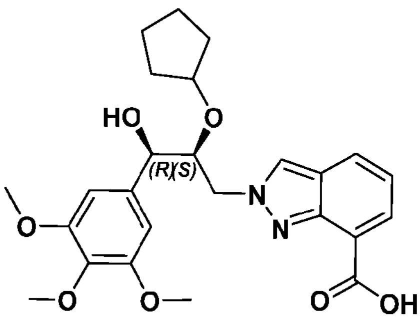 | **471.2 (M+H)+** |
| **148** | 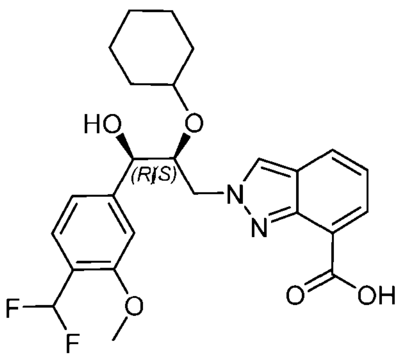 | **474.9 (M+H)+** |

(continued)

| Compound | Structure | LC-MS: m/z |
|---|---|---|
| 149 | 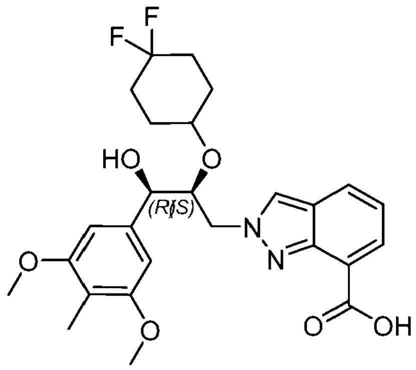 | 505.0 (M+H)+ |
| 150 | 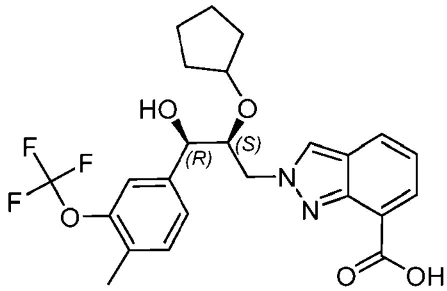 | 479.2 (M+H)+ |
| 151 | 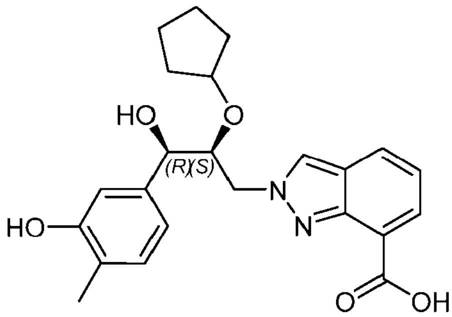 | 411.2 (M+H)+ |
| 152 | 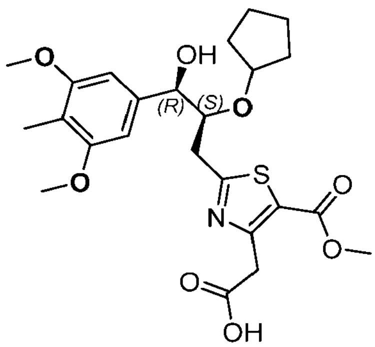 | 493.9 (M+H)+ |
| 153 | 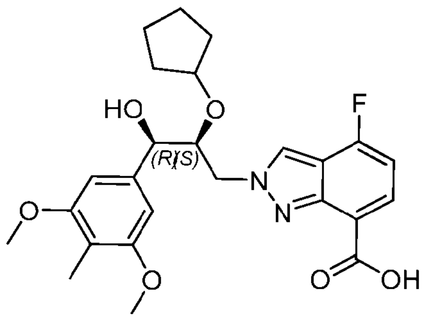 | 473.3 (M+H)+ |

(continued)

| Compound | Structure | LC-MS: m/z |
|---|---|---|
| **154** | 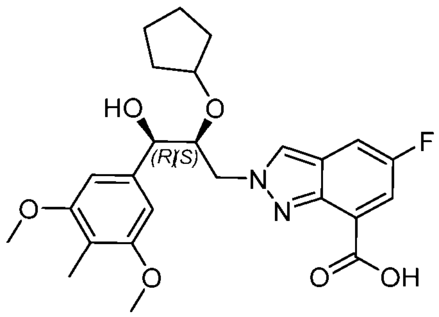 | **473.1 (M+H)+** |
| **155** | 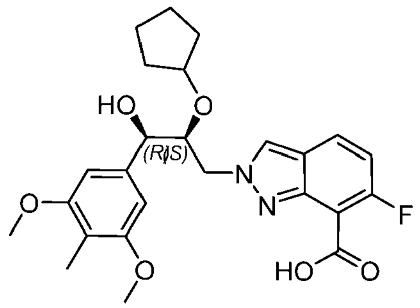 | **473.1 (M+H)+** |
| **156** | 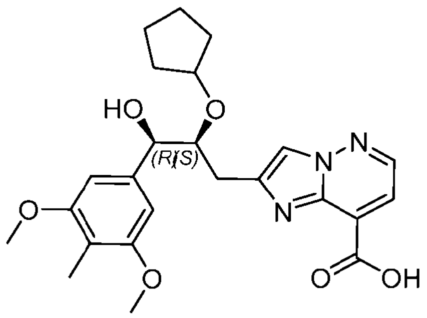 | **456.1 (M+H)+** |
| **157** | 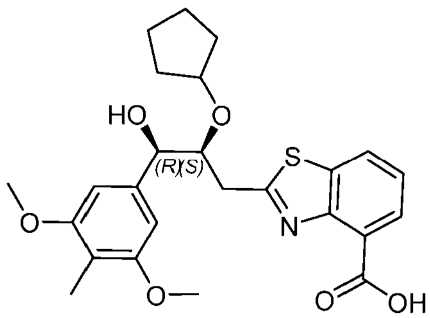 | **471.9 (M+H)+** |
| **158** | 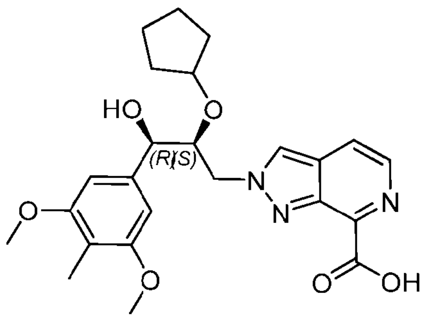 | **456.1 (M+H)+** |

(continued)

| Compound | Structure | LC-MS: m/z |
|---|---|---|
| **159** | 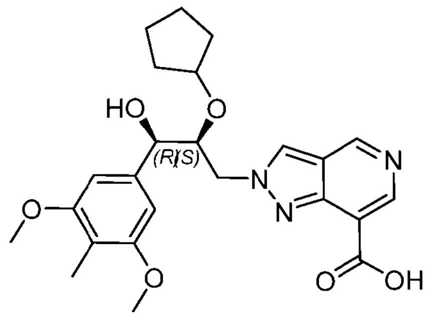 | **456.1 (M+H)+** |
| **160** | 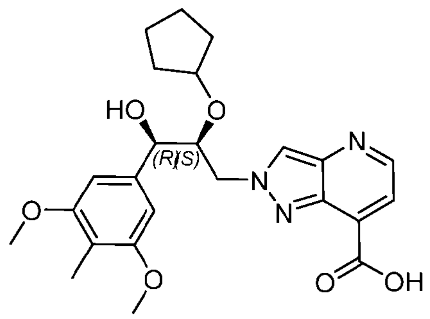 | **456.2 (M+H)+** |
| **161** | 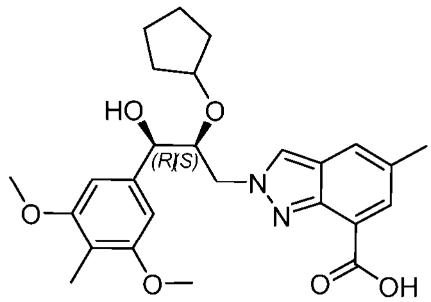 | **469.1 (M+H)+** |
| **162** | 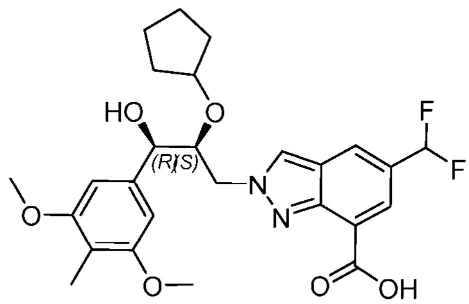 | **505.6 (M+H)+** |
| **163** | 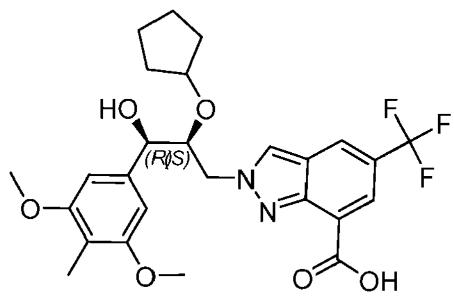 | **523.1 (M+H)+** |

(continued)

| Compound | Structure | LC-MS: m/z |
|---|---|---|
| 164 | 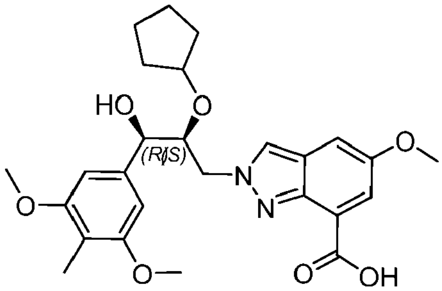 | 485.5 (M+H)+ |
| 165 | 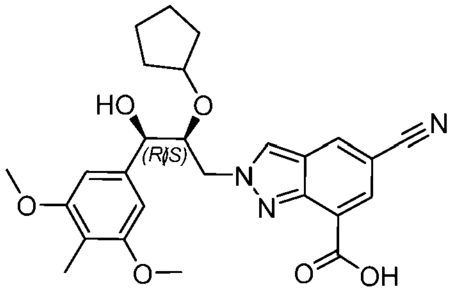 | 480.0 (M+H)+ |
| 166 | 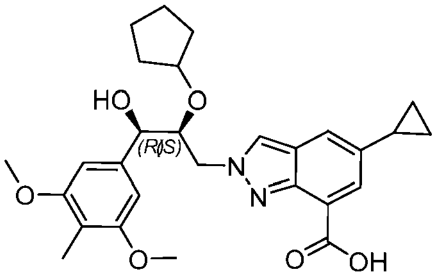 | 495.5 (M+H)+ |
| 167 | 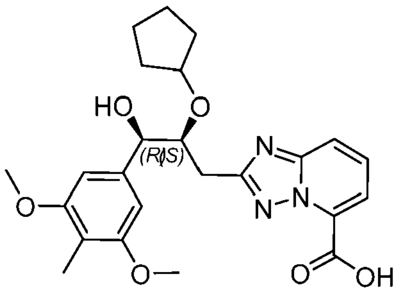 | 456.2 (M+H)+ |
| 168 | 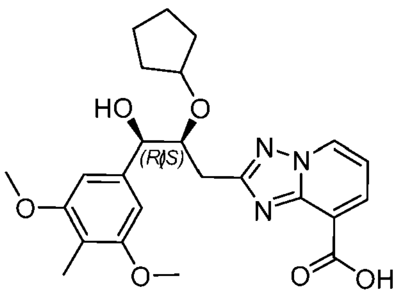 | 456.2 (M+H)+ |

(continued)

| Compound | Structure | LC-MS: m/z |
|---|---|---|
| 169 | 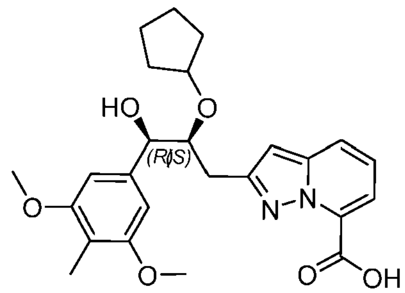 | 455.0 (M+H)+ |
| 170 | 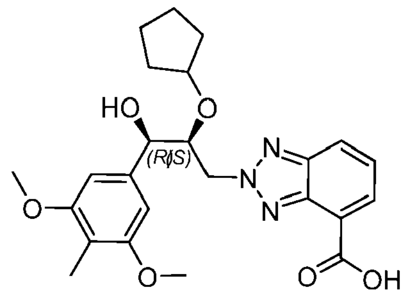 | 456.2 (M+H)+ |
| 171 | 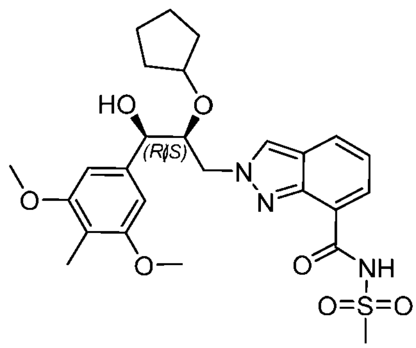 | 532.2 (M+H)+ |
| 172 | 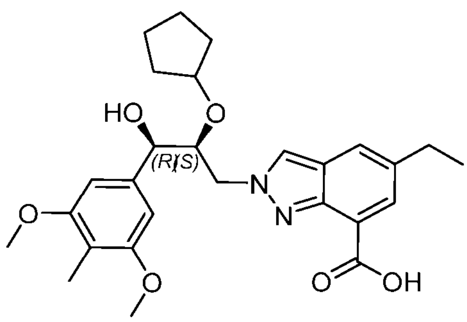 | 483.1 (M+H)+ |
| 173 | 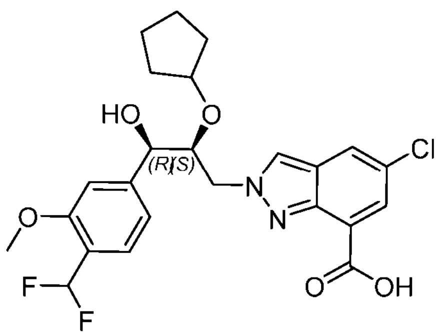 | 495.0 (M+H)+ |

(continued)

| Compound | Structure | LC-MS: m/z |
|---|---|---|
| 174 | 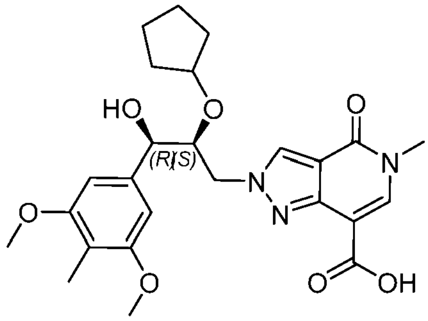 | 486.2 (M+H)+ |
| 175 | 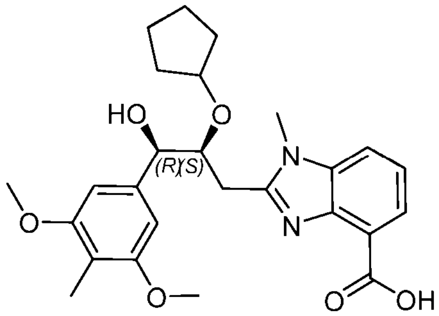 | 469.1 (M+H)+ |
| 176 | 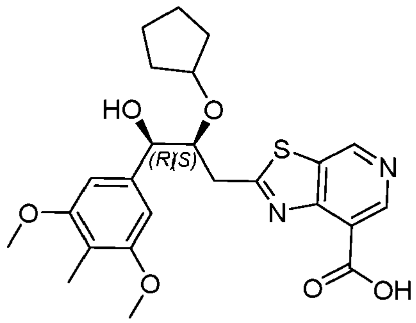 | 473.5 (M+H)+ |
| 177 | 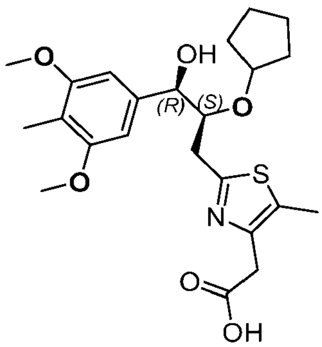 | 450.1 (M+H)+ |

(continued)

| Compound | Structure | LC-MS: m/z |
|---|---|---|
| **178** | 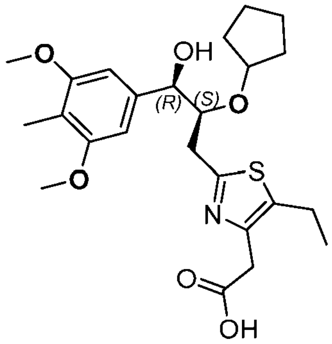 | **464.1 (M+H)+** |
| **179** | 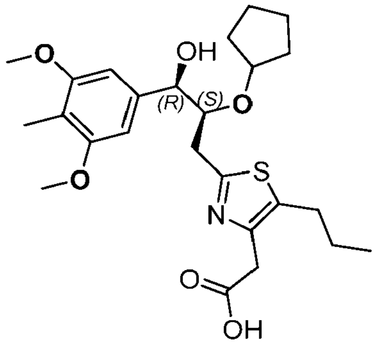 | **478.2 (M+H)+** |
| **180** | 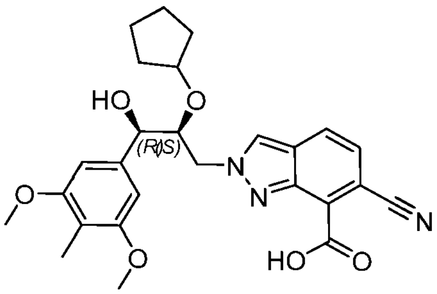 | **480.2 (M+H)+** |
| **181** | 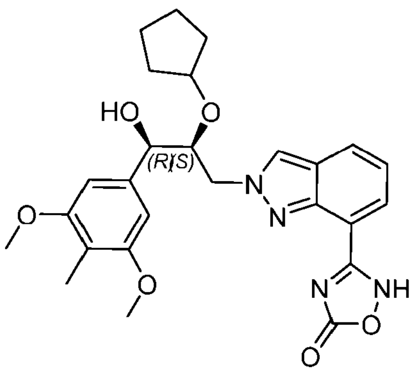 | **495.1 (M+H)+** |

(continued)

| Compound | Structure | LC-MS: m/z |
|---|---|---|
| 182 | 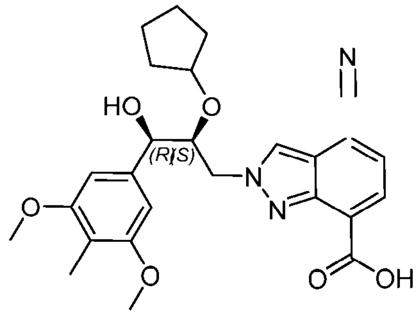 | 480.1 (M+H)+ |
| 183 | 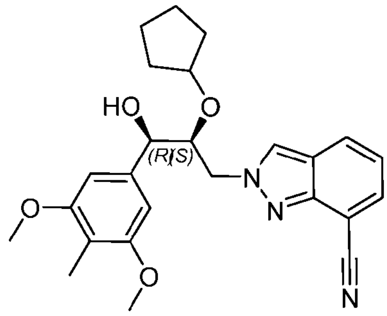 | 436.2 (M+H)+ |
| 184 | 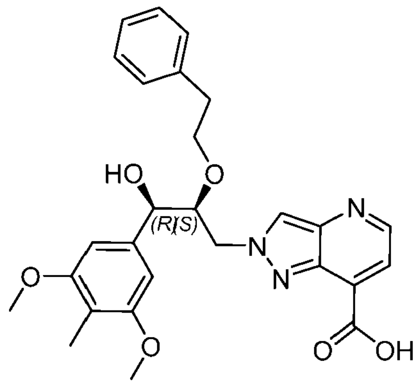 | 492.1 (M+H)+ |
| 185 | 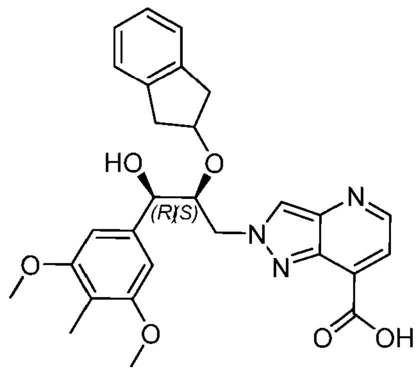 | 504.1 (M+H)+ |

(continued)

| Compound | Structure | LC-MS: m/z |
|---|---|---|
| 186 | 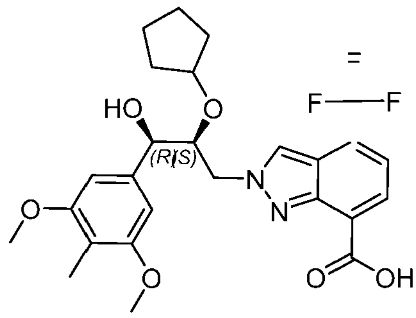 | 523.1 (M+H)+ |
| 187 | 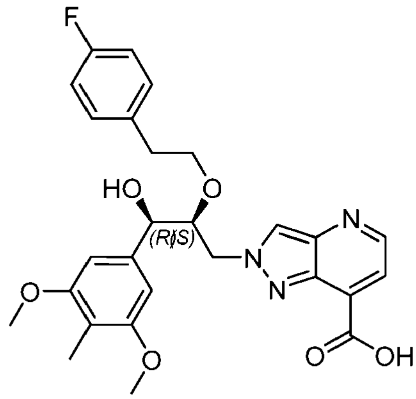 | 510.1 (M+H)+ |
| 188 | 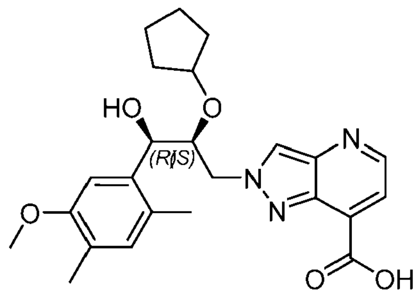 | 440.2 (M+H)+ |
| 189 | 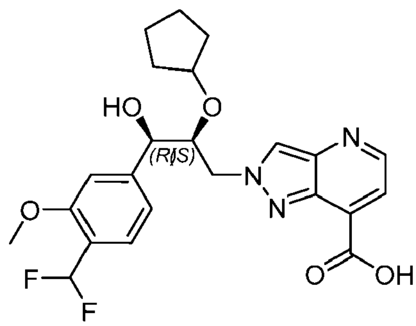 | 462.1 (M+H)+ |
| 190 | 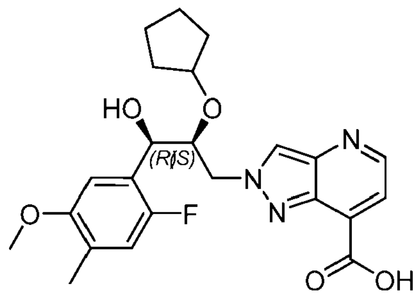 | 444.1 (M+H)+ |

(continued)

| Compound | Structure | LC-MS: m/z |
|---|---|---|
| 191 | 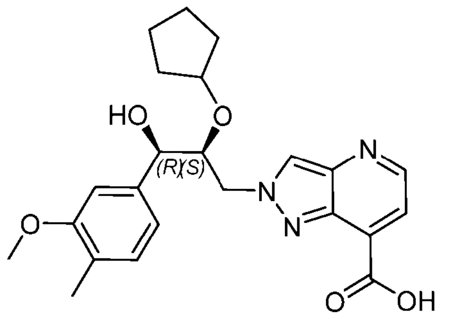 | 426.2 (M+H)+ |
| 192 | 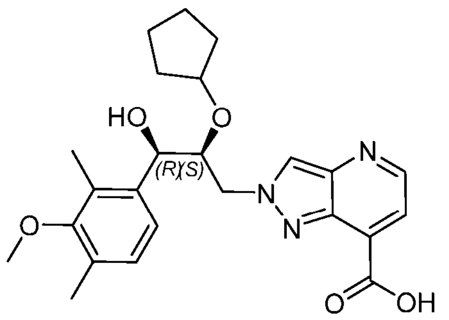 | 440.1 (M+H)+ |
| 193 | 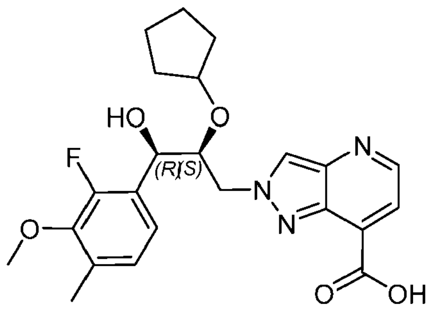 | 444.1 (M+H)+ |
| 194 | 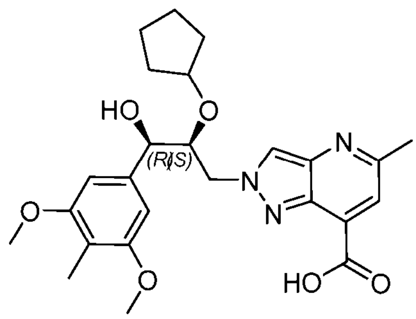 | 470.1 (M+H)+ |
| 195 | 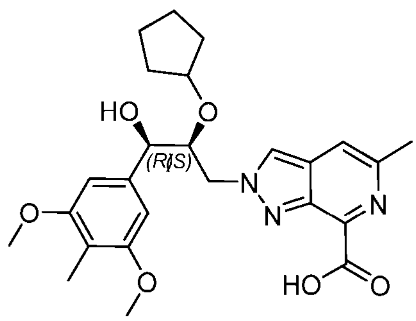 | 470.1 (M+H)+ |

(continued)

| Compound | Structure | LC-MS: m/z |
|---|---|---|
| 196 | 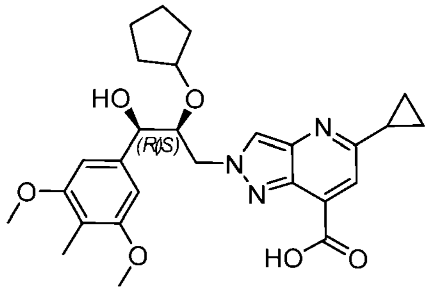 | 496.2 (M+H)+ |
| 197 | 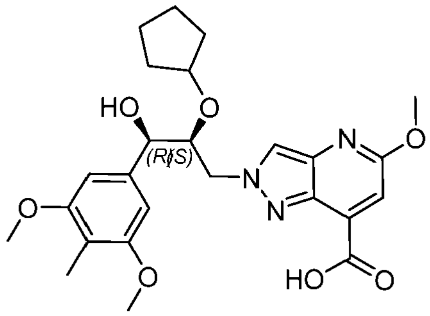 | 486.1 (M+H)+ |
| 198 | 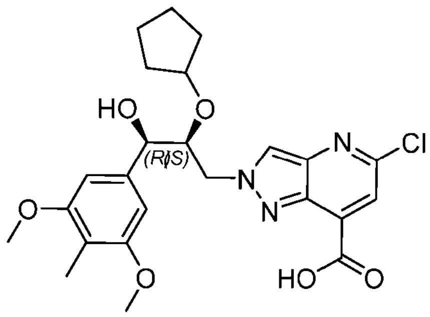 | 490.1 (M+H)+ |
| 199 | 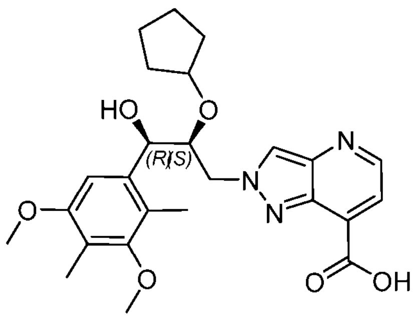 | 470.3 (M+H)+ |
| 200 | 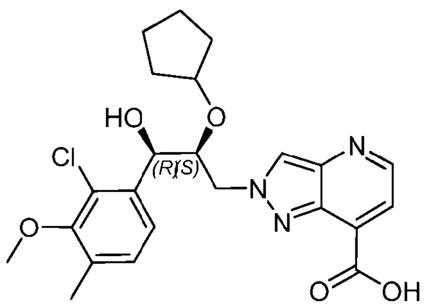 | 460.1 (M+H)+ |

(continued)

| Compound | Structure | LC-MS: m/z |
|---|---|---|
| 201 | 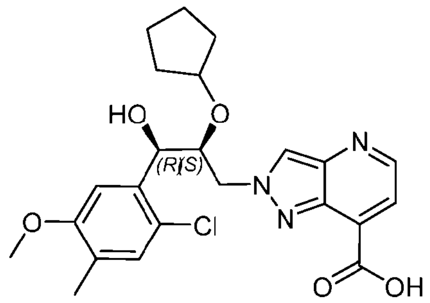 | 460.1 (M+H)+ |
| 202 | 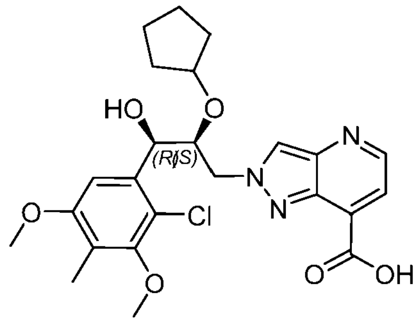 | 490.1 (M+H)+ |
| 203 | 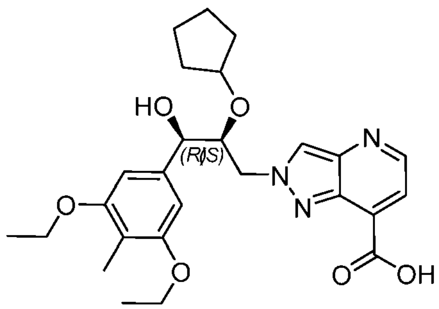 | 484.0 (M+H)+ |
| 204 | 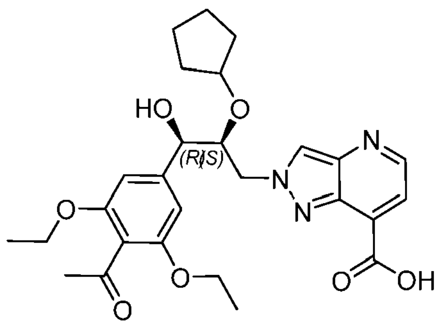 | 512.0 (M+H)+ |
| 205 | 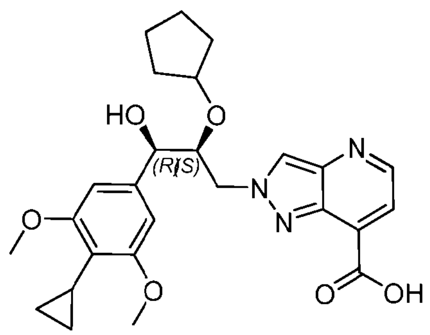 | 482.1 (M+H)+ |

(continued)

| Compound | Structure | LC-MS: m/z |
|---|---|---|
| 206 | 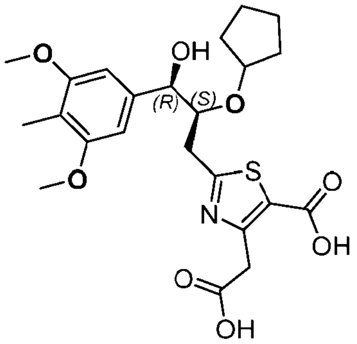 | 480.0 (M+H)+ |
| 207 | 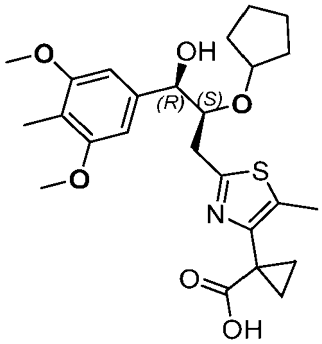 | 476.1 (M+H)+ |
| 208 | 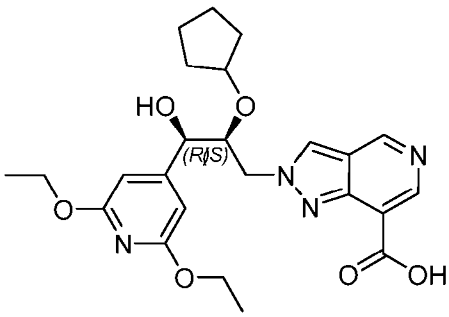 | 471.2 (M+H)+ |
| 209 | 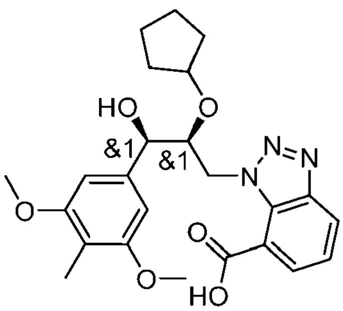 | 478.2 (M+Na)+ |

(continued)

| Compound | Structure | LC-MS: m/z |
|---|---|---|
| 210 | 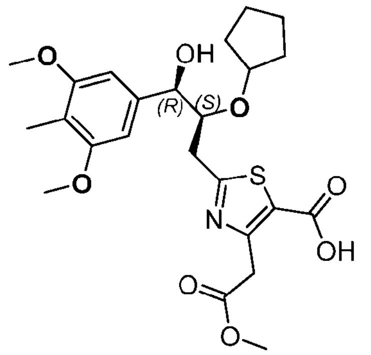 | 494.1 (M+H)+ |
| 211 | 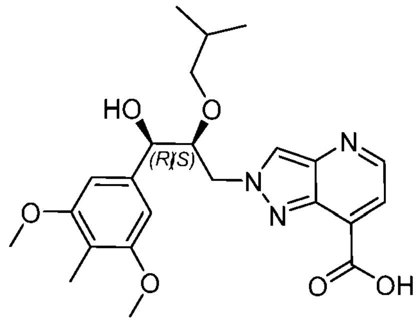 | 444.0 (M+H)+ |
| 212 | 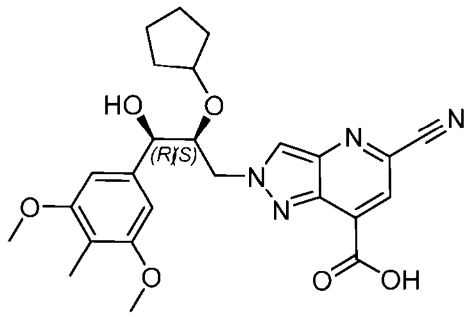 | 481.1 (M+H)+ |
| 213 | 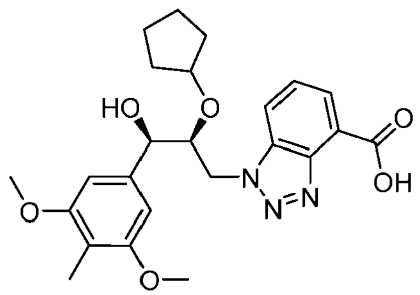 | 456.2 (M+H)+ |

(continued)

| Compound | Structure | LC-MS: m/z |
|---|---|---|
| 214 | 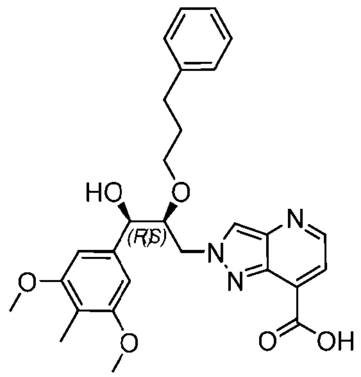 | 506.1 (M+H)+ |
| 215 | 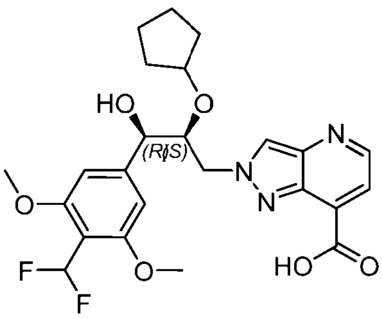 | 492.1 (M+H)+ |
| 216 | 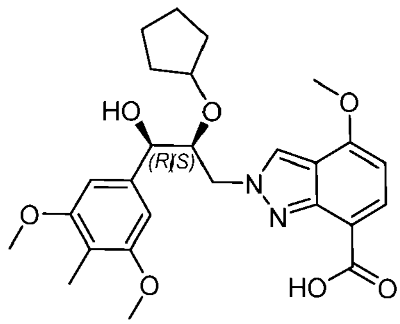 | 485.1 (M+H)+ |
| 217 | 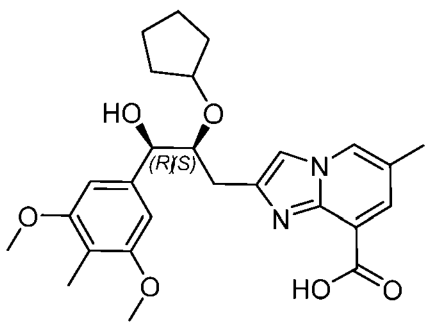 | 469.2 (M+H)+ |

(continued)

| Compound | Structure | LC-MS: m/z |
|---|---|---|
| 218 | 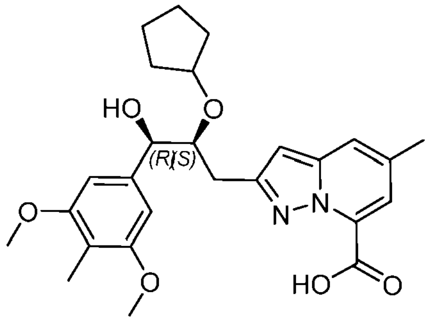 | 469.2 (M+H)+ |
| 219 | 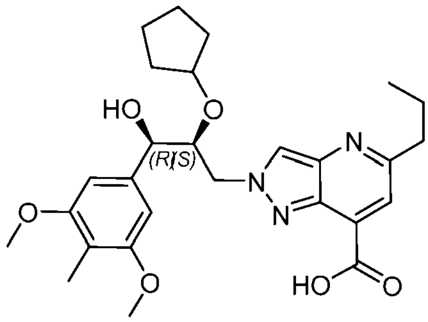 | 498.1 (M+H)+ |
| 220 | 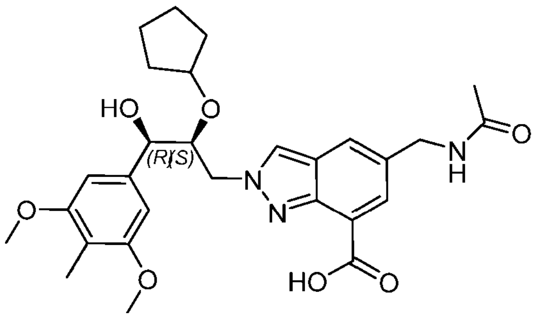 | 526.0 (M+H)+ |
| 221 | 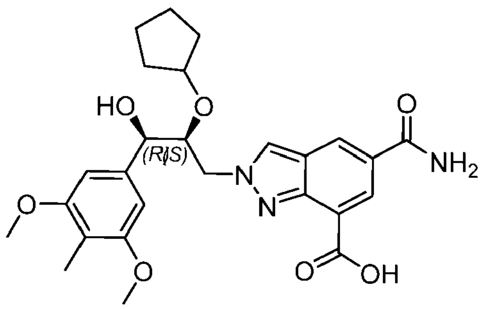 | 498.0 (M+H)+ |
| 222 | 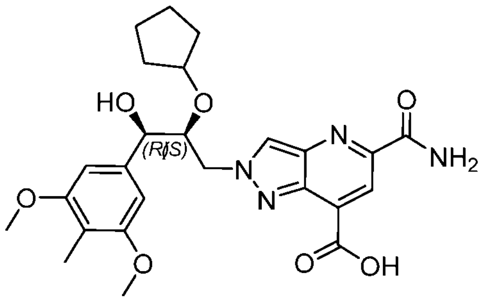 | 499.1 (M+H)+ |

(continued)

| Compound | Structure | LC-MS: m/z |
|---|---|---|
| 223 | 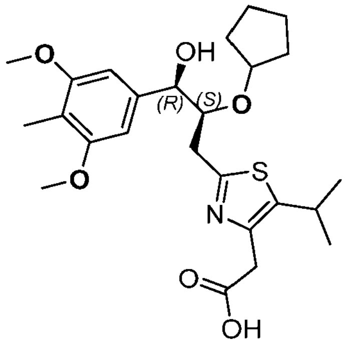 | 478.2 (M+H)+ |
| 224 | 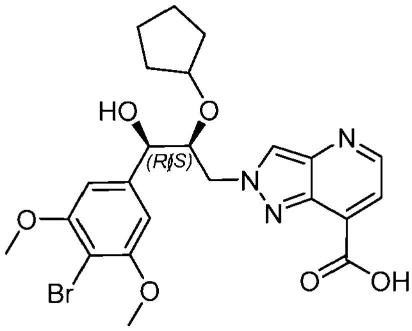 | 520.0 (M+H)+ |
| 225 | 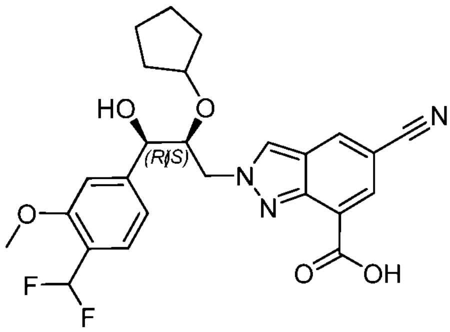 | 486.1 (M+H)+ |
| 226 | 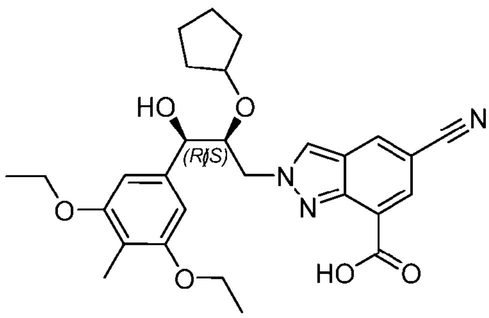 | 508.0 (M+H)+ |

(continued)

| Compound | Structure | LC-MS: m/z |
|---|---|---|
| **227** | 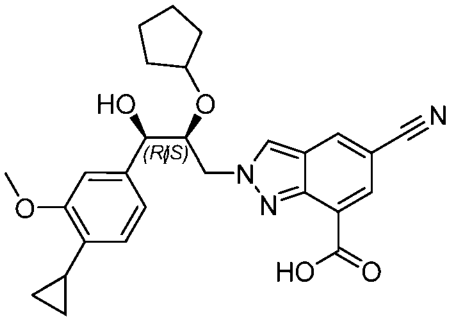 | **476.1 (M+H)+** |
| **228** | 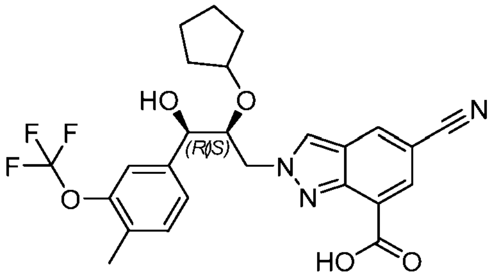 | **504.1 (M+H)+** |
| **229** | 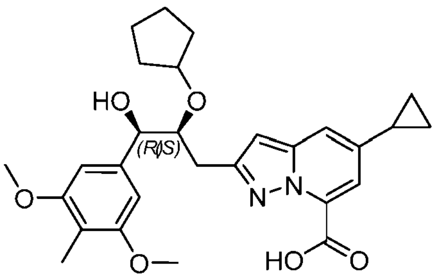 | **495.3 (M+H)+** |
| **230** | 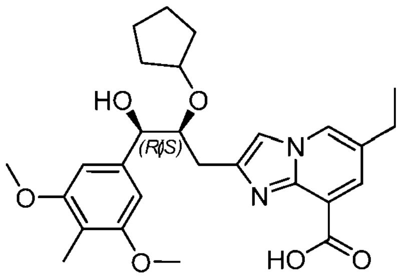 | **483.3 (M+H)+** |
| **231** | 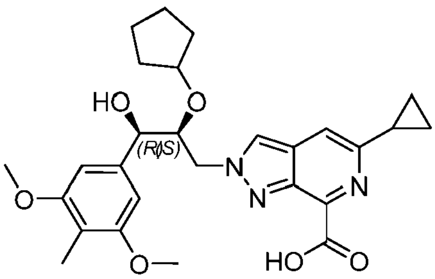 | **496.1 (M+H)+** |

(continued)

| Compound | Structure | LC-MS: m/z |
|---|---|---|
| **232** | 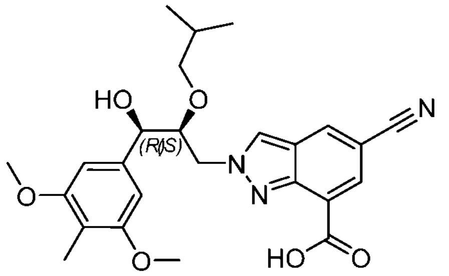 | **468.1 (M+H)+** |
| **233** | 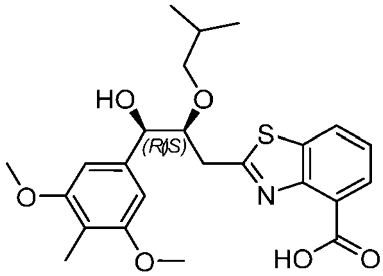 | **460.1 (M+H)+** |
| **234** | 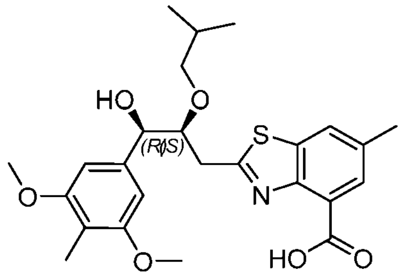 | **474.2 (M+H)+** |
| **235** | 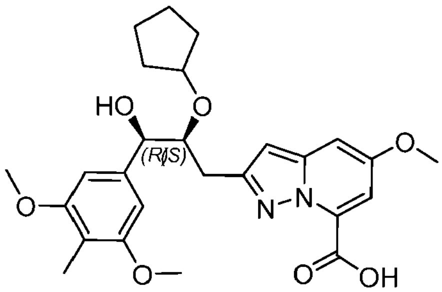 | **485.1 (M+H)+** |
| **236** | 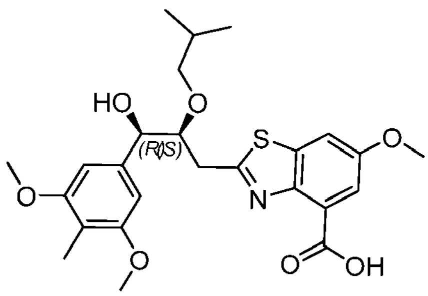 | **490.1 (M+H)+** |

(continued)

| Compound | Structure | LC-MS: m/z |
|---|---|---|
| 237 | 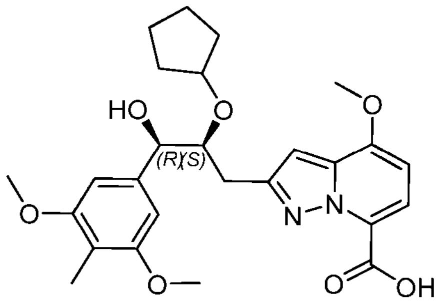 | 485.2 (M+H)+ |
| 238 | 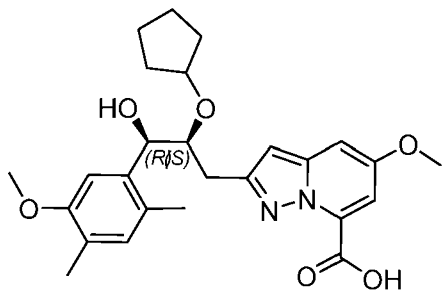 | 469.2 (M+H)+ |
| 239 | 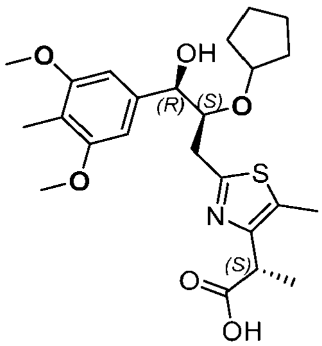 | 464.1 (M+H)+ |
| 240 | 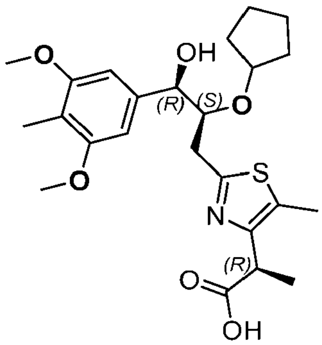 | 464.1 (M+H)+ |
| 241 | 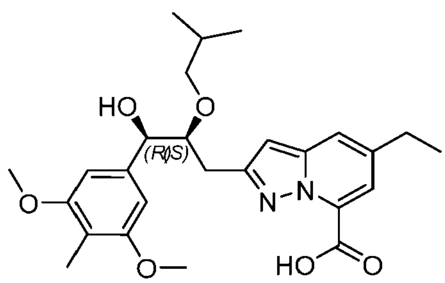 | 471.2 (M+H)+ |

(continued)

| Compound | Structure | LC-MS: m/z |
|---|---|---|
| 242 | 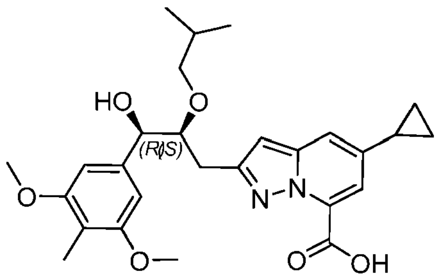 | 483.2 (M+H)+ |
| 243 | 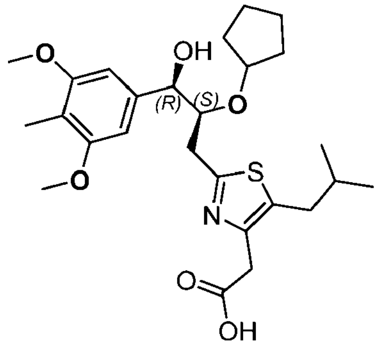 | 492.2 (M+H)+ |
| 244 | 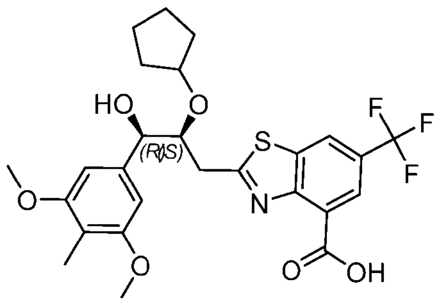 | 540.0 (M+H)+ |
| 245 | 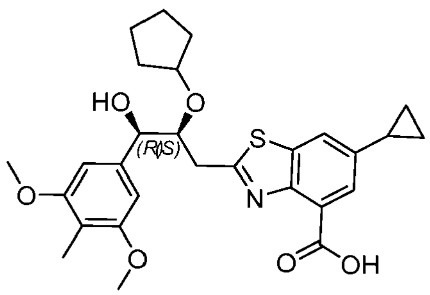 | 512.2 (M+H)+ |
| 246 | 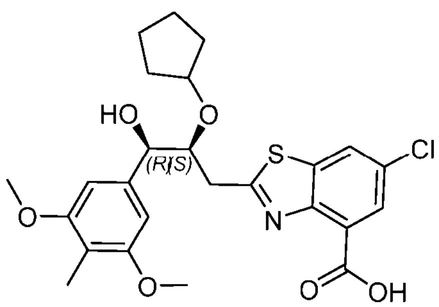 | 506.1 (M+H)+ |

(continued)

| Compound | Structure | LC-MS: m/z |
|---|---|---|
| **247** | 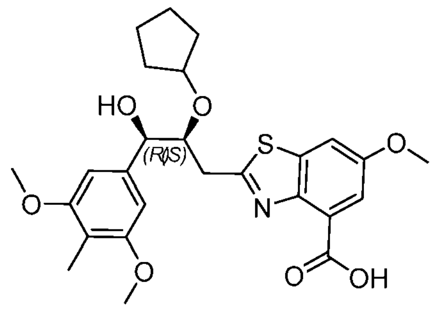 | **502.1 (M+H)+** |
| **248** | 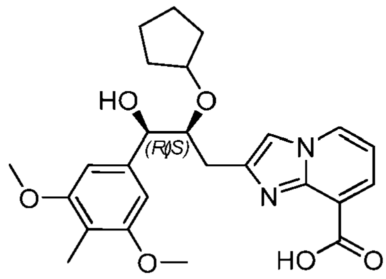 | **455.1 (M+H)+** |
| **249** | 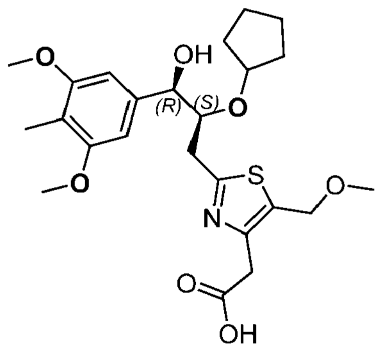 | **480.3 (M+H)+** |
| **250** | 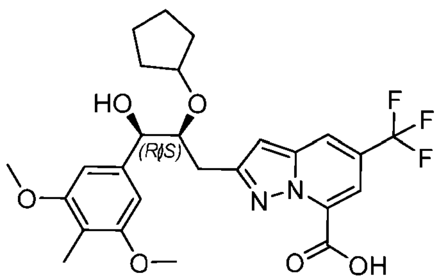 | **523.3 (M+H)+** |
| **251** | 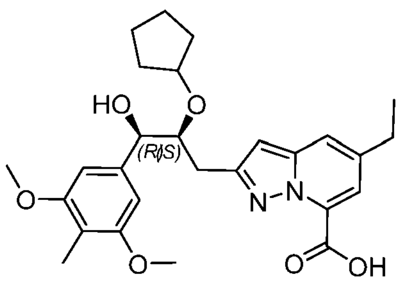 | **483.3 (M+H)+** |

(continued)

| Compound | Structure | LC-MS: m/z |
|---|---|---|
| **252** | 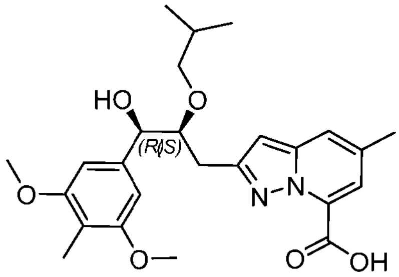 | **457.2 (M+H)+** |
| **253** | 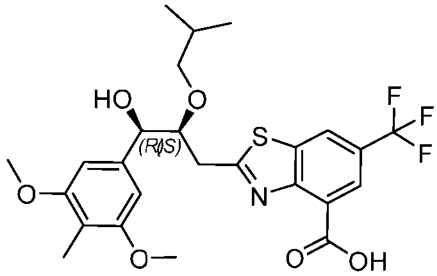 | **528.0 (M+H)+** |
| **254** | 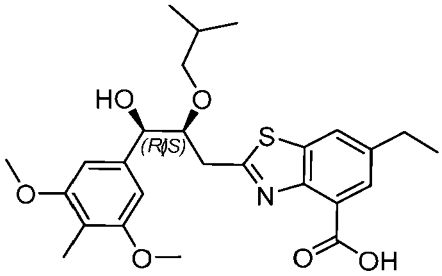 | **488.0 (M+H)+** |
| **255** | 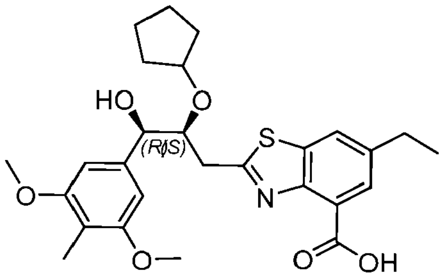 | **500.1 (M+H)+** |
| **256** | 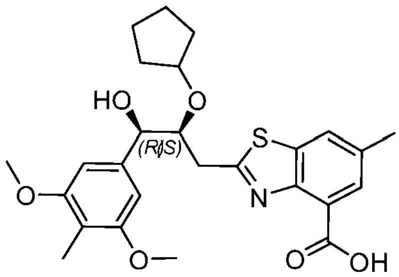 | **486.1 (M+H)+** |

(continued)

| Compound | Structure | LC-MS: m/z |
|---|---|---|
| 257 | 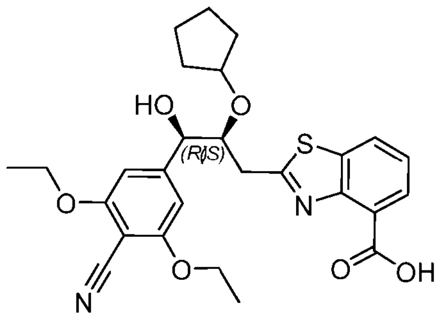 | 511.1 (M+H)+ |
| 258 | 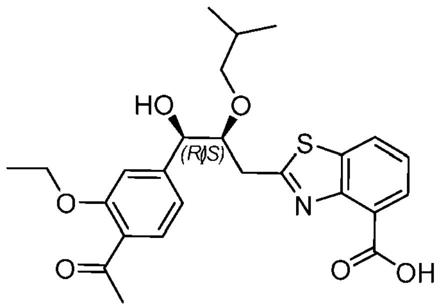 | 472.2 (M+H)+ |
| 259 | 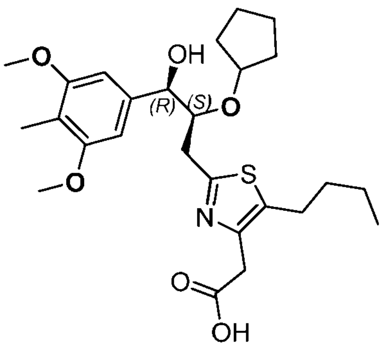 | 492.6 (M+H)+ |
| 260 | 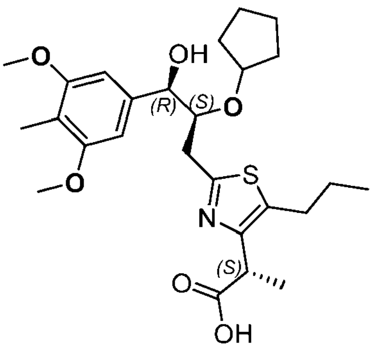 | 492.2 (M+H)+ |

(continued)

| Compound | Structure | LC-MS: m/z |
|---|---|---|
| 261 | 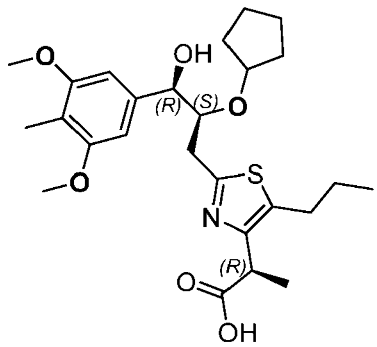 | 492.2 (M+H)+ |
| 262 | 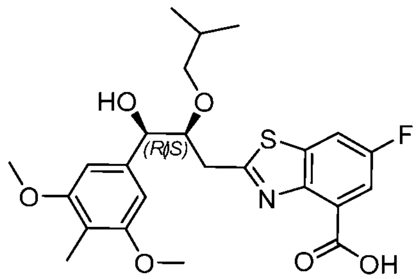 | 478.0 (M+H)+ |
| 263 | 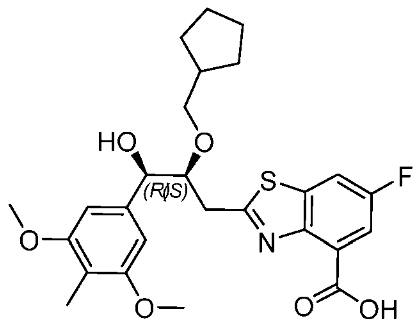 | 504.1 (M+H)+ |
| 264 | 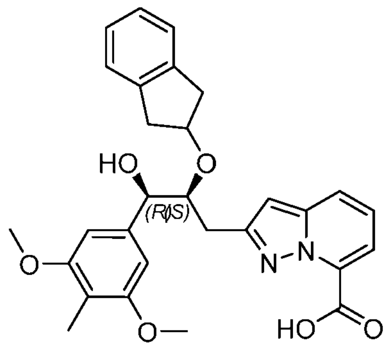 | 503.3 (M+H)+ |
| 265 | 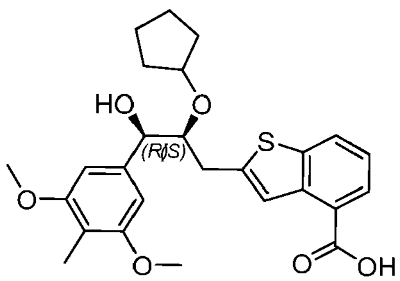 | 493.1 (M+Na)+ |

(continued)

| Compound | Structure | LC-MS: m/z |
|---|---|---|
| 266 | 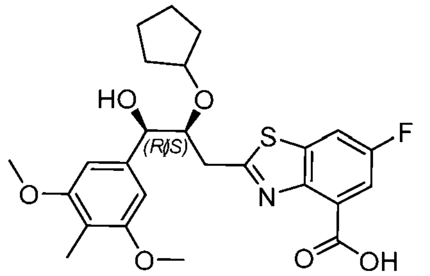 | 490.0 (M+H)+ |
| 267 | 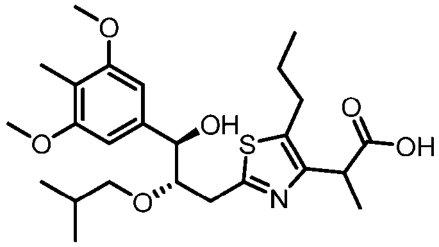 | 480.2 (M+H)+ |
| 268 | 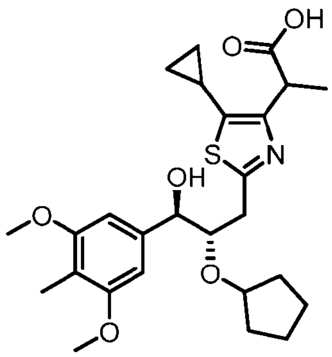 | 490.6 (M+H)+ |
| 269 | 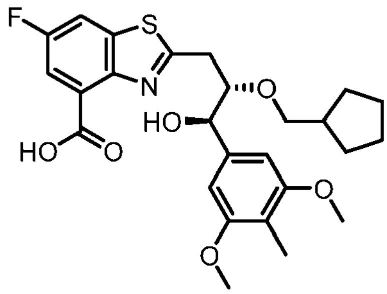 | |
| 270 | 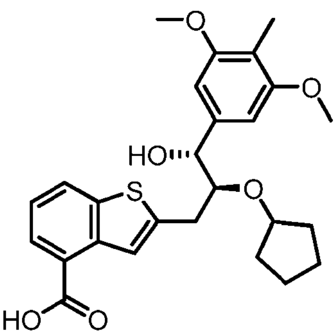 | |

(continued)

| Compound | Structure | LC-MS: m/z |
|---|---|---|
| **271** | 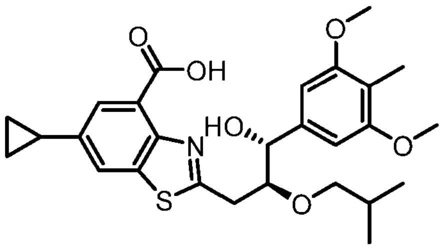 | **500.3 (M+H)+** |
| **272** | 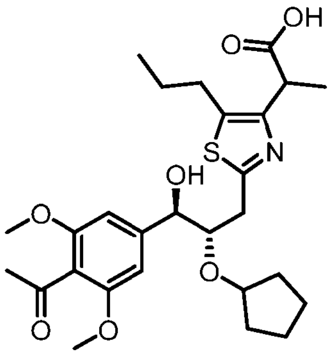 | **520.0 (M+H)+** |
| **273** | 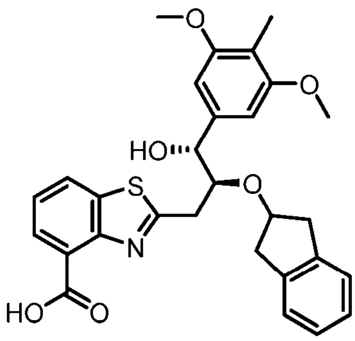 | **520.1 (M+H)+** |
| **274** | 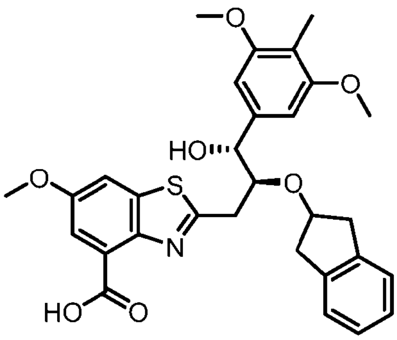 | **550.1 (M+H)+** |
| **275** | 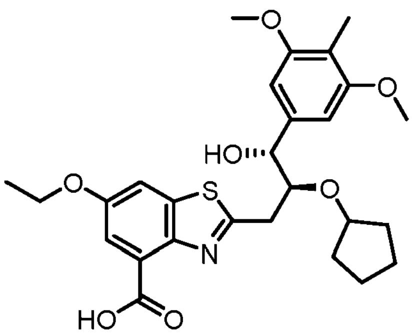 | **516.4 (M+H)+** |

(continued)

| Compound | Structure | LC-MS: m/z |
|---|---|---|
| 276 | 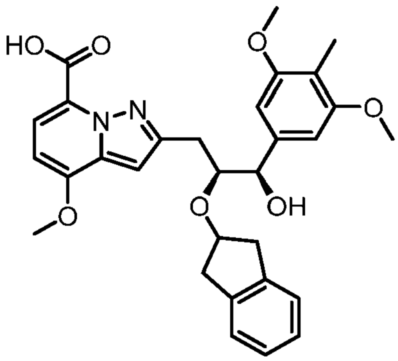 | 533.4 (M+H)+ |
| 277 | 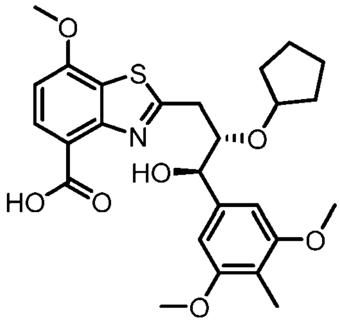 | 502.3 (M+H)+ |
| 278 | 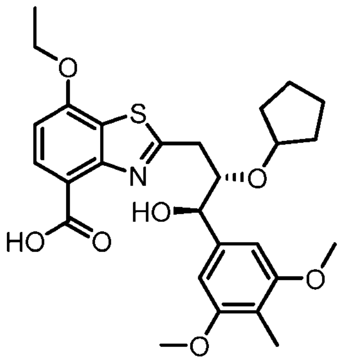 | |
| 279 | 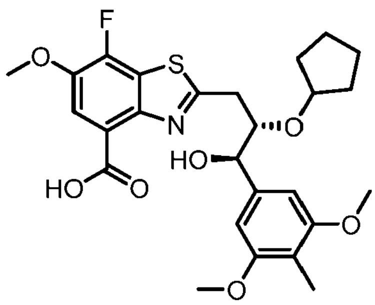 | 520.3 (M+H)+ |

(continued)

| Compound | Structure | LC-MS: m/z |
|---|---|---|
| 280 | 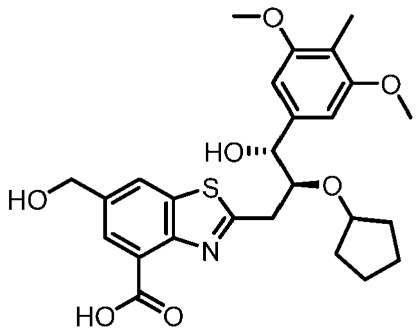 | 502.3 (M+H)+ |
| 281 | 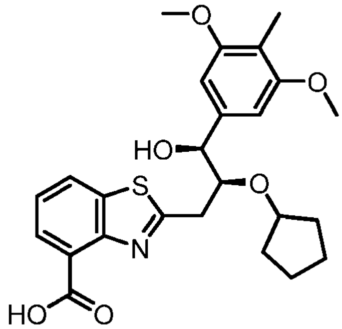 | 472.4 (M+H)+ |
| 282 | 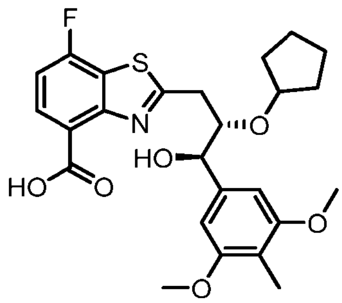 | |
| 283 | 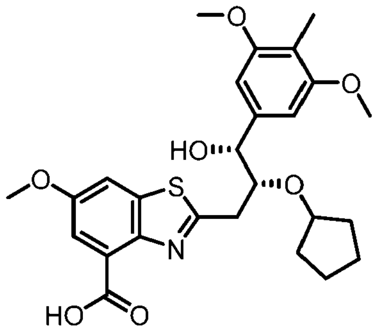 | 502.2 (M+H)+ |
| 284 | 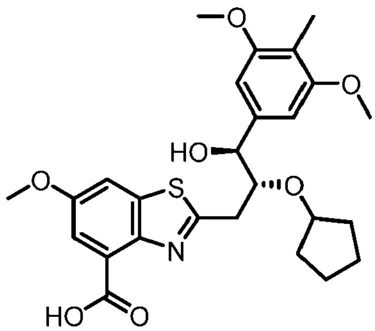 | 502.2 (M+H)+ |

| | |
|---|---|
| **285** | 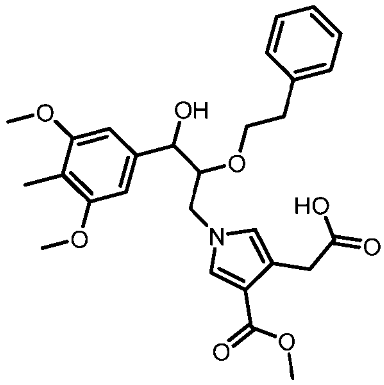 |
| **286** | 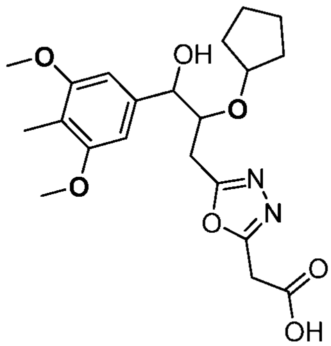 |
| **287** | 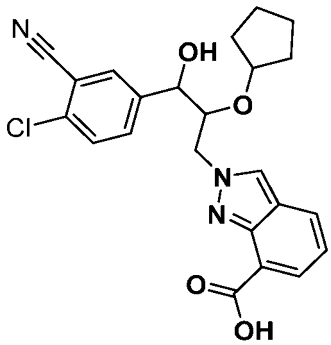 |
| **288** | 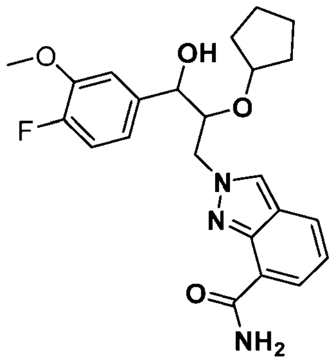 |

(continued)

| | |
|---|---|
| **289** | 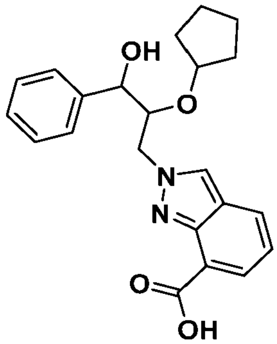 |
| **290** | 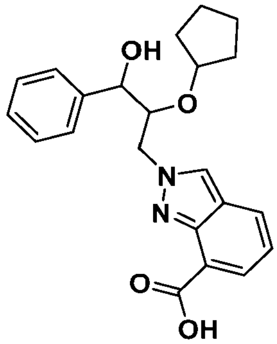 |
| **291** | 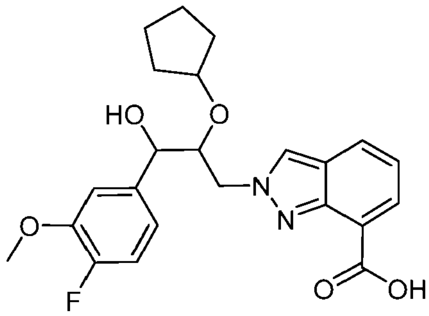 |
| **292** | 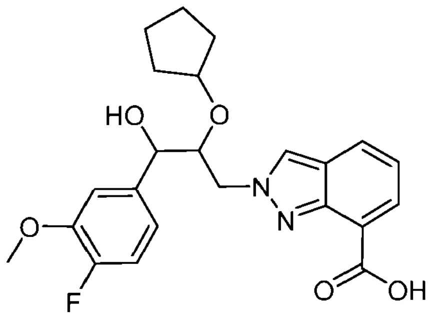 |
| **293** | 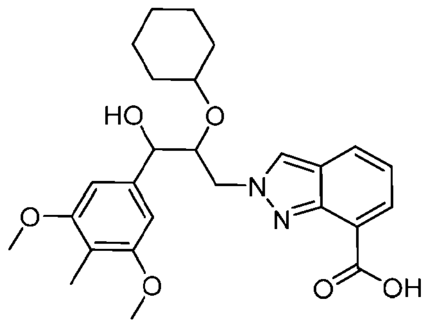 |

(continued)

| | |
|---|---|
| **294** | 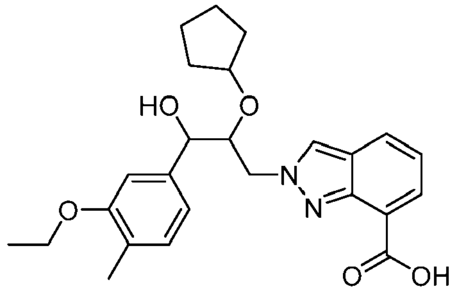 |
| **295** | 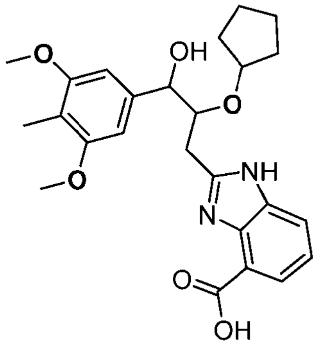 |
| **296** | 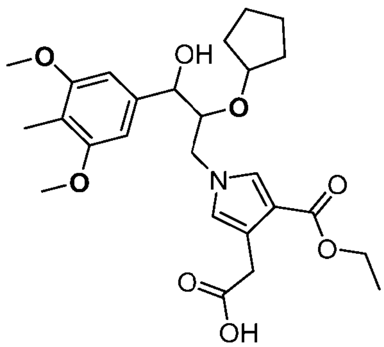 |
| **297** | 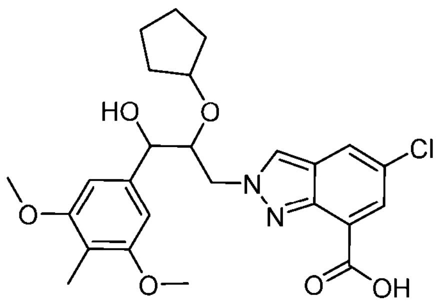 |
| **298** | 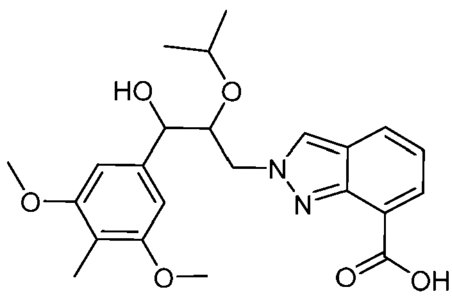 |

| Compound | Structure |
|---|---|
| 299 | 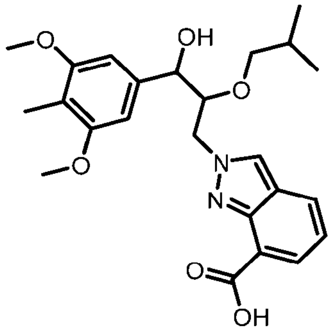 |
| 300 | 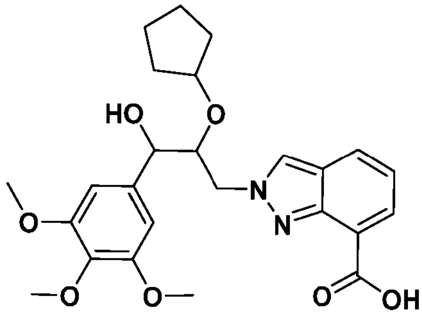 |
| 301 | 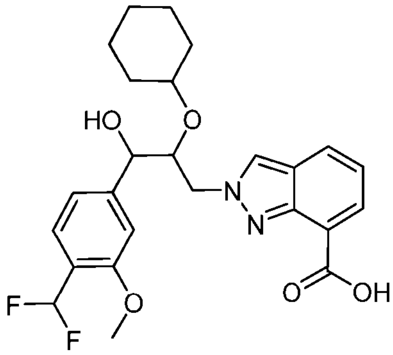 |
| 302 | 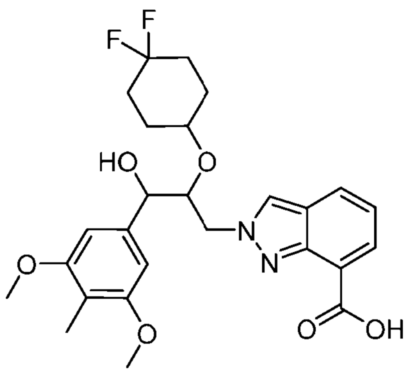 |
| 303 | 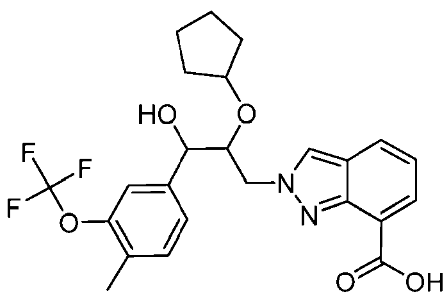 |

(continued)

| Compound | Structure |
|---|---|
| **304** | 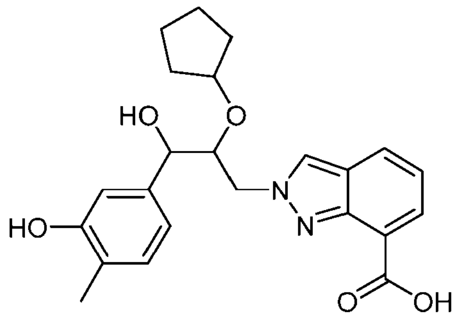 |
| **305** | 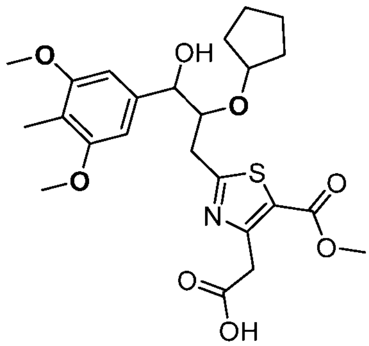 |
| **306** | 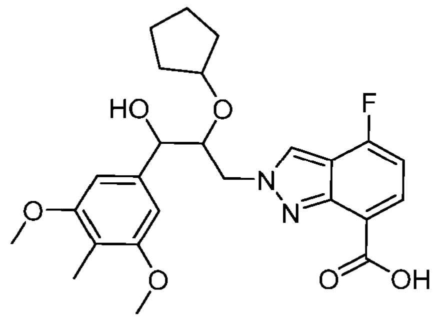 |
| **307** | 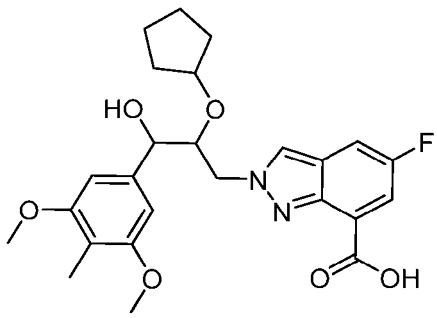 |
| **308** | 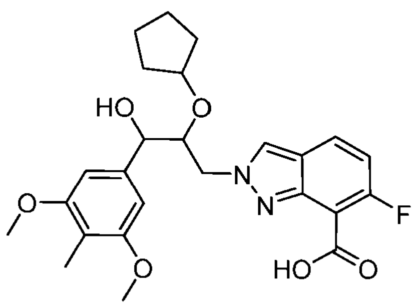 |

(continued)

| Compound | Structure |
|---|---|
| **309** | 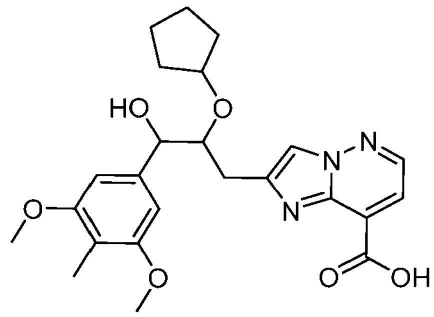 |
| **310** | 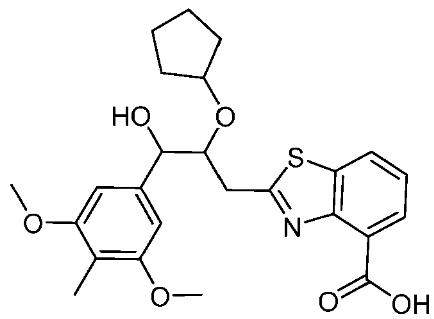 |
| **311** | 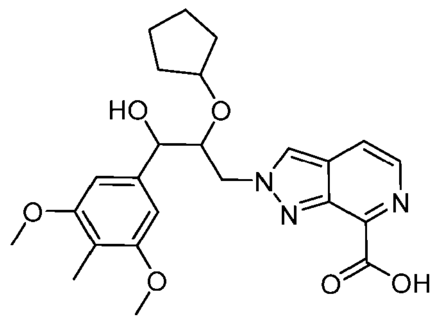 |
| **312** | 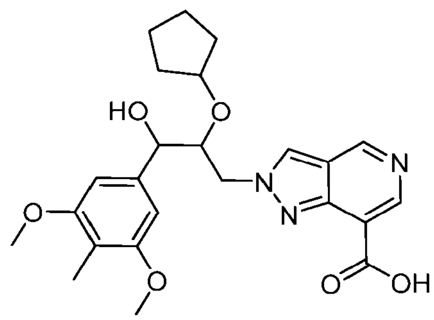 |
| **313** | 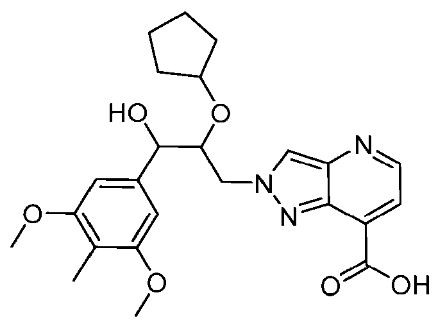 |

(continued)

| Compound | Structure |
|---|---|
| **314** | 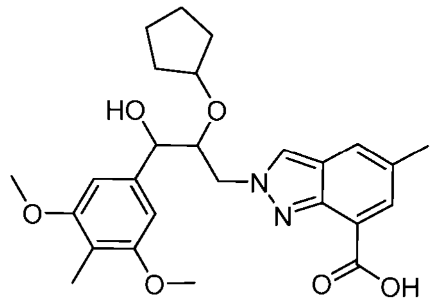 |
| **315** | 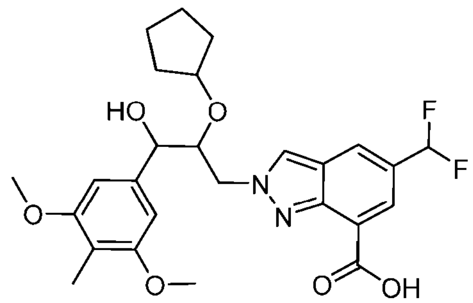 |
| **316** | 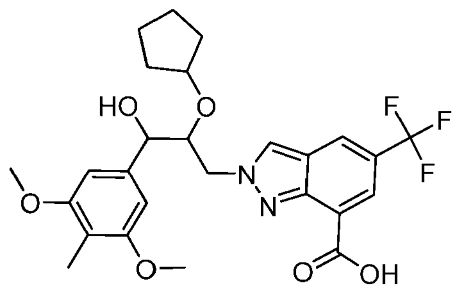 |
| **317** | 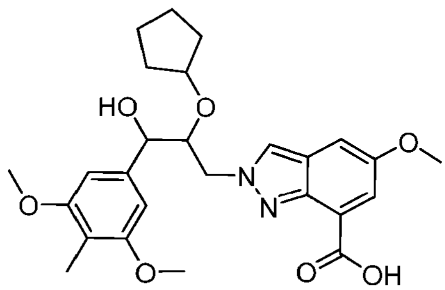 |
| **318** | 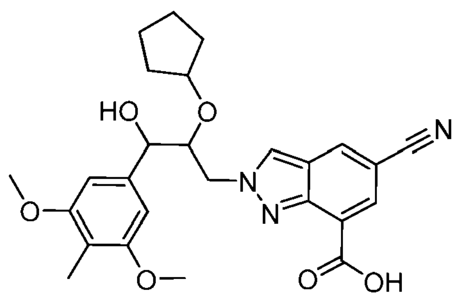 |

(continued)

| Compound | Structure |
|---|---|
| **319** | 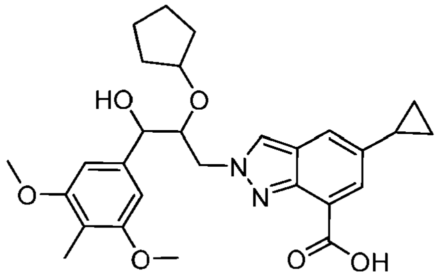 |
| **320** | 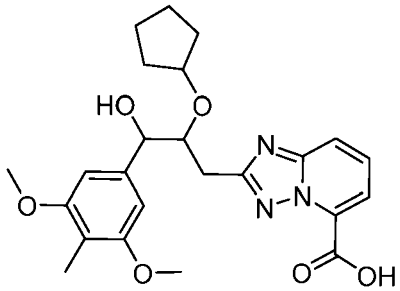 |
| **321** | 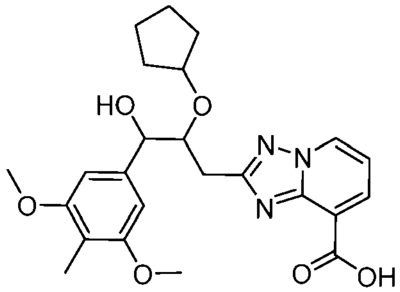 |
| **322** | 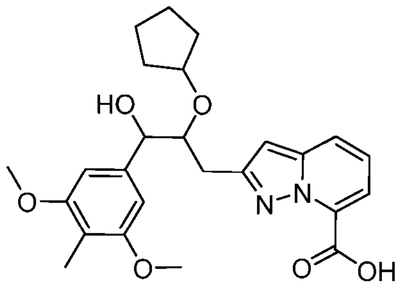 |
| **323** | 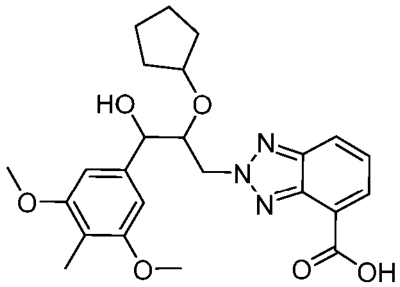 |

(continued)

| Compound | Structure |
|---|---|
| **324** | 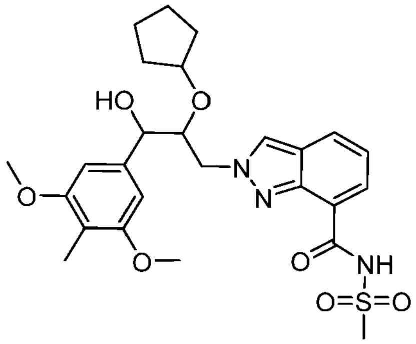 |
| **325** | 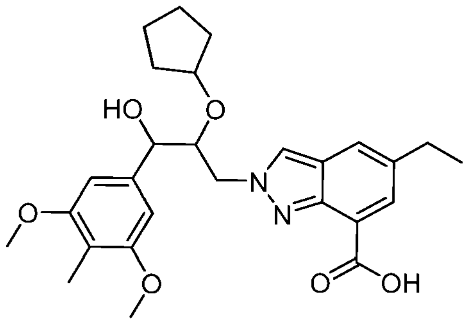 |
| **326** | 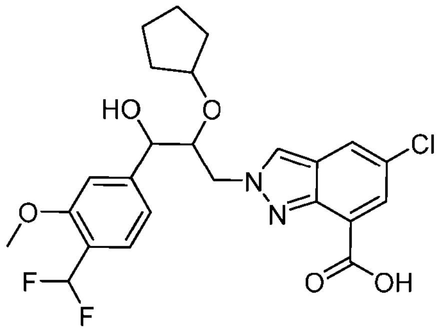 |
| **327** | 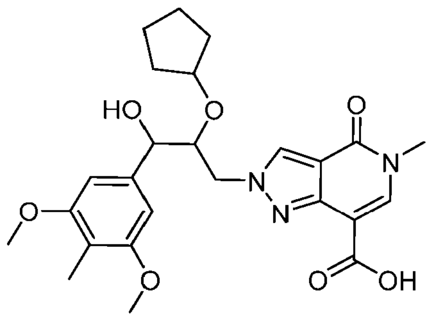 |
| **328** | 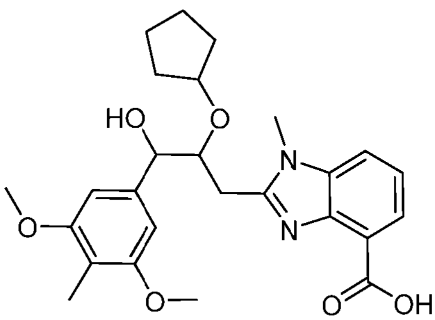 |

(continued)

| Compound | Structure |
|---|---|
| **329** | 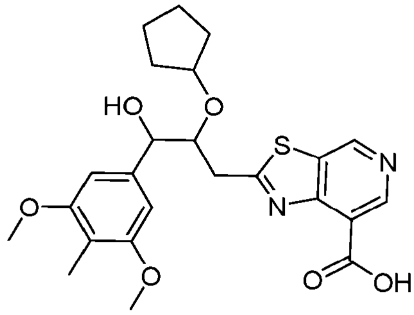 |
| **330** | 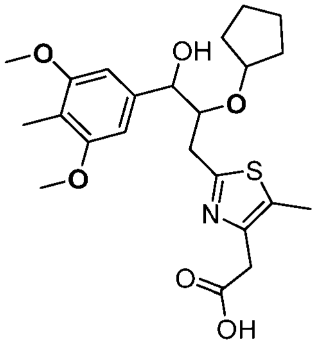 |
| **331** | 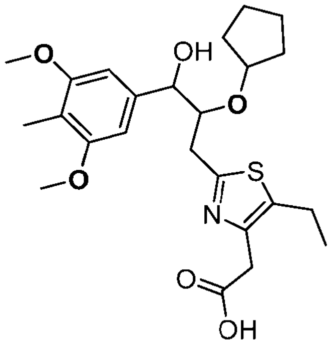 |
| **332** | 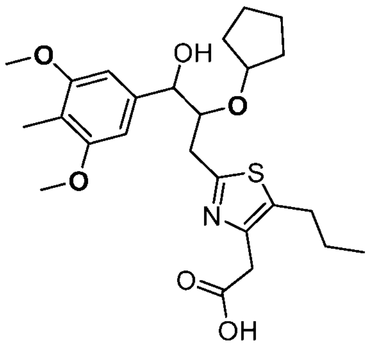 |
| **333** | 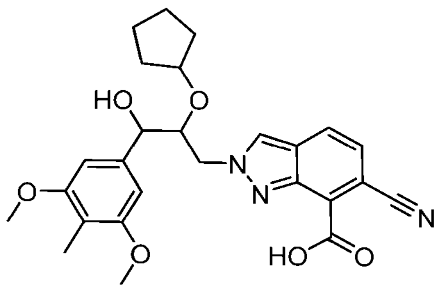 |

(continued)

| Compound | Structure |
|---|---|
| **334** | 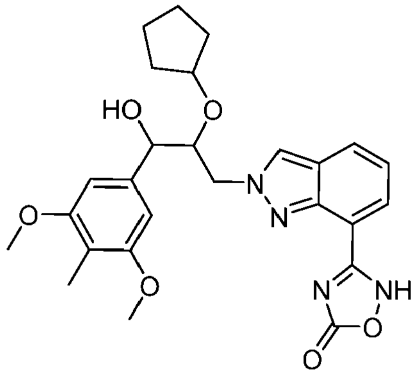 |
| **335** | 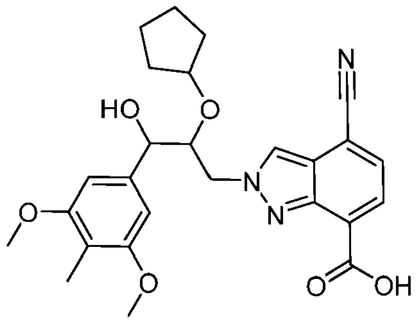 |
| **336** | 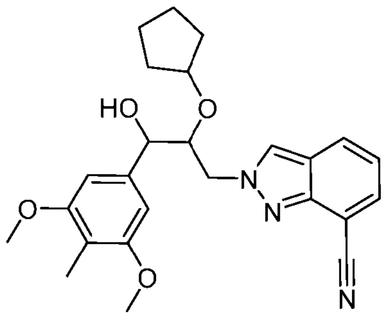 |
| **337** | 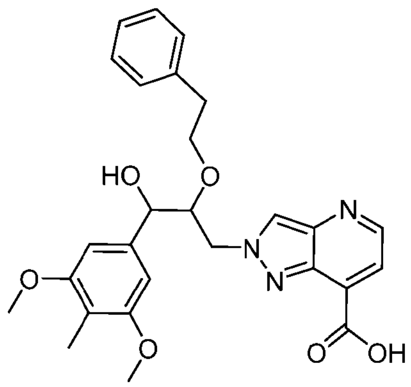 |

(continued)

| Compound | Structure |
|---|---|
| **338** | 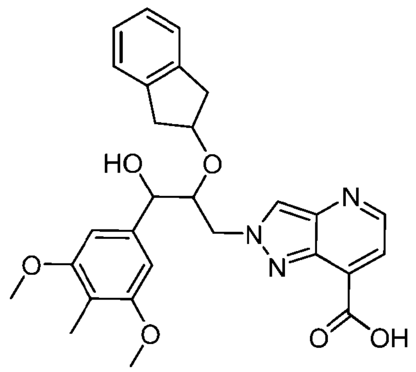 |
| **339** | 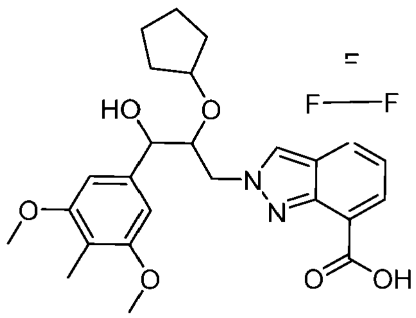 |
| **340** | 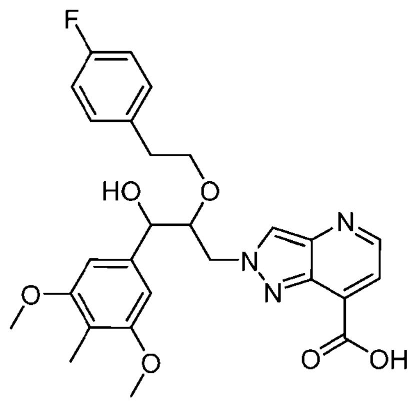 |
| **341** | 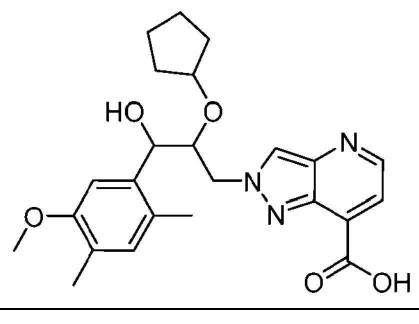 |

(continued)

| Compound | Structure |
|---|---|
| **342** | 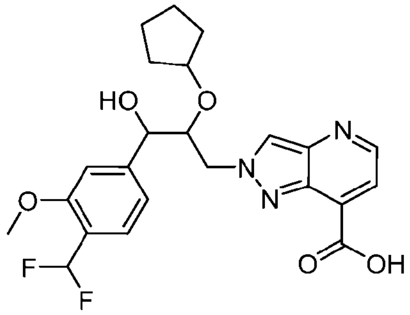 |
| **343** | 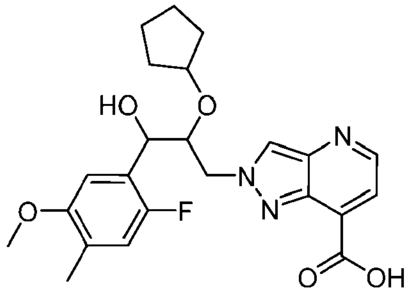 |
| **344** | 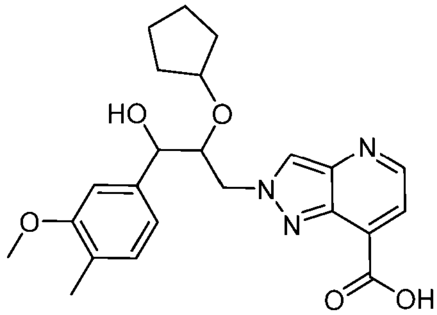 |
| **345** | 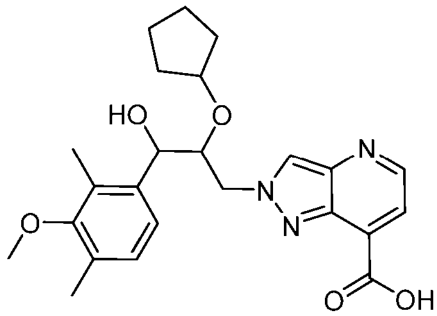 |
| **346** | 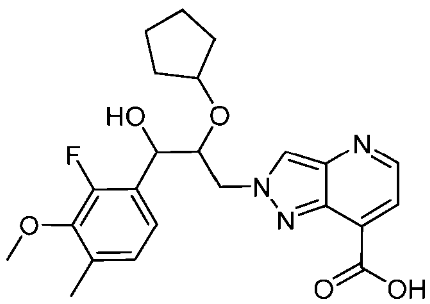 |

(continued)

| Compound | Structure |
|---|---|
| **347** | 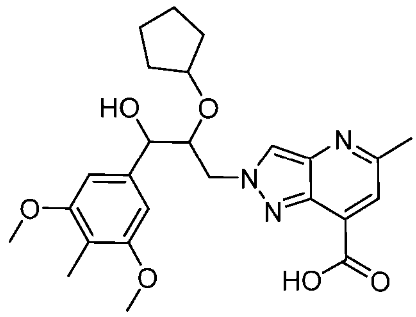 |
| **348** | 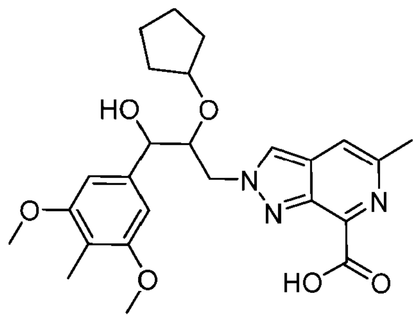 |
| **349** | 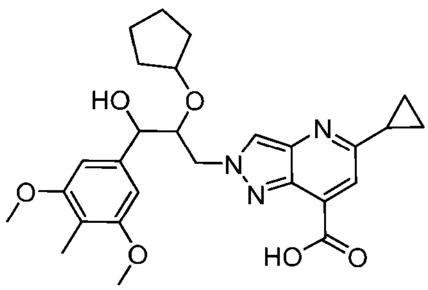 |
| **350** | 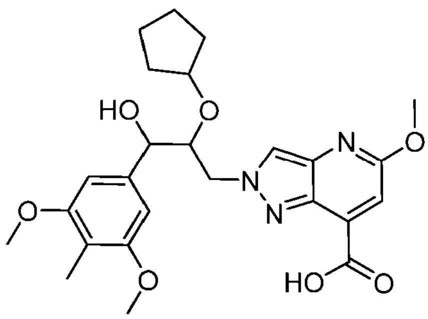 |
| **351** | 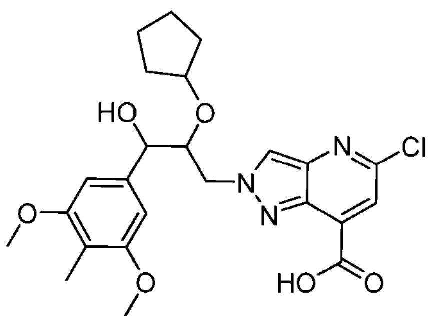 |

(continued)

| Compound | Structure |
|---|---|
| **352** | 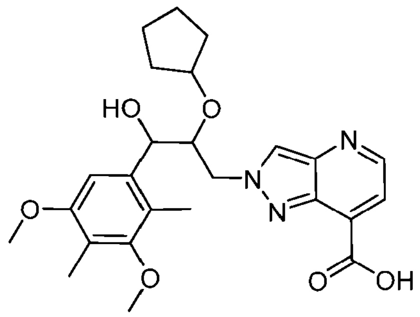 |
| **353** | 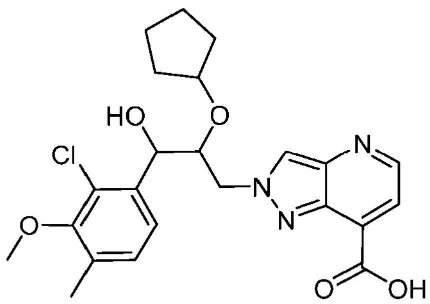 |
| **354** | 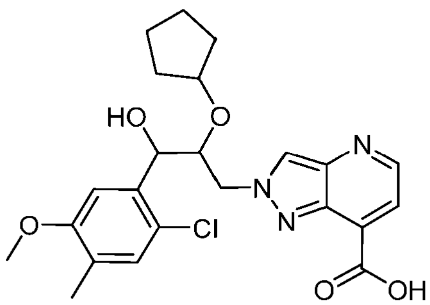 |
| **355** | 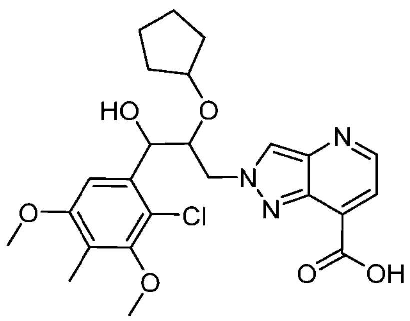 |
| **356** | 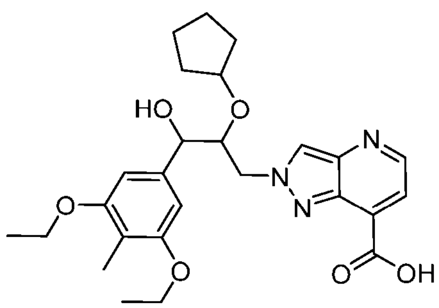 |

(continued)

| Compound | Structure |
|---|---|
| 357 | 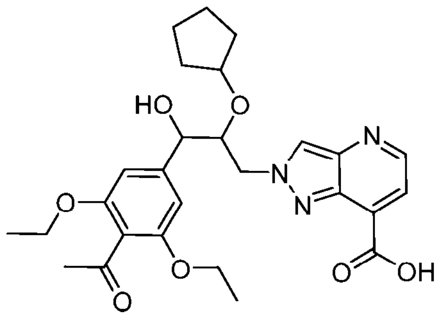 |
| 358 | 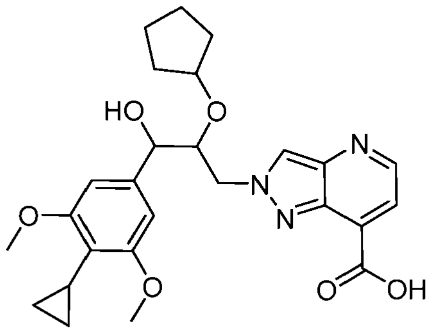 |
| 359 | 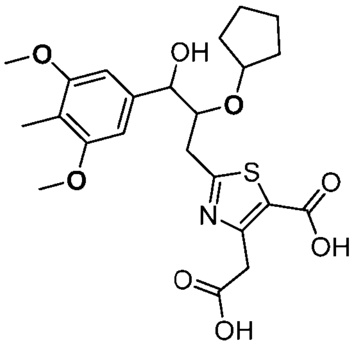 |
| 360 | 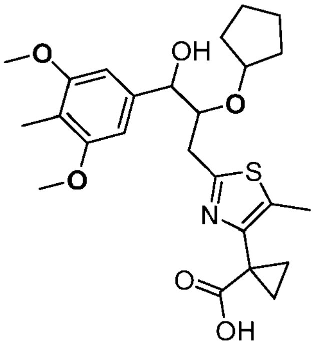 |

(continued)

| Compound | Structure |
|---|---|
| **361** | 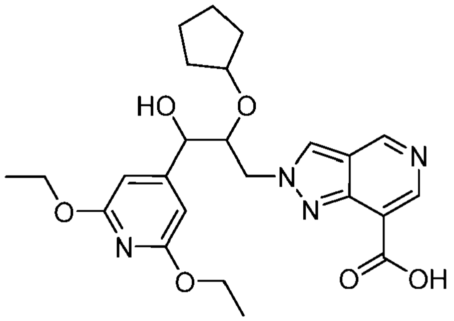 |
| **362** | 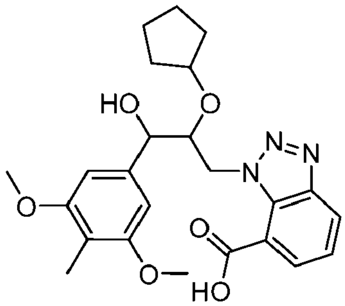 |
| **363** | 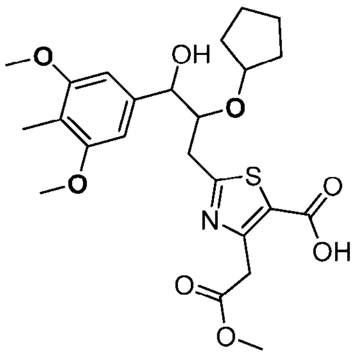 |
| **364** | 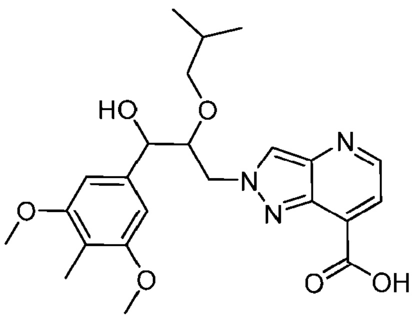 |
| **365** | 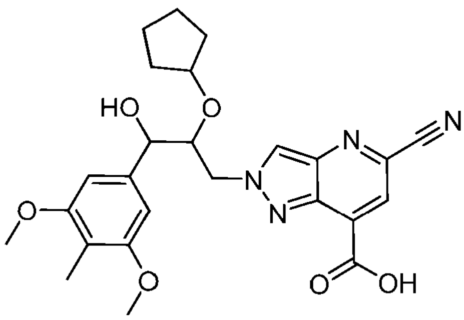 |

(continued)

| Compound | Structure |
|---|---|
| **366** | 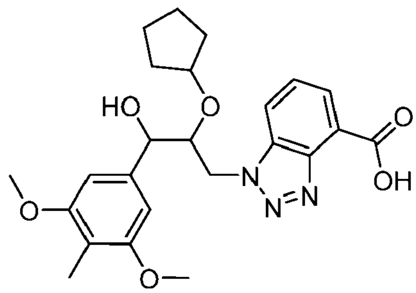 |
| **367** | 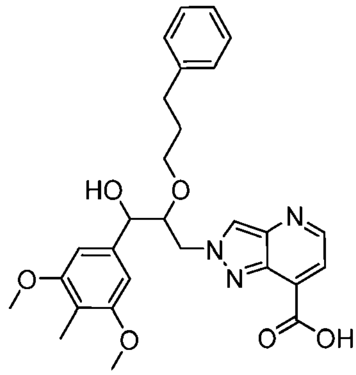 |
| **368** | 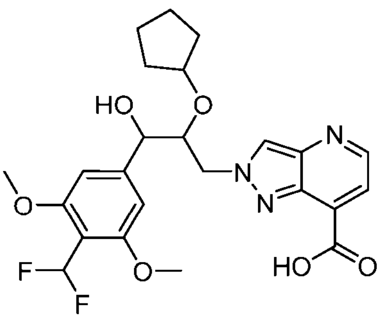 |
| **369** | 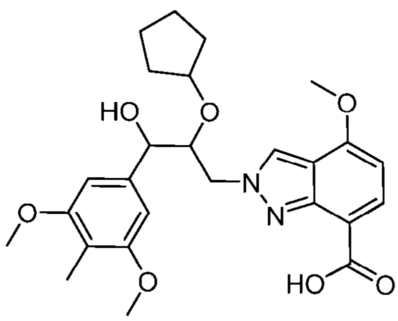 |
| **370** | 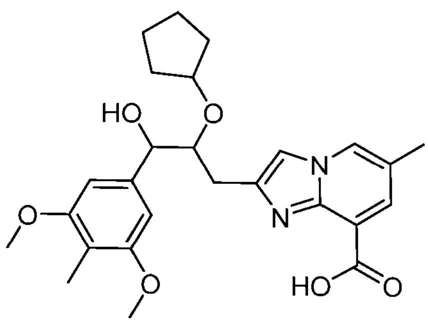 |

(continued)

| Compound | Structure |
|---|---|
| **371** | 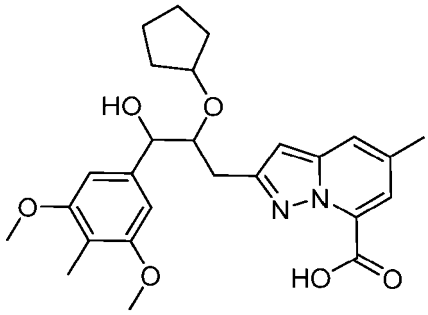 |
| **372** | 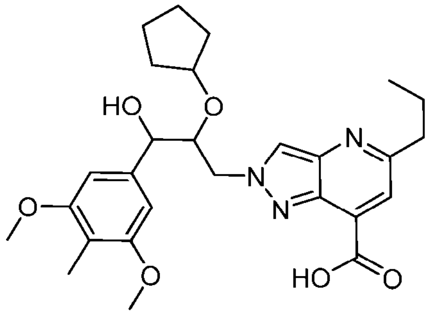 |
| **373** | 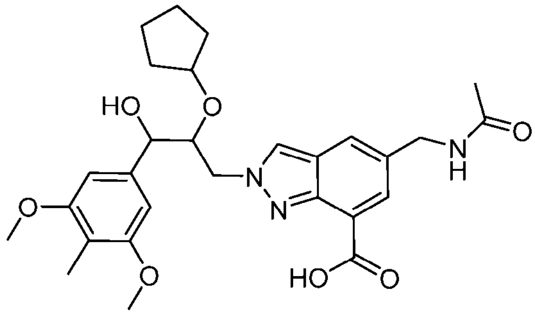 |
| **374** | 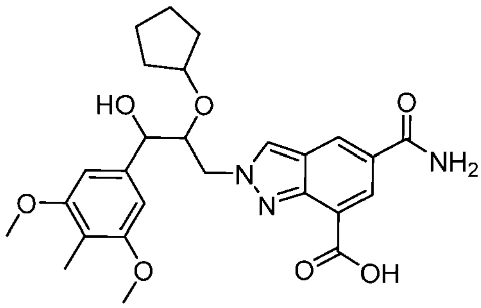 |
| **375** | 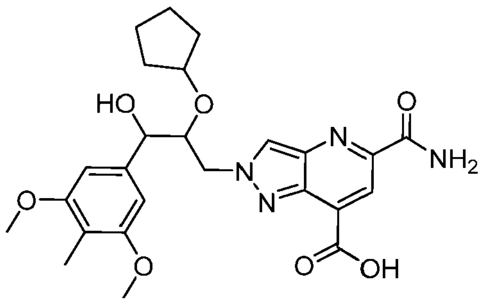 |

(continued)

| Compound | Structure |
|---|---|
| 376 | 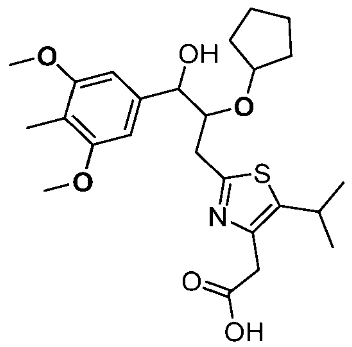 |
| 377 | 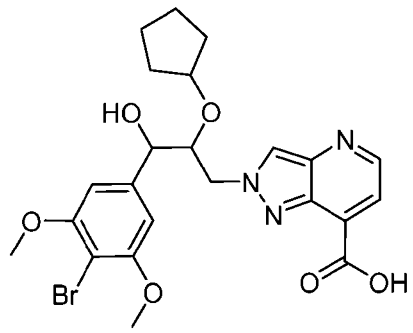 |
| 378 | 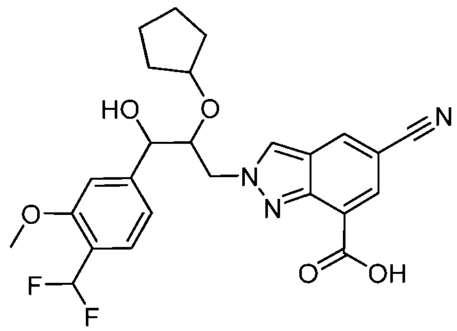 |
| 379 | 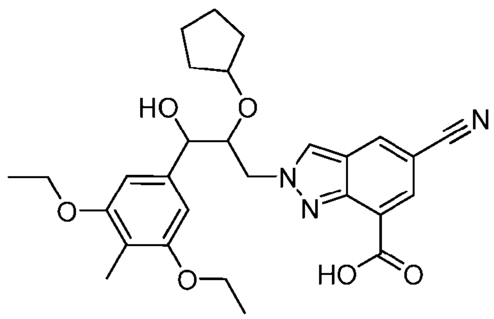 |
| 380 | 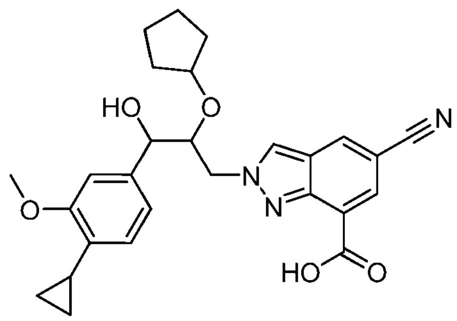 |

(continued)

| Compound | Structure |
|---|---|
| **381** | 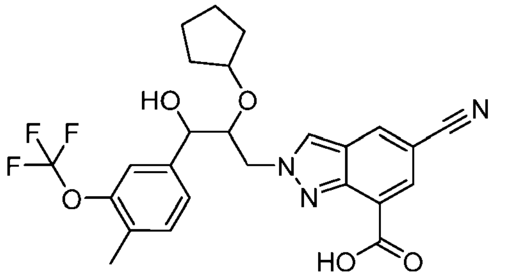 |
| **382** | 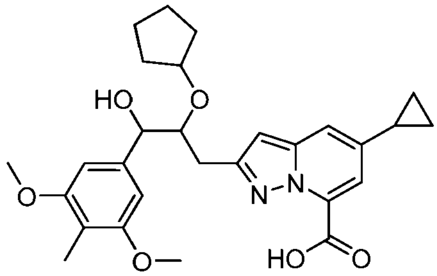 |
| **383** | 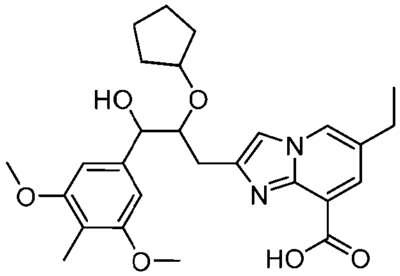 |
| **384** | 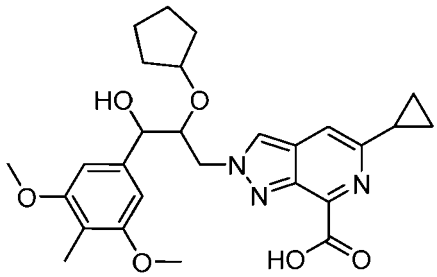 |
| **385** | 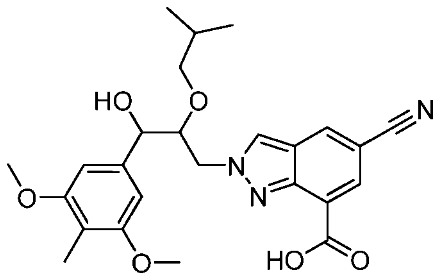 |

(continued)

| Compound | Structure |
|---|---|
| 386 | 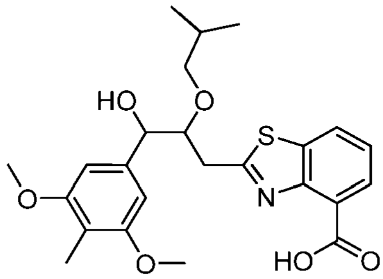 |
| 387 | 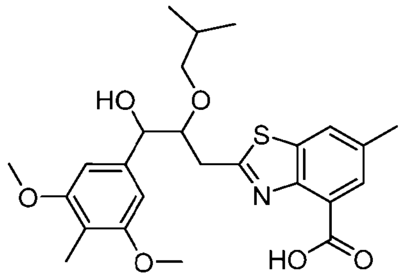 |
| 388 | 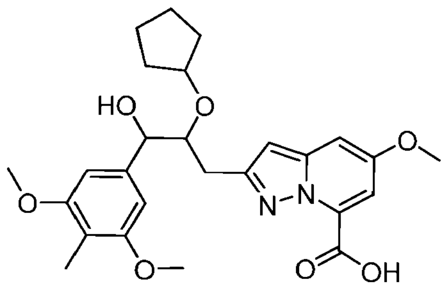 |
| 389 | 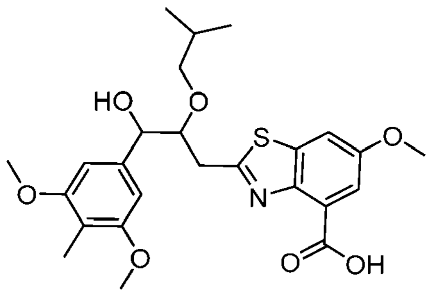 |
| 390 | 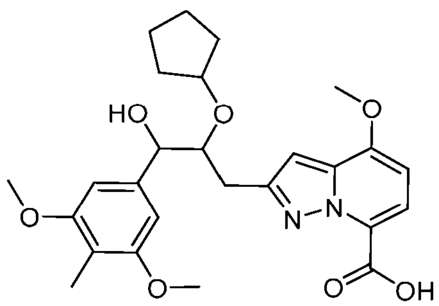 |

(continued)

| Compound | Structure |
|---|---|
| **391** | 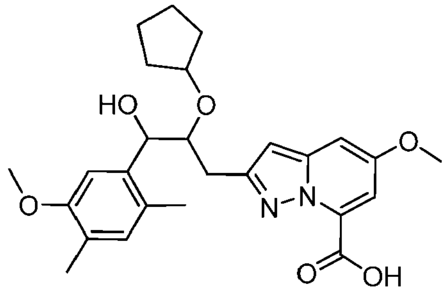 |
| **392** | 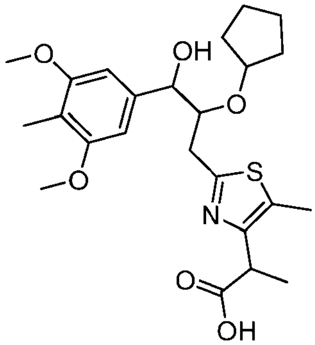 |
| **393** | 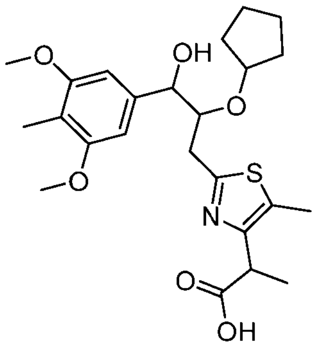 |
| **394** | 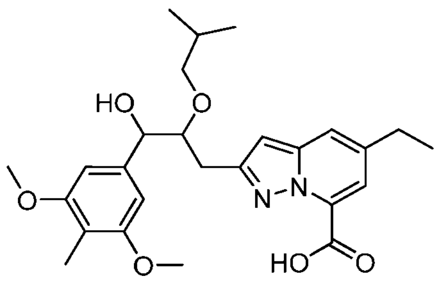 |
| **395** | 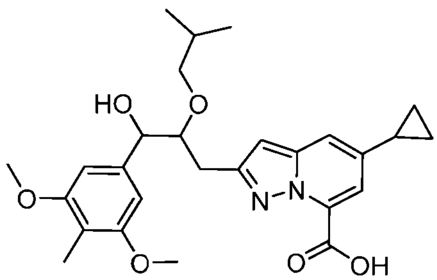 |

(continued)

| Compound | Structure |
|---|---|
| **396** | 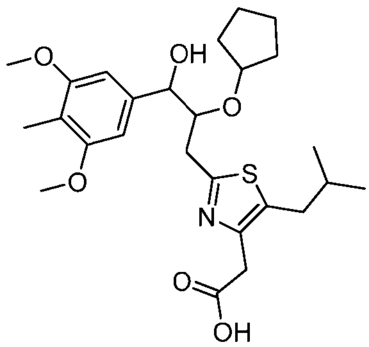 |
| **397** | 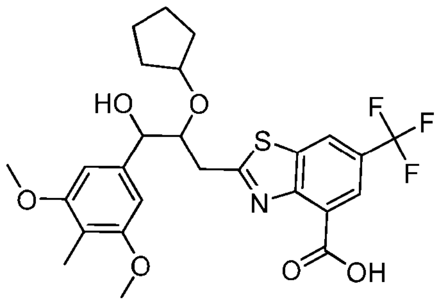 |
| **398** | 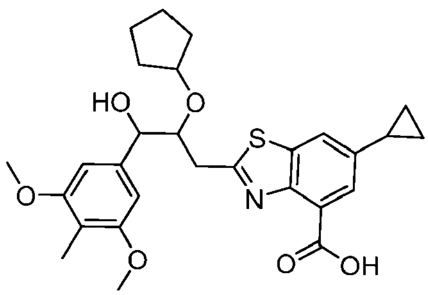 |
| **399** | 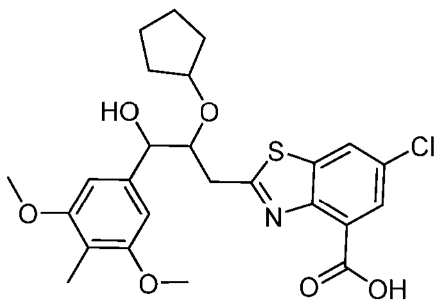 |
| **400** | 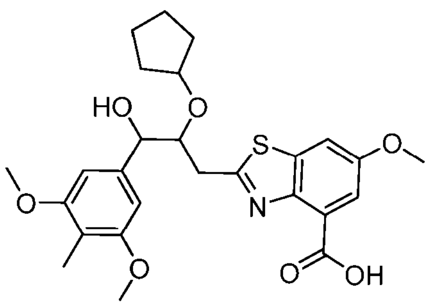 |

(continued)

| Compound | Structure |
|---|---|
| **401** | 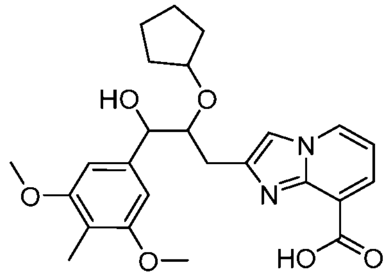 |
| **402** | 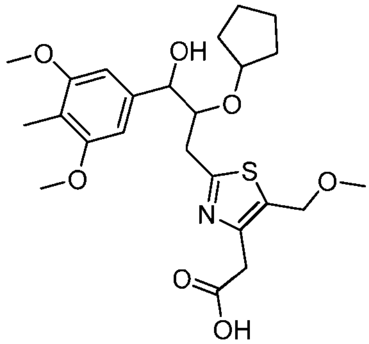 |
| **403** | 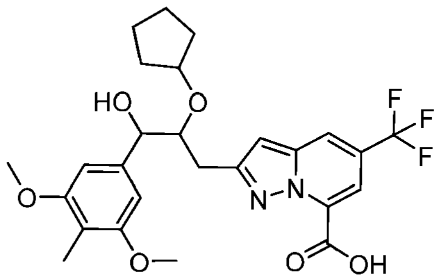 |
| **404** | 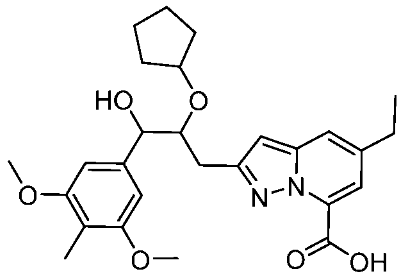 |
| **405** | 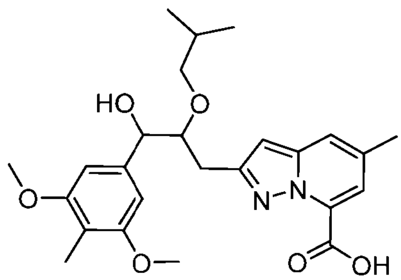 |

(continued)

| Compound | Structure |
|---|---|
| **406** | 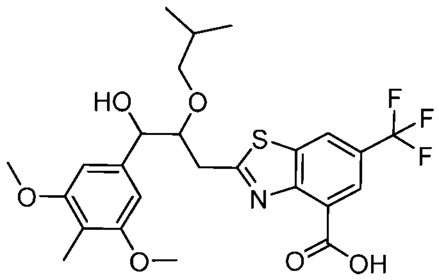 |
| **407** | 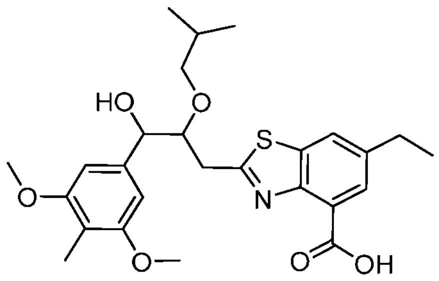 |
| **408** | 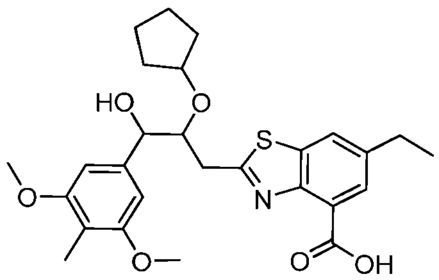 |
| **409** | 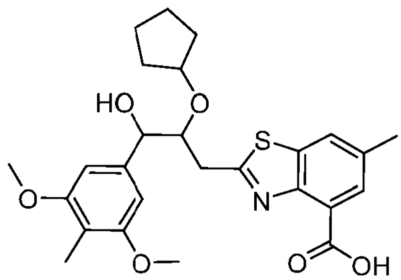 |
| **410** | 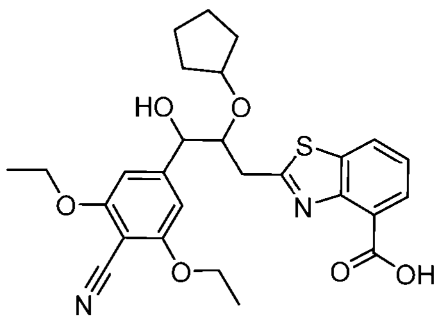 |

(continued)

| Compound | Structure |
|---|---|
| **411** | 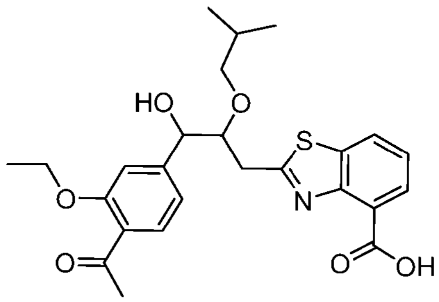 |
| **412** | 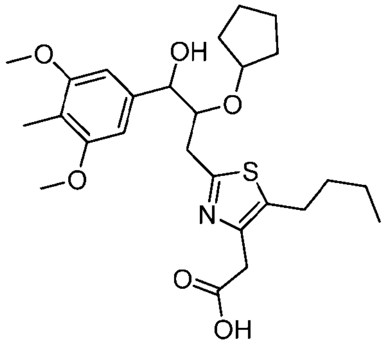 |
| **413** | 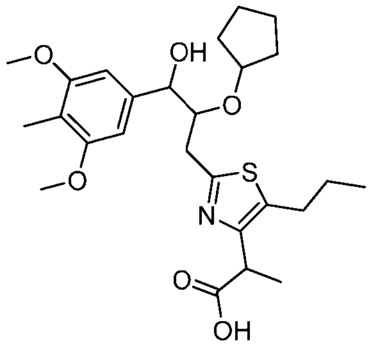 |
| **414** | 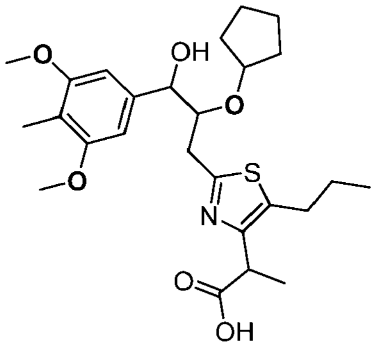 |
| **415** | 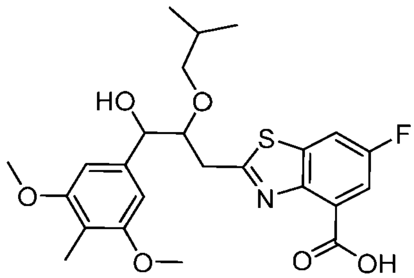 |

(continued)

| Compound | Structure |
| --- | --- |
| **416** | 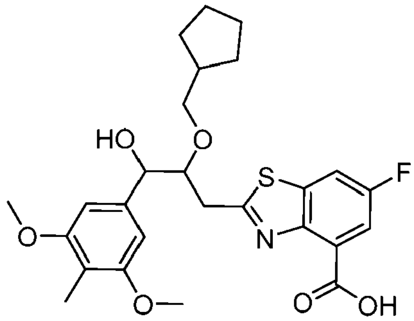 |
| **417** | 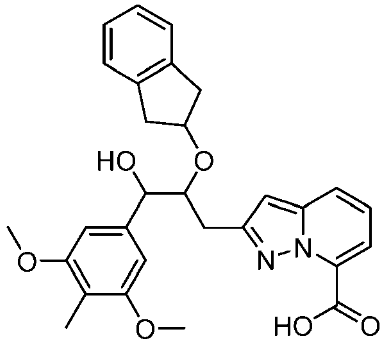 |
| **418** | 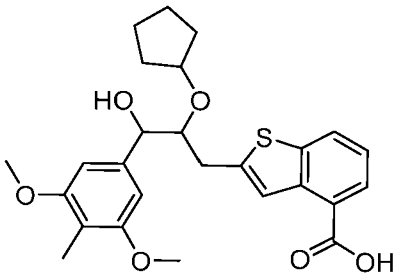 |
| **419** | 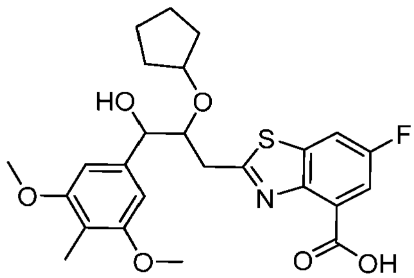 |

| Compound | Structure |
| --- | --- |
| **420** | 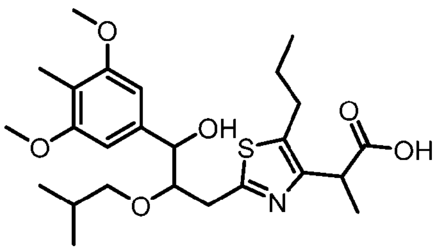 |

(continued)

| Compound | Structure |
|---|---|
| **421** | 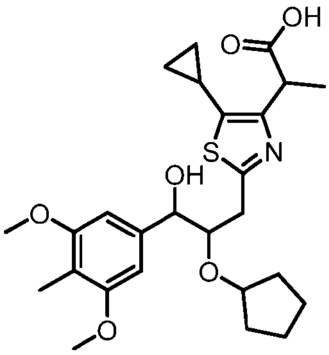 |
| **422** | 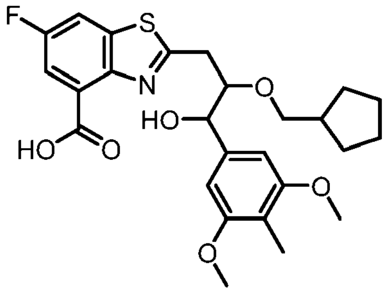 |
| **423** | 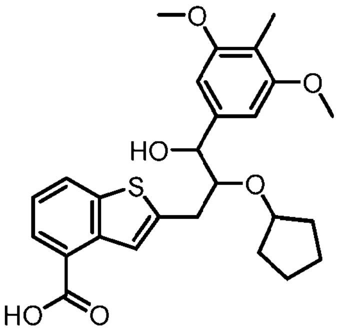 |
| **424** | 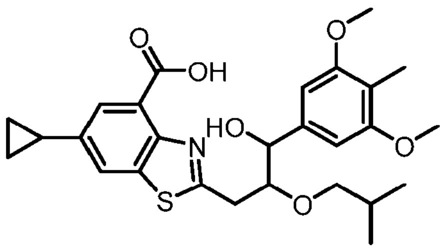 |
| **425** | 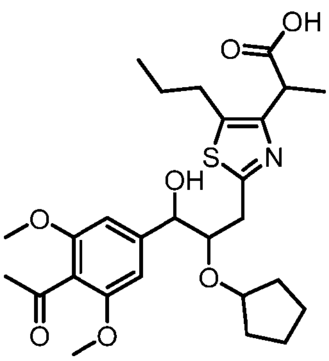 |

(continued)

| Compound | Structure |
|---|---|
| **426** | 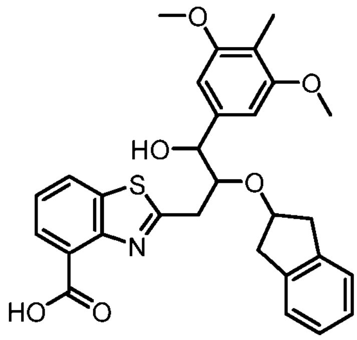 |
| **427** | 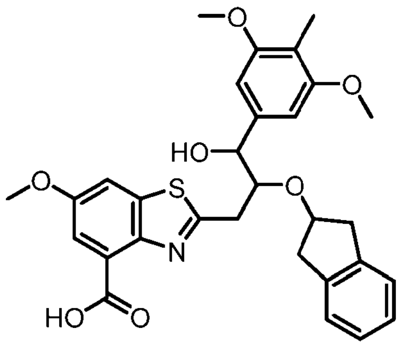 |
| **428** | 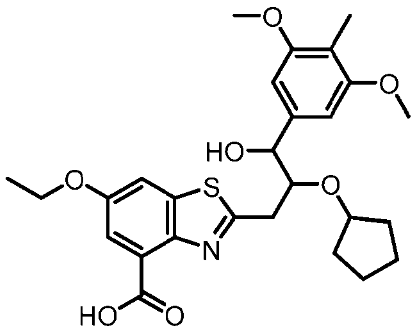 |
| **429** | 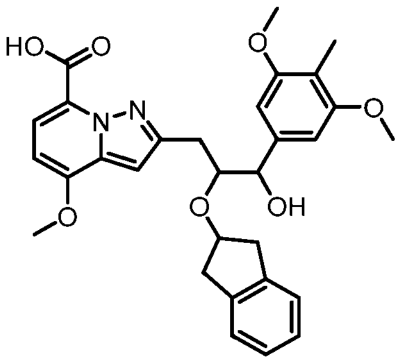 |

(continued)

| Compound | Structure |
|---|---|
| **430** | 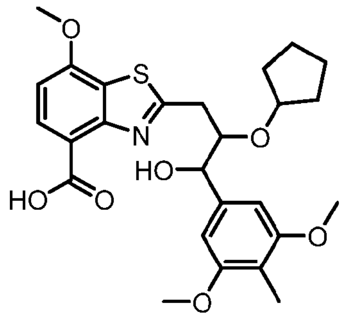 |
| **431** | 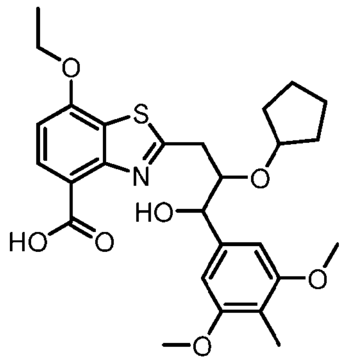 |
| **432** | 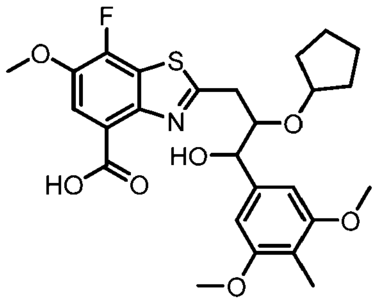 |
| **433** | 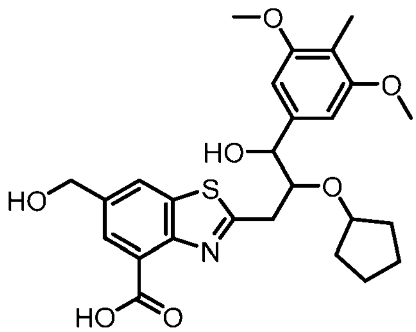 |
| **434** | 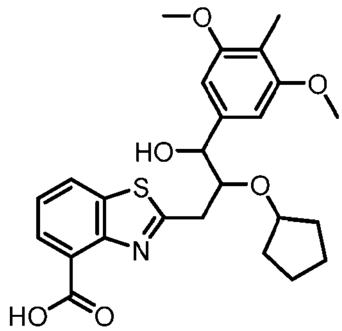 |

(continued)

| Compound | Structure |
|---|---|
| **435** | 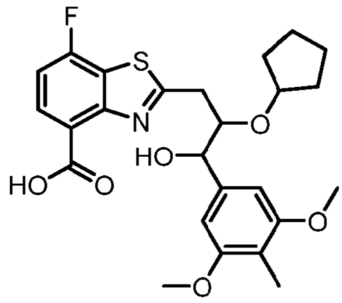 |
| **436** | 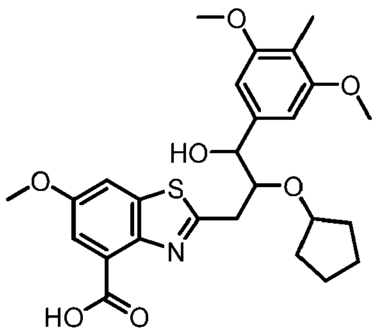 |
| **437** | 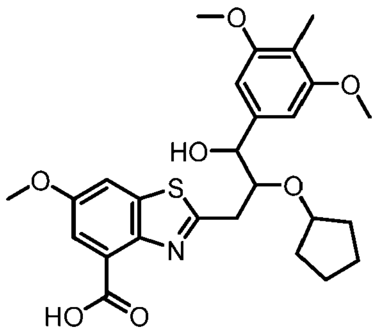 |
| **438** | 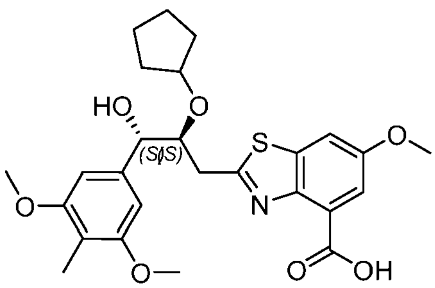 |
| **439** | 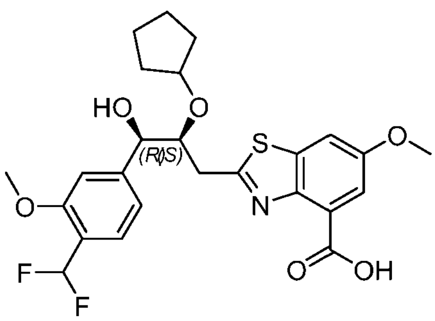 |

(continued)

| Compound | Structure |
|---|---|
| 440 | 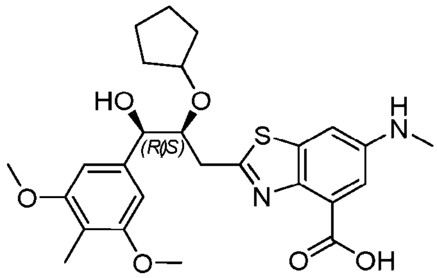 |
| 441 | 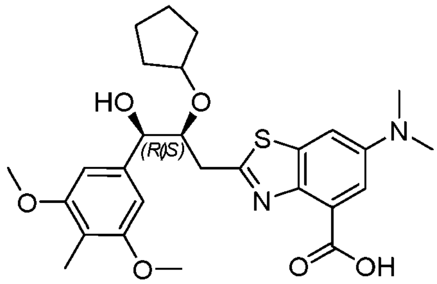 |
| 442 | 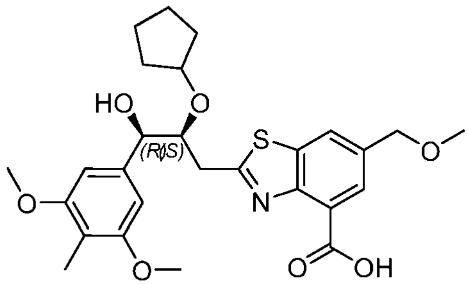 |
| 443 | 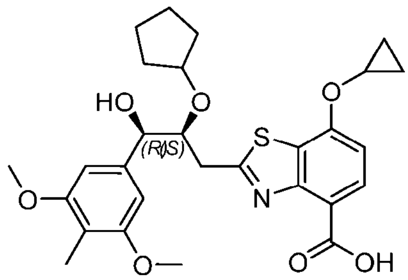 |
| 444 | 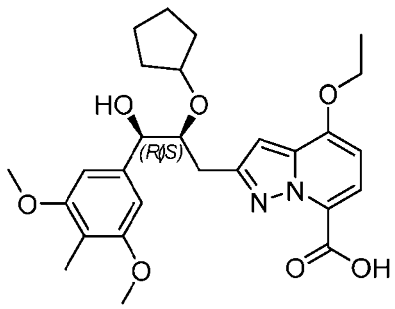 |

(continued)

| Compound | Structure |
|---|---|
| **445** | 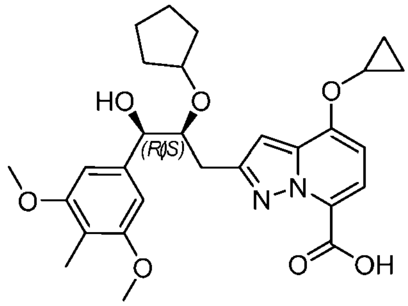 |
| **446** | 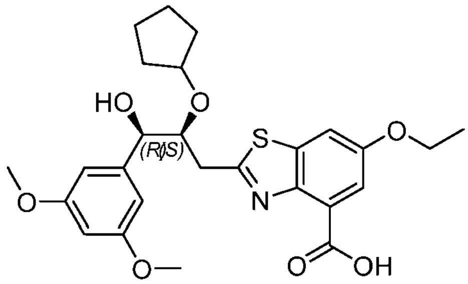 |
| **447** | 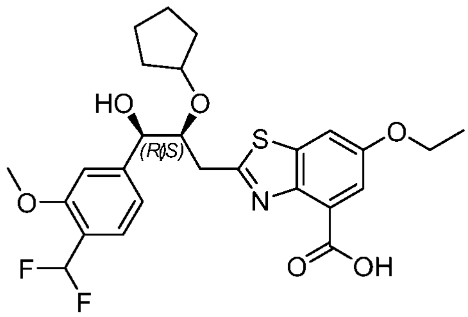 |
| **448** | 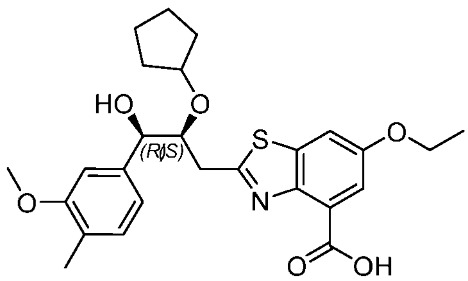 |
| **449** | 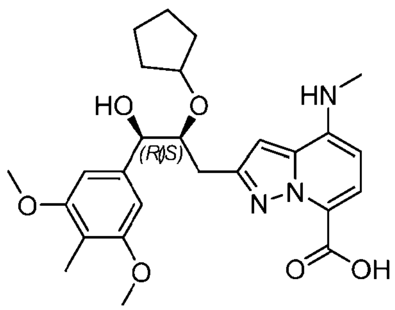 |

(continued)

| Compound | Structure |
|---|---|
| **450** | 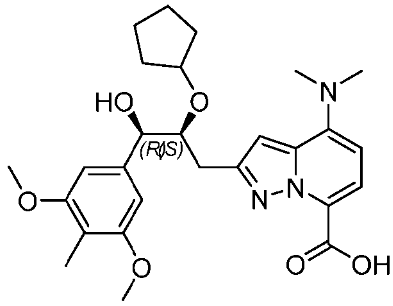 |
| **451** | 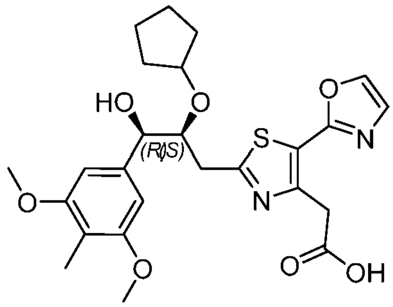 |

The compounds of formula (**I**) provided herein encompass all stereochemical forms, for example, optical isomers, such as enantiomers, diastereomers as well as mixtures thereof, e.g., mixtures of enantiomers and/or diastereomers, including racemic mixtures, as well as equal or non-equal mixtures of individual enantiomers and/or diastereomers. All stereochemical forms are contemplated in this disclosure. Unless otherwise indicated, when a disclosed compound is named or depicted by a structure without specifying the stereochemistry and has one or more chiral centers, it is understood to represent all possible stereoisomers of the compound. Representative stereochemical forms are provided throughout the specification: including but not limited to those delineated in Table C2.

The compounds of Formula (**I**) include pharmaceutically acceptable salts thereof. In addition, the compounds of Formula (**I**) also include other salts of such compounds which are not necessarily pharmaceutically acceptable salts, and which may be useful as intermediates for preparing and/or purifying compounds of Formula (**I**) and/or for separating enantiomers of compounds of Formula (**I**). Non-limiting examples of pharmaceutically acceptable salts of compounds of Formula (**I**) include trifluoroacetic acid salts.

It will further be appreciated that the compounds of Formula (**I**) or their salts may be isolated in the form of solvates, and accordingly that any such solvate is included within the scope of the present disclosure. For example, compounds of Formula (**I**) and salts thereof can exist in unsolvated as well as solvated forms with pharmaceutically acceptable solvents such as water, ethanol, and the like.

*Pharmaceutical Compositions and Administration*

When employed as pharmaceuticals, the compounds of Formula (**I**) (e.g., a compound of Formulas **I-A**, **I-1**, **I-2**, or **I-3**), including pharmaceutically acceptable salts or solvates thereof, can be administered in the form of a pharmaceutical compositions. These compositions can be prepared in a manner well known in the pharmaceutical art, and can be administered by a variety of routes, depending upon whether local or systemic treatment is desired and upon the area to be treated. Administration can be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Oral administration can include a dosage form formulated for once-daily or twice-daily (BID) administration. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or can be, for example, by a continuous perfusion pump. Pharmaceutical compositions and formulations for topical administration can include transdermal patches, ointments, lotions, creams, gels, drops, suppositories, sprays, liquids and powders. Conventional pharmaceutical carriers, aqueous, powder or oily bases, thickeners and the like may be necessary or desirable.

Also provided herein are pharmaceutical compositions which contain, as the active ingredient, a compound of

Formula (**I**) (e.g., a compound of Formulas **I-A, I-1**, **I-2**, or **I-3**), or a pharmaceutically acceptable salt or solvate thereof, in combination with one or more pharmaceutically acceptable excipients (carriers). For example, a pharmaceutical composition prepared using a compound of Formula (**I**) (e.g., a compound of Formulas **I-A, I-1**, **I-2**, or **I-3**), or a pharmaceutically acceptable salt or solvate thereof. In some embodiments, the composition is suitable for topical administration. In making the compositions provided herein, the active ingredient is typically mixed with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders. In some embodiments, the composition is formulated for oral administration. In some embodiments, the composition is a solid oral formulation. In some embodiments, the composition is formulated as a tablet or capsule.

Further provided herein are pharmaceutical compositions containing a compound of Formula (**I**) (e.g., a compound of Formulas **I-A**, **I-1**, **I-2**, or **I-3**), or a pharmaceutically acceptable salt or solvate thereof with a pharmaceutically acceptable excipient. Pharmaceutical compositions containing a compound of Formula (**I**) (e.g., a compound of Formulas **I-A**, **I-1**, **I-2**, or **I-3**), or a pharmaceutically acceptable salt or solvate thereof as the active ingredient can be prepared by intimately mixing the compound of Formula (**I**) (e.g., a compound of Formulas **I-A**, **I-1**, **I-2**, or **I-3**), or a pharmaceutically acceptable salt or solvate thereof with a pharmaceutical carrier according to conventional pharmaceutical compounding techniques. The carrier can take a wide variety of forms depending upon the desired route of administration (*e.g.*, oral, parenteral). In some embodiments, the composition is a solid oral composition.

Suitable pharmaceutically acceptable carriers are well known in the art. Descriptions of some of these pharmaceutically acceptable carriers can be found in *The Handbook of Pharmaceutical Excipients,* published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3-hydroxypropyl-β-cyclodextrins, or other solubilized derivatives can also be used to enhance delivery of compounds as provided herein. Dosage forms or compositions containing a chemical entity as provided herein in the range of 0.005% to 100% with the balance made up from non-toxic excipient may be prepared. The contemplated compositions may contain 0.001%-100% of a chemical entity provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

In some embodiments, a compound of Formula (**I**) (e.g., a compound of Formulas **I-A**, **I-1**, **I-2**, or **I-3**), or a pharmaceutically acceptable salt or solvate thereof, or pharmaceutical compositions as provided herein can be administered to a subject in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intracisternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal (e.g., intranasal), nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In some embodiments, a preferred route of administration is parenteral (e.g., intratumoral).

In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein or pharmaceutical compositions thereof can be formulated for parenteral administration, e.g., formulated for

injection via the intraarterial, intrasternal, intracranial, intravenous, intramuscular, sub-cutaneous, or intraperitoneal routes. For example, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure. In some embodiments, devices are used for parenteral administration. For example, such devices may include needle injectors, microneedle injectors, needle-free injectors, and infusion techniques.

In some embodiments, the pharmaceutical forms suitable for injection include sterile aqueous solutions or dispersions; formulations including sesame oil, peanut oil, or aqueous propylene glycol; and sterile powders for the extemporaneous preparation of sterile injectable solutions or dispersions. In some embodiments, the form must be sterile and must be fluid to the extent that it may be easily injected. In some embodiments, the form should be stable under the conditions of manufacture and storage and must be preserved against the contaminating action of microorganisms, such as bacteria and fungi.

In some embodiments, the carrier also can be a solvent or dispersion medium containing, for example, water, ethanol, polyol (for example, glycerol, propylene glycol, and liquid polyethylene glycol, and the like), suitable mixtures thereof, and vegetable oils. In some embodiments, the proper fluidity can be maintained, for example, by the use of a coating, such as lecithin, by the maintenance of the required particle size in the case of dispersion, and by the use of surfactants. In some embodiments, the prevention of the action of microorganisms can be brought about by various antibacterial and antifungal agents, for example, parabens, chlorobutanol, phenol, sorbic acid, thimerosal, and the like. In some embodiments, isotonic agents, for example, sugars or sodium chloride are included. In some embodiments, prolonged absorption of the injectable compositions can be brought about by the use in the compositions of agents delaying absorption, for example, aluminum monostearate and gelatin.

In some embodiments, sterile injectable solutions are prepared by incorporating a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) in the required amount in the appropriate solvent with various of the other ingredients enumerated above, as required, followed by filtered sterilization. In some embodiments, dispersions are prepared by incorporating the various sterilized active ingredients into a sterile vehicle which contains the basic dispersion medium and the required other ingredients from those enumerated above. In some embodiments, sterile powders are used for the preparation of sterile injectable solutions. In some embodiments, the methods of preparation are vacuum-drying and freeze-drying techniques, which yield a powder of the active ingredient, plus any additional desired ingredient from a previously sterilefiltered solution thereof.

In some embodiments, pharmacologically acceptable excipients usable in a rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol, Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p-oxybenzoate, diethylamine, carbomers, carbopol, methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabisulfite, grapefruit seed extract, methyl sulfonyl methane (MSM) , lactic acid, glycine, vitamins, such as vitamin A and E and potassium acetate.

In some embodiments, suppositories can be prepared by mixing a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) or pharmaceutical compositions as provided herein with suitable non-irritating excipients or carriers such as cocoa butter, polyethylene glycol or a suppository wax which are solid at ambient temperature but liquid at body temperature and therefore melt in the rectum and release the active compound. In some embodiments, compositions for rectal administration are in the form of an enema.

In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein or a pharmaceutical composition thereof is formulated for local delivery to the digestive or GI tract by way of oral administration (e.g., solid or liquid dosage forms.).

In some embodiments, solid dosage forms for oral administration include capsules, tablets, pills, powders, and granules. In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and

sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. For example, in the case of capsules, tablets and pills, the dosage form may also comprise buffering agents. In some embodiments, solid compositions of a similar type may also be employed as fillers in soft and hard-filled gelatin capsules using such excipients as lactose or milk sugar as well as high molecular weight polyethylene glycols and the like.

In some embodiments, the pharmaceutical compositions will take the form of a unit dosage form such as a pill or tablet and thus the composition may contain, along with a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In some embodiments, another solid dosage form, a powder, marume, solution or suspension (e.g., in propylene carbonate, vegetable oils, PEG's, poloxamer 124 or triglycerides) is encapsulated in a capsule (gelatin or cellulose base capsule). In some embodiments, unit dosage forms in which one or more compounds and pharmaceutical compositions as provided herein or additional active agents are physically separated are also contemplated; e.g., capsules with granules (or tablets in a capsule) of each drug; two-layer tablets; two-compartment gel caps, etc. In some embodiments, enteric coated or delayed release oral dosage forms are also contemplated.

In some embodiments, other physiologically acceptable compounds may include wetting agents, emulsifying agents, dispersing agents or preservatives that are particularly useful for preventing the growth or action of microorganisms. For example, various preservatives are well known and include, for example, phenol and ascorbic acid.

In some embodiments, the excipients are sterile and generally free of undesirable matter. For example, these compositions can be sterilized by conventional, well-known sterilization techniques. In some embodiments, for various oral dosage form excipients such as tablets and capsules, sterility is not required. For example, the United States Pharmacopeia/National Formulary (USP/NF) standard can be sufficient.

In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein or a pharmaceutical composition thereof is formulated for ocular administration. In some embodiments, ocular compositions can include, without limitation, one or more of any of the following: viscogens (e.g., carboxymethylcellulose, glycerin, polyvinylpyrrolidone, polyethylene glycol); stabilizers (e.g., pluronic (triblock copolymers), cyclodextrins); preservatives (e.g., benzalkonium chloride, ETDA, SofZia (boric acid, propylene glycol, sorbitol, and zinc chloride; Alcon Laboratories, Inc.), Purite (stabilized oxychloro complex; Allergan, Inc.)).

In some embodiments, a compound of Formula (I), or a pharmaceutically acceptable salt or solvate thereof (e.g., a compound of any one of Formulas I-A, **I-1**, **I-2**, or **I-3**, or a pharmaceutically acceptable salt or solvate thereof) as provided herein or a pharmaceutical composition thereof is formulated for topical administration to the skin or mucosa (e.g., dermally or transdermally). In some embodiments, topical compositions can include ointments and creams. In some embodiments, ointments are semisolid preparations that are typically based on petrolatum or other petroleum derivatives. In some embodiments, creams containing the selected active agent are typically viscous liquid or semisolid emulsions, often either oil-in-water or water-in-oil. For example, cream bases are typically water-washable, and contain an oil phase, an emulsifier and an aqueous phase. For example, the oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. In some embodiments, the emulsifier in a cream formulation is generally a nonionic, anionic, cationic or amphoteric surfactant. In some embodiments, as with other carriers or vehicles, an ointment base should be inert, stable, nonirritating and non-sensitizing.

In any of the foregoing embodiments, pharmaceutical compositions as provided herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

In some embodiments, the dosage for a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof), is determined based on a multiple factors including, but not limited to, type, age, weight, sex, medical condition of the subject, severity of the medical condition of the subject, route of administration, and activity of the compound or pharmaceutically acceptable salt or solvate thereof. In some embodiments, proper dosage for a particular situation can be determined by one skilled in the medical arts. In some embodiments, the total daily dosage may be divided and administered in portions throughout the day or by means providing continuous delivery.

In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof), is administered at a dose from about 0.01 to about 1000 mg. For example, from about 0.1 to about 30 mg, about 10 to about 80

mg, about 0.5 to about 15 mg, about 50 mg to about 200 mg, about 100 mg to about 300 mg, about 200 to about 400 mg, about 300 mg to about 500 mg, about 400 mg to about 600 mg, about 500 mg to about 800 mg, about 600 mg to about 900 mg, or about 700 mg to about 1000 mg. In some embodiments, the dose is a therapeutically effective amount.

In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein is administered at a dosage of from about 0.0002 mg/Kg to about 100 mg/Kg (e.g., from about 0.0002 mg/Kg to about 50 mg/Kg; from about 0.0002 mg/Kg to about 25 mg/Kg; from about 0.0002 mg/Kg to about 10 mg/Kg; from about 0.0002 mg/Kg to about 5 mg/Kg; from about 0.0002 mg/Kg to about 1 mg/Kg; from about 0.0002 mg/Kg to about 0.5 mg/Kg; from about 0.0002 mg/Kg to about 0.1 mg/Kg; from about 0.001 mg/Kg to about 50 mg/Kg; from about 0.001 mg/Kg to about 25 mg/Kg; from about 0.001 mg/Kg to about 10 mg/Kg; from about 0.001 mg/Kg to about 5 mg/Kg; from about 0.001 mg/Kg to about 1 mg/Kg; from about 0.001 mg/Kg to about 0.5 mg/Kg; from about 0.001 mg/Kg to about 0.1 mg/Kg; from about 0.01 mg/Kg to about 50 mg/Kg; from about 0.01 mg/Kg to about 25 mg/Kg; from about 0.01 mg/Kg to about 10 mg/Kg; from about 0.01 mg/Kg to about 5 mg/Kg; from about 0.01 mg/Kg to about 1 mg/Kg; from about 0.01 mg/Kg to about 0.5 mg/Kg; from about 0.01 mg/Kg to about 0.1 mg/Kg; from about 0.1 mg/Kg to about 50 mg/Kg; from about 0.1 mg/Kg to about 25 mg/Kg; from about 0.1 mg/Kg to about 10 mg/Kg; from about 0.1 mg/Kg to about 5 mg/Kg; from about 0.1 mg/Kg to about 1 mg/Kg; from about 0.1 mg/Kg to about 0.5 mg/Kg). In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein is administered as a dosage of about 100 mg/Kg.

In some embodiments, the foregoing dosages of a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof), can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) as provided herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In some embodiments, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) is administered to a subject for a period of time followed by a separate period of time where administration of the compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof)is stopped. In some embodiments, a compound of Formula (**I**), or a pharmaceutically acceptable salt or solvate thereof (e.g., a compound of any one of Formulas **I-A**, **I-1**, **I-2**, or **I-3**, or a pharmaceutically acceptable salt or solvate thereof) is administered for a first period and a second period following the first period, with administration stopped during the second period, followed by a third period where administration of the compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof) is started and then a fourth period following the third period where administration is stopped. For example, the period of administration of a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof) followed by a period where administration is stopped is repeated for a determined or undetermined period of time. In some embodiments, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In some embodiments, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), is orally administered to the subject one or more times per day (e.g., one time per day, two times per day, three times per day, four times per day per day or a single daily dose).

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), is

administered by parenteral administration to the subject one or more times per day (e.g., 1 to 4 times one time per day, two times per day, three times per day, four times per day or a single daily dose).

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), is administered by parenteral administration to the subject weekly.

*Methods of Treatment*

The compounds as provided herein, or pharmaceutically acceptable salts or solvates thereof, or pharmaceutical compositions of such compounds, are useful as inhibitors of one or more LPA receptors. As described further herein, a compound antagonizing to an LPA receptor can be useful for prevention and/or treatment of diseases such as various kinds of disease including, for example, fibrosis (e.g., renal fibrosis, pulmonary fibrosis, hepatic fibrosis, arterial fibrosis, systemic sclerosis), urinary system disease, carcinoma-associated disease, proliferative disease, inflammation/immune system disease, disease by secretory dysfunction, brain-related disease, and chronic disease.

In some embodiments, this disclosure provides methods for treating a subject (e.g., a human) having a disease, disorder, or condition in which inhibition of one or more LPA receptors (i.e., an LPA-associated disease) is beneficial for the treatment of the underlying pathology and/or symptoms and/or progression of the disease, disorder, or condition. In some embodiments, the methods provided herein can include or further include treating one or more conditions associated, co-morbid or sequela with any one or more of the conditions provided herein.

Provided herein is a method for treating a LPA-associated disease, the method comprising administering to a subject in need thereof an effective amount of a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as disclosed herein.

In some embodiments, an LPA-associated disease includes, but is not limited to treating fibrosis of an organ (e.g., liver, kidney, lung, heart, and skin), liver disease (acute hepatitis, chronic hepatitis, liver fibrosis, liver cirrhosis, portal hypertension, regenerative failure, non-alcoholic steatohepatitis (NASH), liver hypofunction, hepatic blood flow disorder, and the like), cell proliferative disease (e.g., cancer, including solid tumors, solid tumor metastasis, vascular fibroma, myeloma, multiple myeloma, Kaposi's sarcoma, leukemia, and chronic lymphocytic leukemia (CLL), and invasive metastasis of cancer cells, inflammatory disease (e.g., psoriasis, nephropathy, and pneumonia), gastrointestinal tract disease (e.g., irritable bowel syndrome (TBS), inflammatory bowel disease (IBD), and abnormal pancreatic secretion), renal disease, urinary tract-associated disease (e.g., benign prostatic hyperplasia or symptoms associated with neuropathic bladder disease, spinal cord tumor, hernia of intervertebral disk, spinal canal stenosis, symptoms derived from diabetes, lower urinary tract disease (e.g., obstruction of lower urinary tract), inflammatory disease of the lower urinary tract, dysuna, and frequent urination), pancreas disease, abnormal angiogenesis-associated disease (e.g., arterial obstruction), scleroderma, brain-associated disease (e.g., cerebral infarction and cerebral hemorrhage), neuropathic pain, peripheral neuropathy, ocular disease (e.g., age-related macular degeneration (AMD), diabetic retinopathy, proliferative vitreoretinopathy (PVR), cicatricial pemphigoid, and glaucoma filtration surgery scarring).

In some embodiments, provided herein are methods of treating or preventing fibrosis, the method comprising administering to a subject in need thereof a therapeutically effective amount of a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as disclosed herein. For example, the methods can include treating renal fibrosis, pulmonary fibrosis, hepatic fibrosis, arterial fibrosis or systemic sclerosis. In some embodiments, provided herein are methods of treating pulmonary fibrosis (e.g., Idiopathic Pulmonary Fibrosis (IPF)), the method comprising administering to a subject in need thereof a therapeutically effective amount of a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is used to treat or prevent fibrosis in a subject. For example, a compound of Formula (**I**), or a pharmaceutically acceptable salt or solvate thereof (e.g., a compound of any one of Formulas **I-A**, **I-1**, **I-2**, or **I-3**, or a pharrnaceutically acceptable salt or solvate thereof), or a pharmaceutical composition as provided herein, can be used to treat fibrosis of an organ or tissue in a subject. In some embodiments, provided herein is a method for preventing a fibrosis condition in a subject, the method comprising administering to the subject at risk of developing one or more fibrosis conditions a therapeutically effective amount of a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein. For example, the subject may have been exposed to one or more environmental conditions that are known to increase the risk of fibrosis of an organ or tissue. In some embodiments, the subject has been exposed to one or more environmental conditions that are known to increase the risk of

lung, liver or kidney fibrosis. In some embodiments, the subject has a genetic predisposition of developing fibrosis of an organ or tissue. In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is administered to a subject to prevent or minimize scarring following injury. For example, the injury can include surgery.

Exemplary diseases, disorders, or conditions that involve fibrosis include, but are not limited to: lung diseases associated with fibrosis, for example, idiopathic pulmonary fibrosis, iatrogenic drug induced, occupational/environmental induced fibrosis (Farmer lung), granulomatous diseases (sarcoidosis, hypersensitivity pneumonia), collagen vascular disease (scleroderma and others), alveolar proteinosis, langerhans cell granulonmatosis, lymphangioleiomyomatosis, inherited diseases (e.g., Hermansky-Pudlak Syndrome, Tuberous sclerosis, neurofibromatosis, metabolic storage disorders, and familial interstitial lung disease), pulmonary fibrosis secondary to systemic inflammatory disease such as rheumatoid arthritis, scleroderma, lupus, cryptogenic fibrosing alveolitis, radiation induced fibrosis, chronic obstructive pulmonary disease (COPD), scleroderma, bleomycin induced pulmonary fibrosis, chronic asthma, silicosis, asbestos induced pulmonary or pleural fibrosis, acute lung injury, acute respiratory distress syndrome (ARDS), and acute respiratory distress (including bacterial pneumonia induced, trauma induced, viral pneumonia induced, ventilator induced, non-pulmonary sepsis induced, and aspiration induced). Chronic nephropathies associated with injury/tibrosis, kidney fibrosis (renal fibrosis), glomerulonephritis secondary to systemic inflammatory diseases such as lupus and scleroderma, tubulointerstitium fibrosis, glomerular nephritis, glomerular sclerosis, focal segmental, diabetes, glomerular nephritis, focal segmental glomerular sclerosis, IgA nephropathy, hypertension, allograft and Alport Syndrome; dermatological disorders, gut fibrosis, for example, scleroderma, and radiation induced gut fibrosis, liver fibrosis, for example, cirrhosis, alcohol induced liver fibrosis, nonalcoholic steatohepatitis (NASH), non-alcoholic fatty liver disease (NAFLD), toxic/drug induced liver fibrosis (e.g., hemochromatosis), biliary duct injury, primary biliary cirrhosis, infection or viral induced liver fibrosis (e.g., chronic HCV infection), inflammatory/immune disorders, and autoimmune hepatitis; head and neck fibrosis, for example, corneal scarring, e.g., LASIK (laser-assisted in situ keratomileusis), corneal transplant, and trabeculectomy; hypertrophic scarring, Duputren disease, cutaneous fibrosis, cutaneous sceroderma, keloids, e.g., burn induced or surgical; and other fibrotic diseases, e.g., sarcoidosis, scleroderma, spinal cord injury/fibrosis, myelofibrosis, vascular restenosis, atherosclerosis, arteriosclerosis, Wegener's granulomatosis, chronic lymphocytic leukemia, tumor metastasis, transplant organ rejection (e.g., Bronchiolitis obliterans), endometriosis, neonatal respiratory distress syndrome, and neuropathic pain, fibromyalgia, mixed connective tissue disease, and Peyronie's disease.

Provided herein is a method of improving lung function in a subject comprising administering a therapeutically effective amount of a compound of Formula (**I**), or a pharmaceutically acceptable salt or solvate thereof (e.g., a compound of any one of Formulas **I**-A, **I-1**, **I-2**, or **I-3,** or a pharmaceutically acceptable salt or solvate thereof), or a pharmaceutical composition as provided herein, to the subject in need thereof. In some embodiments, the subject has been diagnosed as having lung fibrosis. In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is used to treat idiopathic pulmonary fibrosis in a subject. In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is used to treat usual interstitial pneumonia in a subject.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein is used to treat diffuse parenchymal interstitial lung diseases in subject such as iatrogenic drug induced, occupational/environmental induced fibrosis (Farmer lung), granulomatous diseases (sarcoidosis, hypersensitivity pneumonia), collagen vascular disease (scleroderma and others), alveolar proteinosis, langerhans cell granulonmatosis, lymphangioleiomyomatosis, inherited diseases (e.g., Hermansky-Pudlak Syndrome, Tuberous sclerosis, neurofibromatosis, metabolic storage disorders, and familial interstitial lung disease).

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein is useful to treat post-transplant fibrosis associated with chronic rejection in a subject such as Bronchiolitis obliterans following a lung transplant.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein is useful to treat cutaneous fibrosis in a subject such as cutaneous scleroderma, Dupuytren disease, and keloids.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein is useful to treat hepatic fibrosis with or without cirrhosis in a subject. For example, toxic/drug induced (hemochromatosis), alcoholic liver disease, viral hepatitis (hepatitis B virus, hepatitis C virus,

HCV), nonalcoholic liver disease (NAFLD, NASH), and metabolic and auto-immune disease.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein is useful to treat renal fibrosis in a subject (e.g., tubulointerstitium fibrosis and glomerular sclerosis).

Further examples of diseases, disorders, or conditions as provided herein include atherosclerosis, thrombosis, heart disease, vasculitis, formation of scar tissue, restenosis, phlebitis, COPD (chronic obstructive pulmonary disease), pulmonary hypertension, pulmonary fibrosis, pulmonary inflammation, bowel adhesions, bladder fibrosis and cystitis, fibrosis of the nasal passages, sinusitis, inflammation mediated by neutrophils, and fibrosis mediated by fibroblasts.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is administered to a subject with fibrosis of an organ or tissue or with a predisposition of developing fibrosis of an organ or tissue with one or more other agents that are used to treat fibrosis. In some embodiments, the one or more agents include corticosteroids, immunosuppressants, B-cell antagonists, and uteroglobin.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is used to treat a dermatological disorder in a subject. Such dermatological disorders include, but are not limited to, proliferative or inflammatory disorders of the skin such as, atopic dermatitis, bullous disorders, collagenoses, psoriasis, scleroderma, psoriatic lesions, dermatitis, contact dermatitis, eczema, urticaria, rosacea, wound healing, scarring, hypertrophic scarring, keloids, Kawasaki Disease, rosacea, Sjogren-Larsso Syndrome, or urticaria. In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof) is used to treat systemic sclerosis.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof) is useful to treat or prevent inflammation in a subject. For example, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof) can be used in the treatment or prevention of inflammatory/immune disorders in a subject.

Examples of inflammatory/immune disorders include psoriasis, rheumatoid arthritis, vasculitis, inflammatory bowel disease, dermatitis, osteoarthritis, asthma, inflammatory muscle disease, allergic rhinitis, vaginitis, interstitial cystitis, scleroderma, eczema, allogeneic or xenogeneic transplantation (organ, bone marrow, stem cells and other cells and tissues) graft rejection, graft-versus-host disease, lupus erythematosus, inflammatory disease, type I diabetes, pulmonary fibrosis, dermatomyositis, Sjogren's syndrome, thyroiditis ( e.g ., Hashimoto's and autoimmune thyroiditis), myasthenia gravis, autoimmune hemolytic anemia, multiple sclerosis, cystic fibrosis, chronic relapsing hepatitis, primary biliary cirrhosis, allergic conjunctivitis and atopic dermatitis.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is used in the treatment of pain in a subject. In some embodiments, the pain is acute pain or chronic pain. In some embodiments, the pain is neuropathic pain.

In some embodiments, a compound of Formula (**I**), or a pharmaceutically acceptable salt or solvate thereof (e.g., a compound of any one of Formulas **I-A**, **I-1**, **I-2**, or **I-3,** or a pharmaceutically acceptable salt or solvate thereof), or a pharmaceutical composition as provided herein, is used in the treatment of fibromyalgia. Fibromyalgia is believed to stem from the formation of fibrous scar tissue in contractile (voluntary) muscles. Fibrosis binds the tissue and inhibits blood flow, resulting in pain.

In some embodiments, a compound of Formula (I), or a pharmaceutically acceptable salt or solvate thereof (e.g., a compound of any one of Formulas I-A, I-1, I-2, or I-3, or a pharmaceutically acceptable salt or solvate thereof), or a pharmaceutical composition as provided herein, is used in the treatment of cancer. In some embodiments, a compound of Formula (I), or a pharmaceutically acceptable salt or solvate thereof (e.g., a compound of any one of Formulas I-A, I-1, I-2, or I-3, or a pharmaceutically acceptable salt or solvate thereof), or a pharmaceutical composition as provided herein, is used in the treatment of malignant and benign proliferative disease. In some embodiments, a compound of Formula (I), or a pharmaceutically acceptable salt or solvate thereof (e.g., a compound of any one of Formulas I-A, I-1, I-2, or I-3, or a pharmaceutically acceptable salt or solvate thereof), or a pharmaceutical composition as provided herein, is used to prevent or reduce proliferation of tumor cells, invasion and metastasis of carcinomas, pleural mesothelioma (Yamada, Cancer Sci., 2008, 99(8), 1603-1610) or peritoneal mesothelioma, cancer pain, bone metastases (Boucharaba et al, J Clin. Invest., 2004, 114(12), 1714-1725; Boucharaba et al, Proc. Natl. acad Sci., 2006, 103(25) 9643-9648). Provided herein is a method of treating cancer in a subject, the method comprising administering to the subject a therapeutically effective amount a compound of Formula (I), or a pharmaceutically acceptable salt or solvate thereof (e.g., a compound of

any one of Formulas I-A, I-1, I-2, or I-3, or a pharmaceutically acceptable salt or solvate thereof), or a pharmaceutical composition as provided herein. In some embodiments, the methods provided herein further include administration of a second therapeutic agent, wherein the second therapeutic agent is an anti-cancer agent.

The term "cancer," as used herein refers to an abnormal growth of cells which tend to proliferate in an uncontrolled way and, in some cases, to metastasize (spread). The types of cancer include, but is not limited to, solid tumors (such as those of the bladder, bowel, brain, breast, endometrium, heart, kidney, lung, lymphatic tissue (lymphoma), ovary, pancreas or other endocrine organ (thyroid), prostate, skin (melanoma or basal cell cancer) or hematological tumors (such as the leukemias) at any stage of the disease with or without metastases.

Further non-limiting examples of cancers include, acute lymphoblastic leukemia, acute myeloid leukemia, adrenocortical carcinoma, anal cancer, appendix cancer, astrocytomas, atypical teratoid/rhabdoid tumor, basal cell carcinoma, bile duct cancer, bladder cancer, bone cancer (osteosarcoma and malignant fibrous histiocytoma), brain stem glioma, brain tumors, brain and spinal cord tumors, breast cancer, bronchial tumors, Burkitt lymphoma, cervical cancer, chronic lymphocytic leukemia, chronic myelogenous leukemia, colon cancer, colorectal cancer, craniopharyngioma, cutaneous T- Cell lymphoma, embryonal tumors, endometrial cancer, ependymoblastoma, ependymoma, esophageal cancer, ewing sarcoma family of tumors, eye cancer, retinoblastoma, gallbladder cancer, gastric (stomach) cancer, gastrointestinal carcinoid tumor, gastrointestinal stromal tumor (GIST), gastrointestinal stromal cell tumor, germ cell tumor, glioma, hairy cell leukemia, head and neck cancer, hepatocellular (liver) cancer, Hodgkin lymphoma, hypopharyngeal cancer, intraocular melanoma, islet cell tumors (endocrine pancreas), Kaposi sarcoma, kidney cancer, Langerhans cell histiocytosis, laryngeal cancer, leukemia, Acute lymphoblastic leukemia, acute myeloid leukemia, chronic lymphocytic leukemia, chronic myelogenous leukemia, hairy cell leukemia, liver cancer, non-small cell lung cancer, small cell lung cancer, Burkitt lymphoma, cutaneous T-cell lymphoma, Hodgkin lymphoma, non-Hodgkin lymphoma, lymphoma, Waldenstrom macroglobulinemia, medulloblastoma, medulloepithelioma, melanoma, mesothelioma, mouth cancer, chronic myelogenous leukemia, myeloid leukemia, multiple myeloma, nasopharyngeal cancer, neuroblastoma, non-Hodgkin lymphoma, non-small cell lung cancer, oral cancer, oropharyngeal cancer, osteosarcoma, malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, Ewing sarcoma family of tumors, sarcoma, kaposi, Sezary syndrome, skin cancer, small cell Lung cancer, small intestine cancer, soft tissue sarcoma, squamous cell carcinoma, stomach (gastric) cancer, supratentorial primitive neuroectodermal tumors, T-cell lymphoma, testicular cancer, throat cancer, thymoma and thymic carcinoma, thyroid cancer, urethral cancer, uterine cancer, uterine sarcoma, vaginal cancer, vulvar cancer, Waldenstrom macroglobulinemia, and Wilms tumor.

In some embodiments, provided herein is a method of treating an allergic disorder in a subject, the method comprising administration of a therapeutically effective amount of a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof) as provided herein. In some embodiments, a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof), is useful for the treatment of respiratory diseases, disorders or conditions in a subject. For example, a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof) can treat asthma (e.g., chronic asthma) in a subject.

The term "respiratory disease," as used herein, refers to diseases affecting the organs that are involved in breathing, such as the nose, throat, larynx, eustachian tubes, trachea, bronchi, lungs, related muscles (e.g., diaphram and intercostals), and nerves. Non-limiting examples of respiratory diseases include asthma, adult respiratory distress syndrome and allergic (extrinsic) asthma, non-allergic (intrinsic) asthma, acute severe asthma, chronic asthma, clinical asthma, nocturnal asthma, allergen-induced asthma, aspirin-sensitive asthma, exercise-induced asthma, isocapnic hyperventilation, child-onset asthma, adult-onset asthma, cough-variant asthma, occupational asthma, steroid-resistant asthma, seasonal asthma, seasonal allergic rhinitis, perennial allergic rhinitis, chronic obstructive pulmonary disease, including chronic bronchitis or emphysema, pulmonary hypertension, interstitial lung fibrosis and/or airway inflammation and cystic fibrosis, and hypoxia.

The term "asthma" as used herein refers to any disorder of the lungs characterized by variations in pulmonary gas flow associated with airway constriction of whatever cause (intrinsic, extrinsic, or both; allergic or non-allergic). The term asthma may be used with one or more adjectives to indicate cause.

Further provided herein are methods for treating or preventing chronic obstructive pulmonary disease in a subject comprising administering a therapeutically effective amount of a compound of Formula (I), or a pharmaceutically acceptable salt or solvate thereof (e.g., a compound of any one of Formulas **I-A, I-1, I-2,** or **I-3**, or a pharmaceutically

acceptable salt or solvate thereof) as provided herein. Examples of chronic obstructive pulmonary disease include, but are not limited to, chronic bronchitis or emphysema, pulmonary hypertension, interstitial lung fibrosis and/or airway inflammation, and cystic fibrosis.

In some embodiments, a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof) is useful in the treatment or prevention of a nervous system disorder in a subject. The term "nervous system disorder," as used herein, refers to conditions that alter the structure or function of the brain, spinal cord or peripheral nervous system, including but not limited to Alzheimer's Disease, cerebral edema, cerebral ischemia, stroke, multiple sclerosis, neuropathies, Parkinson's Disease, those found after blunt or surgical trauma (including post-surgical cognitive dysfunction and spinal cord or brain stem injury), as well as the neurological aspects of disorders such as degenerative disk disease and sciatica.

In some embodiments, provided herein is a method for treating or preventing a CNS disorder in a subject. Non-limiting examples of CNS disorders include multiple sclerosis, Parkinson's disease, Alzheimer's disease, stroke, cerebral ischemia, retinal ischemia, post-surgical cognitive dysfunction, migraine, peripheral neuropathy/neuropathic pain, spinal cord injury, cerebral edema and head injury.

Also provided herein are methods of treating or preventing cardiovascular disease in a subject. The term "cardiovascular disease," as used herein refers to diseases affecting the heart or blood vessels or both, including but not limited to: arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. For example, provided herein are methods for treating or preventing vasoconstriction, atherosclerosis and its sequelae myocardial ischemia, myocardial infarction, aortic aneurysm, vasculitis and stroke comprising administering a therapeutically effective amount of a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof).

In some embodiments, provided herein are methods for reducing cardiac reperfusion injury following myocardial ischemia and/or endotoxic shock comprising administering to a subject in need thereof a therapeutically effective amount of a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof).

Further provided herein are methods for reducing the constriction of blood vessels in a subject comprising administering a therapeutically effective amount of a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof). For example, methods for lowering or preventing an increase in blood pressure of a subject comprising administering a therapeutically effective amount of a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof) are provided herein.

*Combination Therapy*

In some embodiments, this disclosure contemplates both monotherapy regimens as well as combination therapy regimens.

In some embodiments, the methods provided herein can further include administering one or more additional therapies (e.g., one or more additional therapeutic agents and/or one or more therapeutic regimens) in combination with administration of the compounds provided herein.

In some embodiments, a compound of Formula **(I),** or a pharmaceutically acceptable salt or solvate thereof (e.g., a compound of any one of Formulas **I-A, I-1, I-2,** or **I-3,** or a pharmaceutically acceptable salt or solvate thereof) as provided herein can be administered in combination with one or more additional therapeutic agents.

Representative additional therapeutic agents include, but are not limited to, corticosteroids (e.g., dexamethasone or fluticasone), immunosuppresants (e.g., tacrolimus & pimecrolimus), immunosuppressants (e.g., tacrolimus & pimecrolimus), analgesics, anti-cancer agent, anti-inflammatories, chemokine receptor antagonists, bronchodilators, leukotriene receptor antagonists (e.g., montelukast or zafirlukast), leukotriene formation inhibitors, monoacylglycerol kinase inhibitors, phospholipase Ai inhibitors, phospholipase A2 inhibitors, and lysophospholipase D (lysoPLD) inhibitors, autotaxin inhibitors, decongestants, antihistamines (e.g., loratidine), mucolytics, anticholinergics, antitussives, expectorants, anti-infectives (e.g., fusidic acid, particularly for treatment of atopic dermatitis), anti-fungals (e.g., clotriazole, particularly for atopic dermatitis), anti-IgE antibody therapies (e.g., omalizumab), $\beta$-2 adrenergic agonists (e.g., albuterol or salmeterol), other PGD2 antagonists acting at other receptors such as DP antagonists, PDE4 inhibitors (e.g., cilomilast), drugs that modulate cytokine production, e.g., TACE inhibitors, drugs that modulate activity of Th2 cytokines

IL-4 & IL-5 (e.g., blocking monoclonal antibodies & soluble receptors), PPARy agonists (e.g., rosiglitazone and pioglitazone), 5-lipoxygenase inhibitors (e.g., zileuton), pirfenidone, nintedanib, thalidomide, carlumab, FG-3019, fresolimumab, interferon alpha, lecithinized superoxide dismutase, simtuzumab, tanzisertib, tralokinumab, hu3G9, AM-152, IFN-gamma-lb, IW-001, PRM-151, PXS-25, pentoxifylline/N-acetyl-cysteine, pentoxifylline/vitamin E, salbutamol sulfate, [Sar9,Met(02) 11 J-Substance P, pentoxifylline, mercaptamine bitartrate, obeticholic acid, aramchol, GFT-505, eicosapentyl ethyl ester, metformin, metreleptin, muromonab-CD3, oltipraz, IMM-124-E, MK-4074, PX-102, RO-5093151, angiotensin converting enzyme (ACE) inhibitors, ramipril, angiotensin II receptor subtype 1 (AT1) antagonists, irbesartan, antiarrhythmic, dronedarone, peroxisome proliferator-activated receptor-alpha (PPAR-α) activators, peroxisome proliferator-activated receptor-gamma (PPAR-y) activators, pioglitazone, rosiglitazone, prostanoids, endothelin receptor antagonists, bosentan, elastase inhibitors, calcium antagonists, beta blockers, diuretics, aldosterone receptor antagonists, eplerenone, renin inhibitors, rho kinase inhibitors, soluble guanylate cyclase (sGC) activators, sGC sensitizers, phosphodiesterase (PDE) inhibitors, phosphodiesterase type 5 (PDE5) inhibitors, NO donors, digitalis drugs, angiotensin converting enzyme/neutral endopeptidase (ACE/NEP) inhibitors, statins, bile acid reuptake inhibitors, platelet derived growth factor (PDGF) receptor antagonists, vasopressin antagonists, aquaretics, sodium hydrogen exchanger subtype 1 (NHE1) inhibitors, factor I I/factor Ma antagonists, factor IX/factor IXa antagonists, factor X/factor Xa antagonists, factor XllI/factor XIIIa antagonists, anticoagulants, antithrombotics, platelet inhibitors, profibrinolytics, thrombin-activatable fibrinolysis inhibitors (TAFI), plasminogen activator inhibitor-1 (PAI 1), coumarins, heparins, thromboxane antagonists, serotonin antagonists, cyclooxygenase inhibitors, acetylsalicylic acid, therapeutic antibodies, glycoprotein IIb/IIIa (GPIIb/IIIa) antagonists including abciximab, chymase inhibitors, cytostatics, taxanes, paclitaxel, docetaxel, aromatase inhibitors, estrogen receptor antagonists, selective estrogen receptor modulators (SERM), tyrosine kinase inhibitors, imatinib, receptor tyrosine kinase inhibitors, RAF kinase inhibitors, p38 mitogen-activated protein kinase (p38 MAPK) inhibitors, pirfenidone, multi-kinase inhibitors, and sorafenib.

In any of the previous embodiments, the compound(s) of Formula **(I)** (e.g., a compound of Formulas **I-A, I-1, I-2,** or **I-3),** or a pharmaceutically acceptable salt or solvate thereof, and the additional agent(s) can be administered simultaneously or sequentially. For example, the agents can be formulated into a single pharmaceutical composition or may be administered together as separate formulations.

## EXAMPLES

The invention is further described in the following examples, which do not limit the scope of the invention described in the claims.

**General information:** All evaporations were carried out *in vacuo* with a rotary evaporator. Analytical samples were dried *in vacuo* (1-5 mmHg) at rt. Thin layer chromatography (TLC) was performed on silica gel plates, spots were visualized by UV light (214 and 254 nm). Purification by column and flash chromatography was carried out using silica gel (100-200 mesh). Solvent systems were reported as mixtures by volume. NMR spectra were recorded on a Bruker 400 or Varian (400 MHz) spectrometer. $^1H$ chemical shifts are reported in δ values in ppm with the deuterated solvent as the internal standard. Data are reported as follows: chemical shift, multiplicity (s = singlet, d = doublet, t = triplet, q = quartet, br = broad, m = multiplet), coupling constant (Hz), integration. LCMS spectra were obtained on SHIMADZU LC20-MS2020 or Agilent 1260 series 6125B mass spectrometer or Agilent 1200 series, 6110 or 6120 mass spectrometer with electrospray ionization and excepted as otherwise indicated.

### Example 1

2-((2S, 3R)-2-((2, 3-dihydro-1H-inden-2-yl) oxy)-3-(3, 5-dimethoxy-4-methylphenyl)-3-hydroxypropyl)-2H-indazole-7-carboxylic acid **(Compound 113a)**

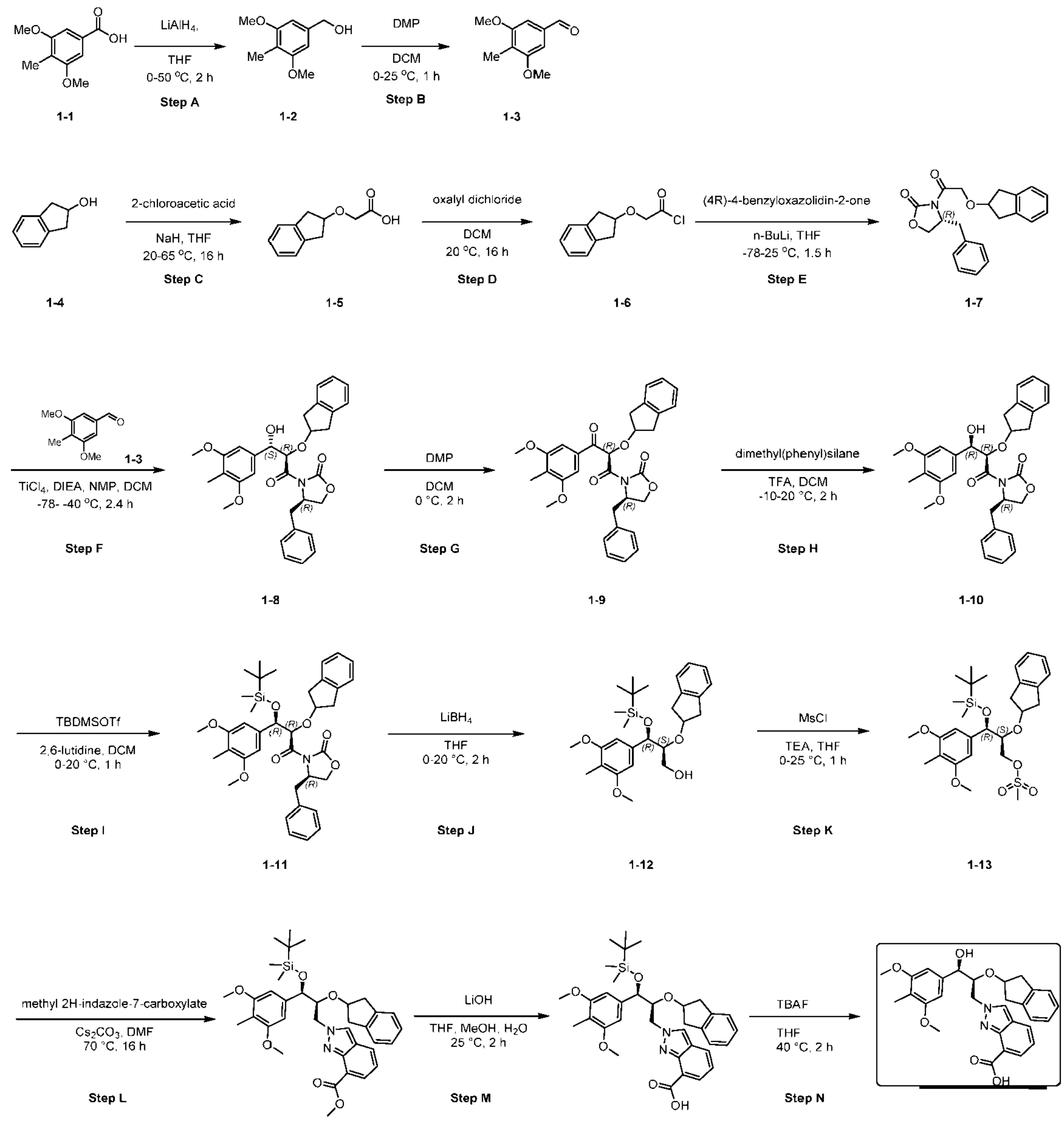


1-1, 1-2, 1-3, 1-4, 1-5, 1-6, 1-7, 1-8, 1-9, 1-10, 1-11, 1-12, 1-13, 1-14, 1-15, 113a

Step A (3, 5-dimethoxy-4-methylphenyl) methanol

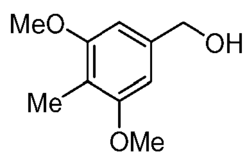

To a solution of 3, 5-dimethoxy-4-methyl-benzoic acid (10.0 g, 50.9 mmol) in THF (100 mL) was added $LiAlH_4$ (1.0 M in THF, 56.1 mL) at 0 °C. The mixture was stirred at 50 °C for 2 hrs. The mixture was quenched by water (50 mL) and extracted with EtOAc (30 mL*3). The combined organic layer was dried over anhydrous $Na_2SO_4$, filtered and the filtrate was concentrated in vacuum to give (3,5-dimethoxy-4-methyl-phenyl) methanol (7.31 g, crude) as a brown solid. **$^1$H NMR** (400 MHz, $CDCl_3$) $\delta$ 6.56 (s, 2H), 4.68 (d, $J$ = 4.0 Hz, 2H), 3.84 (s, 6H), 2.09 (s, 3H), 1.67 (t, $J$ = 4.2 Hz, 1H).

Step B 3, 5-dimethoxy-4-methylbenzaldehyde

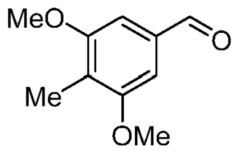

To a solution of (3, 5-dimethoxy-4-methyl-phenyl) methanol (7.31 g, crude) in DCM (70 mL) was added DMP (20.4 g, 48.1 mmol) at 0 °C. The mixture was stirred at 25 °C for 1 hr. To the mixture was added *sat. aq.* $NaHCO_3$ (100 mL) and *sat. aq.* $Na_2S_2O_3$ (100 mL). The resulting mixture was stirred at 0 °C for 30 mins and then filtrated. The filtrate was separated. The aqueous layer was extracted with DCM (30 mL*3). The combined organic layer was concentrated in vacuum to give 3, 5-dimethoxy-4-methyl-benzaldehyde (7.17 g, 99.2% yield) as brown solid. **$^1$H NMR** (400 MHz, $CDCl_3$) $\delta$ 9.91 (s, 1H), 7.06 (s, 2H), 3.90 (s, 6H), 2.16 (s, 3H).

Step C 2-((2,3-dihydro-1H-inden-2-yl)oxy)acetic acid

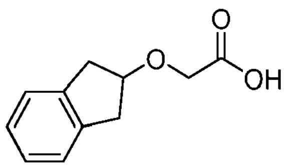

To a solution of indan-2-ol (30.0 g, 223 mmol) in THF (300 mL) was added NaH (17.9 g, 447 mmol). The reaction was stirred at 60 °C for 0.5 hr. The mixture was cooled to 20 °C, 2-chloroacetic acid (24.5 g, 259 mmol) was added to the mixture. The reaction was stirred at 65 °C for 16 hrs. After cooling, the mixture was quenched by water (50 mL) and the mixture was extracted with EtOAc (30 mL*3). The aqueous layer was acidified by 2N HCl to *p*H = 5 and extracted with EtOAc (30 mL*3). The combined organic layer was dried over anhydrous $Na_2SO_4$, filtered and the filtrate was concentrated in vacuum to give 2-indan-2-yloxyacetic acid (36.5 g, crude) as brown solid. **$^1$H NMR** (400MHz, DMSO-*d6*) $\delta$ 7.25 - 7.18 (m, 2H), 7.16 - 7.09 (m, 2H), 4.44 - 4.33 (m, 1H), 4.04 - 4.03 (s, 2H), 3.10 (dd, *J* = 16.4, 4.0 Hz, 2H), 2.91 (dd, *J* = 16.8, 3.6 Hz, 2H).

Step D 2-((2,3-dihydro-1H-inden-2-yl)oxy)acetyl chloride

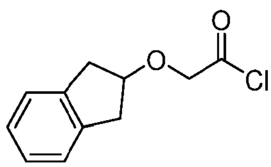

To a solution of 2-indan-2-yloxyacetic acid (36.5 g, crude) in DCM (400 mL) was added DMF (693 mg, 9.49 mmol), followed by oxalyl dichloride (72.3 g, 569 mmol). The reaction mixture was stirred at 20 °C for 16 hrs. The mixture was concentrated in vacuum to give 2-indan-2-yloxyacetyl chloride (41.6 g, crude) as brown oil which used in next step directly.

Step E (R)-4-benzyl-3-(2-((2,3-dihydro-1H-inden-2-yl)oxy)acetyl)oxazolidin-2-one

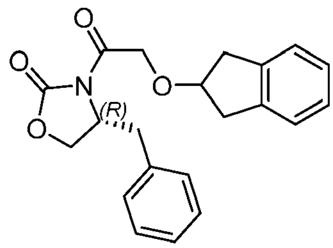

To a solution of (4R)-4-benzyloxazolidin-2-one (42.0 g, 237 mmol) in THF (400 mL) was added *n*-BuLi (2.5 M, 237 mmol) at -78 °C under $N_2$. 2-indan-2-yloxyacetyl chloride (41.6 g, crude) in THF (100 mL) was added to the reaction mixture at -78 °C and the mixture was stirred at 25 °C for 1.5 hrs. The mixture was quenched by water (400 mL) and extracted with EtOAc (80 mL*3). The combined organic layer was dried over anhydrous $Na_2SO_4$, filtered and the filtrate was concentrated in vacuum to give crude (73.5 g) as brown oil. The residue was purified by flash chromatography (PE/EtOAc = 1/1) to give (R)-4-benzyl-3-(2-((2, 3-dihydro-1H-inden-2-yl) oxy)acetyl)oxazolidin-2-one (48.6 g, 62.1% yield over 3 steps) as brown oil. **$^1$H NMR** (400MHz, $CDCl_3$) $\delta$ 7.38 - 7.27 (m, 3H), 7.25 - 7.14 (m, 6H), 4.75 (d, *J*= 1.6 Hz, 2H), 4.74 - 4.66 (m, 1H), 4.52 - 4.49 (m, 1H), 4.33 - 4.21 (m, 2H), 3.38 - 3.30 (m, 1H), 3.30 - 3.20 (m, 2H), 3.17 - 3.08 (m, 2H), 2.85 - 2.79 (m, 1H).

Step F (R)-4-benzyl-3-((2R,3S)-2-((2,3-dihydro-1H-inden-2-yl)oxy)-3-(3,5-dimethoxy-4-methylphenyl)-3-hydroxypropanoyl) oxazolidin-2-one

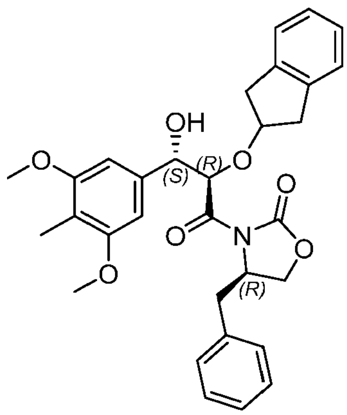

To a solution of (R)-4-benzyl-3-(2-((2,3-dihydro-1H-inden-2-yl)oxy)acetyl)oxazolidin-2-one (12.1 g, 34.4 mmol) in DCM (200 mL) was added $TiCl_4$ (6.86 g, 36.2 mmol) dropwise at -70 °C under $N_2$. The solution was stirred at - 70 °C for 15 mins. To the solution was added DIEA (11.1 g, 86.1 mmol,). The solution was stirred at -70 °C for 1 hr. To the solution was added NMP (3.41 g, 34.4 mmol) at -70 °C. The solution was stirred at -70 °C for 10 mins. To the solution was added 3,5-dimethoxy-4-methyl-benzaldehyde (6.21 g, 34.4 mmol). The mixture was stirred at -70 °C for 1 hr, and then stirred at -40 °C for 1 hr. The mixture was quenched by water (200 mL) and extracted with EtOAc (60 mL*3). The combined organic layer was dried over anhydrous $Na_2SO_4$, filtered and the filtrate was concentrated in vacuum to give crude (23.0 g). The residue was purified by flash chromatography (PE/EtOAc = 1/1) to give (R)-4-benzyl-3-((2R,3S)-2-((2,3-dihydro-1H-inden-2-yl) oxy)-3-(3,5-dimethoxy-4-methylphenyl)-3-hydroxypropanoyl)oxazolidin-2-one (12.0 g, 61.8% yield) as yellow oil. **$^1$H NMR** (400MHz, $CDCl_3$) $\delta$ 7.29 - 7.17 (m, 4H), 7.16 - 6.98 (m, 5H), 6.45 (s, 2H), 5.46 (d, *J* = 5.2 Hz, 1H), 4.76 (t, *J* = 4.8 Hz, 1H), 4.32 - 4.29 (m, 1H), 4.25 - 4.21 (m, 1H), 3.98 (dd, *J* = 8.8, 4.0 Hz, 1H), 3.65 (s, 6H), 3.25 - 3.17 (m, 1H), 3.17 - 3.08 (m, 1H), 3.07 - 2.96 (m, 2H), 2.95 - 2.94 (m, 1H), 2.79 - 2.68 (m, 2H).

Step G (2R)-1-[(4R)-4-benzyl-2-oxo-oxazolidin-3-yl]-3-(3,5-dimethoxy-4-methyl-phenyl)-2-indan-2-yloxy-propane-1,3-dione

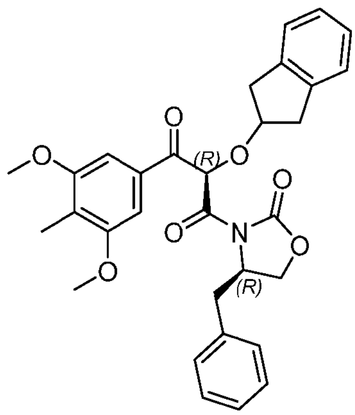

To a solution of (R)-4-benzyl-3-((2R,3S)-2-((2,3-dihydro-1H-inden-2-yl)oxy)-3-(3,5-dimethoxy-4-methylphenyl)-3-hydroxypropanoyl)oxazolidin-2-one (7.00 g, 13.2 mmol) in DCM (70 mL) was added DMP (6.70 g, 15.8 mmol). The mixture was stirred at 0 °C for 2 hrs. To the mixture was added *sat. aq.* $NaHCO_3$ and *sat. aq.* $Na_2S_2O_3$ (1:1, 40 mL) and stirred at 0 °C for 30 mins. Then the organic layer was separated. The aqueous layer was extracted with DCM (20 mL*3). The combined organic layer was dried over anhydrous $Na_2SO_4$ and filtered. The filtrate was concentrated in vacuo and the residue was purified by flash chromatography (PE/EtOAc = 1/1) to give (2R)-1-[(4R)-4-benzyl-2-oxo-oxazolidin-3-yl]-3-(3,5-dimethoxy-4-methyl-phenyl)-2-indan-2-yloxy-propane-1,3-dione (4.84 g, 69.4% yield) as white solid. **LC-MS:** m/z 530.2 $(M+H)^+$.

Step H (4R)-4-benzyl-3-[(2R,3R)-3-(3,5-dimethoxy-4-methyl-phenyl)-3-hydroxy-2-indan-2-yloxy-propanoyl]oxazolidin-2-one

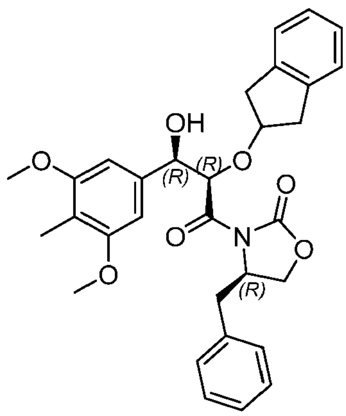

To a solution of (2R)-1-[(4R)-4-benzyl-2-oxo-oxazolidin-3-yl]-3-(3,5-dimethoxy-4-methyl-phenyl)-2-indan-2-yloxy-propane-1,3-dione (4.84 g, 9.14 mmol) in DCM (50 mL) was added TFA (77.0 g, 675 mmol) at 20 °C and then dimethyl(phenyl)silane (3.74 g, 27.4 mmol) was added to the mixture at -10 °C. The resulting mixture was stirred at -10 °C for 2 hrs. Then the *p*H value was adjusted to 7-8 with *sat. aq.* $NaHCO_3$ solution and the mixture was extracted with DCM (30 mL*3). The combined organic layers were dried over anhydrous $Na_2SO_4$ and filtered. The filtrate was concentrated in vacuo and the residue was purified by flash chromatography (PE/EtOAc = 2/1) to give (4R)-4-benzyl-3-[(2R,3R)-3-(3,5-dimethoxy-4-methyl-phenyl)-3-hydroxy-2-indan-2-yloxy-propanoyl]oxazolidin-2-one (4.51 g, 92.9% yield) as yellow oil. **$^1$H NMR** (400 MHz, $CDCl_3$) $\delta$ 7.41 - 7.27 (m, 4H), 7.24 - 7.06 (m, 5H), 6.59 (s, 2H), 5.76 (d, *J* = 6.8 Hz, 1H), 4.94 - 4.91 (m, 1H), 4.47 - 4.40 (m, 1H), 4.40 - 4.32 (m, 1H), 4.09 (dd, *J* = 6.4, 2.4 Hz, 1H), 3.83 - 3.77 (m, 1H), 3.76 (s, 6H), 3.32 - 3.28 (m, 1H), 3.23 - 3.13 (m, 1H), 3.08 - 2.98 (m, 2H), 2.86 - 2.72 (m, 3H), 2.06 (s, 3H). **LC-MS:** m/z 577.2 $(M+2Na)^+$.

Step I (R)-4-benzyl-3-((2R,3R)-3-((tert-butyldimethylsilyl)oxy)-2-((2,3-dihydro-1H-inden-2-yl) oxy)-3-(3,5-dimethoxy-4-methylphenyl)propanoyl)oxazolidin-2-one

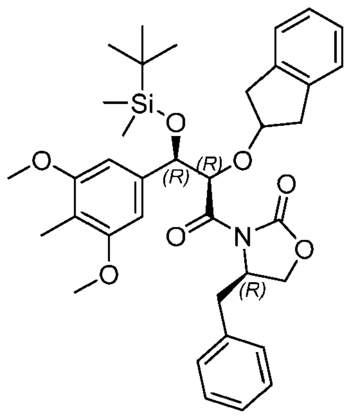

To the solution of the (4R)-4-benzyl-3-[(2R,3R)-3-(3,5-dimethoxy-4-methyl-phenyl)-3-hydroxy-2-indan-2-yloxy-propanoyl]oxazolidin-2-one (4.51 g, 8.48 mmol) in DCM (18 mL) were added TBDMSOTf (4.49 g, 16.9 mmol) and 2,6-lutidine (1.82 g, 16.7 mmol) at 0 °C. The reaction mixture was warmed to 20 °C for 1 hr. The mixture was diluted with water (30 mL) and extracted with DCM (20 mL*3). The combined organic layer was dried over anhydrous $Na_2SO_4$ and filtered. The filtrate was concentrated in vacuo and the residue was purified by flash chromatography (PE/EtOAc = 3/1) to give (4R)-4-benzyl-3-[(2R,3R)-3-[tert-butyl(dimethyl)silyl]oxy-3-(3,5-dimethoxy-4-methyl-phenyl)-2-indan-2-yloxy-propanoyl]oxazolidin-2-one (5.11 g, 84.9% yield) as light yellow oil. **$^1$H NMR** (400MHz, $CDCl_3$) $\delta$ 7.49 - 7.28 (m, 4H), 7.22 - 7.05 (m, 3H), 6.99 - 6.87 (m, 1H), 6.62 (s, 2H), 5.64 (d, *J* = 8.4 Hz, 1H), 4.86 - 4.75 (m, 1H), 4.71 (d, J = 8.8 Hz, 1H), 4.33 - 4.23 (m, 2H), 4.18 - 4.09 (m, 1H), 3.73 (s, 6H), 3.42 - 3.38 (m, 1H), 3.16 -3.06 (m, 1H), 3.01 - 2.90 (m, 2H), 2.93 - 2.79 (m, 1H), 2.40 - 2.35 (m, 1H), 2.14 (s, 3H), 0.86 (s, 9H), 0.00 (s, 3H), -0.17 (s, 3H).

Step J (2S, 3R)-3-((tert-butyldimethylsilyl)oxy)-2-((2,3-dihydro-1H-inden-2-yl)oxy)-3-(3,5-dimethoxy-4-methylphenyl) propan-1-ol

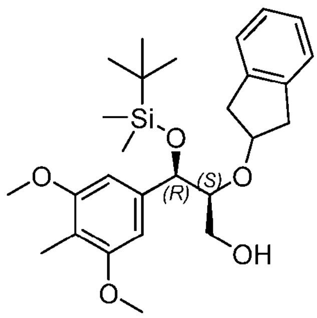

To a solution of (4R)-4-benzyl-3-[(2R,3R)-3-[tert-butyl(dimethyl)silyl]oxy-3-(3,5-dimethoxy-4-methyl-phenyl)-2-indan-2-yloxy-propanoyl]oxazolidin-2-one (5.11 g, 7.91 mmol) in THF (50 mL) was added $LiBH_4$ (258 mg, 11.9 mmol) at 0 °C. The mixture was stirred at 20 °C for 2 hrs. The mixture was quenched by water (40 mL) and extracted with EtOAc (30 mL*3). The combined organic layer was dried over anhydrous $Na_2SO_4$, filtered and the filtrate was concentrated in vacuum to give (2S,3R)-3-[tert-butyl(dimethyl)silyl]oxy-3-(3,5-dimethoxy-4-methyl-phenyl)-2-indan-2-yloxy-propan-1-ol (4.23 g, crude) as light yellow oil. **LC-MS:** m/z 495.2 $(M+Na)^+$.

Step K (2S,3R)-3-((tert-butyldimethylsilyl)oxy)-2-((2,3-dihydro-1H-inden-2-yl)oxy)-3-(3,5-dimethoxy-4-methylphenyl) propyl methanesulfonate

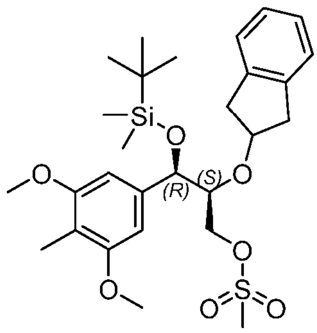

To a solution of (2S,3R)-3-[tert-butyl(dimethyl)silyl]oxy-3-(3,5-dimethoxy-4-methyl-phenyl)-2-indan-2-yloxy-propan-1-ol (4.23 g, crude) in DCM (40 mL) was added TEA (1.36 g, 13.4 mmol) and MsCl (1.70 g, 14.8 mmol) at 0 °C. The mixture was stirred at 25 °C for 1 hr. The mixture was quenched by *sat.* $NaHCO_3$ (20 mL) and extracted with DCM (15 mL*3). The combined organic layer was dried over anhydrous $Na_2SO_4$ and filtered. The filtrate was concentrated in vacuo and the residue was purified by flash chromatography (PE/EtOAc = 3/1) to give [(2S,3R)-3-[tert-butyl(dimethyl)silyl]oxy-3-(3,5-dimethoxy-4-methyl-phenyl)-2-indan-2-yloxy-propyl]methanesulfonate (3.13 g, 5.61 mmol) as colorless oil. **$^1$H NMR** (400 MHz, $CDCl_3$) $\delta$ 7.23 - 7.17 (m, 1H), 7.16 - 7.08 (m, 2H), 7.05 - 6.98 (m, 1H), 6.48 (s, 2H), 4.59 (d, *J* = 6.4 Hz, 1H), 4.44 (dd, *J* = 8.4, 2.4 Hz, 1H), 4.31 - 4.27 (m, 1H), 4.19 - 4.14 (m, 1H), 3.74 (s, 6H), 3.70 - 3.63 (m, 1H), 3.07 - 2.94 (m, 2H), 2.85 - 2.80 (m, 1H), 2.75 (s, 3H), 2.50 (d, *J* = 2.8 Hz, 1H), 2.09 (s, 3H), 0.96 - 0.86 (s, 9H), 0.06 (s, 3H), -0.14 (s, 3H).

Step L Methyl 2-[(2S,3R)-3-[tert-butyl(dimethyl)silyl]oxy-3-(3,5-dimethoxy-4-methyl-phenyl)-2-indan-2-yloxy-propyl]indazole-7-carboxylate

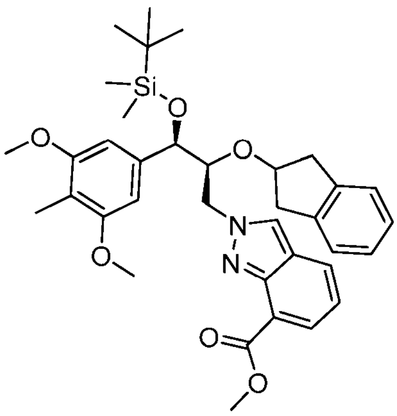

To a solution of [(2S,3R)-3-[tert-butyl(dimethyl)silyl]oxy-3-(3,5-dimethoxy-4-methyl-phenyl)-2-indan-2-yloxy-propyl] methanesulfonate (400 mg, 0.726 mmol) in DMF (4 mL) was added $Cs_2CO_3$ (710 mg, 2.18 mmol) and methyl 2H-indazole-7-carboxylate (154 mg, 0.871 mmol). The mixture was stirred at 70 °C for 16 hrs. The mixture was diluted with water (20 mL) and extracted with EtOAc (15 mL*3). The combined organic layer was dried over anhydrous $Na_2SO_4$ and filtered. The filtrate was concentrated in vacuo and the residue was purified by flash chromatography (PE/EtOAc = 1/1) to give methyl 2-[(2S,3R)-3-[tert-butyl(dimethyl)silyl]oxy-3-(3,5-dimethoxy-4-methyl-phenyl)-2-indan-2-yloxy-propyl]indazole-7-carboxylate (141 mg, 30.8% yield) as colorless oil. **$^1$H NMR** (400MHz, $CDCl_3$) $\delta$ 8.09 (d, *J* = 6.4 Hz, 1H), 7.84 (s, 1H), 7.75 (d, *J* = 8.0 Hz, 1H), 7.15 - 7.09 (m, 1H), 7.08 - 7.02 (m, 1H), 7.01 - 6.93 (m, 2H), 6.57 (m, 3H), 5.05 - 4.92 (m, 1H), 4.66 (d, *J* = 6.0 Hz, 1H), 4.27 - 4.12 (m, 2H), 4.02 (s, 3H), 3.78 (s, 6H), 3.56 - 3.49 (m, 1H), 2.69 - 2.63 (m, 1H), 2.53 - 2.36 (m, 2H), 2.10 (s, 3H), 1.96-1.86 (m, 1H), 0.94 (s, 9H), 0.08 (s, 3H), -0.11 (s, 3H).

Step M 2-[(2S, 3R)-3-[tert-butyl (dimethyl) silyl] oxy-3-(3, 5-dimethoxy-4-methyl-phenyl)-2-indan-2-yloxy-propyl]indazole-7-carboxylic acid

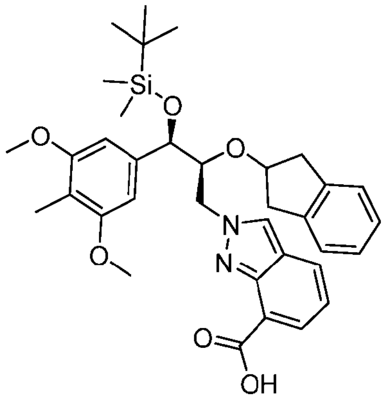

To a solution of methyl 2-[(2S, 3R)-3-[tert-butyl (dimethyl) silyl] oxy-3-(3, 5-dimethoxy-4-methyl-phenyl)-2-indan-2-yloxy-propyl] indazole-7-carboxylate (120 mg, 0.190 mmol) in THF (1 mL), MeOH (1 mL) and $H_2O$ (1 mL) was added LiOH.$H_2O$ (24 mg, 0.571 mmol). The mixture was stirred at 25 °C for 2 hrs. The mixture was concentrated in vacuum. The residue was diluted with water (10 mL) and extracted with EtOAc (10 mL*3). The combined organic layer was dried over anhydrous $Na_2SO_4$, filtered and the filtrate was concentrated in vacuum to give 2-[(2S, 3R)-3-[tert-butyl (dimethyl) silyl] oxy-3-(3, 5-dimethoxy-4-methyl-phenyl)-2-indan-2-yloxy-propyl] indazole-7-carboxylic acid (91 mg, crude) as white solid. **LC-MS:** m/z 617.3 $(M+H)^+$.

Step N 2-[(2S,3R)-3-(3,5-dimethoxy-4-methyl-phenyl)-3-hydroxy-2-indan-2-yloxy-propyl]indazole-7-carboxylic acid **(Compound 113a)**

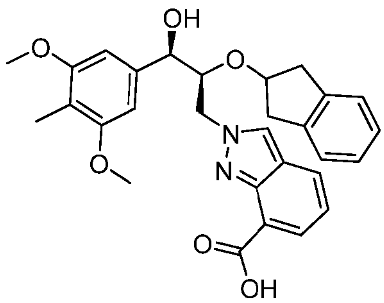

To a solution of 2-[(2S, 3R)-3-[tert-butyl (dimethyl) silyl] oxy-3-(3, 5-dimethoxy-4-methyl-phenyl)-2-indan-2-yloxy-propyl] indazole-7-carboxylic acid (91 mg, 0.147 mmol) in THF (1 mL) was added TBAF (1 M in THF, 0.295 mL). The mixture was stirred at 40 °C for 2 hrs. The mixture was concentrated in vacuum to give crude and the residue was diluted with water (10 mL) and extracted with EtOAc (10 mL*3). The combined organic layer was dried over anhydrous $Na_2SO_4$ and filtered. The filtrate was concentrated in vacuo and the residue was purified by Prep-HPLC (column: Phenomenex Gemini-NX C18 75*30 mm*3 um; mobile phase: [water (0.225% FA)-ACN]; B%: 35%-75%, 9 min). The fraction was dried by lyophilization to give 2-[(2S, 3R)-3-(3,5-dimethoxy-4-methyl-phenyl)-3-hydroxy-2-indan-2-yloxy-propyl]indazole -7-carboxylic acid (32.71 mg, 34.2% yield for two steps)(Compound 113a) as a white solid. **$^1$H NMR** (400MHz, DMSO-*d6*) $\delta$ 8.30 (s, 1H), 7.99 - 7.74 (m, 2H), 7.14 (t, *J* = 7.2 Hz, 1H), 7.04 - 6.96 (m, 1H), 6.95 - 6.88 (m, 2H), 6.67 (s, 2H), 6.54 (d, *J* = 7.2 Hz, 1H), 5.80 (br d, *J* = 2.8 Hz, 1H), 4.77 - 4.74 (m, 1H), 4.58 - 4.54 (m, 1H), 4.45 - 4.31 (m, 1H), 3.99 - 3.96 (m, 1H), 3.71 (s, 6H), 3.68 - 3.65 (m, 1H), 2.67 - 2.61 (m, 1H), 2.49 - 2.38 (m, 2H), 1.99 (s, 3H), 1.89 (br d, *J* = 14.8 Hz, 1H). **LC-MS:** m/z 503.2 $(M+H)^+$.

**Example 2**

2-(1-((2S,3R)-2-((2,3-dihydro-1H-inden-2-yl)oxy)-3-(3,5-dimethoxy-4-methylphenyl)-3-hydroxypropyl)-4-(methoxycarbonyl)-1H-pyrrol-3-yl)acetic acid **(Compound 103a)**

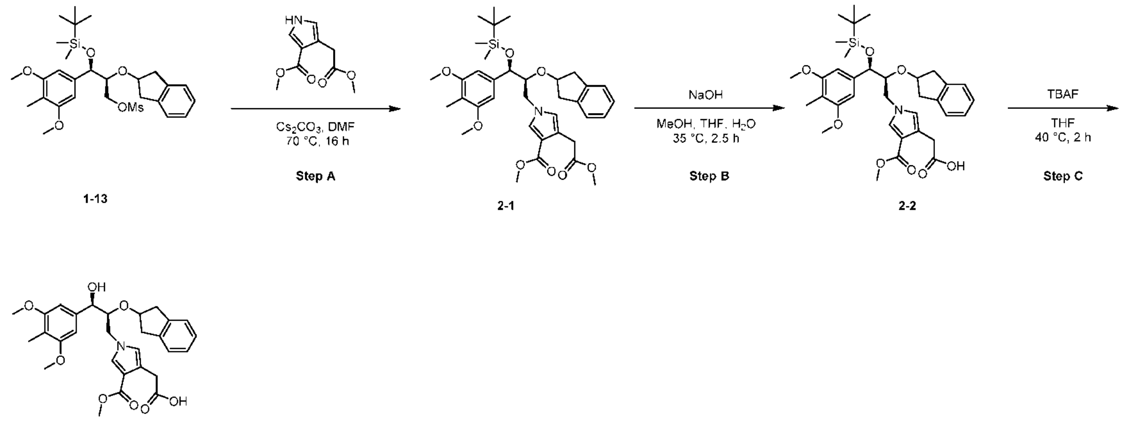


1-13

2-1

2-2

103a

Step A methyl 1-[(2S, 3R)-3-[tert-butyl (dimethyl) silyl] oxy-3-(3,5-dimethoxy-4-methyl-phenyl)-2-indan-2-yloxy-propyl]-4-(2-methoxy-2-oxo-ethyl) pyrrole-3-carboxylate

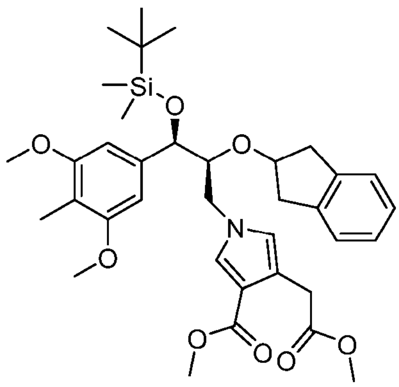

To a solution of [(2S,3R)-3-[tert-butyl(dimethyl)silyl]oxy-3-(3,5-dimethoxy-4-methyl-phenyl)-2-indan-2-yloxy-propyl] methanesulfonate (122 mg, 0.221 mmol) in DMF (2 mL) was added $Cs_2CO_3$ (216 mg, 0.665 mmol) and methyl 4-(2-methoxy-2-oxo-ethyl)-1H-pyrrole-3-carboxylate (52.4 mg, 0.266 mmol). The mixture was stirred at 70 °C for 16 hrs. The mixture was diluted with water (15 mL) and extracted with EtOAc (10 mL*3). The combined organic layer was dried over anhydrous Na2SO4 and filtered. The filtrate was concentrated in vacuo and the residue was purified by flash chromatography (PE/EtOAc = 3/1) to give methyl 1-[(2S,3R)-3-[tert-butyl(dimethyl)silyl]oxy-3-(3,5-dimethoxy-4-methyl-phenyl)-2-indan-2-yloxy-propyl]-4-(2-methoxy-2-oxo-ethyl)pyrrole-3-carboxylate (100 mg, 69.2% yield) as colorless oil. **$^1$H NMR** (400 MHz, $CDCl_3$) $\delta$ 7.19 (d, $J$ = 2.4 Hz, 1H), 7.10 - 7.05 (m, 2H), 7.03 - 6.94 (m, 2H), 6.54 (d, $J$ = 2.4 Hz, 1H), 6.49 (s, 2H), 4.78 (br. s, 1H), 4.51 (d, $J$ = 6.4 Hz, 1H), 4.21 (d, $J$ = 14.0 Hz, 1H), 3.81 - 3.71 (m, 10H), 3.67 (s, 3H), 3.59 - 3.50 (m, 2H), 2.73 - 2.61 (m, 2H), 2.40 (d, $J$ = 2.8 Hz, 1H), 2.27 (d, $J$ = 2.8 Hz, 1H), 2.10 (s, 3H), 0.93 (s, 9H), 0.05 (s, 3H), -0.14 (s, 3H).

Step B 2-[1-[(2S,3R)-3-[tert-butyl(dimethyl)silyl]oxy-3-(3,5-dimethoxy-4-methyl-phenyl)-2-indan-2-yloxy-propyl]-4-methoxycarbonyl-pyrrol-3-yl]acetic acid

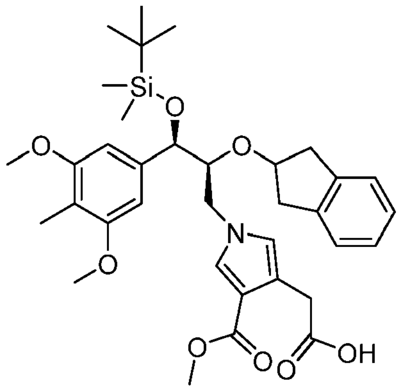

To a solution of methyl 1-[(2S,3R)-3-[tert-butyl(dimethyl)silyl]oxy-3-(3,5-dimethoxy-4-methyl-phenyl)-2-indan-2-yloxy-propyl]-4-(2-methoxy-2-oxo-ethyl)pyrrole-3-carboxylate (80 mg, 0.123 mmol) in THF (0.8 mL) and MeOH (0.8 mL) was added NaOH (1 M, 0.438 mL). The mixture was stirred at 35 °C for 2.5 hrs. The *p*H value was adjusted to 2-3 with 1 M *aq.* HCl. The mixture was diluted with water (20 mL) and then extracted with EtOAc (15 mL*3). The combined organics were dried over anhydrous $Na_2SO_4$, filtered and concentrated under reduced pressure to give 2-[1-[(2S,3R)-3-[tert-butyl(dimethyl)silyl]oxy-3-(3,5-dimethoxy-4-methyl-phenyl)-2-indan-2-yloxy-propyl]-4-methoxycarbonyl-pyrrol-3-yl]acetic acid (83 mg, crude) as a yellow oil. **LCMS:** m/z 660.2 $(M+Na)^+$.

Step C 2-[1-[(2S, 3R)-3-(3, 5-dimethoxy-4-methyl-phenyl)-3-hydroxy-2-indan-2-yloxy-propyl]-4-methoxycarbonyl-pyrrol-3-yl]acetic acid **(Compound 103a)**

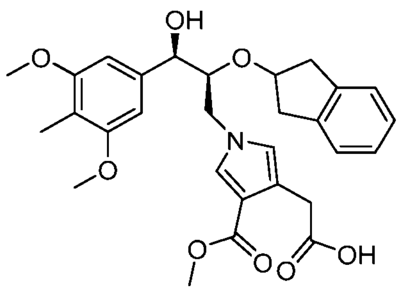

To a solution of 2-[1-[(2S,3R)-3-[tert-butyl(dimethyl)silyl]oxy-3-(3,5-dimethoxy-4-methyl-phenyl)-2-indan-2-yloxy-propyl]-4-methoxycarbonyl-pyrrol-3-yl]acetic acid (106 mg, crude) in THF (2 mL) was added TBAF (1 M, 0.332 mL). The mixture was stirred at 40 °C for 2 hrs. Then the mixture was diluted with water (15 mL) and extracted with EtOAc (10 mL*3). The combined organic layer was dried over anhydrous $Na_2SO_4$ and filtered. The filtrate was concentrated in vacuo and the residue was purified by Prep-HPLC (column: Boston Prime C18 150* 30 mm* 5 um; mobile phase: [water (0.05% ammonia hydroxide v/v)-ACN]; B%:17%-57%, 9 min) to give 2-[1-[(2S, 3R)-3-(3, 5-dimethoxy-4-methyl-phenyl)-3-hydroxy-2-indan-2-yloxy-propyl]-4-methoxycarbonyl-pyrrol-3-yl] acetic acid (Compound 103a) (10.64 mg, 9.2% yield over 2 steps) as a white solid. **$^1$H NMR** (400 MHz, $CD_3OD$) $\delta$ 7.25 (s 1H), 7.06 - 6.98 (m, 3H), 6.90 - 6.82 (m, 1H), 6.66 (s, 1H), 6.56 (s, 2H), 4.35 (d, *J* = 8.0 Hz, 1H), 4.30 (d, *J* = 11.6 Hz, 1H), 3.96 -3.91 (m, 1H), 3.82 - 3.75 (m, 1H), 3.72 (s, 3H), 3.69 (s, 6H), 3.67 - 3.59 (m, 3H), 2.78 (dd, *J* = 16.0, 8.0 Hz ,1H), 2.62 (dd, *J* = 16.0, 4.0 Hz, 1H), 2.49 - 2.41 (m, 1H), 2.33 (br d, *J* = 16.0 Hz, 1H), 2.03 (s, 3H). **LCMS:** m/z 546.1 $(M+H)^+$.

**Example 3**

2-(1-((2S,3R)-2-(cyclopentyloxy)-3-(3,5-dimethoxy-4-methylphenyl)-3-hydroxypropyl)-4-(methoxycarbonyl)-1H-pyrrol-3-yl)acetic acid **(Compound 102a)**

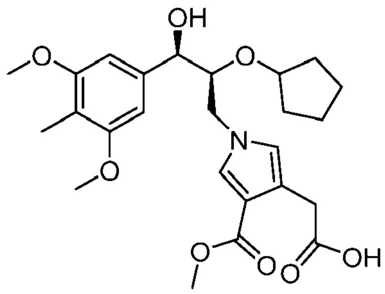

**Example 3 (Compound 102a)** was synthesized according to the procedures described for the preparation of **Example 1** (step A to step N in Scheme 1) by using cyclopentanol in step C and methyl 4-(2-methoxy-2-oxo-ethyl)-1H-pyrrole-3-carboxylate in step L. **$^1$H NMR** (400 MHz, DMSO-d6) $\delta$ 11.93 (br. s, 1H), 7.30 (d, $J$ = 2.4 Hz, 1H), 6.69 (d, $J$ = 2.4 Hz, 1H), 6.60 (s, 2H), 5.59 (br. m, 1H), 4.24 (d, $J$ = 6.4 Hz, 1H), 4.15 (dd, $J$ = 14.0, 2.4 Hz, 1H), 3.92 (dd, $J$ = 14.0, 6.8 Hz, 1H), 3.75 (s, 6H), 3.64 (s, 3H), 3.56 (s, 2H), 3.45 - 3.53 (m, 2H), 1.97 (s, 3H), 1.05 - 1.40 (m, 8H). **LC-MS:** m/z 476.2 (M+H)$^+$.

**Example 4**

2-(1-((2S,3R)-3-(3,5-dimethoxy-4-methylphenyl)-3-hydroxy-2-(pent-3-yn-1-yloxy)propyl)-4-(methoxycarbonyl)-1H-pyrrol-3-yl)acetic acid **(Compound 101a)**

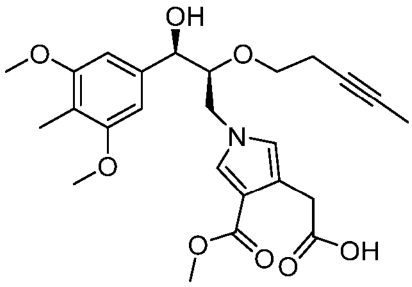

**Example 4 (Compound 101a)** was synthesized according to the procedures described for the preparation of **Example 1** (step A to step N in Scheme 1) by using pent-3-yn-1-ol in step C and methyl 4-(2-methoxy-2-oxo-ethyl)-1H-pyrrole-3-carboxylate in step L. **$^1$H NMR** (400 MHz, DMSO-d6) $\delta$ 11.94 (s, 1H), 7.35 (d, $J$ = 2.0 Hz, 1H), 6.72 (d, $J$ = 2.0 Hz, 1H), 6.62 (s, 2H), 5.62 (d, $J$ = 4.0 Hz, 1H), 4.31 (dd, $J$ = 6.4, 4.0 Hz, 1H), 4.13 (dd, $J$ = 14.4, 2.4 Hz, 1H), 3.96 (dd, $J$ = 14.4, 6.8 Hz, 1H), 3.76 (s, 6H), 3.64 (s, 3H), 3.58 (d, $J$ = 2.8 Hz, 1H), 3.55 (s, 2H), 3.10 (t, $J$ = 6.8 Hz, 2H), 1.98 - 2.02 (m, 2H), 1.97 (s, 3H), 1.67 (t, $J$ = 2.4 Hz, 3H). **LCMS:** m/z 474.2 (M+H)$^+$.

**Example 5**

2-(1-((2S,3R)-2-(cyclobutylmethoxy)-3-(3,5-dimethoxy-4-methylphenyl)-3-hydroxypropyl)-4-(methoxycarbonyl)-1H-pyrrol-3-yl)acetic acid **(Compound 117a)**

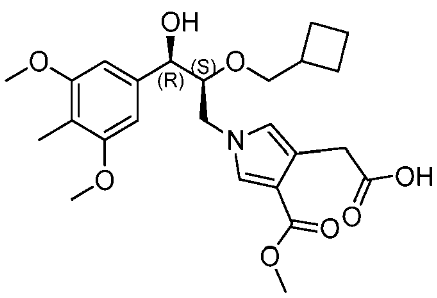

**Example 5 (Compound 117a)** was synthesized according to the procedures described for the preparation of **Example 1** (step A to step N in Scheme 1) by using cyclobutylmethanol in step C and methyl 4-(2-methoxy-2-oxo-ethyl)-1H-pyrrole-3-carboxylate in step L. **$^1$H NMR** (400 MHz, $CD_3OD$) $\delta$ 7.35 (d, *J* = 2.4 Hz, 1H), 6.69 (d, *J* = 2.4 Hz, 1H), 6.65 (s, 2H), 4.42 (d, *J* = 7.2 Hz, 1H), 4.20 (dd, *J* = 14.0, 2.4 Hz, 1H), 3.97 (dd, *J* = 14.0, 7.2 Hz, 1H), 3.81 (s, 6H), 3.74 (s, 3H), 3.66 (s, 2H), 3.57 - 3.48 (m, 1H), 3.02 (dd, *J* = 6.4, 2.4 Hz, 2H), 2.31 - 2.20 (m, 1H), 2.02 (s, 3H), 1.87 - 1.75 (m, 3H), 1.75 - 1.63 (m, 1H), 1.58 - 1.40 (m, 2H). **LCMS:** m/z 498.2 (M+ Na)$^+$.

**Example 6**

2-((2S,3R)-2-(cyclopentyloxy)-3-(3,5-dimethoxy-4-methylphenyl)-3-hydroxypropyl)-2H-indazole-7-carboxylic acid **(Compound 108a)**

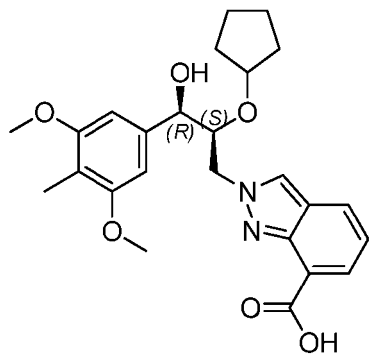

**Example 6 (Compound 108a)** was synthesized according to the procedures described for the preparation of **Example 1** (step A to step N in Scheme 1) by using cyclopentanol in step C and methyl 2H-indazole-7-carboxylate in step L. **$^1$H NMR** (400 MHz, $CD_3OD$) $\delta$ 8.33 (s, 1H), 8.03 (d, *J* = 7.2 Hz, 1H), 7.95 (d, *J* = 7.6 Hz m, 1H), 7.18 (d, *J* = 8.0, 0.8 Hz, 1H), 6.71 (s, 2H), 4.91 - 4.77 (m, 1H), 4.64 (d, *J* = 6.4 Hz, 1H), 4.56 - 4.50 (m, 1H), 4.09 - 3.99 (m, 1H), 3.84 (s, 6H), 3.53 - 3.32 (m, 1H), 2.03 (s, 3H), 1.40 - 1.09 (m, 7H), 0.94 - 0.80 (m, 1H). LC-MS: m/z 455.2 (M+H)$^+$.

**Example 7**

2-((2S,3R)-2-(cyclobutylmethoxy)-3-(3,5-dimethoxy-4-methylphenyl)-3-hydroxypropyl)-2H-indazole-7-carboxylic acid **(Compound 118a)**

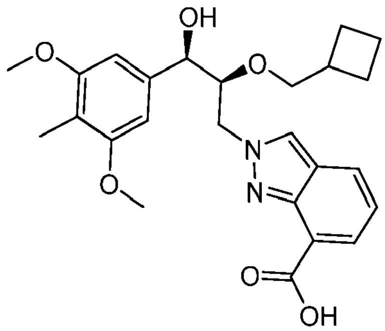

**Example 7 (Compound 118a)** was synthesized according to the procedures described for the preparation of **Example 1** (step A to step N in Scheme 1) by using cyclobutylmethanol in step C and methyl 2H-indazole-7-carboxylate in step L. **$^1$H NMR** (400 MHz, DMSO-*d6*) $\delta$ 8.49 (s, 1H), 7.99 (d, *J* = 8.4 Hz, 1H), 7.90 (d, *J* = 6.8 Hz, 1H), 7.14 - 7.10 (m, 1H), 6.71 (s, 2H), 5.74 (br d, *J* = 2.8 Hz, 1H), 4.73 - 4.64 (m, 1H) 4.60 (br d, *J* = 3.2 Hz, 1H), 4.53 - 4.42 (m, 1H), 3.89 - 3.82 (m, 1H), 3.77 (s, 6H) 3.05 (dd, *J* = 9.2, 6.4 Hz, 1H), 2.76 (dd, *J* = 9.2, 6.8 Hz, 1H), 2.06 (brs, 1H), 1.97 (s, 3H), 1.61 - 1.57 (m, 3H), 1.55 - 1.42 (m, 1H) 1.29 - 1.23 (m, 2H). **LC-MS:** m/z 455.2 (M+H)$^+$.

**Example 8**

2-((2S,3S)-3-(3,5-dimethoxy-4-methylphenyl)-3-hydroxy-2- phenethoxypropyl)-2H-indazole-7-carboxylic acid **(Compound 105b)** and

2-((2R,3R)-3-(3,5-dimethoxy-4-methylphenyl)-3-hydroxy-2-phenethoxypropyl)-2H-indazole-7-carboxylic acid **(Compound 105a)**

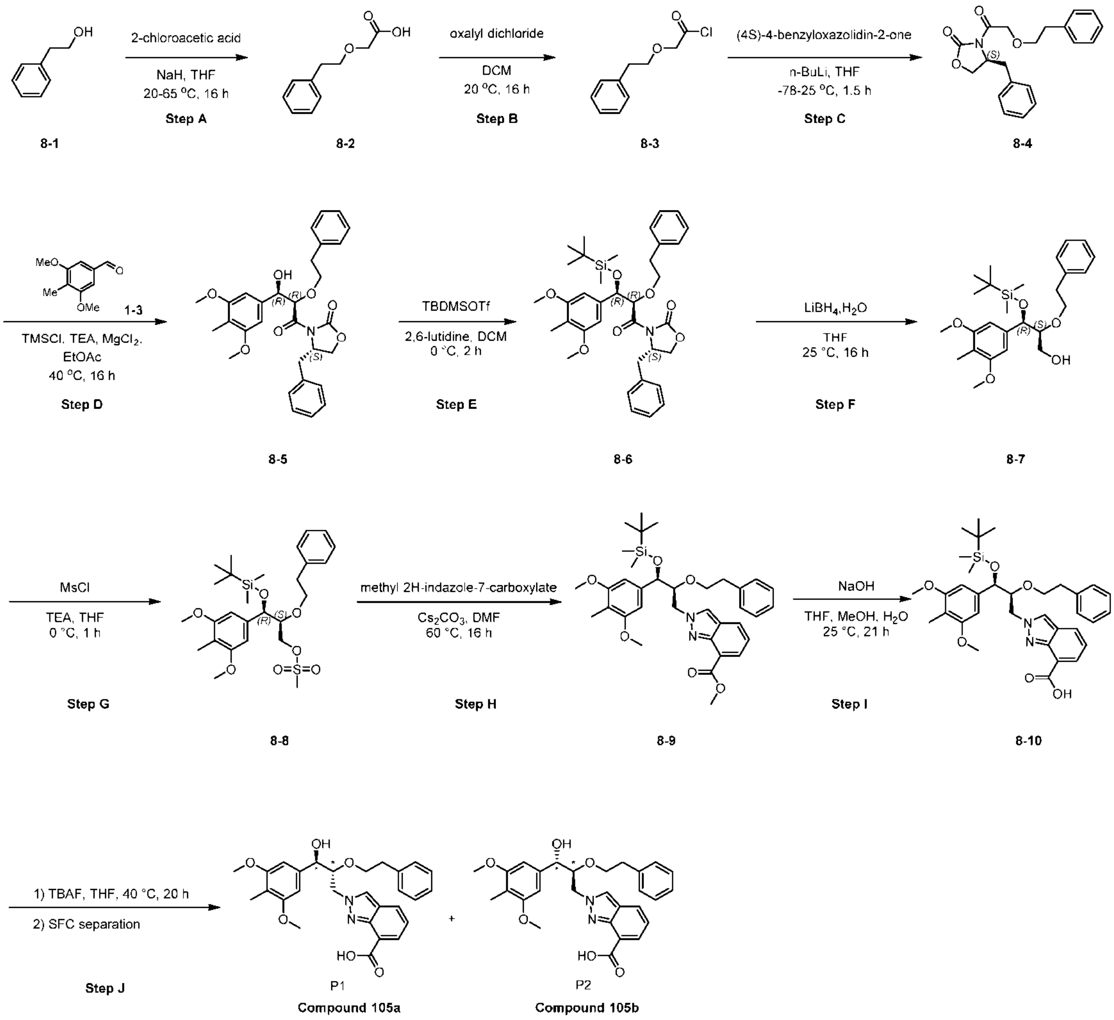


(S)-4-benzyl-3-(2-phenethoxyacetyl) oxazolidin-2-one was synthesized according to the procedures described for the preparation of **Example 1** (step C to step E in Scheme 1) by using 2-phenylethanol in step C and (4S)-4-benzyloxazolidin-2-one in step E. **LC-MS:** m/z 339.9 $(M+H)^+$.

Step D (S)-4-benzyl-3-((2R,3R)-3-(3,5-dimethoxy-4-methylphenyl)-3-hydroxy-2-phenethoxypropanoyl)oxazolidin-2-one

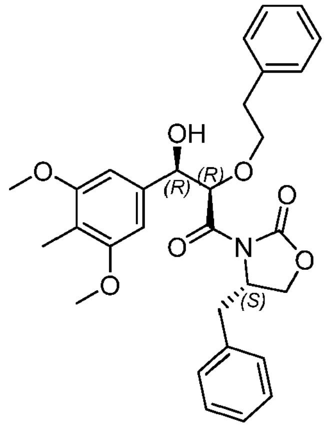

To a solution of (4S)-4-benzyl-3-[2-(2-phenylethoxy)acetyl]oxazolidin-2-one (6.41 g, 13.73 mmol) in EtOAc (5 mL) was added 3,5-dimethoxy-4-methyl-benzaldehyde (2.25 g, 12.48 mmol), TMSCl (2.03 g, 18.72 mmol), TEA (2.53 g, 24.96 mmol) and $MgCl_2$ (237.68 mg, 2.50 mmol). The mixture was stirred at 40 °C for 16 hrs under N2. The mixture was concentrated under reduced pressure. Then MeOH (20 mL) and TFA (2 mL) was added. After stirring for 1 hr, the solution was adjusted to pH=6 with *sat.* $NaHCO_3$ and then concentrated under reduced pressure. The residue was purification by flash chromatography (PE/EtOAc = 2/1) to give (4S)-4-benzyl-3-[(2R,3R)-3-(3,5-dimethoxy-4-methyl-phenyl)-3-hydroxy-2-(2-phenylethoxy)propanoyl]oxazolidin-2-one (5.23 g, 65% yield) as white solid. **LC-MS:** m/z 542.0 $(M+Na)^+$.

Step E (S)-4-benzyl-3-((2R,3R)-3-((tert-butyldimethylsilyl)oxy)-3- (3,5-dimethoxy-4-methylphenyl)-2-phenethoxypropanoyl)oxazolidin-2-one

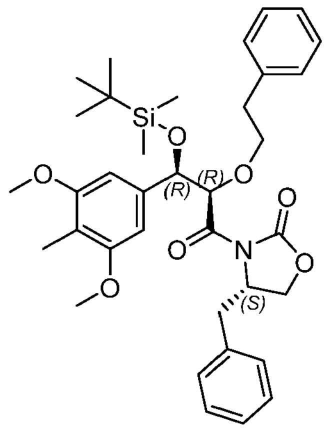

To a solution of (4S)-4-benzyl-3-[(2R,3R)-3-(3,5-dimethoxy-4-methyl-phenyl) -3-hydroxy-2-(2-phenylethoxy)propanoyl] oxazolidin-2-one (2 g, 3.85 mmol) in DCM (10 mL) was added 2,6-lutidine (495 mg, 4.62 mmol) and TBDMSOTf (1.22 g, 4.62 mmol). The mixture was stirred at 0 °C for 2 hrs. The mixture was washed with 1N HCl (20 mL), extracted with DCM (20 mL*2). The organics were collected and concentrated. The residue was purified by flash chromatography (PE/EtOAc = 3/1) to give (4S)-4-benzyl-3-[(2R,3R)-3-[tert-butyl(dimethyl)silyl]oxy-3-(3,5-dimethoxy-4-methyl-phenyl)-2-(2-phenylethoxy)propanoyl]oxazolidin-2-one (2.07 g, 84% yield) as yellow oil. **LC-MS:** m/z 656.1 $(M+Na)^+$.

Step F (2S,3R)-3-((tert-butyldimethylsilyl)oxy)-3-(3,5-dimethoxy-4-methylphenyl) - 2-phenethoxypropan-1-ol

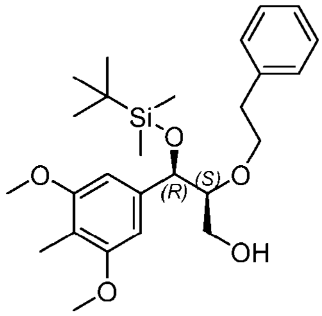

To a solution of $LiBH_4$ (1.50 g, 68.79 mmol) in THF (30 mL) was added $H_2O$ (0.28 mL, 15.20 mmol) at 0 °C. The mixture was stirred at 0 °C for 0.5 hrs. (4S)-4-benzyl-3-[(2R,3R)-3-[tert-butyl(dimethyl)silyl]oxy-3-(3,5-dimethoxy-4-methyl-phenyl)-2-(2-phenylethoxy)propanoyl]oxazolidin-2-one (4.36 g, 6.88 mmol) was added to the mixture and the mixture was stirred at 25°C for 16 hrs. The mixture was quenched with 1N HCl (30 mL) slowly, extracted with EtOAc (40 mL*2). The organics were collected and concentrated. The residue was purified by flash chromatography (PE/EtOAc = 4/1) to give (2S,3R)-3-[tert-butyl(dimethyl)silyl]oxy-3-(3,5-dimethoxy-4-methyl-phenyl)-2-(2-phenylethoxy)propan-1-ol (2.46 g, 74% yield) as a yellow oil. **LC-MS:** m/z 483.1 $(M+Na)^+$.

Step G (2S,3R)-3-((tert-butyldimethylsilyl)oxy)-3-(3,5-dimethoxy-4-methylphenyl) - 2-phenethoxypropyl methanesulfonate

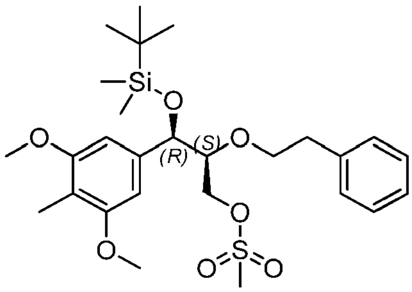

To a solution of (2S,3R)-3-[tert-butyl(dimethyl)silyl]oxy-3- (3,5-dimethoxy-4-methyl-phenyl)-2-(2-phenylethoxy)propan-1-ol (800 mg, 1.74 mmol) in THF (2 mL) was added MsCl (299 mg, 2.60 mmol) and TEA (316.30 mg, 3.13 mmol) at 0 °C. The mixture was stirred at 0 °C for 1 hr. The mixture was poured into ice cold water, extracted with EtOAc (25 mL*2). The organics were collected and concentrated to give [(2S,3R)-3-[tert-butyl(dimethyl)silyl] oxy-3-(3,5-dimethoxy-4-methyl-phenyl)-2-(2-phenyetho xy)propyl] methanesulfonate (829.3 mg, 88% yield) as a colorless oil. **LC-MS:** m/z 561.0 $(M+Na)^+$.

Step H methyl 2-((2S,3R)-3-((tert-butyldimethylsilyl)oxy)-3-(3,5-dimethoxy- 4-methylphenyl)-2-phenethoxypropyl)-2H-indazole-7-carboxylate

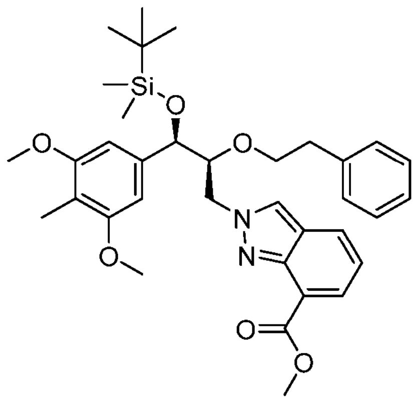

To a solution of [(2S,3R)-3-[tert-butyl(dimethyl)silyl]oxy-3-(3,5-dimethoxy-4 - methyl-phenyl)-2-(2-phenylethoxy)propyl] methanesulfonate (1.37 g, 2.54 mmol) in DMF (10 mL) was added $Cs_2CO_3$ (2.49 g, 7.63 mmol) and methyl-2H- indazole-7-carboxylate (538 mg, 3.05 mmol). The mixture was stirred at 60 °C for 16 hrs. The mixture was washed with $H_2O$ (30 mL), extracted with EtOAc (20 mL*2). The organics were collected and concentrated. The residue was purified by flash chromatography (PE/EtOAc = 2/1) to give methyl 2-[(2S,3R)-3-[tert-butyl(dimethyl)silyl]oxy-3-(3,5-dimethoxy-4-methyl-phenyl)-2-(2-phenylethoxy)propyl]indazole-7-carboxylate (1.19 g, 70% yield) as a yellow oil. LC-MS: m/z 641.3 $(M+Na)^+$.

Step I 2-((2S,3R)-3-((tert-butyldimethylsilyl)oxy)-3-(3,5-dimethoxy-4-methylphenyl) -2-phenethoxypropyl)-2H-indazole-7-carboxylic acid

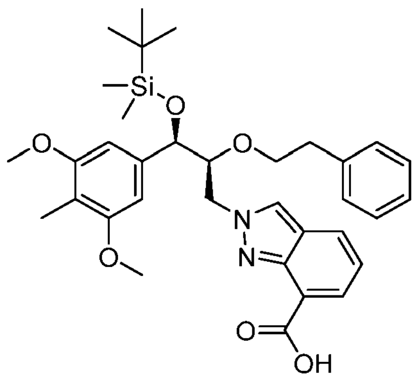

To a solution of methyl 2-[(2S,3R)-3-[tert-butyl(dimethyl)silyl]oxy-3- (3,5-dimethoxy-4-methyl-phenyl)-2-(2-phenylethoxy) propyl]indazole-7-carboxylate (1.19 g, 1.92 mmol) in THF (4 mL) and MeOH (4 mL) was added NaOH (2 M, 4.81 mL). The mixture was stirred at 25 °C for 21 hrs. The solution was adjusted to *p*H = 7 with 1N HCl, extracted with EtOAc (20 mL*2). The organics were collected and concentrated to give 2-[(2S,3R)-3-[tert-butyl(dimethyl)silyl]oxy-3-(3,5-dimethoxy-4-methyl-phenyl)-2-(2-phenylethoxy)propyl]indazole-7-carboxylic acid (817.9 mg, 48% yield) as a yellow oil. **LC-MS:** m/z 627.3$(M+Na)^+$.

Step J 2-((2R,3R)-3-(3,5-dimethoxy-4-methylphenyl)-3-hydroxy-2-phenethoxypropyl)-2H-indazole-7-carboxylic acid **(Compound 105a)** and 2-((2S,3S)-3-(3,5-dimethoxy-4-methylphenyl)-3-hydroxy-2-phenethoxypropyl)-2H-indazole-7-carboxylic acid **(Compound 105b)**

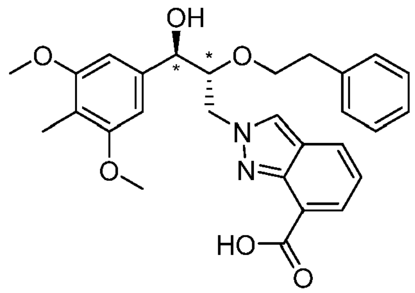

+

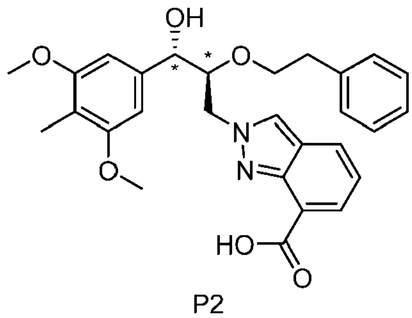

P1 P2

**A8a Compound 105a** **A8b Compound 105b**

To a solution of 2-[(2S,3R)-3-[tert-butyl(dimethyl)silyl]oxy-3- (3,5-dimethoxy-4-methyl-phenyl)-2-(2-phenylethoxy)propyl] indazole-7-carboxylic acid (817.9 mg, 1.35 mmol) in THF (4 mL) was added TBAF (1 M, 13.52 mL). The mixture was stirred at 40°C for 20 hrs. The reaction was diluted with EtOAc (40 mL), washed with $H_2O$ (20 mL*9). The organics were collected and concentrated under reduced pressure. The crude product was purified by prep-HPLC (column: DAICEL CHIRALPAK IG (250 mm*30mm, 10 um); mobile phase: [Neu-MeOH]; B%: 50%-50%)) to give 2-[(2R,3R)-3-(3,5-dimethoxy-4-methyl-phenyl)-3-hydroxy-2-(2-phenylethoxy)propyl]indazole-7-carboxylic acid **(Compound 105a)** (29.76 mg, 74% yield) as a white solid and 2-[(2S,3S)-3-(3,5-dimethoxy-4-methyl-phenyl)-3-hydroxy-2-(2-phenylethoxy)propyl] indazole-7-carboxylic acid **(Compound 105b)** (14.94 mg, 37% yield) as a white solid.

2-[(2R,3R)-3-(3,5-dimethoxy-4-methyl-phenyl)-3-hydroxy-2-(2-phenylethoxy)propyl] indazole-7-carboxylic acid **(Compound 105a): $^1$H NMR** (400 MHz, $CD_3OD$) $\delta$ 8.07 (d, $J$ = 7.2 Hz, 1H), 7.97 (s, 1H), 7.90 (d, $J$ = 8.4 Hz, 1 H), 7.19 - 7.16 (m, 1H), 7.15 - 7.09 (m, 3H), 6.91 - 6.88 (m, 2H), 6.67 (s, 2H), 4.82 - 4.71 (m, 1H), 4.68 - 4.62 (m, 1H), 4.54 - 4.48 (m, 1H), 4.01 - 3.98 (m, 1H), 3.80 (s, 6H), 3.38 - 3.34 (m, 1H), 3.15 - 3.08 (m, 1H), 2.52 - 2.48 (m, 2H), 2.01 (s, 3H). **LC-MS:** m/z 491.2 $(M+H)^+$.

2-[(2S,3 S)-3-(3,5-dimethoxy-4-methyl-phenyl)-3-hydroxy-2-(2-phenylethoxy)propyl] indazole-7-carboxylic acid **(Compound 105b): $^1$H NMR** (400 MHz, $CD_3OD$) $\delta$ 8.15 - 7.87 (m, 3H), 7.27 - 7.07 (m, 4H), 6.95 - 6.80 (m, 2H), 6.75 - 6.60 (m, 2H), 4.80 - 4.72 (m, 1H), 4.67 - 4.52 (m, 2H), 4.45 - 4.31 (m, 1H), 4.20 - 4.05 (m, 1H), 3.79 (s, 6H), 3.05 - 2.92 (m, 1H), 2.60 - 2.45 (m, 2H), 2.01 (s, 3H). **LC-MS:** m/z 491.2 $(M+H)^+$.

**Example 9**

1-(3-(3,5-dimethoxy-4-methylphenyl)-3-hydroxy-2-phenethoxypropyl)-1H-indazole-7-carboxylic acid **(Compound 107)**

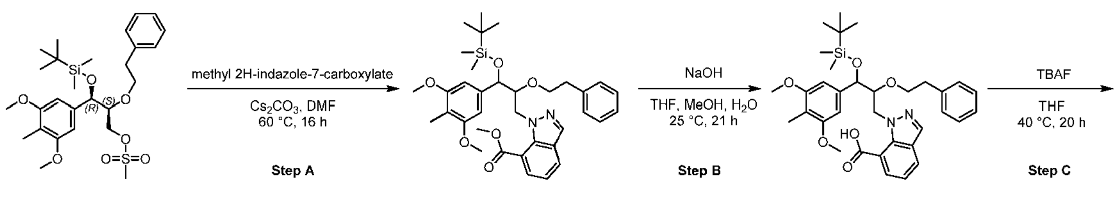


8-8 9-1 9-2

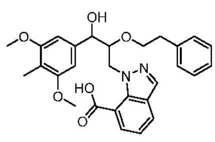

Compound 107

Step A methyl 1-(3-((tert-butyldimethylsilyl)oxy)-3-(3,5-dimethoxy-4-methylphenyl) -2-phenethoxypropyl)-1H-indazole-7-carboxylate

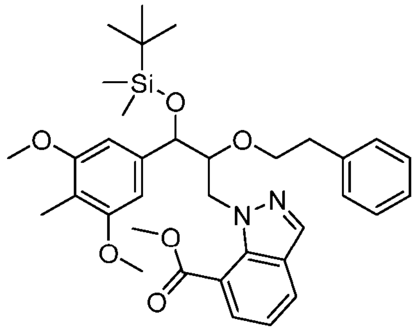

Methyl 1-(3-((tert-butyldimethylsilyl)oxy)-3-(3,5-dimethoxy-4-methylphenyl)-2-phenethoxypropyl)-1H-indazole-7-carboxylate (561.5 mg, 34% yield) was isolated as isomer according to the procedure described in step H for the preparation of **Example 8. LC-MS:** m/z 619.2$(M+H)^+$.

Step B 1-(3-((tert-butyldimethylsilyl)oxy)-3-(3,5-dimethoxy-4-methylphenyl)-2-phenethoxypropyl)-1H-indazole-7-carboxylic acid

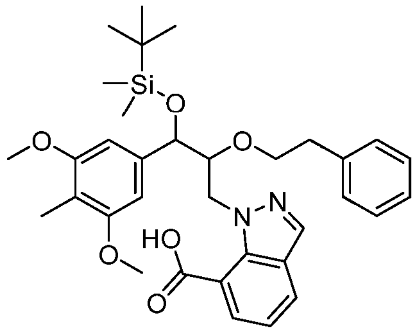

1-(3-((tert-butyldimethylsilyl)oxy)-3-(3,5-dimethoxy-4-methylphenyl)-2-phenethoxy propyl)-1H-indazole-7-carboxylic acid was synthesized according to the procedure described in step I for the preparation of **Example 8. LC-MS:** m/z 627.1$(M+Na)^+$.

Step C 1-(3-(3,5-dimethoxy-4-methylphenyl)-3-hydroxy-2-phenethoxypropyl)-1H-indazole-7-carboxylic acid **(Compound 107)**

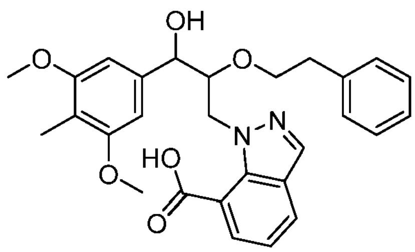

1-(3-(3,5-dimethoxy-4-methylphenyl)-3-hydroxy-2-phenethoxypropyl)-1H-indazole-7-carboxylic acid **(Compound 107)** was synthesized according to the procedure described in step J for the preparation of Example **8. $^1$H NMR** (400 MHz, $CD_3OD$) $\delta$ 8.11 (s, 1H), 7.93 - 7.88 (m, 2H), 7.23 - 7.18 (m, 1H), 7.07 - 7.01 (m, 3H), 6.68 - 6.61 (m, 4H), 5.20 - 5.13 (m, 1H),

5.02 - 4.95 (m, 0.8H), 4.89 - 4.77 (m, 0.2H), 4. 52 (d, *J* = 6.4 Hz, 1H), 3.76 (s, 3H), 3.69 - 3.65 (m, 1H), 3.00 - 2.77 (m, 1H), 2.60 - 2.42 (m, 1H), 2.30 - 2.05 (m, 2H), 1.99 (s, 3H). **LC-MS:** m/z 513.1 $(M+Na)^+$.

**Example 10**

2-(1-((2S,3R)-3-(3,5-dimethoxy-4-methylphenyl)-3-hydroxy-2-phene thoxypropyl)-4-(methoxycarbonyl)-1H-pyrrol-3-yl) acetic acid **(Compound 104a)**

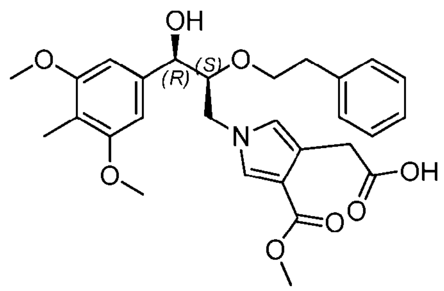

**Example 10 (Compound 104a)** was synthesized according to the procedures described for the preparation of **Example 8** (step H to step J in Scheme 8) by using methyl 4-(2-methoxy-2-oxo-ethyl)-1H-pyrrole-3-carboxylate in step H. The crude mixture was isolated via SFC (DAICEL CHIRALPAK IG (250 mm*30 mm,10 um); mobile phase: [Neu -IPA]; B%: 35% -35%, min) to give 2-[1-[(2S,3R)-3-(3,5-dimethoxy-4-methyl-phenyl)-3-hydroxy-2-(2-phenylethoxy)propyl]-4-methoxycarbonyl-pyrrol-3-yl]acetic acid (37.38 mg, 19% yield) as a white solid. **$^1$H NMR** (400 MHz, $CD_3OD$) $\delta$ 7.29 (s, 1H), 7.22 - 7.10 (m, 3H), 6.97 - 6.90 (m, 2H), 6.62 (s, 2H), 6.55 - 6.45 (m, 1H), 4.39 (d, *J* = 7.2 Hz, 1H), 4.20 - 4.12 (m, 1H), 4.00 - 3.92 (m, 1H), 3.78 (s, 6H), 3.73 (s, 3H), 3.63 (s, 2H), 3.54 - 3.53 (m, 1H), 3.29 - 3.22 (m, 2H), 2.59 - 2.55 (m, 2H), 2.03 (s, 3H). **LC-MS:** m/z 534.1$(M+Na)^+$.

**Example 11**

2-(6-((2S,3R)-3-(3,5-dimethoxy-4-methylphenyl)-3-hydroxy-2-phenethoxypropyl) pyridin-3-yl)acetic acid **(Compound 106a)**

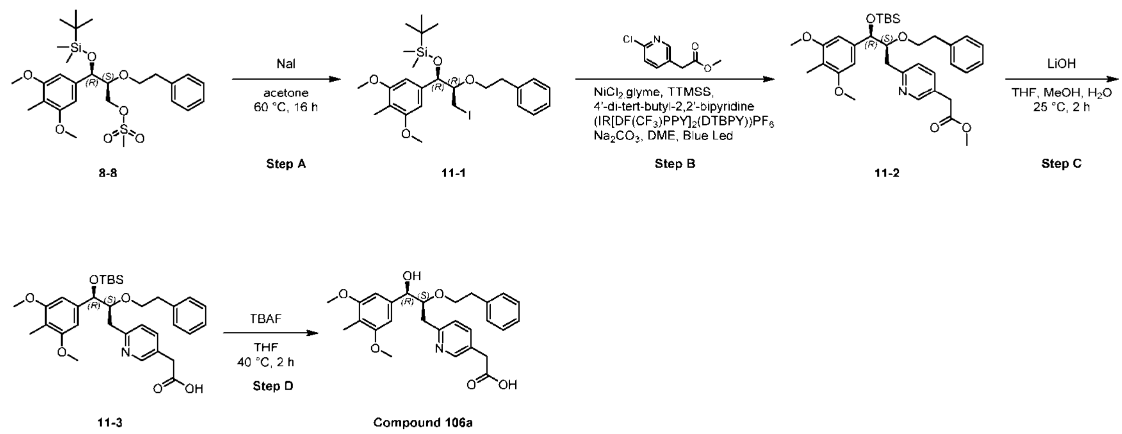


Step A tert-butyl((1R,2R)-1-(3,5-dimethoxy-4-methylphenyl)-3-iodo-2-henethoxy propoxy)dimethylsilane

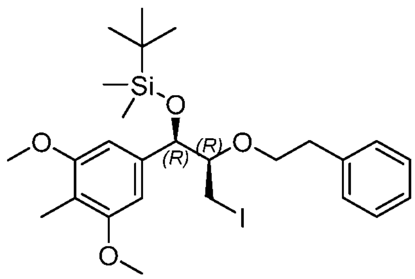

To a solution of [(2S,3R)-3-[tert-butyl(dimethyl)silyl]oxy-3-(3,5-dimethoxy-4-methyl-phenyl)-2-(2-phenylethoxy)propyl] methanesulfonate (6.3 g, 11.32 mmol) in acetone (40 mL) was added NaI (16.97 g, 113.22 mmol). The mixture was stirred at 60 °C for 16 hrs. After cooling, the mixture reaction was diluted with $H_2O$ (60 mL), extracted with EtOAc (50 mL*2). The organics were collected and concentrated under reduced pressure. The residue was purified by flash chromatography (PE/EtOAc = 10/1) to give tert-butyl-[(1R,2R)-1-(3,5-dimethoxy-4-methyl-phenyl)-3-iodo-2-(2-phenylethoxy)propoxy]-dimethyl-silane (4.69 g, 72% yield) as pale yellow oil. **LC-MS:** m/z 439.1(M-OTBS)$^+$.

Step B methyl 2-(6-((2S,3R)-3-((tert-butyldimethylsilyl)oxy)-3-(3,5-dimethoxy -4-methylphenyl)-2-phenethoxypropyl) pyridin-3-yl)acetate

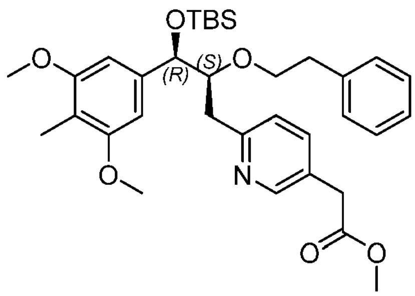

Dichloronickel;1,2-dimethoxyethane (19 mg, 87.63 umol) and 4-tert-butyl-2-(4-tert-butyl-2-pyridyl)pyridine (24 mg, 87.63 umol) in dry DME (5 mL) was stirred at 25 °C for 25 mins. Then tert-butyl-[(1R,2R)-1 -(3,5-dimethoxy-4-methyl-phenyl)-3-iodo-2-(2-phenylethoxy)propoxy]-dimethyl-silane (500 mg, 876.31 umol), methyl 2-(6-chloro-3-pyridyl)acetate (243.97 mg, 1.31 mmol), bis[3,5-difluoro-2-[5-(trifluoromethyl)-2-pyridyl]phenyl]iridium(1+); 4-tert-butyl-2-(4-tert-butyl-2-pyridyl) pyridine; hexafluorophosphate (98 mg, 87.63 umol), $Na_2CO_3$ (279 mg, 2.63 mmol) was added. The solution was bubbled with $N_2$ for 5 mins. Then bis(trimethylsilyl)silyl-trimethyl-silane (0.4 mL, 1.31 mmol) was added. The solution was irradiated with Blue Led at 25 °C for 12 hrs. The reaction mixture was filtered. The filtrate was collected and concentrated. The residue was purified by flash chromatography (PE/EtOAc = 3/1) to give methyl 2-[6-[(2S,3R)-3-[tert-butyl(dimethyl)silyl] oxy-3-(3,5-dimethoxy-4-methylphenyl)-2-(2-phenylethoxy)propyl]-3-pyridyl]acetate (170 mg, 6% yield) as a yellow oil. **LC-MS:** m/z 594.3 (M+H)$^+$.

Step C 2-(6-((2S,3R)-3-((tert-butyldimethylsilyl)oxy)-3-(3,5-dimethoxy-4-methylphenyl)-2-phenethoxypropyl)pyridin-3-yl)acetic acid

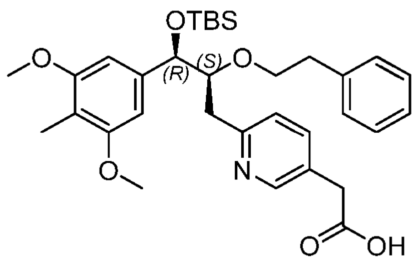

2-(6-((2S,3R)-3-((tert-butyldimethylsilyl)oxy)-3-(3,5-dimethoxy-4- methylphenyl)-2-phenethoxypropyl)pyridin-3-yl) acetic acid was synthesized according to the procedure described in step M for the preparation of **Example 1. LC-MS:** m/z 580.2 $(M+H)^+$.

Step D 1-(3-(3,5-dimethoxy-4-methylphenyl)-3-hydroxy-2-phenethoxypropyl)-1H-indazole-7-carboxylic acid **(Compound 106a)**

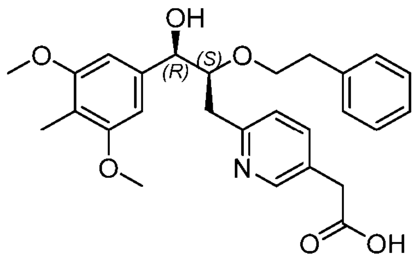

1-(3-(3,5-dimethoxy-4-methylphenyl)-3-hydroxy-2-phenethoxypropyl)-1H-indazole-7-carboxylic acid **(Compound 106a)** was synthesized according to the procedure described in step N for the preparation of **Example 1. $^1$H NMR** (400 MHz, $CD_3OD$) $\delta$ 8.28 (s, 1H), 7. 58 (d, $J$ = 8.0 Hz, 1H), 7.21 - 7.04 (m, 4H), 6.99 - 6.87 (m, 2H), 6.64 (s, 2H), 4.66 (d, $J$ = 5.2 Hz, 1H), 3.78 (s, 6H), 3.60 (s, 2H), 3.46 - 3.37 (m, 1H), 3.23 - 3.14 (m, 1H), 3.03 - 2.96 (m, 1H), 2.90 - 2.82 (m, 1H), 2.56 - 2.46 (m, 2H), 2.00 (s, 3H). **LC-MS:** m/z 466.2 $(M+H)^+$.

## Example 12

2-(6-((2S,3R)-3-(3,5-dimethoxy-4-methylphenyl)-3-hydroxy-2- phenethoxypropyl)-2-methoxypyridin-3-yl)acetic acid **(Compound 130a)**

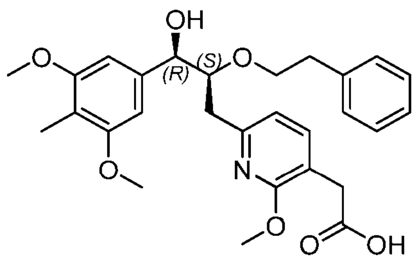

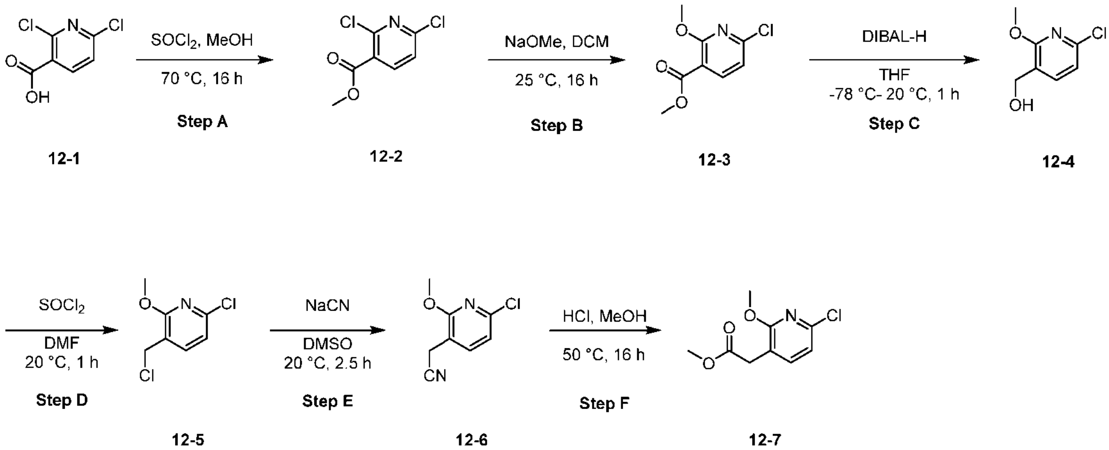


12-1 12-2 12-3 12-4

12-5 12-6 12-7

Step A methyl 2,6-dichloronicotinate

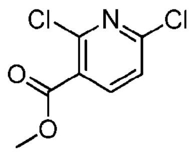

To a solution of 2,6-dichloropyridine-3-carboxylic acid (25 g, 130.21 mmol) in MeOH (130 mL) was added $SOCl_2$ (18.59 g, 156.25 mmol) at 0 °C and then the mixture was stirred at 20 °C for 0.5 hr. Then the reaction mixture was stirred at 70 °C for 16 hrs. The reaction mixture was concentrated under reduced pressure to remove the solvent and give a residue. Then the residue was dissolved in EtOAc (350 mL) and washed with *sat. aq.* $NaHCO_3$ (200 mL*4), dried over anhydrous $Na_2SO_4$ and concentrated to give methyl 2,6-dichloronicotinate (25.40 g, 94% yield) as white solid. **LC-MS:** m/z 205.8 $(M+H)^+$.

Step B methyl 6-chloro-2-methoxynicotinate

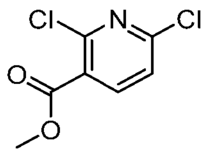

To a solution of methyl 2,6-dichloronicotinate (5 g, 24.27 mmol) in DCM (70 mL) was added $CH_3ONa$ (1.97 g, 36.40 mmol) at 0 °C and then the mixture was stirred at 25 °C for 16 hrs. The pH was adjusted to 6-7 with 1N HCl and the mixture was extracted with DCM (50 mL*3). The organics were collected, dried over anhydrous $Na_2SO_4$ and concentrated under reduced pressure to give a residue. The residue was purified by flash chromatography (PE/EtOAc = 5/1) to give methyl 6-chloro-2-methoxynicotinate (4 g, 77% yield) as light yellow oil. **LC-MS:** m/z 202.0 $(M+H)^+$.

Step C (6-chloro-2-methoxypyridin-3-yl)methanol

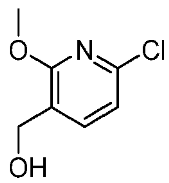

To a solution of methyl 6-chloro-2-methoxynicotinate (4 g, 19.84 mmol) in THF (30 mL) was added DIBAL-H (1 M, 70.00 mL) at -78°C and then the mixture was stirred at 20 °C for 1 hr. The reaction was diluted with a saturated aqueous solution of sodium potassium tartrate (300 mL) and extracted with EtOAc (200 mL). The combined organic extracts were dried over anhydrous $Na_2SO_4$ and concentrated under reduced pressure. The residue was purified by flash chromatography (PE/EtOAc = 4/1) to give (6-chloro-2-methoxypyridin-3-yl)-methanol (3.5 g, 98% yield) as white solid. **LC-MS:** m/z 173.8 $(M+H)^+$.

Step D 6-chloro-3-(chloromethyl)-2-methoxypyridine

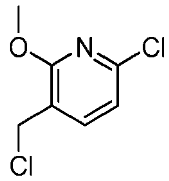

To a solution of (6-chloro-2-methoxypyridin-3-yl)methanol (505 mg, 2.91 mmol) in DCM (10 mL) was added $SOCl_2$ (865.22 mg, 7.27 mmol) dropwise at 0 °C and then the mixture was stirred at 20 °C for 1 hr. The reaction was diluted with $H_2O$ (50 mL) and then extracted with DCM (30 mL*3). The organics were collected, dried over anhydrous $Na_2SO_4$ and concentrated under reduced pressure to give 6-chloro-3-(chloromethyl)-2-methoxypyridine (525 mg, crude) as white solid. **LC-MS:** m/z 191.7 $(M+H)^+$.

Step E 2-(6-chloro-2-methoxypyridin-3-yl)acetonitrile

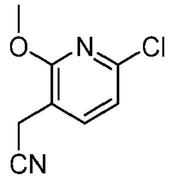

To a solution of 6-chloro-3-(chloromethyl)-2-methoxypyridine (525 mg, 2.72 mmol) in DMSO (10 mL) was added NaCN (173 mg, 3.44 mmol) and then the mixture was stirred at 20 °C for 2.5 hrs. The reaction was diluted with $H_2O$ (10 mL) and then extracted with EtOAc (10 mL*3). The organics were collected, dried over anhydrous $Na_2SO_4$ and concentrated under reduced pressure to give 2-(6-chloro-2-methoxypyridin-3-yl)acetonitrile (460 mg, 70% yield) as light yellow solid. **LC-MS:** m/z 182.9 $(M+H)^+$.

Step F methyl 2-(6-chloro-2-methoxypyridin-3-yl)acetate

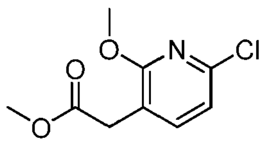

To a solution of 2-(6-chloro-2-methoxypyridin-3-yl)acetonitrile (3.22 g, 17.63 mmol) in MeOH (15 mL) was added HCl/MeOH (15 mL) and then the mixture was stirred at 50 °C for 16 hrs. The combined reaction was concentrated under reduced pressure to give a residue. The residue was diluted $H_2O$ (50 mL) and then the pH was adjusted to 7-8 with 1 M NaOH. The mixture was extracted with EtOAc (30 mL x 3). The combined organic layers were washed with brine (30 mL*3), dried over anhydrous $Na_2SO_4$ and concentrated in vacuo. The residue was purified by flash chromatography (PE/EtOAc = 5/1) to give methyl 2-(6-chloro-2-methoxypyridin-3-yl)acetate (2.3 g, 46% yield) as colorless oil. **LC-MS:** m/z 215.8$(M+H)^+$.

Step G 2-(6-((2S,3R)-3-(3,5-dimethoxy-4-methylphenyl)-3-hydroxy-2-phenethoxypropyl)-2-methoxypyridin-3-yl)acetic acid **(Compound 130a)**

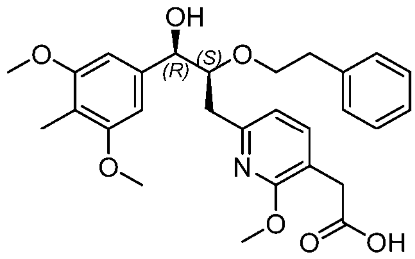

**Example 12 (Compound 130a)** was synthesized according to the procedures described for the preparation of **Example 11** (step A to step D in Scheme 11) by using methyl 2-(6-chloro-2-methoxypyridin-3-yl)acetate (12-7) in step B. **$^1$H NMR** (400 MHz, $CD_3OD$) $\delta$ 7.35 (d, *J* = 7.2 Hz, 1H), 7.18 - 7.04 (m, 3H), 6.97 - 6.90 (m, 2H), 6.68 - 6.61 (m, 3H), 4.62 (d, *J* = 5.6 Hz, 1H), 3.99 - 3.92 (m, 1H), 3.87 (s, 3H), 3.76 (s, 6H), 3.50 (s, 2H), 3.43 - 3.35 (m, 2H), 2.98 - 2.91 (m, 1H), 2.83 - 2.73 (m, 1H), 2.59 - 2.48 (m, 2H), 2.00 (s, 3H). **LC-MS:** m/z 496.2 $(M+H)^+$.

**Example 13**

2-(6-((2S,3R)-2-(cyclopentyloxy)-3-(3,5-dimethoxy-4-methylphenyl)-3-hydroxypropyl)-2-methoxypyridin-3-yl)acetic acid **(Compound 110a)**

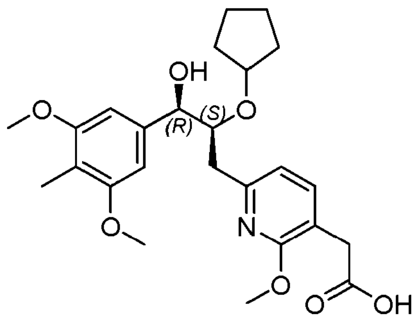

**Example 13 (Compound 110a)** was synthesized according to the procedures described for the preparation of **Example 11** (step A to step D in Scheme 11) by using [(2S,3R)-3-[tert-butyl(dimethyl)silyl]oxy-2-(cyclopentoxy)-3- (3,5-dimethoxy-4-methyl-phenyl)propyl] methanesulfonate in step A and methyl 2-(6-chloro-2-methoxypyridin-3-yl)acetate in step B. **$^1$H NMR** (400 MHz, $CD_3OD$) $\delta$ 7.40 (d, *J* = 8.0 Hz, 1H), 6.72 (d, *J* = 8.0 Hz, 1H), 6.64 (s, 2H), 4.55 (d, *J* = 8.0 Hz, 1H), 3.96 - 3.91 (m, 1H), 3.89 (s, 3H), 3.79 (s, 6H), 3.75 - 3.70 (m, 1H), 3.45 - 3.42 (m, 1H), 2.98 - 2.92 (m, 1H), 2.80 - 2.75 (m, 1H), 2.00 (s, 3H), 1.41 - 1.18 (m, 9H). **LC-MS:** m/z 460.2 $(M+H)^+$.

**Example 14**

2-(2-((2S,3R)-2-(cyclopentyloxy)-3-(3,5-dimethoxy-4-methylphenyl)-3-hydroxypropyl)thiazol-4-yl)acetic acid **(Compound 112a)**

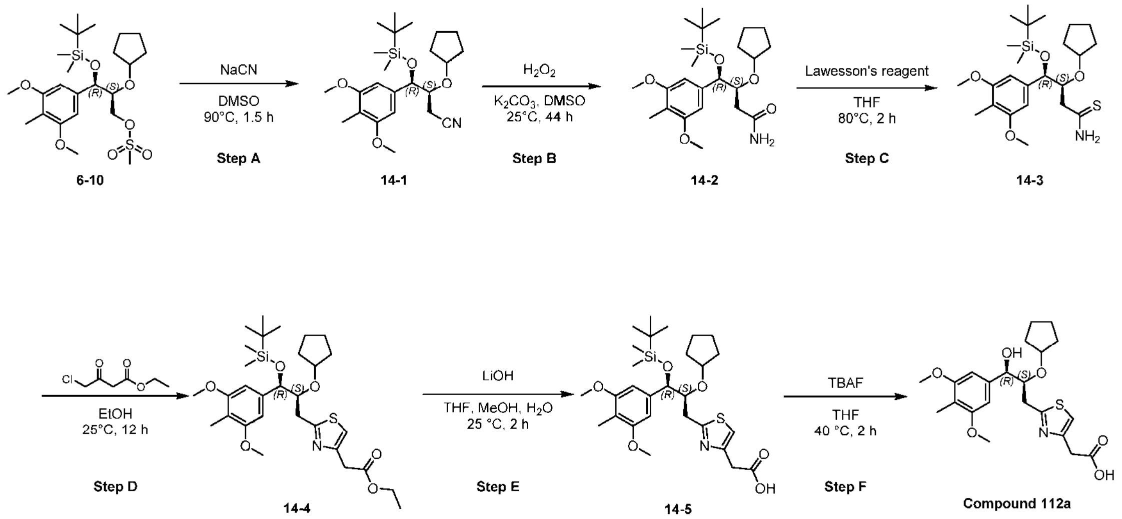

6-10 14-1 14-2 14-3 14-4 14-5 Compound 112a

Step A (3S,4R)-4-((tert-butyldimethylsilyl)oxy)-3-(cyclopentyloxy)-4-(3,5-dimethoxy-4-methylphenyl)butanenitrile

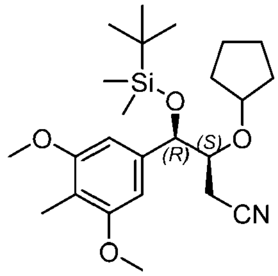

To a solution of [(2S,3R)-3-[tert-butyl(dimethyl)silyl]oxy-2-(cyclopentoxy)-3- (3,5-dimethoxy-4-methyl-phenyl)propyl] methanesulfonate (1 g, 1.99 mmol) ( 6-10, which was synthesized according to the procedures described for the preparation of **Example 1** by using cyclopentanol in step C) in DMSO (10 mL) was added NaCN (487 mg, 9.95 mmol) and then the mixture was stirred at 90 °C for 1.5 hrs. The reaction mixture was diluted with $H_2O$ (30 mL) and then extracted with EtOAc (20 mL*3). The combined organic layers were washed with brine (10 mL*2), dried over anhydrous $Na_2SO_4$ and filtered. The filtrate was concentrated in vacuo and the residue was purified by flash chromatography (PE/EtOAc = 8/1) to give (3S,4R)-4-[tert-butyl(dimethyl)silyl]oxy-3-(cyclopentoxy)-4-(3,5-dimethoxy-4-methyl-phenyl)butanenitrile (849 mg, 97% yield) as colorless oil. **LC-MS:** m/z 456.3 $(M+ Na)^+$.

Step B (3S,4R)-4-((tert-butyldimethylsilyl)oxy)-3-(cyclopentyloxy)-4-(3,5-dimethoxy-4-methylphenyl)butanamide

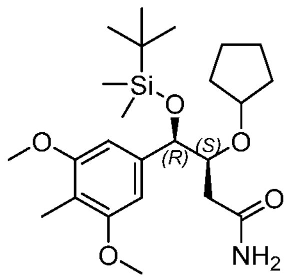

To a solution of (3S,4R)-4-[tert-butyl(dimethyl)silyl]oxy-3-(cyclopentoxy)-4- (3,5-dimethoxy-4-methyl-phenyl)butanenitrile (495 mg, 1.14 mmol) in DMSO (4 mL) was added $K_2CO_3$ (316 mg, 2.28 mmol) and $H_2O_2$ (1.29 g, 11.41 mmol, 30% purity) at 0 °C. The mixture was stirred at 25 °C for 44 hrs. The reaction was diluted with $H_2O$ (50 mL) and extracted with

EtOAc (30 mL*3). The combined organic layers were washed with $H_2O$ (20 mL*2), dried over anhydrous $Na_2SO_4$ and filtered. The filtrate was concentrated in vacuo and the residue was purified by flash chromatography (PE/EtOAc = 3/1) to give (3S,4R)-4-[tert-butyl(dimethyl)silyl]oxy-3-(cyclopentoxy)-4-(3,5-dimethoxy-4-methyl-phenyl) butanamide (383 mg, 74% yield) as a white solid. **LC-MS:** m/z 474.2 (M+ Na)$^+$.

Step C (3S,4R)-4-((tert-butyldimethylsilyl)oxy)-3-(cyclopentyloxy)-4- (3,5-dimethoxy -4-methylphenyl)butanethioamide

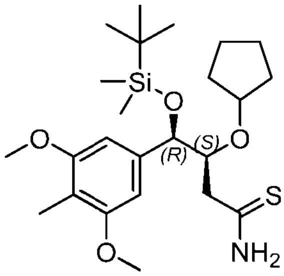

To a solution of (3S,4R)-4-[tert-butyl(dimethyl)silyl]oxy-3-(cyclopentoxy)-4- (3,5-dimethoxy-4-methyl-phenyl)butanamide (383 mg, 847.96 umol) in THF (15 mL) was added Lawesson's reagent (202 mg, 498.60 umol). The mixture was stirred at 80 °C for 2 hrs. The reaction was concentrated in vacuo and the residue was purified by flash chromatography (PE/EtOAc = 5/1) to give (3S,4R)-4-[tert-butyl(dimethyl)silyl] oxy-3-(cyclopentoxy)-4-(3,5-dimethoxy-4-methyl-phenyl)butanethioamide (216 mg, 53% yield) as a light yellow oil. **LC-MS:** m/z 490.2 (M+Na)$^+$.

Step D ethyl 2-(2-((2S,3R)-3-((tert-butyldimethylsilyl)oxy)-2-(cyclopentyloxy) -3-(3,5-dimethoxy-4-methylphenyl)propyl) thiazol-4-yl)acetate

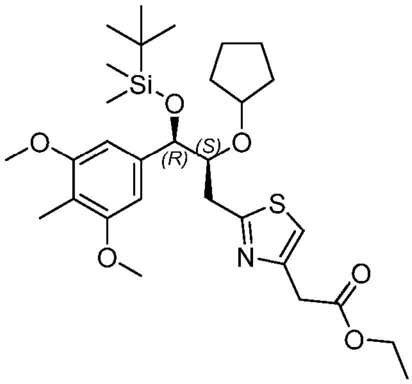

To a solution of (3S,4R)-4-[tert-butyl(dimethyl)silyl]oxy-3-(cyclopentoxy)-4- (3,5-dimethoxy-4-methyl-phenyl)butanethioamide (216 mg, 461.80 umol) in EtOH (5 mL) was added ethyl 4-chloro-3-oxo-butanoate (76 mg, 461.80 umol) and the mixture was stirred at 25 °C for 16 hrs. The reaction mixture was concentrated in vacuo and the residue was purified by flash chromatography (PE/EtOAc = 16/1) to give ethyl 2-[2-[(2S,3R)-3-[tert-butyl(dimethyl)silyl]oxy-2-(cyclopentoxy)-3-(3,5-dimethoxy-4-methyl-phenyl) propyl]thiazol-4-yl]acetate (36 mg, 12% yield) as a colorless oil. **LC-MS:** m/z 578.2 (M+H)$^+$.

Step E 2-(2-((2S,3R)-3-((tert-butyldimethylsilyl)oxy)-2-(cyclopentyloxy)-3- (3,5-dimethoxy-4-methylphenyl)propyl)thiazol-4-yl)acetic acid

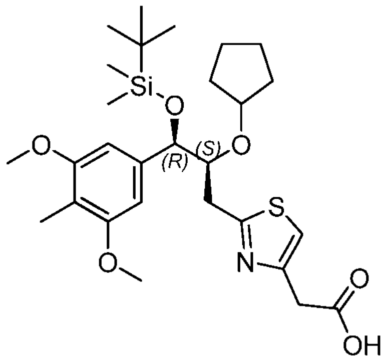

To a solution of ethyl 2-[2-[(2S,3R)-3-[tert-butyl(dimethyl)silyl]oxy-2-(cyclopentoxy)-3-(3,5-dimethoxy-4-methyl-phenyl) propyl]thiazol-4-yl]acetate (36 mg, 62.30 umol) in THF (0.5 mL), EtOH (0.5 mL) and $H_2O$ (0.5 mL) was added $LiOH.H_2O$ (13 mg, 311.50 umol). The mixture was stirred at 25 °C for 0.5 hrs. The reaction was acidified with 1N HCl and then diluted with $H_2O$ (10 mL). The mixture was extracted with EtOAc (10 mL*4). The combined organic layers were dried over anhydrous $Na_2SO_4$ and filtered to give 2-[2-[(2S,3R)-3-[tert-butyl(dimethyl)silyl]oxy-2-(cyclopentoxy)-3-(3,5-dimethoxy-4-methyl-phenyl)propyl] thiazol-4-yl]acetic acid (25 mg, 69 % yield) as a red oil. **LC-MS:** m/z 550.2 $(M+H)^+$.

Step F 2-(2-((2S,3R)-2-(cyclopentyloxy)-3-(3,5-dimethoxy-4-methylphenyl)-3-hydroxypropyl)thiazol-4-yl)acetic acid **(Compound 112a)**

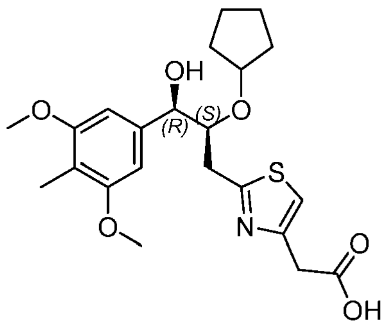

To a solution of 2-[2-[(2S,3R)-3-[tert-butyl(dimethyl)silyl]oxy-2-(cyclopentoxy) -3-(3,5-dimethoxy-4-methyl-phenyl)propyl] thiazol-4-yl]acetic acid (25 mg, 45.47 umol) in THF (2 mL) was added TBAF (1 M, 0.46 mL) and the mixture was stirred at 40 °C for 2 hrs. The reaction was diluted with EtOAc (20 mL). The mixture was washed with $H_2O$ (10 mL*6), dried over anhydrous $Na_2SO_4$ and filtered. The filtrate was concentrated in vacuo and the residue was purified by prep-HPLC (column: Phenomenex Gemini-NX 80*30mm*3um; mobile phase: [water (10mM NH4HCO3)-ACN]; B%: 10%-80%, 9 min) to give 2-[2-[(2S,3R)-2-(cyclopentoxy)-3-(3,5-dimethoxy-4-methyl-phenyl)-3-hydroxy-propyl]thiazol-4-yl]acetic acid **(Compound 112a)** (3.92 mg, 20% yield) as white solid. **$^1$H NMR** (400 MHz, $CD_3OD$) $\delta$ 7.22 - 7.14 (m, 1H), 6.67 (s, 2H), 4.67 - 4.65 (m, 1H), 3.88 - 3.79 (m, 8H), 3.69 (s, 2H), 3.27 - 3.15 (m, 2H), 2.03 (s, 3H), 1.52 - 1.32 (m, 8H). **LC-MS:** m/z 436.1 $(M+H)^+$.

**Example 15**

2-(2-((2S,3R)-2-((2,3-dihydro-1H-inden-2-yl)oxy)-3-(3,5-dimethoxy-4-methylphenyl)-3-hydroxypropyl)thiazol-4-yl) acetic acid **(Compound 129a)**

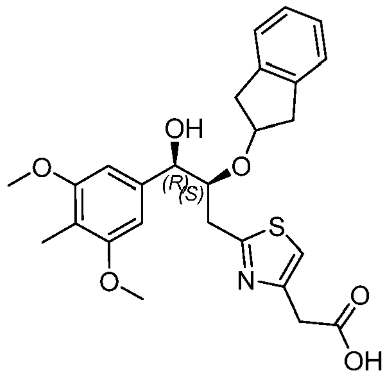

**Example 15 (Compound 129a)** was synthesized according to the procedures described for the preparation of **Example 14** (step A to step F in Scheme 14) by using (2S,3R)-3-((tert-butyldimethylsilyl)oxy)-2-((2,3-dihydro-1H-inden-2-yl) oxy)-3-(3,5-dimethoxy-4-methylphenyl)propyl methanesulfonate in step A. **$^1$H NMR** (400 MHz, $CD_3OD$) $\delta$ 7.18 (s, 1H), 7.09 - 6.94 (m, 3H), 6.93 - 6.92 (m, 1H), 6.60 (s, 2H) 4.55 - 4.46 (m, 1H), 4.11 - 4.04 (m, 1H), 3.94 - 3.88 (m, 1H), 3.72 - 3.70 (m, 8H) 3.37 - 3.09 (m, 2H), 2.86 - 2.76 (m, 2H), 2.56 - 2.50 (m, 2H), 2.02 (s, 3H). LC-MS: m/z 484.3 $(M+H)^+$.

## Example 16

2-((2S,3R)-2-(cyclopentyloxy)-3-(3,5-dimethoxy-4-methylphenyl)-3-hydroxypropyl)-1H-benzo[d]imidazole-5-carboxylic acid **(Compound 109a)**

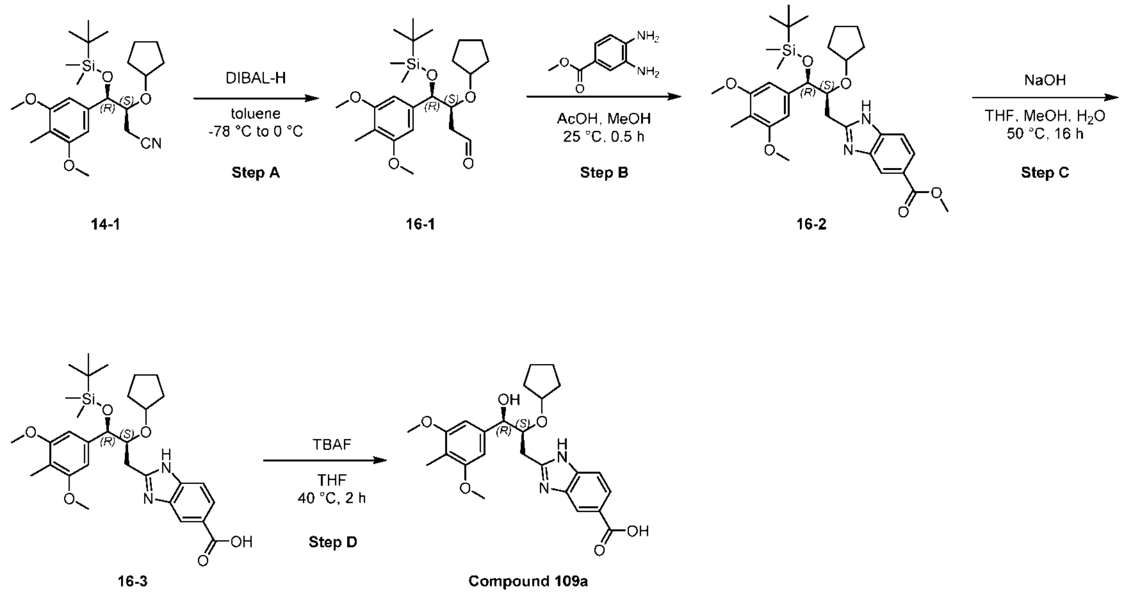


14-1 16-1 16-2

16-3 Compound 109a

Step A (3S,4R)-4-((tert-butyldimethylsilyl)oxy)-3-(cyclopentyloxy)-4-(3,5-dimethoxy-4-methylphenyl)butanal

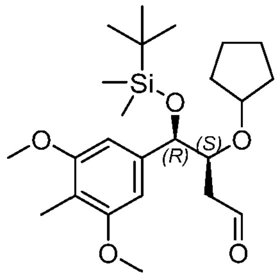

To a solution of (3S,4R)-4-[tert-butyl(dimethyl)silyl]oxy-3-(cyclopentoxy)-4- (3,5-dimethoxy-4-methyl-phenyl)butanenitrile (730 mg, 1.68 mmol) in toluene (10 mL) was added DIBAL-H (1 M, 3.37 mL) at -78 °C under $N_2$ and the mixture was stirred at -78 °C for 10 mins. And the resulting mixture was stirred at 0 °C under $N_2$ for 50 mins. The reaction was diluted with $H_2O$ (50 mL) and extracted with EtOAc (30 mL*3). The combined organic layers were washed brine (20 mL*3), dried over anhydrous $Na_2SO_4$ and filtered to give (3S,4R)-4-[tert-butyl(dimethyl)silyl]oxy-3-(cyclopentoxy)-4-(3,5-dimethoxy-4-methyl-phenyl)butanal (721 mg, 98% yield) as colorless oil. **LC-MS:** m/z 459.2 $(M+Na)^+$.

Step B methyl 2-((2S,3R)-3-((tert-butyldimethylsilyl)oxy)-2-(cyclopentyloxy)-3- (3,5-dimethoxy-4-methylphenyl)propyl)-1H-benzo[d]imidazole-5-carboxylate

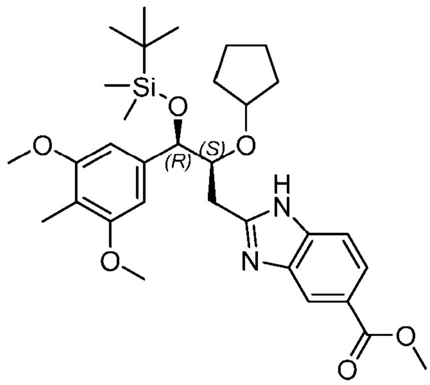

To a solution of (3S,4R)-4-[tert-butyl(dimethyl)silyl]oxy-3-(cyclopentoxy)-4- (3,5-dimethoxy-4-methyl-phenyl)butanal (400 mg, 916.06 umol) in MeOH (5 mL) was added methyl 3,4-diaminobenzoate (152 mg, 916.06 umol) and AcOH (2.60 g, 43.30 mmol). The mixture was stirred at 25°C for 0.5 hr. The pH value was adjusted to 7 with *sat. aq.* $NaHCO_3$ solution. The mixture was diluted with $H_2O$ (30 mL) and then the mixture was extracted with EtOAc (20 mL*3). The combined organics were dried over anhydrous $Na_2SO_4$ and filtered. The filtrate was concentrated in vacuo and the residue was purified by flash chromatography (PE/EtOAc = 4/1) to give methyl 2-[(2S,3R)-3-[tert-butyl(dimethyl)silyl]oxy-2-(cyclopentoxy)-3-(3,5-dimethoxy-4-methyl-phenyl)propyl]-1H-benzimidazole-5-carboxylate (163 mg, 30% yield) as an orange oil. **LC-MS:** m/z 583.2 $(M+H)^+$.

Step C 2-((2S,3R)-3-((tert-butyldimethylsilyl)oxy)-2-(cyclopentyloxy)-3- (3,5-dimethoxy-4-methylphenyl)propyl)-1H-benzo[d]imidazole-5-carboxylic acid

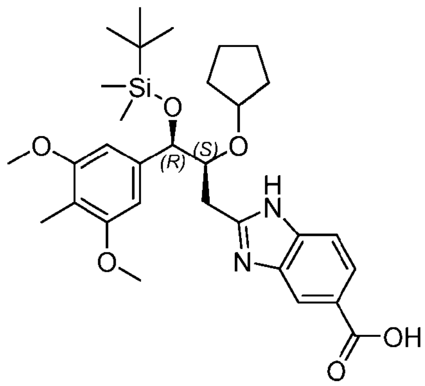

To a solution of methyl 2-[(2S,3R)-3-[tert-butyl(dimethyl)silyl]oxy-2-(cyclopentoxy) -3-(3,5-dimethoxy-4-methyl-phenyl) propyl]-1H-benzimidazole-5-carboxylate (163 mg, 279.68 umol) in THF (1.5 mL), MeOH (1.5 mL) and $H_2O$ (1.5 mL) was added NaOH (56 mg, 1.40 mmol). The mixture was stirred at 50°C for 16 hrs. The pH value was adjusted to 2-3 with 1N HCl. The mixture was diluted with $H_2O$ (30 mL) and extracted with EtOAc (20 mL*3). The combined organics were dried over anhydrous $Na_2SO_4$, filtered and concentrated to give 2-[(2S,3R)-3-[tert-butyl(dimethyl)silyl]oxy-2-(cyclopentoxy)-3-(3,5-dimethoxy-4-methyl-phenyl)propyl]-1H-benzimidazole-5-carboxylic acid (160 mg, crude) as an orange solid. **LC-MS:** m/z 569.2 $(M+H)^+$.

Step D 2-((2S,3R)-2-(cyclopentyloxy)-3-(3,5-dimethoxy-4-methylphenyl)-3-hydroxypropyl)-1H-benzo[d]imidazole-5-carboxylic acid **(Compound 109a)**

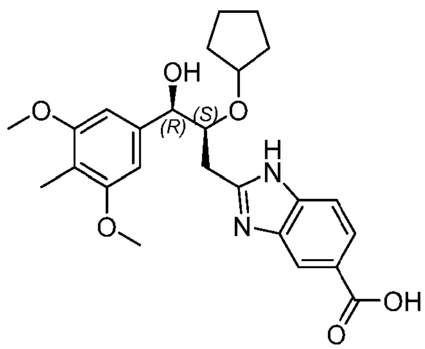

To a solution of 2-[(2S,3R)-3-[tert-butyl(dimethyl)silyl]oxy-2-(cyclopentoxy)-3- (3,5-dimethoxy-4-methyl-phenyl)propyl]-1H-benzimidazole-5-carboxylic acid (160 mg, 281.31 umol) in THF (2 mL) was added TBAF (1 M, 2.81 mL) and the mixture was stirred at 40°C for 2 hrs. The reaction was diluted with EtOAc (15 mL) and then washed with $H_2O$ (8 mL*6). The organics were dried over anhydrous $Na_2SO_4$ and filtered. The filtrate was concentrated in vacuo and the residue was purified by prep-HPLC (column: Phenomenex Gemini-NX 80*30mm*3um; mobile phase: [water (10mM NH4HCO3)-ACN]; B%: 10%-80%, 9min) to give 2-[(2S,3R)-2-(cyclopentoxy)-3-(3,5-dimethoxy-4-methylphenyl)-3-hydroxypropyl]-1H-benzimidazole-5-carboxylic acid (53.94 mg, 42% yield) **(Compound 109a)** as white solid. **$^1$H NMR** (400 MHz, $CD_3OD$) $\delta$ 8.22 - 8.21 (m, 1H), 7.94 - 7.93 (m, 1H), 7.56 - 7.52 (m, 1H), 6.71 (s, 2H), 4.78 - 4.76 (m, 1H), 4.01 - 3.94 (m, 1H), 3.84 (s, 6H), 3.77 - 3.71 (m, 1H), 3.21 - 3.12 (m, 1H), 3.09 - 2.98 (m, 1H), 2.02 (s, 3H), 1.55 - 1.49 (m, 3H), 1.34 - 1.03 (m, 5H). **LC-MS:** m/z 455.1 $(M+H)^+$.

**Example 17**

2-((2S, 3R)-2-((2, 3-dihydro-1H-inden-2-yl) oxy)-3-(3, 5-dimethoxy-4-methylphenyl)-3-hydroxypropyl)-1H-benzo[d]imidazole-5-carboxylic acid **(Compound 114a)**

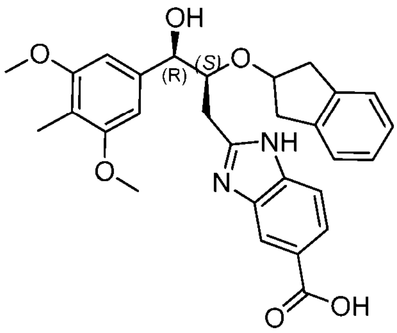

**Example 17 (Compound 114a)** was synthesized according to the procedures described for the preparation of **Example 16** (step A to step D in Scheme 165) by using (3S,4R)-4-[tert-butyl(dimethyl)silyl]oxy-4-(3,5-dimethoxy-4-methyl-phenyl)-3-indan-2-yloxy-butanenitrile in step A. **$^1$H NMR** (400MHz, $CD_3OD$) $\delta$ 8.13 (s, 1H), 7.91 (dd, *J* = 8.4, 1.6 Hz, 1H), 7.46 (d, *J* = 8.4 Hz, 1H), 7.01 - 6.91 (m, 2H), 6.89 - 6.78 (m, 1H), 6.67 (s, 2H), 6.46 (d, *J* = 7.2 Hz, 1H), 4.71 (d, *J* = 5.6, 1H), 4.04 - 3.94 (m, 2H), 3.76 (s, 6H), 3.21 (dd, *J* = 11.2, 3.2 Hz, 1H), 2.98 - 2.92 (m, 1H), 2.83 - 2.72 (m, 1H), 2.68 - 2.52 (m, 2H), 2.27 - 2.18 (m, 1H), 2.02 (s, 3H). **LC-MS:** m/z 503.1 $(M+H)^+$.

**Example 18**

2-((2S,3R)-2-(cyclobutylmethoxy)-3-(3,5-dimethoxy-4-methylphenyl)-3-hydroxypropyl)-1H-benzo[d]imidazole-5-carboxylic acid **(Compound 131a)**

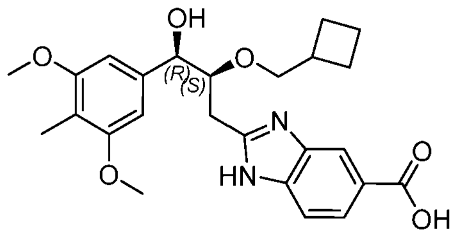

**Example 18 (Compound 131a)** was synthesized according to the procedures described for the preparation of **Example 16** (step A to step D in Scheme 16) by using (3S,4R)-4-((tert-butyldimethylsilyl)oxy)-3-(cyclobutylmethoxy)-4-(3,5-dimethoxy-4-methylphenyl)butanenitrile in step A. **$^1$H NMR** (400 MHz, DMSO-*d6*) $\delta$ 12.44 (s, 1H), 8.07 - 7.96 (m, 1H), 7.74 (s, 1H), 7.51 - 7.34 (m, 1H), 6.68 (s, 2H), 5.54 (s, 1H), 4.65 (s, 1H), 3.94 - 3.85 (m, 1H), 3.78 (s, 6H), 3.28 - 3.23 (m, 2H), 3.11 - 3.01 (m, 2H), 2.96 - 2.86 (m, 1H), 2.20 - 2.09 (m, 1H), 1.97 (s, 3H), 1.70 - 1.43 (m, 4H), 1.40 - 1.26 (m, 2H). **LC-MS:** m/z 455.2 $(M+H)^+$.

**Example 19**

2-[(2S,3R)-2-(cyclobutylmethoxy)-3-(3,5-dimethoxy-4-methyl-phenyl)-3-hydroxypropyl]-1-methyl-benzimidazole-5-carboxylic acid **(Compound 119a)**

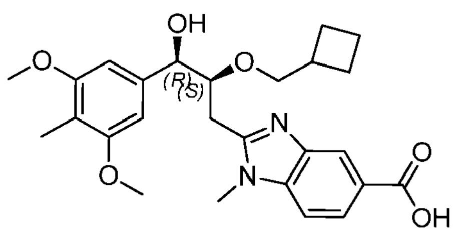

Step A methyl 3-amino-4-(methylamino)benzoate

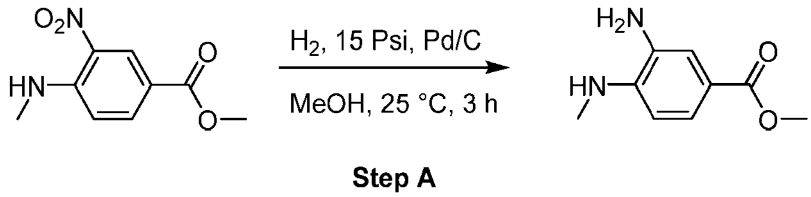


**19-1** **19-2**

To a solution of methyl 4-(methylamino)-3-nitro-benzoate (500 mg, 2.38 mmol) in MeOH (5 mL) was added Pd/C (100 mg). The mixture was stirred under 15 Psi $H_2$ at 25 °C for 3 hrs. The mixture was filtered and the filtrate was concentrated in vacuum to give methyl 3-amino-4-(methylamino)benzoate (309 mg, 72.1% yield) as a brown solid. It was used for next step without further purification. **$^1$H NMR** (400 MHz, DMSO-*d6)* $\delta$ 7.25 - 7.22 (dd, *J* = 8.0, 1.6 Hz, 1H), 7.16 (d, *J* = 1.6 Hz, 1H), 6.39 (d, *J* = 8.0 Hz, 1H), 5.44 - 5.40 (m, 1H), 4.69 (s, 2H), 3.72 (s, 1H), 2.77 (d, *J* = 4.8 Hz, 3H).

Step B 2-[(2S,3R)-2-(cyclobutylmethoxy)-3-(3,5-dimethoxy-4-methyl-phenyl)-3-hydroxy-propyl]-1-methyl-benzimidazole-5-carboxylic acid **(Compound 119a)**

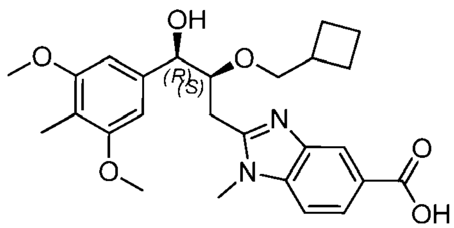

**Example 19 (Compound 119a)** was synthesized according to the procedures described for the preparation of **Example 16** (step A to step D in Scheme 16) by using (3S,4R)-4-((tert-butyldimethylsilyl)oxy)-3-(cyclobutylmethoxy)-4-(3,5-dimethoxy-4-methylphenyl)butanenitrile in step A and methyl 3-amino-4-(methylamino)benzoate in step B. **$^1$H NMR** (400 MHz, DMSO-*d6*) $\delta$ 8.09 (d, *J* = 0.8 Hz, 1H), 7.81 (dd, *J* = 8.4, 1.2 Hz, 1H), 7.55 (d, *J* = 8.4 Hz, 1H), 6.70 (s, 2H), 5.57 - 5.53 (m, 1H), 4.73 - 4.69 (m, 1H), 3.88 - 3.81 (m, 1H), 3.78 (s, 6H), 3.76 (s, 3H), 3.27 - 3.18 (m, 2H), 3.05 - 2.95 (m, 2H), 2.21 - 2.05 (m, 1H), 1.97 (s, 3H), 1.69 - 1.47 (m, 4H), 1.34 - 1.21 (m, 2H). **LCMS:** m/z 469.2 $(M+H)^+$.

**Example 20**

2-(2-((2S,3R)-2-(cyclopentyloxy)-3-(3,5-dimethoxy-4-methylphenyl)-3-hydroxypropyl)-1H-benzo[d]imidazol-5-yl)acetic acid **(Compound 111a)**

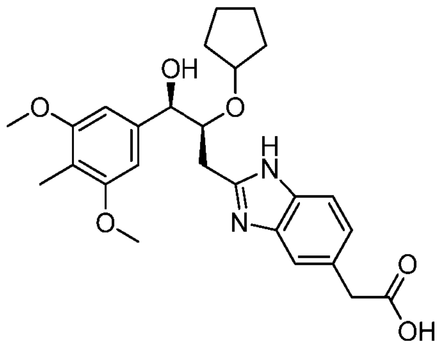

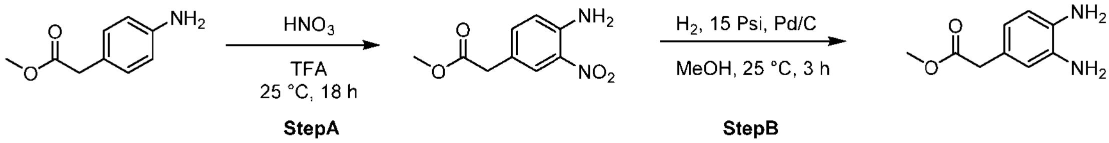


**20-1** **20-2** **20-3**

Step A methyl 2-(4-amino-3-nitrophenyl) acetate

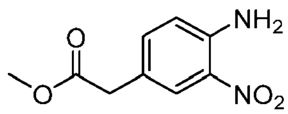

The solution of methyl 2-(4-aminophenyl) acetate (2 g, 12.11 mmol) in TFA (20 mL) and cooled to -10 °C. Then $HNO_3$ (0.57 mL, 12.71 mmol) was added dropwise. The reaction mixture was warmed to 25 °C and stirred for 18 hrs. The reaction mixture was neutralized with 1N NaOH and extracted with EtOAc (30 mL*3). The organic layer was washed with $H_2O$ (50 mL), dried over $Na_2SO_4$ and filtered. The filtrate was concentrated in vacuo and the residue was purified by flash chromatography (PE/EtOAc = 5/1) to give methyl 2-(4-amino-3-nitrophenyl) acetate (930 mg, 37% yield) as yellow solid. **LC-MS:** m/z 210.1 $(M+H)^+$.

Step B methyl 2-(3,4-diaminophenyl) acetate

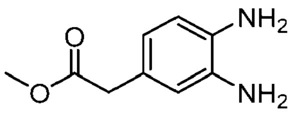

To a solution of methyl 2-(4-amino-3-nitro-phenyl) acetate (500 mg, 2.38 mmol) in MeOH (5 mL) was added Pd/C (0.2 g, 10% purity). The mixture was stirred at 25 °C under H2 (15 Psi) for 3 hrs. The mixture was filtered, and the filtrate was concentrated in vacuo and the residue was purified by flash chromatography (PE/EtOAc = 1/1) to give methyl 2-(3,4-diaminophenyl) acetate (384 mg, 89.5% yield) as a brown oil. **$^1$H NMR** (400 MHz, DMSO-*d6*) $\delta$ 6.44 - 6.37 (m, 2H), 6.25 (d, *J* = 6.8, 1H), 4.43 - 4.35 (m, 4H), 3.56 (s, 3H), 3.34 (s, 2H).

Step C 2-(2-((2S,3R)-2-(cyclopentyloxy)-3-(3,5-dimethoxy-4-methylphenyl) -3-hydroxypropyl)-1H-benzo[d]imidazol-5-yl)acetic acid **(Compound 111a)**

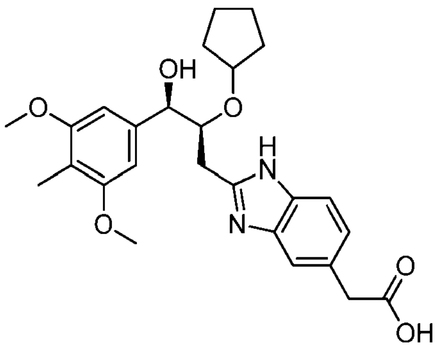

**Example 20 (Compound 111a)** was synthesized according to the procedures described for the preparation **of Example 16** (step A to step D in Scheme 16) by using methyl 2-(3,4-diaminophenyl)acetate in step B. $^1$H **NMR** (400 MHz, $CD_3OD$) $\delta$ 7.50 - 7.43 (m, 2H), 7.24 - 7.17 (d, *J* = 8.8 Hz, 1H), 6.70 (s, 2H), 4.74 (d, *J* = 5.2 Hz, 1H), 3.99 - 3.90 (m, 1H), 3.84 (s, 6H), 3.77 - 3.71 (m, 1H), 3.70 (s, 1H), 3.19 - 3.12 (m, 1H), 3.07 - 3.04 (m, 1H), 2.03 (s, 3H), 1.52 - 1.36 (m, 3H), 1.34 - 1.03 (m, 5H). **LC-MS:** m/z 469.1 $(M+H)^+$.

**Example 21**

2-[2-[(2S,3R)-3-(3,5-dimethoxy-4-methyl-phenyl)-3-hydroxy-2-indan-2-yloxy-propyl]-1H-benzimidazol-5-yl]acetic acid **(Compound 115a)**

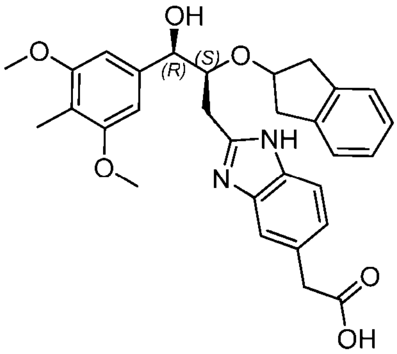

**Example 21 (Compound 115a)** was synthesized according to the procedures described for the preparation of **Example 16** (step A to step D in Scheme 16) by using [(2S,3R)-3-[tert-butyl(dimethyl)silyl]oxy-3-(3,5-dimethoxy-4-methyl-phenyl)-2-indan-2-yloxy-propyl]methanesulfonate in step A and methyl 2-(3,4-diaminophenyl)acetate in step B. **$^1$H NMR** (400 MHz, $CD_3OD$) $\delta$ 7.44 - 7.34 (m, 2H), 7.20 (d, *J* = 5.6 Hz, 1H), 7.02 -6.93 (m, 2H), 6.92 - 6.85 (m, 1H), 6.66 (s, 2H), 6.51 (d, *J* = 7.6 Hz, 1H), 4.68 (d, *J* = 5.6 Hz, 1H), 4.02 - 3.94 (m, 2H), 3.76 (s, 6H), 3.68 (s, 2H), 3.23 - 3.18 (m, 1H), 2.97 -2.91 (m, 1H), 2.80 - 2.72 (m, 1H), 2.66 - 2.55 (m, 2H), 2.28 - 2.20 (m, 1H), 2.02 (s, 3 H). **LC-MS:** m/z 517.1 $(M+H)^+$.

**Example 22**

2-[2-[(2S,3R)-2-(cyclobutylmethoxy)-3-(3,5-dimethoxy-4-methyl-phenyl)-3-hydroxypropyl]-1-methyl-benzimidazol-5-yl] acetic acid **(Compound 120a)**

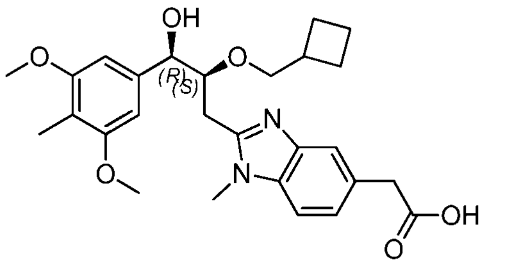

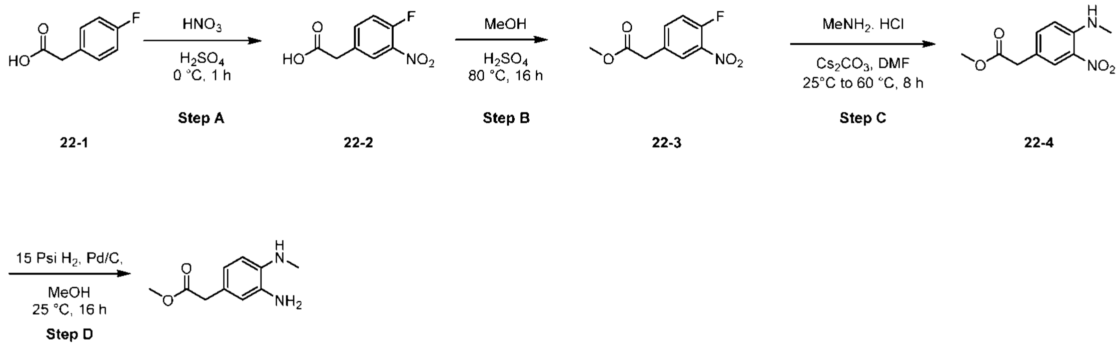


22-1 22-2 22-3 22-4

22-5

Step A 2-(4-fluoro-3-nitro-phenyl)acetic acid

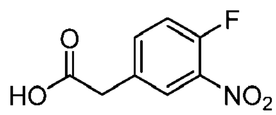

To a solution of 2-(4-fluorophenyl)acetic acid (3.00 g, 19.5 mmol) in H2SO4 (20 mL) was added HNO3 (1.28 g, 19.3 mmol,) dropwise at 0 °C and stirred for 1 hr. The reaction mixture was poured onto ice. The reaction mixture was extracted with EtOAc (25 mL* 2). The organic phase was washed with brine, dried over $Na_2SO_4$ and filtered. The filtrate was concentrated in vacuo to give 2-(4-fluoro-3-nitro-phenyl)acetic acid (4.11 g, crude) as a yellow solid. **$^1$H NMR** (400MHz, $CDCl_3$) $\delta$ 8.01 (dd, *J* = 6.8, 2.4 Hz, 1H), 7.64 - 7.53 (m, 1H), 7.32 - 7.22 (m, 1H), 3.74 (s, 2H).

Step B 2-(4-fluoro-3-nitro-phenyl)acetate

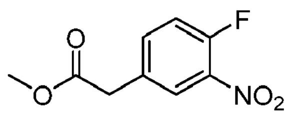

To the solution of 2-(4-fluoro-3-nitro-phenyl)acetic acid (4.11 g, 20.6 mmol) in MeOH (20 mL) was added $H_2SO_4$ (376 mg, 3.75 mmol) dropwise. Then the mixture was stirred at 80 °C for 16 hrs. The reaction mixture was poured onto a mixture of *sat. aq.* $NaHCO_3$ and ice. The mixture was extracted with EtOAc (30 mL* 3). The combined organics were dried over anhydrous $Na_2SO_4$, filtered and concentrated to give methyl 2-(4-fluoro-3-nitro-phenyl)acetate (4.04 g, crude) as brown gum. **$^1$H NMR** (400MHz, $CDCl_3$) $\delta$ 8.01 (dd, *J* = 7.2, 2.4 Hz, 1H), 7.60 - 754 (m, 1H), 7.31 - 7.24 (m, 1H), 3.74 (s, 3H).

Step C methyl 2-[4-(methylamino)-3-nitro-phenyl]acetate

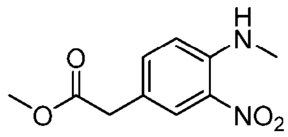

To the solution of methyl 2-(4-fluoro-3-nitro-phenyl)acetate (2.00 g, crude) and methanamine (425 mg, 6.29 mmol, HCl salt) in DMF (10 mL) was added $Cs_2CO_3$ (4.27 g, 13.1 mmol) at 25 °C. The mixture was stirred at 25 °C for 0.5 hr, and then stirred at 60 °C for 8 hrs. The reaction mixture was diluted with *sat. aq.* $NH_4Cl$ (30 mL) and $H_2O$ (20 mL). The mixture was extracted with EtOAc (30 mL* 3). The organic layers were combined, washed with brine (50 mL), dried over $Na_2SO_4$. The solvent was removed to give a residue, the residue was purified by flash chromatography (PE/EtOAc = 3/1) to give methyl 2-[4-(methylamino)-3-nitrophenyl]acetate (0.957 g, 43.9% over 3 steps) as an orange solid. **$^1$H NMR** (400MHz, $CDCl_3$) $\delta$ 8.08 (d, *J* = 2.0 Hz, 1H), 8.03 (br s, 1H), 7.42 (dd, *J* = 8.8, 2.0 Hz, 1H), 6.84 (d, *J* = 8.8 Hz, 1H), 3.71 (s, 3H), 3.56 (s, 2H), 3.03 (d, *J* = 5.2 Hz, 3H).

Step D methyl 2-(3-amino-4-(methylamino)phenyl)acetate

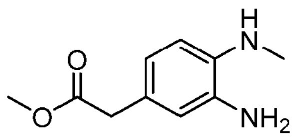

To a solution of methyl 2-[4-(methylamino)-3-nitro-phenyl] acetate (950mg, 4.24 mmol) in MeOH (20 mL) was added 10% Pd/C (200 mg). The reaction mixture was stirred under 15 Psi $H_2$ for 16 hrs. The reaction mixture was filtered and concentrated to give methyl 2-[3-amino-4-(methylamino)phenyl]acetate (800 mg, crude) as a black oil. The residue was used into next step without further purification. **$^1$H NMR** (400 MHz, $CDCl_3$) $\delta$ 6.75 (dd, *J* = 8.0, 2.0 Hz, 1H), 6.67 (d, *J* = 2.0 Hz, 1H), 6.61 (d, *J* = 8.0 Hz, 1H), 3.68 (s, 3H), 3.50 (s, 2H), 3.33 (br s, 2H), 2.86 (s, 3H).

Step E 2-[2-[(2S,3R)-2-(cyclobutylmethoxy)-3-(3,5-dimethoxy-4-methyl-phenyl)-3-hydroxy-propyl]-1-methyl-benzimidazol-5-yl]acetic acid **(Compound 120a)**

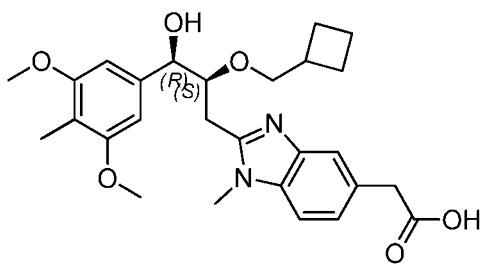

**Example 22 (Compound 120a)** was synthesized according to the procedures described for the preparation of **Example 16** (step A to step D in Scheme 165) by using (3S,4R)-4-((tert-butyldimethylsilyl)oxy)-3-(cyclobutylmethoxy)-4-(3,5-dimethoxy-4-methyl phenyl)butanenitrile in step A and methyl 2-[3-amino-4-(methylamino)phenyl]acetate in step B. **$^1$H NMR** (400 MHz, DMSO-*d6*) $\delta$ 7.48 (d, *J* = 6.0 Hz, 2H), 7.06 (dd, *J* = 7.6, 1.2 Hz, 1H), 6.69 (s, 2H), 5.56 (br s, 1H), 4.67 (d, *J* = 5.2 Hz, 1H), 3.84 - 3.80 (m, 1H), 3.77 (s, 6H), 3.70 (s, 3H), 3.61 (s, 2H), 3.20 (dd, *J* = 9.4, 6.4 Hz, 1H), 3.06 - 2.97 (m, 3H), 2.15-2.12 (m, 1H), 1.98 (s, 3H), 1.71 - 1.57 (m, 3H), 1.57 - 1.47 (m, 1H), 1.38 - 1.25 (m, 2H). **LC-MS:** m/z 483.2 $(M+H)^+$.

**Example 23**

2-((2S,3R)-3-(4-chloro-3-methoxyphenyl)-2-(cyclopentyloxy)-3-hydroxypropyl)-2H-indazole-7-carboxylic acid **(Compound 126a)**

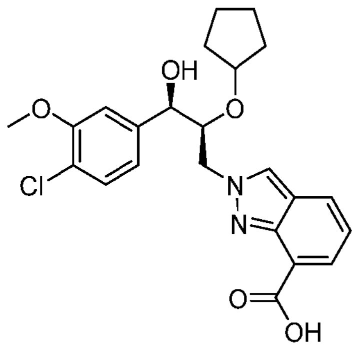

**Example 23 (Compound 126a)** was synthesized according to the procedures described for the preparation of **Example 1** (step C to step N in Scheme 1) by using cyclopentanol in step C and 4-chloro-3-methoxybenzaldehyde in step F. **$^1$H NMR** (400 MHz, $CD_3OD$) $\delta$ 8.33 (s, 1H), 8.02 (dd, *J* = 7.2, 1.2 Hz, 1H), 7.95 (dd, *J* = 8.4, 1.2 Hz, 1H), 7.33 (d, *J* = 8.0 Hz, 1H), 7.20 - 7.14 (m, 2H), 7.01 (dd, *J* = 8.4, 2.0 Hz, 1H), 4.77 (dd, *J* = 13.6, 2.8 Hz, 1H), 4.63 (d, *J* = 4.0 Hz, 1H), 4.55 (dd, *J* = 14.0, 8.0 Hz, 1H), 4.06 - 3.98 (m, 1H), 4.01 (s, 3H), 3.58 - 3.51 (m, 1H), 1.39 - 1.30 (m, 4H), 1.19 - 1.12 (m, 3H), 0.98 - 0.87 (m, 1H). **LC-MS:** m/z 445.1 $(M+H)^+$.

**Example 24**

2-((2S,3R)-2-(cyclopentyloxy)-3-(3,5-dimethoxy-4-methylphenyl)-3-hydroxypropyl)benzo[d]thiazole-4-carboxylic acid **(compound 157)**

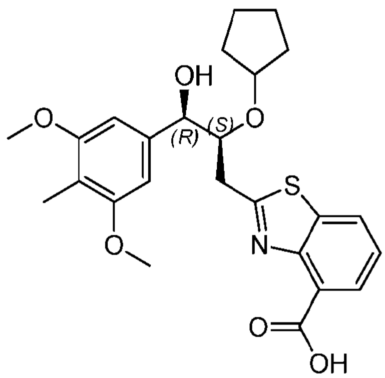

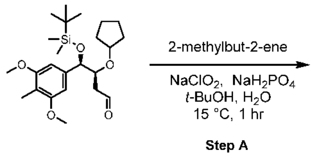


16-1 24-1

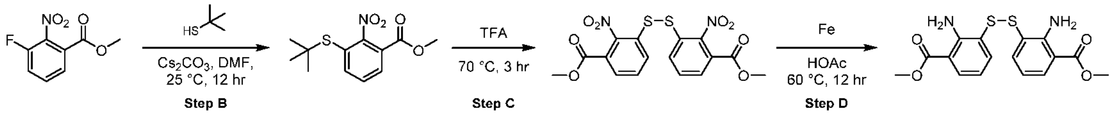


24-2 24-3 24-4 24-5

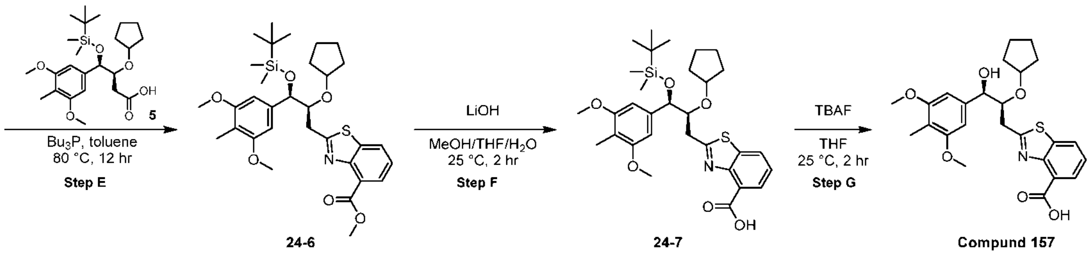


24-6 24-7 Compund 157

Step A (3S,4R)-4-((tert-butyldimethylsilyl)oxy)-3-(cyclopentyloxy)-4-(3,5-dimethoxy-4-methylphenyl)butanoic acid

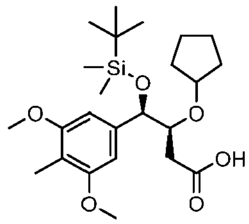

To a solution of (3S,4R)-4-[tert-butyl(dimethyl)silyl]oxy-3-(cyclopentoxy)-4-(3,5-dimethoxy-4-methyl-phenyl)butanal (1.85 g, 4.24 mmol) in t-BuOH (40 mL) and $H_2O$ (10 mL) was added $NaH_2PO_4$ (762.47 mg, 6.36 mmol), $NaClO_2$ (1.38 g, 15.25 mmol) and 2-methylbut-2-ene (1.34 g, 19.07 mmol) at 0 °C. The mixture was stirred at 15 °C for 1 hr. The mixture was diluted with $H_2O$ (40 mL), extracted with EtOAc (50 mL*3). The combined organic layers were washed with brine, dried over anhydrous $Na_2SO_4$, filtered and concentrated to afford the title compound (3S,4R)-4-[tert-butyl(dimethyl)silyl]oxy-3-(cyclopentoxy)-4-(3,5-dimethoxy-4-methylphenyl)butanoic acid (1.9 g, crude) as yellow oil which was used for next step without further purification. **LC-MS:** m/z 475.2$(M+Na)^+$.

Step B methyl 3-(tert-butylthio)-2-nitrobenzoate

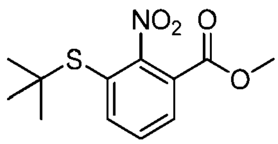

To a solution of methyl 3-fluoro-2-nitro-benzoate (2 g, 10.04 mmol) in DMF (30 mL) was added $Cs_2CO_3$ (6.54 g, 20.09 mmol) and 2-methylpropane-2-thiol (815 mg, 9.04 mmol). The solution was stirred at 25 °C for 12 hrs. The solution was diluted with $H_2O$ (100 mL), extracted with EtOAc (30 mL*2). The organics were combined, dried over $Na_2SO_4$, filtered and concentrated. The residue was purified by silica gel column (PE/EtOAc = 5/1) to give methyl 3-tert-butylsulfanyl-2-nitro-benzoate (2.3 g, 85.0% yield). **$^1$H NMR** (400 MHz, $CD_3OD$) $\delta$ 8.07 (dd, $J$ = 9.8, 1.4 Hz, 1H), 7.96 (dd, $J$ = 8.0, 1.6 Hz, 1H),

7.63 (t, $J$ = 8.0 Hz, 1H), 3.86 (s, 3H), 1.28 (s, 9H).

Step C dimethyl 3,3'-disulfanediylbis(2-nitrobenzoate)

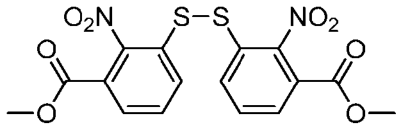

The solution of methyl 3-tert-butylsulfanyl-2-nitro-benzoate (7 g, 25.99 mmol) in TFA (70 mL) was stirred at 70 °C for 3 hrs. The solvent was removed in vacuo. The residue was dissolved in EtOAc (200 mL), washed with *aq.* $NaHCO_3$ (100 mL). The organics were combined, dried over $Na_2SO_4$, filtered and concentrated. The residue was purified by silica gel column (PE/EtOAc = 0/1) to give methyl 3-[(3-methoxycarbonyl-2-nitro-phenyl)disulfanyl]-2-nitro-benzoate (2.7 g, crude) as yellow solid. **LC-MS:** m/z 447.0 $(M+Na)^+$.

Step D dimethyl 3,3'-disulfanediylbis(2-aminobenzoate)

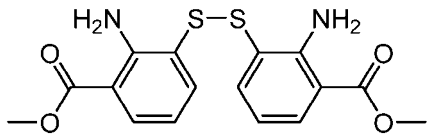

To a solution of methyl 3-[(3-methoxycarbonyl-2-nitro-phenyl)disulfanyl]-2-nitrobenzoate (2.7 g, 6.36 mmol) in AcOH (20 mL) was added Fe (1.78 g, 31.81 mmol). The solution was stirred at 60 °C for 12 hrs. The reaction mixture was filtered. The filtrate was collected and concentrated. The residue was dissolved in EtOAc (200 mL), washed with *aq.* $NaHCO_3$ (100 mL). The organics were combined, dried over $Na_2SO_4$, filtered and concentrated. The residue was purified by silica gel column (PE/EtOAc = 10/1) to give methyl 2-amino-3-[(2-amino-3-methoxycarbonyl-phenyl)disulfanyl]benzoate (1.86 g, 74.0% yield) as yellow solid. **LC-MS:** m/z 364.7 $(M+H)^+$.

Step E methyl 2-((2S,3R)-3-((tert-butyldimethylsilyl)oxy)-2-(cyclopentyloxy)-3-(3,5-dimethoxy-4-methylphenyl)propyl) benzo[d]thiazole-4-carboxylate

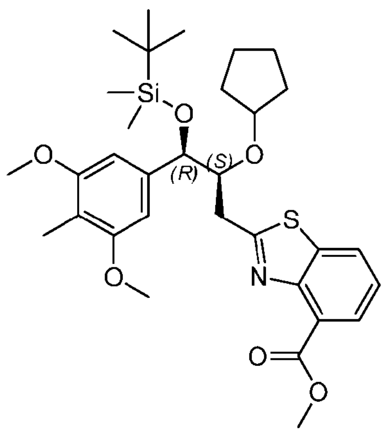

To a solution of methyl 2-amino-3-[(2-amino-3-methoxycarbonyl-phenyl)disulfanyl]benzoate (1.37 g, 3.76 mmol) in toluene (10 mL) was added tributylphosphane (2.28 g, 11.27 mmol). The solution was stirred at 25 °C for 10 mins. Then (3S,4R)-4-[tert-butyl(dimethyl)silyl]oxy-3-(cyclopentoxy)-4-(3,5-dimethoxy-4-methyl-phenyl)butanoic acid (1.7 g, 3.76 mmol) was added. The solution was stirred at 80 °C for 12 hrs. The solvent was removed in vacuo to give a residue. The residue was purified by silica gel column (PE/EtOAc = 5/1) to give methyl 2-[(2S,3R)-3-[tert-butyl(dimethyl)silyl]oxy-2-(cyclopentoxy)-3-(3,5-dimethoxy-4-methylphenyl)propyl]-1,3-benzothiazole-4-carboxylate (1.73 g, 48.4% yield) as yellow oil. **LC-MS:** m/z 600.1 $(M+H)^+$.

Step F 2-((2S,3R)-3-((tert-butyldimethylsilyl)oxy)-2-(cyclopentyloxy)-3-(3,5-dimethoxy-4-methylphenyl)propyl)benzo[d] thiazole-4-carboxylic acid

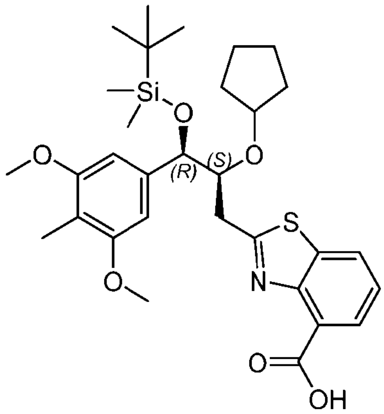

To a solution of methyl 2-[(2S,3R)-3-[tert-butyl(dimethyl)silyl]oxy-2-(cyclopentoxy)-3-(3,5-dimethoxy-4-methyl-phenyl) propyl]-1,3-benzothiazole-4-carboxylate (1.73 g, 2.88 mmol) in MeOH (8 mL), THF (8 mL) and $H_2O$ (8 mL) was added NaOH (1.73 g, 43.26 mmol). The solution was stirred at 25 °C for 2 hrs. The solution was adjusted to *p*H = 6 with 1N HCl, extracted with EtOAc (30 mL*3). The organics were combined, dried over $Na_2SO_4$, filtered and concentrated to give 2-[(2S,3R)-3-[tert-butyl(dimethyl)silyl]oxy-2-(cyclopentoxy)-3-(3,5-dimethoxy-4-methylphenyl)propyl]-1,3-benzothiazole-4-carboxylic acid (1.4 g, crude) as yellow oil, which was used directly for the next step without further purification.

Step G 2-((2S,3R)-2-(cyclopentyloxy)-3-(3,5-dimethoxy-4-methylphenyl)-3-hydroxypropyl)benzo[d]thiazole-4-carboxylic acid **(compound 157)**

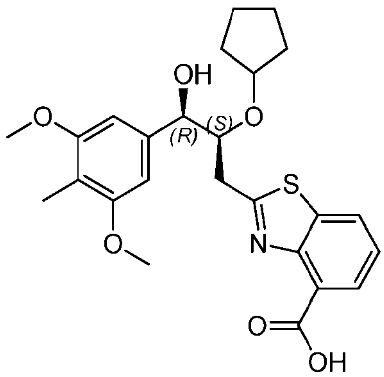

To a solution of 2-[(2S,3R)-3-[tert-butyl(dimethyl)silyl]oxy-2-(cyclopentoxy)-3-(3,5-dimethoxy-4-methyl-phenyl)propyl]-1,3-benzothiazole-4-carboxylic acid (1.4 g, 2.39 mmol) in THF (10 mL) was added TBAF (1 M, 23.9 mL). The solution was stirred at 25 °C for 2 hrs. The solution was diluted with $H_2O$ (100 mL), extracted with EtOAc (200 mL). The organics were collected, washed with $H_2O$ (150 mL*5). The organics were combined, dried over $Na_2SO_4$, filtered and concentrated. The residue was purified by *prep-HPLC* (Neu) (column: Daisogel C18 250*50mm*8um; mobile phase: [water (10 mM $NH_4HCO_3$)-ACN]; B%: 5%-55%, 23 min) to give 2-[(2S,3R)-2-(cyclopentoxy)-3-(3,5-dimethoxy-4-methyl-phenyl)-3-hydroxy-propyl]-1,3-benzothiazole-4-carboxylic acid (765.89 mg, 67.9% yield) as white solid. **$^1$H NMR** (400 MHz, $CD_3OD$) $\delta$ 8.11 (d, *J* = 8.0 Hz, 1H), 7.99 (d, *J* = 8.0 Hz, 1H), 7.50 (t, *J* = 8.0 Hz, 1H), 6.67 (s, 2H), 4.67 (d, *J* = 8.0 Hz, 1H), 4.03 - 3.97 (m, 1H), 3.95 -3.89 (m, 1H), 3.83 (s, 6H), 3.48 (d, *J* = 4.0 Hz, 2H), 2.01 (s, 3H), 1.55 - 1.32 (m, 8H). LC-MS: m/z 472.1$(M+H)^+$.

**Example 25**

2-((2*S*,3*R*)-2-(cyclopentyloxy)-3-(3,5-dimethoxy-4-methylphenyl)-3-hydroxypropyl)-2*H*-pyrazolo[4,3-*b*]pyridine-7-carboxylic acid **(compound 160)**

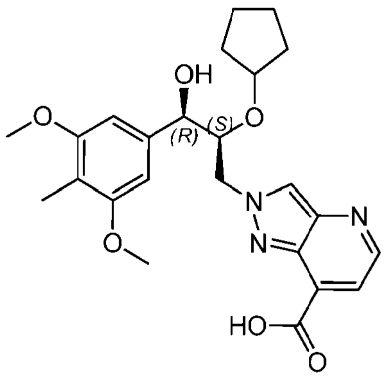

**Example** 25 (compound 160) was synthesized according to the procedures described for the preparation of **Example 1** (step A to step N in Scheme 1) by using cyclopentanol in step C and methyl 2H-pyrazolo[4,3-b]pyridine-7-carboxylate in step L. **$^1$H NMR** (400 MHz, $CD_3OD$) $\delta$ 8.55 (d, *J* = 4.4 Hz, 1H), 8.48 (s, 1H), 7.77 (d, *J* = 4.4 Hz, 1H), 6.60 (s, 2H), 4.72 - 4.66 (m, 1H), 4.57 (d, *J* = 6.0 Hz, 1H), 4.54 - 4.46 (m, 1H), 4.00 - 3.93 (m, 1H), 3.73 (s, 6H), 3.51 - 3.42 (m, 1H), 1.91 (s, 3H), 1.33 - 0.95 (m, 7H), 0.82 - 0.68 (m, 1H). LC-MS: m/z 456.4 $(M+H)^+$.

**Example 26**

2-((2S,3R)-2-(cyclopentyloxy)-3-(3,5-dimethoxy-4-methylphenyl)-3-hydroxypropyl)-4-methoxypyrazolo[1,5-a]pyridine-7-carboxylic acid **(compound 237)**

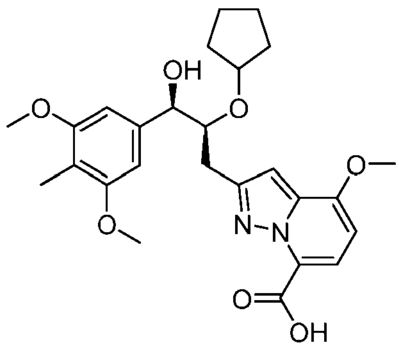

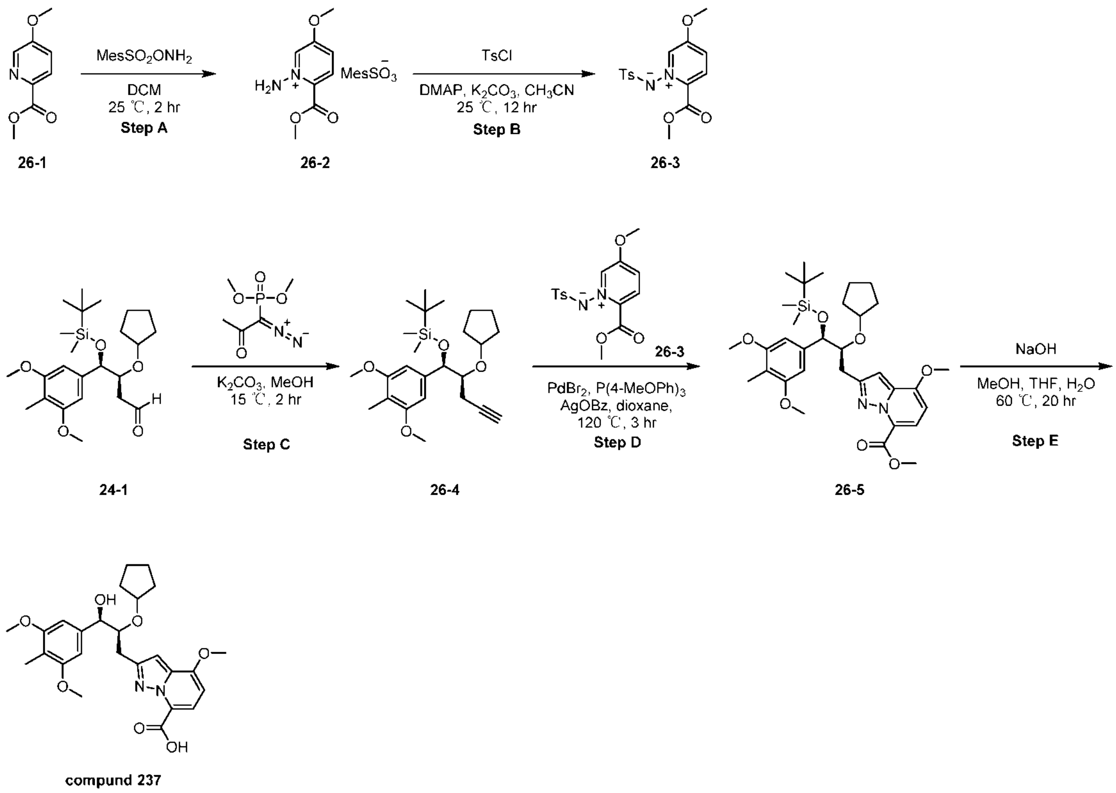


Step A 1-amino-5-methoxy-2-(methoxycarbonyl)pyridin-1-ium 2,4,6-trimethylbenzenesulfonate

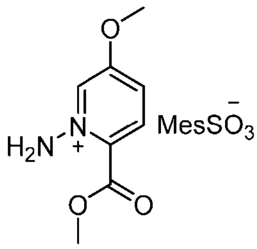

To a solution of *O*-mesitylenesulphonylhydroxylamine (5.92 g, 27.5 mmol) in DCM (50 mL) was added methyl 5-methoxypyridine-2-carboxylate (2 g, 12.0 mmol) in one portion, the resulting mixture was stirred at 25 °C for 2 hrs. The solvent was removed under reduced pressure to give the product 1-amino-5-methoxy-2-(methoxycarbonyl)pyridin-1-ium 2,4,6-trimethylbenzenesulfonate (6 g, crude), which used directly in the next step without further purification.

Step B (5-methoxy-2-(methoxycarbonyl)pyridin-1-ium-1-yl)(tosyl)amide

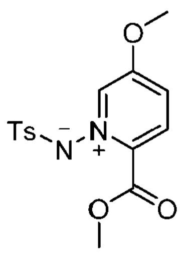

To a solution of methyl 1-amino-5-methoxy-pyridin-1-ium-2-carboxylate;2,4,6-trimethylbenzenesulfonate (6 g, crude) in

MeCN (100 mL) was added DMAP (192 mg, 1.6 mmol) and $K_2CO_3$ (6.5 g, 47.1 mmol), followed by addition of *p*-TsCl (3.29 g, 17.3 mmol) in portions at 0 °C. The resulting mixture was stirred at 25 °C for 12 hrs. The mixture was diluted with MeOH (50 mL), the mixture was filtered and washed with MeOH (40 mL*3). After concentration of the filtration, the residue was purified by flash silica gel chromatography (ISCO®; 80 g SepaFlash® Silica Flash Column, Eluent of 0~3% MeOH/DCM gradient @ 100 mL/min) to give (5-methoxy-2-(methoxycarbonyl)pyridin-1-ium-1-yl)(tosyl)amide (1.7 g, 32.2% yield) as a white solid. **LC-MS:** m/z 336.9 $(M+H)^+$.

Step C tert-butyl(((1R,2S)-2-(cyclopentyloxy)-1-(3,5-dimethoxy-4-methylphenyl)pent-4-yn-1-yl)oxy)dimethylsilane

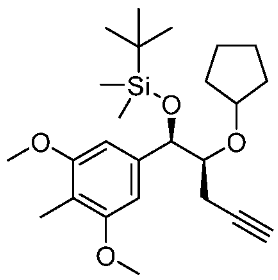

At 0 °C, to the mixture of (3S,4R)-4-[tert-butyl(dimethyl)silyl]oxy-3-(cyclopentoxy)-4-(3,5-dimethoxy-4-methyl-phenyl) butanal (7.2 g, 16.5 mmol) and $K_2CO_3$ (4.56 g, 33.0 mmol) in MeOH (70 mL) was added dimethyl 1-(1-diazo-2-oxopropyl) phosphonate (3.8 g, 19.8 mmol) in dropwise. The mixture was stirred under nitrogen at 15 °C for 16 hrs. The mixture was concentrated under vacuum. The residue was purified by flash silica gel chromatography (ISCO®; 220g SepaFlash® Silica Flash Column, Eluent of 0~4% Ethyl acetate/Petroleum ether gradient @ 150 mL/min) to give tert-butyl-[(1R,2S)-2-(cyclopentoxy)-1-(3,5-dimethoxy-4-methylphenyl)pent-4-ynoxy]-dimethyl-silane (3.2 g, 44.9% yield) as a colorless oil. **$^1$H NMR** (400 MHz, $CDCl_3$) $\delta$ 6.54 (s, 2H), 4.59 - 4.58 (m, 1H), 3.85 - 3.81 (m, 7H), 3.50 - 3.46 (m, 1H), 2.48 - 2.46 (m, 2H), 2.07 (s, 3H), 1.97 (s, 1H), 1.46 - 1.34 (m, 8H), 0.90 (s, 9H), 0.07 (s, 3H), -0.15 (s, 3H).

Step D methyl 2-((2S,3R)-3-((tert-butyldimethylsilyl)oxy)-2-(cyclopentyloxy)-3-(3,5-dimethoxy-4-methylphenyl)propyl)-4-methoxypyrazolo[1,5-a]pyridine-7-carboxylate

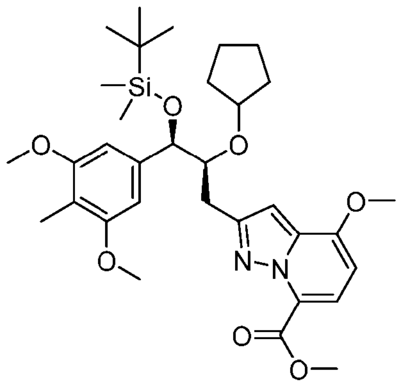

To the mixture of (5-methoxy-2-methoxycarbonyl-pyridin-1-ium-1-yl)-(p-tolylsulfonyl)azanide (296 mg, 881 umol) and tert-butyl-[(1R,2S)-2-(cyclopentoxy)-1-(3,5-dimethoxy-4-methyl-phenyl)pent-4-ynoxy]-dimethyl-silane (400 mg, 925 umol) in dioxane (5 mL) were added tris(4-methoxyphenyl)phosphane (46.5 mg, 132 umol), silver benzoate (605 mg, 2.6 mmol) and dibromopalladium (11.7 mg, 44.0 umol). The mixture was stirred under nitrogen at 120 °Cfor 3 hrs. After cooling, the mixture was diluted with ethyl acetate (30 mL), washed with 30% *aq.* $K_2CO_3$ (30 mL). The organic layer was dried over $Na_2SO_4$ and concentrated. The residue was purified by flash silica gel chromatography (ISCO®; 20 g SepaFlash® Silica Flash Column, Eluent of 0~22% Ethyl acetate/Petroleum ether gradient @ 40 mL/min) and further purified by Prep-HPLC to give methyl 2-[(2S,3R)-3-[tert-butyl(dimethyl)silyl]oxy-2-(cyclopentoxy)-3-(3,5-dimethoxy-4-methyl-phenyl)propyl]-4-methoxy-pyrazolo[1,5-a]pyridine-7-carboxylate (120 mg, 22.2% yield) as a colorless oil. **$^1$H NMR** (400 MHz, $CDCl_3$) $\delta$ 8.04 (s, 1H), 7.54 - 7.51 (m, 1H), 6.60 (s, 2H), 6.35 (d, *J* = 8.0 Hz, 1H), 4.66 - 4.65 (m, 1H), 4.00 (s, 3H),

3.97 (s, 3H), 3.82 - 3.80 (m, 7H), 3.64 - 3.62 (m, 1H), 3.25 - 3.21 (m, 1H), 2.99 - 2.93 (m, 1H), 2.06 (s, 3H), 1.47 - 1.19 (m, 8H), 0.91 (s, 9H), 0.07 (s, 3H), -0.13 (s, 3H).

Step E 2-((2S,3R)-2-(cyclopentyloxy)-3-(3,5-dimethoxy-4-methylphenyl)-3-hydroxypropyl)-4-methoxypyrazolo[1,5-a]pyridine-7-carboxylic acid **(compound 237)**

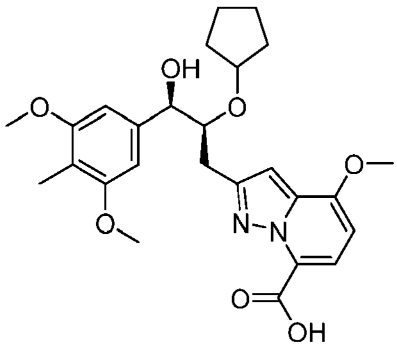

To the mixture of methyl 2-[(2S,3R)-3-[tert-butyl(dimethyl)silyl]oxy-2-(cyclopentoxy)-3-(3,5-dimethoxy-4-methyl-phenyl)propyl]-4-methoxy-pyrazolo[1,5-a]pyridine-7-carboxylate (80 mg, 131 umol) in MeOH (6 mL) and THF (2 mL) was added NaOH (1.57 g, 39.2 mmol) in $H_2O$ (2 mL). The mixture was stirred at 60 °C for 20 hrs. After removal of the organic solvent, the aqueous layer was acidified with 6 N HCl to pH = 4-5. The mixture was extracted with ethyl acetate (30 mL*3). The combined organic layer was dried over $Na_2SO_4$ and concentrated under vacuum. The residue was purified by Prep-HPLC (Column: Kromasil 100-5-C18; Eluent: 65% to 100% water (0.1% HCOOH)-ACN) to give 2-[(2S,3R)-2-(cyclopentoxy)-3-(3,5-dimethoxy-4-methyl-phenyl)-3-hydroxy-propyl]-4-methoxy-pyrazolo[1,5-a]pyridine-7-carboxylic acid (20.07 mg, 30.9% yield) as a white solid. **$^1$H NMR** (400 MHz, $CD_3OD$) $\delta$ 7.68 (d, $J$ = 8.0 Hz, 1H), 6.68 (d, $J$ = 8.0 Hz, 1H), 6.58 (s, 1H), 6.52 (s, 2H), 4.51 - 4.49 (m, 1H), 3.97 (s, 3H), 3.76 - 3.70 (m, 8H), 3.12 - 3.07 (m, 1H), 2.99 - 2.96 (m, 1H), 1.87 (s, 3H), 1.21 - 1.36 (m, 8H). **LC-MS:** m/z 485.2 $(M+H)^+$.

**Example 27**

2-((2S,3R)-2-(cyclopentyloxy)-3-(3,5-dimethoxy-4-methylphenyl)-3-hydroxypropyl)pyrazolo[1,5-a]pyridine-7-carboxylic acid **(compound 169)**

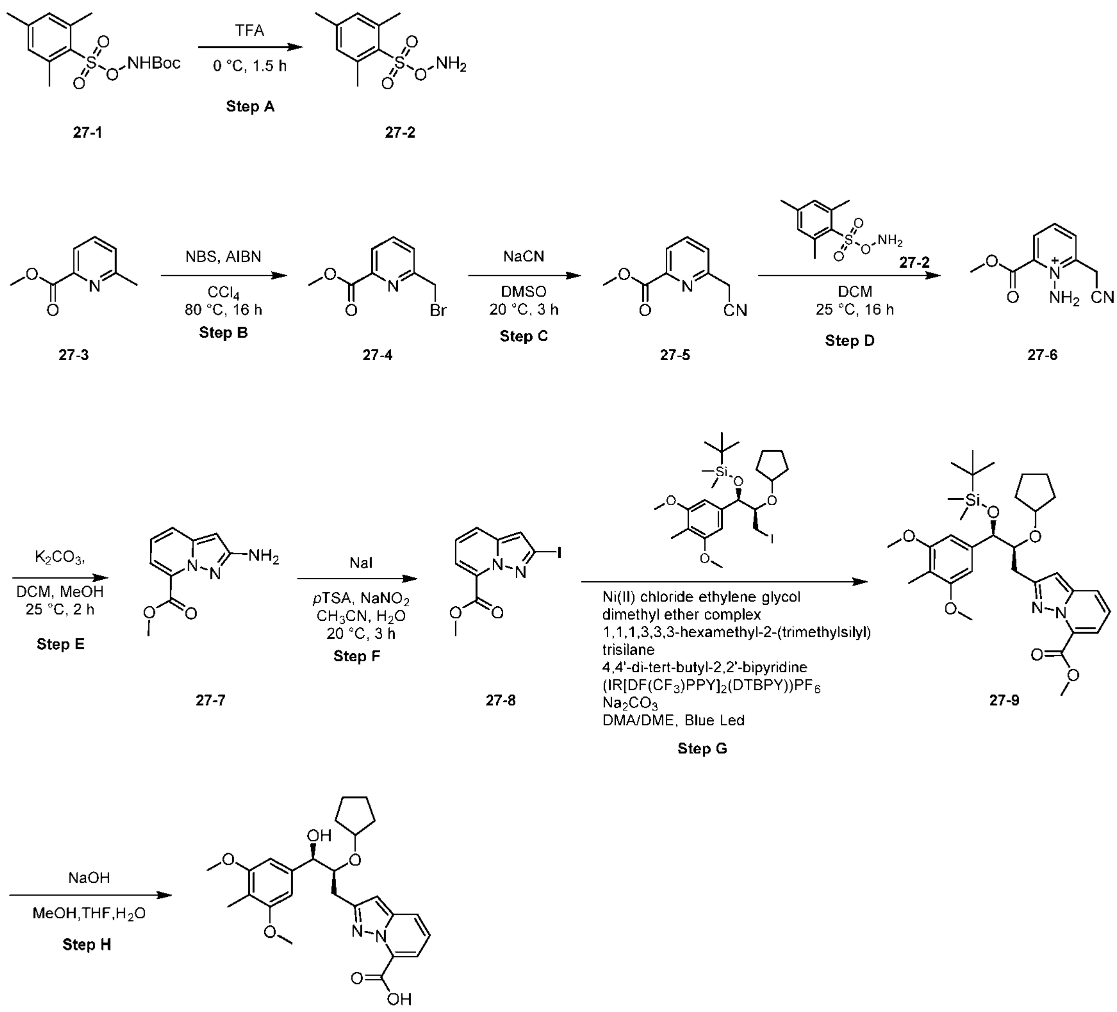


compound 169

Step A O-(mesitylsulfonyl)hydroxylamine

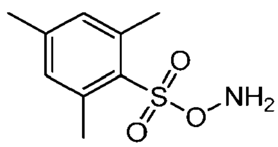

The solution of tert-butyl (mesitylsulfonyl)oxycarbamate (18 g, 57.07 mmol) in TFA (80 mL) was stirred at 0 °C for 1.5 hrs. The mixture was poured into the ice water, then filtered and the solid was washed with $H_2O$ and dried to give *O*-(mesitylsulfonyl)hydroxylamine (12.49 g, crude) as white solid.

Step B methyl 6-(bromomethyl)picolinate

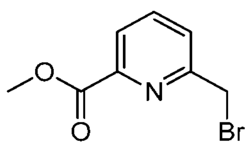

To a solution of methyl 6-methylpyridine-2-carboxylate (25 g, 165.39 mmol) and AIBN (2.72 g, 16.54 mmol) in $CCl_4$ (300

mL) was added NBS (32.38 g, 181.92 mmol). The mixture was stirred at 80 °C for 16 hrs. The reaction mixture was concentrated under reduced pressure and then the residue was diluted with $H_2O$ (300 mL) and extracted with DCM (300 mL*3). The combined organics were dried over anhydrous $Na_2SO_4$, filtered and concentrated under reduced pressure. The residue was purified by silica gel column (PE/EtOAc = 4/1) to give the product methyl 6-(bromomethyl)pyridine-2-carboxylate (10.09 g, 26.5% yield) as light yellow solid. **LC-MS:** m/z 231.7 $(M+H)^+$ .

Step C methyl 6-(cyanomethyl)picolinate

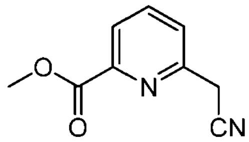

To a solution of methyl 6-(bromomethyl)pyridine-2-carboxylate (10.09 g, 43.86 mmol) in DMSO (100 mL) was added NaCN (4.3 g, 87.72 mmol). The mixture was stirred at 20 °C for 3 hrs. The mixture was diluted with $H_2O$ (150 mL), extracted with EtOAc (100 mL*3). The combined organic layers were dried over anhydrous $Na_2SO_4$, filtered and concentrated under reduced pressure. The residue was purified by silica gel column (PE/EtOAc = 0/1) to give the product methyl 6-(cyanomethyl)pyridine-2-carboxylate (4.64 g, 59.1% yield) as yellow oil. **LC-MS:** m/z 176.7 $(M+H)^+$ .

Step D 1-amino-2-(cyanomethyl)-6-(methoxycarbonyl)pyridin-1-ium

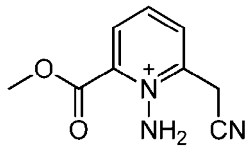

To a solution of methyl 6-(cyanomethyl)pyridine-2-carboxylate (4.64 g, 26.34 mmol) in DCM (25 mL) was added *O*-(mesitylsulfonyl)hydroxylamine (10.21 g, 47.41 mmol). The mixture was stirred at 25 °C for 16 hrs. The solution was used directly in the next step. **LC-MS:** m/z 191.7 $(M+H)^+$ .

Step E methyl 2-aminopyrazolo[1,5-a]pyridine-7-carboxylate

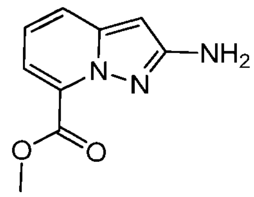

To a solution of methyl 1-amino-6-(cyanomethyl)pyridin-1-ium-2-carboxylate (5.06 g, theory amount) in DCM (25 mL) and MeOH (100 mL) was added $K_2CO_3$ (7.28 g, 52.66 mmol). The mixture was stirred at 25 °C for 2 hrs. The reaction mixture was diluted with $H_2O$ (80 mL), extracted with DCM (60 mL*3). The combined organic layers were washed with brine (60 mL*2), dried over anhydrous $Na_2SO_4$, filtered and concentrated. The residue was purified by silica gel column (PE/EtOAc = 0/1) to give the product methyl 2-aminopyrazolo[1,5-a]pyridine-7-carboxylate (1.6 g, 31.8% yield) as yellow solid. **LC-MS:** m/z 191.8 $(M)^+$.

Step F methyl 2-iodopyrazolo[1,5-a]pyridine-7-carboxylate

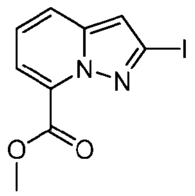

To a solution of methyl 2-aminopyrazolo[1,5-a]pyridine-7-carboxylate (1.6 g, 8.37 mmol) in $CH_3CN$ (15 mL) was added 4-methylbenzenesulfonic acid hydrate (4.78 g, 25.11 mmol) and then a solution of $NaNO_2$ (1.15 g, 16.74 mmol) in $H_2O$ (3 mL) at 0 °C dropwise. The mixture was stirred at 0 °C for 30 mins and a solution of NaI (3.14 g, 20.92 mmol) in $H_2O$ (3 mL) was added dropwise. The mixture was stirred at 20 °C for 3 hrs. The mixture was diluted with $H_2O$ (60 mL), extracted with EtOAc (40 mL*3). The combined organic layers were dried over anhydrous $Na_2SO_4$, filtered and concentrated under reduced pressure. The residue was purified by silica gel column (PE/EtOAc = 3/1) to give the product methyl 2-iodopyrazolo[1,5-a]pyridine-7-carboxylate (323 mg, 12.8% yield) as yellow solid. **LC-MS:** m/z 302.6 $(M+H)^+$.

Step G methyl 2-((2S,3R)-3-((tert-butyldimethylsilyl)oxy)-2-(cyclopentyloxy)-3-(3,5-dimethoxy-4-methylphenyl)propyl) pyrazolo[1,5-a]pyridine-7-carboxylate

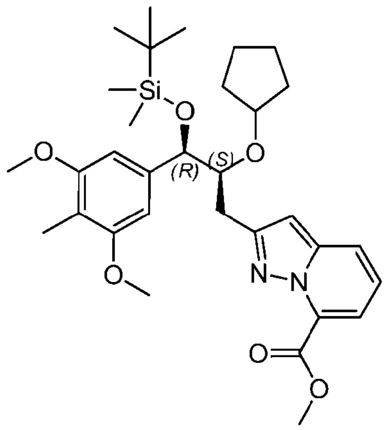

methyl 2-((2S,3R)-3-((tert-butyldimethylsilyl)oxy)-2-(cyclopentyloxy)-3-(3,5-dimethoxy-4-methylphenyl)propyl)pyrazolo [1,5-a]pyridine-7-carboxylate was synthesized according to the procedures described for the preparation of **Example 11 (Compound 106a)** (step A to step B in Scheme 11) by using methyl [(2S,3R)-3-[tert-butyl(dimethyl)silyl]oxy-2-(cyclopentoxy)-3- (3,5-dimethoxy-4-methyl-phenyl)propyl] methanesulfonate in step A and methyl 2-iodopyrazolo[1,5-a] pyridine-7-carboxylate in step B.

Step H 2-((2S,3R)-2-(cyclopentyloxy)-3-(3,5-dimethoxy-4-methylphenyl)-3-hydroxypropyl)pyrazolo[1,5-a]pyridine-7-carboxylic acid (compound 169)

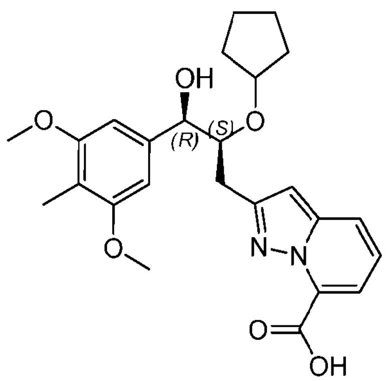

2-((2S,3R)-2-(cyclopentyloxy)-3-(3,5-dimethoxy-4-methylphenyl)-3-hydroxypropyl)pyrazolo[1,5-a]pyridine-7-carboxylic acid was synthesized according to the procedures described for the preparation of **Example 26 (compound 237)** (step E in Scheme 26) by using methyl 2-((2S,3R)-3-((tert-butyldimethylsilyl)oxy)-2-(cyclopentyloxy)-3-(3,5-dimethoxy-4-methylphenyl)propyl)pyrazolo[1,5-a]pyridine-7-carboxylate in step E. **$^1$H NMR** (400 MHz, $CD_3OD$) $\delta$ 7.90 (dd, *J* = 8.0, 1.2 Hz, 1H), 7.81 (dd, *J* = 7.2, 1.2 Hz, 1H), 7.40 (dd, *J* = 8.0, 7.2 Hz, 1H), 6.71 (s, 1H), 6.66 (s, 2H), 4.62 (d, *J* = 4.0 Hz, 1H), 3.91 - 3.85 (m, 1H), 3.84 - 3.78 (m, 7H), 3.27 - 3.20 (m, 1H), 3.16 - 3.07 (m, 1H), 2.00 (s, 3H), 1.51 - 1.26 (m, 8H). **LC-MS:** m/z 455.0 $(M+H)^+$.

**Example 28**

2-(2-((2S,3R)-2-(cyclopentyloxy)-3-(3,5-dimethoxy-4-methylphenyl)-3-hydroxypropyl)-5-methylthiazol-4-yl)acetic acid **(compound 177)**

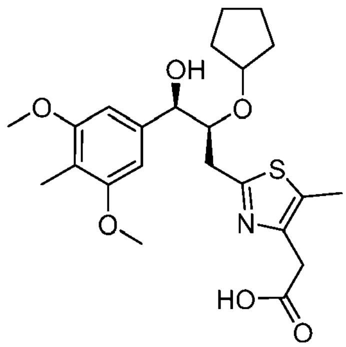

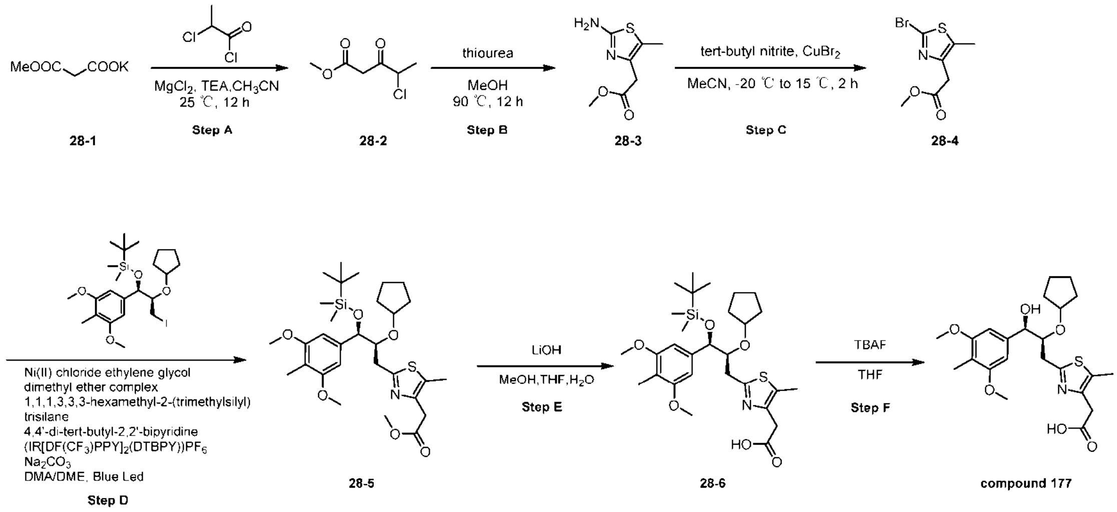


Step A methyl 4-chloro-3-oxopentanoate

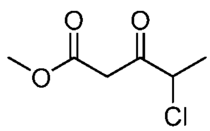

To a suspension of (3-methoxy-3-oxo-propanoyl)oxypotassium (20 g, 128 mmol) in MeCN (150 mL) was added TEA (26.7 mL, 192 mmol). Then $MgCl_2$ (14.5 g, 152 mmol) was added in portions. After being stirred for 3 hrs, the mixture was cooled to 0 °C and 2-chloropropanoyl chloride (9.21 mL, 95.0 mmol) was added dropwise. The resulting mixture was stirred at 25 °C for 12 hrs. The mixture was diluted with EtOAc (100 mL), acidified with 5 N HCl to *p*H = 6. The organics were separated,

dried over anhydrous $Na_2SO_4$, filtered and concentrated. The residue was purified by silica gel column (PE/EtOAc = 10/1) to give the product methyl 4-chloro-3-oxo-pentanoate (9 g, 42.7% yield) as yellow liquid. **$^1$H NMR** (400 MHz, $CDCl_3$) $\delta$ 4.56 - 4.36 (m, 1H), 3.89 - 3.64 (m, 5H), 1.73 - 1.60 (m, 3H).

Step B methyl 2-(2-amino-5-methylthiazol-4-yl)acetate

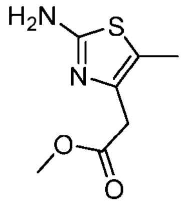

A mixture of methyl 4-chloro-3-oxo-pentanoate (7 g, 42.5 mmol) and thiourea (3.56 g, 46.8 mmol) in MeOH (50 mL) was stirred at 90 °C for 12 hrs. The solvent was removed under reduced pressure to give a residue. The residue was purified by silica gel column (PE/EtOAc = 2/1) to give the product methyl 2-(2-amino-5-methyl-thiazol-4-yl)acetate (5.3 g, 66.9% yield) as white solid. **$^1$H NMR** (400 MHz, $CDCl_3$) $\delta$ 4.77 (br. s, 2H), 3.71 (s, 3H), 3.50 (s, 2H), 2.22 (s, 3H)

Step C methyl 2-(2-bromo-5-methylthiazol-4-yl)acetate

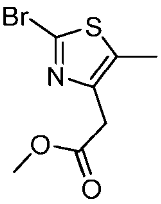

To an ice-cooled suspension of tert-butyl nitrite (1.92 mL, 16.1 mmol) and $CuBr_2$ (3.60 g, 16.11 mmol) in MeCN (100 mL) was added a solution of methyl 2-(2-amino-5-methyl-thiazol-4-yl)acetate (2 g, 10.7 mmol) at -20 °C. The resulting mixture was stirred at 15 °C for 2 hrs. The mixture was poured in $H_2O$ (250 mL), extracted with EtOAc (120 mL*3). The combined organic phase was dried over anhydrous $Na_2SO_4$, filtered and evaporated to give a residue. The residue was purified by silica gel column (PE/EtOAc = 20/1) to give the product methyl 2-(2-bromo-5-methyl-thiazol-4-yl)acetate (1.06 g, 39.5% yield) as colorless oil. **$^1$H NMR** (400 MHz, $CDCl_3$) $\delta$ 3.72 - 3.71 (m, 5H), 2.37 (s, 3H).

Step D methyl 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)acetate

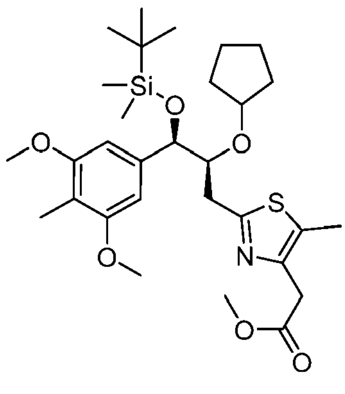

methyl 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)acetate was synthesized according to the procedures described for the preparation of **Example 11 (Compound 106a)** (step A to step B in Scheme 11) by using methyl [(2S,3R)-3-[tert-butyl(dimethyl)silyl]oxy-2-(cyclopentoxy)-3- (3,5-dimethoxy-4-methyl-phenyl)propyl] methanesulfonate in step A in Scheme 11 and methyl 2-(2-bromo-5-methylthiazol-4-yl)acetate in step B in Scheme 11. **LC-MS:** m/z 578.1(M+H)$^+$.

Step E 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)acetic acid

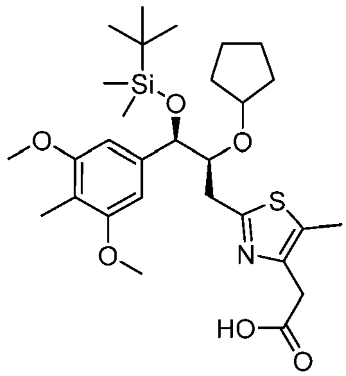

2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)acetic acid was synthesized according to the procedure described in step M for the preparation of **Example 1. LC-MS:** m/z 564.6(M+H)$^+$.

Step F 2-(2-((2S,3R)-2-(cyclopentyloxy)-3-(3,5-dimethoxy-4-methylphenyl)-3-hydroxypropyl)-5-methylthiazol-4-yl) acetic acid

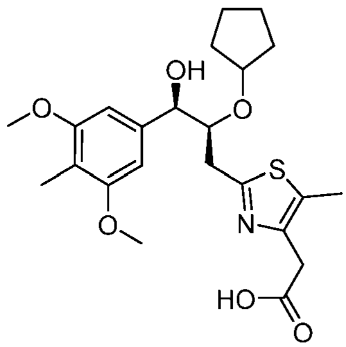

2-(2-((2S,3R)-2-(cyclopentyloxy)-3-(3,5-dimethoxy-4-methylphenyl)-3-hydroxypropyl)-5-methylthiazol-4-yl)acetic acid was synthesized according to the procedure described in step N for the preparation of **Example 1. $^1$H NMR** (400 MHz, $CD_3OD$) $\delta$ 6.67 (s, 2H), 4.67 (d, $J$ = 5.2 Hz, 1H), 3.83 (s, 6H), 3.82 - 3.77 (m, 2H), 3.68 (s, 2H), 3.18 - 3.06 (m, 2H), 2.38 (s, 3H), 2.03 (s, 3H), 1.52 -1.37 (m, 7H), 1.36 - 1.32 (m, 1H). **LC-MS:** m/z 450.5 (M+H)$^+$.

**Example 29**

2-((2S,3R)-3-(3,5-dimethoxy-4-methylphenyl)-3-hydroxy-2-isobutoxypropyl)-6-methylbenzo[d]thiazole-4-carboxylic acid (compound 234)

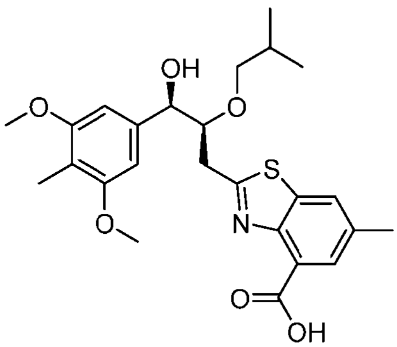

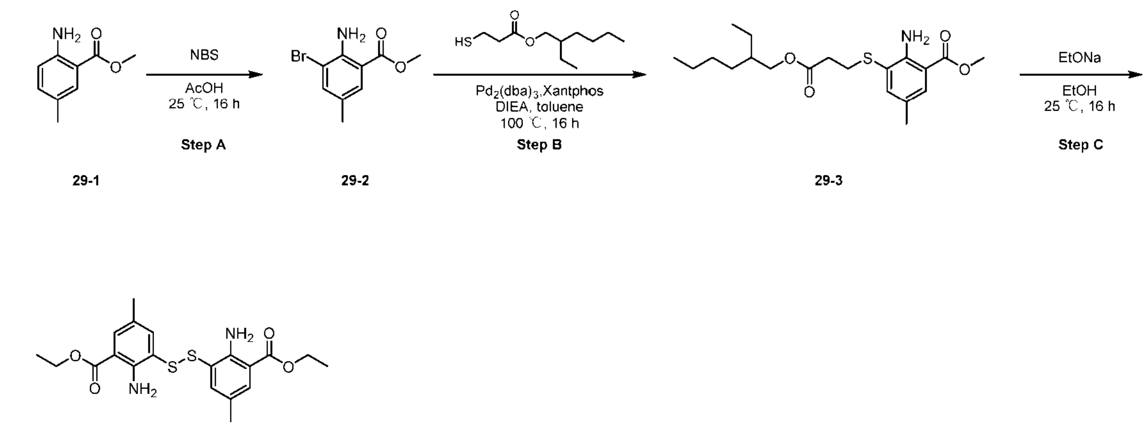


29-1 29-2 29-3

29-4

Step A methyl 2-amino-3-bromo-5-methylbenzoate

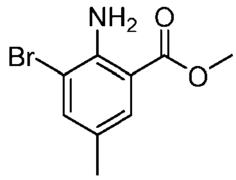

To a solution of methyl 2-amino-5-methylbenzoate (5 g, 30.27 mmol) in AcOH (30 mL) was added NBS (5.39 g, 30.27 mmol), and the mixture was stirred at 25 °C for 2 hrs. The *p*H was adjusted to 6-7 with *sat. aq.* $NaHCO_3$ solution. The mixture was extracted with EtOAc (80 mL*3). The combined organics were dried over anhydrous $Na_2SO_4$, filtered and concentrated under reduced pressure to give a residue. The residue was purified by silica gel column (PE/EtOAc = 49/1) to give the product methyl 2-amino-3-bromo-5-methylbenzoate (6.84 g, 92.2% yield) as a colorless oil. **$^1$H NMR** (400 MHz, $CD_3OD$) $\delta$ 7.59 (d, *J* = 1.2 Hz, 1H), 7.52 (d, *J* = 2.0 Hz, 1H), 6.49 (s, 2H), 3.81 (s, 3H), 2.17 (s, 3H). **LC-MS:** m/z 243.8 $(M+H)^+$.

Step B methyl 2-amino-3-((3-((2-ethylhexyl)oxy)-3-oxopropyl)thio)-5-methylbenzoate

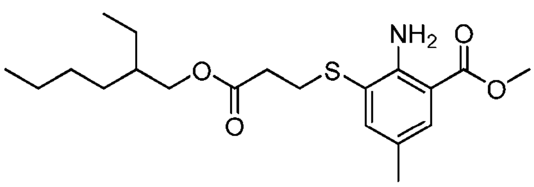

To a solution of methyl 2-amino-3-bromo-5-methylbenzoate (6.84 g, 28.02 mmol) in toluene (50 mL) was added 2-ethylhexyl 3-sulfanylpropanoate (6.73 g, 30.83 mmol), DIEA (14.6 mL, 84.07 mmol), Xantphos (3.24 g, 5.60 mmol) and $Pd_2(dba)_3$ (2.57 g, 2.80 mmol). The mixture was stirred under $N_2$ at 100 °C for 16 hrs. After cooling, the reaction mixture was filtrated and the filtrate was concentrated under reduced pressure to give a residue. The residue was purified by silica gel column (PE/EtOAc = 24/1) to give the product methyl 2-amino-3-((3-((2-ethylhexyl)oxy)-3-oxopropyl)thio)-5-methylbenzoate (6.88 g, 61.7% yield) as a yellow oil. **LC-MS:** m/z 382.4 $(M+H)^+$.

Step C diethyl 3,3'-disulfanediylbis(2-amino-5-methylbenzoate)

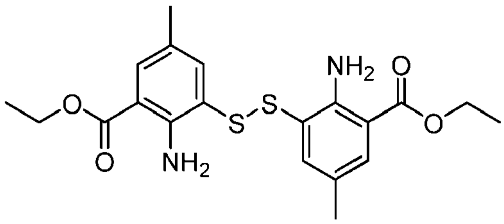

To a solution of methyl 2-amino-3-((3-((2-ethylhexyl)oxy)-3-oxopropyl)thio)-5-methylbenzoate (1 g, 2.62 mmol) in EtOH (10 mL) was added EtONa (445.90 mg, 6.55 mmol) at 0 °C. After addition, the reaction mixture was stirred at 25 °C for 12 hrs. The mixture was diluted with $H_2O$ (40 mL), and the *p*H was adjusted to 7 with 1 N HCl. The mixture was extracted with EtOAc (30 mL*3). The combined organics were dried over anhydrous $Na_2SO_4$ and concentrated under reduced pressure to give a residue. The residue was purified by silica gel column (PE/EtOAc = 28/1) to give the product diethyl 3,3'-disulfanediylbis(2-amino-5-methylbenzoate) (264 mg, 23.5% yield) as a yellow solid. **LC-MS:** m/z 421.4 $(M+H)^+$.

Step D ethyl 2-((2S,3R)-3-((tert-butyldimethylsilyl)oxy)-3-(3,5-dimethoxy-4-methylphenyl)-2-isobutoxypropyl)-6-methylbenzo[d]thiazole-4-carboxylate

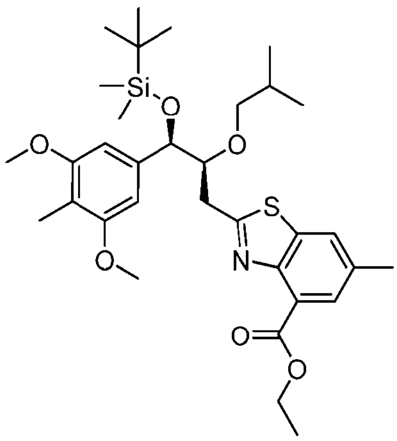

ethyl 2-((2S,3R)-3-((tert-butyldimethylsilyl)oxy)-3-(3,5-dimethoxy-4-methylphenyl)-2-isobutoxypropyl)-6-methylbenzo [d]thiazole-4-carboxylate was synthesized according to the procedures described for the preparation of **Example 24 (compound 157)** by using diethyl 3,3'-disulfanediylbis(2-amino-5-methylbenzoate) and (3S,4R)-4-((tert-butyldimethylsilyl)oxy)-4-(3,5-dimethoxy-4-methylphenyl)-3-isobutoxybutanoic acid in step E.

Step E 2-((2S,3R)-3-((tert-butyldimethylsilyl)oxy)-3-(3,5-dimethoxy-4-methylphenyl)-2-isobutoxypropyl)-6-methylbenzo[d]thiazole-4-carboxylic acid

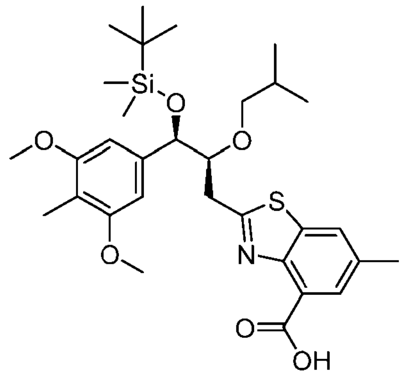

2-((2S,3R)-3-((tert-butyldimethylsilyl)oxy)-3-(3,5-dimethoxy-4-methylphenyl)-2-isobutoxypropyl)-6-methylbenzo[d]thiazole-4-carboxylic acid was synthesized according to the procedures described for the preparation of **Example 24 (compound 157)** by using ethyl 2-((2S,3R)-3-((tert-butyldimethylsilyl)oxy)-3-(3,5-dimethoxy-4-methylphenyl)-2-isobutoxypropyl)-6-methylbenzo[d]thiazole-4-carboxylate in step F.

Step F 2-((2S,3R)-3-(3,5-dimethoxy-4-methylphenyl)-3-hydroxy-2-isobutoxypropyl)-6-methylbenzo[d]thiazole-4-carboxylic acid (Compound 234)

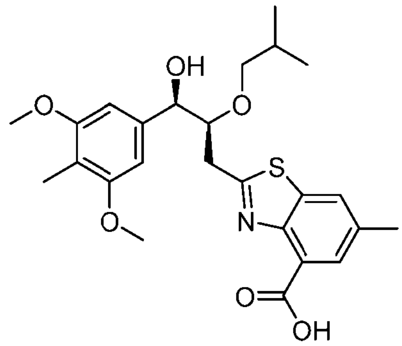

2-((2S,3R)-3-(3,5-dimethoxy-4-methylphenyl)-3-hydroxy-2-isobutoxypropyl)-6-methylbenzo[d]thiazole-4-carboxylic acid was synthesized according to the procedures described for the preparation of **Example 24** by using 2-((2S,3R)-3-((tertbutyldimethylsilyl)oxy)-3-(3,5-dimethoxy-4-methylphenyl)-2-isobutoxypropyl)-6-methylbenzo[d]thiazole-4-carboxylic acid in step G. **$^1$H NMR** (400 MHz, $CD_3OD$) $\delta$ 7.92 (d, *J* = 4.0 Hz, 2H), 6.57 (s, 2H), 4.67 (d, *J* = 4.0 Hz, 1H), 3.90 - 3.79 (m, 1H), 3.71 (s, 6H), 3.44 - 3.28 (m, 2H), 3.06 (d, *J* = 8.0 Hz, 2H), 2.44 (s, 3H), 1.87 (s, 3H), 1.62 - 1.54 (m, 1H), 0.63 (t, *J* = 7.2 Hz, 6H). **LC-MS:** m/z 474.2 $(M+H)^+$.

**Example 30**

(S)-2-(2-((2S,3R)-2-(cyclopentyloxy)-3-(3,5-dimethoxy-4-methylphenyl)-3-hydroxypropyl)-5-methylthiazol-4-yl)propanoic acid **(compound 239)** and (R)-2-(2-((2S,3R)-2-(cyclopentyloxy)-3-(3,5-dimethoxy-4-methylphenyl)-3-hydroxypropyl)-5-methylthiazol-4-yl)propanoic acid (compound 240)

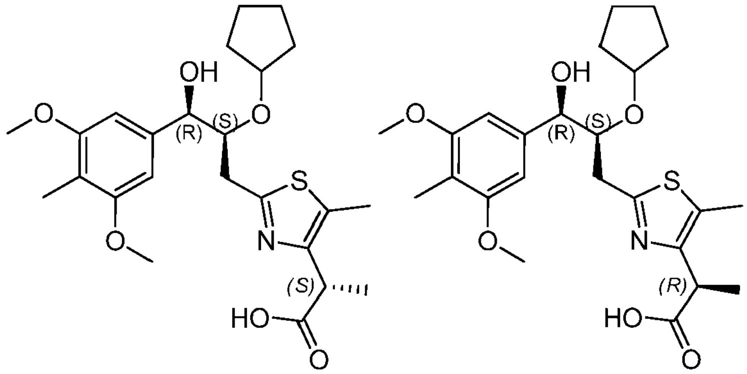

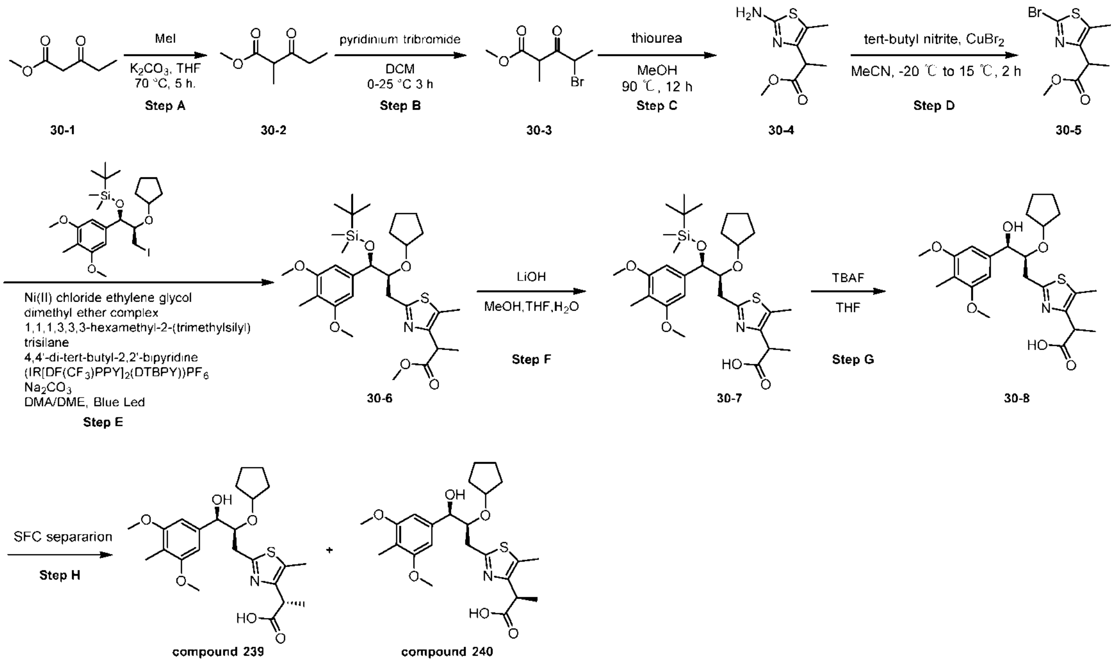


Step A methyl 2-methyl-3-oxopentanoate

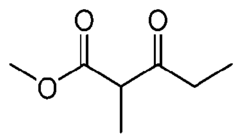

To a solution of methyl 3-oxopentanoate (3.5 g, 26.89 mmol) in THF (10 mL) was added $K_2CO_3$ (7.43 g, 53.79 mmol) and MeI (2.1 mL, 33.62 mmol) at 20 °C under $N_2$ atmosphere. The mixture was stirred at 70 °C for 5 hrs. The reaction was concentrated under reduced pressure to remove THF. The residue was diluted with $H_2O$ (10 mL) and extracted with EtOAc (20 mL*2). The combined organic layers were washed with brine (10 mL*2), dried over $Na_2SO_4$, filtered and concentrated to give the crude product methyl 2-methyl-3-oxopentanoate (3.7 g, 95.4% yield) as yellow oil, which was used directly for the next step without further purification. **$^1$H NMR** (400MHz, DMSO-$d_6$) $\delta$ 3.78 - 3.70 (m, 1H), 3.63 (s, 3H), 2.57 (q, $J$ = 7.2 Hz, 2H), 1.18 (d, $J$ = 7.1 Hz, 3H), 0.92 (t, $J$ = 7.2 Hz, 3H).

Step B methyl 4-bromo-2-methyl-3-oxopentanoate

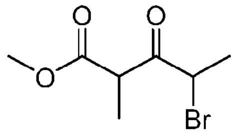

To a solution of methyl 2-methyl-3-oxopentanoate (4.79 g, 33.25 mmol) in DCM (50 mL) was added pyridinium tribromide (11.70 g, 36.58 mmol) at 0 °C and the mixture was stirred at 25 °C for 3 h. The reaction mixture was quenched with $H_2O$ (30 mL) and the mixture was extracted with DCM (20 mL*3). The combined organics were concentrated under reduced pressure to give the crude product methyl 4-bromo-5-methyl-3-oxohexanoate (8.14 g, 86.6% yield) as light yellow oil. **$^1$H NMR** (400MHz, DMSO-*d*$_6$) $\delta$ 5.04 - 4.91 (m, 1H), 4.17 - 3.93 (m, 1H), 3.61 (s, 3H), 1.62 - 1.58 (m, 3H), 1.24 - 1.20 (m, 3H).

Step C methyl 2-(2-amino-5-methylthiazol-4-yl)propanoate

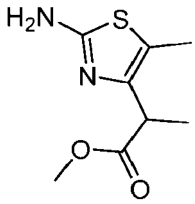

methyl 2-(2-amino-5-methylthiazol-4-yl)propanoate was synthesized according to the procedures described for the preparation of **Example 28** by using methyl 4-bromo-2-methyl-3-oxopentanoate in step B. **$^1$H** NMR (400MHz, DMSO-*d*$_6$) $\delta$ 6.69 (s, 2H), 3.73 - 3.67 (m, 1H), 3.54 (s, 3H), 2.13 (s, 3H), 1.24 (d, *J* = 7.0 Hz, 3H).

Step D methyl 2-(2-bromo-5-methylthiazol-4-yl)propanoate

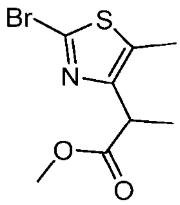

methyl 2-(2-bromo-5-methylthiazol-4-yl)propanoate was synthesized according to the procedures described for the preparation of **Example 28** by using methyl 2-(2-amino-5-methylthiazol-4-yl)propanoate in step C. **$^1$H NMR** (400MHz, DMSO-*d*$_6$) $\delta$ 4.02 (q, *J* = 6.8 Hz, 1H), 3.58 (s, 3H), 2.36 (s, 3H), 1.34 (d, *J* = 7.2 Hz, 3H). **LC-MS:** m/z 266.1 $(M+H)^+$.

Step E methyl 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)propanoate

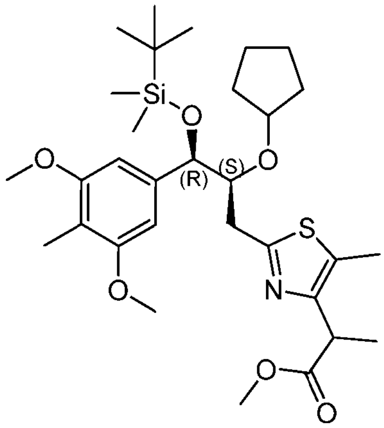

methyl 2-(2-((2S,3R)-3-((tert-butyldimethylsilyl)oxy)-2-(cyclopentyloxy)-3-(3,5-dimethoxy-4-methylphenyl)propyl)-5-ethylthiazol-4-yl)acetate was synthesized according to the procedures described for the preparation of **Example 28** by using methyl 2-(2-bromo-5-methylthiazol-4-yl)propanoate in step D. **LC-MS:** m/z 592.3 $(M+H)^+$.

Step F 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)propanoic acid

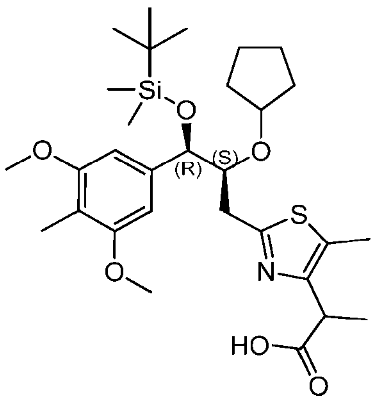

2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)propanoic acid was synthesized according to the procedures described for the preparation of **Example 1** by using methyl 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)propanoate in step M. **LC-MS:** m/z 578.3 $(M+H)^+$.

Step G 2-(2-((2S,3R)-2-(cyclopentyloxy)-3-(3,5-dimethoxy-4-methylphenyl)-3-hydroxypropyl)-5-methylthiazol-4-yl)propanoic acid

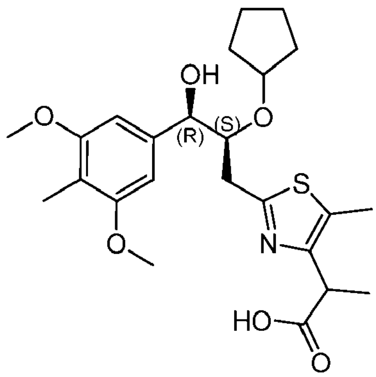

2-(2-((2S,3R)-2-(cyclopentyloxy)-3-(3,5-dimethoxy-4-methylphenyl)-3-hydroxypropyl)-5-methylthiazol-4-yl)propanoic acid was synthesized according to the procedures described for the preparation of **Example 1** by using 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)propanoic acid in step N. **LC-MS:** m/z 464.1 $(M+H)^+$.

Step H (S)-2-(2-((2S,3R)-2-(cyclopentyloxy)-3-(3,5-dimethoxy-4-methylphenyl)-3-hydroxypropyl)-5-methylthiazol-4-yl) propanoic acid **(compound 239)** and (R)-2-(2-((2S,3R)-2-(cyclopentyloxy)-3-(3,5-dimethoxy-4-methylphenyl)-3-hydroxypropyl)-5-methylthiazol-4-yl)propanoic acid **(compound 240)**

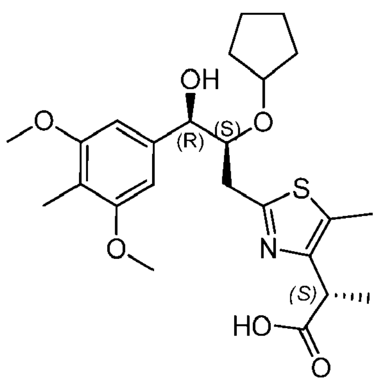

2-(2-((2S,3R)-2-(cyclopentyloxy)-3-(3,5-dimethoxy-4-methylphenyl)-3-hydroxypropyl)-5-methylthiazol-4-yl)propanoic acid was further separated by SFC (column: DAICEL CHIRALPAK IG (250 mm*30 mm, 10 um); mobile phase: [Neu-MeOH]; B%: 25%-25%) to give two isomers. The chiral center was assigned randomly.

Isomer **1** (S)-2-(2-((2S,3R)-2-(cyclopentyloxy)-3-(3,5-dimethoxy-4-methylphenyl)-3-hydroxypropyl)-5-methylthiazol-4-yl)propanoic acid **(compound 239).** $^1$**H NMR** (400 MHz, $CD_3OD$) $\delta$ 6.67 (s, 2H), 4.65 (d, $J$ = 5.6 Hz, 1H), 3.94 - 3.89 (m, 1H), 3.84 (s, 6H), 3.82 - 3.77 (m, 2H), 3.21 - 3.13 (m, 1H), 3.11 - 3.02 (m, 1H), 2.40 (s, 3H), 2.04 (s, 3H), 1.46 (d, $J$ = 8.0 Hz, 3H), 1.42 - 1.30 (m, 8H). **LC-MS:** m/z 464.1 $(M+H)^+$.
SFC analysis condition: Column: ChiralPak IG-3 100×4.6mm I.D., 3 um; Mobile phase: A: $CO_2$ B: Methanol (0.05% DEA); Gradient: from 5% to 40% of B in 4.5min, then 5% of B for 1.5 min, Flow rate: 2.5mL/min; Retention time: 3.202 min.

Isomer 2 (R)-2-(2-((2S,3R)-2-(cyclopentyloxy)-3-(3,5-dimethoxy-4-methylphenyl)-3-hydroxypropyl)-5-methylthiazol-4-yl)propanoic acid **(compound 240)**

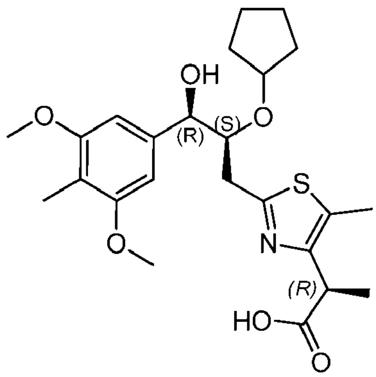

**$^1$H NMR** (400 MHz, $CD_3OD$) $\delta$ 6.67 (s, 2H), 4.66 (d, $J$ = 5.6 Hz, 1H), 3.94 - 3.89 (m, 1H), 3.84 (s, 6H), 3.83 - 3.79 (m, 2H), 3.19 - 3.04 (m, 2H), 2.40 (s, 3H), 2.04 (s, 3H), 1.46 (d, $J$ = 8.0 Hz, 3H), 1.42 - 1.31 (m, 8H). **LC-MS:** m/z 464.1 $(M+H)^+$. SFC analysis condition: Column: ChiralPak IG-3 100×4.6mm I.D., 3 um; Mobile phase: A: $CO_2$ B: Methanol (0.05% DEA); Gradient: from 5% to 40% of B in 4.5min, then 5% of B for 1.5 min, Flow rate: 2.5mL/min; Retention time: 3.371 min.

## Example 31

2-((2S,3R)-3-(4-cyano-3,5-diethoxyphenyl)-2-(cyclopentyloxy)-3-hydroxypropyl)benzo[d]thiazole-4-carboxylic acid **(Compound 257)**

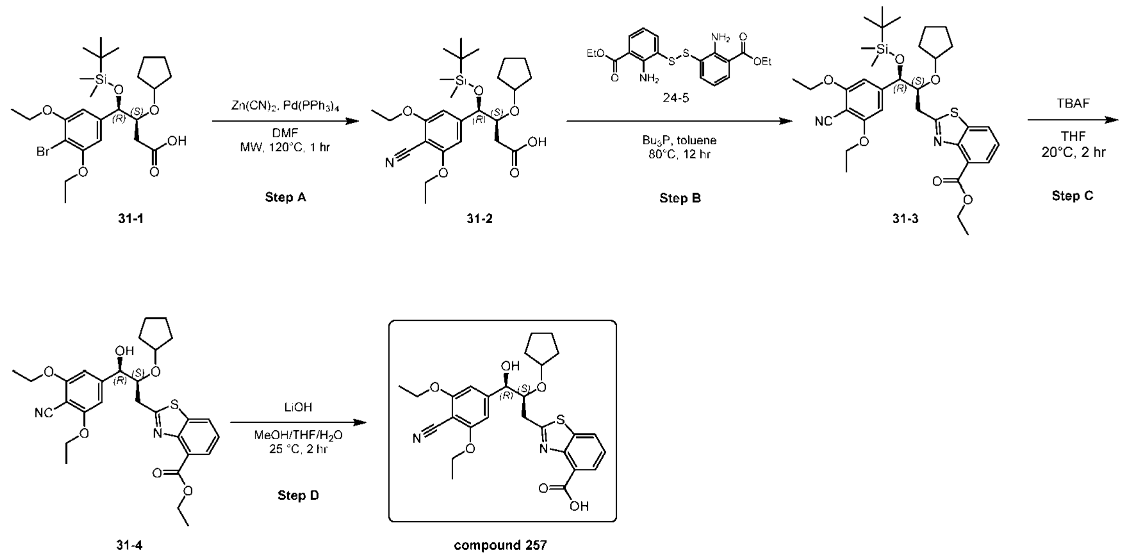


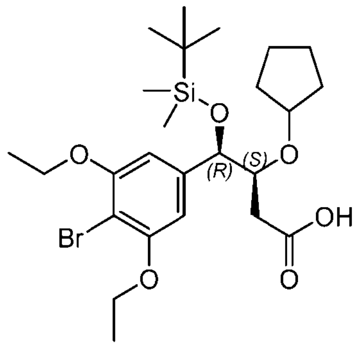

(3S,4R)-4-(4-bromo-3,5-diethoxyphenyl)-4-((tert-butyldimethylsilyl)oxy)-3-(cyclopentyloxy)butanoic acid was synthesized according to the procedures described for the preparation of **Example 1** (step F to step K in Scheme 1) by using 2-(2-((2S,3R)-3-((tert-butyldimethylsilyl)oxy)-2-(cyclopentyloxy)-3-(3,5-dimethoxy-4-methylphenyl)propyl)-5-methylthiazol-4-yl)propanoic acid in step F, **Example 14** (step A in Scheme 14) by using (2S,3R)-3-(4-bromo-3,5-diethoxyphenyl)-3-((tertbutyldimethylsilyl)oxy)-2-(cyclopentyloxy)propyl methanesulfonate in step A and **Example 24** (step A in Scheme 24) by using (3S,4R)-4-(4-bromo-3,5-diethoxyphenyl)-4-((tert-butyldimethylsilyl)oxy)-3-(cyclopentyloxy)butanal in step A.

Step A (3S,4R)-4-((tert-butyldimethylsilyl)oxy)-4-(4-cyano-3,5-diethoxyphenyl)-3-(cyclopentyloxy)butanoic acid

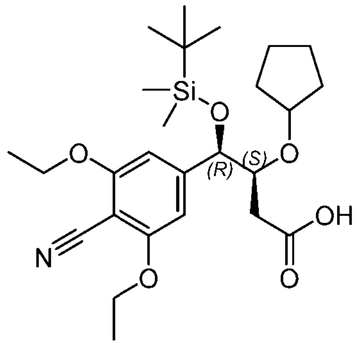

To a solution of (3S,4R)-4-(4-bromo-3,5-diethoxy-phenyl)-4-[tert-butyl (dimethyl) silyl]oxy-3-(cyclopentoxy) butanoic acid (200 mg, 366.58 umol) in DMF (2 mL) was added $Zn(CN)_2$ (129.14 mg, 1.10 mmol) and $Pd(PPh_3)_4$ (42.36 mg, 36.66 umol). The mixture was stirred at 120 °C for 1 hr under microwave. The reaction mixture was diluted with $H_2O$ (10 mL) and extracted with EtOAc (10 mL*3). The combined organic layers were washed with brine (10 mL*3), dried over anhydrous $Na_2SO_4$, filtered and concentrated. The residue was purified by silica gel column (PE/EtOAc = 3/1) to afford (3S,4R) -4-((tert-butyldimethylsilyl)oxy)-4-(4-cyano-3,5-diethoxyphenyl)-3-(cyclopentyloxy) butanoic acid (70 mg, 38.8% yield) as yellow oil. **LC-MS:** m/z 492.2 $(M+H)^+$.

Step B ethyl 2-((2S,3R)-3-((tert-butyldimethylsilyl)oxy)-3-(4-cyano-3,5-diethoxyphenyl)-2-(cyclopentyloxy)propyl)benzo[d]thiazole-4-carboxylate

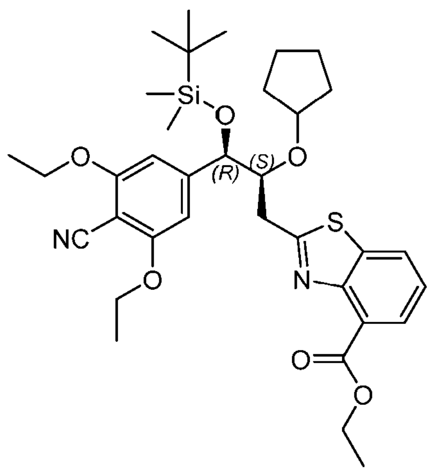

To a solution of ethyl 2-amino-3-[(2-amino-3-ethoxycarbonyl-phenyl) disulfanyl]benzoate (55.88 mg, 142.37 umol) in toluene (1 mL) was added tributylphosphane (86.41 mg, 427.11 umol). Then (3S,4R) -4-[tert-butyl (dimethyl) silyl]oxy-4-(4-cyano-3,5-diethoxy-phenyl) -3- (cyclopentoxy) butanoic acid (70.00 mg, 142.37 umol) was added. The solution was stirred at 80 °C for 12 hrs. The reaction mixture was diluted with $H_2O$ (30 mL) and extracted with EtOAc (30 mL*3). The combined organic layers were washed with brine (30 mL), dried over anhydrous $Na_2SO_4$, filtered and concentrated to

afford ethyl 2-[(2S,3R) -3-[tert-butyl (dimethyl) silyl]oxy-3-(4-cyano-3,5-diethoxy-phenyl)-2-(cyclopentoxy)propyl]-1,3-benzothiazole-4-carboxylate (100 mg, crude) as yellow oil, which used in the next step without further purification. **LC-MS:** m/z 653.2 (M+H)+.

Step C ethyl 2-((2S,3R)-3-(4-cyano-3,5-diethoxyphenyl)-2-(cyclopentyloxy)-3-hydroxypropyl)benzo[d]thiazole-4-carboxylate

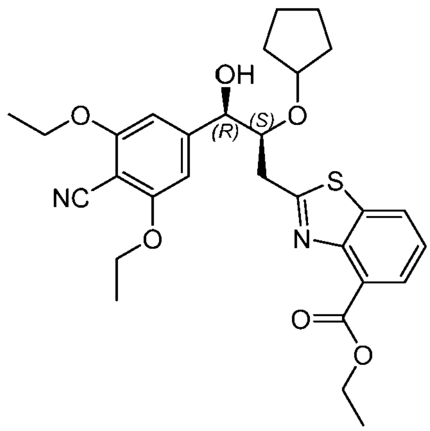

To a solution of ethyl 2-[(2S,3R)-3-[tert-butyl (dimethyl) silyl]oxy-3-(4-cyano-3,5-diethoxy-phenyl)-2-(cyclopentoxy) propyl]-1,3-benzothiazole-4-carboxylate (100 mg, 153.16 umol) in THF (1 mL) was added TBAF (1 M THF solution, 1.53 mL) . The mixture was stirred at 20 °C for 2 hrs. The reaction mixture was diluted with $H_2O$ (10 mL) and extracted with EtOAc (10 mL*3). The combined organic layers were washed with brine (20 mL), dried over anhydrous $Na_2SO_4$, filtered and concentrated to afford ethyl 2-[ (2S,3R) -3- (4-cyano-3,5-diethoxy-phenyl)-2-(cyclopentoxy) -3-hydroxypropyl]-1,3-benzothiazole-4-carboxylate (80 mg, 97.0% yield) as a yellow oil, which was used for next step without further purification. **LC-MS:** m/z 539.0 (M+H)+.

Step D 2-((2S,3R)-3-(4-cyano-3,5-diethoxyphenyl)-2-(cyclopentyloxy)-3-hydroxypropyl) benzo [d]thiazole-4-carboxylic acid **(Compound 257)**

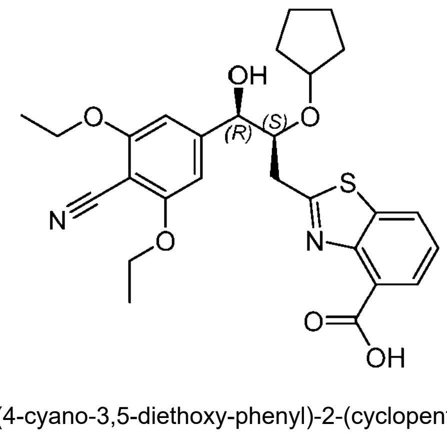

To a solution of ethyl 2-[(2S,3R)-3-(4-cyano-3,5-diethoxy-phenyl)-2-(cyclopentoxy)-3-hydroxy-propyl]-1,3-benzothiazole-4-carboxylate (80 mg, 148.52 umol) in THF (0.8 mL), MeOH (0.2 mL) and $H_2O$ (0.2 mL) was added LiOH (17.78 mg, 742.59 umol). The mixture was stirred at 20 °C for 0.5 hr. The reaction mixture was diluted with $H_2O$ (10 mL) and extracted with EtOAc (10 mL*3). The combined organic layers were washed with brine (10 mL), dried over anhydrous $Na_2SO_4$, filtered and concentrated. The residue was purified by prep-HPLC (Column: Kromasil 100-5-C18; Eluent: 50% to 90% water (0.1% HCOOH)-ACN) to give the product 2-[(2S,3R) -3-(4-cyano-3,5-diethoxy-phenyl) -2- (cyclopentoxy) -3-hydroxy-propyl]-1,3-benzothiazole-4-carboxylic acid (15 mg, 19.78% yield) as yellow solid. **$^1$H NMR** (400MHz, $CD_3OD$) $\delta$ 8.28 - 8.16 (m, 2H), 7.59 (t, *J* = 7.8 Hz, 1H), 6.76 (s, 2H), 4.84 - 4.82 (m, 1 H), 4.69 (d, *J* = 8.0 Hz, 1H), 4.18 (q, *J* = 8.0 Hz, 4H),

4.05 - 3.97 (m, 2H), 3.60 - 3.48 (m, 2H), 1.54 - 1.46 (m, 2H), 1.44 - 1.40 (m, 12H). **LC-MS:** m/z 511.1 $(M+H)^+$.

**Example 32**

2-((2S,3R)-2-(cyclopentyloxy)-3-(3,5-dimethoxy-4-methylphenyl)-3-hydroxypropyl)benzo[b]thiophene-4-carboxylic acid **(compound 265)**

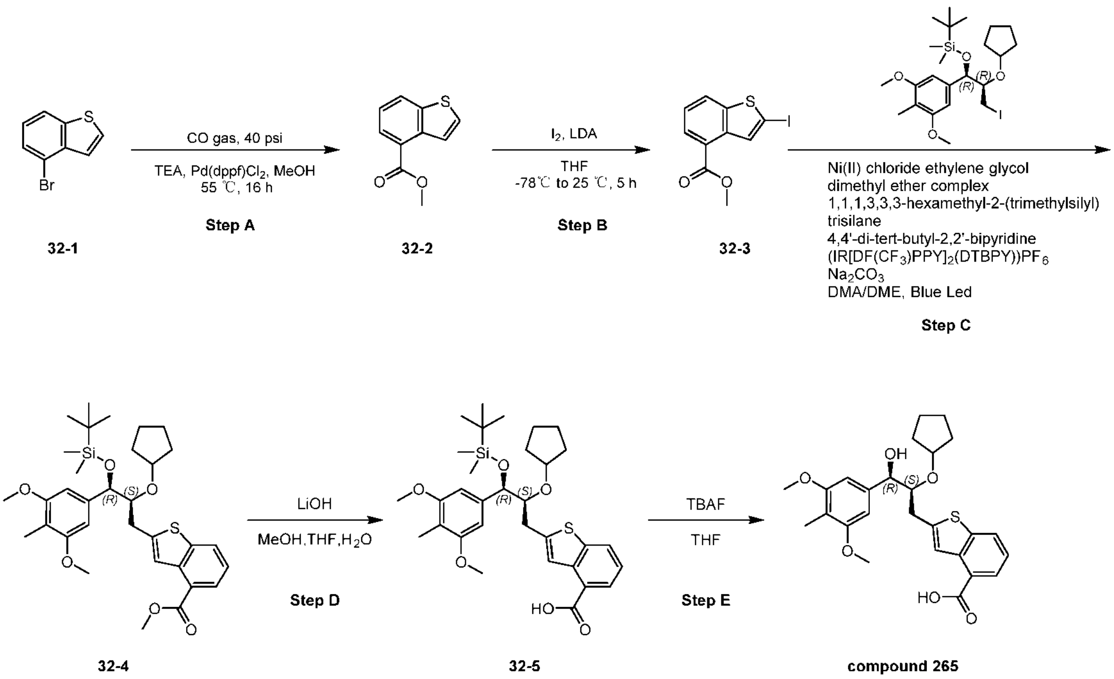


Step A methyl benzo[b]thiophene-4-carboxylate

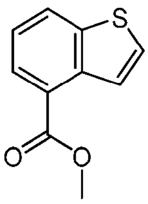

To a solution of 4-bromobenzothiophene (5 g, 23.46 mmol) in MeOH (50 mL) was added TEA (9.8 mL, 70.39 mmol) and Pd(dppf)$Cl_2$ (1.72 g, 2.35 mmol). The mixture was stirred under 40 psi CO gas at 55 °C for 16 hrs. The reaction was filtrated and the filtrate was concentrated under reduced pressure to give a residue. The residue was purified by silica gel column (PE/EtOAc = 24/1) to give the product methyl benzo[b]thiophene-4-carboxylate (3.32 g, 73.6% yield) as light yellow oil. **$^1$H NMR** (400 MHz, $CD_3OD$) $\delta$ 8.20-8.00 (m, 3H), 7.73 (d, *J* = 3.6 Hz, 1H), 7.38 (t, *J* = 8.0 Hz, 1H), 3.95 (s, 3H).

Step B methyl 2-iodobenzo[b]thiophene-4-carboxylate

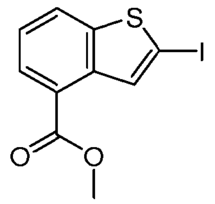

To a solution of diisopropylamine (0.26 mL, 1.79 mmol) in THF (4 mL) was added *n*-BuLi (2.5 M in hexane) (0.66 mL, 1.65 mmol) at 0 °C and the mixture was stirred at 0 °C for 10 min. A solution of methyl benzo[b]thiophene-4-carboxylate (260 mg, 1.35 mmol) in THF (2 mL) was added at -78 °C under $N_2$. After 30 min, a solution of $I_2$ (412 mg, 1.62 mmol) in THF (1 mL) was added. The mixture was stirred at 25 °C for 5 hrs. The reaction was quenched with $H_2O$ (40 mL), extracted with EtOAc (30 mL*3). The combined organics were dried over anhydrous $Na_2SO_4$, filtered and concentrated under reduced pressure to give a residue. The residue was purified by silica gel column (PE/EtOAc = 32/1) to give the product methyl 2-iodobenzo [b]thiophene-4-carboxylate (240 mg, 27.1% yield) as light yellow oil. **LC-MS:** m/z 318.5 $(M+H)^+$ .

Step C methyl 2-((2S,3R)-3-((tert-butyldimethylsilyl)oxy)-2-(cyclopentyloxy)-3-(3,5-dimethoxy-4-methylphenyl)propyl) benzo[b]thiophene-4-carboxylate

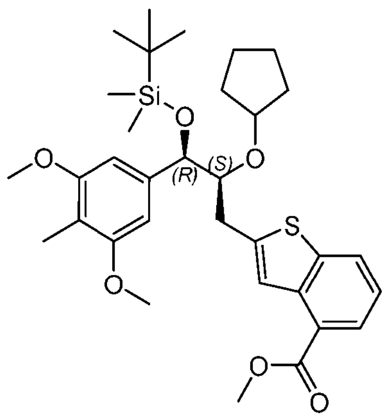

methyl 2-((2S,3R)-3-((tert-butyldimethylsilyl)oxy)-2-(cyclopentyloxy)-3-(3,5-dimethoxy-4-methylphenyl)propyl)benzo[b] thiophene-4-carboxylate was synthesized according to the procedures described for the preparation of **Example 11 (Compound 106a)** (step A to step B in Scheme 11) by using methyl [(2S,3R)-3-[tert-butyl(dimethyl)silyl]oxy-2-(cyclopentoxy)-3- (3,5-dimethoxy-4-methyl-phenyl)propyl] methanesulfonate in step A and methyl 2-iodobenzo[b]thiophene-4-carboxylate in step B.

Step D 2-((2S,3R)-3-((tert-butyldimethylsilyl)oxy)-2-(cyclopentyloxy)-3-(3,5-dimethoxy-4-methylphenyl)propyl)benzo[b] thiophene-4-carboxylic acid

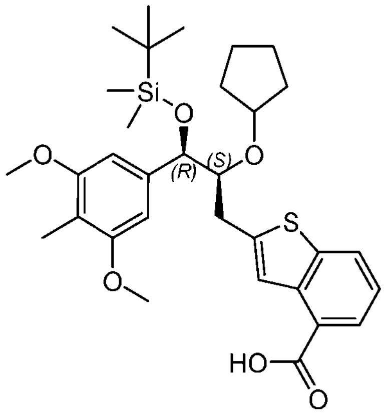

2-((2S,3R)-3-((tert-butyldimethylsilyl)oxy)-2-(cyclopentyloxy)-3-(3,5-dimethoxy-4-methylphenyl)propyl)benzo[b]thiophene-4-carboxylic acid was synthesized according to the procedure described in step M for the preparation of **Example 1 (Compound 113a). LC-MS:** m/z 607.3 $(M+Na)^+$.

Step E 2-((2S,3R)-2-(cyclopentyloxy)-3-(3,5-dimethoxy-4-methylphenyl)-3-hydroxypropyl)benzo[b]thiophene-4-carboxylic acid

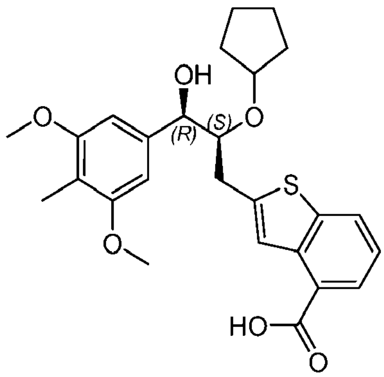

2-((2S,3R)-2-(cyclopentyloxy)-3-(3,5-dimethoxy-4-methylphenyl)-3-hydroxypropyl)benzo[b]thiophene-4-carboxylic acid was synthesized according to the procedure described in step N for the preparation of **Example 1 (Compound 113a).** **$^1$H NMR** (400 MHz, $CD_3OD$) $\delta$ 8.09 - 7.95 (m, 3H), 7.35 (t, $J$ = 8.0 Hz, 1H), 6.67 (s, 2H), 4.58 (d, $J$ = 4.0 Hz, 1H), 3.89 - 3.78 (m, 7H), 3.77 - 3.72 (m, 1H), 3.29 - 3.16 (m, 2H), 2.04 (s, 3H), 1.52 - 1.27 (m, 8H). **LC-MS:** m/z 493.1 $(M+Na)^+$.

**Example A: Assays**

*In Vitro LPA1 functional antagonist Assay*

CHO-K1 cells overexpressing human LPA1 were seeded in a total volume of 20 μL into black-walled, clear-bottom, Poly-D-lysine coated 384-well microplates and incubated at 37°C for the appropriate time prior to testing. Assays were performed in 1 x Dye Loading Buffer consisting of 1x Dye, 1x Additive A and 2.5 mM Probenecid in HBSS / 20 mM Hepes. Probenicid was prepared fresh. Cells were loaded with dye prior to testing. Media was aspirated from cells and replaced with 20 μL Dye Loading Buffer. Cells were incubated for 30-60 minutes at 37°C. After dye loading, cells were removed from the incubator and 10 μL 3X test compound was added. Cells were incubated for 30 minutes at room temperature in the dark to equilibrate plate temperature followed by Oleoyl LPA challenge at the 0.018 μM. Compound antagonist activity was measured on a FLIPR Tetra (MDS). Calcium mobilization was monitored for 2 minutes and 10 μL Oleoyl LPA in HBSS / 20 mM Hepes was added to the cells 5 seconds into the assay. Compound activity was analyzed using CBIS data analysis suite (ChemInnovation, CA). Percentage inhibition is calculated using the following formula:

% Inhibition =100% x (1 - (mean RFU of test sample - mean RFU of vehicle control) / (mean RFU of LPA control - mean RFU of vehicle control)).

**Table B1** shows the biological activity of compounds in in vitro LPA1 functional antagonist assay

| Compound | $IC_{50}$ (nM) |
|---|---|
| **101a** | 213 |
| **102a** | 14.5 |
| **103a** | 4.74 |
| **104a** | 5.24 |
| **105a** | 3.35 |
| **105b** | 49 |
| **106a** | 411 |
| **107** | > 10 E+03 |
| **108a** | 3.05 |
| **109a** | > 10 E+03 |
| **110a** | 1650 |
| **111a** | > 10 E+03 |
| **112a** | 56.1 |
| **113a** | 3.69 |
| **114a** | 125 |
| **115a** | 30.8 |
| **116a** | 1470 |
| **117a** | 61.9 |
| **118a** | 26.6 |
| **119a** | > 10 E+03 |
| **120a** | > 10 E+03 |
| **121a** | 11.2 |
| **122a** | 1450 |
| **123a** | / |
| **124a** | 5700 |
| **125a** | 80.7 |
| **126a** | 64.8 |
| **127a** | > 10 E+03 |
| **128a** | 207 |
| **129a** | 3.38 |
| **130a** | 1190 |
| **131a** | > 10 E+03 |
| **132** | 1540 |
| **133** | > 10 E+03 |
| **134** | > 10 E+03 |
| **135** | > 10 E+03 |

(continued)

| Compound | $IC_{50}$ (nM) |
|---|---|
| **136** | > 10 E+03 |
| **137** | > 10 E+03 |
| **138** | > 10 E+03 |
| **139** | 1350 |
| **140** | 11.8 |
| **141** | 193 |
| **142** | > 10 E+03 |
| **143** | 7.66 |
| **144** | 2.04 |
| **145** | 72.6 |

*In vitro LPA1 Calcium flux antagonist assay - Bioduro protocol*

CHO-K1 cells overexpressing human LPA1 and G15a were seeded at a total volume of 20 uL (15000 cells/well) into Matrigel pre-coated 384-well plates (corning -3764) and incubated at 37°C. After overnight incubation, the cells were serum starved for 4h. Assays were performed in dye loading buffer containing 1x Fluo-8 AM (AAT Bioquest, 21080) and 2.5 mM probenecid (Thermo Fisher, 36400) in HBSS / 20 mM Hepes. After cell starvation, the medium was replaced with 20 uL of dye loading buffer and incubated at 37°C for 30 min. Then 5 uL of 5X compound titrated in dye loading buffer was added to the cells and incubated for 30 min followed by LPA challenge at EC80. Calcium mobilization was measured on a FLIPR Tetra (MDS). For LPA EC80 determination, starved cells were incubated with 20 uL of dye loading buffer for 1h, then 5 uL of 5X LPA titrated in dye loading buffer was added to the cells. Calcium signals induced by LPA was monitors on a FLIPR.

Percentage inhibition is calculated using the following formula:

% Inhibition =100% x (1 - (mean RFU of test sample - mean RFU of DMSO) / (mean RFU of LPA control - mean RFU of DMSO)).

**Table B2** shows the biological activity of compounds in in vitro LPA1 Calcium flux antagonist assay - Bioduro protocol

| Compound | $IC_{50}$ (nM) in Assay B2 |
|---|---|
| **146** | 36.8 |
| **147** | 126.0 |
| **148** | 525.0 |
| **149** | 299.0 |
| **150** | 146.0 |
| **151** | > 10.0E+03 |
| **152** | 8.53 |
| **153** | 5.42 |
| **154** | 8.64 |
| **155** | 308.0 |
| **156** | 301.0 |
| **157** | 6.3 |
| **158** | 55.8 |
| **159** | 49.6 |
| **160** | 138.0 |

(continued)

| Compound | $IC_{50}$ (nM) in Assay B2 |
|---|---|
| **161** | 6.28 |
| **162** | 3.93 |
| **163** | 4.23 |
| **164** | 11.9 |
| **165** | 6.28 |
| **166** | 4.94 |
| **167** | > 10.0E+03 |
| **168** | > 10.0E+03 |
| **169** | 150.0 |
| **170** | > 10.0E+03 |
| **171** | > 10.0E+03 |
| **172** | 4.01 |
| **173** | 6.09 |
| **174** | 103.0 |
| **175** | > 10.0E+03 |
| **176** | 9.95 |
| **177** | 154.0 |
| **178** | 73.8 |
| **179** | 20.3 |
| **180** | 153.0 |
| **181** | > 10.0E+03 |
| **182** | 79.2 |
| **183** | > 10.0E+03 |
| **184** | 24.0 |
| **185** | 15.0 |
| **186** | 7640.0 |
| **187** | 132.0 |
| **188** | 447.0 |
| **189** | 454.0 |
| **190** | 1530.0 |
| **191** | 662.0 |
| **192** | 1120.0 |
| **193** | 757.0 |
| **194** | 26.9 |
| **195** | 32.3 |
| **196** | 16.3 |
| **197** | 23.8 |
| **198** | 6.87 |
| **199** | 667.0 |

(continued)

| Compound | $IC_{50}$ (nM) in Assay B2 |
|---|---|
| **200** | 1130.0 |
| **201** | 938.0 |
| **202** | 4260.0 |
| **203** | 530.0 |
| **204** | 122.0 |
| **205** | 507.0 |
| **206** | 2120.0 |
| **207** | 723.0 |
| **208** | > 10.0E+03 |
| **209** | 812.0 |
| **210** | 7810.0 |
| **211** | 459.0 |
| **212** | 10.8 |
| **213** | > 10.0E+03 |
| **214** | > 10.0E+03 |
| **215** | 140.0 |
| **216** | 9.61 |
| **217** | 1300.0 |
| **218** | 13.0 |
| **219** | 20.4 |
| **220** | 106.0 |
| **221** | 91.8 |
| **222** | 43.4 |
| **223** | 99.4 |
| **224** | 327.0 |
| **225** | 29.3 |
| **226** | 28.9 |
| **227** | 149.0 |
| **228** | 62.2 |
| **229** | 7.26 |
| **230** | 872.0 |
| **231** | 11.1 |
| **232** | 27.2 |
| **233** | 17.9 |
| **234** | 3.71 |
| **235** | 40.8 |
| **236** | 9.18 |
| **237** | 10.3 |
| **238** | 53.3 |

(continued)

| Compound | $IC_{50}$ (nM) in Assay B2 |
|---|---|
| **239** | 37.4 |
| **240** | 885.0 |
| **241** | 26.7 |
| **242** | 7.5 |
| **243** | 37.1 |
| **244** | 3.34 |
| **245** | 2.51 |
| **246** | 1.65 |
| **247** | 1.93 |
| **248** | 10.0E+03 |
| **249** | 60.2 |
| **250** | 17.4 |
| **251** | 37.3 |
| **252** | 24.5 |
| **253** | 5.79 |
| **254** | 8.93 |
| **255** | 5.75 |
| **256** | 4.12 |
| **257** | 47.2 |
| **258** | 449 |
| **259** | 31.9 |
| **260** | 28.0 |
| **261** | 366.0 |
| **262** | 15.8 |
| **263** | 10.1 |
| **264** | 5.33 |
| **265** | 22.7 |
| **266** | 7.18 |
| **267** | 103 |
| **268** | 52 |
| **269** | 10.1 |
| **270** | 22.7 |
| **271** | 2.19 |
| **272** | 71.63 |
| **273** | 3.81 |
| **274** | 3.71 |
| **275** | 1.65 |
| **276** | 5 |
| **279** | 2.71 |

(continued)

| Compound | $IC_{50}$ (nM) in Assay B2 |
|---|---|
| **281** | 717 |
| **283** | 1760 |
| **284** | >10000 |

**Example** B: Exemplary X-ray crystallography procedure for absolute configuration determination

Instrument: Rigaku Oxford diffraction XtaLAB synergy four-circle diffractometer equipped with a HyPix-6000HE area detector; micro-focus tube/Cu.

1. Single crystal cultivation
Single crystal was cultivated from solution evaporation at room temperature.
2. X-ray test and analysis

Reflections were collected and the structure was solved using SHELXT and refined using SHELXL.

## OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Numbered Clauses

The compounds, compositions, methods, and other subject matter described herein are futther described in the following numbered clauses:

**1.** A compound of Formula **(I):**

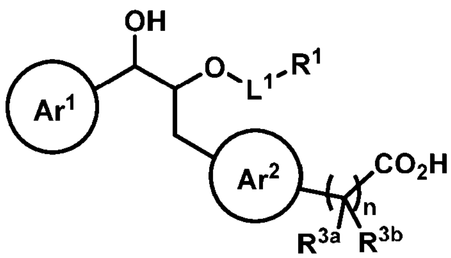

Formula (**I**)

or a pharmaceutically acceptable salt thereof, wherein:

**$L^1$** is selected from the group consisting of:

- a bond; and
- $C_{1-6}$ alkylene optionally substituted with from 1-6 **$R^a$**;

**$R^1$** is selected from the group consisting of: **$R^b$**; $C_{2-6}$ alkenyl optionally substituted with from 1-6 **$R^a$**; and $C_{2-6}$ alkynyl optionally substituted with from 1-6 **$R^a$**;

**$Ar^1$** is selected from the group consisting of:

- $C_{6-10}$ aryl optionally substituted with from 1-4 substituents each independently selected from the group consisting of: **$R^{c1}$** and **-($L^b$)$_b$-$R^b$**; and
- heteroaryl including from 5-10 ring atoms, wherein from 1-4 ring atoms are ring heteroatoms each

independently selected from the group consisting of: N, N(H), N(**R^d^**), O, and S, wherein the heteroaryl is optionally substituted with from 1-4 substituents each independently selected from the group consisting of: $\mathbf{R^{c1}}$ and - $\mathbf{(L^b)_b}$-$\mathbf{R^b}$;

$\mathbf{Ar^2}$ is selected from the group consisting of:

- $C_{6-10}$ arylene optionally substituted with from 1-4 $\mathbf{R^{c2}}$; and
- heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$;

n is 0 or 1;

$\mathbf{R^{3a}}$ and $\mathbf{R^{3b}}$ are independently H, -halo, $C_{1-6}$ alkyl, or $C_{1-4}$ haloalkyl; or

$\mathbf{R^{3a}}$ and $\mathbf{R^{3b}}$ taken together with the carbon atom to which each is attached forms a $C_{3-6}$ cycloalkyl;

each occurrence of $\mathbf{R^a}$ is independently selected from the group consisting of: -OH; -halo; -N$\mathbf{R^eR^f}$; $C_{1-4}$ alkoxy; $C_{1-4}$ haloalkoxy; -C(=O)O($C_{1-4}$ alkyl); -C(=O)($C_{1-4}$ alkyl); -C(=O)OH; -CON**R'R";** -$S(O)_{1-2}$N**R'R"**; -$S(O)_{1-2}$($C_{1-4}$ alkyl); and cyano;

each occurrence of $\mathbf{R^b}$ is independently selected from the group consisting of:

- $C_{3-10}$ cycloalkyl or $C_{3-10}$ cycloalkenyl, each of which is optionally substituted with from 1-4 $\mathbf{R^g}$;
- heterocyclyl or heterocycloalkenyl including from 3-10 ring atoms, wherein from 1-3 ring atoms are heteroatoms, each independently selected from the group consisting of N, N(H), N($\mathbf{R^d}$), O, and $S(O)_{0-2}$, and wherein the heterocyclyl or heterocycloalkenyl is optionally substituted with from 1-4 $\mathbf{R^g}$;
- heteroaryl including from 5-10 ring atoms, wherein from 1-4 ring atoms are heteroatoms, each independently selected from the group consisting of N, N(H), N($\mathbf{R^d}$), O, and $S(O)_{0-2}$, and wherein the heteroaryl is optionally substituted with from 1-4 $\mathbf{R^g}$; and
- $C_{6-10}$ aryl optionally substituted with from 1-4 $\mathbf{R^g}$;

**b** is 0, 1, 2, or 3;

each occurrence of $\mathbf{L^b}$ is selected from the group consisting of: $C_{1-3}$ alkylene; - N(H)-; N($\mathbf{R^d}$)-; -O-; -S-; C(=O); and $S(O)_{1-2}$;

each occurrence of $\mathbf{R^{c1}}$ and $\mathbf{R^{c2}}$ is independently selected from the group consisting of: halo; cyano; $C_{1-10}$ alkyl which is optionally substituted with from 1-6 independently selected $\mathbf{R^a}$; $C_{2-6}$ alkenyl; $C_{2-6}$ alkynyl; $C_{1-4}$ alkoxy; $C_{1-4}$ haloalkoxy; - $S(O)_{0-2}$($C_{1-4}$ alkyl); -N$\mathbf{R^eR^f}$; -OH; -$S(O)_{1-2}$N**R'R"**; -$NO_2$; -C(=O)($C_{1-10}$ alkyl); - C(=O)O($C_{1-4}$ alkyl); -C(=O)OH; and -C(=O)N**R'R"**;

each occurrence of $\mathbf{R^d}$ is independently selected from the group consisting of: $C_{1-6}$ alkyl optionally substituted with from 1-3 independently selected $\mathbf{R^a}$; -C(O)($C_{1-4}$ alkyl); -C(O)O($C_{1-4}$ alkyl); -CON**R'R"**; -$S(O)_{1-2}$N**R'R"**; -$S(O)_{1-2}$($C_{1-4}$ alkyl); -OH; and $C_{1-4}$ alkoxy;

each occurrence of $\mathbf{R^e}$ and $\mathbf{R^f}$ is independently selected from the group consisting of: H; $C_{1-6}$ alkyl; -C(O)($C_{1-4}$ alkyl); -C(O)O($C_{1-4}$ alkyl); -CON**R'R";** - $S(O)_{1-2}$N**R'R"**; -$S(O)_{1-2}$($C_{1-4}$ alkyl); -OH; and $C_{1-4}$ alkoxy; and

each occurrence of $\mathbf{R^g}$ is independently selected from the group consisting of: halo; cyano; $C_{1-6}$ alkyl; $C_{1-6}$ haloalkyl; $C_{1-4}$ alkoxy; $C_{1-4}$ haloalkoxy; -OH; and N**R'R";** and

each occurrence of **R'** and **R"** is independently selected from the group consisting of: H; -OH; and $C_{1-4}$ alkyl.

**2.** The compound of clause 1, wherein the compound is a compound of Formula **(I-A):**

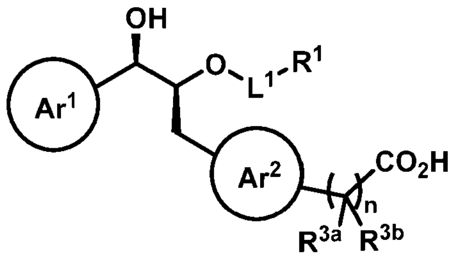

Formula (**I-A**)

or a pharmaceutically acceptable salt thereof.

**3.** The compound of clause 1 or 2, wherein **Ar¹** is $C_{6-10}$ aryl optionally substituted with from 1-4 substituents, each of which is independently selected from the group consisting of: $\mathbf{R^{c1}}$ and -$\mathbf{(L^b)_b}$-$\mathbf{R^b}$.

**4.** The compound of any one of clauses 1-3, wherein $\mathbf{Ar^1}$ is phenyl optionally substituted with from 1-4 substituents, each of which is independently selected from the group consisting of: $\mathbf{R^{c1}}$ and -$\mathbf{(L^b)_b}$-$\mathbf{R^b}$, such as phenyl substituted with from 1-4 substituents, each of which is independently selected from the group consisting of: $\mathbf{R^{c1}}$ and -$\mathbf{(L^b)_b}$-$\mathbf{R^b}$; or phenyl substituted with from 1-4 independently selected $\mathbf{R^{c1}}$.

**5.** The compound of any one of clauses 1-4, wherein each occurrence of $\mathbf{R^{c1}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; and cyano.

**6.** The compound of any one of clauses 1-5, wherein each occurrence of - $\mathbf{(L^b)_b}$-$\mathbf{R^b}$ is an independently selected $C_{3-6}$ cycloalkyl optionally substituted with from 1-2 $\mathbf{R^g}$.

**7.** The compound of any one of clauses 1-6, wherein $\mathbf{Ar^1}$ is

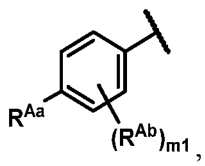

wherein **m1** is 0, 1, 2, or 3; and $\mathbf{R^{Aa}}$ and $\mathbf{R^{Ab}}$ are each independently selected from the group consisting of: $\mathbf{R^{c1}}$ and -$\mathbf{(L^b)_b}$-$\mathbf{R^b}$.

**8.** The compound of clause 7, wherein $\mathbf{R^{Aa}}$ is selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

**9.** The compound of clauses 7 or 8, wherein $\mathbf{R^{Aa}}$ is $C_{1-6}$ alkyl.

**10.** The compound of any one of clauses 7-9, wherein $\mathbf{R^{Aa}}$ is $C_{1-3}$ alkyl.

**11.** The compound of any one of clauses 7-10, wherein $\mathbf{R^{Aa}}$ is methyl.

**12.** The compound of clauses 7 or 8, wherein $\mathbf{R^{Aa}}$ is $C_{1-6}$ alkyl substituted with from 1-6 independently selected halo.

**13.** The compound of any one of clauses 7-8 or 12, wherein $\mathbf{R^{Aa}}$ is $C_{1-3}$ alkyl substituted with 1-6 F.

**14.** The compound of clause 13, wherein $\mathbf{R^{Aa}}$ is $CF_3$ or $CHF_2$.

**15.** The compound of clauses 7 or 8, wherein $\mathbf{R^{Aa}}$ is halo, such as -Cl.

**16.** The compound of clauses 7 or 8, wherein $\mathbf{R^{Aa}}$ is $C_{3-6}$ cycloalkyl.

17. The compound of any one of clauses 7-8 or 16, wherein $\mathbf{R^{Aa}}$ is cyclopropyl.

**18.** The compound of any one of clauses 7-17, wherein **m1** is 2.

**19.** The compound of any one of clauses 7-17, wherein **m1** is 1 or 3, such as 1.

**20.** The compound of any one of clauses 7-17, wherein **m1** is 0.

**21.** The compound of clauses 18 or 19, wherein when **m1** is 1 or 2, one or both occurrences of $\mathbf{R^{Ab}}$ are attached to the ring atom or atoms that are *ortho* to the ring atom attached to $\mathbf{R^{Aa}}$.

**22.** The compound of any one of clauses 7-19 or 21, wherein each $\mathbf{R^{Ab}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

**23.** The compound of any one of clauses 7-19 or 21, wherein each $\mathbf{R^{Ab}}$ is independently $C_{1-4}$ alkoxy or $C_{1-4}$ haloalkoxy.

**24.** The compound of any one of clauses 7-19 or 21-23, wherein each $\mathbf{R^{Ab}}$ is $C_{1-4}$ alkoxy.

**25.** The compound of clause 24, wherein each $\mathbf{R^{Ab}}$ is methoxy.

**26.** The compound of any one of clauses 1-7, wherein $\mathbf{Ar^1}$ is

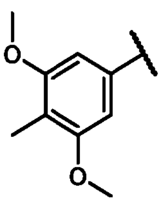

.

**27.** The compound of any one of clauses 1-7, wherein $\mathbf{Ar^1}$ is selected from the group consisting of:

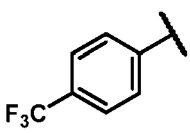 ; 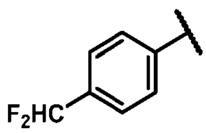 ; 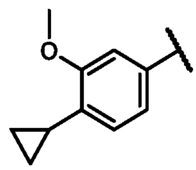 ;

and

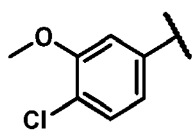

.

**28.** The compound of clauses 1 or 2, wherein $\mathbf{Ar^1}$ is heteroaryl including from 5-10 ring atoms, wherein from 1-4 ring atoms are ring heteroatoms each independently selected from the group consisting of: N, N(H), N($\mathbf{R^d}$), O, and S, wherein the heteroaryl is optionally substituted with from 1-4 substituents, each of which is independently selected from the group consisting of: $\mathbf{R^{c1}}$ and $\mathbf{-(L^b)_b-R^b}$.

**29.** The compound of any one of clauses 1-2 or 28, wherein $\mathbf{Ar^1}$ is heteroaryl including from 5-6 ring atoms, wherein from 1-4 ring atoms are ring heteroatoms each independently selected from the group consisting of: N, N(H), N($\mathbf{R^d}$), O, and S, wherein the heteroaryl is optionally substituted with from 1-4 substituents, each of which is independently selected from the group consisting of: $\mathbf{R^{c1}}$ and -$\mathbf{(L^b)_b-R^b}$.

**30.** The compound of any one of clauses 1-2 or 28-29, wherein $\mathbf{Ar^1}$ is heteroaryl including 6 ring atoms, wherein from

1-2 ring atoms are ring nitrogen atoms, wherein the heteroaryl is optionally substituted with from 1-4 substituents, each of which is independently selected from the group consisting of: $\mathbf{R^{c1}}$ and -$\mathbf{(L^b)_b}$-$\mathbf{R^b}$.

**31.** The compound of any one of clauses 1-2 or 28-30, wherein $\mathbf{Ar^1}$ is pyridyl optionally substituted with from 1-3 substituents selected from the group consisting of: $\mathbf{R^{c1}}$ and -$\mathbf{(L^b)_b}$-$\mathbf{R^b}$.

**32.** The compound of any one of clauses 1-2 or 28-31, wherein $\mathbf{Ar^1}$ is 3-pyridyl optionally substituted with from 1-3 substituents selected from the group consisting of: $\mathbf{R^{c1}}$ and -$\mathbf{(L^b)_b}$-$\mathbf{R^b}$.

**33.** The compound of any one of clauses 28-32, wherein each occurrence of $\mathbf{R^{c1}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; and cyano.

**34.** The compound of any one of clauses 28-32, wherein each occurrence of -$\mathbf{(L^b)_b}$-$\mathbf{R^b}$ is an independently selected $C_{3-6}$ cycloalkyl optionally substituted with from 1-2 $\mathbf{R^g}$.

**35.** The compound of any one of clauses 28-32, wherein $\mathbf{Ar^1}$ is 3-pyridyl substituted with 1-3 independently selected $C_{1-6}$ alkyl.

**36.** The compound of any one of clauses 28-35, wherein $\mathbf{Ar^1}$ is

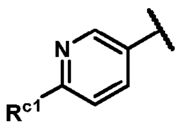

,

such as

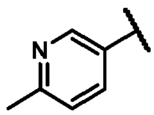

.

**37.** The compound of any one of clauses 1-36, wherein $\mathbf{L^1}$ is a bond.

**38.** The compound of any one of clauses 1-36, wherein $\mathbf{L^1}$ is $C_{1-6}$ alkylene optionally substituted with from 1-6 $\mathbf{R^a}$.

**39.** The compound of any one of clauses 1-36 or 38, wherein $\mathbf{L^1}$ is $C_{1-3}$ alkylene optionally substituted with from 1-6 $\mathbf{R^a}$.

**40.** The compound of any one of clauses 1-36 or 38-39, wherein $\mathbf{L^1}$ is unsubstituted $C_{1-3}$ alkylene.

**41.** The compound of any one of clauses 1-36 or 38-40, wherein $\mathbf{L^1}$ is $CH_2CH_2$.

**42.** The compound of any one of clauses 1-36 or 38-40, wherein $\mathbf{L^1}$ is $CH_2$.

**43.** The compound of any one of clauses 1-42, wherein $\mathbf{R^1}$ is $\mathbf{R^b}$.

**44.** The compound of any one of clauses 1-43, wherein $\mathbf{R^1}$ is selected from the group consisting of:

- $C_{3-10}$ cycloalkyl or $C_{3-10}$ cycloalkenyl, each of which is optionally substituted with from 1-4 $\mathbf{R^g}$; and
- $C_{6-10}$ aryl optionally substituted with from 1-4 $\mathbf{R^g}$.

**45.** The compound of any one of clauses 1-44, wherein $\mathbf{R^1}$ is $C_{6-10}$ aryl optionally substituted with from 1-4 $\mathbf{R^g}$.

**46.** The compound of any one of clauses 1-45, wherein $\mathbf{R^1}$ is phenyl optionally substituted with from 1-4 $\mathbf{R^g}$.

**47.** The compound of any one of clauses 1-46, wherein $\mathbf{R^1}$ is phenyl optionally substituted with from 1-2 $\mathbf{R^g}$.

**48.** The compound of any one of clauses 1-47, wherein $\mathbf{R^1}$ is unsubstituted phenyl.

**49.** The compound of any one of clauses 1-45, wherein $\mathbf{R^1}$ is $C_{8-10}$ bicyclic aryl optionally substituted with from 1-4 $\mathbf{R^g}$.

**50.** The compound of any one of clauses 1-45 or 49, wherein $\mathbf{R^1}$ is $C_{9-10}$ bicyclic aryl optionally substituted with from 1-2 $\mathbf{R^g}$.

**51.** The compound of any one of clauses 1-45 or 49-50, wherein $\mathbf{R^1}$ is indanyl optionally substituted with from 1-2 $\mathbf{R^g}$.

**52.** The compound of any one of clauses 1-45 or 49-51, wherein $\mathbf{R^1}$ is

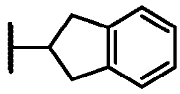,

which is optionally substituted with from 1-2 $\mathbf{R^g}$.

**53.** The compound of clause 52, wherein $\mathbf{R^1}$ is

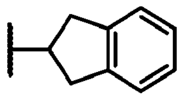.

**54.** The compound of any one of clauses 1-44, wherein $\mathbf{R^1}$ is $C_{3-10}$ cycloalkyl or $C_{3-10}$ cycloalkenyl, each of which is optionally substituted with from 1-4 $\mathbf{R^g}$.

**55.** The compound of any one of clauses 1-44 or 54, wherein $\mathbf{R^1}$ is $C_{3-10}$ cycloalkyl which is optionally substituted with from 1-4 $\mathbf{R^g}$.

**56.** The compound of any one of clauses 1-44 or 54-55, wherein $\mathbf{R^1}$ is $C_{3-6}$ cycloalkyl which is optionally substituted with from 1-2 $\mathbf{R^g}$.

**57.** The compound of any one of clauses 1-44 or 54-56, wherein $\mathbf{R^1}$ is cyclobutyl or cyclopentyl, each of which is optionally substituted with from 1-2 $\mathbf{R^g}$, such as cyclopentyl optionally substituted with from 1-2 $\mathbf{R^g}$.

**58.** The compound of any one of clauses 1-44 or 54-57, wherein $\mathbf{R^1}$ is unsubstituted cyclobutyl or cyclopentyl, such as unsubstituted cyclopentyl.

**59.** The compound of any one of clauses 1-42, wherein $\mathbf{R^1}$ is $C_{2-6}$ alkynyl optionally substituted with from 1-6 $\mathbf{R^a}$.

**60.** The compound of any one of clauses 1-42 or 59, wherein $\mathbf{R^1}$ is $C_{2-4}$ alkynyl optionally substituted with from 1-3 $\mathbf{R^a}$.

**61.** The compound of any one of clauses 1-42 or 59-60, wherein $\mathbf{R^1}$ is

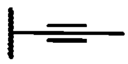.

**62.** The compound of any one of clauses 1-61, wherein $\mathbf{Ar^2}$ is heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$.

**63.** The compound of any one of clauses 1-62, wherein $\mathbf{Ar^2}$ is heteroarylene including from 5-6 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$.

**64.** The compound of any one of clauses 1-63, wherein $\mathbf{Ar^2}$ is heteroarylene including from 5 ring atoms, wherein from

1-4 ring atoms are ring heteroatoms each independently selected from the group consisting of: N, N(H), N(**R^d^**), O, and S, wherein the heteroarylene is optionally substituted with from 1-4 $\mathbf{R^{c2}}$.

**65.** The compound of any one of clauses 1-64, wherein $\mathbf{Ar^2}$ is selected from the group consisting of pyrrolylene, pyrazolylene, and thiazolylene, each of which is optionally substituted with $\mathbf{R^{c2}}$.

**66.** The compound of any one of clauses 1-64, wherein $\mathbf{Ar^2}$ is

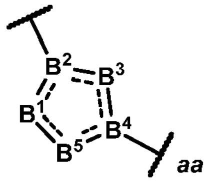

,

wherein:

each 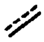 is independently a single bond or a double bond, provided that the ring including $\mathbf{B^1}$, $\mathbf{B^2}$, $\mathbf{B^3}$, $\mathbf{B^4}$, and $\mathbf{B^5}$ is heteroaryl;
***aa*** is the point of attachment to $-(C\mathbf{R^{3a}R^{3b}})_n$-C(O)OH;
$\mathbf{B^2}$ and $\mathbf{B^4}$ are C or N; and
$\mathbf{B^1}$, $\mathbf{B^3}$, and $\mathbf{B^5}$ are independently O, S, N, N(H), N($\mathbf{R^d}$), CH, or C$\mathbf{R^{c2}}$, provided that:
from 1-4 of $\mathbf{B^1}$, $\mathbf{B^2}$, $\mathbf{B^3}$, $\mathbf{B^4}$, and $\mathbf{B^5}$ are independently selected heteroatoms.

**67.** The compound of clause 66, wherein $\mathbf{B^2}$ is N.

**68.** The compound of clauses 66 or 67, wherein $\mathbf{B^4}$ is C.

**69.** The compound of any one of clauses 66-68, wherein $\mathbf{B^1}$, $\mathbf{B^3}$, and $\mathbf{B^5}$ are independently CH or C$\mathbf{R^{c2}}$.

**70.** The compound of any one of clauses 66-69, wherein $\mathbf{B^5}$ is C$\mathbf{R^{c2}}$; and $\mathbf{B^1}$ and $\mathbf{B^3}$ are CH.

**71.** The compound of clause 66, wherein $\mathbf{B^2}$ is N; $\mathbf{B^4}$ is C; and $\mathbf{B^1}$, $\mathbf{B^3}$, and $\mathbf{B^5}$ are independently CH or C$\mathbf{R^{c2}}$.

72. The compound of any one of clauses 1-66, wherein $\mathbf{Ar^2}$ is

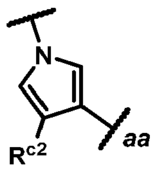

,

wherein ***aa*** is the point of attachment to $-(C\mathbf{R^{3a}R^{3b}})_n$-C(O)OH.

73. The compound of clause 72, wherein $\mathbf{R^{c2}}$ is C(O)OC$_{1-4}$ alkyl, such as C(O)OMe.

74. The compound of clause 66, wherein $\mathbf{B^2}$ is C.

75. The compound of clauses 66 or 74, wherein $\mathbf{B^4}$ is C.

76. The compound of any one of clauses 66 or 74-75, wherein $\mathbf{B^5}$ is CH or C$\mathbf{R^{c2}}$; one of $\mathbf{B^1}$ and $\mathbf{B^3}$ is N; and the other of $\mathbf{B^1}$ and $\mathbf{B^3}$ is NH, N($\mathbf{R^d}$), O, or S, such as S.

77. The compound of any one of clauses 1-66, wherein $\mathbf{Ar^2}$ is

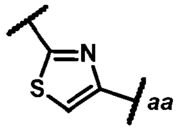

,

wherein ***aa*** is the point of attachment to -(C$\mathbf{R^{3a}R^{3b}}$)$_n$-C(O)OH.

78. The compound of clauses 66 or 74, wherein $\mathbf{B^4}$ is N.

79. The compound of any one of clauses 66, 74, or 78, wherein $\mathbf{B^3}$ is N; and $\mathbf{B^1}$ and $\mathbf{B^5}$ are independently CH or C$\mathbf{R^{c2}}$.

**80.** The compound of any one of clauses 66, 74, or 78-79, wherein $\mathbf{Ar^2}$ is

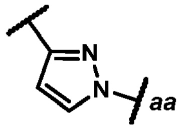

,

wherein ***aa*** is the point of attachment to -(C$\mathbf{R^{3a}R^{3b}}$)$_n$ C(O)OH.

**81.** The compound of any one of clauses 1-63, wherein $\mathbf{Ar^2}$ is heteroarylene including 6 ring atoms, wherein from 1-2 ring atoms are ring nitrogen atoms, wherein the heteroarylene is optionally substituted with from 1-4 $\mathbf{R^{c2}}$.

**82.** The compound of any one of clauses 1-63 or 81, wherein $\mathbf{Ar^2}$ is pyridylene which is optionally substituted with from 1-2 $\mathbf{R^{c2}}$.

83. The compound of any one of clauses 1-63 or 81-82, wherein $\mathbf{Ar^2}$ is

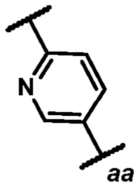

or

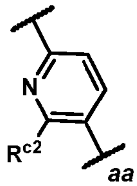

,

wherein ***aa*** is the point of attachment to -(C$\mathbf{R^{3a}R^{3}}$)$_n$-C(O)OH.

84. The compound of clause 83, wherein $\mathbf{R^{c2}}$ is $C_{1-4}$ alkoxy or $C_{1-4}$ haloalkoxy, such as -OMe.

85. The compound of any one of clauses 1-62, wherein $\mathbf{Ar^2}$ is bicyclic heteroarylene including from 9-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 $\mathbf{R^{c2}}$.

86. The compound of any one of clauses 1-62 or 85, wherein $\mathbf{Ar^2}$ is benzimidazolylene or indazolylene, each of which is optionally substituted with from 1-4 $\mathbf{R^{c2}}$.

87. The compound of any one of clauses 1-62 or 85, wherein $\mathbf{Ar^2}$ is:

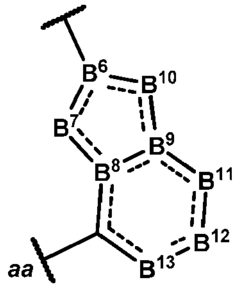

,

wherein:

each 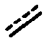 is independently a single bond or a double bond, provided that the 5-membered ring including $\mathbf{B}^{6}$, $\mathbf{B}^{7}$, $\mathbf{B}^{8}$, $\mathbf{B}^{9}$, and $\mathbf{B}^{10}$ is heteroaryl, and the 6-membered ring including $\mathbf{B}^{8}$, $\mathbf{B}^{9}$, $\mathbf{B}^{11}$, $\mathbf{B}^{12}$, and $\mathbf{B}^{13}$ is aryl or heteroaryl;
***aa*** is the point of attachment to $-(C\mathbf{R}^{3a}\mathbf{R}^{3b})_n-C(O)OH$;
$\mathbf{B}^{6}$, $\mathbf{B}^{8}$, and $\mathbf{B}^{9}$ are independently C or N;
$\mathbf{B}^{7}$ and $\mathbf{B}^{10}$ are independently selected from the group consisting of: O, S, N, N(H), N($\mathbf{R}^{d}$), CH, and C$\mathbf{R}^{c2}$;
$\mathbf{B}^{11}$, $\mathbf{B}^{12}$, and $\mathbf{B}^{13}$ are independently N, CH, or C$\mathbf{R}^{c2}$, provided that:

from 1-4 of $\mathbf{B}^{6}$, $\mathbf{B}^{7}$, $\mathbf{B}^{8}$, $\mathbf{B}^{9}$, $\mathbf{B}^{10}$, $\mathbf{B}^{11}$, $\mathbf{B}^{12}$, and $\mathbf{B}^{13}$ is an independently selected heteroatom; and no more than 3 of $\mathbf{B}^{7}$, $\mathbf{B}^{10}$, $\mathbf{B}^{11}$, $\mathbf{B}^{12}$, and $\mathbf{B}^{13}$ are C$\mathbf{R}^{c2}$.

88. The compound of clause 87, wherein $\mathbf{B}^{8}$ and $\mathbf{B}^{9}$ are C.

89. The compound of clauses 87 or 88, wherein $\mathbf{B}^{11}$, $\mathbf{B}^{12}$, and $\mathbf{B}^{13}$ are independently CH or C$\mathbf{R}^{c2}$.

90. The compound of any one of clauses 87-89, wherein $\mathbf{B}^{11}$, $\mathbf{B}^{12}$, and $\mathbf{B}^{13}$ are CH.

91. The compound of any one of clauses 87-90, wherein $\mathbf{B}^{6}$ is N.

92. The compound of any one of clauses 87-91, wherein $\mathbf{B}^{7}$ is N.

93. The compound of any one of clauses 87-92, wherein $\mathbf{B}^{10}$ is CH or C$\mathbf{R}^{c2}$.

94. The compound of any one of clauses 87-93, wherein $\mathbf{B}^{10}$ is CH.

95. The compound of any one of clauses 1-62 or 85-87, wherein $\mathbf{Ar}^{2}$ is

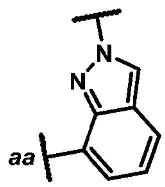

,

which is optionally substituted with from 1-2 $\mathbf{R}^{c2}$, wherein ***aa*** is the point of attachment to $-(C\mathbf{R}^{3a}\mathbf{R}^{3b})_n-C(O)OH$.

96. The compound of any one of clauses 1-62 or 85, wherein $\mathbf{Ar}^{2}$ is:

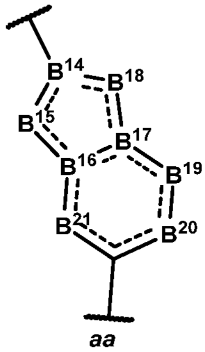

,

wherein:

each 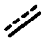 is independently a single bond or a double bond, provided that the 5-membered ring including **B$^{14}$**, **B$^{15}$**, **B$^{16}$**, **B$^{17}$**, and **B$^{18}$** is heteroaryl, and the 6-membered ring including **B$^{16}$**, **B$^{17}$**, **B$^{19}$**, **B$^{20}$**, and **B$^{21}$** is aryl or heteroaryl;
***aa*** is the point of attachment to -(C**R$^{3a}$R$^{3b}$**)$_n$-C(O)OH;
**B$^{16}$**, **B$^{17}$**, and **B$^{14}$** are independently C or N;
**B$^{15}$** and **B$^{18}$** are independently selected from the group consisting of: O, S, N, N(H), N(**R$^d$**), CH, and **CR$^{c2}$**;
**B$^{19}$**, **B$^{20}$**, and **B$^{21}$** are independently N, CH, or C**R$^{c2}$**, provided that:
from 1-4 of **B$^{14}$**, **B$^{15}$**, **B$^{16}$**, **B$^{17}$**, **B$^{18}$**, **B$^{19}$**, **B$^{20}$**, and **B$^{21}$** is an independently selected heteroatom; and no more than 3 of **B$^{15}$**, **B$^{18}$**, **B$^{19}$**, **B$^{20}$**, and **B**$^{21}$ are C**R$^{c2}$**.

97. The compound of clause 96, wherein **B$^{16}$** and **B$^{17}$** are C.

98. The compound of clauses 96 or 97, wherein **B$^{19}$**, **B$^{20}$**, and **B$^{21}$** are independently N, CH, or **CR$^{c2}$.**

99. The compound of any one of clauses 96-98, wherein **B$^{14}$** is C.

100. The compound of any one of clauses 96-99, wherein one of **B$^{15}$** and **B$^{18}$** is N; and the other of **B$^{15}$** and **B$^{18}$** is O, S, NH, or N(**R$^d$**), such as NH or N(**R$^d$**), such as NH or N($C_{1-3}$ alkyl).

**101.** The compound of any one of clauses 1-62, 85-86, or 96, wherein **Ar$^2$** is

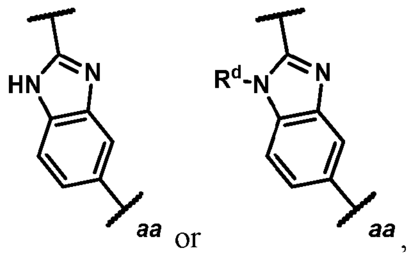

each of which is optionally substituted with from 1-2 **R$^{c2}$**, wherein ***aa*** is the point of attachment to -(C**R$^{3a}$R$^{3b}$**)$_n$-C(O)OH.

102. The compound of any one of clauses 1-101, wherein n is 0.

103. The compound of any one of clauses 1-101, wherein n is 1, and optionally wherein **R$^{3a}$** and **R$^{3b}$** are H.

104. The compound of clause 1, wherein the compound is a compound of Formula (**I-1**):

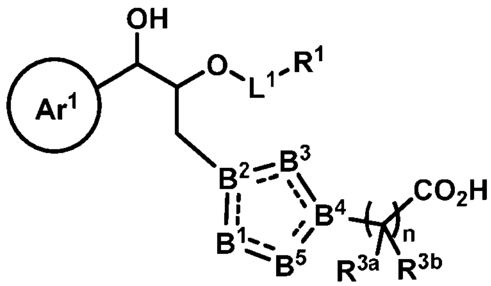

Formula (**I-1**)

or a pharmaceutically acceptable salt thereof, wherein:

each 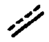 is independently a single bond or a double bond, provided that the ring including **B**$^1$, **B**$^2$, **B**$^3$, **B**$^4$, and **B**$^5$ is heteroaryl;
**B**$^2$ and **B**$^4$ are C or N;
**B**$^1$, **B**$^3$, and **B**$^5$ are independently O, S, N, N(H), N(**R**$^d$), CH, or C**R**$^{c2}$, provided that: from 1-4 of **B**$^1$, **B**$^2$, **B**$^3$, **B**$^4$, and **B**$^5$ are independently selected heteroatoms; and
**Ar**$^1$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^g$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^g$.

105. The compound of clause 104, wherein **B**$^2$ is N; and **B**$^4$ is C.

106. The compound of clauses 104 or 105, wherein **B**$^1$, **B**$^3$, and **B**$^5$ are independently CH or C**R**$^{c2}$.

107. The compound of any one of clauses 104-106, wherein the ring including **B**$^1$-**B**$^5$ is is

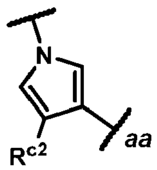

,

wherein ***aa*** is the point of attachment to - (C**R**$^{3a}$**R**$^{3b}$)$_n$-C(O)OH, optionally wherein **R**$^{c2}$ is C(O)O$C_{1-4}$ alkyl.

108. The compound of clause 104, wherein **B**$^2$ is C; and **B**$^4$ is N.

109. The compound of clauses 104 or 108, wherein **B**$^3$ is N; and **B**$^1$ and **B**$^5$ are independently CH or C**R**$^{c2}$.

110. The compound of any one of clauses 104 or 108-109, wherein the ring including **B**$^1$-**B**$^5$ is

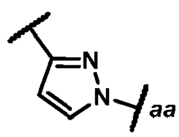

,

wherein ***aa*** is the point of attachment to - (C**R**$^{3a}$**R**$^{3b}$)$_n$-C(O)OH.

111. The compound of clause 104, wherein **B²** is C; and **B⁴** is C.

112. The compound of clauses 104 or 111, wherein **B³** is CH or **CR^c2**; one of **B¹** and **B³** is N; and the other of **B¹** and **B³** is NH, N(**R^d**), O, or S, such as S.

**113.** The compound of any one of clauses 104 or 111-112, wherein the ring including **B¹-B⁵** is

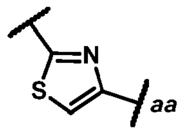

wherein ***aa*** is the point of attachment to - ($CR^{3a}R^{3b}$)$_n$C(O)OH.

114. The compound of clause 1, wherein the compound is a compound of Formula **(I-2)**:

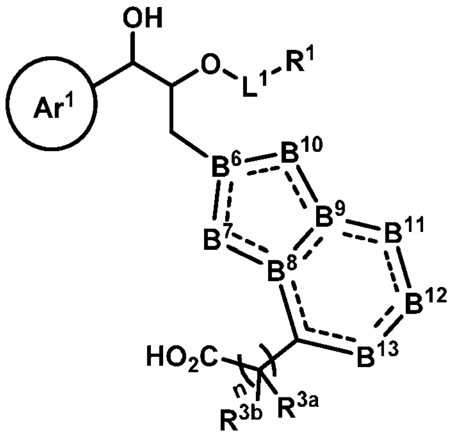

Formula (**I-2**)

or a pharmaceutically acceptable salt thereof, wherein:

each 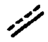 is independently a single bond or a double bond, provided that the 5-membered ring including $B^6$, $B^7$, $B^8$, $B^9$, and $B^{10}$ is heteroaryl, and the 6-membered ring including $B^8$, $B^9$, $B^{11}$, $B^{12}$, and $B^{13}$ is aryl or heteroaryl;
$B^6$, $B^8$, and $B^9$ are independently C or N;
$B^7$ and $B^{10}$ are independently selected from the group consisting of: O, S, N, N(H), N($R^d$), CH, or $CR^{c2}$;
$B^{11}$, $B^{12}$, and $B^{13}$ are independently N, CH, or $CR^{c2}$, provided that:

from 1-4 of $B^6$, $B^7$, $B^8$, $B^9$, $B^{10}$, $B^{11}$, $B^{12}$, and $B^{13}$ is an independently selected heteroatom; and no more than 3 of $B^7$, $B^{10}$, $B^{11}$, $B^{12}$, and $B^{13}$ are $CR^{c2}$; and

$Ar^1$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$.

115. The compound of clause 114, wherein $B^8$ and $B^9$ are C.

116. The compound of clauses 114 or 115, wherein $B^{11}$, $B^{12}$, and $B^{13}$ are independently CH or $CR^{c2}$.

117. The compound of any one of clauses 114-116, wherein **B⁶** is N; **B⁷** is N; and **B¹⁰** is CH or C**R**ᶜ².

118. The compound of any one of clauses 114-117, wherein the ring including **B⁶-B¹³** is

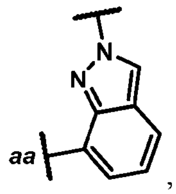

which is optionally substituted with from 1-2 $\mathbf{R}^{c2}$, wherein ***aa*** is the point of attachment to -(C$\mathbf{R}^{3a}\mathbf{R}^{3b}$)$_n$-C(O)OH.

119. The compound of clause 1, wherein the compound is a compound of Formula (**I-3**):

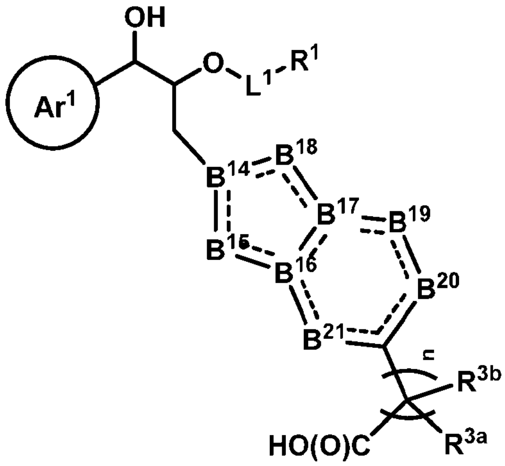

Formula (**I-3**)

or a pharmaceutically acceptable salt thereof, wherein:

each 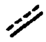 is independently a single bond or a double bond, provided that the 5-membered ring including $\mathbf{B}^{14}$, $\mathbf{B}^{15}$, $\mathbf{B}^{16}$, $\mathbf{B}^{17}$, and $\mathbf{B}^{18}$ is heteroaryl, and the 6-membered ring including $\mathbf{B}^{16}$, $\mathbf{B}^{17}$, $\mathbf{B}^{19}$, $\mathbf{B}^{20}$, and $\mathbf{B}^{21}$ is aryl or heteroaryl;

$\mathbf{B}^{16}$, $\mathbf{B}^{17}$, and $\mathbf{B}^{14}$ are independently C or N;

$\mathbf{B}^{15}$ and $\mathbf{B}^{18}$ are independently selected from the group consisting of: O, S, N, N(H), N($\mathbf{R}^{d}$), CH, and C$\mathbf{R}^{c2}$;

$\mathbf{B}^{19}$, $\mathbf{B}^{20}$, and $\mathbf{B}^{21}$ are independently N, CH, or C$\mathbf{R}^{c2}$, provided that:

from 1-4 of $\mathbf{B}^{14}$, $\mathbf{B}^{15}$, $\mathbf{B}^{16}$, $\mathbf{B}^{17}$, $\mathbf{B}^{18}$, $\mathbf{B}^{19}$, $\mathbf{B}^{20}$, and $\mathbf{B}^{21}$ is an independently selected heteroatom; and no more than 3 of $\mathbf{B}^{15}$, $\mathbf{B}^{18}$, $\mathbf{B}^{19}$, $\mathbf{B}^{20}$, and $\mathbf{B}^{21}$ are C$\mathbf{R}^{c2}$; and

$\mathbf{Ar}^1$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R}^{g}$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R}^{g}$.

120. The compound of clause 119, wherein $\mathbf{B}^{16}$ and $\mathbf{B}^{17}$ are C.

121. The compound of clauses 119 or 120, wherein **$B^{19}$**, **$B^{20}$**, and **$B^{21}$** are independently CH or C**$R^{c2}$**.

122. The compound of any one of clauses 119-121, wherein **$B^{14}$** is C; one of **$B^{15}$** and **$B^{18}$** is N; and the other of **$B^{15}$** and **$B^{18}$** is O, S, NH, or N(**$R^d$**), such as NH or N(**$R^d$**), such as NH or N($C_{1-3}$ alkyl).

**123.** The compound of any one of clauses 119-122, wherein the ring including **$B^{14}$-$B^{21}$** is

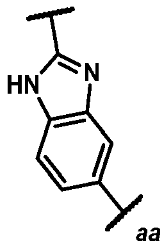 or 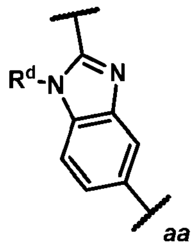 ,

each of which is optionally substituted with from 1-2 **$R^{c2}$**, wherein ***aa*** is the point of attachment to -(C**$R^{3a}R^{3b}$**)$_n$-C(O)OH.

124. The compound of any one of clauses 104-123, wherein n is 1, and optionally wherein **$R^{3a}$** and **$R^{3b}$** are H.

125. The compound of any one of clauses 104-123, wherein n is 0.

126. The compound of any one of clauses 104-125, wherein **$Ar^1$** is

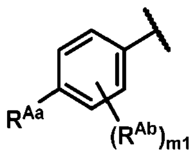 ;

**m1** is 0, 1, 2, or 3; and each occurrence of **$R^{Aa}$** and **$R^{Ab}$** are independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **$R^g$**.

127. The compound of any one of clauses 104-126, wherein **m1** is 0.

128. The compound of any one of clauses 104-127, wherein **m1** is 1 or 2, optionally wherein each **$R^{Ab}$** is *ortho* to **$R^{Aa}$**.

129. The compound of any one of clauses 104-128, wherein each **$R^{Ab}$** when present is $C_{1-4}$ alkoxy or $C_{1-4}$ haloalkoxy, such as $C_{1-4}$ alkoxy, such as methoxy.

130. The compound of any one of clauses 104-129, wherein **$R^{Aa}$** is $C_{1-3}$ alkyl; $C_{1-3}$ alkyl substituted with 1-6 F; halo; or $C_{3-6}$ cycloalkyl.

**131.** The compound of any one of clauses 104-126, wherein **Ar**$^1$ is

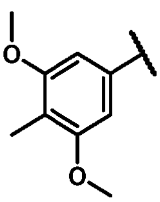 .

**132.** The compound of any one of clauses 104-126, wherein $Ar^1$ is selected from the group consisting of:

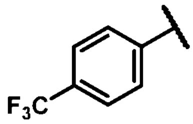 ; 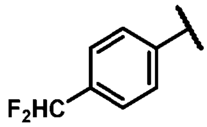 ; 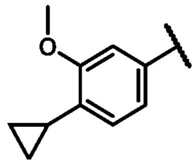 ;

and

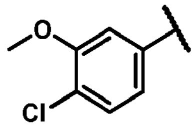 .

**133.** The compound of any one of clauses 104-132, wherein $\mathbf{L^1}$ is a bond.

**134.** The compound of any one of clauses 104-132, wherein $L^1$ is $C_{1-3}$ alkylene optionally substituted with from 1-6 $\mathbf{R^a}$.

135. The compound of any one of clauses 104-132 or 134, wherein $\mathbf{L^1}$ is $CH_2$ or $CH_2CH_2$.

**136.** The compound of any one of clauses 104-135, wherein $\mathbf{R^1}$ is phenyl optionally substituted with from 1-2 $\mathbf{R^g}$.

137. The compound of any one of clauses 104-135, wherein $\mathbf{R^1}$ is $C_{9-10}$ bicyclic aryl optionally substituted with from 1-2 $\mathbf{R^g}$.

**138.** The compound of any one of clauses 104-135 or 137, wherein $\mathbf{R^1}$ is

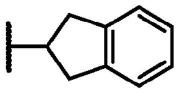 ,

which is optionally substituted with from 1-2 $\mathbf{R^g}$.

**139.** The compound of any one of clauses 104-135, wherein $\mathbf{R^1}$ is $C_{3-6}$ cycloalkyl which is optionally substituted with from 1-2 $\mathbf{R^g}$.

140. The compound of any one of clauses 104-135 or 139, wherein $\mathbf{R^1}$ is cyclobutyl or cyclopentyl, each of which is optionally substituted with from 1-2 $\mathbf{R^g}$.

141. The compound of any one of clauses 104-140, wherein the

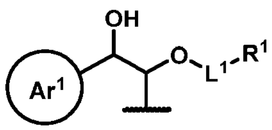

moiety has the following formula:

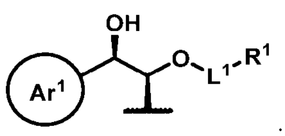 .

**142.** The compound of clause 1, wherein the compound is selected from the group consisting of compounds delineated in **Tables C1** and **C2**, or a pharmaceutically acceptable salt thereof.

143. A pharmaceutical composition comprising a compound of any one of clauses 1-142, or a pharmaceutically

acceptable salt or solvate thereof, and a pharmaceutically acceptable excipient.

144. A method for treating or preventing an LPA-associated disease in a subject in need thereof, the method comprising administering to subject a therapeutically effective amount of a compound of any one of clauses 1-142, or a pharmaceutically acceptable salt or solvate thereof, or a pharmaceutical composition of clause 143.

145. The method of clause 144, wherein the LPA-associated disease is an $LPA_1$-associated disease.

146. The method of any one of clause 144 or 145, wherein the LPA-associated disease is selected from the group consisting of fibrosis, transplant rejection, cancer, osteoporosis, or inflammatory disorders.

147. The method of clause 146, wherein the fibrosis is pulmonary, liver, renal, cardiac, dermal, ocular, or pancreatic fibrosis.

148. The method of any one of clauses 144 or 145, wherein the LPA-associated disease is selected from the group consisting of idiopathic pulmonary fibrosis (IPF), non-alcoholic steatohepatitis (NASH), non-alcoholic fatty liver disease (NAFLD), chronic kidney disease, diabetic kidney disease, and systemic sclerosis.

149. The method of clause 146, wherein the cancer is of the bladder, blood, bone, brain, breast, central nervous system, cervix, colon, endometrium, esophagus, gall bladder, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid.

150. A method for treating or preventing fibrosis in a subject in need thereof, the method comprising administering to subject a therapeutically effective amount of a compound of any one of clauses 1-142, or a pharmaceutically acceptable salt or solvate thereof, or a pharmaceutical composition of clause 143.

151. The method of clause 150, wherein the fibrosis is idiopathic pulmonary fibrosis (IPF), nonalcoholic steatohepatitis (NASH), chronic kidney disease, diabetic kidney disease, and systemic sclerosis.

152. The method of clause 151, wherein the fibrosis is IPF.

The invention is further described by the following numbered embodiments:

1. A compound of Formula (I):

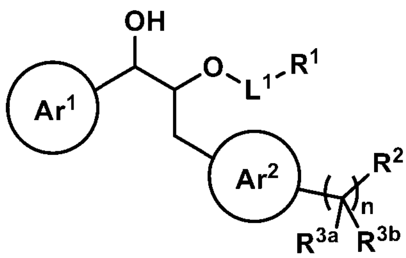

Formula (**I**)

or a pharmaceutically acceptable salt thereof, wherein:

**$L^1$** is selected from the group consisting of:

- a bond; and
- $C_{1-6}$ alkylene optionally substituted with from 1-6 **$R^a$**;

**$R^1$** is selected from the group consisting of: **$R^b$**; $C_{1-6}$ alkyl optionally substituted with from 1-6 **$R^a$**; $C_{2-6}$ alkenyl optionally substituted with from 1-6 **$R^a$**; and $C_{2-6}$ alkynyl optionally substituted with from 1-6 **$R^a$**;

**$Ar^1$** is selected from the group consisting of:

- $C_{6-10}$ aryl optionally substituted with from 1-4 substituents each independently selected from the group consisting of: $\mathbf{R^{c1}}$ and $-\mathbf{(L^b)_b-R^b}$; and
- heteroaryl including from 5-10 ring atoms, wherein from 1-4 ring atoms are ring heteroatoms each independently selected from the group consisting of: N, N(H), N($\mathbf{R^d}$), O, and S, wherein the heteroaryl is optionally substituted with from 1-4 substituents each independently selected from the group consisting of: $\mathbf{R^{c1}}$ and $-\mathbf{(L^b)_b-R^b}$;

**Ar²** is selected from the group consisting of:

- $C_{6-10}$ arylene optionally substituted with from 1-4 substituents each independently selected from the group consisting of: $\mathbf{R^{c2}}$ and $-\mathbf{(L^b)_b-R^b}$; and
- heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents each independently selected from the group consisting of: $\mathbf{R^{c2}}$ and $-\mathbf{(L^b)_b-R^b}$;

$\mathbf{R^2}$ is -C(=O)OH or carboxylic acid bioisostere;

n is 0 or 1;

$\mathbf{R^{3a}}$ and $\mathbf{R^{3b}}$ are independently H, -halo, $C_{1-6}$ alkyl, or $C_{1-4}$ haloalkyl; or

$\mathbf{R^{3a}}$ and $\mathbf{R^{3b}}$ taken together with the carbon atom to which each is attached forms a $C_{3-6}$ cycloalkyl;

each occurrence of $\mathbf{R^a}$ is independently selected from the group consisting of: -OH; - halo; -N$\mathbf{R^eR^f}$; $C_{1-4}$ alkoxy; $C_{1-4}$ haloalkoxy; -C(=O)O($C_{1-4}$ alkyl); -C(=O)($C_{1-4}$ alkyl); - C(=O)OH; -CON**R'R"**; $-S(O)_{1-2}$N**R'R"**; $-S(O)_{1-2}$($C_{1-4}$ alkyl); and cyano;

each occurrence of $\mathbf{R^b}$ is independently selected from the group consisting of:

- $C_{3-10}$ cycloalkyl or $C_{3-10}$ cycloalkenyl, each of which is optionally substituted with from 1-4 $\mathbf{R^g}$;
- heterocyclyl or heterocycloalkenyl including from 3-10 ring atoms, wherein from 1-3 ring atoms are heteroatoms, each independently selected from the group consisting of N, N(H), N($\mathbf{R^d}$), O, and $S(O)_{0-2}$, and wherein the heterocyclyl or heterocycloalkenyl is optionally substituted with from 1-4 $\mathbf{R^g}$;
- heteroaryl including from 5-10 ring atoms, wherein from 1-4 ring atoms are heteroatoms, each independently selected from the group consisting of N, N(H), N($\mathbf{R^d}$), O, and $S(O)_{0-2}$, and wherein the heteroaryl is optionally substituted with from 1-4 $\mathbf{R^g}$; and
- $C_{6-10}$ aryl optionally substituted with from 1-4 $\mathbf{R^g}$;

**b** is 0, 1, 2, or 3;

each occurrence of $\mathbf{L^b}$ is selected from the group consisting of: $C_{1-3}$ alkylene; -N(H)-; N($\mathbf{R^d}$)-; -O-; -S-; C(=O); and $S(O)_{1-2}$;

each occurrence of $\mathbf{R^{c1}}$ and $\mathbf{R^{c2}}$ is independently selected from the group consisting of: halo; cyano; $C_{1-10}$ alkyl which is optionally substituted with from 1-6 independently selected $\mathbf{R^a}$; $C_{2-6}$ alkenyl; $C_{2-6}$ alkynyl; $C_{1-4}$ alkoxy; $C_{1-4}$ haloalkoxy; $-S(O)_{0-2}$($C_{1-4}$ alkyl); -N$\mathbf{R^eR^f}$; -OH; $-S(O)_{1-2}$N**R'R"**; $-NO_2$; -C(=O)($C_{1-10}$ alkyl); -C(=O)O($C_{1-4}$ alkyl); -C(=O)OH; and - C(=O)N**R'R"**;

each occurrence of $\mathbf{R^d}$ is independently selected from the group consisting of: $C_{1-6}$ alkyl optionally substituted with from 1-3 independently selected $\mathbf{R^a}$; -C(O)($C_{1-4}$ alkyl); -C(O)O($C_{1-4}$ alkyl); -CON**R'R"**; $-S(O)_{1-2}$N**R'R"**; $-S(O)_{1-2}$($C_{1-4}$ alkyl); -OH; and $C_{1-4}$ alkoxy;

each occurrence of $\mathbf{R^e}$ and $\mathbf{R^f}$ is independently selected from the group consisting of: H; $C_{1-6}$ alkyl; -C(O)($C_{1-4}$ alkyl); -C(O)O($C_{1-4}$ alkyl); -CON**R'R"**; $-S(O)_{1-2}$N**R'R"**; $-S(O)_{1-2}$($C_{1-4}$ alkyl); -OH; and $C_{1-4}$ alkoxy; and

each occurrence of $\mathbf{R^g}$ is independently selected from the group consisting of: halo; cyano; $C_{1-6}$ alkyl; $C_{1-6}$ haloalkyl; $C_{1-4}$ alkoxy; $C_{1-4}$ haloalkoxy; -OH; and N**R'R"**; and

each occurrence of **R'** and **R"** is independently selected from the group consisting of: H; -OH; and $C_{1-4}$ alkyl.

2. The compound of embodiment 1, wherein the compound is a compound of Formula **(I-A):**

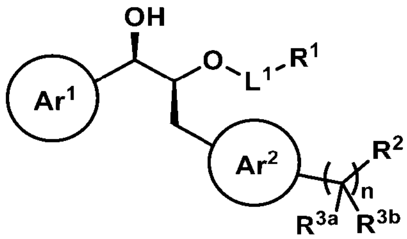

Formula **(I-A)**

or a pharmaceutically acceptable salt thereof.

3. The compound of embodiments 1 or 2, wherein **Ar$^1$** is $C_{6-10}$ aryl optionally substituted with from 1-4 substituents, each of which is independently selected from the group consisting of: **R$^{c1}$** and -**(L$^b$)$_b$-R$^b$**.

4. The compound of any one of embodiments 1-3, wherein **Ar$^1$** is phenyl optionally substituted with from 1-4 substituents, each of which is independently selected from the group consisting of: **R$^{c1}$** and -**(L$^b$)$_b$-R$^b$**, such as phenyl substituted with from 1-4 substituents, each of which is independently selected from the group consisting of: **R$^{c1}$** and - **(L$^b$)$_b$-R$^b$**; or phenyl substituted with from 1-4 independently selected **R$^{c1}$**.

5. The compound of any one of embodiments 1-4, wherein each occurrence of **R$^{c1}$** is independently selected from the group consisting of: halo; $C_{1-6}$ alkyl; $C_{1-6}$ alkyl substituted with from 1-6 independently selected halo; -C(=O)($C_{1-10}$ alkyl); $C_{1-4}$ alkoxy; $C_{1-4}$ haloalkoxy; and cyano.

6. The compound of any one of embodiments 1-5, wherein each occurrence of - **(L$^b$)$_b$-R$^b$** is an independently selected $C_{3-6}$ cycloalkyl optionally substituted with from 1-2 **R$^g$**.

7. The compound of any one of embodiments 1-6, wherein **Ar$^1$** is

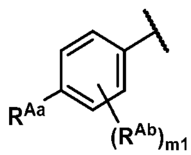,

wherein **m1** is 0, 1, 2, or 3; and **R$^{Aa}$** and **R$^{Ab}$** are each independently selected from the group consisting of: **R$^{c1}$** and -**(L$^b$)$_b$-R$^b$**.

8. The compound of embodiment 7, wherein **R$^{Aa}$** is selected from the group consisting of: halo; $C_{1-6}$ alkyl; $C_{1-6}$ alkyl substituted with from 1-6 independently selected halo; -C(=O)($C_{1-10}$ alkyl); $C_{1-4}$ alkoxy; $C_{1-4}$ haloalkoxy; cyano; and $C_{3-6}$ cycloalkyl optionally substituted with from 1-2 **R$^g$**.

9. The compound of embodiments 7 or 8, wherein **R$^{Aa}$** is $C_{1-6}$ alkyl.

10. The compound of any one of embodiments 7-9, wherein **R$^{Aa}$** is $C_{1-3}$ alkyl.

11. The compound of any one of embodiments 7-10, wherein **R$^{Aa}$** is methyl.

12. The compound of embodiments 7 or 8, wherein **R$^{Aa}$** is $C_{1-6}$ alkyl substituted with from 1-6 independently selected halo.

13. The compound of any one of embodiments 7-8 or 12, wherein **R$^{Aa}$** is $C_{1-3}$ alkyl substituted with 1-6 F, optionally wherein **R$^{Aa}$** is $CF_3$ or $CHF_2$.

**14.** The compound of embodiment 7 or 8, wherein $\mathbf{R^{Aa}}$ is cyano.

15. The compound of embodiments 7 or 8, wherein $\mathbf{R^{Aa}}$ is halo, such as -Cl.

16. The compound of embodiments 7 or 8, wherein $\mathbf{R^{Aa}}$ is $C_{3-6}$ cycloalkyl.

17. The compound of any one of embodiments 7-8 or 16, wherein $\mathbf{R^{Aa}}$ is cyclopropyl.

18. The compound of any one of embodiments 7-17, wherein **m1** is 2.

19. The compound of any one of embodiments 7-17, wherein **m1** is 1 or 3, such as 1.

20. The compound of any one of embodiments 7-17, wherein **m1** is 0.

21. The compound of embodiments 18 or 19, wherein when **m1** is 1 or 2, one or both occurrences of $\mathbf{R^{Ab}}$ are attached to the ring atom or atoms that are *ortho* to the ring atom attached to $\mathbf{R^{Aa}}$.

22. The compound of any one of embodiments 7-19 or 21, wherein each $\mathbf{R^{Ab}}$ is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

23. The compound of any one of embodiments 7-19 or 21-22, wherein each $\mathbf{R^{Ab}}$ is independently $C_{1-4}$ alkoxy or $C_{1-4}$ haloalkoxy.

24. The compound of any one of embodiments 7-19 or 21-23, wherein each $\mathbf{R^{Ab}}$ is $C_{1-4}$ alkoxy.

25. The compound of embodiment 24, wherein each $\mathbf{R^{Ab}}$ is methoxy.

**26.** The compound of any one of embodiments 1-7, wherein $\mathbf{Ar^1}$ is

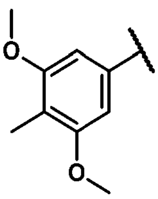

.

**27.** The compound of any one of embodiments 1-7, wherein $\mathbf{Ar^1}$ is selected from the group consisting of:

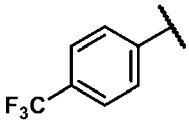 ; 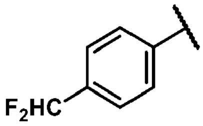 ; 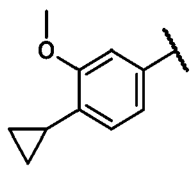 ; 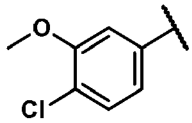 ;

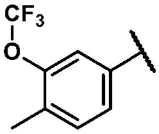 ; 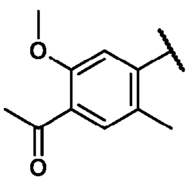 ; 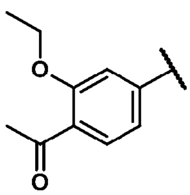 ; 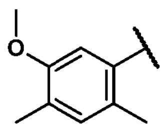 ; 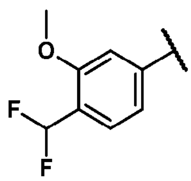 ; 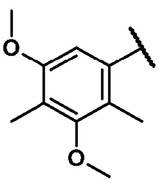 ;

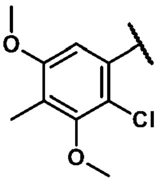; 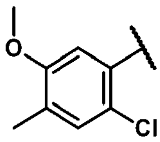; 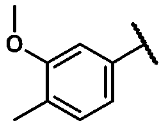; 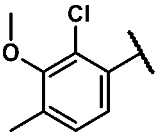; 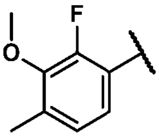; 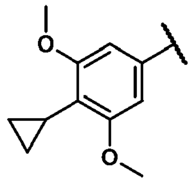; 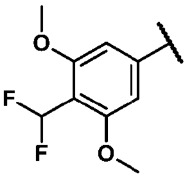; 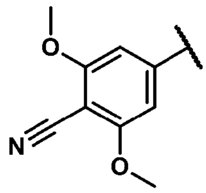; 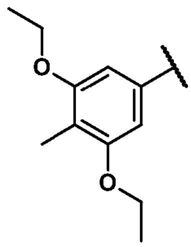; 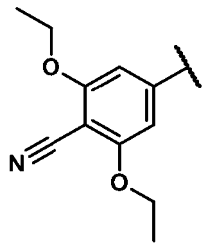; 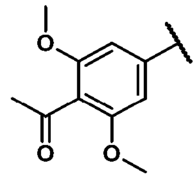; 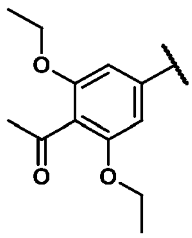; 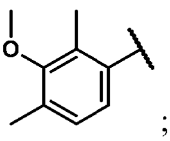; and 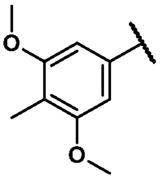.

28. The compound of embodiments 1 or 2, wherein **Ar$^1$** is heteroaryl including from 5-10 ring atoms, wherein from 1-4 ring atoms are ring heteroatoms each independently selected from the group consisting of: N, N(H), N(**R$^d$**), O, and S, wherein the heteroaryl is optionally substituted with from 1-4 substituents, each of which is independently selected from the group consisting of: **R$^{c1}$** and -**(L$^b$)$_b$-R$^b$**.

29. The compound of any one of embodiments 1-2 or 28, wherein **Ar$^1$** is heteroaryl including from 5-6 ring atoms, wherein from 1-4 ring atoms are ring heteroatoms each independently selected from the group consisting of: N, N(H), N(**R$^d$**), O, and S, wherein the heteroaryl is optionally substituted with from 1-4 substituents, each of which is independently selected from the group consisting of: **R$^{c1}$** and -**(L$^b$)$_b$-R$^b$**.

30. The compound of any one of embodiments 1-2 or 28-29, wherein **Ar$^1$** is heteroaryl including 6 ring atoms, wherein from 1-2 ring atoms are ring nitrogen atoms, wherein the heteroaryl is optionally substituted with from 1-4 substituents, each of which is independently selected from the group consisting of: **R$^{c1}$** and -**(L$^b$)$_b$-R$^b$**.

31. The compound of any one of embodiments 1-2 or 28-30, wherein **Ar$^1$** is pyridyl optionally substituted with from 1-3 substituents selected from the group consisting of: **R$^{c1}$** and -**(L$^b$)$_b$-R$^b$**.

32. The compound of any one of embodiments 1-2 or 28-31, wherein **Ar$^1$** is 3-pyridyl, or **Ar$^1$** is 4-pyridyl, each of which is optionally substituted with from 1-3 substituents selected from the group consisting of: **R$^{c1}$** and -**(L$^b$ )$_b$-R$^b$**.

33. The compound of any one of embodiments 28-32, wherein each occurrence of **R$^{c1}$** is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; and cyano.

34. The compound of any one of embodiments 28-32, wherein each occurrence of -**(L$^b$)$_b$-R$^b$** is an independently selected $C_{3-6}$ cycloalkyl optionally substituted with from 1-2 **R$^g$**.

35. The compound of any one of embodiments 28-32, wherein **Ar$^1$** is 3-pyridyl substituted with 1-3 independently selected $C_{1-6}$ alkyl, or **Ar$^1$** is 4-pyridyl substituted with 1-3 independently selected $C_{1-6}$ alkoxy.

**36.** The compound of any one of embodiments 28-35, wherein **Ar$^1$** is

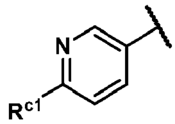

, such as

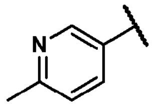

,

or **Ar$^1$** is

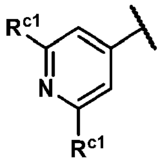

such as

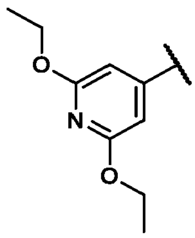

.

37. The compound of any one of embodiments 1-36, wherein **L$^1$** is a bond.

38. The compound of any one of embodiments 1-36, wherein **L$^1$** is $C_{1-6}$ alkylene optionally substituted with from 1-6 **R$^a$**.

39. The compound of any one of embodiments 1-36 or 38, wherein **L$^1$** is $C_{1-3}$ alkylene optionally substituted with from 1-6 **R$^a$**.

40. The compound of any one of embodiments 1-36 or 38-39, wherein **L$^1$** is unsubstituted $C_{1-3}$ alkylene.

41. The compound of any one of embodiments 1-36 or 38-40, wherein **L$^1$** is $CH_2CH_2$.

42. The compound of any one of embodiments 1-36 or 38-40, wherein **L$^1$** is $CH_2$.

43. The compound of any one of embodiments 1-42, wherein **R$^1$** is **R$^b$**.

44. The compound of any one of embodiments 1-43, wherein **R$^1$** is selected from the group consisting of:

- $C_{3-10}$ cycloalkyl or $C_{3-10}$ cycloalkenyl, each of which is optionally substituted with from 1-4 **R$^g$**; and
- $C_{6-10}$ aryl optionally substituted with from 1-4 **R$^g$**.

**45.** The compound of any one of embodiments 1-44, wherein **R$^1$** is $C_{6-10}$ aryl optionally substituted with from 1-4 **R$^g$**.

46. The compound of any one of embodiments 1-45, wherein **R$^1$** is phenyl optionally substituted with from 1-4 **R$^g$**.

47. The compound of any one of embodiments 1-46, wherein **R$^1$** is phenyl optionally substituted with from 1-2 **R$^g$**.

48. The compound of any one of embodiments 1-47, wherein **$R^1$** is unsubstituted phenyl.

49. The compound of any one of embodiments 1-45, wherein **$R^1$** is $C_{8-10}$ bicyclic aryl optionally substituted with from 1-4 **$R^g$**.

50. The compound of any one of embodiments 1-45 or 49, wherein **$R^1$** is $C_{9-10}$ bicyclic aryl optionally substituted with from 1-2 **$R^g$**.

51. The compound of any one of embodiments 1-45 or 49-50, wherein **$R^1$** is indanyl optionally substituted with from 1-2 **$R^g$**.

52. The compound of any one of embodiments 1-45 or 49-51, wherein **$R^1$** is

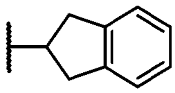,

which is optionally substituted with from 1-2 **$R^g$**.

**53.** The compound of embodiment 52, wherein **$R^1$** is

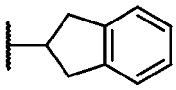.

**54.** The compound of any one of embodiments 1-44, wherein **$R^1$** is $C_{3-10}$ cycloalkyl or $C_{3-10}$ cycloalkenyl, each of which is optionally substituted with from 1-4 **$R^g$**.

55. The compound of any one of embodiments 1-44 or 54, wherein **$R^1$** is $C_{3-10}$ cycloalkyl which is optionally substituted with from 1-4 **$R^g$**.

56. The compound of any one of embodiments 1-44 or 54-55, wherein **$R^1$** is $C_{3-6}$ cycloalkyl which is optionally substituted with from 1-2 **$R^g$**.

57. The compound of any one of embodiments 1-44 or 54-56, wherein **$R^1$** is cyclobutyl or cyclopentyl, each of which is optionally substituted with from 1-2 **$R^g$**, such as cyclopentyl optionally substituted with from 1-2 **$R^g$**.

58. The compound of any one of embodiments 1-44 or 54-57, wherein **$R^1$** is unsubstituted cyclobutyl or cyclopentyl, such as unsubstituted cyclopentyl.

59. The compound of any one of embodiments 1-42, wherein **$R^1$** is $C_{2-6}$ alkynyl optionally substituted with from 1-6 **$R^a$**.

60. The compound of any one of embodiments 1-42 or 59, wherein **$R^1$** is $C_{2-4}$ alkynyl optionally substituted with from 1-3 **$R^a$**.

61. The compound of any one of embodiments 1-42 or 59-60, wherein **$R^1$** is

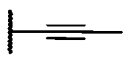.

**62.** The compound of any one of embodiments 1-42, wherein **$R^1$** is $C_{1-6}$ alkyl optionally substituted with from 1-6 **$R^2$**.

63. The compound of any one of embodiments 1-42 or 62, wherein **$R^1$** is $C_{1-6}$ alkyl.

64. The compound of anyone of embodiments 1-42 or 62-63, wherein **$R^1$** is $C_{2-4}$ alkyl.

65. The compound of any one of embodiments 1-42, or 62-64, wherein **$R^1$** is $C_3$ alkyl, such as *n*-propyl and *i*-propyl.

66. The compound of any one of embodiments 1-42, or 62-65, wherein $\mathbf{R^1}$ is *i*-propyl.

67. The compound of any one of embodiments 1-42 or 62-63, wherein $\mathbf{R^1}$ is $C_{3-5}$ alkyl.

68. The compound of any one of embodiments 1-42, 62-63 or 67, wherein $\mathbf{R^1}$ is $C_4$ alkyl, such as *n*-butyl, *i*-butyl, *sec*-butyl, and *tert*-butyl.

69. The compound of any one of embodiments 1-42, 62-63, 67 or 68, wherein $\mathbf{R^1}$ is *i*-butyl.

70. The compound of any one of embodiments 1-42, 62-69, wherein in $\mathbf{L^1}$ is a bond.

71. The compound of any one of embodiments 1-70, wherein $\mathbf{Ar^2}$ is heteroarylene including from 5-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents, each of which is independently selected from the group consisting of: $\mathbf{R^{c2}}$ and -$(\mathbf{L^b})_b$-$\mathbf{R^b}$ .

72. The compound of any one of embodiments 1-71, wherein $\mathbf{Ar^2}$ is heteroarylene including from 5-6 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents, each of which is independently selected from the group consisting of: $\mathbf{R^{c2}}$ and -$(\mathbf{L^b})_b$-$\mathbf{R^b}$ .

73. The compound of any one of embodiments 1-72, wherein $\mathbf{Ar^2}$ is heteroarylene including from 5 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents, each of which is independently selected from the group consisting of: $\mathbf{R^{c2}}$ and -$(\mathbf{L^b})_b$-$\mathbf{R^b}$ .

74. The compound of any one of embodiments 1-73, wherein $\mathbf{Ar^2}$ is selected from the group consisting of pyrrolylene, pyrazolylene, thiazolylene and 1,3,4-oxadiazolylene, each of which is optionally substituted with from 1-4 substituents, each of which is independently selected from the group consisting of: $\mathbf{R^{c2}}$ and -$(\mathbf{L^b})_b$-$\mathbf{R^b}$ .

75. The compound of any one of embodiments 1-73, wherein $\mathbf{Ar^2}$ is

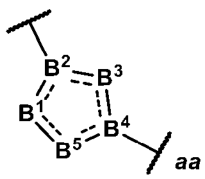
,

wherein:

each 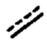 is independently a single bond or a double bond, provided that the ring including $\mathbf{B^1}$, $\mathbf{B^2}$, $\mathbf{B^3}$, $\mathbf{B^4}$, and $\mathbf{B^5}$ is heteroaryl;
***aa*** is the point of attachment to -(C$\mathbf{R^{3a}R^{3b}})_n$-$\mathbf{R^2}$;
$\mathbf{B^2}$ and $\mathbf{B^4}$ are C or N; and
$\mathbf{B^1}$, $\mathbf{B^3}$, and $\mathbf{B^5}$ are independently O, S, N, N(H), N($\mathbf{R^d}$), CH, C$\mathbf{R^{c2}}$, or C-$(\mathbf{L^b})_b$-$\mathbf{R^b}$, provided that:
from 1-4 of $\mathbf{B^1}$, $\mathbf{B^2}$, $\mathbf{B^3}$, $\mathbf{B^4}$, and $\mathbf{B^5}$ are independently selected heteroatoms.

76. The compound of embodiment 75, wherein $\mathbf{B^2}$ is N.

77. The compound of embodiments 75 or 76, wherein $\mathbf{B^4}$ is C.

78. The compound of any one of embodiments 75-77, wherein $\mathbf{B^1}$, $\mathbf{B^3}$, and $\mathbf{B^5}$ are independently CH, $\mathbf{CR^{c2}}$, or C-$(\mathbf{L^b})_b$-$\mathbf{R^b}$.

79. The compound of any one of embodiments 75-78, wherein $\mathbf{B^5}$ is C$\mathbf{R^{c2}}$; and $\mathbf{B^1}$ and $\mathbf{B^3}$ are CH.

80. The compound of embodiment 75, wherein $\mathbf{B^2}$ is N; $\mathbf{B^4}$ is C; and $\mathbf{B^1}$, $\mathbf{B^3}$, and $\mathbf{B^5}$ are independently CH, $\mathbf{CR^{c2}}$, or C-$\mathbf{(L^b)_b\text{-}R^b}$.

**81.** The compound of any one of embodiments 1-75, wherein $\mathbf{Ar^2}$ is

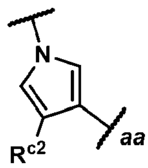

,

wherein ***aa*** is the point of attachment to -(C$\mathbf{R^{3a}R^{3b})_n\text{-}R^2}$.

82. The compound of embodiment 81, wherein $\mathbf{R^{c2}}$ is C(O)O$C_{1\text{-}4}$ alkyl, such as C(O)OMe.

83. The compound of embodiment 75, wherein $\mathbf{B^2}$ is C.

84. The compound of embodiment 83, wherein $\mathbf{B^4}$ is C.

85. The compound of any one of embodiments 75 or 83-84, wherein $\mathbf{B^5}$ is CH, C$\mathbf{R^{c2}}$, or C-$\mathbf{(L^b)_b\text{-}R^b}$; and one of $\mathbf{B^1}$ and $\mathbf{B^3}$ is N; and the other of $\mathbf{B^1}$ and $\mathbf{B^3}$ is NH, N($\mathbf{R^d}$), O, or S, such as S.

86. The compound of any one of embodiments 75 or 83-85, wherein $\mathbf{Ar^2}$ is selected from the list consisting of

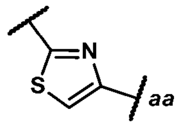

,

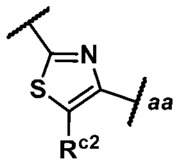

, and

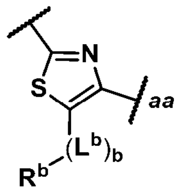

,

wherein ***aa*** is the point of attachment to -(C$\mathbf{R^{3a}R^{3b})_n\text{-}R2}$.

87. The compound of any one of embodiment 85-86, wherein $\mathbf{R^{c2}}$ is C(=O)$C_{1\text{-}4}$ alkyl, such as C(=O)Et, or C(=O)Me.

88. The compound any one of embodiment 85-86, wherein $\mathbf{R^{c2}}$ is $C_{1\text{-}6}$ alkyl, such as methyl, ethyl, propyl, such as *n*-propyl.

89. The compound any one of embodiment 85-86, wherein -$\mathbf{(L^b)_b\text{-}R^b}$ is $C_{3\text{-}10}$ cycloalkyl, such as cycloproyl.

90. The compound of any one of embodiments 75 or 83-84, wherein $\mathbf{Ar^2}$ is

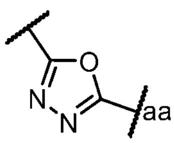

,

wherein ***aa*** is the point of attachment to -(C$\mathbf{R^{3a}R^{3b})_n\text{-}R^2}$.

91. The compound of embodiments 75 or 83, wherein $\mathbf{B^2}$ is C; and $\mathbf{B^4}$ is N.

92. The compound of any one of embodiments 75, 83, or 91, wherein $\mathbf{B^3}$ is N; and $\mathbf{B^1}$ and $\mathbf{B^5}$ are independently CH, C$\mathbf{R^{c2}}$, or C-$\mathbf{(L^b)_b\text{-}R^b}$.

93. The compound of any one of embodiments 75, 83, or 91-92, wherein $\mathbf{Ar^2}$ is

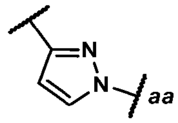,

wherein ***aa*** is the point of attachment to -(C$\mathbf{R^{3a}R^{3b}}$)$_n$-$\mathbf{^{R2}}$.

94. The compound of any one of embodiments 1-72, wherein $\mathbf{Ar^2}$ is heteroarylene including 6 ring atoms, wherein from 1-2 ring atoms are ring nitrogen atoms, wherein the heteroarylene is optionally substituted with from 1-4 substituents, each of which is independently selected from the group consisting of: $\mathbf{R^{c2}}$ and -$\mathbf{(L^b)_b}$-$\mathbf{R^b}$.

95. The compound of any one of embodiments 1-72 or 94, wherein $\mathbf{Ar^2}$ is pyridylene which is optionally substituted with from 1-4 substituents, each of which is independently selected from the group consisting of: $\mathbf{R^{c2}}$ and -$\mathbf{(L^b)_b}$-$\mathbf{R^b}$.

96. The compound of any one of embodiments 1-72 or 94-95, wherein $\mathbf{Ar^2}$ is

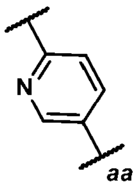 or 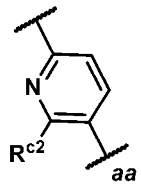,

wherein ***aa*** is the point of attachment to -(C$\mathbf{R^{3a}R^{3b}}$)$_n$-$\mathbf{^{R2}}$.

97. The compound of embodiment 96, wherein $\mathbf{R^{c2}}$ is $C_{1-4}$ alkoxy or $C_{1-4}$ haloalkoxy, such as -OMe.

98. The compound of any one of embodiments 1-71, wherein $\mathbf{Ar^2}$ is bicyclic heteroarylene including from 9-10 ring atoms, wherein from 1-4 ring atoms are ring heteroatoms each independently selected from the group consisting of: N, N(H), N($\mathbf{R^d}$), O, and S, wherein the heteroarylene is optionally substituted with from 1-4 substituents, each of which is independently selected from the group consisting of: $\mathbf{R^{c2}}$ and -$\mathbf{(L^b)_b}$-$\mathbf{R^b}$.

99. The compound of any one of embodiments 1-71 or 98, wherein $\mathbf{Ar^2}$ is selected from the group consisting of benzimidazolylene, indazolylene, benzothiazolylene, and imidazo[1,2-a]pyridylene (e.g. benzothiazolylene), each of which is optionally substituted with from 1-4 substituents, each of which is independently selected from the group consisting of: $\mathbf{R^{c2}}$ and -$\mathbf{(L^b)_b}$-$\mathbf{R^b}$.

100. The compound of any one of embodiments 1-71 or 98, wherein $\mathbf{Ar^2}$ is:

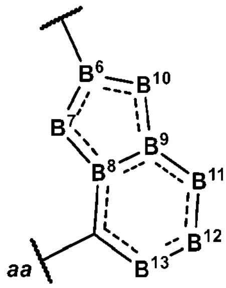,

wherein:

each 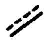 is independently a single bond or a double bond, provided that the 5-membered ring including $\mathbf{B^6}$, $\mathbf{B^7}$, $\mathbf{B^8}$, $\mathbf{B^9}$, and $\mathbf{B^{10}}$ is heteroaryl, and the 6-membered ring including $\mathbf{B^8}$, $\mathbf{B^9}$, $\mathbf{B^{11}}$, $\mathbf{B^{12}}$, and $\mathbf{B^{13}}$ is aryl or heteroaryl;
***aa*** is the point of attachment to -(C$\mathbf{R^{3a}R^{3b}}$)$_n$-$\mathbf{R^2}$;
$\mathbf{B^6}$, $\mathbf{B^8}$, and $\mathbf{B^9}$ are independently C or N;
$\mathbf{B^7}$ and $\mathbf{B^{10}}$ are independently selected from the group consisting of: O, S, N, N(H), N($\mathbf{R^d}$), CH, C$\mathbf{R^{c2}}$. and C-$\mathbf{(L^b)_b}$-

**R$^b$**;
**B$^{11}$**, **B$^{12}$**, and **B$^{13}$** are independently N, CH, C**R$^{c2}$**, or C-**(L$^b$)$_b$-R$^b$**, provided that:
from 1-4 of **B$^6$**, **B$^7$**, **B$^8$**, **B$^9$**, **B$^{10}$**, **B$^{11}$**, **B$^{12}$**, and **B$^{13}$** is an independently selected heteroatom; and no more than 3 of **B$^7$**, **B$^{10}$**, **B$^{11}$**, **B$^{12}$**, and **B$^{13}$** are **CR$^{c2}$**, or C-**(L$^b$)$_b$-R$^b$**.

101. The compound of embodiment 100, wherein **B$^8$** and **B$^9$** are C.

102. The compound of embodiments 100 or 101, wherein **B$^{11}$**, **B$^{12}$**, and **B$^{13}$** are independently CH, C**R$^{c2}$**, or C-**(L$^b$)$_b$-R$^b$**.

103. The compound of any one of embodiments 100-102, wherein **B$^{11}$**, **B$^{12}$**, and **B$^{13}$** are CH.

104. The compound of any one of embodiments 100-103, wherein **B$^6$** is N.

105. The compound of any one of embodiments 100-104, wherein **B$^7$** is N.

106. The compound of any one of embodiments 100-105, wherein **B$^{10}$** is CH or C**R$^{c2}$**, or C-**(L$^b$)$_b$-R$^b$**.

107. The compound of any one of embodiments 100-106, wherein **B$^{10}$** is CH.

108. The compound of any one of embodiments 1-71 or 100-107, wherein **Ar$^2$** is

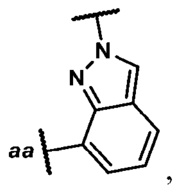

which is optionally substituted with from 1-2 substituents, each of which is independently selected from the group consisting of: **R$^{c2}$** and -(**L$^b$**)$_b$-**R$^b$** , wherein ***aa*** is the point of attachment to -(C**R$^{3a}$R$^{3b}$**)$_n$-**R$^2$**.

109. The compound of any one of embodiments 100-101, wherein **B$^7$** is N.

110. The compound of any one of embodiments 100-101 or 109, wherein **B$^{10}$** is S.

111. The compound of any one of embodiments 100-101 or 109-110, wherein **Ar$^2$** is

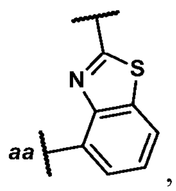

which is optionally substituted with from 1-2 substituents, each of which is independently selected from the group consisting of: **R$^{c2}$** and -**(L$^b$)$_b$-R$^b$**, wherein ***aa*** is the point of attachment to -(C**R$^{3a}$R$^{3b}$**)$_n$-**R$^2$**.

112. The compound of embodiment 100, wherein **B$^8$** is C and **B$^9$** is N.

113. The compound of any one of embodiments 100 or 112, wherein **B$^7$** is N.

114. The compound of any one of embodiments 100 or 112-113, wherein **B$^{10}$**, **B$^{11}$**, **B$^{12}$**, and **B$^{13}$** are CH, C**R$^{c2}$**, or C-**(L$^b$)$_b$-R$^b$**.

115. The compound of any one of embodiments 100 or 112-114, wherein **Ar$^2$** is

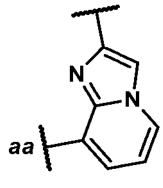,

which is optionally substituted with from 1-2 substituents, each of which is independently selected from the group consisting of: $\mathbf{R^{c2}}$ and $-\mathbf{(L^b)_b\text{-}R^b}$, wherein ***aa*** is the point of attachment to $-(C\mathbf{R^{3a}R^{3b}})_n\text{-}\mathbf{R^2}$.

**116.** The compound of embodiment 100, wherein $\mathbf{Ar^2}$ is selected from the group consisting of:

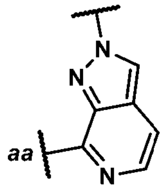, 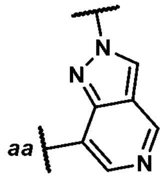 and 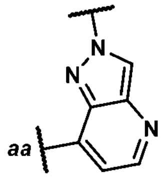,

each of which is optionally substituted with from 1-2 substituents, each of which is independently selected from the group consisting of: $\mathbf{R^{c2}}$ and $-\mathbf{(L^b)_b\text{-}R^b}$, wherein ***aa*** is the point of attachment to $-(C\mathbf{R^{3a}R^{3b}})_n\text{-}\mathbf{R^2}$.

117. The compound of embodiment 100, wherein $\mathbf{Ar^2}$ is selected from the group consisting of:

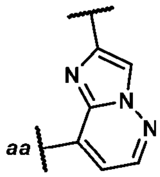, 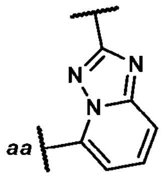, and 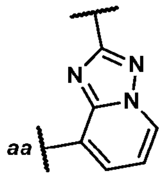,

each of which is optionally substituted with from 1-2 substituents, each of which is independently selected from the group consisting of: $\mathbf{R^{c2}}$ and $-(\mathbf{L^b})_\mathbf{b}\text{-}\mathbf{R^b}$, wherein ***aa*** is the point of attachment to $-(C\mathbf{R^{3a}R^{3b}})_n\text{-}\mathbf{R^2}$.

**118.** The compound of embodiment 100, wherein $\mathbf{Ar^2}$ is selected from the group consisting of

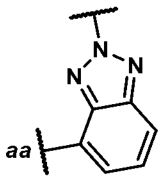, 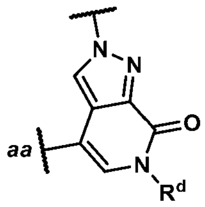, 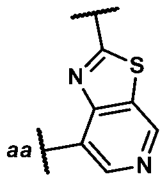, 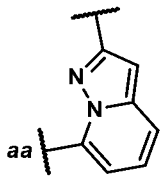, 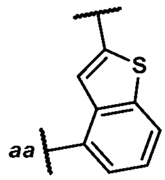,

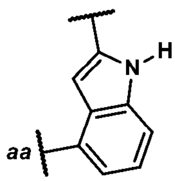 and 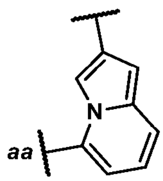,

each of which is optionally substituted with from 1-2 substituents, each of which is independently selected from the group consisting of: $\mathbf{R^{c2}}$ and - $\mathbf{(L^b)_n\text{-}R^b}$, wherein ***aa*** is the point of attachment to $-(C\mathbf{R^{3a}R^{3b}})_n\text{-}\mathbf{R^2}$.

119. The compound of any one of embodiments 1-71 or 98, wherein $\mathbf{Ar^2}$ is:

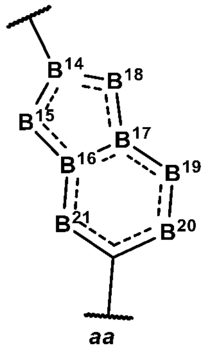

,

wherein:

each 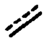 is independently a single bond or a double bond, provided that the 5-membered ring including **B14, B15**, **B16**, **B17**, and **B18** is heteroaryl, and the 6-membered ring including **B16**, **B17**, **B19**, **B20**, and **B21** is aryl or heteroaryl;
***aa*** is the point of attachment to -(C$\mathbf{R^{3a}R^{3b}}$)$_n$-$\mathbf{R^2}$;
**B16**, **B17**, and **B14** are independently C or N;
**B15** and **B18** are independently selected from the group consisting of: O, S, N, N(H), N($\mathbf{R^d}$), CH, and C$\mathbf{R^{c2}}$;
**B19**, **B20**, and **B21** are independently N, CH, C$\mathbf{R^{c2}}$, or C-$\mathbf{(L^b)_b}$-$\mathbf{R^b}$, provided that:
from 1-4 of **B14, B15**, **B16**, **B17**, **B18**, **B19**, **B20**, and **B21** is an independently selected heteroatom; and no more than 3 of **B15**, **B18**, **B19**, **B20**, and **B21** are **C**$\mathbf{R^{c2}}$**.** or C-$\mathbf{(L^b)_b}$-$\mathbf{R^b}$.

120. The compound of embodiment 119, wherein **B16** and **B17** are C.

121. The compound of embodiments 119 or 120, wherein **B19, B20,** and **B21** are independently N, CH, C$\mathbf{R^{c2}}$**,** or or C-($\mathbf{L}^b$)$_b$-$\mathbf{R}^b$.

122. The compound of any one of embodiments 119-121, wherein **B14** is C.

123. The compound of any one of embodiments 119-122, wherein one of **B15** and **B18** is N; and the other of **B15** and **B13** is O, S, NH, or N($\mathbf{R^d}$), such as NH or N($\mathbf{R^d}$), such as NH or N($C_{1-3}$ alkyl).

124. The compound of any one of embodiments 1-71, or 98, wherein $\mathbf{Ar^2}$ is

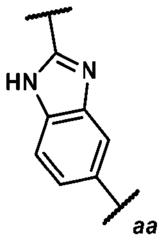

or

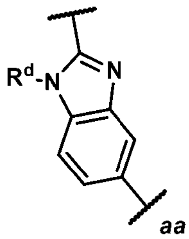

,

each of which is optionally substituted with from 1-2 $\mathbf{R^{c2}}$, wherein ***aa*** is the point of attachment to -(C$\mathbf{R^{3a}R^{3b}}$)$_n$-$\mathbf{R^2}$.

125. The compound of any one of embodiments 1-71 or 98, wherein Ar$\mathbf{^2}$ is

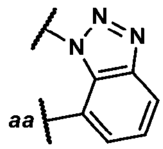

,

which is optionally substituted with from 1-2 substituents, each of which is independently selected from the group

consisting of: $R^{c2}$ and **-(L^b)_b-R^b**, wherein ***aa*** is the point of attachment to $((CR^{3a}R^{3b})_n$-$R^2$,

126. The compound of any one of embodiments 1-125, wherein **n** is 0.

127. The compound of any one of embodiments 1-125, wherein **n** is 1, and optionally wherein $R^{3a}$ and $R^{3b}$ are H.

128. The compound of any one of embodiments 1-125, wherein **n** is 1, and wherein one of $R^{3a}$ and $R^{3b}$ is H, and the other one of $R^{3a}$ and $R^{3b}$ is $C_{1-6}$ alkyl, such as ethyl or methyl.

**129.** The compound of any one of embodiments 1-125, wherein n is 1, and wherein $R^{3a}$ and $R^{3b}$ together with the carbon atom to which each is attached forms a $C_{3-6}$ cycloalkyl, such as a cyclopropyl.

130. The compound of any one of embodiments 1-129, wherein $R^2$ is -C(=O)OH.

131. The compound of any one of embodiments 1-129, wherein $R^2$ is carboxylic acid bioisostere.

132. The compound of any one of embodiments 1-129 or 131, wherein $R^2$ is - $C(=O)NH_2$, $-C(=O)NHSO_2Me$ or

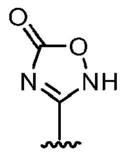

133. The compound of embodiment 1, wherein the compound is a compound of Formula **(I-1)**:

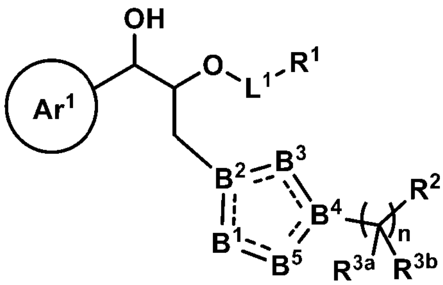

Formula (**I-1**)

or a pharmaceutically acceptable salt thereof, wherein:

each 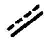 is independently a single bond or a double bond, provided that the ring including $B^1$, $B^2$, $B^3$, $B^4$, and $B^5$ is heteroaryl;
$B^2$ and $B^4$ are C or N;
$B^1$, $B^3$, and $B^5$ are independently O, S, N, N(H), N($R^d$), CH, C$R^{c2}$, or C-$(L^b)_b$-$R^b$, provided that: from 1-4 of $B^1$, $B^2$, $B^3$, $B^4$, and $B^5$ are independently selected heteroatoms; and
**Ar¹** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$.

134. The compound of embodiment 133, wherein $B^2$ is N; and $B^4$ is C.

135. The compound of embodiments 133 or 134, wherein **B**$^1$**, B**$^3$**,** and **B**$^5$ are independently CH, C**R**$^{c2}$ or C-**(L**$^b$**)**$_b$**-R**$^b$**;**

136. The compound of any one of embodiments 133-135, wherein the ring including **B**$^1$**-B**$^5$ is is

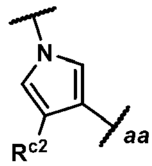

,

wherein ***aa*** is the point of attachment to -(C**R**$^{3a}$**R**$^{3b}$)$_n$**-R**$^2$**,** optionally wherein **R**$^{c2}$ is C(O)OC$_{1-4}$ alkyl.

137. The compound of embodiment 133, wherein **B**$^2$ is C; and **B**$^4$ is N.

138. The compound of embodiments 133 or 137, wherein **B**$^3$ is N; and **B**$^1$ and **B**$^5$ are independently CH, C**R**$^{c2}$ or C-(**L**$^b$)$_b$-**R**$^b$.

139. The compound of any one of embodiments 133 or 137-138, wherein the ring including **B**$^1$**-B**$^5$ is

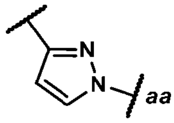

,

wherein ***aa*** is the point of attachment to -(C**R**$^{3a}$**R**$^{3b}$)$_n$-**R**$^2$.

**140.** The compound of embodiment 133, wherein **B**$^2$ is C; and **B**$^4$ is C.

141. The compound of embodiments 133 or 140, wherein **B**$^5$ is CH or C**R**$^{c2}$**;** one of **B**$^1$ and **B**$^3$ is N; and the other of **B**$^1$ and **B**$^3$ is NH, N(**R**$^d$), O, or S, such as S.

**142.** The compound of any one of embodiments 133 or 140-141, wherein the ring including **B**$^1$**-B**$^5$ is

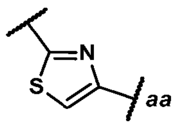

,

wherein ***aa*** is the point of attachment to -(C**R**$^{3a}$**R**$^{3b}$)$_n$-**R**$^2$.

**143.** The compound of any one of embodiments 133 or 140-141, wherein the ring including **B**$^1$**-B**$^5$ is

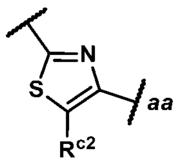

,

wherein ***aa*** is the point of attachment to -(C**R**$^{3a}$**R**$^{3b}$)$_n$**-R**$^2$, optionally wherein **R**$^{c2}$ is C(O)OC$_{1-4}$ alkyl, C$_{1-4}$ alkyl, or C$_{1-4}$ alkoxy or C$_{1-4}$ haloalkoxy.

144. The compound of any one of embodiments 133 or 140-141, wherein the ring including **B**$^1$**-B**$^5$ is ,

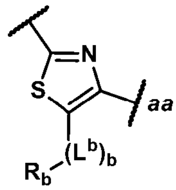

,

wherein ***aa*** is the point of attachment to -(C**R$^{3a}$R$^{3b}$**)$_n$-**R$^2$**, optionally wherein -(**L$^b$**)$_b$-**R$^b$** is $C_{3-10}$ cycloalkyl, such as cyclopropyl.

145. The compound of any one of embodiments 133 or 140-141, wherein the ring including **B$^1$-B$^5$** is

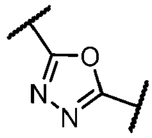

,

wherein ***aa*** is the point of attachment to -(C**R$^{3a}$R$^{3b}$**)$_n$-**R$^2$**.

**146.** The compound of embodiment 1, wherein the compound is a compound of Formula **(I-2):**

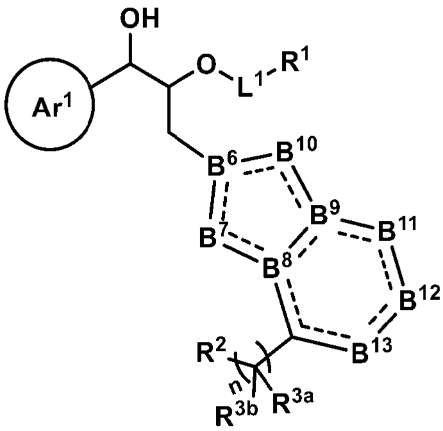

Formula **(I-2)**

or a pharmaceutically acceptable salt thereof, wherein:

each 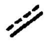 is independently a single bond or a double bond, provided that the 5-membered ring including **B$^6$, B$^7$, B$^8$, B$^9$**, and **B$^{10}$** is heteroaryl, and the 6-membered ring including **B$^8$, B$^9$, B$^{11}$, B$^{12}$,** and **B$^{13}$** is aryl or heteroaryl;
**B$^6$, B$^8$,** and **B$^9$** are independently C or N;
**B$^7$** and **B$^{10}$** are independently selected from the group consisting of: O, S, N, N(H), N(**R$^d$**), CH, C**R$^{c2}$,** or C-**(L$^b$)$_b$-R$^b$;**
**B$^{11}$, B$^{12}$,** and **B$^{13}$** are independently N, CH, C**R$^{c2}$,** or C-**(L$^b$)$_b$-R$^b$,** provided that:

from 1-4 of **B$^6$, B$^7$, B$^8$, B$^9$, B$^{10}$, B$^{11}$, B$^{12}$,** and **B$^{13}$** is an independently selected heteroatom; and no more than 3 of **B$^7$, B$^{10}$, B$^{11}$, B$^{12}$,** and **B$^{13}$** are C**R$^{c2}$** or C-**(L$^b$)$_b$-R$^b$;**

**Ar$^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R$^g$;** and
heteroaryl including 6 ring atoms, wherein from 1-2 ring atoms are ring nitrogen atoms, wherein the

heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **$R^g$.**

147. The compound of embodiment 146, wherein **$B^8$** and **$B^9$** are C.

148. The compound of embodiments 146 or 147, wherein **$B^{11}$, $B^{12}$,** and **$B^{13}$** are independently CH or C**$R^{c2}$.**

149. The compound of any one of embodiments 146-148, wherein **$B^6$** is N; **$B^7$** is N; and **$B^{10}$** is CH or C**$R^{c2}$.**

**150.** The compound of any one of embodiments 146-149, wherein the ring including **$B^6$-$B^{13}$** is

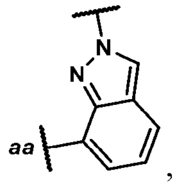

which is optionally substituted with from 1-2 **$R^{c2}$**, wherein ***aa*** is the point of attachment to -(C**$R^{3a}$$R^{3b}$**)$_n$-**$R^2$**.

151. The compound of any one of embodiments 146-147, wherein **$B^7$** is N.

152. The compound of any one of embodiments 146-147 or 151, wherein **$B^{10}$** is S.

153. The compound of any one of embodiments 146-147 or 151-152, wherein the ring including **$B^6$-$B^{13}$** is

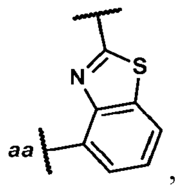

which is optionally substituted with from 1-2 substituents, each of which is independently selected from the group consisting of: **$R^{c2}$** and - (**$L^b$**)$_b$-**$R^b$,** wherein ***aa*** is the point of attachment to -(C**$R^{3a}$$R^{3b}$**)$_n$-**$R^2$**.

154. The compound of embodiment 146, wherein **$B^8$** is C; and **$B^9$** is N.

155. The compound of any one of embodiments146 or 154, wherein B7 is N.

156. The compound of any one of embodiments 146 or 154-155, wherein **$B^{10}$, $B^{11}$, $B^{12}$,** and **$B^{13}$** are CH, C**$R^{c2}$,** or C-(**$L^b$**)$_b$-**$R^b$**.

157. The compound of any one of embodiments 146 or 154-156, wherein the ring including **$B^6$-$B^{13}$** is

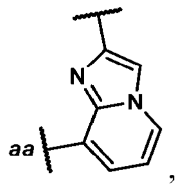

which is optionally substituted with from 1-2 substituents, each of which is independently selected from the group consisting of: **$R^{c2}$** and -(**$L^b$**)$_b$-**$R^b$,** wherein ***aa*** is the point of attachment to -(C**$R^{3a}$$R^{3b}$**)$_n$-**$R^2$**.

**158.** The compound of embodiment 146, wherein the ring including **$B^6$-$B^{13}$** is selected from the group consisting of

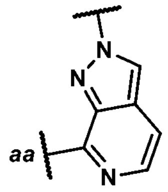, 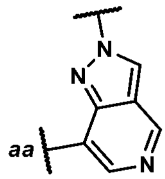 and 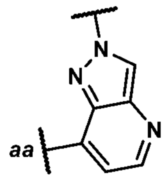,

each of which is optionally substituted with from 1-2 substituents, each of which is independently selected from the group consisting of: $\mathbf{R^{c2}}$ and -($\mathbf{L^b}$)$_\mathbf{b}$-$\mathbf{R^b}$, wherein ***aa*** is the point of attachment to -(C$\mathbf{R^{3a}R^{3b}}$)$_n$-$\mathbf{R^2}$.

159. The compound of embodiment 146, wherein the ring including $\mathbf{B^6}$-$\mathbf{B^{13}}$ is selected from the group consisting of

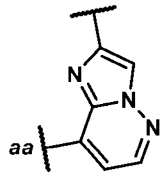, 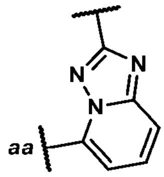, and 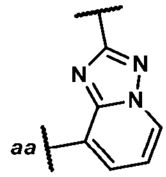,

each of which is optionally substituted with from 1-2 substituents, each of which is independently selected from the group consisting of: $\mathbf{R^{c2}}$ and -($\mathbf{L^b}$)$_\mathbf{b}$-$\mathbf{R^b}$, wherein ***aa*** is the point of attachment to -(C$\mathbf{R^{3a}R^{3b}}$)$_n$-$\mathbf{R^2}$.

160. The compound of embodiment 146, wherein the ring including $\mathbf{B^6}$-$\mathbf{B^{13}}$ is selected from the group consisting of

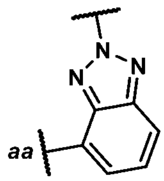, 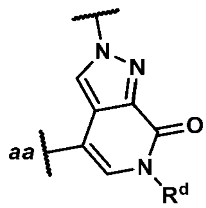, 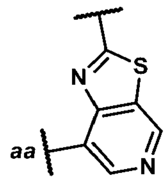,

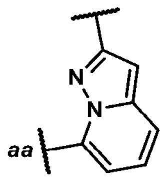, 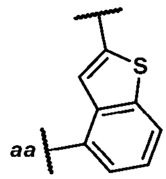, 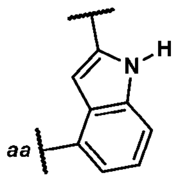 and 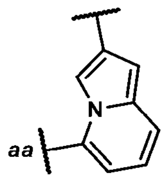,

each of which is optionally substituted with from 1-2 substituents, each of which is independently selected from the group consisting of: $\mathbf{R^{c2}}$ and -($\mathbf{L^b}$)$_\mathbf{b}$-$\mathbf{R^b}$, wherein ***aa*** is the point of attachment to -(C$\mathbf{R^{3a}R^{3b}}$)$_n$-$\mathbf{R^2}$.

161. The compound of embodiment 1, wherein the compound is a compound of Formula **(I-3):**

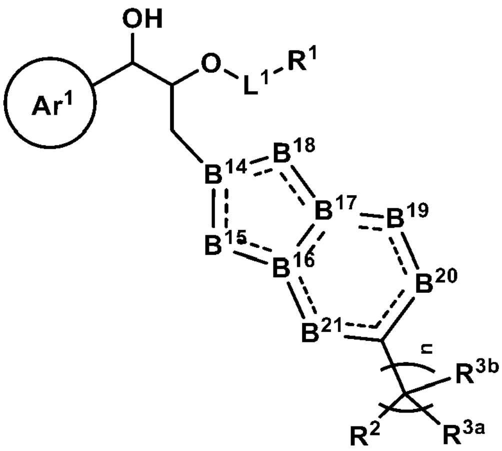

Formula (**I-3**)

or a pharmaceutically acceptable salt thereof, wherein:

each 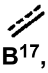 is independently a single bond or a double bond, provided that the 5-membered ring including $\mathbf{B^{14}}$, $\mathbf{B^{15}}$, $\mathbf{B^{16}}$, $\mathbf{B^{17}}$, and $\mathbf{B^{18}}$ is heteroaryl, and the 6-membered ring including $\mathbf{B^{16}}$, $\mathbf{B^{17}}$, $\mathbf{B^{19}}$, $\mathbf{B^{20}}$, and $\mathbf{B^{21}}$ is aryl or heteroaryl;

$\mathbf{B^{16}}$, $\mathbf{B^{17}}$, and $\mathbf{B^{14}}$ are independently C or N;

$\mathbf{B}^{15}$ and $\mathbf{B^{18}}$ are independently selected from the group consisting of: O, S, N, N(H), N($\mathbf{R^d}$), CH, C$\mathbf{R^{c2}}$, and C-($\mathbf{L^b}$)$_b$-$\mathbf{R}^b$.

$\mathbf{B^{19}}$, $\mathbf{B^{20}}$, and $\mathbf{B^{21}}$ are independently N, CH, C$\mathbf{R^{c2}}$, or C-$\mathbf{(L^b)_b}$-$\mathbf{R^b}$, provided that:

from 1-4 of $\mathbf{B^{14}}$, $\mathbf{B^{15}}$, $\mathbf{B^{16}}$, $\mathbf{B^{17}}$, $\mathbf{B^{18}}$, $\mathbf{B^{19}}$, $\mathbf{B^{20}}$, and $\mathbf{B^{21}}$ is an independently selected heteroatom; and no more than 3 of $\mathbf{B^{15}}$, $\mathbf{B^{18}}$, $\mathbf{B^{19}}$, $\mathbf{B^{20}}$, and $\mathbf{B^{21}}$ are C$\mathbf{R^{c2}}$ or C-$\mathbf{(L^b)_n}$-$\mathbf{R^b}$; and

$\mathbf{Ar^1}$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

162. The compound of embodiment 161, wherein $\mathbf{B^{16}}$ and $\mathbf{B^{17}}$ are C.

163. The compound of embodiments 161 or 162, wherein $\mathbf{B^{19}}$, $\mathbf{B^{20}}$, and $\mathbf{B^{21}}$ are independently CH, C$\mathbf{R^{c2}}$, or C-($\mathbf{L}^b$)$_b$-$\mathbf{R}^b$.

164. The compound of any one of embodiments 161-163, wherein $\mathbf{B^{14}}$ is C; one of $\mathbf{B}^{15}$ and $\mathbf{B^{18}}$ is N; and the other of $\mathbf{B^{15}}$ and $\mathbf{B^{18}}$ is O, S, NH, or N($\mathbf{R^d}$), such as NH or N($\mathbf{R^d}$), such as NH or N($C_{1-3}$ alkyl).

165. The compound of any one of embodiments 161-164, wherein the ring including $\mathbf{B^{14}}$-$\mathbf{B^{21}}$ is

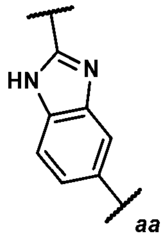 or 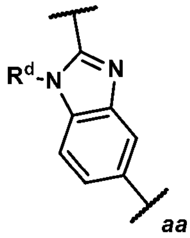 ,

each of which is optionally substituted with from 1-2 $\mathbf{R^{c2}}$, wherein ***aa*** is the point of attachment to -(C$\mathbf{R^{3a}R^{3b}}$)$_n$-$\mathbf{R^2}$.

166. The compound of any one of embodiments 133-165, wherein n is 1, and optionally wherein $\mathbf{R^{3a}}$ and $\mathbf{R^{3b}}$ are H.

167. The compound of any one of embodiments 133-165, wherein n is 1, and wherein one of $\mathbf{R^{3a}}$ and $\mathbf{R^{3b}}$ is H, and the other one of $\mathbf{R^{3a}}$ and $\mathbf{R^{3b}}$ is $C_{1-6}$ alkyl, such as ethyl or methyl.

168. The compound of any one of embodiments 133-165, wherein n is 1, and wherein $\mathbf{R^{3a}}$ and $\mathbf{R^{3b}}$ together with the carbon atom to which each is attached forms a $C_{3-6}$ cycloalkyl, such as a cyclopropyl.

169. The compound of any one of embodiments 133-165, wherein n is 0.

170. The compound of any one of embodiments 133-169, wherein $\mathbf{Ar^1}$ is

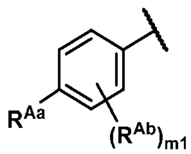 ;

**m1** is 0, 1, 2, or 3; and each occurrence of $\mathbf{R^{Aa}}$ and $\mathbf{R^{Ab}}$ are independently selected from the group consisting of: halo; -C(=O)($C_{1-10}$ alkyl); $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$.

171. The compound of any one of embodiments 133-170, wherein **m1** is 0.

172. The compound of any one of embodiments 133-170, wherein **m1** is 1 or 2, optionally wherein each $\mathbf{R^{Ab}}$ is *ortho* to $\mathbf{R^{Aa}}$.

173. The compound of any one of embodiments 133-170 or 172, wherein each $\mathbf{R^{Ab}}$ when present is $C_{1-4}$ alkoxy or $C_{1-4}$ haloalkoxy, such as $C_{1-4}$ alkoxy, such as methoxy.

174. The compound of any one of embodiments 133-170 or 172-173, wherein $\mathbf{R^{Aa}}$ is $C_{1-3}$ alkyl; $C_{1-3}$ alkyl substituted with 1-6 F; halo; or $C_{3-6}$ cycloalkyl.

175. The compound of any one of embodiments 133-170 or 172-173, wherein $\mathbf{Ar^1}$ is

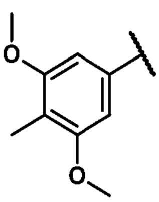 .

**176.** The compound of any one of embodiments 133-170 or 172-173, wherein $\mathbf{Ar^1}$ is selected from the group consisting of:

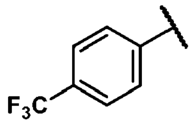; 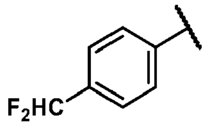; 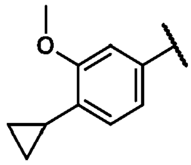;

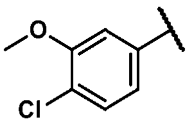; 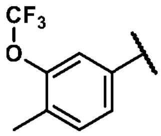; 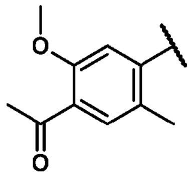; 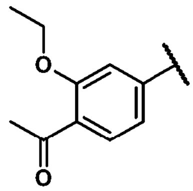; 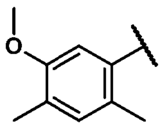; 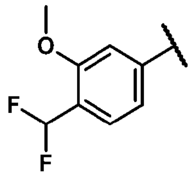;

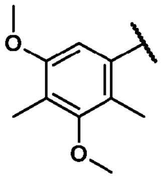; 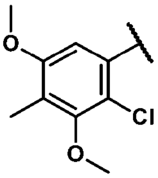; 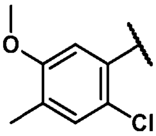; 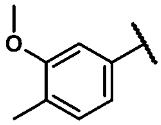; 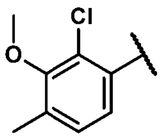; 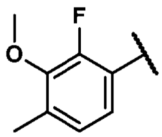;

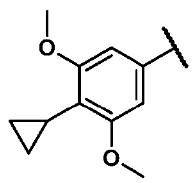; 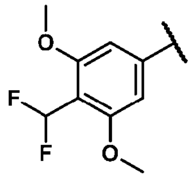; 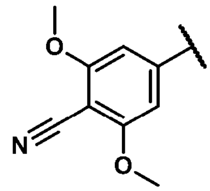; 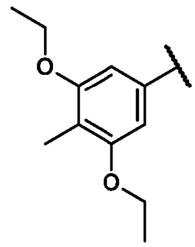; 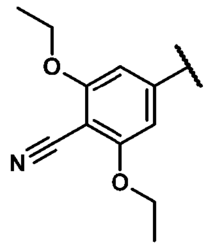;

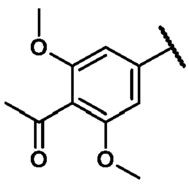; 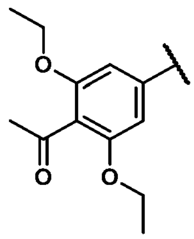; 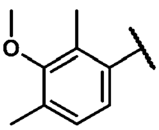; and 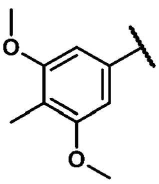.

**177.** The compound of any one of embodiments 133-176, wherein **$L^1$** is a bond.

178. The compound of any one of embodiments 133-176, wherein **$L^1$** is $C_{1-3}$ alkylene optionally substituted with from 1-6 **$R^a$.**

179. The compound of any one of embodiments 133-176 or 176, wherein **$L^1$** is $CH_2$ or $CH_2CH_2$.

180. The compound of any one of embodiments 133-179, wherein **$R^1$** is phenyl optionally substituted with from 1-2 **$R^g$.**

181. The compound of any one of embodiments 133-179, wherein **$R^1$** is $C_{9-10}$ bicyclic aryl optionally substituted with from 1-2 **$R^g$.**

182. The compound of any one of embodiments 133-179 or 181, wherein **$R^1$** is

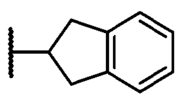,

which is optionally substituted with from 1-2 **R$^g$.**

**183.** The compound of any one of embodiments 133-179, wherein **R$^1$** is $C_{3-6}$ cycloalkyl which is optionally substituted with from 1-2 **R$^g$.**

184. The compound of any one of embodiments 133-179 or 183, wherein **R$^1$** is cyclobutyl or cyclopentyl, each of which is optionally substituted with from 1-2 **R$^g$.**

**185.** The compound of any one of embodiments 133-179, wherein **R$^1$** is $C_{1-6}$ alkyl optionally substituted with from 1-6 **R$^a$.**

186. The compound of any one of embodiments 133-179 or 185, wherein **R$^1$** is $C_{1-6}$ alkyl.

187. The compound of anyone of embodiments 133-179 or 185-186, wherein **R$^1$** is $C_{2-4}$ alkyl.

188. The compound of any one of embodiments 133-179 or 185-187, wherein **R$^1$** is $C_3$ alkyl, such as n-propyl and i-propyl.

189. The compound of any one of embodiments 133-179 or 185-188, wherein **R$^1$** is i-propyl.

190. The compound of any one of embodiments 133-179 or 185-186, wherein **R$^1$** is $C_{3-5}$ alkyl.

191. The compound of any one of embodiments 133-179, 185-186 or 190, wherein **R$^1$** is $C_4$ alkyl, such as n-butyl, i-butyl, sec-butyl, and tert-butyl.

192. The compound of any one of embodiments 133-179, 185-186 or 190-191, wherein **R$^1$** is i-butyl.

193. The compound of any one of embodiments 133-179, 185-192, wherein in **L$^1$** is a bond.

194. The compound of any one of embodiments 133-193, wherein the

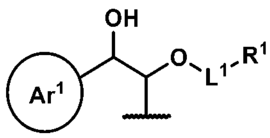

moiety has the following formula:

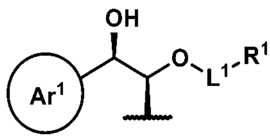

.

**195.** The compound of embodiment 1, wherein the compound is selected from the group consisting of compounds delineated in **Tables C1** and **C2,** or a pharmaceutically acceptable salt thereof.

196. The compound of embodiment 1, wherein the compound is selected from the group consisting of compounds 101-437 and 101a-131a, or a pharmaceutically acceptable salt thereof.

197. A pharmaceutical composition comprising a compound of any one of embodiments 1-196, or a pharmaceutically acceptable salt or solvate thereof, and a pharmaceutically acceptable excipient.

198. A method for treating or preventing an LPA-associated disease in a subject in need thereof, the method comprising administering to subject a therapeutically effective amount of a compound of any one of embodiments 1-196, or a pharmaceutically acceptable salt or solvate thereof, or a pharmaceutical composition of embodiment 143.

199. The method of embodiment 198, wherein the LPA-associated disease is an $LPA_1$-associated disease.

200. The method of any one of embodiment 198 or 199, wherein the LPA-associated disease is selected from the group consisting of fibrosis, transplant rejection, cancer, osteoporosis, or inflammatory disorders.

201. The method of embodiment 200, wherein the fibrosis is pulmonary, liver, renal, cardiac, dermal, ocular, or pancreatic fibrosis.

202. The method of any one of embodiments 198 or 199, wherein the LPA-associated disease is selected from the group consisting of idiopathic pulmonary fibrosis (IPF), non-alcoholic steatohepatitis (NASH), non-alcoholic fatty liver disease (NAFLD), chronic kidney disease, diabetic kidney disease, and systemic sclerosis.

**203.** The method of embodiment 200, wherein the cancer is of the bladder, blood, bone, brain, breast, central nervous system, cervix, colon, endometrium, esophagus, gall bladder, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid.

204. A method for treating or preventing fibrosis in a subject in need thereof, the method comprising administering to subject a therapeutically effective amount of a compound of any one of embodiments 1-196, or a pharmaceutically acceptable salt or solvate thereof, or a pharmaceutical composition of embodiment 197.

205. The method of embodiment 204, wherein the fibrosis is idiopathic pulmonary fibrosis (IPF), nonalcoholic steatohepatitis (NASH), chronic kidney disease, diabetic kidney disease, and systemic sclerosis.

206. The method of embodiment 205, wherein the fibrosis is IPF.

## Claims

**1.** A compound of Formula (I):

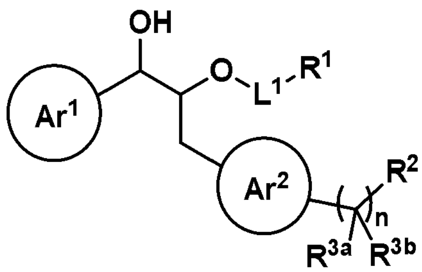

Formula (**I**)

or a pharmaceutically acceptable salt thereof, wherein:

**$L^1$** is selected from the group consisting of:

- a bond; and
- $C_{1-6}$ alkylene optionally substituted with from 1-6 **$R^a$;**

**$R^1$** is selected from the group consisting of: **$R^b$**; $C_{1-6}$ alkyl optionally substituted with from 1-6 **$R^a$;** $C_{2-6}$ alkenyl optionally substituted with from 1-6 **$R^a$;** and $C_{2-6}$ alkynyl optionally substituted with from 1-6 **$R^a$;**
**$Ar^1$** is selected from the group consisting of:

- $C_{6-10}$ aryl optionally substituted with from 1-4 substituents each independently selected from the group consisting of: **$R^{c1}$** and -(**$L^b$**)$_b$-**$R^b$;** and
- heteroaryl including from 5-10 ring atoms, wherein from 1-4 ring atoms are ring heteroatoms each independently selected from the group consisting of: N, N(H), N(**$R^d$**), O, and S, wherein the heteroaryl is optionally substituted with from 1-4 substituents each independently selected from the group consisting of: **$R^{c1}$** and -(**$L^b$**)**b-$R^b$.**

**$Ar^2$** is selected from the group consisting of:

- $C_{6-10}$ arylene optionally substituted with from 1-4 substituents each independently selected from the group consisting of: **$R^{c2}$** and -**$(L^b)_b$-$R^b$;** and
- heteroarylene including from 5-10 ring atoms, wherein from 1-4 ring atoms are ring heteroatoms each independently selected from the group consisting of: N, N(H), N(**$R^d$**), O, and S, wherein the heteroarylene is optionally substituted with from 1-4 substituents each independently selected from the group consisting of: **$R^{c2}$** and - **$(L^b)_b$-$R^b$;**

**$R^2$** is -C(=O)OH or carboxylic acid bioisostere;
**n** is 0 or 1;
**$R^{3a}$** and **$R^{3b}$** are independently H, -halo, $C_{1-6}$ alkyl, or $C_{1-4}$ haloalkyl; or
**$R^{3a}$** and **$R^{3b}$** taken together with the carbon atom to which each is attached forms a $C_{3-6}$ cycloalkyl;
each occurrence of **$R^a$** is independently selected from the group consisting of: -OH; -halo; -N**$R^eR^f$;** $C_{1-4}$ alkoxy; $C_{1-4}$ haloalkoxy; -C(=O)O($C_{1-4}$ alkyl); -C(=O)($C_{1-4}$ alkyl); -C(=O)OH; -CON**R'R"**; -$S(O)_{1-2}$N**R'R";** -$S(O)_{1-2}$($C_{1-4}$ alkyl); and cyano;
each occurrence of **$R^b$** is independently selected from the group consisting of:

- $C_{3-10}$ cycloalkyl or $C_{3-10}$ cycloalkenyl, each of which is optionally substituted with from 1-4 **$R^g$;**
- heterocyclyl or heterocycloalkenyl including from 3-10 ring atoms, wherein from 1-3 ring atoms are heteroatoms, each independently selected from the group consisting of N, N(H), N(**$R^d$**), O, and $S(O)_{0-2}$, and wherein the heterocyclyl or heterocycloalkenyl is optionally substituted with from 1-4 **$R^g$;**
- heteroaryl including from 5-10 ring atoms, wherein from 1-4 ring atoms are heteroatoms, each independently selected from the group consisting of N, N(H), N(**$R^d$**), O, and $S(O)_{0-2}$, and wherein the heteroaryl is optionally substituted with from 1-4 **$R^g$;** and
- $C_{6-10}$ aryl optionally substituted with from 1-4 **$R^g$;**

**b** is 0, 1, 2, or 3;
each occurrence of **$L^b$** is selected from the group consisting of: $C_{1-3}$ alkylene; - N(H)-; N(**$R^d$**)-; -O-; -S-; C(=O); and $S(O)_{1-2}$;
each occurrence of **$R^{c1}$** and **$R^{c2}$** is independently selected from the group consisting of: halo; cyano; $C_{1-10}$ alkyl which is optionally substituted with from 1-6 independently selected **$R^a$;** $C_{2-6}$ alkenyl; $C_{2-6}$ alkynyl; $C_{1-4}$ alkoxy; $C_{1-4}$ haloalkoxy; - $S(O)_{0-2}$($C_{1-4}$ alkyl); -N**$R^eR^f$;** -OH; -$S(O)_{1-2}$N**R'R";** -$NO_2$; -C(=O)($C_{1-10}$ alkyl); - C(=O)O($C_{1-4}$ alkyl); -C(=O)OH; and -C(=O)N**R'R"**;
each occurrence of **$R^d$** is independently selected from the group consisting of: $C_{1-6}$ alkyl optionally substituted with from 1-3 independently selected **$R^a$;** -C(O)($C_{1-4}$ alkyl); -C(O)O($C_{1-4}$ alkyl); -CON**R'R"**; -$S(O)_{1-2}$N**R'R";** -$S(O)_{1-2}$($C_{1-4}$ alkyl); -OH; and $C_{1-4}$ alkoxy;
each occurrence of **$R^e$** and **$R^f$** is independently selected from the group consisting of: H; $C_{1-6}$ alkyl; -C(O)($C_{1-4}$ alkyl); -C(O)O($C_{1-4}$ alkyl); -CON**R'R"**; -$S(O)_{1-2}$N**R'R"**; -$S(O)_{1-2}$($C_{1-4}$ alkyl); -OH; and $C_{1-4}$ alkoxy; and
each occurrence of **$R^g$** is independently selected from the group consisting of: halo; cyano; $C_{1-6}$ alkyl; $C_{1-6}$ haloalkyl; $C_{1-4}$ alkoxy; $C_{1-4}$ haloalkoxy; -OH; and N**R'R"**; and
each occurrence of **R'** and **R"** is independently selected from the group consisting of: H; -OH; and $C_{1-4}$ alkyl;
optionally wherein the compound is a compound of Formula (I-A):

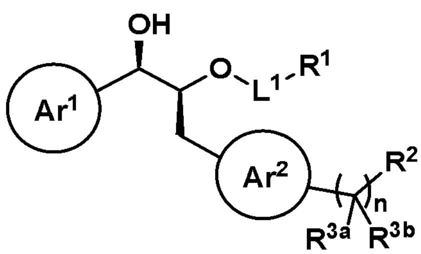

Formula **(I-A)**

or a pharmaceutically acceptable salt thereof.

**2.** The compound of claim 1, wherein:

(a) **$Ar^1$** is $C_{6-10}$ aryl optionally substituted with from 1-4 substituents, each of which is independently selected from the group consisting of: **$R^{c1}$** and **$-(L^b)_3-R^b$;** and/or

(b) **$Ar^1$** is phenyl optionally substituted with from 1-4 substituents, each of which is independently selected from the group consisting of: **$R^{c1}$** and **$-(L^b)_b-R^b$,** such as phenyl substituted with from 1-4 substituents, each of which is independently selected from the group consisting of: **$R^{c1}$** and **$-(L^b)_b-R^b$;** or phenyl substituted with from 1-4 independently selected **$R^{c1}$;** and/or

(c) each occurrence of **$R^{c1}$** is independently selected from the group consisting of: halo; $C_{1-6}$ alkyl; $C_{1-6}$ alkyl substituted with from 1-6 independently selected halo; - C(=O)($C_{1-10}$ alkyl); $C_{1-4}$ alkoxy; $C_{1-4}$ haloalkoxy; and cyano; and/or

(d) each occurrence of **$-(L^b)_3-R^b$** is an independently selected $C_{3-6}$ cycloalkyl optionally substituted with from 1-2 **$R^g$;** and/or

(e) **$Ar^1$** is

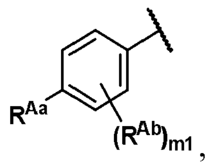,

wherein **m1** is 0, 1, 2, or 3; and **$R^{Aa}$** and **$R^{Ab}$** are each independently selected from the group consisting of: **$R^{c1}$** and **$-(L^b)_b-R^b$;** optionally wherein:

(i) **$R^{Aa}$** is selected from the group consisting of: halo; $C_{1-6}$ alkyl; $C_{1-6}$ alkyl substituted with from 1-6 independently selected halo; -C(=O)($C_{1-10}$ alkyl); $C_{1-4}$ alkoxy; $C_{1-4}$ haloalkoxy; cyano; and $C_{3-6}$ cycloalkyl optionally substituted with from 1-2 **$R^g$;** or

**$R^{Aa}$** is $C_{1-6}$ alkyl; or
**$R^{Aa}$** is $C_{1-3}$ alkyl; or
**$R^{Aa}$** is methyl; or
**$R^{Aa}$** is $C_{1-6}$ alkyl substituted with from 1-6 independently selected halo; or
**$R^{Aa}$** is $C_{1-3}$ alkyl substituted with 1-6 F, optionally wherein **$R^{Aa}$** is $CF_3$ or $CHF_2$; or
**$R^{Aa}$** is cyano; or
**$R^{Aa}$** is halo, such as -Cl; or
**$R^{Aa}$** is $C_{3-6}$ cycloalkyl; or
**$R^{Aa}$** is cyclopropyl; and/or

(ii)

**m1** is 2; or
**m1** is 1 or 3, such as 1; or
**m1** is 0;

optionally wherein when **m1** is 1 or 2, one or both occurrences of **$R^{Ab}$** are attached to the ring atom or atoms that are *ortho* to the ring atom attached to **$R^{Aa}$;** and/or

(iii) each **$R^{Ab}$** is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **$R^g$;** or

each **$R^{Ab}$** is independently $C_{1-4}$ alkoxy or $C_{1-4}$ haloalkoxy; or
each **$R^{Ab}$** is $C_{1-4}$ alkoxy; or
each **$R^{Ab}$** is methoxy.

**3.** The compound of claim 1, wherein **$Ar^1$** is

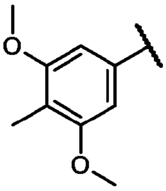 ;

or

**Ar¹** is selected from the group consisting of:

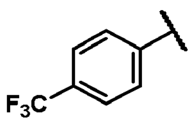 ; 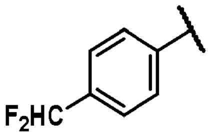 ;

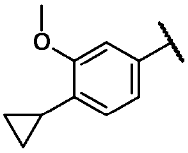 ; 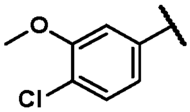 ; 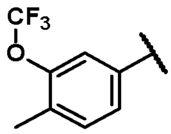 ; 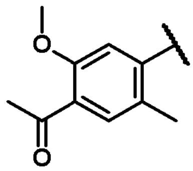 ; 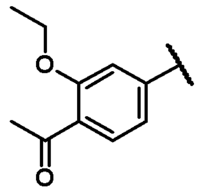 ;

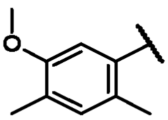 ; 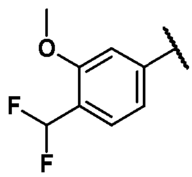 ; 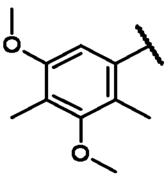 ; 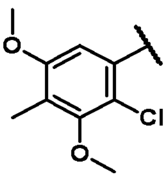 ; 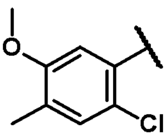 ;

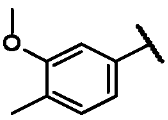 ; 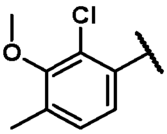 ; 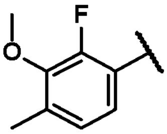 ; 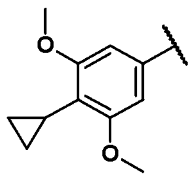 ; 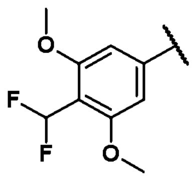 ;

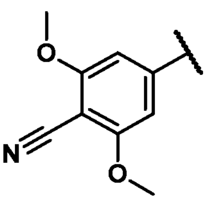 ; 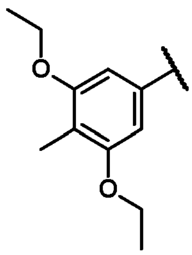 ; 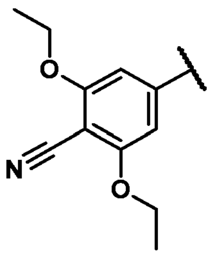 ; 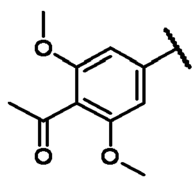 ; 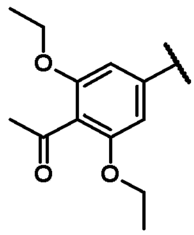 ;

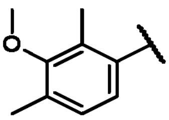 ; and 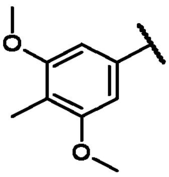 .

**4.** The compound of claim 1, wherein:

(a) **Ar¹** is:

(i) heteroaryl including from 5-10 ring atoms, wherein from 1-4 ring atoms are ring heteroatoms each independently selected from the group consisting of: N, N(H), N(**$R^d$**), O, and S, wherein the heteroaryl is optionally substituted with from 1-4 substituents, each of which is independently selected from the group consisting of: **$R^{c1}$** and -(**$L^b$**)$_b$-**$R^b$**; or

(ii) heteroaryl including from 5-6 ring atoms, wherein from 1-4 ring atoms are ring heteroatoms each independently selected from the group consisting of: N, N(H), N(**$R^d$**), O, and S, wherein the heteroaryl is optionally substituted with from 1-4 substituents, each of which is independently selected from the group consisting of: **$R^{c1}$** and -(**$L^b$**)$_b$-**$R^b$**; or

(iii) heteroaryl including 6 ring atoms, wherein from 1-2 ring atoms are ring nitrogen atoms, wherein the heteroaryl is optionally substituted with from 1-4 substituents, each of which is independently selected from the group consisting of: **$R^{c1}$** and -(**$L^b$**)$_b$-**$R^b$**; or

(iv) pyridyl optionally substituted with from 1-3 substituents selected from the group consisting of: **$R^{c1}$** and -(**$L^b$**)$_b$-**$R^b$**; or

(v) 3-pyridyl, or **$Ar^1$** is 4-pyridyl, each of which is optionally substituted with from 1-3 substituents selected from the group consisting of: **$R^{c1}$** and-(**$L^b$**)$_b$-**$R^b$**; and/or

(b) each occurrence of **$R^{c1}$** is independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; and cyano; or

each occurrence of -(**$L^b$**)$_b$-**$R^b$** is an independently selected $C_{3-6}$ cycloalkyl optionally substituted with from 1-2 **$R^g$**; or

**$Ar^1$** is 3-pyridyl substituted with 1-3 independently selected $C_{1-6}$ alkyl, or **$Ar^1$** is 4-pyridyl substituted with 1-3 independently selected $C_{1-6}$ alkoxy; or

**$Ar^1$** is

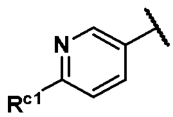

,

such as

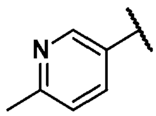

,

or **$Ar^1$** is

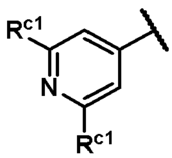

such as

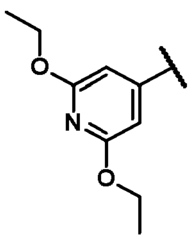

.

**5.** The compound of any one of claims 1-4, wherein:

(a) **$L^1$** is a bond; or
(b) **$L^1$** is $C_{1-6}$ alkylene optionally substituted with from 1-6 **$R^a$;** or
(c) **$L^1$** is $C_{1-3}$ alkylene optionally substituted with from 1-6 **$R^a$;** or
(d) **$L^1$** is unsubstituted $C_{1-3}$ alkylene; or
(e) **$L^1$** is $CH_2CH_2$; or
(f) **$L^1$** is $CH_2$.

**6.** The compound of any one of claims 1-5, wherein:

(i) **$R^1$** is **$R^b$**; or
(ii) **$R^1$** is selected from the group consisting of:

- $C_{3-10}$ cycloalkyl or $C_{3-10}$ cycloalkenyl, each of which is optionally substituted with from 1-4 **$R^g$;** and
- $C_{6-10}$ aryl optionally substituted with from 1-4 **$R^g$;** or

(iii) **$R^1$** is $C_{6-10}$ aryl optionally substituted with from 1-4 **$R^g$;** or
(iv) **$R^1$** is phenyl optionally substituted with from 1-4 **$R^g$;** or
(v) **$R^1$** is phenyl optionally substituted with from 1-2 **$R^g$;** or
(vi) **$R^1$** is unsubstituted phenyl; or
(vii) **$R^1$** is $C_{8-10}$ bicyclic aryl optionally substituted with from 1-4 **$R^g$;** or
(viii) **$R^1$** is $C_{9-10}$ bicyclic aryl optionally substituted with from 1-2 **$R^g$;** or
(ix) **$R^1$** is indanyl optionally substituted with from 1-2 **$R^g$;** or
(x) **$R^1$** is

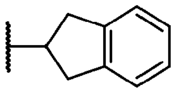,

which is optionally substituted with from 1-2 **$R^g$;** or
(xi) **$R^1$** is

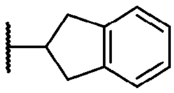;

or
(xii) **$R^1$** is $C_{3-10}$ cycloalkyl or $C_{3-10}$ cycloalkenyl, each of which is optionally substituted with from 1-4 **$R^g$;** or
(xiii) **$R^1$** is $C_{3-10}$ cycloalkyl which is optionally substituted with from 1-4 **$R^g$;** or
(xiv) **$R^1$** is $C_{3-6}$ cycloalkyl which is optionally substituted with from 1-2 **$R^g$;** or
(xv) **$R^1$** is cyclobutyl or cyclopentyl, each of which is optionally substituted with from 1-2 **$R^g$**, such as cyclopentyl optionally substituted with from 1-2 **$R^g$;** or
(xvi) **$R^1$** is unsubstituted cyclobutyl or cyclopentyl, such as unsubstituted cyclopentyl; or
(xvii) **$R^1$** is $C_{2-6}$ alkynyl optionally substituted with from 1-6 **$R^a$;** or
(xviii) **$R^1$** is $C_{2-4}$ alkynyl optionally substituted with from 1-3 **$R^a$;** or
(xix) **$R^1$** is

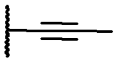;

or
(xx) **$R^1$** is $C_{1-6}$ alkyl optionally substituted with from 1-6 **$R^a$;** or
(xxi) **$R^1$** is $C_{1-6}$ alkyl; or
(xxii) **$R^1$** is $C_{2-4}$ alkyl; or
(xxiii) **$R^1$** is $C_3$ alkyl, such as n-propyl and i-propyl; or
(xxiv) **$R^1$** is i-propyl; or

(xxv) **$R^1$** is $C_{3-5}$ alkyl; or
(xxvi) **$R^1$** is $C_4$ alkyl, such as n-butyl, i-butyl, sec-butyl, and tert-butyl; or
(xxvii) **$R^1$** is i-butyl.

**7.** The compound of any one of claims 1-6, wherein:

(a) **$Ar^2$** is heteroarylene including from 5-10 ring atoms, wherein from 1-4 ring atoms are ring heteroatoms each independently selected from the group consisting of: N, N(H), N(**$R^d$**), O, and S, wherein the heteroarylene is optionally substituted with from 1-4 substituents, each of which is independently selected from the group consisting of: **$R^{c2}$** and -(**$L^b$**)$_b$-**$R^b$**; or
(b) **$Ar^2$** is heteroarylene including from 5-6 ring atoms, wherein from 1-4 ring atoms are ring heteroatoms each independently selected from the group consisting of: N, N(H), N(**$R^d$**), O, and S, wherein the heteroarylene is optionally substituted with from 1-4 substituents, each of which is independently selected from the group consisting of: **$R^{c2}$** and -(**$L^b$**)$_b$-**$R^b$;** or
(c) **$Ar^2$** is heteroarylene including from 5 ring atoms, wherein from 1-4 ring atoms are ring heteroatoms each independently selected from the group consisting of: N, N(H), N(**$R^d$**), O, and S, wherein the heteroarylene is optionally substituted with from 1-4 substituents, each of which is independently selected from the group consisting of: **$R^{c2}$** and -(**$L^b$**)$_b$-**$R^b$;** or
(d) **$Ar^2$** is selected from the group consisting of pyrrolylene, pyrazolylene, thiazolylene and 1,3,4-oxadiazolylene, each of which is optionally substituted with from 1-4 substituents, each of which is independently selected from the group consisting of: **$R^{c2}$** and -(**$L^b$**)$_b$-**$R^b$;** or
(e) **$Ar^2$** is

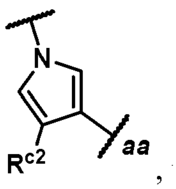
,

wherein ***aa*** is the point of attachment to -(C**$R^{3a}$$R^{3b}$**)n-**R,** optionally wherein **$R^{c2}$** is C(O)O$C_{1-4}$ alkyl, such as C(O)OMe; or
(f) **$Ar^2$** is

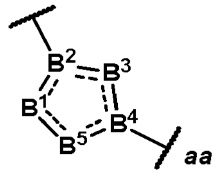
,

wherein:

each 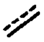 is independently a single bond or a double bond, provided that the ring including **$B^1$, $B^2$, $B^3$, $B^4$,** and **$B^5$** is heteroaryl;
***aa*** is the point of attachment to -(C**$R^{3a}$$R^{3b}$**)$_n$-**$R^2$;**
**$B^2$** and **$B^4$** are C or N; and
**$B^1$**, **$B^3$**, and **$B^5$** are independently O, S, N, N(H), N(**$R^d$**), CH, C**$R^{c2}$,** or C-**($L^b$)$_b$-$R^b$**, provided that:
from 1-4 of **$B^1$**, **$B^2$**, **$B^3$**, **$B^4$**, and **$B^5$** are independently selected heteroatoms;
optionally wherein:

(i) **$B^2$** is N; and/or **$B^4$** is C; and/or **$B^1$**, **$B^3$**, and **$B^5$** are independently CH, C**$R^{c2}$,** or C-**($L^b$)$_b$-$R^b$;** and/or **$B^5$** is C**$R^{c2}$;** and **$B^1$** and **$B^3$** are CH; or
(ii) **$B^2$** is N; **$B^4$** is C; and **$B^1$, $B^3$,** and **$B^5$** are independently CH, C**$R^{c2}$,** or C-**($L^b$)$_b$-$R^b$;** or
(iii) **$B^2$** is C, optionally wherein **$B^4$** is C; or
(iv) **$B^5$** is CH, C**$R^{c2}$,** or C-**($L^b$)$_b$-$R^b$;** and one of **$B^1$** and **$B^3$** is N; and the other of **$B^1$** and **$B^3$** is NH, N(**$R^d$**), O, or S, such as S; and/or **$Ar^2$** is selected from the list consisting of

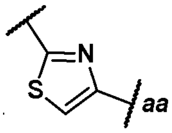, 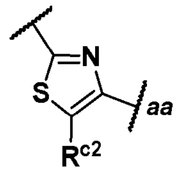, and 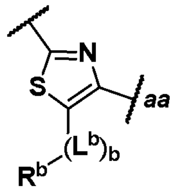,

wherein ***aa*** is the point of attachment to -(C**$R^{3a}R^{3b}$**)$_n$-**$R^2$**,

optionally wherein **$R^{c2}$** is C(=O)$C_{1-4}$ alkyl, such as C(=O)Et, or C(=O)Me; or
optionally wherein **$R^{c2}$** is $C_{1-6}$ alkyl, such as methyl, ethyl, propyl, such as n-propyl; or
optionally wherein **-($L^b$)$_b$-$R^b$** is $C_{3-10}$ cycloalkyl, such as cycloproyl; or

(v) **$Ar^2$** is

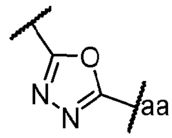,

wherein ***aa*** is the point of attachment to - (C**$R^{3a}R^{3b}$**)$_n$-**$R^2$;** or
(vi) **$B^2$** is C; and **$B^4$** is N; or
(vii) **$B^3$** is N; and **$B^1$** and **$B^5$** are independently CH, C**$R^{c2}$,** or C-**($L^b$)$_b$-$R^b$;** or.
(viii) **$Ar^2$** is

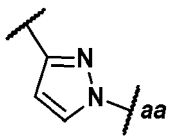,

wherein ***aa*** is the point of attachment to - (C**$R^{3a}R^{3b}$**)$_n$-**$R^2$.**

**8.** The compound of any one of claims 1-6, wherein:

(a) **$Ar^2$** is heteroarylene including 6 ring atoms, wherein from 1-2 ring atoms are ring nitrogen atoms, wherein the heteroarylene is optionally substituted with from 1-4 substituents, each of which is independently selected from the group consisting of: **$R^{c2}$** and -(**$L^b$**)$_b$-**$R^b$;** or
(b) **$Ar^2$** is pyridylene which is optionally substituted with from 1-4 substituents, each of which is independently selected from the group consisting of: **$R^{c2}$** and **-($L^b$)$_b$**-$R^b$**;** or
(c) **$Ar^2$** is

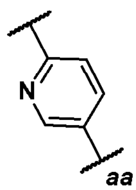 or 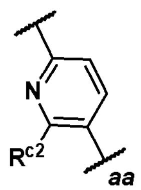,

wherein ***aa*** is the point of attachment to - (C**$R^{3a}R^{3b}$**)$_n$-**$R^2$;** optionally wherein **$R^{c2}$** is $C_{1-4}$ alkoxy or $C_{1-4}$ haloalkoxy, such as -OMe; or
(d) **$Ar^2$** is bicyclic heteroarylene including from 9-10 ring atoms, wherein from 1-4 ring atoms are ring heteroatoms each independently selected from the group consisting of: N, N(H), N(**$R^d$**), O, and S, wherein the heteroarylene is optionally substituted with from 1-4 substituents, each of which is independently selected from the group consisting of: **$R^{c2}$** and **-($L^b$)$_b$-$R^b$;** or
(e) **$Ar^2$** is selected from the group consisting of benzimidazolylene, indazolylene, benzothiazolylene, and imidazo[1,2-a]pyridylene (e.g. benzothiazolylene), each of which is optionally substituted with from 1-4 substituents, each of which is independently selected from the group consisting of: $\mathbf{R^{c2}}$ and $\mathbf{-(L^b)_b-R^b}$; or

(f) $\mathbf{Ar^2}$ is:

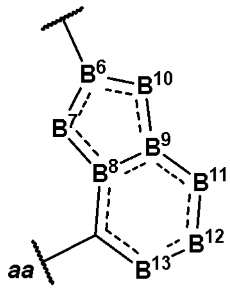

,

wherein:

each 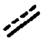 is independently a single bond or a double bond, provided that the 5-membered ring including $\mathbf{B^6}$, $\mathbf{B^7}$, $\mathbf{B^8}$, $\mathbf{B^9}$, and $\mathbf{B^{10}}$ is heteroaryl, and the 6-membered ring including $\mathbf{B^8}$, $\mathbf{B^9}$, $\mathbf{B^{11}}$, $\mathbf{B^{12}}$, and $\mathbf{B^{13}}$ is aryl or heteroaryl;

***aa*** is the point of attachment to -(C$\mathbf{R^{3a}R^{3b}}$)$_n$-$\mathbf{R^2}$;

$\mathbf{B^6}$, $\mathbf{B^8}$, and $\mathbf{B^9}$ are independently C or N;

$\mathbf{B^7}$ and $\mathbf{B^{10}}$ are independently selected from the group consisting of: O, S, N, N(H), N($\mathbf{R^d}$), CH, C$\mathbf{R^{c2}}$ and C-$\mathbf{(L^b)_b-R^b}$;

$\mathbf{B^{11}}$, $\mathbf{B^{12}}$, and $\mathbf{B^{13}}$ are independently N, CH, C$\mathbf{R^{c2}}$, or C-$\mathbf{(L^b)_b-R^b}$, provided that:

from 1-4 of $\mathbf{B^6}$, $\mathbf{B^7}$, $\mathbf{B^8}$, $\mathbf{B^9}$, $\mathbf{B^{10}}$, $\mathbf{B^{11}}$, $\mathbf{B^{12}}$, and $\mathbf{B^{13}}$ is an independently selected heteroatom; and no more than 3 of $\mathbf{B^7}$, $\mathbf{B^{10}}$, $\mathbf{B^{11}}$, $\mathbf{B^{12}}$, and $\mathbf{B^{13}}$ are C$\mathbf{R^{c2}}$, or C-$\mathbf{(L^b)_b-R^b}$;

optionally wherein:

(i) $\mathbf{B^8}$ and $\mathbf{B^9}$ are C; and/or

$\mathbf{B^{11}}$, $\mathbf{B^{12}}$, and $\mathbf{B^{13}}$ are independently CH, C$\mathbf{R^{c2}}$, or C-$\mathbf{(L^b)_b-R^b}$; and/or

$\mathbf{B^{11}}$, $\mathbf{B^{12}}$, and $\mathbf{B^{13}}$ are CH; and/or

$\mathbf{B^6}$ is N; and/or

$\mathbf{B^7}$ is N; and/or

$\mathbf{B^{10}}$ is CH or C$\mathbf{R^{c2}}$, or C-$\mathbf{(L^b)_b-R^b}$; and/or

$\mathbf{B^{10}}$ is CH; and/or

$\mathbf{Ar^2}$ is

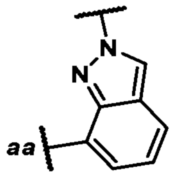

,

which is optionally substituted with from 1-2 substituents, each of which is independently selected from the group consisting of: $\mathbf{R^{c2}}$ and $\mathbf{-(L^b)_b-R^b}$ , wherein ***aa*** is the point of attachment to -(C$\mathbf{R^{3a}R^{3b}}$)$_n$-$\mathbf{R^2}$; or

(ii) $\mathbf{B^7}$ is N; and/or

$\mathbf{B^{10}}$ is S; and/or

$\mathbf{Ar^2}$ is

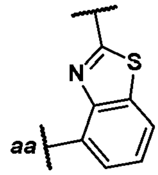,

which is optionally substituted with from 1-2 substituents, each of which is independently selected from the group consisting of: $\mathbf{R^{c2}}$ and $\mathbf{-(L^b)_b-R}^b$, wherein ***aa*** is the point of attachment to $-(C\mathbf{R^{3a}R^{3b}})_n\mathbf{-R^2}$; or

(iii) $\mathbf{B^8}$ is C and $\mathbf{B^9}$ is N; and/or

$\mathbf{B^7}$ is N; and/or
$\mathbf{B^{10}}$, $\mathbf{B^{11}}$, $\mathbf{B^{12}}$, and $\mathbf{B^{13}}$ are CH, $C\mathbf{R^{c2}}$, or $C\mathbf{-(L^b)_b-R^b}$; and/or
$\mathbf{Ar^2}$ is

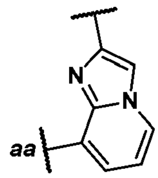,

which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to $-(C\mathbf{R^{3a}R^{3b}})_n\mathbf{-R^2}$; or

(iv) $\mathbf{Ar^2}$ is selected from the group consisting of:

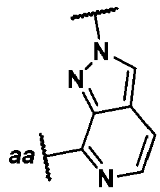,

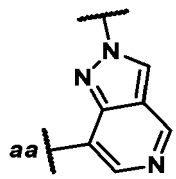 and 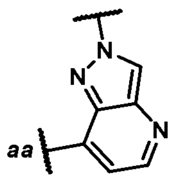,

each of which is optionally substituted with from 1-2 substituents, each of which is independently selected from the group consisting of: $\mathbf{R^{c2}}$ and $-(\mathbf{L^b})_b\mathbf{-R^b}$, wherein ***aa*** is the point of attachment to $-(C\mathbf{R^{3a}R^{3b}})_n\mathbf{-R^2}$; or

(v) $\mathbf{Ar^2}$ is selected from the group consisting of:

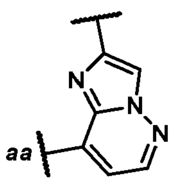,

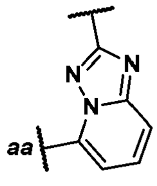, and 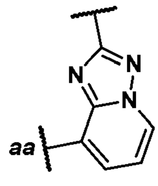,

each of which is optionally substituted with from 1-2 substituents, each of which is independently selected from the group consisting of: $R^{c2}$ and $-(L^b)_b-R^b$, wherein *aa* is the point of attachment to $-(CR^{3a}R^{3b})_n-R^2$; or

(vi) $Ar^2$ is selected from the group consisting of

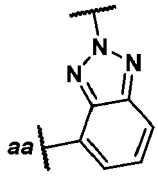,

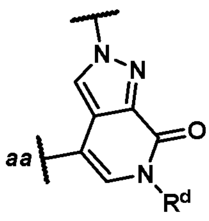, 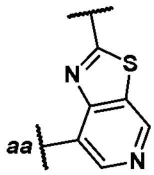, 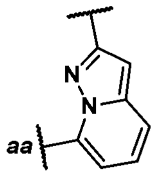, 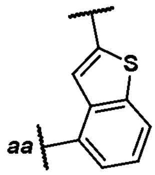, 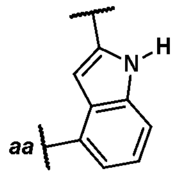

and

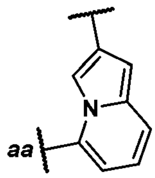,

each of which is optionally substituted with from 1-2 substituents, each of which is independently selected from the group consisting of: $R^{c2}$ and $-(L^b)_b-R^b$, wherein *aa* is the point of attachment to $-(CR^{3a}R^{3b})_n-R^2$; or

(g) $Ar^2$ is:

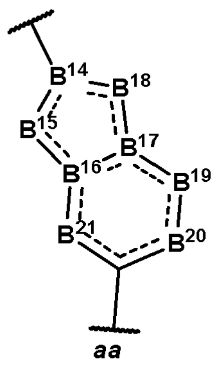,

wherein:

each 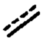 is independently a single bond or a double bond, provided that the 5-membered ring including **$B^{14}$, $B^{15}$, $B^{16}$, $B^{17}$**, and **$B^{18}$** is heteroaryl, and the 6-membered ring including **$B^{16}$, $B^{17}$, $B^{19}$, $B^{20}$**, and **$B^{21}$** is aryl or heteroaryl;
***aa*** is the point of attachment to -(C**$R^{3a}R^{3b}$**)$_n$**-$R^2$;**
**$B^{16}$, $B^{17}$**, and **$B^{14}$** are independently C or N;
$B^{15}$ and **$B^{18}$** are independently selected from the group consisting of: O, S, N, N(H), N(**$R^d$**), CH, and C**$R^{c2}$;**
**$B^{19}$, $B^{20}$**, and **$B^{21}$** are independently N, CH, C**$R^{c2}$**, or C-**($L^b$)$_b$-$R^b$**, provided that:

from 1-4 of **$B^{14}$, $B^{15}$, $B^{16}$, $B^{17}$, $B^{18}$, $B^{19}$, $B^{20}$**, and **$B^{21}$** is an independently selected heteroatom; and no more than 3 of **$B^{15}$, $B^{18}$, $B^{19}$, $B^{20}$**, and **$B^{21}$** are C**$R^{c2}$** or **C-($L^b$)$_b$-$R^b$;**
optionally wherein:

**$B^{16}$** and **$B^{17}$** are C; and/or
**$B^{19}$, $B^{20}$**, and **$B^{21}$** are independently N, CH, C**$R^{c2}$**, or C-**($L^b$)$_b$-$R^b$**; and/or
**$B^{14}$** is C; and/or
one of **$B^{15}$** and **$B^{18}$** is N; and the other of **$B^{15}$** and **$B^{18}$** is O, S, NH, or N**($R^d$)**, such as NH or N**($R^d$)**, such as NH or N($C_{1-3}$ alkyl); or

(h) **$Ar^2$** is

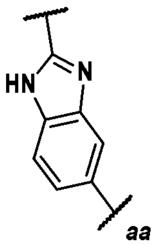
or
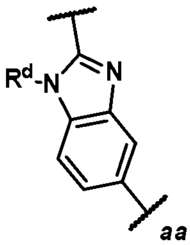
,

each of which is optionally substituted with from 1-2 **$R^{c2}$**, wherein ***aa*** is the point of attachment to -(C**$R^{3a}R^{3b}$**)$_n$-**$R^2$**; or
(i) **$Ar^2$** is

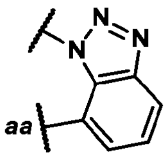
,

which is optionally substituted with from 1-2 substituents, each of which is independently selected from the group consisting of: **$R^{c2}$** and -**($L^b$)$_b$-$R^b$**, wherein ***aa*** is the point of attachment to -(C**$R^{3a}R^{3b}$**)$_n$-**$R^2$**.

**9.** The compound of any one of claims 1-8, wherein:

(a)

**n is** 0; or
**n** is 1, and optionally wherein **$R^{3a}$** and **$R^{3b}$** are H; or
**n** is 1, and wherein one of **$R^{3a}$** and **$R^{3b}$** is H, and the other one of **$R^{3a}$** and **$R^{3b}$** is $C_{1-6}$ alkyl, such as ethyl or methyl; or
**n** is 1, and wherein **$R^{3a}$** and **$R^{3b}$** together with the carbon atom to which each is attached forms a $C_{3-6}$ cycloalkyl, such as a cyclopropyl; and/or

(b)

**$R^2$** is -C(=O)OH; or

$\mathbf{R^2}$ is carboxylic acid bioisostere; or
$\mathbf{R^2}$ is $-C(=O)NH_2$, $-C(=O)NHSO_2Me$ or

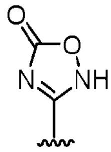

.

**10.** The compound of claim 1, wherein:

(A) the compound is a compound of Formula (1-1):

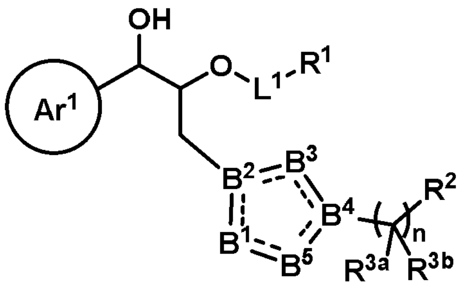

Formula (**I-1**)

or a pharmaceutically acceptable salt thereof, wherein:

each 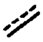 is independently a single bond or a double bond, provided that the ring including $\mathbf{B^1}$, $\mathbf{B^2}$, $\mathbf{B^3}$, $\mathbf{B^4}$, and $\mathbf{B^5}$ is heteroaryl;
$\mathbf{B^2}$ and $\mathbf{B^4}$ are C or N;
$\mathbf{B^1}$, $\mathbf{B^3}$, and $\mathbf{B^5}$ are independently O, S, N, N(H), N($\mathbf{R^d}$), CH, C$\mathbf{R^{c2}}$, or C-$\mathbf{(L^b)_b}$-$\mathbf{R^b}$, provided that: from 1-4 of $\mathbf{B^1}$, $\mathbf{B^2}$, $\mathbf{B^3}$, $\mathbf{B^4}$, and $\mathbf{B^5}$ are independently selected heteroatoms; and
$\mathbf{Ar^1}$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$; and
heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $\mathbf{R^g}$;
optionally wherein:

(a) $\mathbf{B^2}$ is N; and $\mathbf{B^4}$ is C; and/or

$\mathbf{B^1}$, $\mathbf{B^3}$, and $\mathbf{B^5}$ are independently CH, C$\mathbf{R^{c2}}$ or C-$\mathbf{(L^b)_b}$-$\mathbf{R^b}$; and/or
the ring including $\mathbf{B^1}$-$\mathbf{B^5}$ is

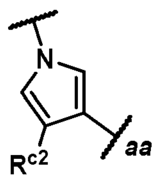

,

wherein ***aa*** is the point of attachment to -(C$\mathbf{R^{3a}R^{3b}}$)$_n$-$\mathbf{R^2}$, optionally wherein $\mathbf{R^{c2}}$ is $C(O)OC_{1-4}$ alkyl; or

(b) **$B^2$** is C; and **$B^4$** is N; and/or

**$B^3$** is N; and **$B^1$** and **$B^5$** are independently CH, C**$R^{c2}$** or C-**$(L^b)_b$-$R^b$;** and/or the ring including **$B^1$-$B^5$** is

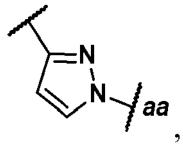

,

wherein ***aa*** is the point of attachment to -(C**$R^{3a}R^{3b}$**)$_n$-**$R^2$**; or

(c) **$B^2$** is C; and **$B^4$** is C; and/or

**$B^5$** is CH or C**$R^{c2}$**; one of **$B^1$** and **$B^3$** is N; and the other of **$B^1$** and **$B^3$** is NH, N(**$R^d$**), O, or S, such as S; and/or the ring including **$B^1$-$B^5$** is:

(i)

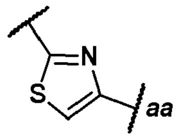

,

wherein ***aa*** is the point of attachment to -(C**$R^{3a}R^{3b}$**)$_n$-**$R^2$;** or
(ii)

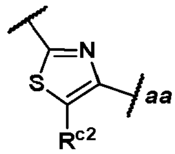

,

wherein ***aa*** is the point of attachment to -(C**$R^{3a}R^{3b}$**)$_n$-**$R^2$,** optionally wherein **$R^{c2}$** is C(O)OC$_{1-4}$ alkyl, C$_{1-4}$ alkyl, or C$_{1-4}$ alkoxy or C$_{1-4}$ haloalkoxy; or
(iii)

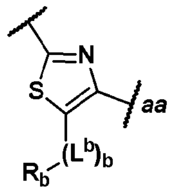

,

wherein ***aa*** is the point of attachment to -(C**$R^{3a}R^{3b}$**)$_n$-**$R^2$,** optionally wherein -**$(L^b)_b$-$R^b$** is C$_{3-10}$ cycloalkyl, such as cyclopropyl; or
(iv)

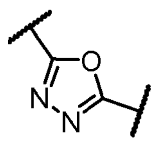

,

wherein *aa* is the point of attachment to -(C**R**$^{3a}$**R**$^{3b}$)$_n$-**R**$^2$; or

(B) the compound is a compound of Formula **(I-2):**

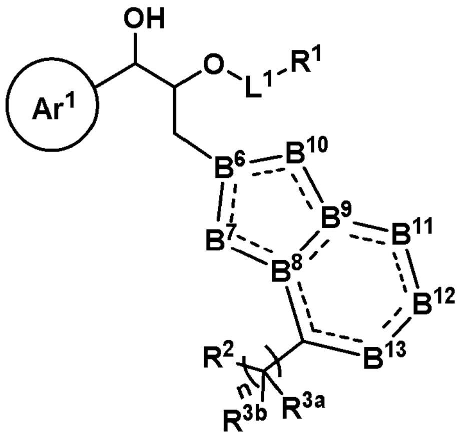

Formula (**I-2)**

or a pharmaceutically acceptable salt thereof, wherein:

each 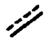 is independently a single bond or a double bond, provided that the 5-membered ring including **B**$^6$**, B**$^7$**, B**$^8$**, B**$^9$, and **B**$^{10}$ is heteroaryl, and the 6-membered ring including **B**$^8$, **B**$^9$**, B**$^{11}$, **B**$^{12}$**,** and **B**$^{13}$ is aryl or heteroaryl;

**B**$^6$**, B**$^8$**,** and **B**$^9$ are independently C or N;

**B**$^7$ and **B**$^{10}$ are independently selected from the group consisting of: O, S, N, N(H), N**(R**$^d$**)**, CH, C**R**$^{c2}$, or C-**(L**$^b$**)**$_b$**-R**$^b$**;**

**B**$^{11}$, **B**$^{12}$**,** and **B**$^{13}$ are independently N, CH, C**R**$^{c2}$, or C-**(L**$^b$**)**$_b$**-R**$^b$, provided that:

from 1-4 of **B**$^6$, **B**$^7$**, B**$^8$**, B**$^9$**, B**$^{10}$**, B**$^{11}$, **B**$^{12}$**,** and **B**$^{13}$ is an independently selected heteroatom; and no more than 3 of **B**$^7$, **B**$^{10}$**, B**$^{11}$, **B**$^{12}$**,** and **B**$^{13}$ are C**R**$^{c2}$ or C-**(L**$^b$**)**$_b$**-R**$^b$;

**Ar**$^1$ is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^g$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^g$;

optionally wherein:

(a) **B**$^8$ and **B**$^9$ are C; and/or

**B**$^{11}$**, B**$^{12}$**,** and **B**$^{13}$ are independently CH or C**R**$^{c2}$; and/or

**B**$^6$ is N; **B**$^7$ is N; and **B**$^{10}$ is CH or C**R**$^{c2}$; and/or

the ring including **B**$^6$**-B**$^{13}$ is

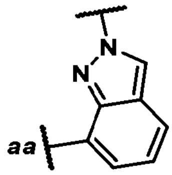,

which is optionally substituted with from 1-2 $\mathbf{R^{c2}}$, wherein ***aa*** is the point of attachment to -(C$\mathbf{R^{3a}R^{3b}}$)$_n$-$\mathbf{R^2}$; or

(b) $\mathbf{B^7}$ is N; and/or

$\mathbf{B^{10}}$ is S; and/or
the ring including $\mathbf{B^6}$-$\mathbf{B^{13}}$ is

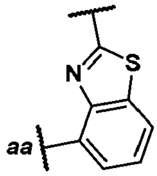,

which is optionally substituted with from 1-2 substituents, each of which is independently selected from the group consisting of: $\mathbf{R^{c2}}$ and **-($\mathbf{L^b}$)$_b$-$\mathbf{R^b}$,** wherein ***aa*** is the point of attachment to -(C$\mathbf{R^{3a}R^{3b}}$)$_n$-$\mathbf{R^2}$; or

(c) $\mathbf{B^8}$ is C; and $\mathbf{B^9}$ is N; and/or

$\mathbf{B^7}$ is N; and/or
$\mathbf{B^{10}}$, $\mathbf{B^{11}}$, $\mathbf{B^{12}}$ , and $\mathbf{B^{13}}$ are CH, C$\mathbf{R^{c2}}$, or C-($\mathbf{L^b}$)$_b$-$\mathbf{R^b}$; and/or
the ring including $\mathbf{B^6}$-$\mathbf{B^{13}}$ is

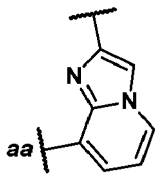,

which is optionally substituted with from 1-2 substituents, each of which is independently selected from the group consisting of: $\mathbf{R^{c2}}$ and -($\mathbf{L^b}$)$_b$-$\mathbf{R^b}$, wherein ***aa*** is the point of attachment to -(C$\mathbf{R^{3a}R^{3b}}$)$_n$-$\mathbf{R^2}$**;** or

(d) the ring including $\mathbf{B^6}$-$\mathbf{B^{13}}$ is selected from the group consisting of

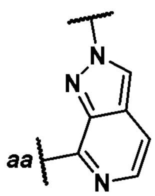, 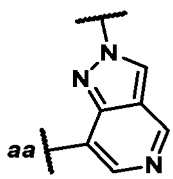 and 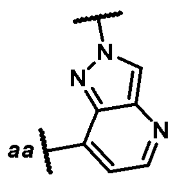,

each of which is optionally substituted with from 1-2 substituents, each of which is independently selected from the group consisting of: $\mathbf{R^{c2}}$ and -($\mathbf{L^b}$)$_b$-$\mathbf{R^b}$, wherein ***aa*** is the point of attachment to -(C$\mathbf{R^{3a}R^{3b}}$)$_n$-$\mathbf{R^2}$**;** or
(e) the ring including $\mathbf{B^6}$-$\mathbf{B^{13}}$ is selected from the group consisting of

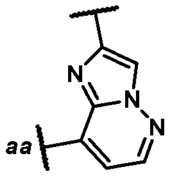, 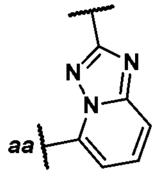, and 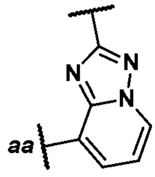,

each of which is optionally substituted with from 1-2 substituents, each of which is independently selected from the group consisting of: $\mathbf{R^{c2}}$ and $\mathbf{-(L^b)_b-R^b}$, wherein ***aa*** is the point of attachment to $-(C\mathbf{R^{3a}R^{3b}})_n-\mathbf{R^2}$; or

(f) the ring including $\mathbf{B^6}$-$\mathbf{B^{13}}$ is selected from the group consisting of

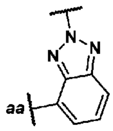, 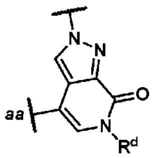, 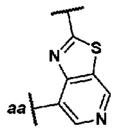, 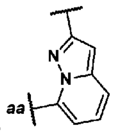, 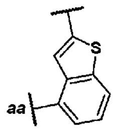, 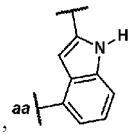

and

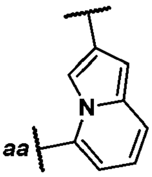,

each of which is optionally substituted with from 1-2 substituents, each of which is independently selected from the group consisting of: $\mathbf{R^{c2}}$ and $-\mathbf{(L^b)b-R^b}$, wherein ***aa*** is the point of attachment to $-(C\mathbf{R^{3a}R^{3b}})_n-\mathbf{R^2}$; or

(C) the compound is a compound of Formula **(I-3)**:

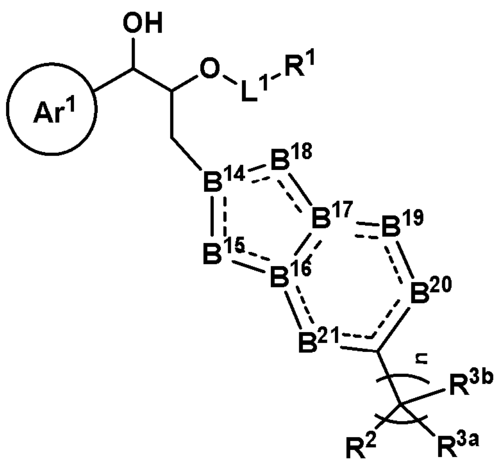

Formula (**I-3**)

or a pharmaceutically acceptable salt thereof, wherein:

each 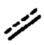 is independently a single bond or a double bond, provided that the 5-membered ring including $\mathbf{B^{14}}$, $\mathbf{B^{15}}$, $\mathbf{B^{16}}$, $\mathbf{B^{17}}$, and $\mathbf{B^{18}}$ is heteroaryl, and the 6-membered ring including $\mathbf{B^{16}}$, $\mathbf{B^{17}}$, $\mathbf{B^{19}}$, $\mathbf{B^{20}}$, and $\mathbf{B^{21}}$ is aryl or heteroaryl;

$\mathbf{B^{16}}$, $\mathbf{B^{17}}$, and $\mathbf{B^{14}}$ are independently C or N;

$B^{15}$ and $B^{18}$ are independently selected from the group consisting of: O, S, N, N(H), N($R^d$), CH, C$R^{c2}$, and C-$(L^b)_b$-$R^b$.

$B^{19}$, $B^{20}$, and $B^{21}$ are independently N, CH, C$R^{c2}$, or C-$(L^b)_b$-$R^b$, provided that:

from 1-4 of $B^{14}$, $B^{15}$, $B^{16}$, $B^{17}$, $B^{18}$, $B^{19}$, $B^{20}$, and $B^{21}$ is an independently selected heteroatom; and no more than 3 of $B^{15}$, $B^{18}$, $B^{19}$, $B^{20}$, and $B^{21}$ are C$R^{c2}$ or C-$(L^b)_b$-$R^b$; and

**Ar$^1$** is selected from the group consisting of:

phenyl substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$; and

heteroaryl including 6 ring atoms, wherein from 1-2 ring atoms are ring nitrogen atoms, wherein the heteroaryl is optionally substituted with from 1-4 substituents each independently selected from the group consisting of: halo; $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 $R^g$;

optionally wherein:

(a) $B^{16}$ and $B^{17}$ are C; and/or

(b) $B^{19}$, $B^{20}$, and $B^{21}$ are independently CH, C$R^{c2}$, or C-$(L^b)_b$-$R^b$; and/or

(c) $B^{14}$ is C; one of $B^{15}$ and $B^{18}$ is N; and the other of $B^{15}$ and $B^{18}$ is O, S, NH, or N($R^d$), such as NH or N($R^d$), such as NH or N($C_{1-3}$ alkyl); and/or

(d) the ring including $B^{14}$-$B^{21}$ is

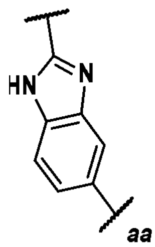 or 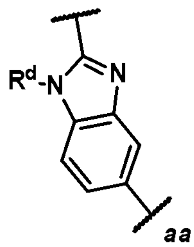,

each of which is optionally substituted with from 1-2 $R^{c2}$, wherein ***aa*** is the point of attachment to - (C$R^{3a}R^{3b}$)$_n$-$R^2$.

**11.** The compound of claim 10, wherein:

(a) **n** is 1, and optionally wherein $R^{3a}$ and $R^{3b}$ are H; or

**n** is 1, and wherein one of $R^{3a}$ and $R^{3b}$ is H, and the other one of $R^{3a}$ and $R^{3b}$ is $C_{1-6}$ alkyl, such as ethyl or methyl; or

**n** is 1, and wherein $R^{3a}$ and $R^{3b}$ together with the carbon atom to which each is attached forms a $C_{3-6}$ cycloalkyl, such as a cyclopropyl; or

**n** is 0; and/or

(b) **Ar$^1$** is

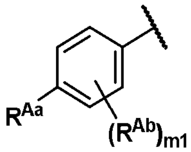;

**m1** is 0, 1, 2, or 3; and each occurrence of $R^{Aa}$ and $R^{Ab}$ are independently selected from the group consisting of:

halo; -C(=O)($C_{1-10}$ alkyl); $C_{1-6}$ alkyl; $C_{1-6}$ alkyl substituted with from 1-6 independently selected halo; $C_{1-4}$ alkoxy; $C_{1-4}$ haloalkoxy; cyano; and $C_{3-6}$ cycloalkyl optionally substituted with from 1-2 **R**$^{g}$;

optionally wherein **m1** is 0; or **m1** is 1 or 2, optionally wherein each $\mathbf{R^{Ab}}$ is *ortho* to $\mathbf{R^{Aa}}$; and/or optionally wherein each $\mathbf{R^{Ab}}$ when present is $C_{1-4}$ alkoxy or $C_{1-4}$ haloalkoxy, such as $C_{1-4}$ alkoxy, such as methoxy; and/or optionally wherein $\mathbf{R^{Aa}}$ is $C_{1-3}$ alkyl; $C_{1-3}$ alkyl substituted with 1-6 F; halo; or $C_{3-6}$ cycloalkyl; and/or

(c) **Ar**$^{1}$ is

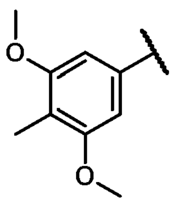 ;

or

**Ar**$^{1}$ is selected from the group consisting of:

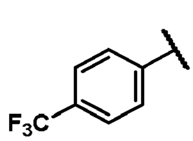 ; 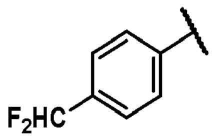 ;

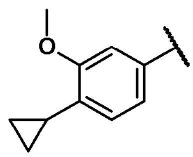 ; 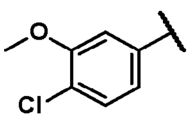 ; 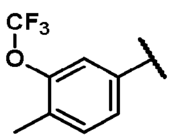 ; 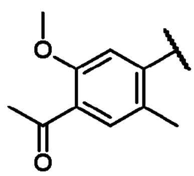 ; 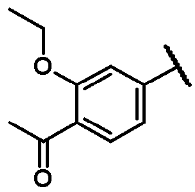 ;

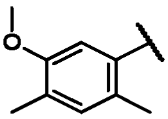 ; 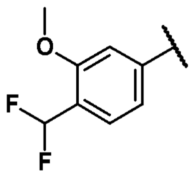 ; 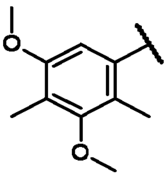 ; 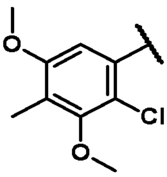 ; 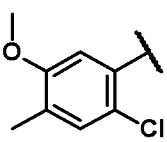 ;

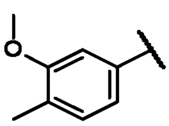 ; 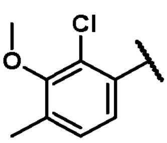 ; 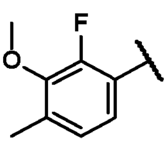 ; 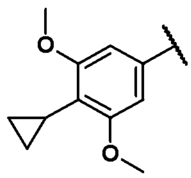 ; 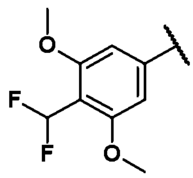 ;

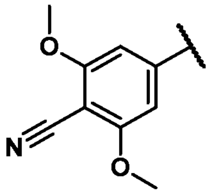 ; 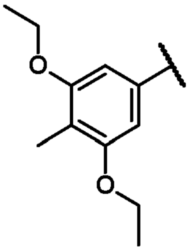 ; 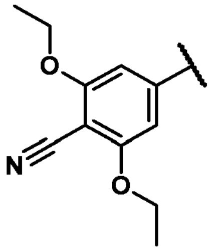 ; 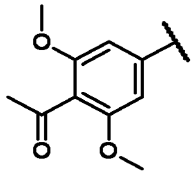 ; 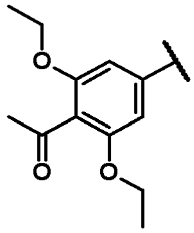 ;

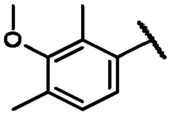 ; and 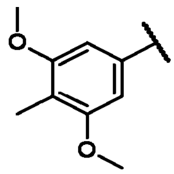 ;

and/or

(d) **L¹** is a bond; or **L¹** is $C_{1-3}$ alkylene optionally substituted with from 1-6 **Rᵃ;** or **L¹** is $CH_2$ or $CH_2CH_2$; and/or

(e) **R¹** is:

(i) phenyl optionally substituted with from 1-2 **Rᵍ**; or
(ii) $C_{9-10}$ bicyclic aryl optionally substituted with from 1-2 **Rᵍ**; or
(iii)

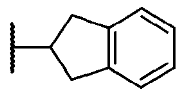 ,

which is optionally substituted with from 1-2 **Rᵍ**; or
(iv) $C_{3-6}$ cycloalkyl which is optionally substituted with from 1-2 **Rᵍ**; or
(v) cyclobutyl or cyclopentyl, each of which is optionally substituted with from 1-2 **Rᵍ**; or
(vi) $C_{1-6}$ alkyl optionally substituted with from 1-6 **Rᵃ**; or
(vii) $C_{1-6}$ alkyl; or
(viii) $C_{2-4}$ alkyl; or
(ix) $C_3$ alkyl, such as *n*-propyl and i-propyl; or
(x) *i*-propyl; or
(xi) $C_{3-5}$ alkyl; or
(xii) $C_4$ alkyl, such as *n*-butyl, i-butyl, *sec*-butyl, and *tert-butyl;* or
(xiii) *i*-butyl; and/or

(f) the

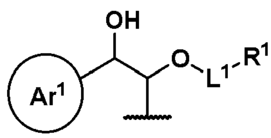

moiety has the following formula:

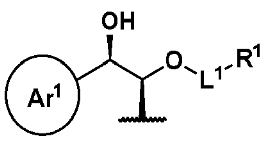 .

**12.** The compound of claim 1, wherein the compound is selected from the group consisting of compounds delineated in

**Tables C1** and **C2,** or a pharmaceutically acceptable salt thereof:

**Table C1**

| Compound | Structure |
|---|---|
| **101** | 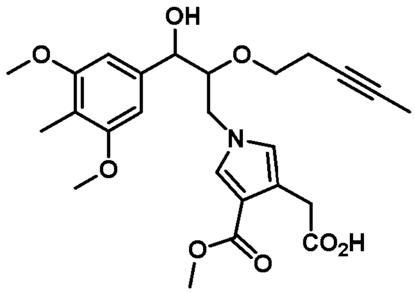 |
| **102** | 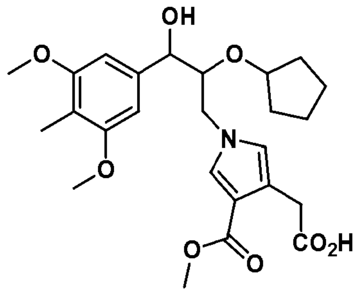 |
| **103** | 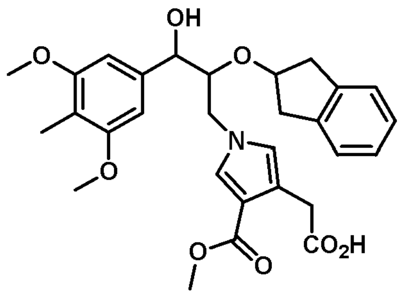 |
| **104** | 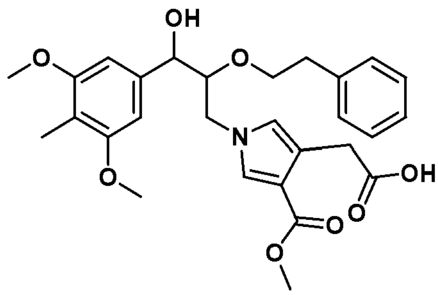 |
| **105** | 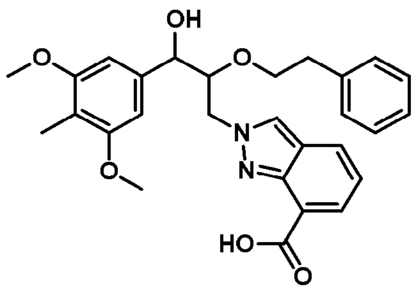 |

(continued)

| Compound | Structure |
|---|---|
| 106 | 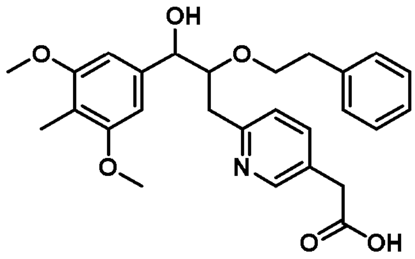 |
| 107 | 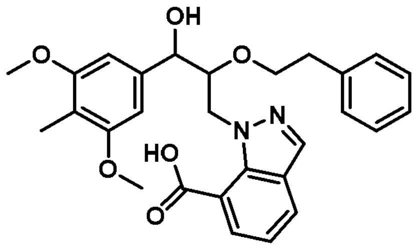 |
| 108 | 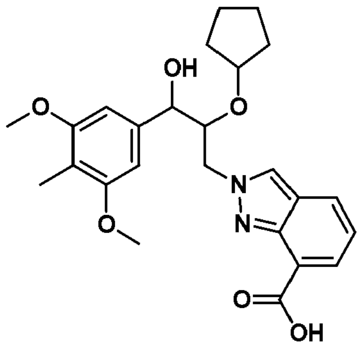 |
| 109 | 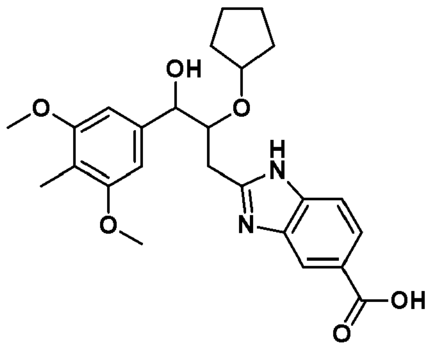 |
| 110 | 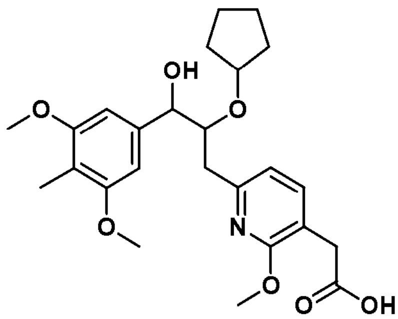 |

(continued)

| Compound | Structure |
|---|---|
| **111** | 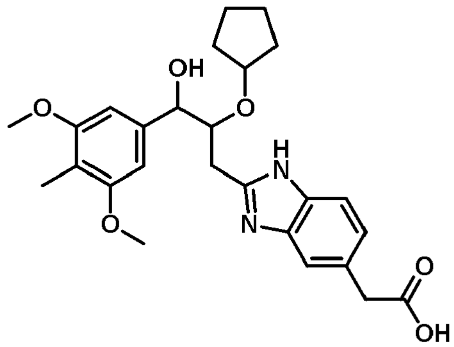 |
| **112** | 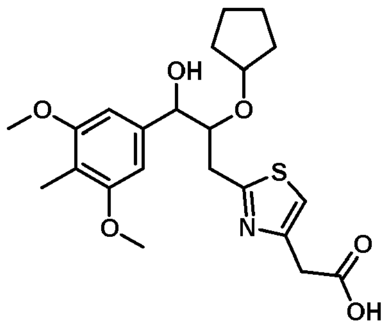 |
| **113** | 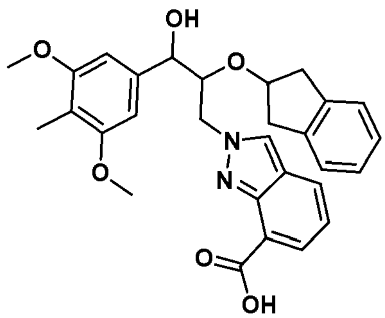 |
| **114** | 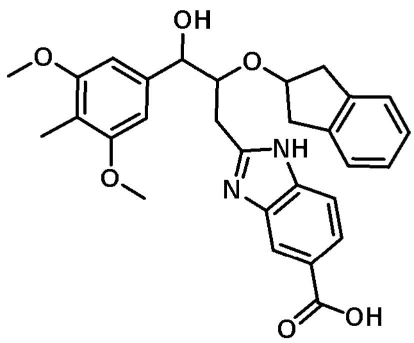 |

(continued)

| Compound | Structure |
|---|---|
| **115** | 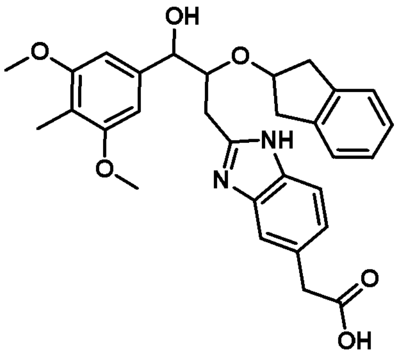 |
| **116** | 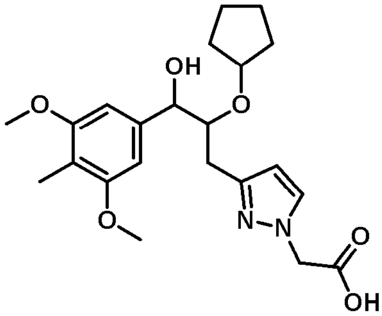 |
| **117** | 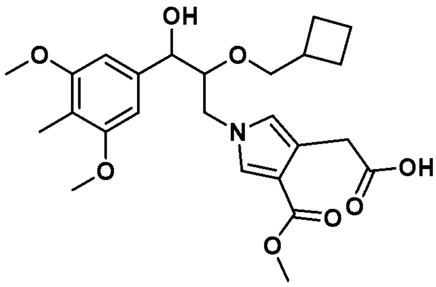 |
| **118** | 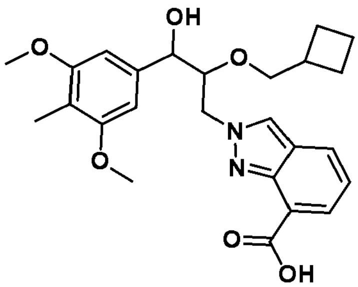 |
| **119** | 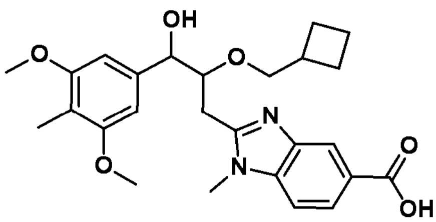 |

(continued)

| Compound | Structure |
|---|---|
| 120 | 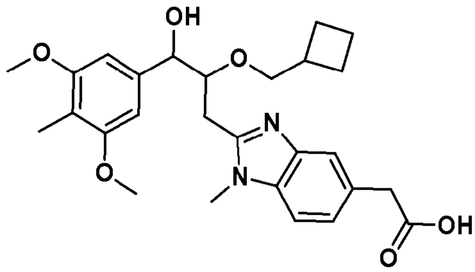 |
| 121 | 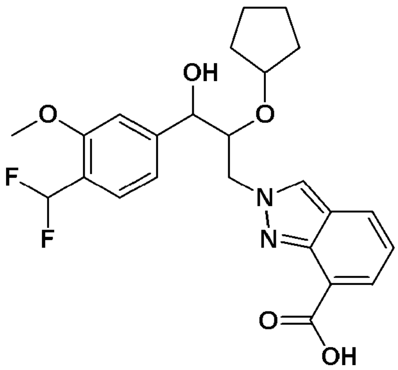 |
| 122 | 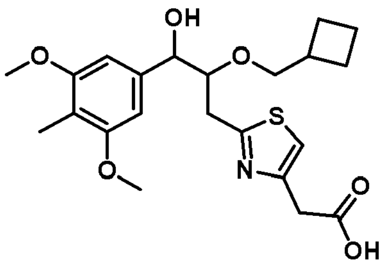 |
| 123 | 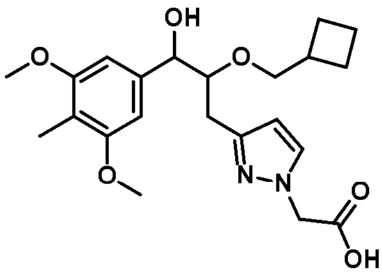 |
| 124 | 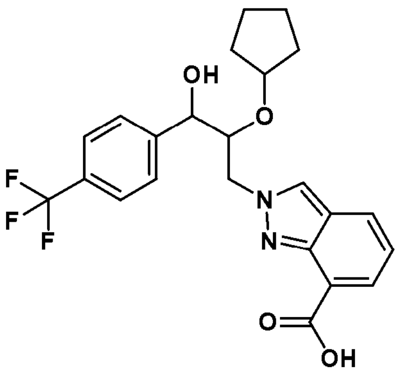 |

(continued)

| Compound | Structure |
|---|---|
| **125** | 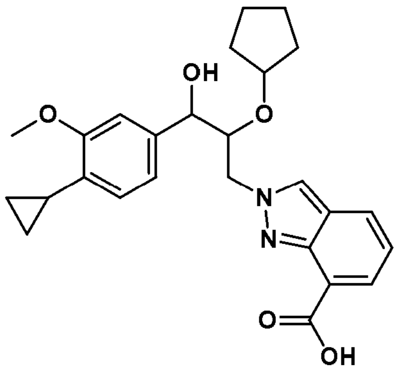 |
| **126** | 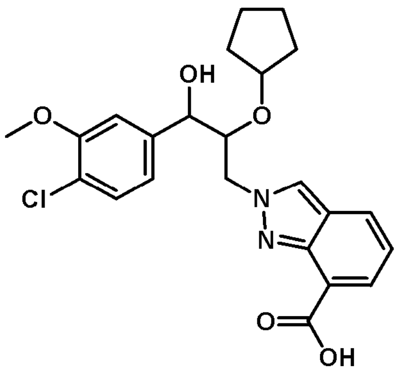 |
| **127** | 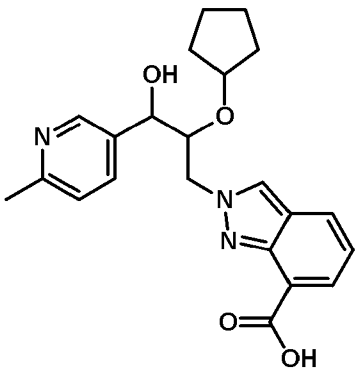 |
| **128** | 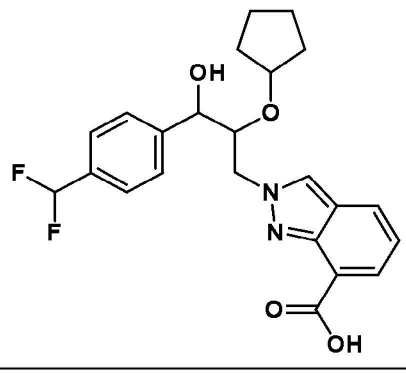 |

(continued)

| Compound | Structure |
|---|---|
| 129 | 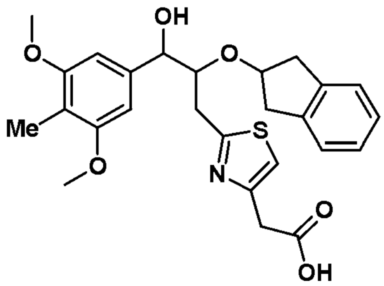 |
| 130 | 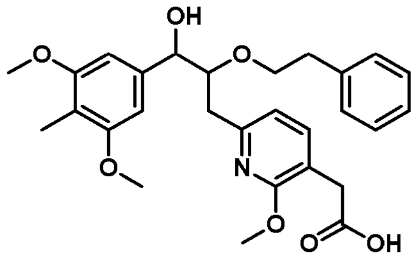 |
| 131 | 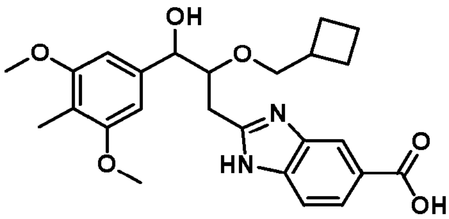 |

**Table C2**

| Compound | Structure |
|---|---|
| 101a | 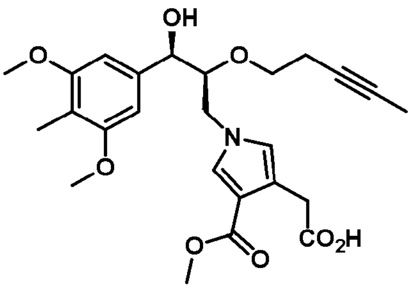 |
| 102a | 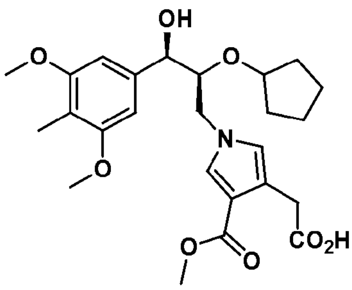 |

(continued)

| Compound | Structure |
|---|---|
| **103a** | 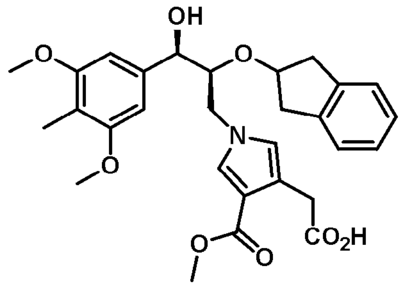 |
| **104a** | 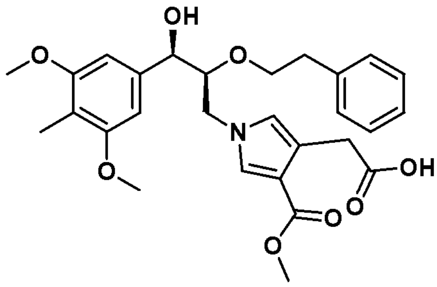 |
| **105a** | 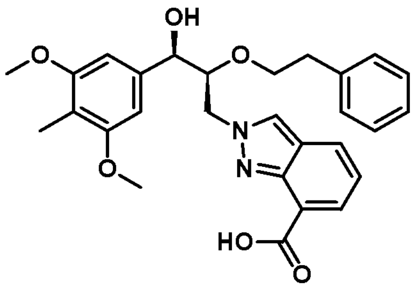 |
| **105b** | 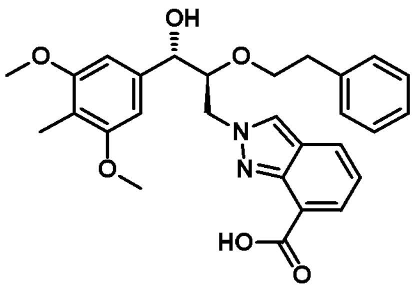 |
| **106a** | 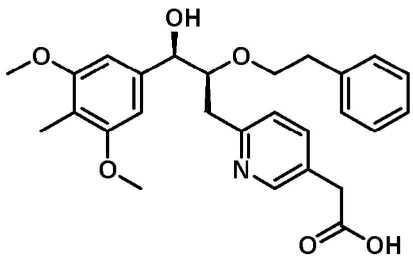 |

(continued)

| Compound | Structure |
|---|---|
| **107** | 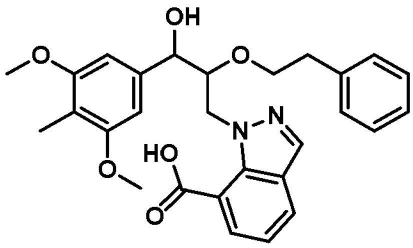 |
| **108a** | 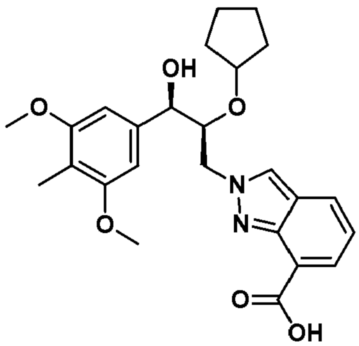 |
| **109a** | 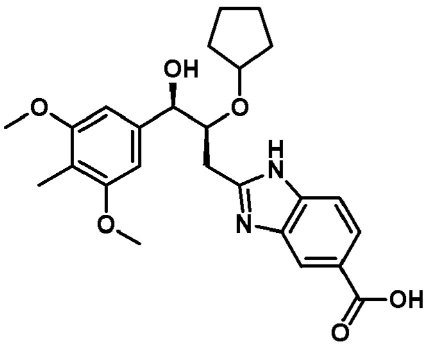 |
| **110a** | 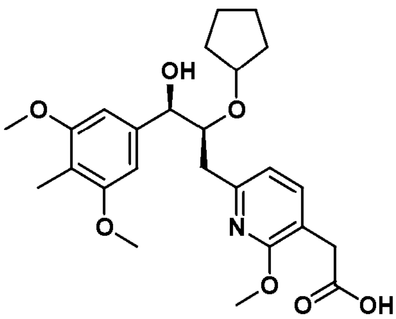 |
| **111a** | 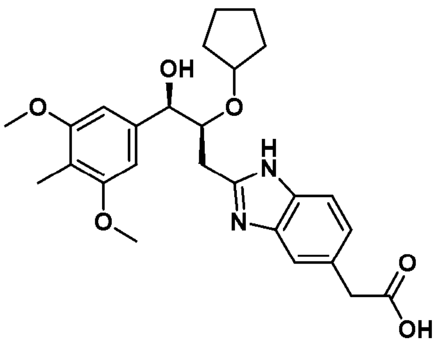 |

(continued)

| Compound | Structure |
|---|---|
| **112a** | 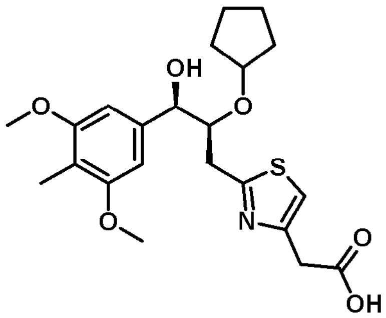 |
| **113a** | 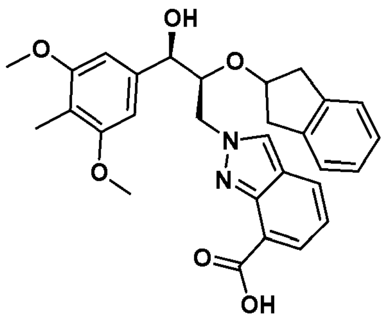 |
| **114a** | 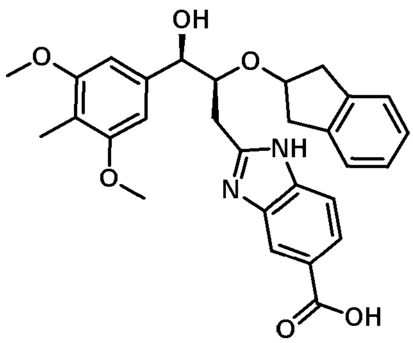 |
| **115a** | 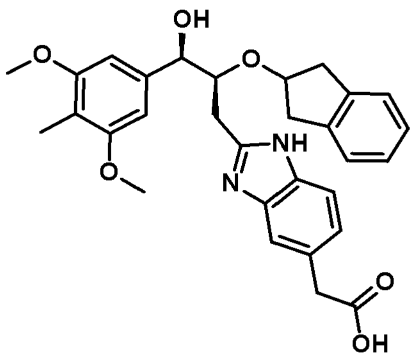 |
| **116a** | 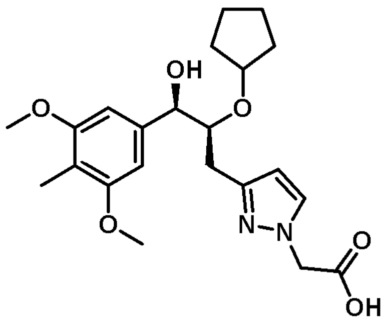 |

(continued)

| Compound | Structure |
|---|---|
| 117a | 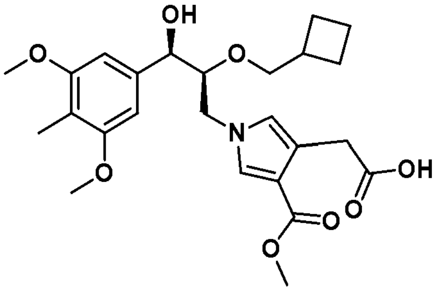 |
| 118a | 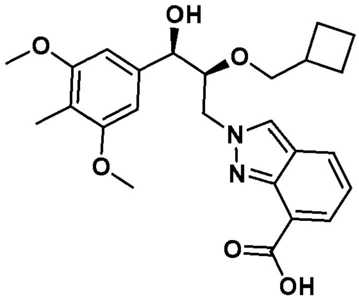 |
| 119a | 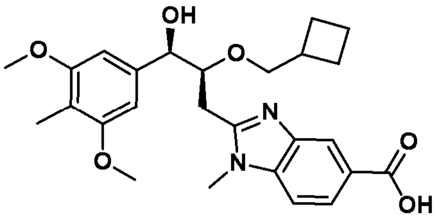 |
| 120a | 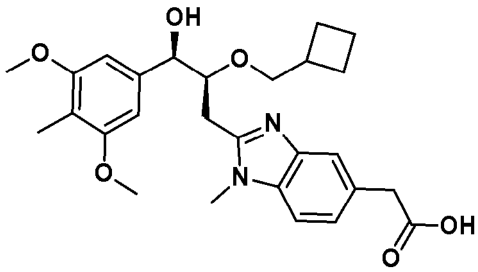 |
| 121a | 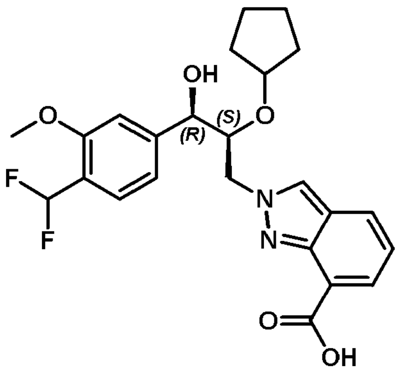 |

(continued)

| Compound | Structure |
|---|---|
| **122a** | 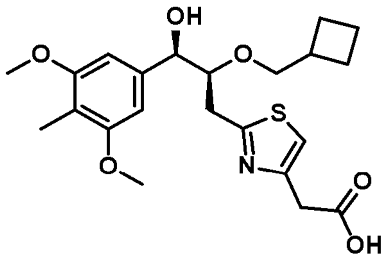 |
| **123a** | 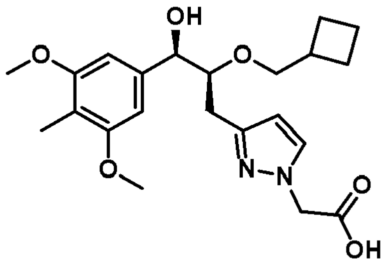 |
| **124a** | 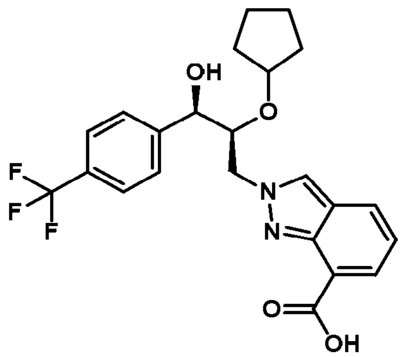 |
| **125a** | 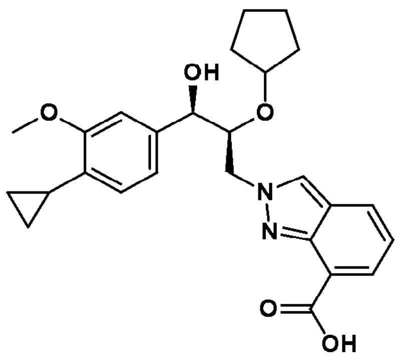 |
| **126a** | 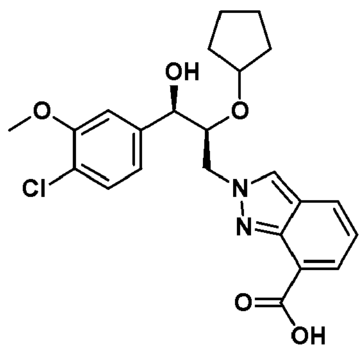 |

(continued)

| Compound | Structure |
|---|---|
| 127a | 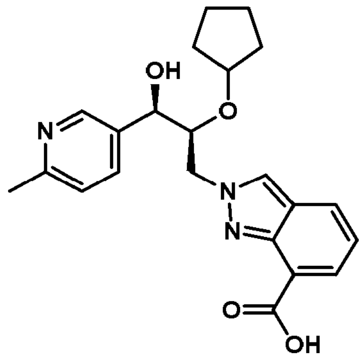 |
| 128a | 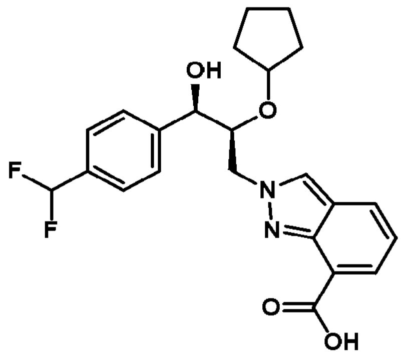 |
| 129a | 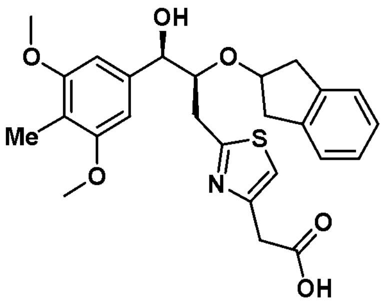 |
| 130a | 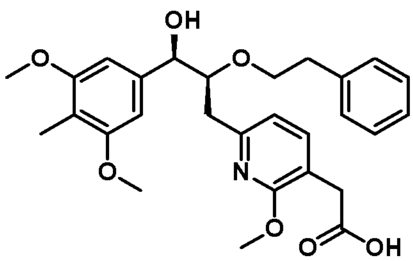 |
| 131a | 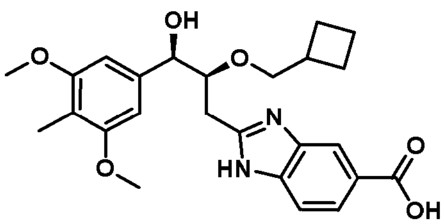 |

(continued)

| Compound | Structure |
|---|---|
| **132** | 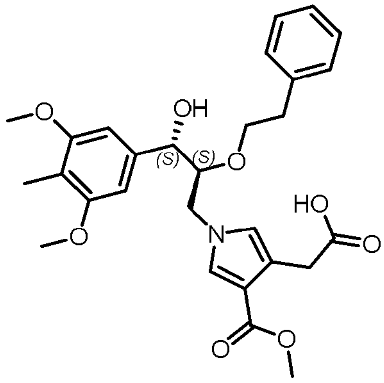 |
| **133** | 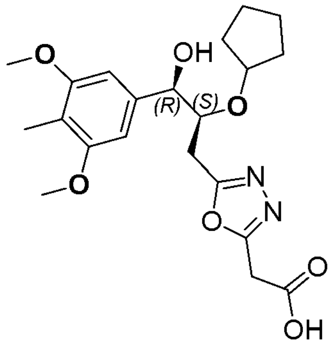 |
| **134** | 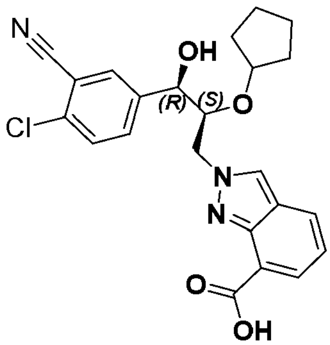 |
| **135** | 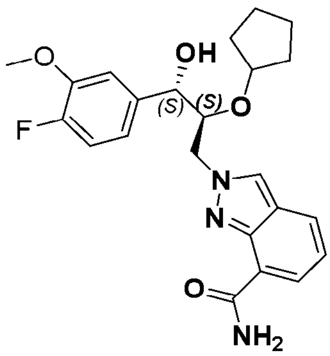 |

(continued)

| Compound | Structure |
|---|---|
| **136** | 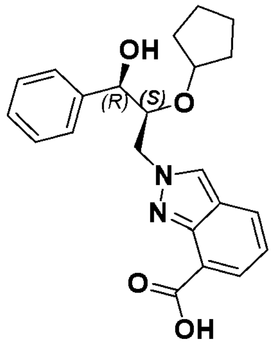 |
| **137** | 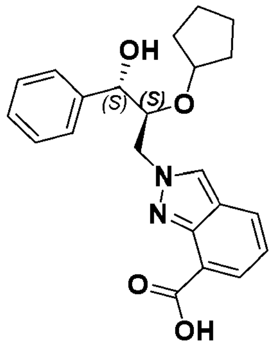 |
| **138** | 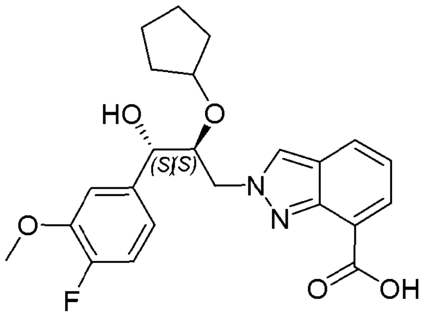 |
| **139** | 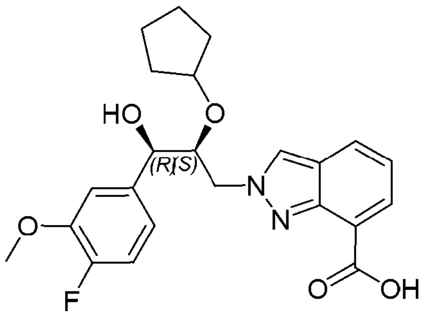 |

(continued)

| Compound | Structure |
|---|---|
| 140 | 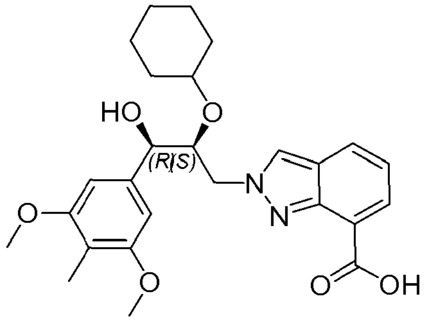 |
| 141 | 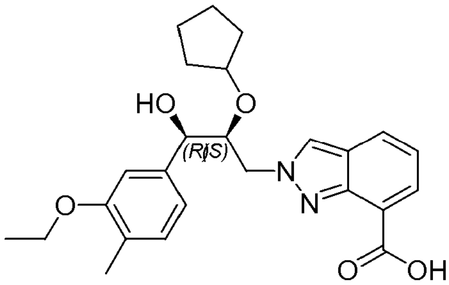 |
| 142 | 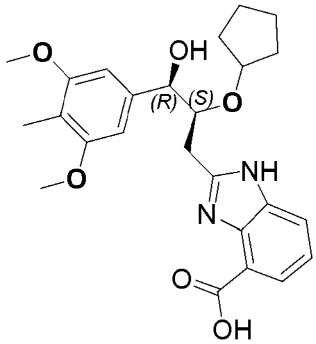 |
| 143 | 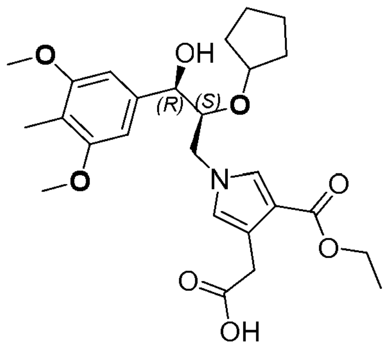 |
| 144 | 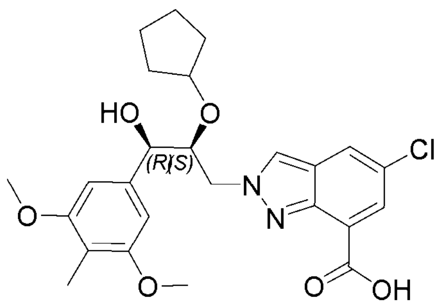 |

(continued)

| Compound | Structure |
|---|---|
| 145 | 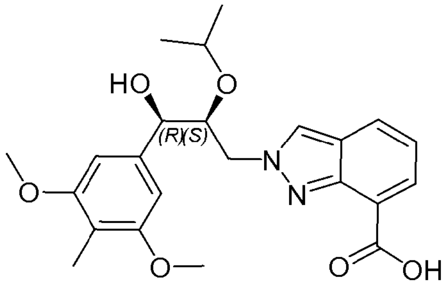 |
| 146 | 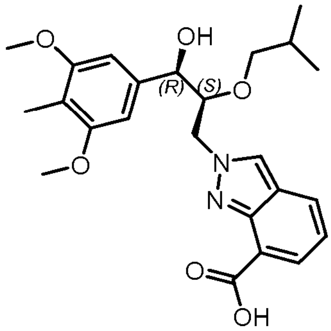 |
| 147 | 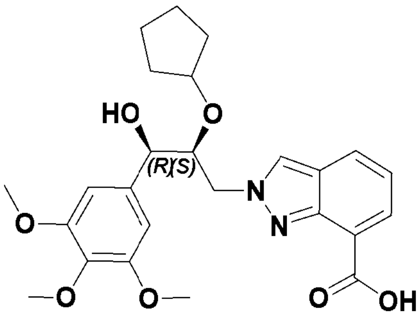 |
| 148 | 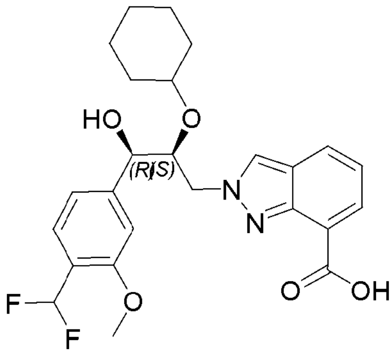 |

(continued)

| Compound | Structure |
|---|---|
| **149** | 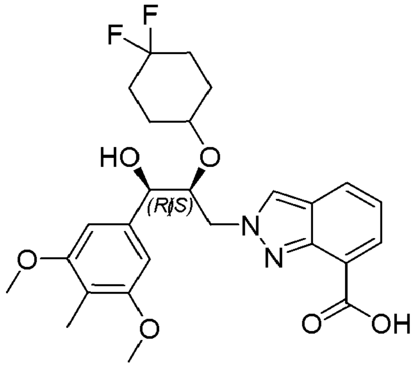 |
| **150** | 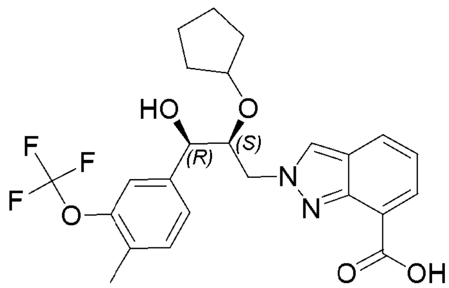 |
| **151** | 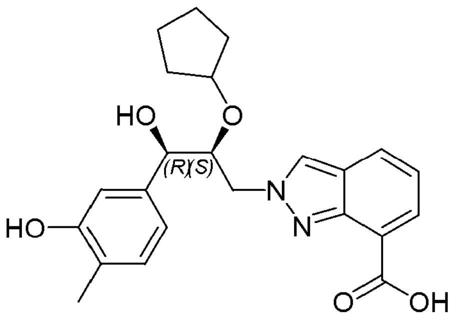 |
| **152** | 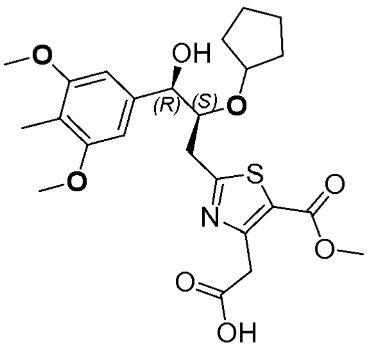 |
| **153** | 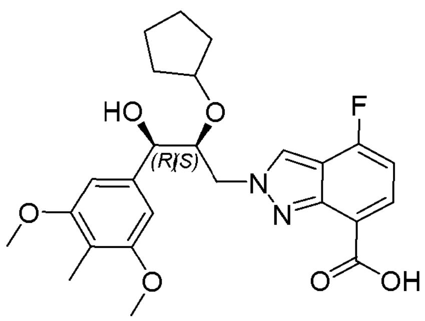 |

(continued)

| Compound | Structure |
|---|---|
| **154** | 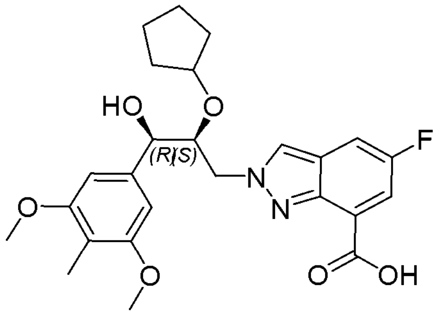 |
| **155** | 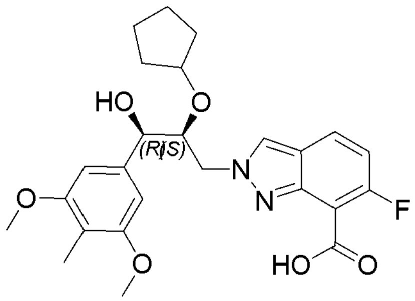 |
| **156** | 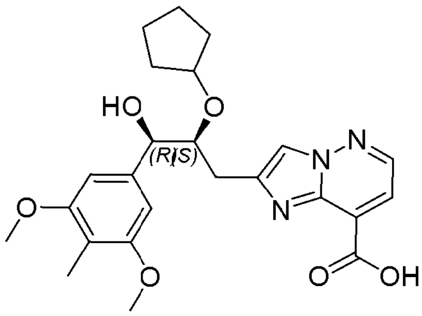 |
| **157** | 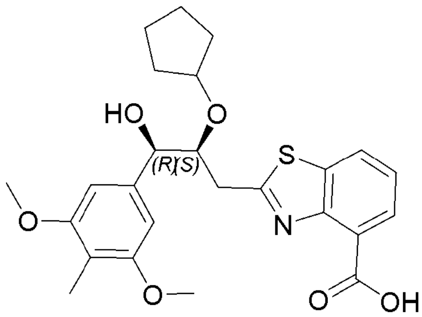 |
| **158** | 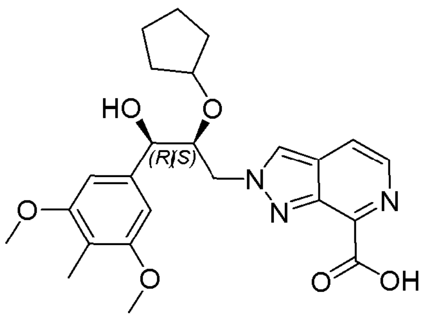 |

(continued)

| Compound | Structure |
|---|---|
| 159 | 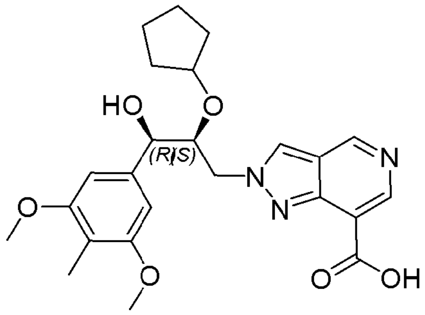 |
| 160 | 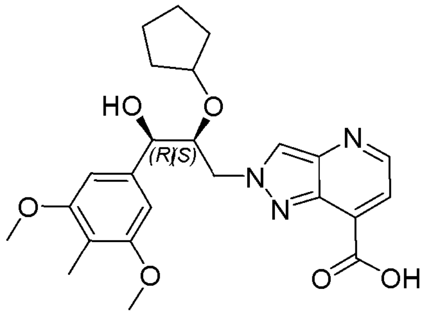 |
| 161 | 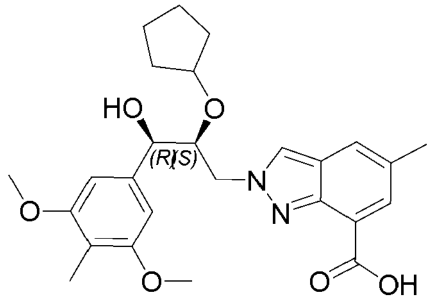 |
| 162 | 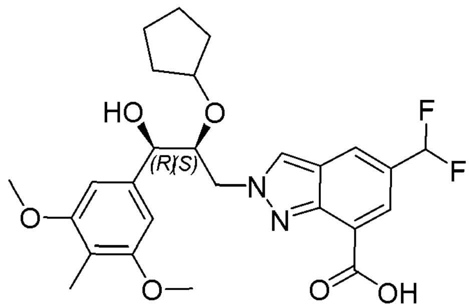 |
| 163 | 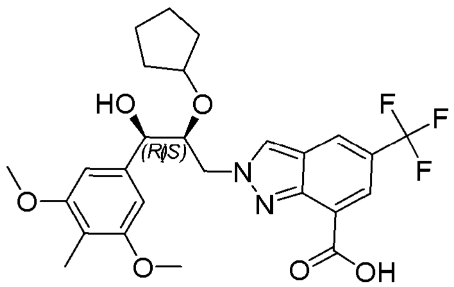 |

(continued)

| Compound | Structure |
|---|---|
| **164** | 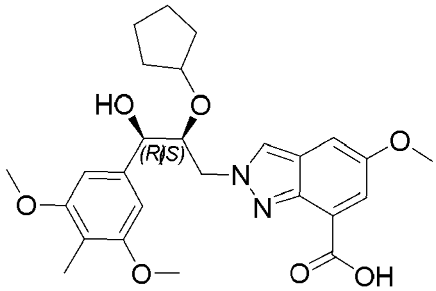 |
| **165** | 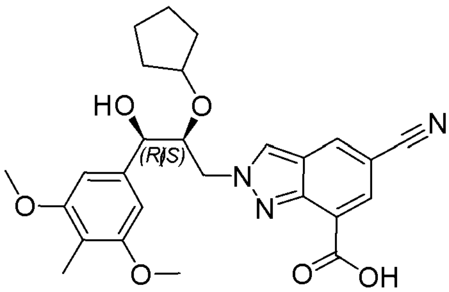 |
| **166** | 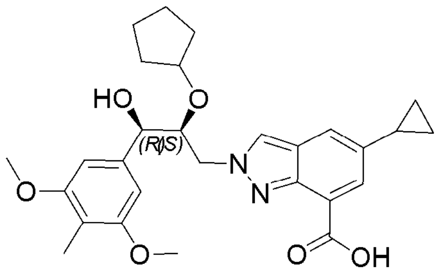 |
| **167** | 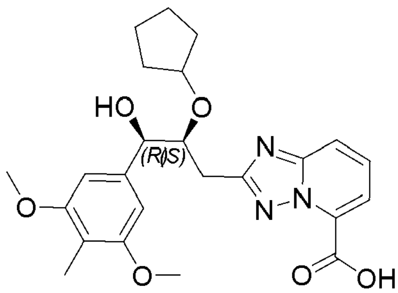 |
| **168** | 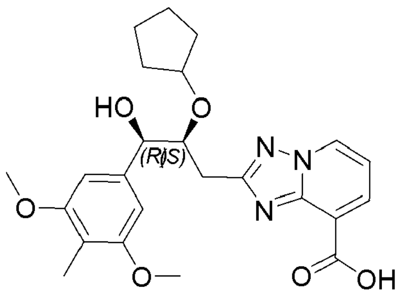 |

(continued)

| Compound | Structure |
|---|---|
| **169** | 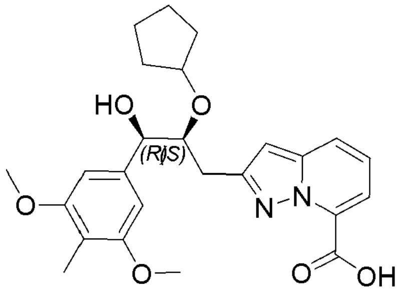 |
| **170** | 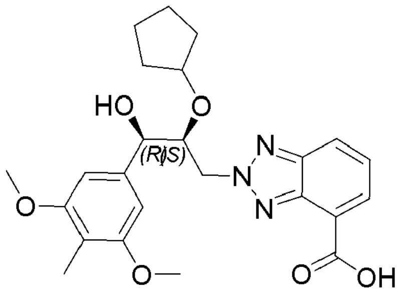 |
| **171** | 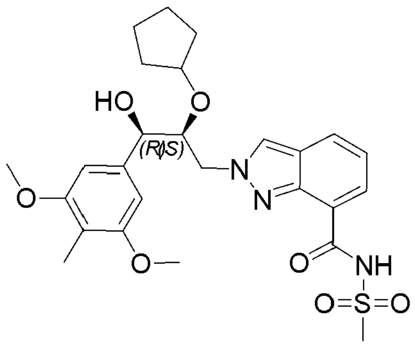 |
| **172** | 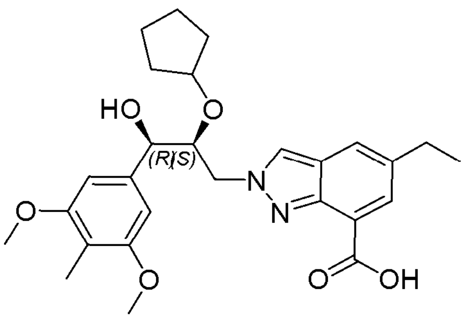 |
| **173** | 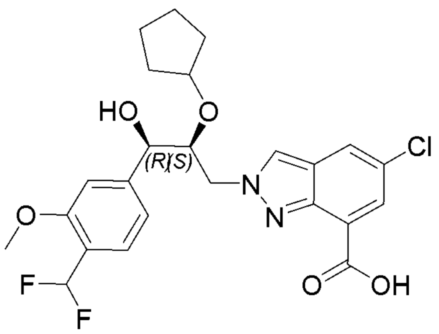 |

(continued)

| Compound | Structure |
|---|---|
| **174** | 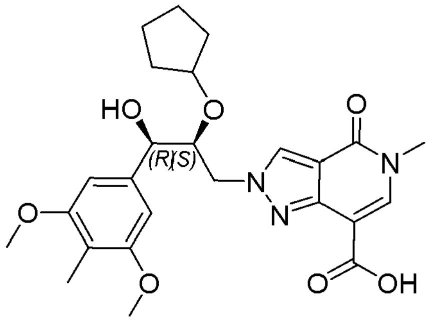 |
| **175** | 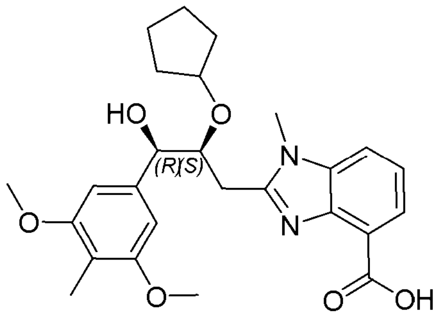 |
| **176** | 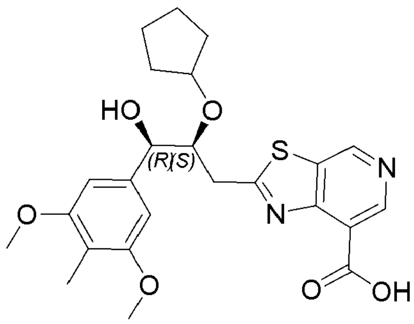 |
| **177** | 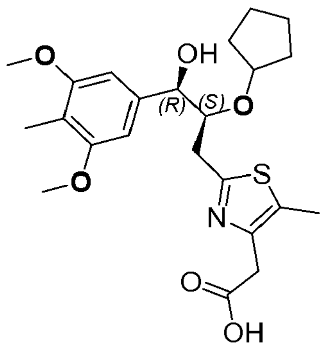 |

(continued)

| Compound | Structure |
|---|---|
| **178** | 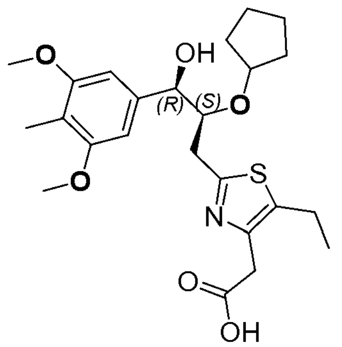 |
| **179** | 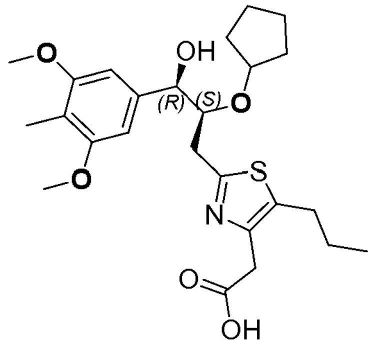 |
| **180** | 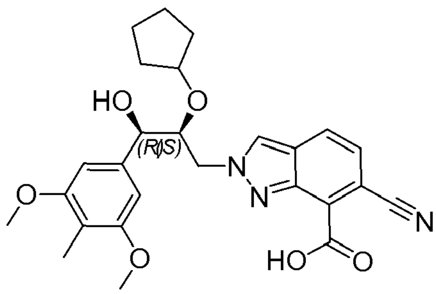 |
| **181** | 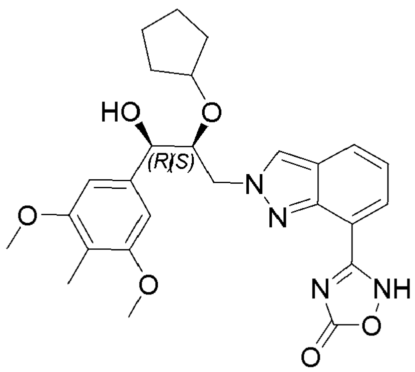 |

(continued)

| Compound | Structure |
|---|---|
| **182** | 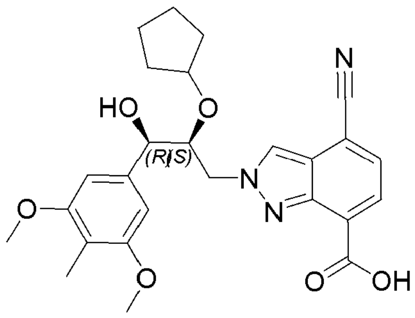 |
| **183** | 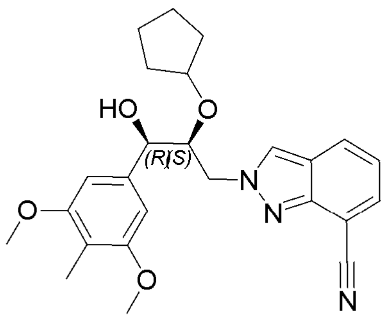 |
| **184** | 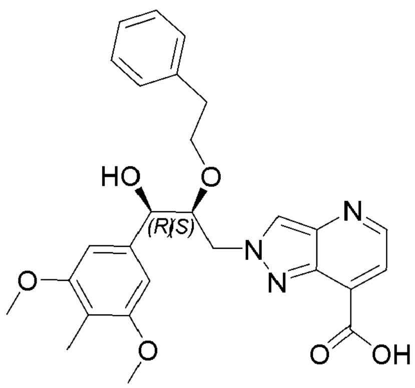 |
| **185** | 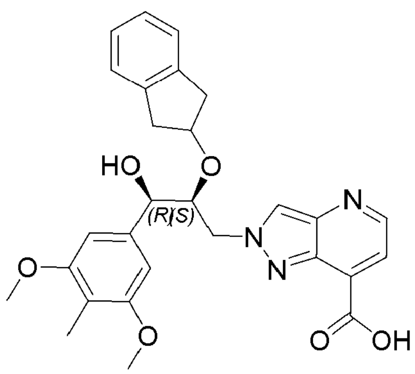 |

(continued)

| Compound | Structure |
|---|---|
| **186** | 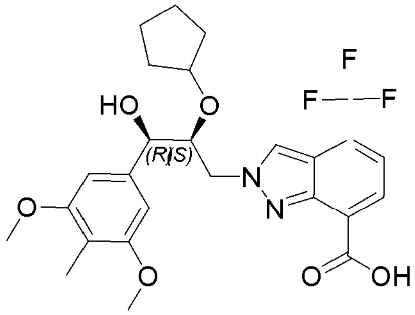 |
| **187** | 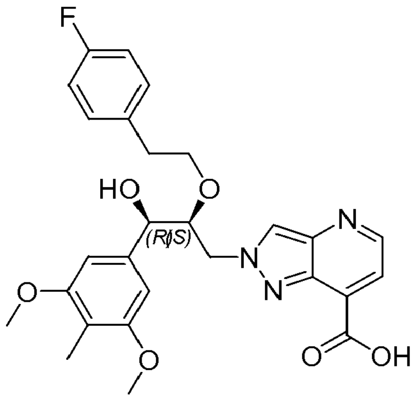 |
| **188** | 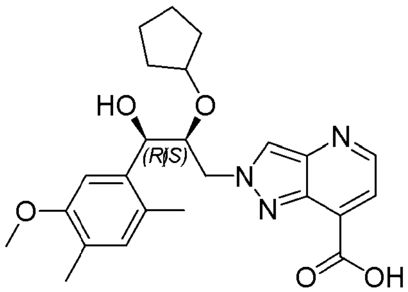 |
| **189** | 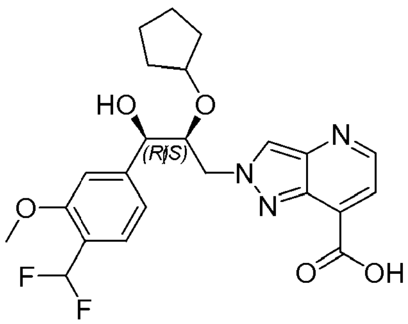 |
| **190** | 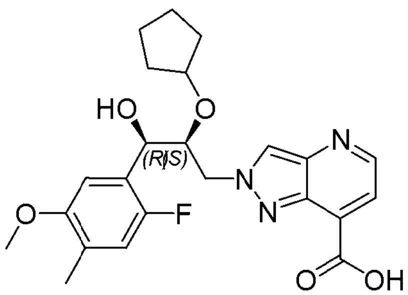 |

(continued)

| Compound | Structure |
|---|---|
| 191 | 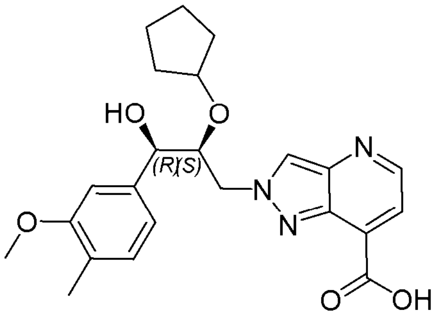 |
| 192 | 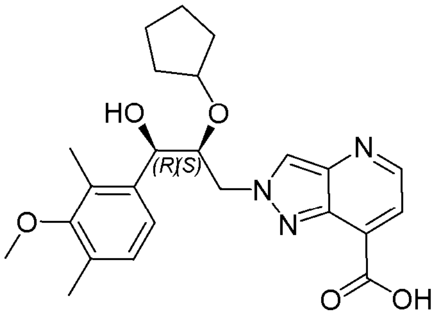 |
| 193 | 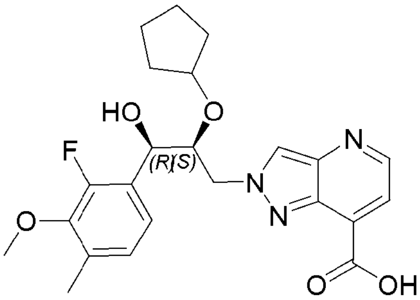 |
| 194 | 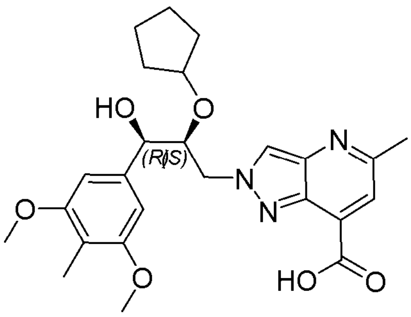 |
| 195 | 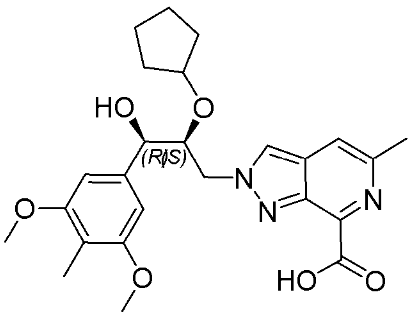 |

(continued)

| Compound | Structure |
|---|---|
| **196** | 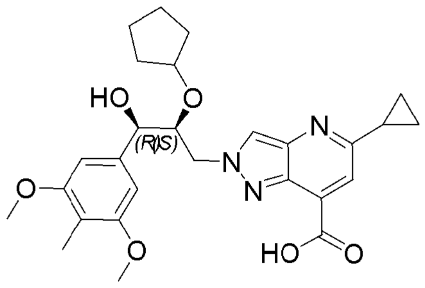 |
| **197** | 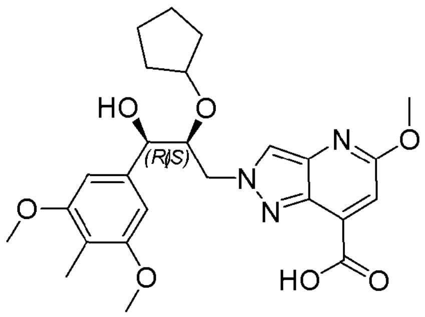 |
| **198** | 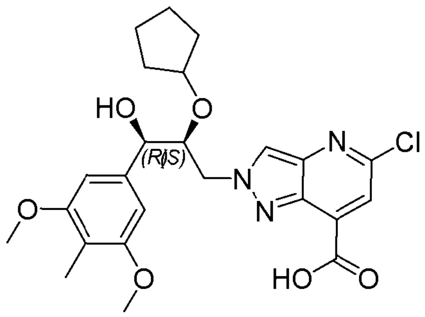 |
| **199** | 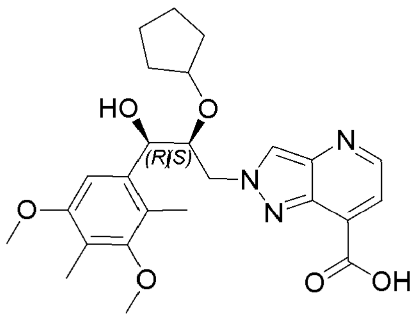 |
| **200** | 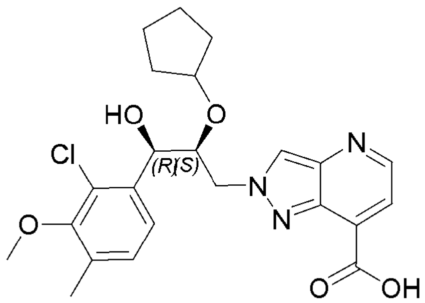 |

(continued)

| Compound | Structure |
|---|---|
| **201** | 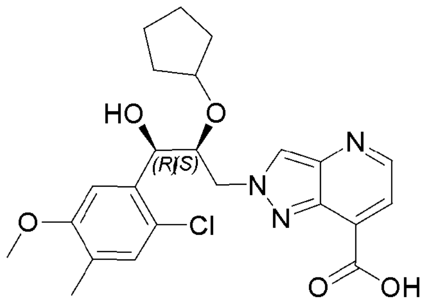 |
| **202** | 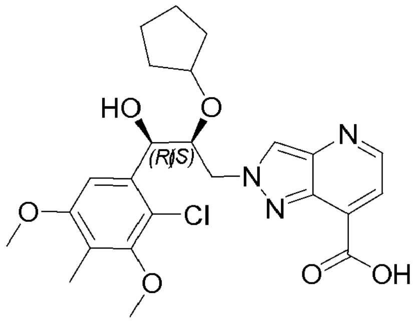 |
| **203** | 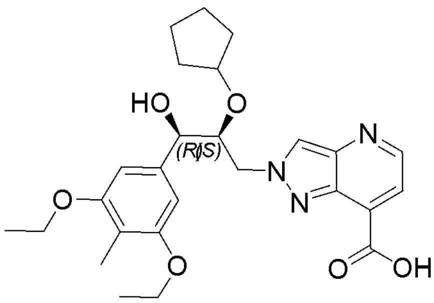 |
| **204** | 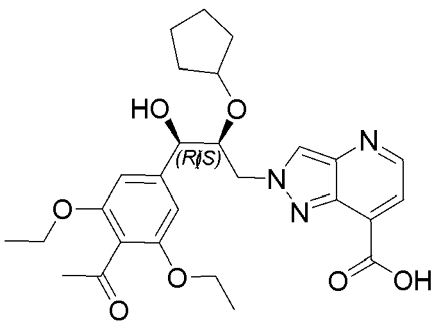 |
| **205** | 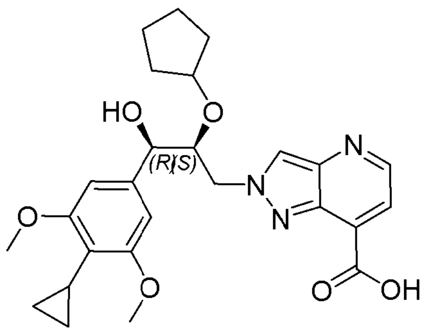 |

(continued)

| Compound | Structure |
|---|---|
| **206** | 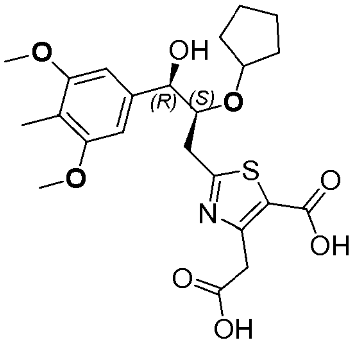 |
| **207** | 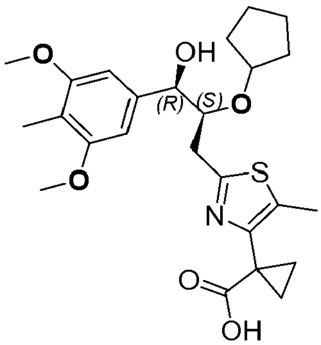 |
| **208** | 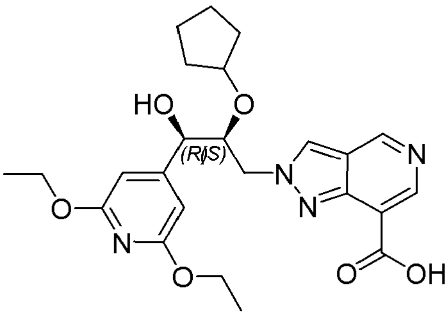 |
| **209** | 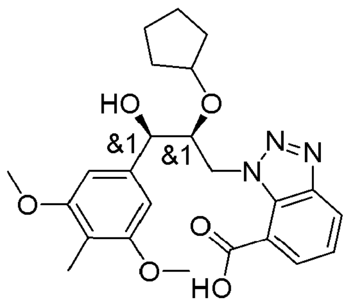 |

(continued)

| Compound | Structure |
|---|---|
| **210** | 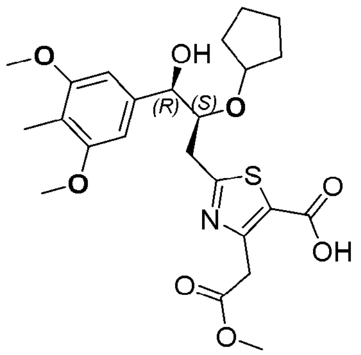 |
| **211** | 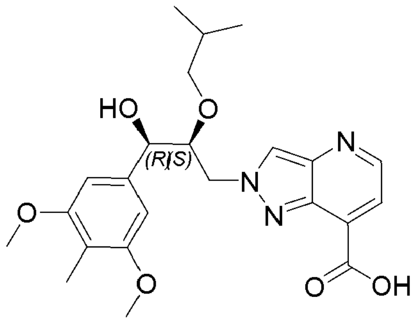 |
| **212** | 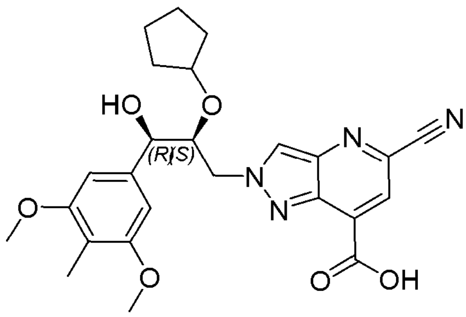 |
| **213** | 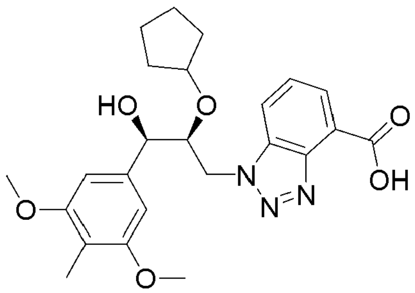 |

(continued)

| Compound | Structure |
|---|---|
| **214** | 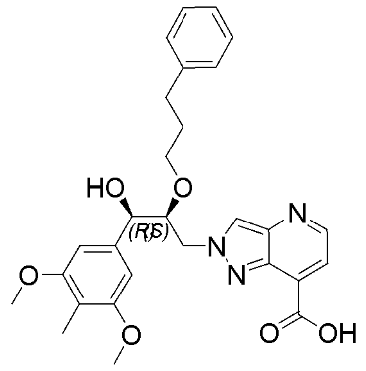 |
| **215** | 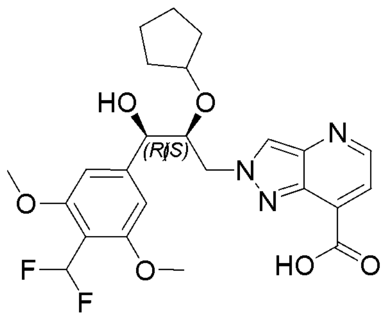 |
| **216** | 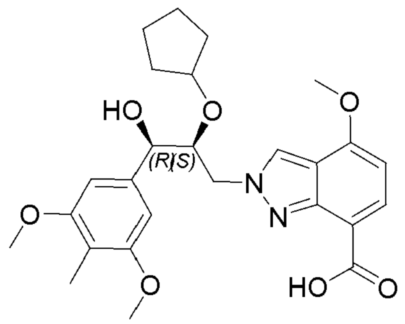 |
| **217** | 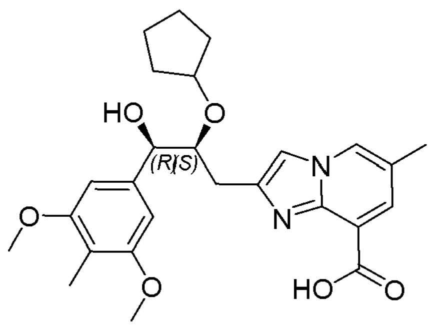 |

(continued)

| Compound | Structure |
|---|---|
| **218** | 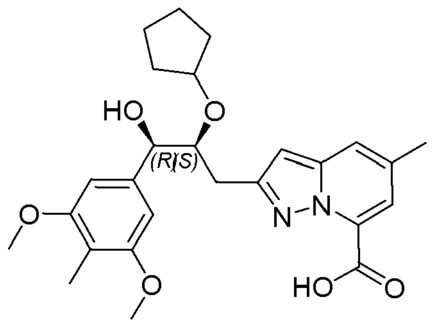 |
| **219** | 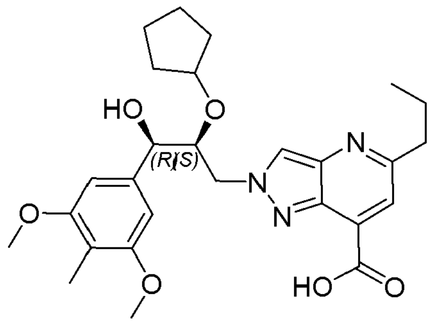 |
| **220** | 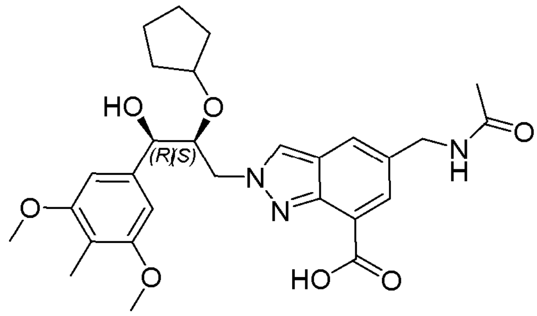 |
| **221** | 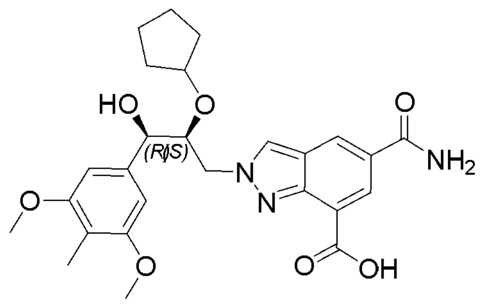 |
| **222** | 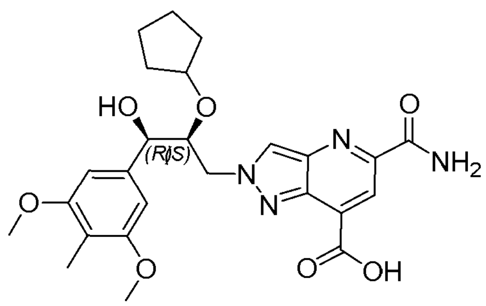 |

(continued)

| Compound | Structure |
|---|---|
| **223** | 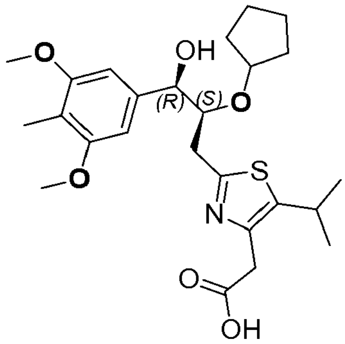 |
| **224** | 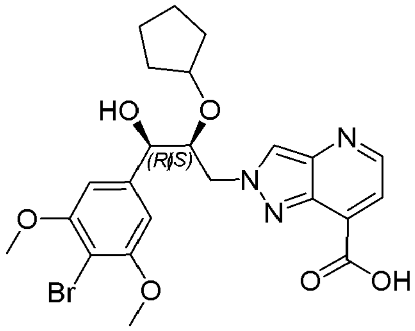 |
| **225** | 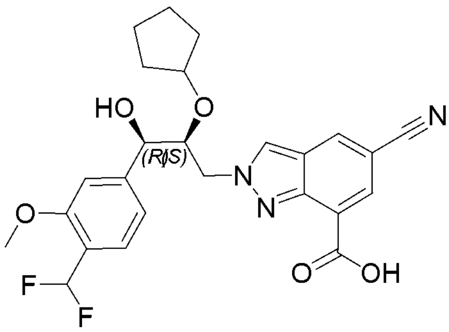 |
| **226** | 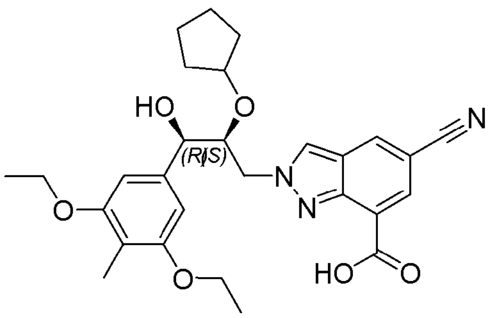 |
| **227** | 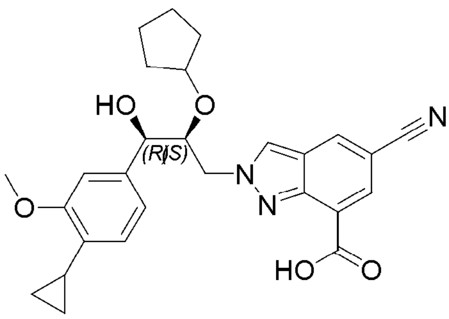 |

(continued)

| Compound | Structure |
|---|---|
| **228** | 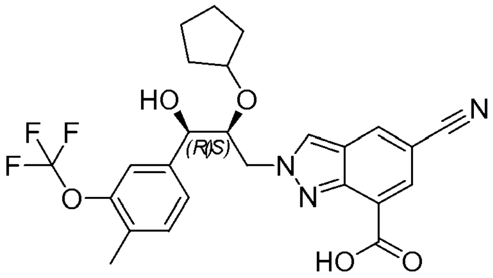 |
| **229** | 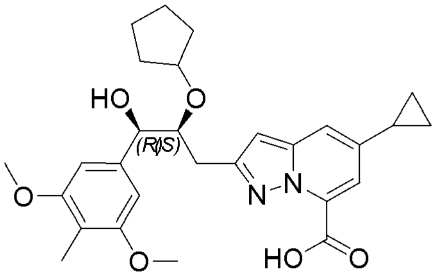 |
| **230** | 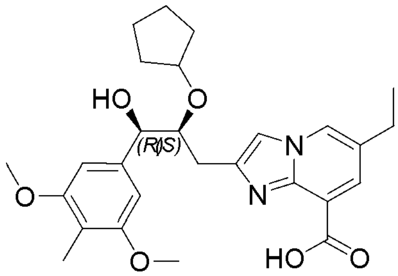 |
| **231** | 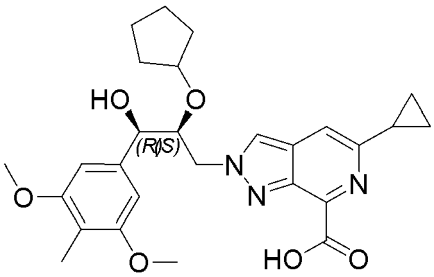 |
| **232** | 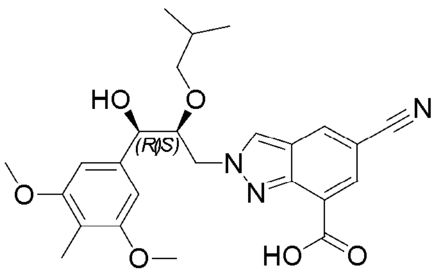 |

(continued)

| Compound | Structure |
|---|---|
| 233 | 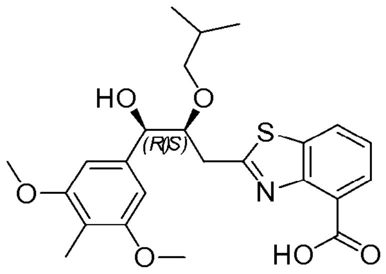 |
| 234 | 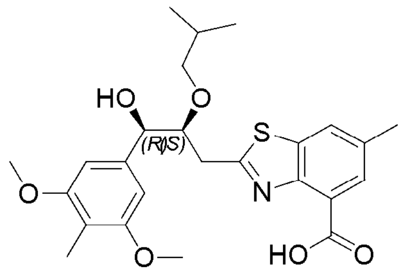 |
| 235 | 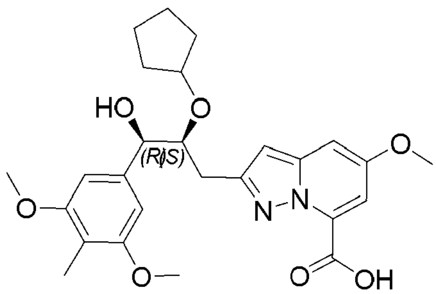 |
| 236 | 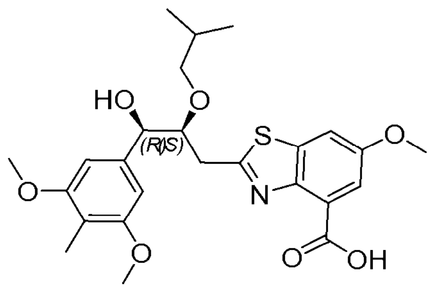 |
| 237 | 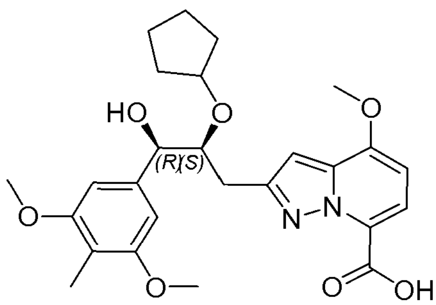 |

(continued)

| Compound | Structure |
|---|---|
| **238** | 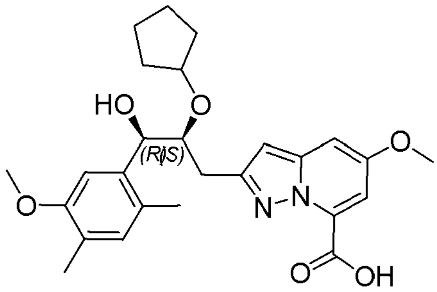 |
| **239** | 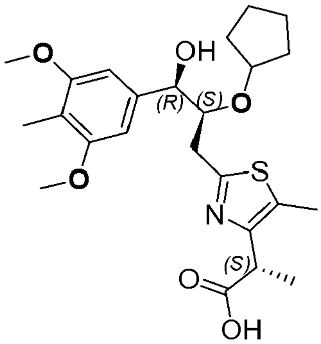 |
| **240** | 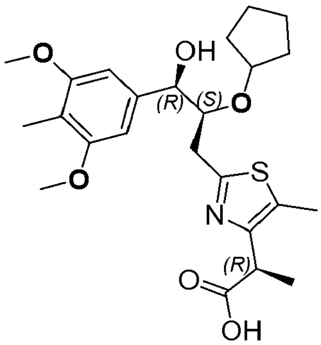 |
| **241** | 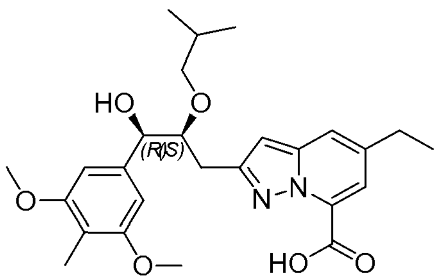 |
| **242** | 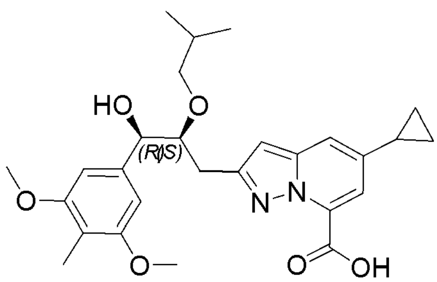 |

(continued)

| Compound | Structure |
|---|---|
| **243** | 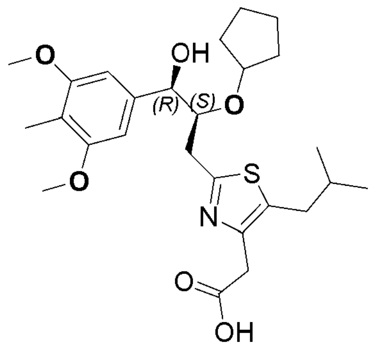 |
| **244** | 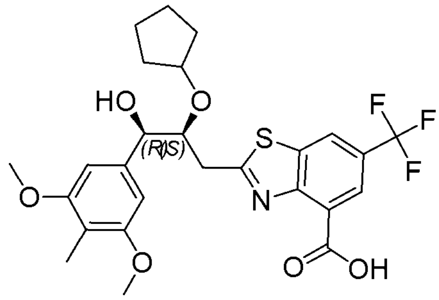 |
| **245** | 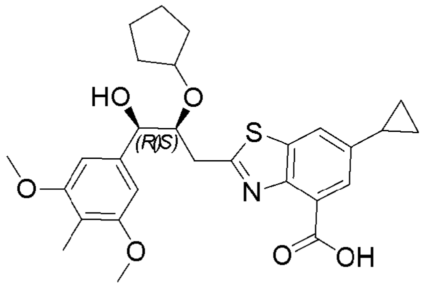 |
| **246** | 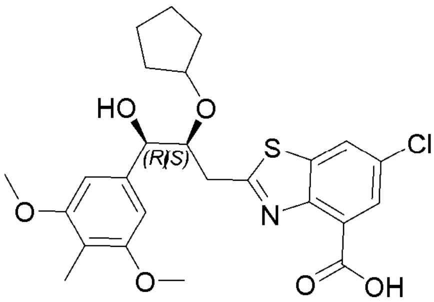 |
| **247** | 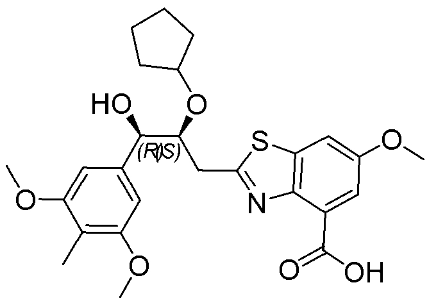 |

(continued)

| Compound | Structure |
|---|---|
| 248 | 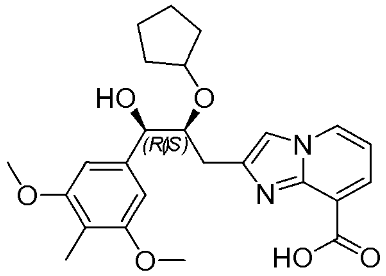 |
| 249 | 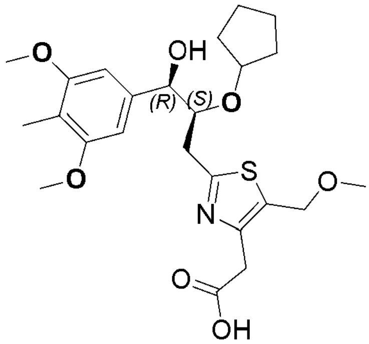 |
| 250 | 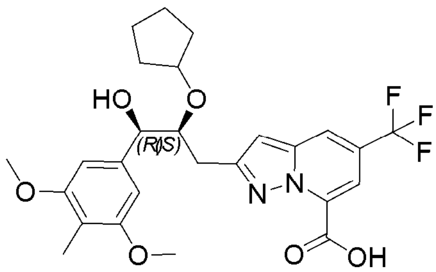 |
| 251 | 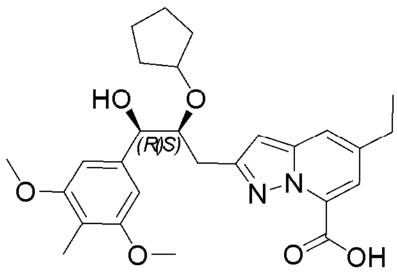 |
| 252 | 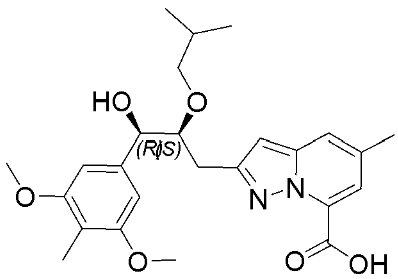 |

(continued)

| Compound | Structure |
|---|---|
| 253 | 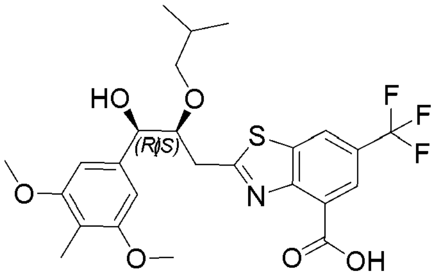 |
| 254 | 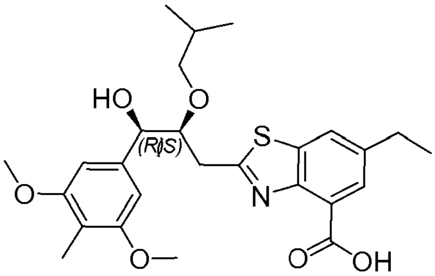 |
| 255 | 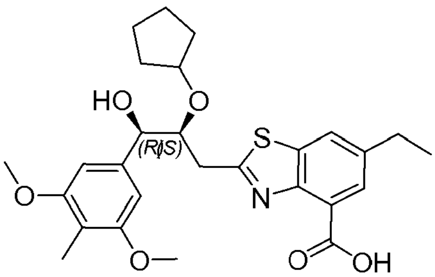 |
| 256 | 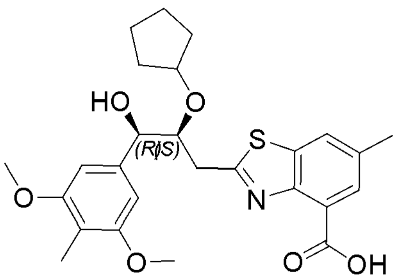 |
| 257 | 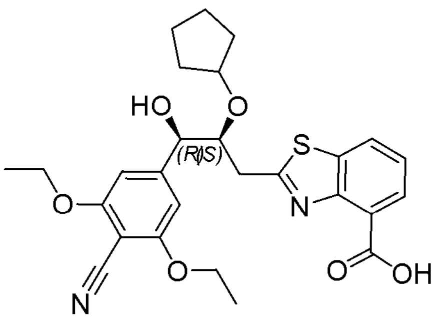 |

(continued)

| Compound | Structure |
|---|---|
| 258 | 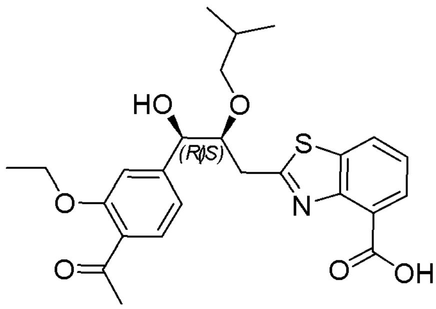 |
| 259 | 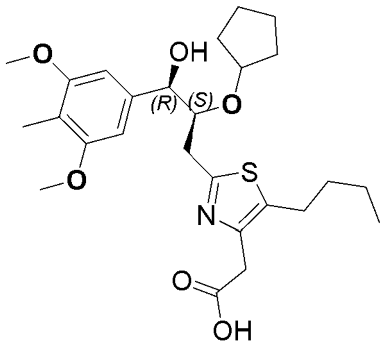 |
| 260 | 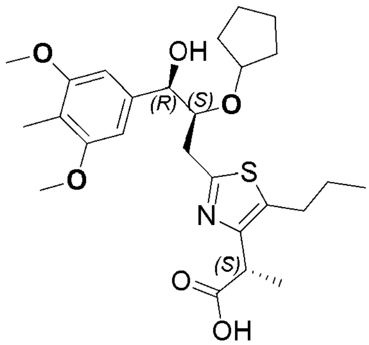 |
| 261 | 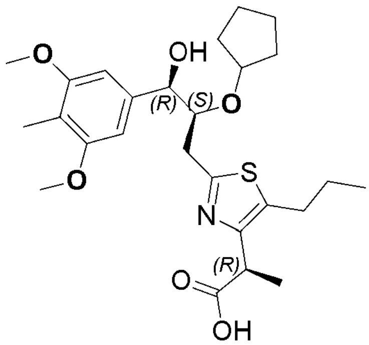 |
| 262 | 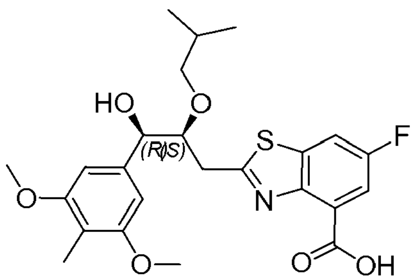 |

(continued)

| Compound | Structure |
|---|---|
| 263 | 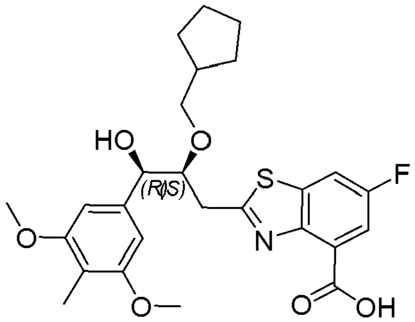 |
| 264 | 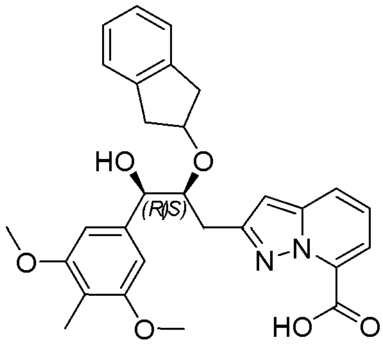 |
| 265 | 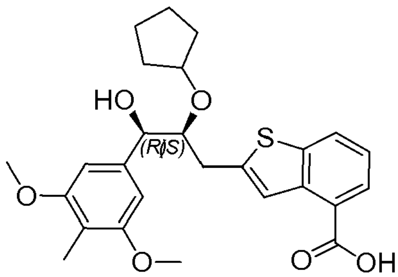 |
| 266 | 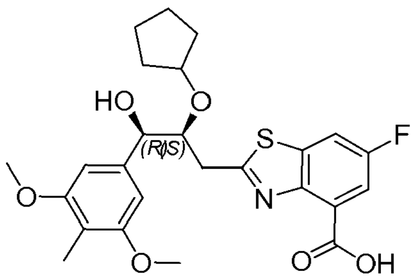 |
| 267 | 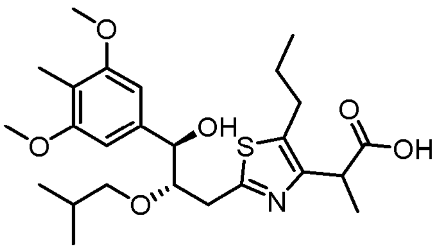 |

(continued)

| Compound | Structure |
|---|---|
| **268** | 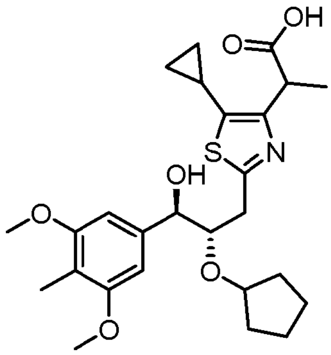 |
| **269** | 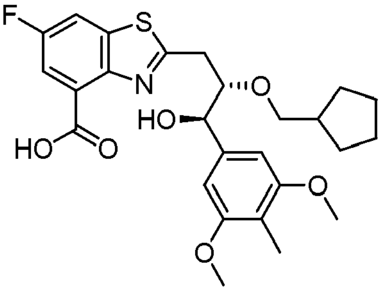 |
| **270** | 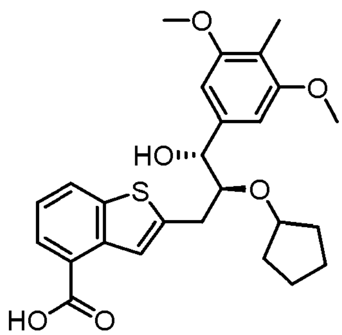 |
| **271** | 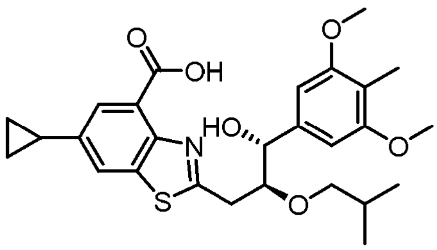 |
| **272** | 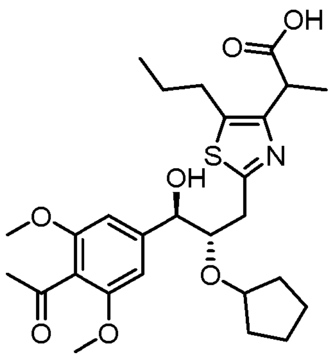 |

(continued)

| Compound | Structure |
|---|---|
| **273** | 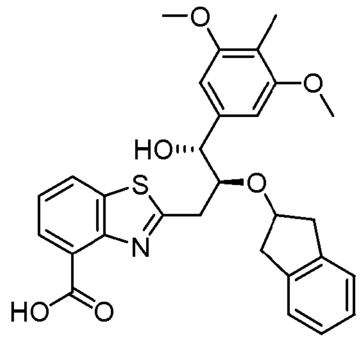 |
| **274** | 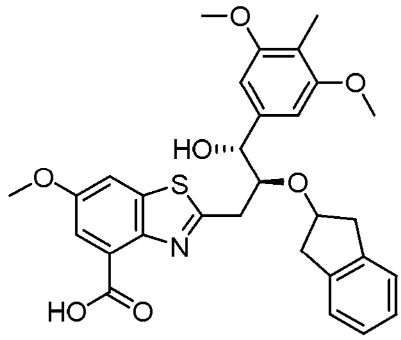 |
| **275** | 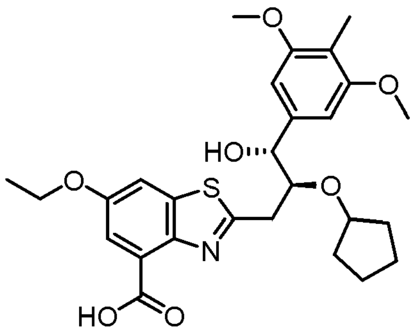 |
| **276** | 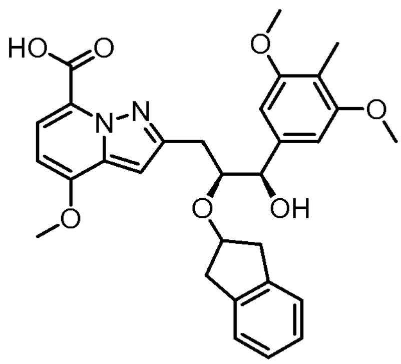 |

(continued)

| Compound | Structure |
|---|---|
| **277** | 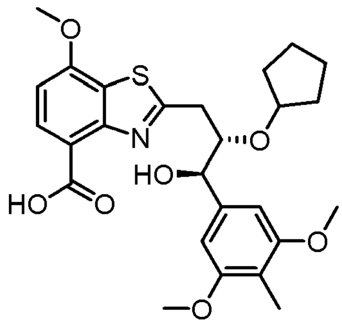 |
| **278** | 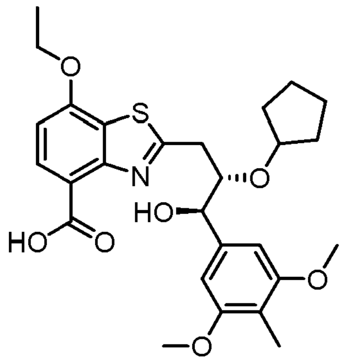 |
| **279** | 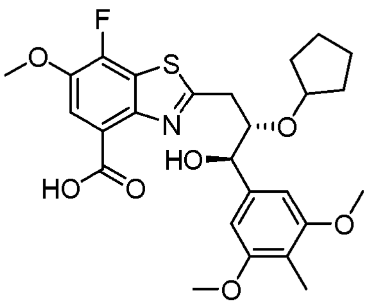 |
| **280** | 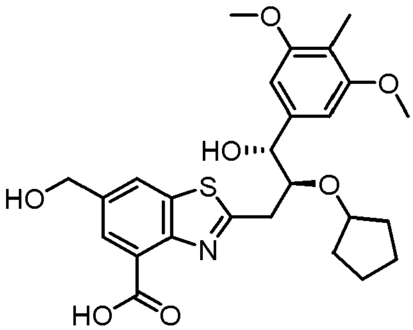 |

(continued)

| Compound | Structure |
|---|---|
| **281** | 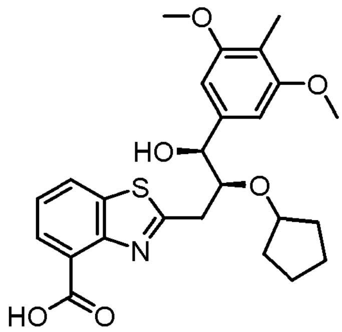 |
| **282** | 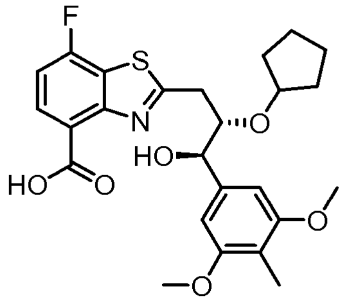 |
| **283** | 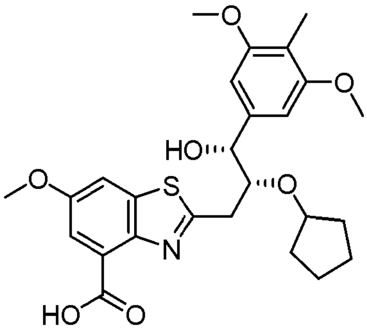 |
| **284** | 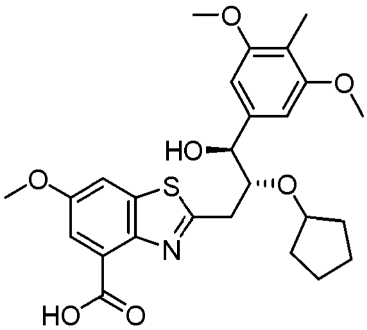 |

(continued)

| Compound | Structure |
|---|---|
| **285** | 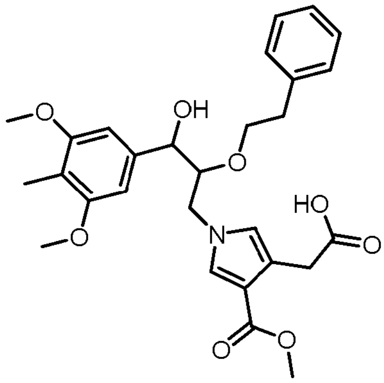 |
| **286** | 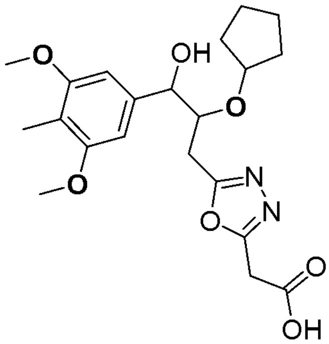 |
| **287** | 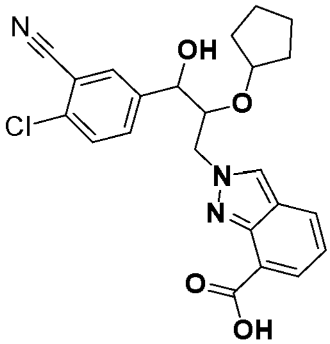 |
| **288** | 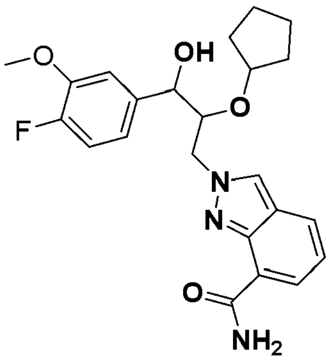 |

(continued)

| Compound | Structure |
|---|---|
| **289** | 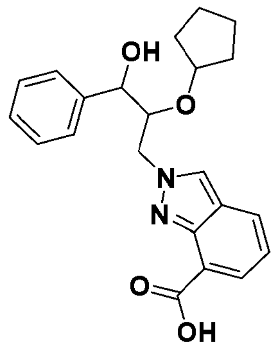 |
| **290** | 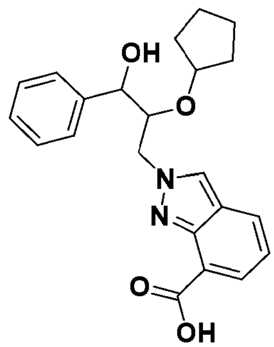 |
| **291** | 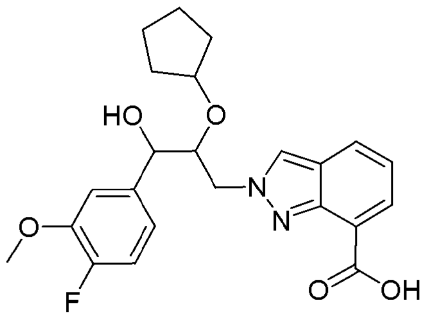 |
| **292** | 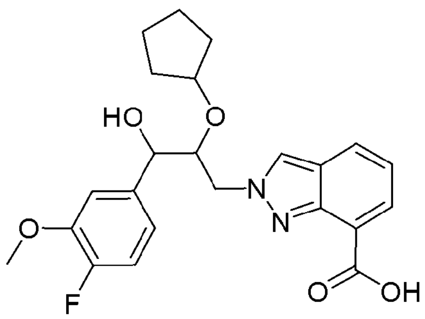 |
| **293** | 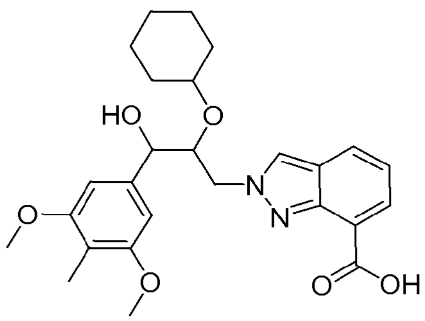 |

(continued)

| Compound | Structure |
|---|---|
| **294** | 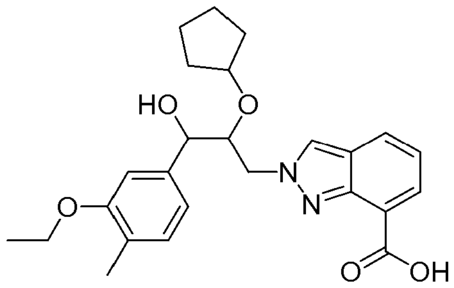 |
| **295** | 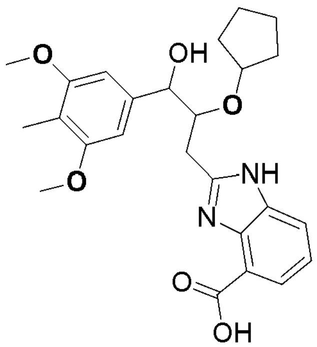 |
| **296** | 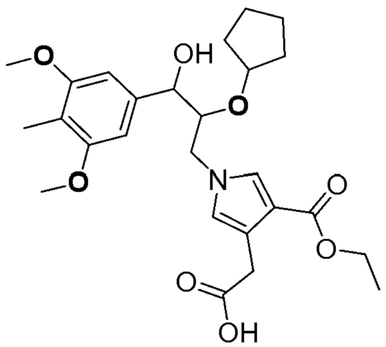 |
| **297** | 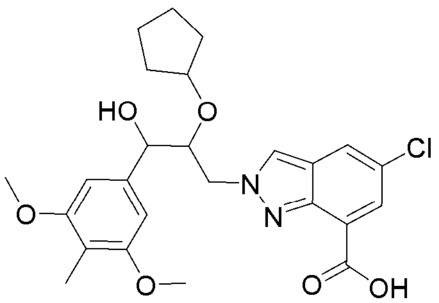 |
| **298** | 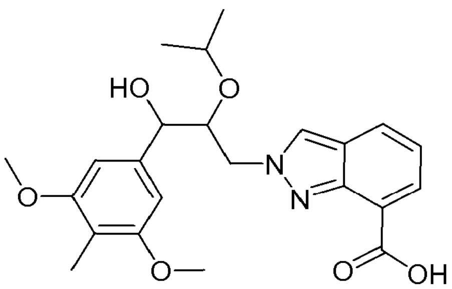 |

(continued)

| Compound | Structure |
|---|---|
| **299** | 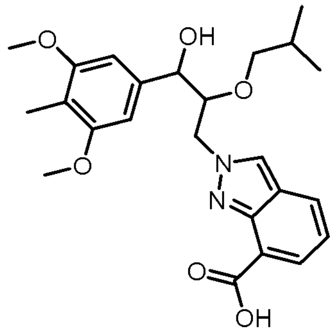 |
| **300** | 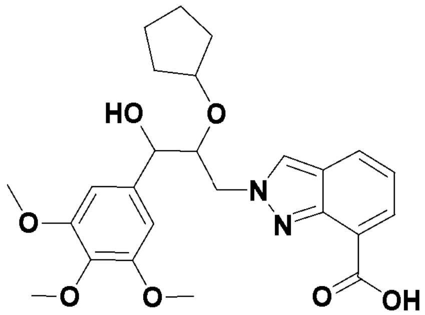 |
| **301** | 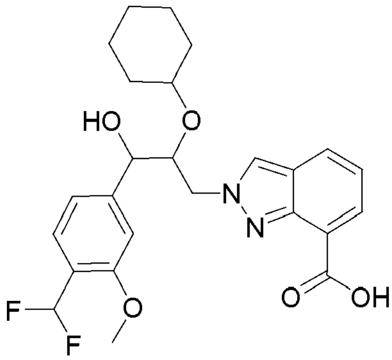 |
| **302** | 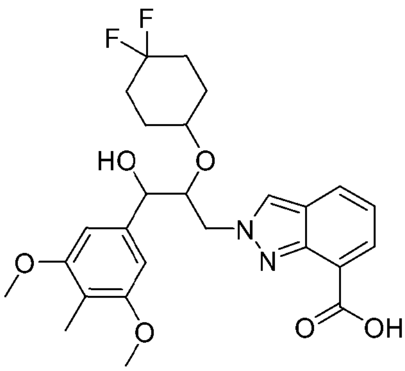 |

(continued)

| Compound | Structure |
|---|---|
| **303** | 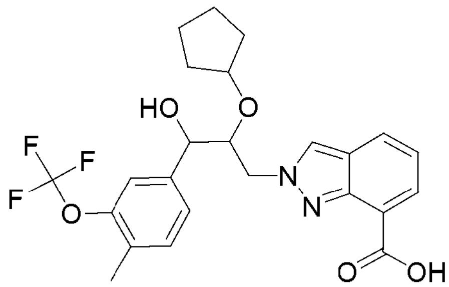 |
| **304** | 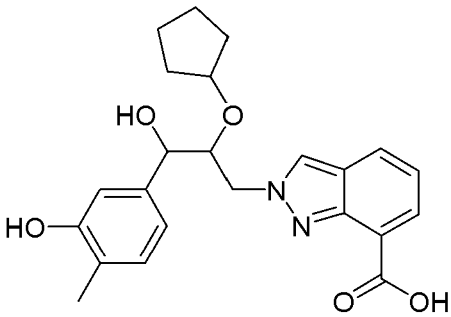 |
| **305** | 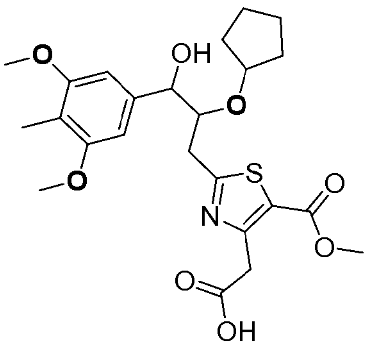 |
| **306** | 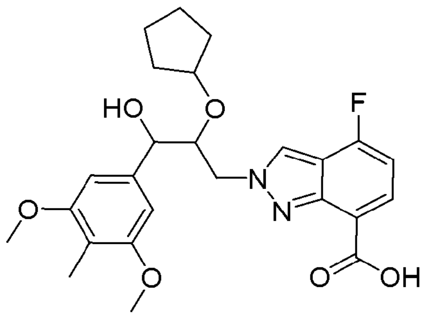 |
| **307** | 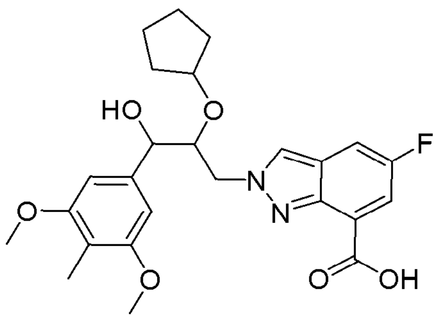 |

(continued)

| Compound | Structure |
|---|---|
| **308** | 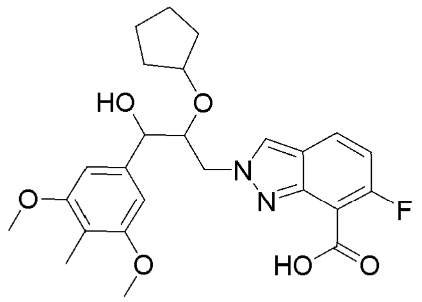 |
| **309** | 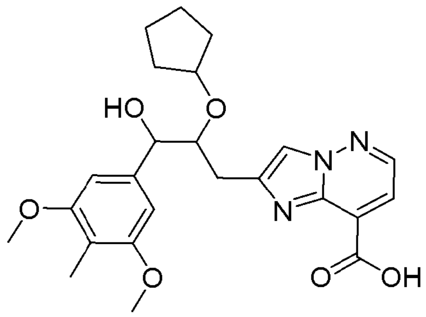 |
| **310** | 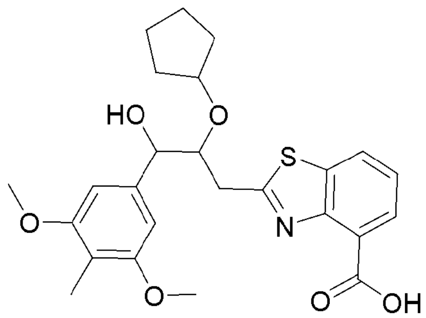 |
| **311** | 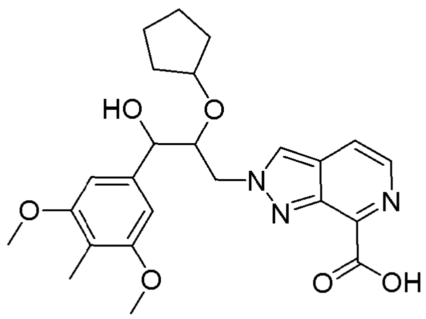 |
| **312** | 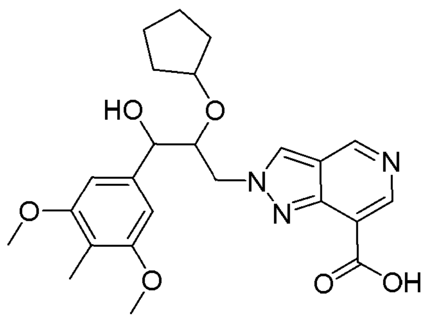 |

(continued)

| Compound | Structure |
| --- | --- |
| 313 | 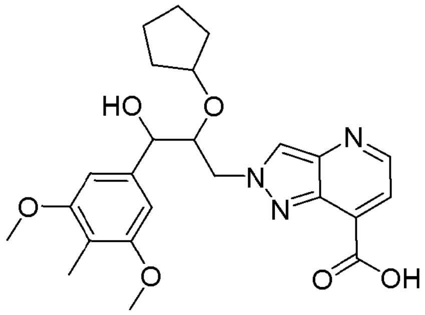 |
| 314 | 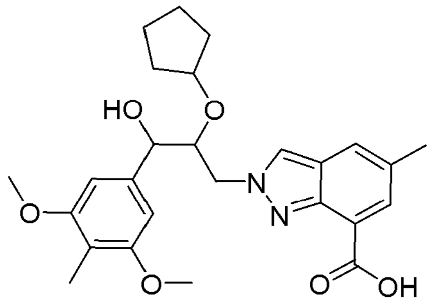 |
| 315 | 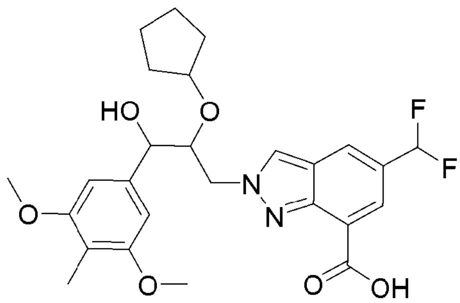 |
| 316 | 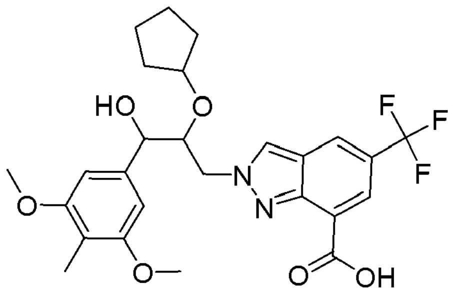 |
| 317 | 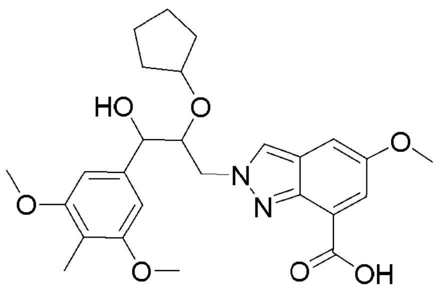 |

(continued)

| Compound | Structure |
|---|---|
| **318** | 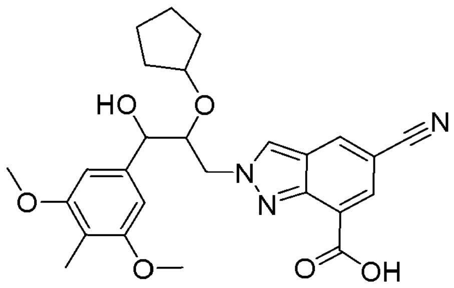 |
| **319** | 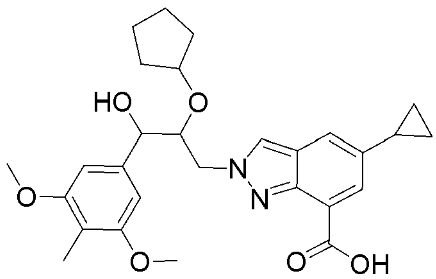 |
| **320** | 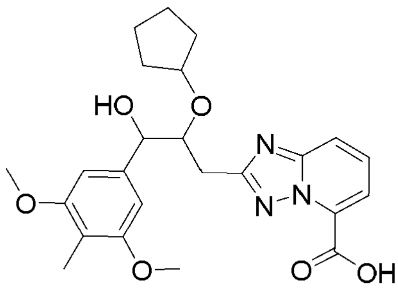 |
| **321** | 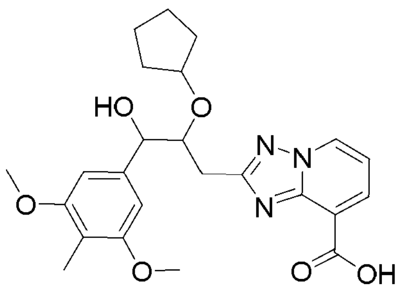 |
| **322** | 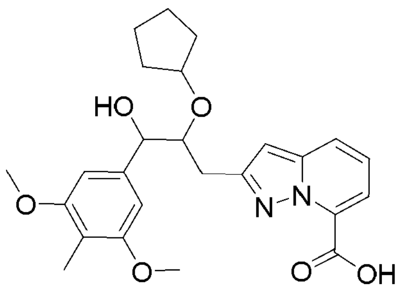 |

(continued)

| Compound | Structure |
|---|---|
| **323** | 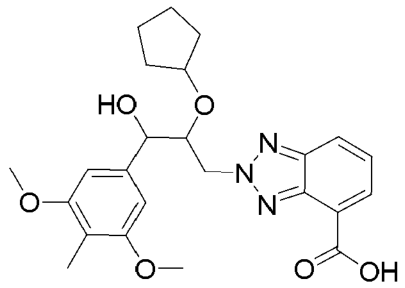 |
| **324** | 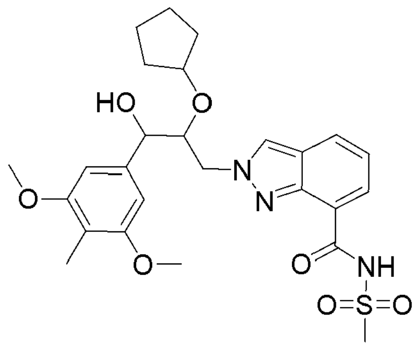 |
| **325** | 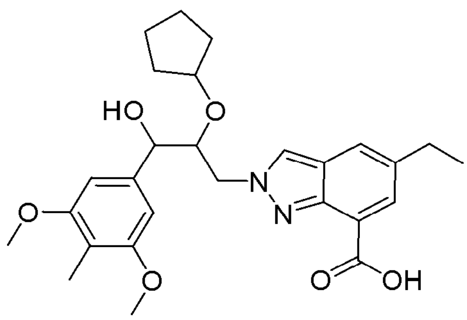 |
| **326** | 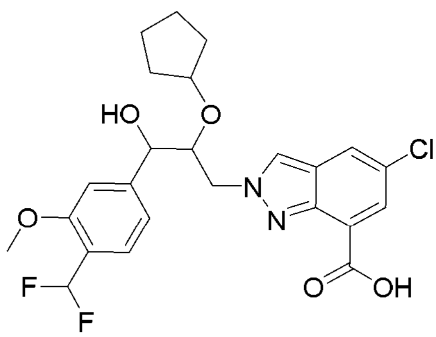 |
| **327** | 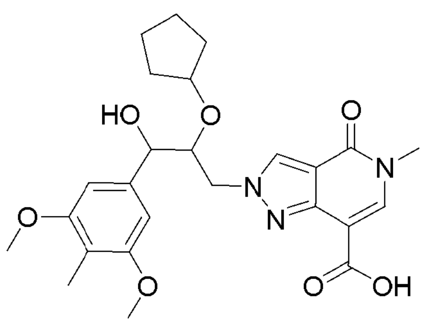 |

(continued)

| Compound | Structure |
|---|---|
| **328** | 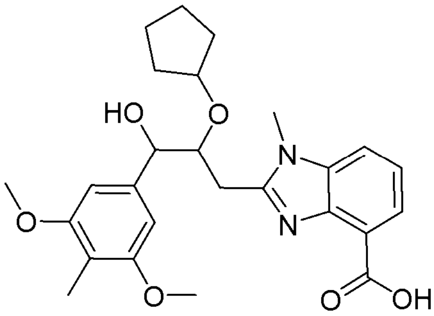 |
| **329** | 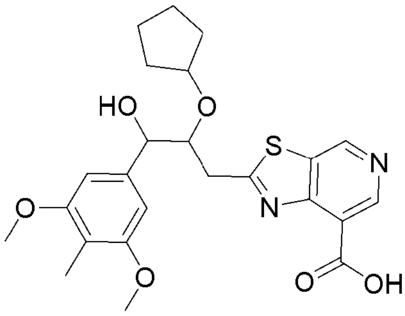 |
| **330** | 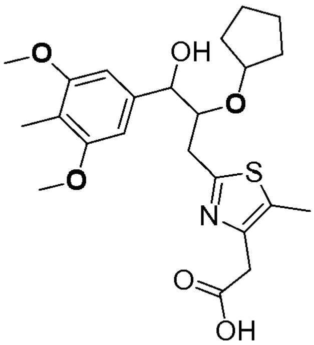 |
| **331** | 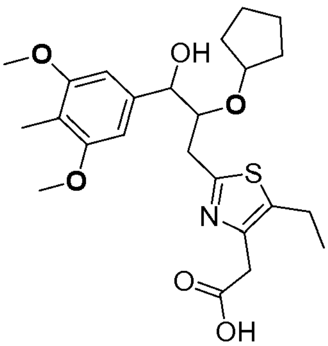 |

(continued)

| Compound | Structure |
|---|---|
| **332** | 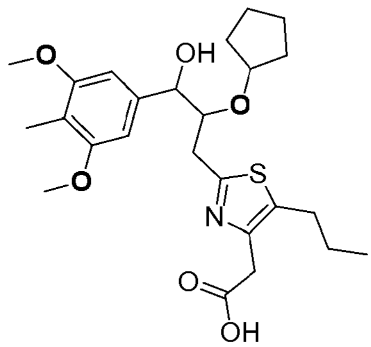 |
| **333** | 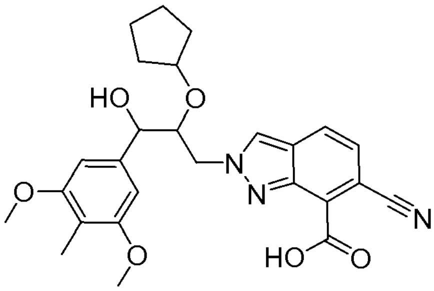 |
| **334** | 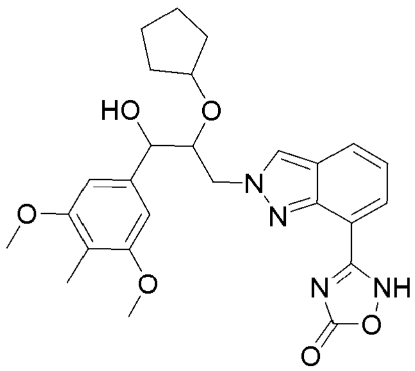 |
| **335** | 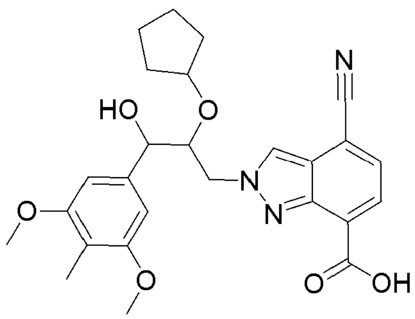 |
| **336** | 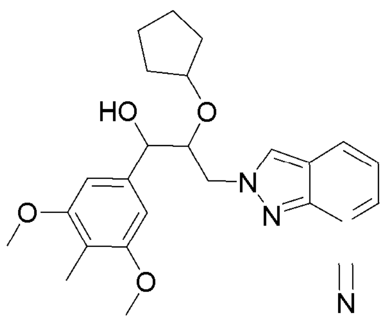 |

(continued)

| Compound | Structure |
|---|---|
| **337** | 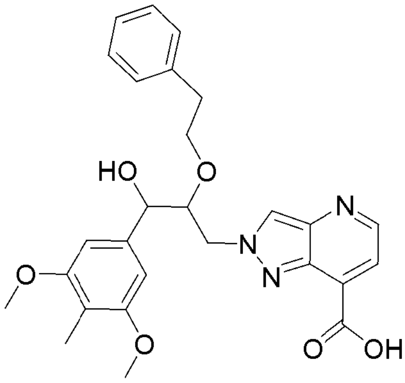 |
| **338** | 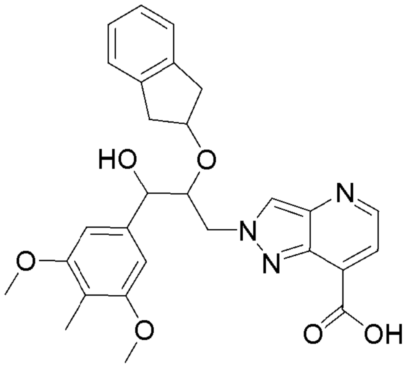 |
| **339** | 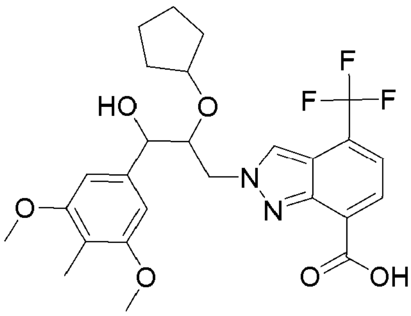 |
| **340** | 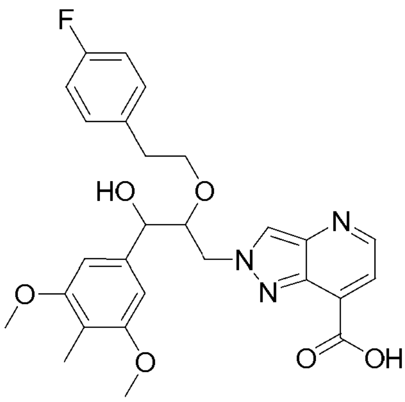 |

(continued)

| Compound | Structure |
|---|---|
| **341** | 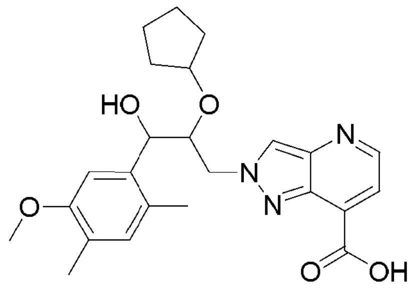 |
| **342** | 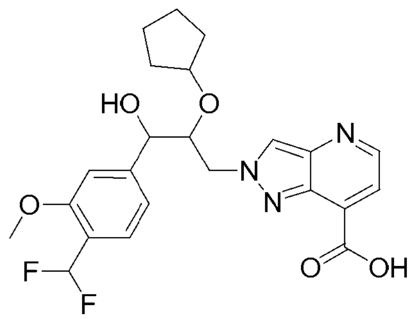 |
| **343** | 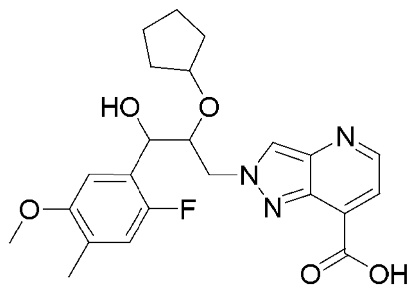 |
| **344** | 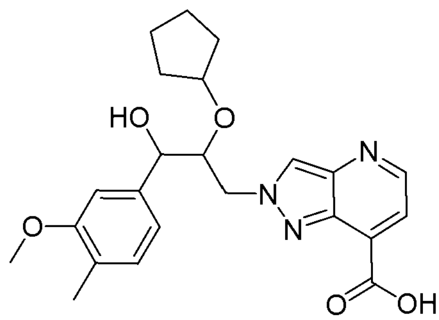 |
| **345** | 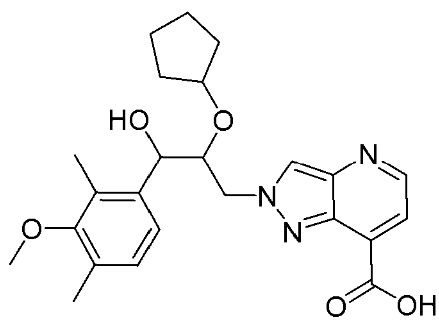 |

(continued)

| Compound | Structure |
|---|---|
| 346 | 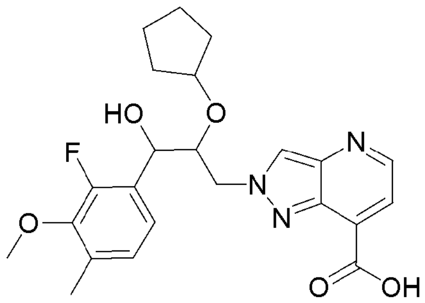 |
| 347 | 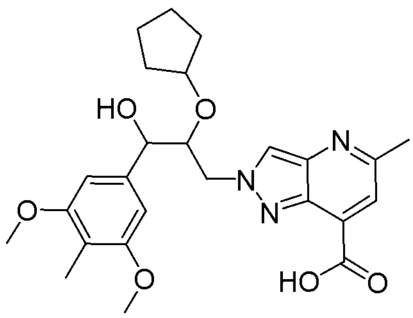 |
| 348 | 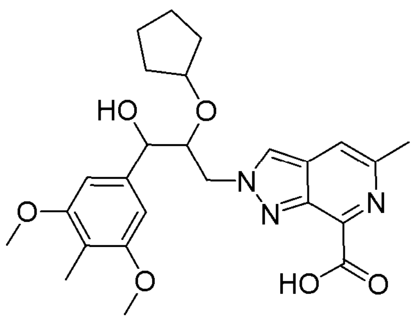 |
| 349 | 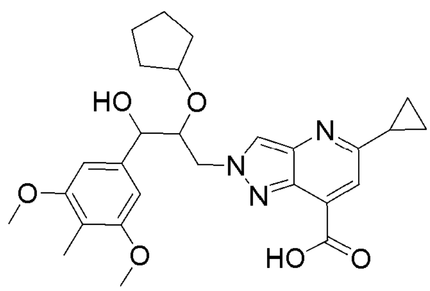 |
| 350 | 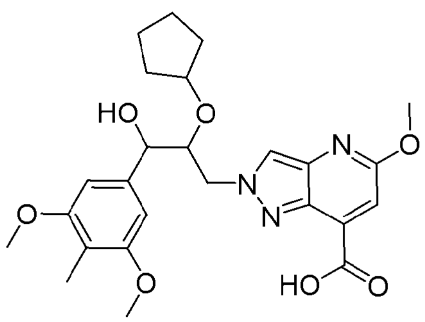 |

(continued)

| Compound | Structure |
|---|---|
| **351** | 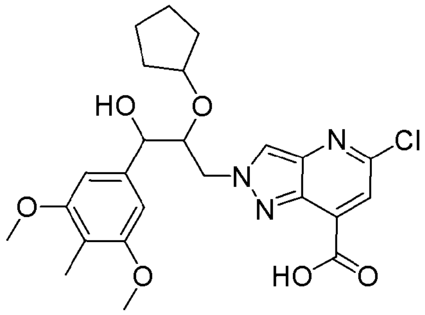 |
| **352** | 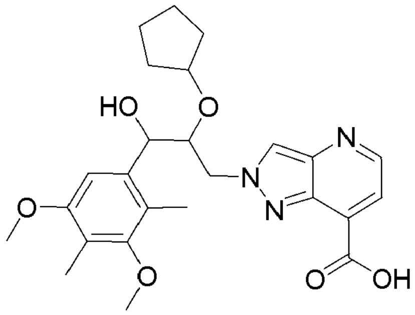 |
| **353** | 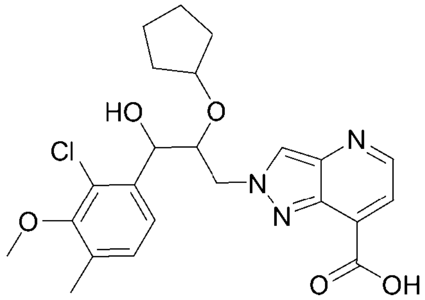 |
| **354** | 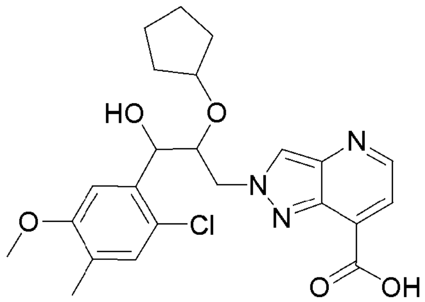 |
| **355** | 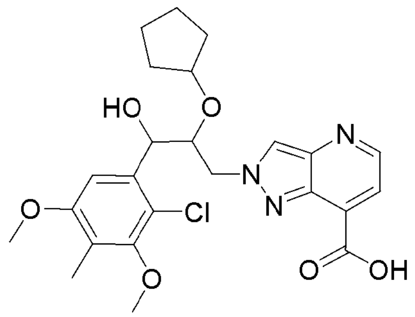 |

(continued)

| Compound | Structure |
|---|---|
| **356** | 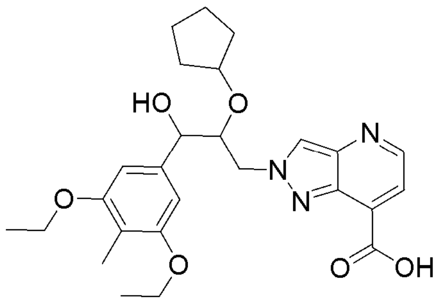 |
| **357** | 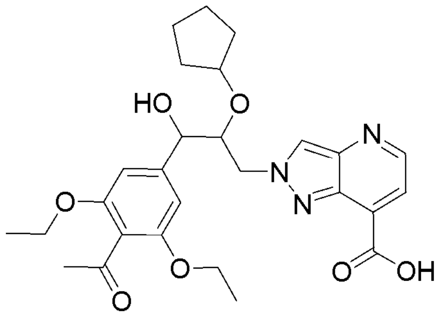 |
| **358** | 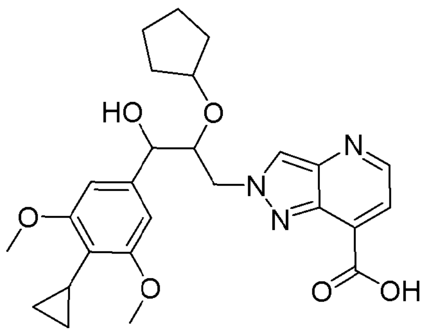 |
| **359** | 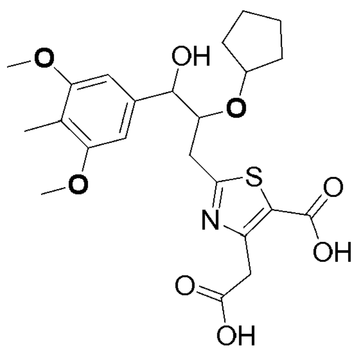 |
| **360** | 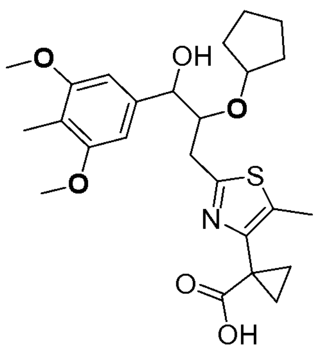 |

(continued)

| Compound | Structure |
|---|---|
| 361 | 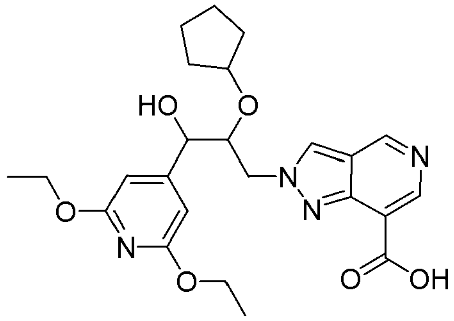 |
| 362 | 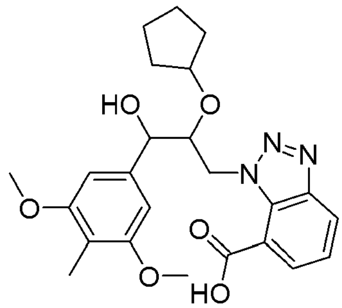 |
| 363 | 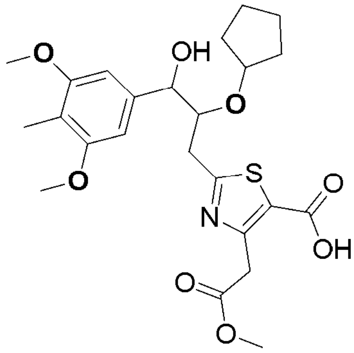 |
| 364 | 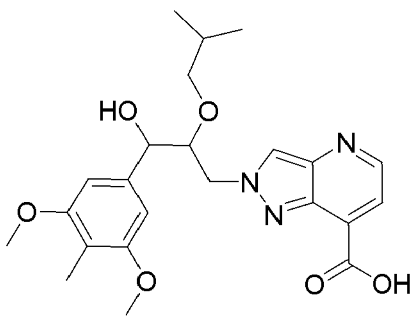 |
| 365 | 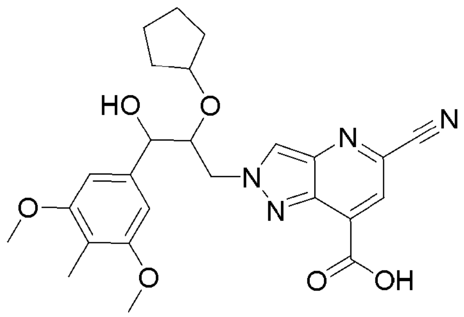 |

(continued)

| Compound | Structure |
|---|---|
| **366** | 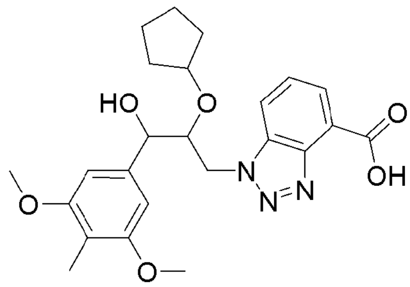 |
| **367** | 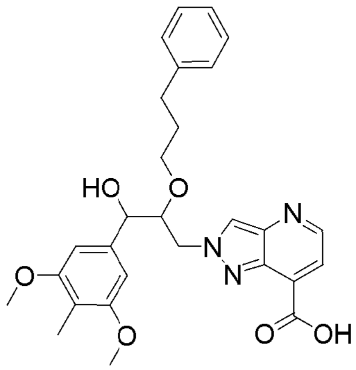 |
| **368** | 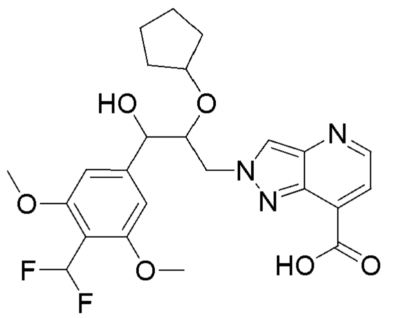 |
| **369** | 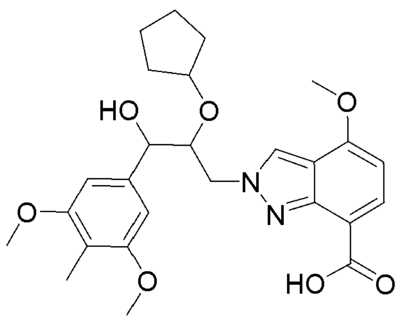 |
| **370** | 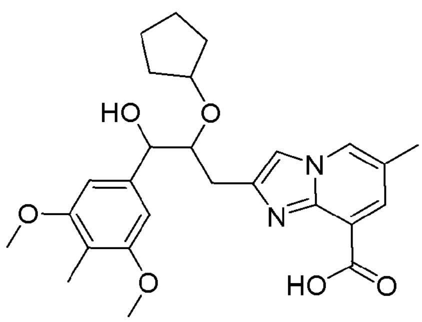 |

(continued)

| Compound | Structure |
|---|---|
| **371** | 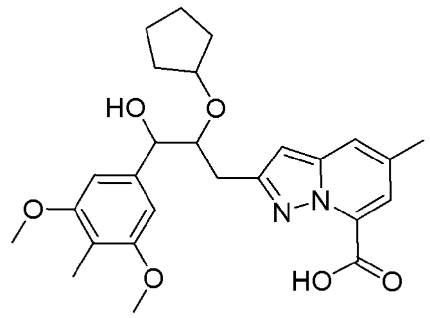 |
| **372** | 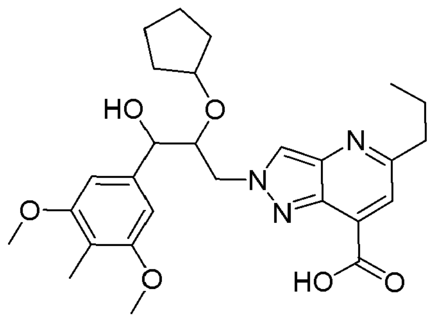 |
| **373** | 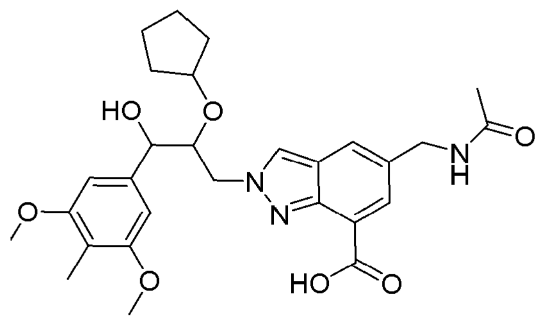 |
| **374** | 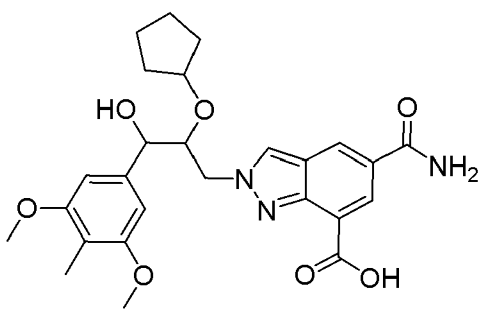 |
| **375** | 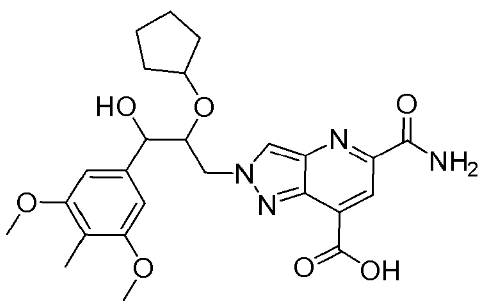 |

(continued)

| Compound | Structure |
|---|---|
| **376** | 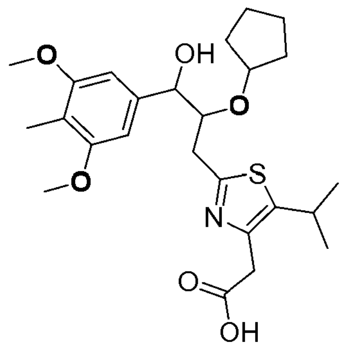 |
| **377** | 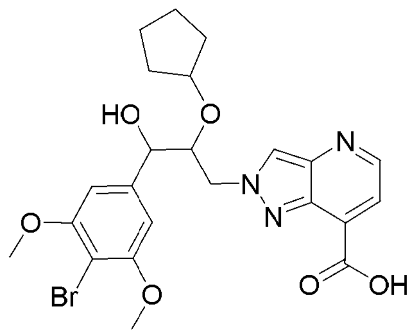 |
| **378** | 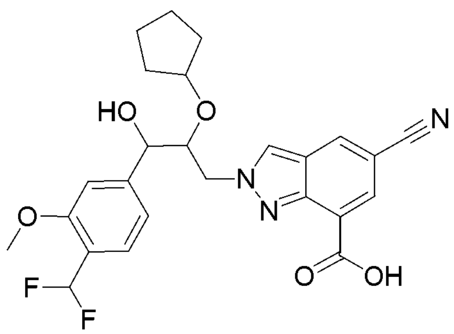 |
| **379** | 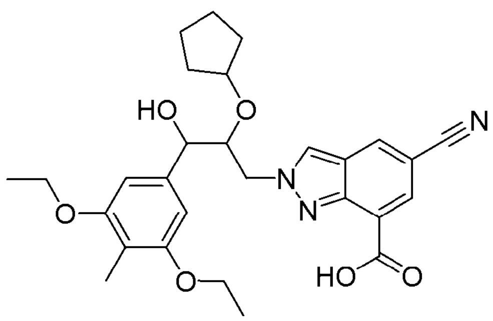 |
| **380** | 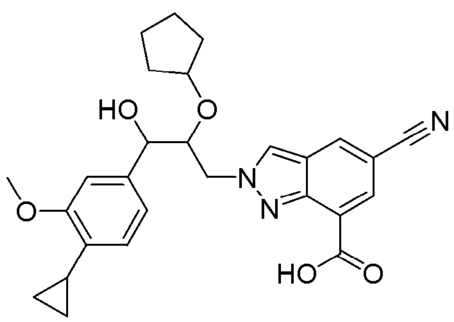 |

(continued)

| Compound | Structure |
|---|---|
| **381** | 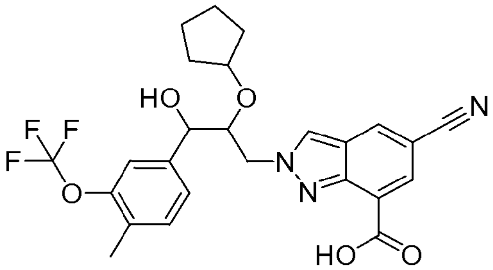 |
| **382** | 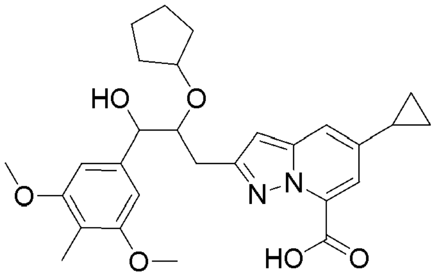 |
| **383** | 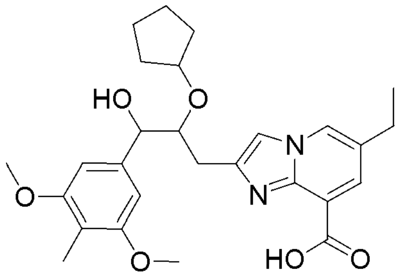 |
| **384** | 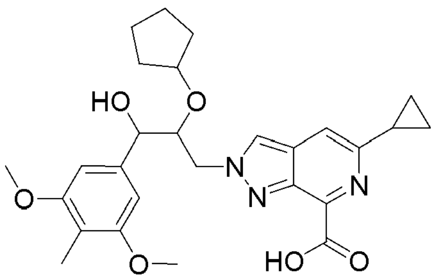 |
| **385** | 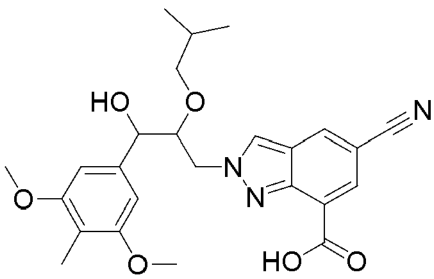 |

(continued)

| Compound | Structure |
|---|---|
| 386 | 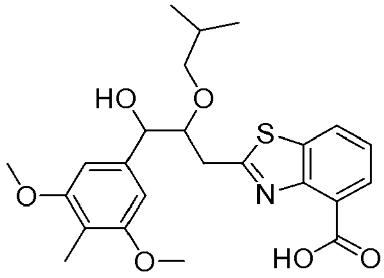 |
| 387 | 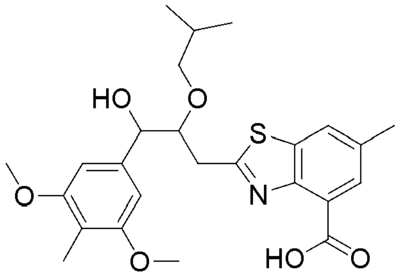 |
| 388 | 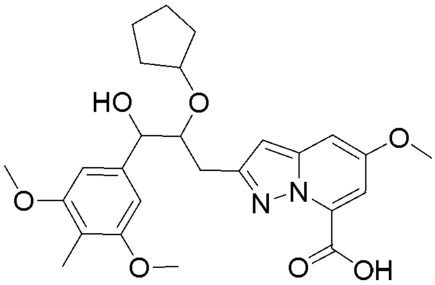 |
| 389 | 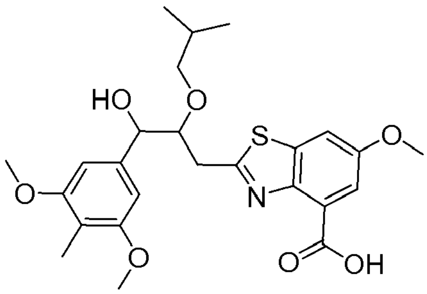 |
| 390 | 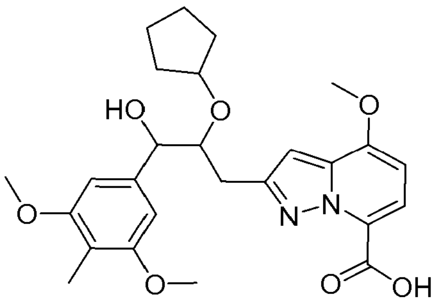 |

(continued)

| Compound | Structure |
|---|---|
| 391 | 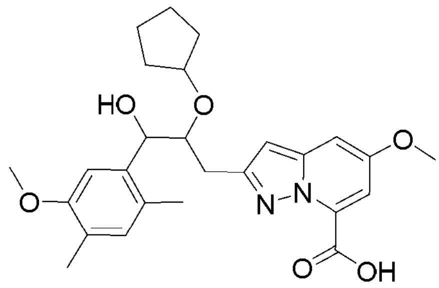 |
| 392 | 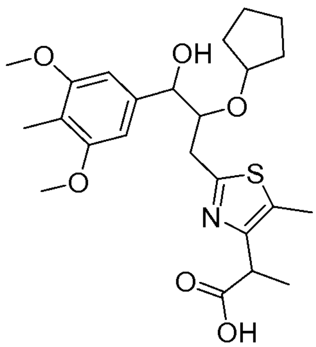 |
| 393 | 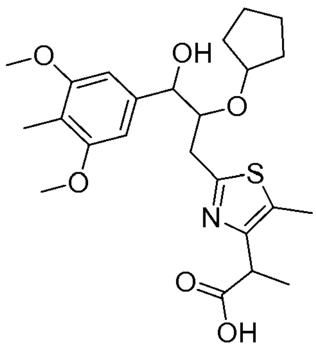 |
| 394 | 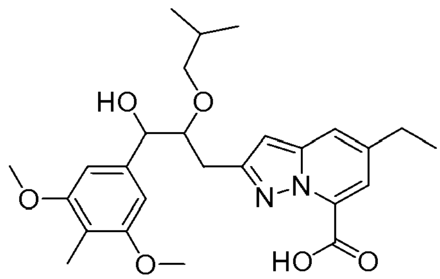 |
| 395 | 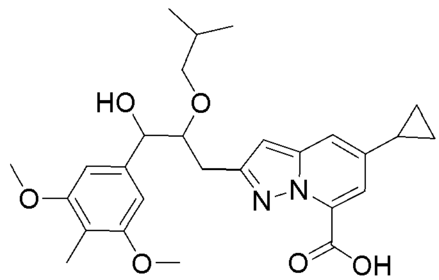 |

(continued)

| Compound | Structure |
|---|---|
| 396 | 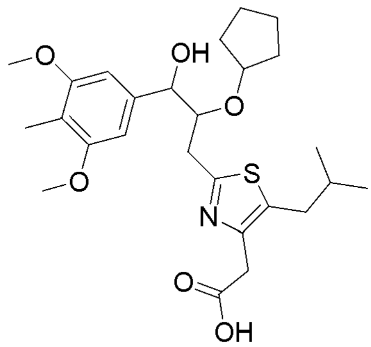 |
| 397 | 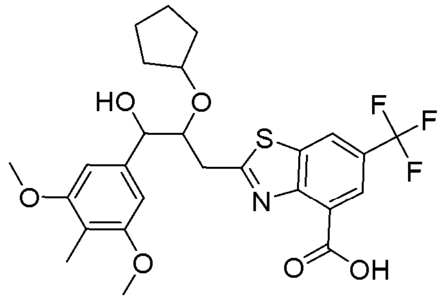 |
| 398 | 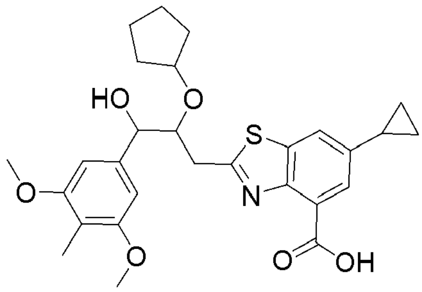 |
| 399 | 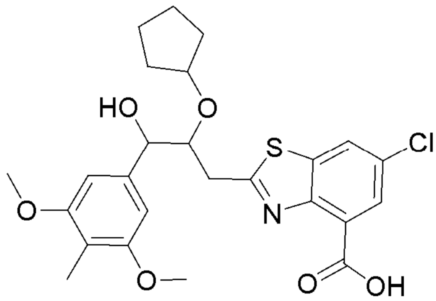 |
| 400 | 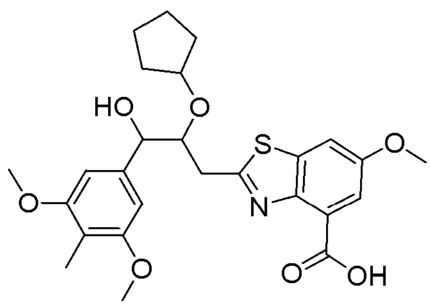 |

(continued)

| Compound | Structure |
|---|---|
| **401** | 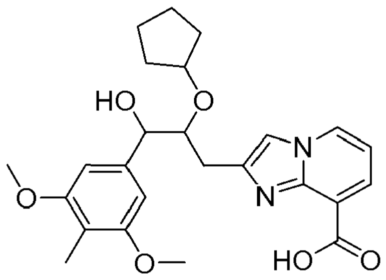 |
| **402** | 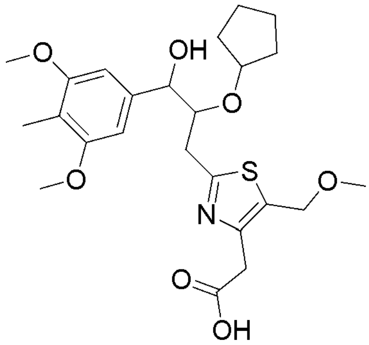 |
| **403** | 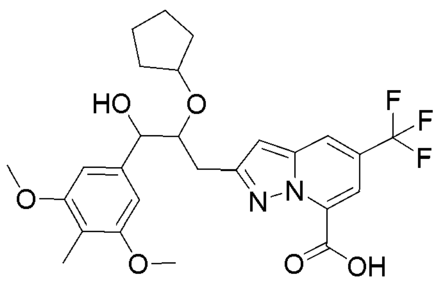 |
| **404** | 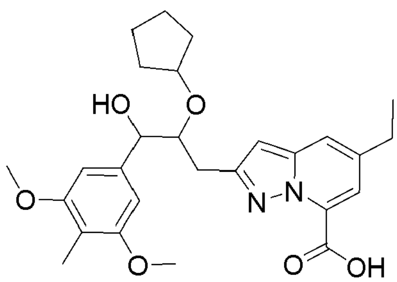 |
| **405** | 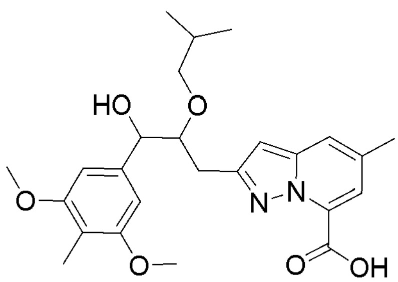 |

(continued)

| Compound | Structure |
|---|---|
| **406** | 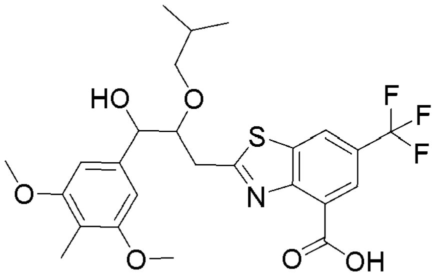 |
| **407** | 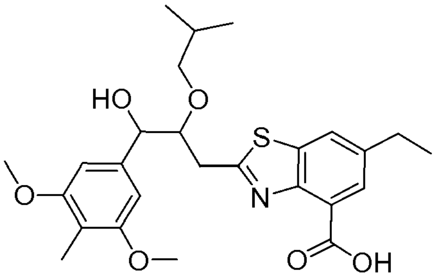 |
| **408** | 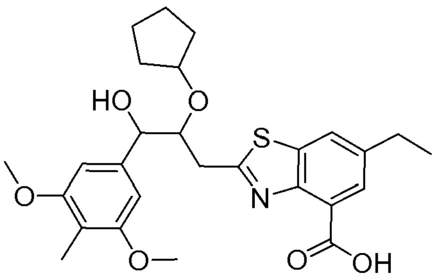 |
| **409** | 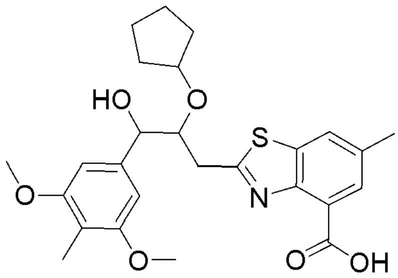 |
| **410** | 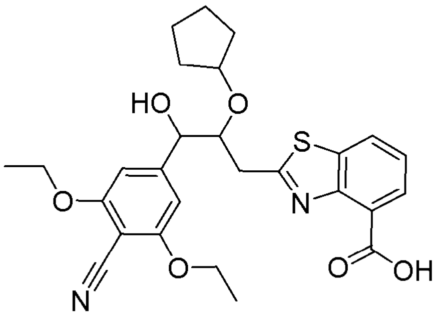 |

(continued)

| Compound | Structure |
|---|---|
| **411** | 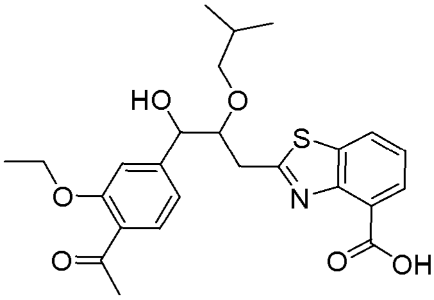 |
| **412** | 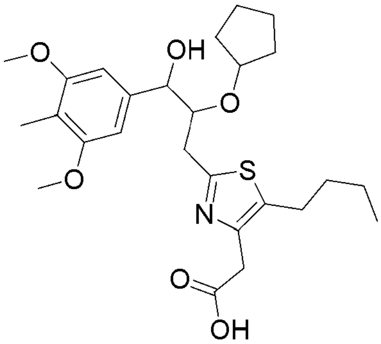 |
| **413** | 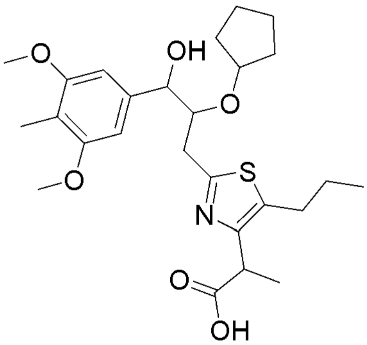 |
| **414** | 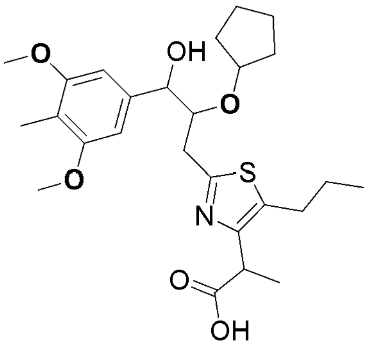 |
| **415** | 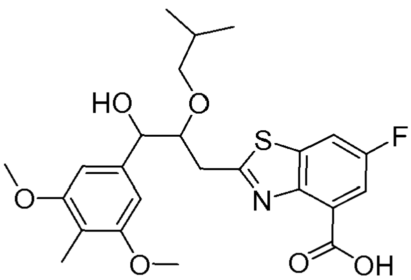 |

(continued)

| Compound | Structure |
|---|---|
| **416** | 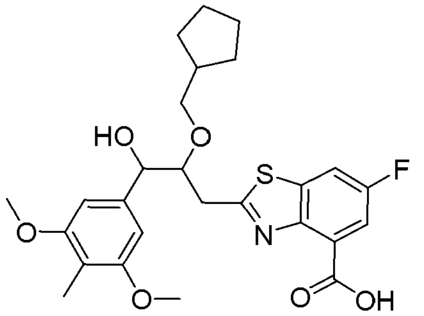 |
| **417** | 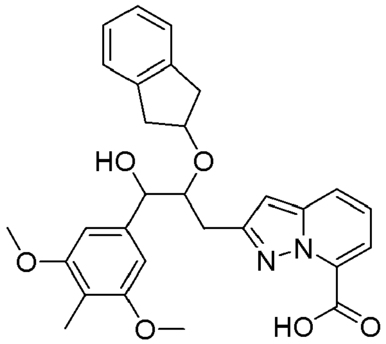 |
| **418** | 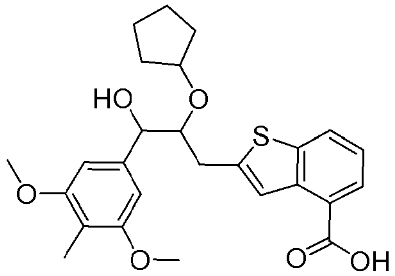 |
| **419** | 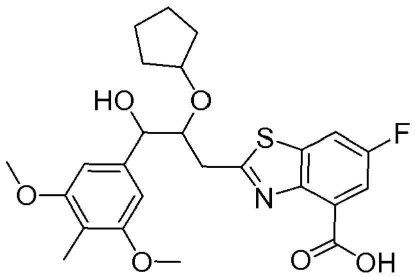 |
| **420** | 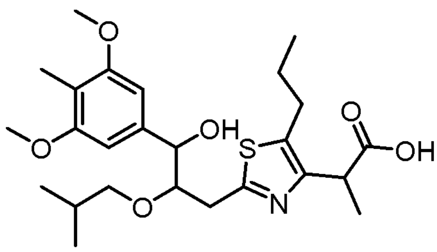 |

(continued)

| Compound | Structure |
|---|---|
| **421** | 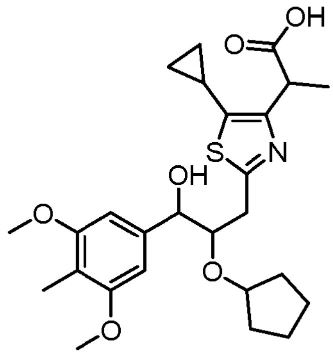 |
| **422** | 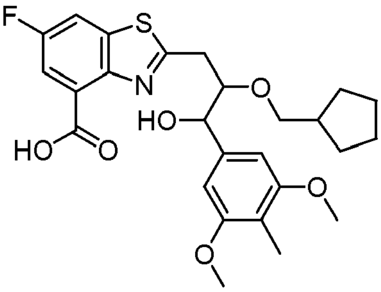 |
| **423** | 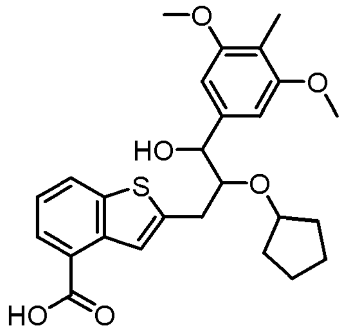 |
| **424** | 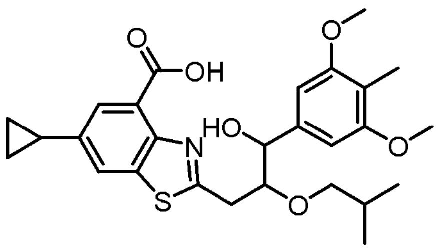 |
| **425** | 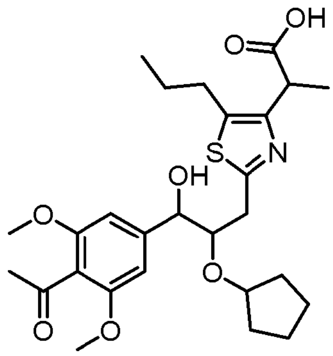 |

(continued)

| Compound | Structure |
|---|---|
| **426** | 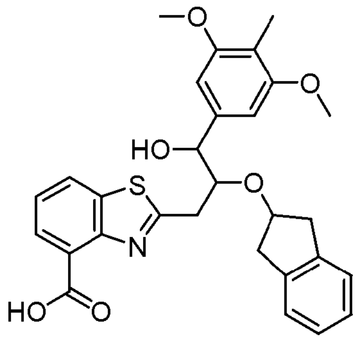 |
| **427** | 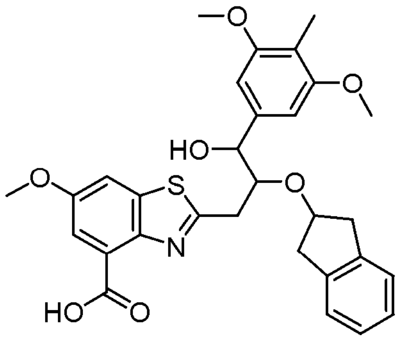 |
| **428** | 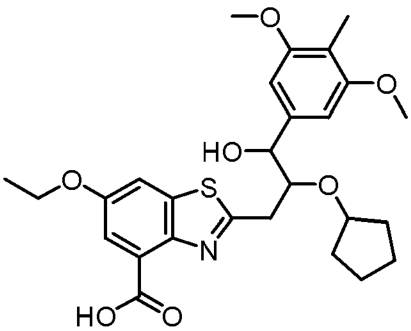 |
| **429** | 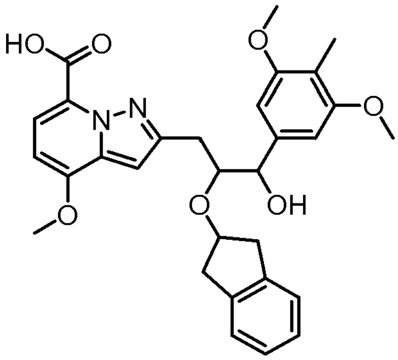 |

(continued)

| Compound | Structure |
|---|---|
| **430** | 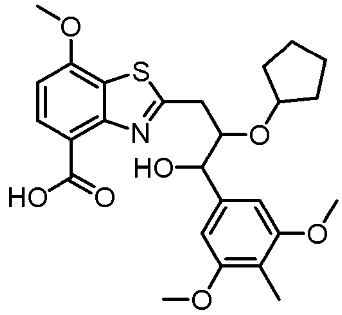 |
| **431** | 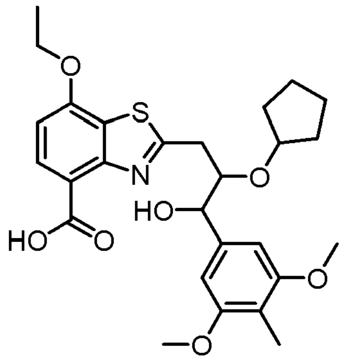 |
| **432** | 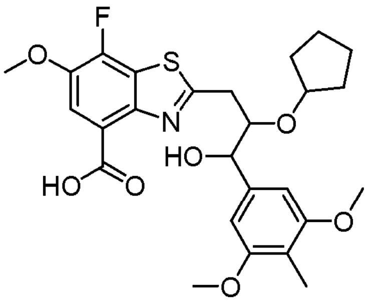 |
| **433** | 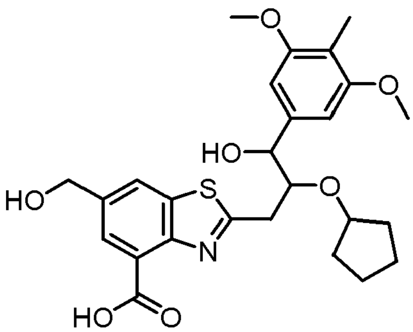 |
| **434** | 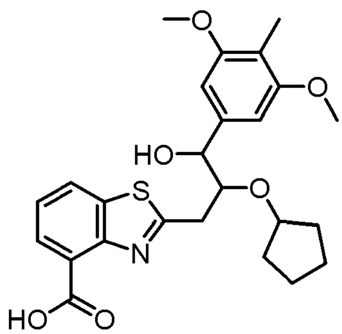 |

(continued)

| Compound | Structure |
|---|---|
| **435** | 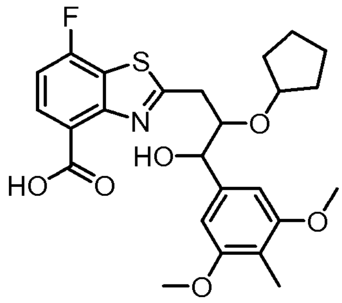 |
| **436** | 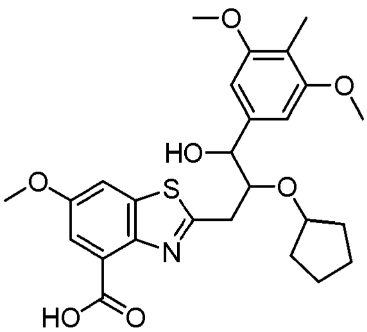 |
| **437** | 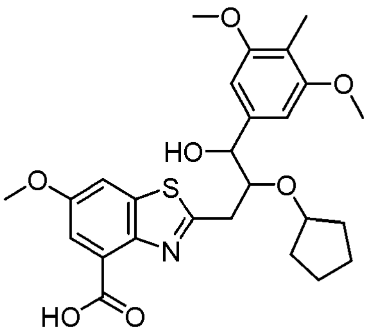 |
| **438** | 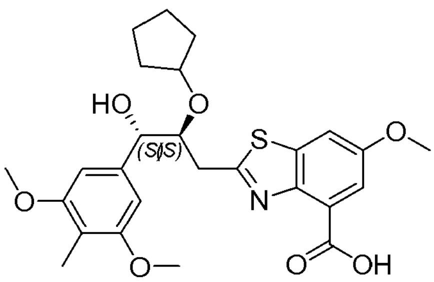 |
| **439** | 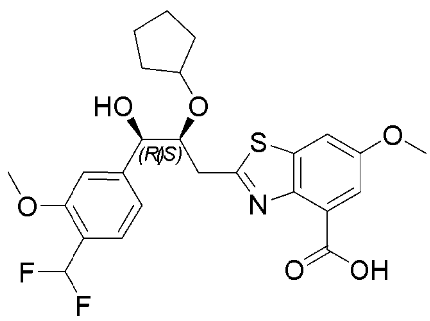 |

(continued)

| Compound | Structure |
|---|---|
| **440** | 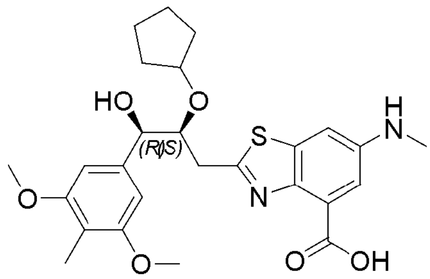 |
| **441** | 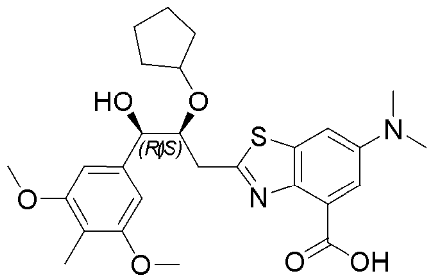 |
| **442** | 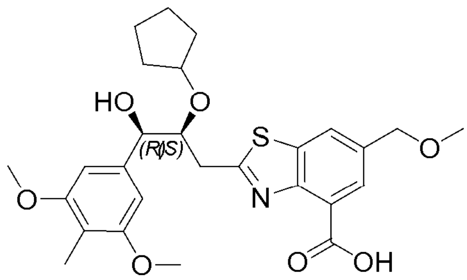 |
| **443** | 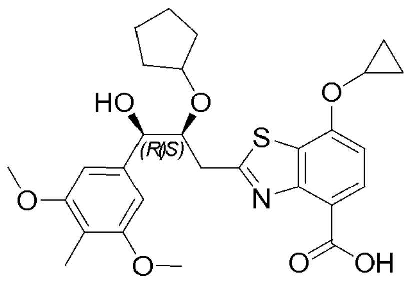 |
| **444** | 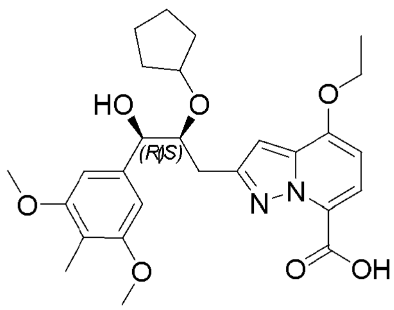 |

(continued)

| Compound | Structure |
|---|---|
| **445** | 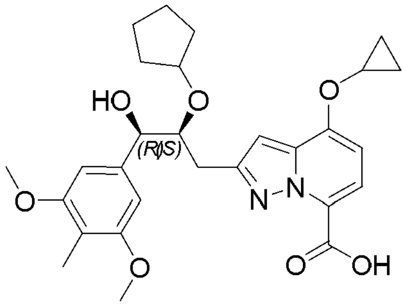 |
| **446** | 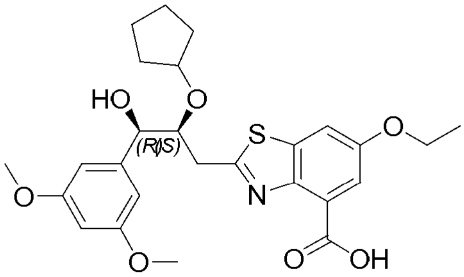 |
| **447** | 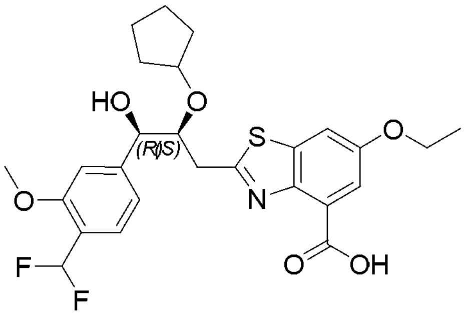 |
| **448** | 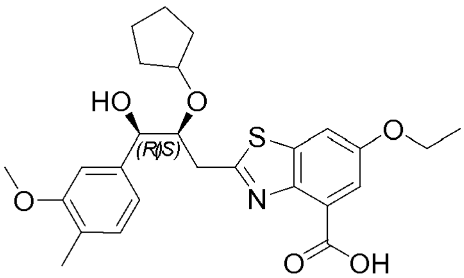 |
| **449** | 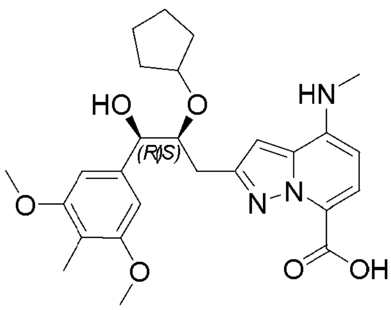 |

(continued)

| Compound | Structure |
|---|---|
| **450** | 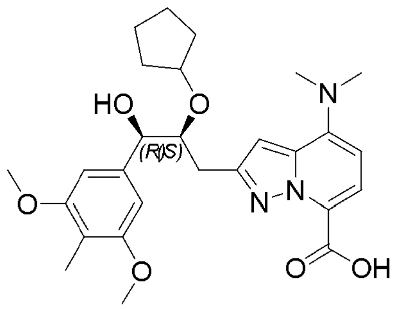 |
| **451** | 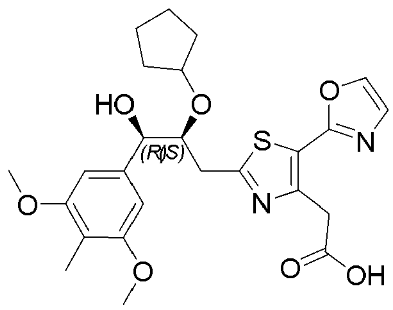 |

or wherein the compound is selected from the group consisting of compounds 101-437 and 101a-131a, or a pharmaceutically acceptable salt thereof.

**13.** A pharmaceutical composition comprising a compound of any one of claims 1-12, or a pharmaceutically acceptable salt or solvate thereof, and a pharmaceutically acceptable excipient.

**14.** A compound of any one of claims 1-12, or a pharmaceutically acceptable salt or solvate thereof, or a pharmaceutical composition according to claim 13, for use in a method for treating or preventing an LPA-associated disease in a subject in need thereof, the method comprising administering to subject a therapeutically effective amount of the compound, or a pharmaceutically acceptable salt or solvate thereof, or the pharmaceutical composition;

optionally wherein the LPA-associated disease is an $LPA_1$-associated disease; or
optionally wherein the LPA-associated disease is selected from the group consisting of fibrosis, transplant rejection, cancer, osteoporosis, or inflammatory disorders;

optionally wherein the cancer is of the bladder, blood, bone, brain, breast, central nervous system, cervix, colon, endometrium, esophagus, gall bladder, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid; or
optionally wherein the fibrosis is pulmonary, liver, renal, cardiac, dermal, ocular, or pancreatic fibrosis; or

optionally wherein the LPA-associated disease is selected from the group consisting of idiopathic pulmonary fibrosis (IPF), non-alcoholic steatohepatitis (NASH), non-alcoholic fatty liver disease (NAFLD), chronic kidney disease, diabetic kidney disease, and systemic sclerosis.

**15.** A compound of any one of claims 1-12, or a pharmaceutically acceptable salt or solvate thereof, or a pharmaceutical composition according to claim 13, for use in a method for treating or preventing fibrosis in a subject in need thereof, the method comprising administering to subject a therapeutically effective amount of the compound, or a pharmaceutically acceptable salt or solvate thereof, or the pharmaceutical composition;
optionally wherein the fibrosis is idiopathic pulmonary fibrosis (IPF), nonalcoholic steatohepatitis (NASH), chronic kidney disease, diabetic kidney disease, and systemic sclerosis; optionally wherein the fibrosis is IPF.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FUJIWARA et al.** *J Biol. Chem.*, 2005, vol. 280, 35038-35050
- **LIPINSKI**. Annual Reports in Medicinal Chemistry. *Bioisosterism In Drug Design*, 1986, vol. 21, 283
- **YUN** ; **HWAHAK SEKYE**. *Application Of Bioisosterism To New Drug Design*, 1993, vol. 33, 576-579
- **ZHAO** ; **HUAXUE TONGBAO**. *Bioisosteric Replacement And Development Of Lead Compounds In Drug Design*, 1995, 34-38
- **GRAHAM**. *Theochem*, 1995, vol. 343, 105-109
- Remington: The Science and Practice of Pharmacy. Lippincott Williams & Wilkins, 2005
- Handbook of Pharmaceutical Excipients. The Pharmaceutical Press and the American Pharmaceutical Association, 2009
- Handbook of Pharmaceutical Additives. Gower Publishing Company, 2007
- Pharmaceutical Preformulation and Formulation. CRC Press LLC, 2009
- Pharmaceutical Dosage Forms. Revised and Expanded, vol. 1-3
- Pharmaceutical Dosage Forms. Parenteral Medications, vol. 1-2
- Pharmaceutical Dosage Forms. Disperse Systems. Marcel Dekker, Inc., vol. 1-2
- Remington: The Science and Practice of Pharmacy. Pharmaceutical Press, 2012
- **YAMADA**. *Cancer Sci.*, 2008, vol. 99 (8), 1603-1610
- **BOUCHARABA et al.** *J Clin. Invest.*, 2004, vol. 114 (12), 1714-1725
- **BOUCHARABA et al.** *Proc. Natl. acad Sci.*, 2006, vol. 103 (25), 9643-9648